(19) Europäisches Patentamt
European Patent Office
Office européen des brevets

(11) EP 4 335 855 B1

(12) EUROPEAN PATENT SPECIFICATION

(45) Date of publication and mention of the grant of the patent:
**04.06.2025 Bulletin 2025/23**

(21) Application number: **23193479.5**

(22) Date of filing: **25.08.2023**

(51) International Patent Classification (IPC):
***C07F 5/02*** *(2006.01)* ***C09K 11/06*** *(2006.01)*
***H10K 85/30*** *(2023.01)* ***H10K 85/60*** *(2023.01)*

(52) Cooperative Patent Classification (CPC):
**C09K 11/06; C07F 5/027; H10K 85/6572; H10K 85/658;** H10K 50/11

(54) **HETEROCYCLIC COMPOUND, ORGANIC LIGHT-EMITTING DEVICE INCLUDING THE SAME, AND DIAGNOSTIC COMPOSITION INCLUDING THE HETEROCYCLIC COMPOUND**

HETEROCYCLISCHE VERBINDUNG, ORGANISCHE LICHTEMITTIERENDE VORRICHTUNG DAMIT UND DIAGNOSTISCHE ZUSAMMENSETZUNG MIT DER HETEROCYCLISCHEN VERBINDUNG

COMPOSÉ HÉTÉROCYCLIQUE, DISPOSITIF ÉLECTROLUMINESCENT ORGANIQUE LE COMPRENANT ET COMPOSITION DE DIAGNOSTIC COMPRENANT LE COMPOSÉ HÉTÉROCYCLIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.08.2022 KR 20220108725**

(43) Date of publication of application:
**13.03.2024 Bulletin 2024/11**

(73) Proprietor: **Samsung Electronics Co., Ltd.**
**Suwon-si, Gyeonggi-do 16677 (KR)**

(72) Inventors:
- **LEE, Eunkyung**
  **16678 Suwon-si (KR)**
- **KIM, Juhyun**
  **16678 Suwon-si (KR)**
- **KIM, Sangmo**
  **16678 Suwon-si (KR)**
- **MARUYAMA, Yusuke**
  **16678 Suwon-si (KR)**
- **JUNG, Yongsik**
  **16678 Suwon-si (KR)**
- **KIM, Jiwhan**
  **16678 Suwon-si (KR)**
- **SON, Youngmok**
  **16678 Suwon-si (KR)**
- **IHN, Sooghang**
  **16678 Suwon-si (KR)**
- **CHWAE, Jun**
  **16678 Suwon-si (KR)**

(74) Representative: **Elkington and Fife LLP**
**Prospect House**
**8 Pembroke Road**
**Sevenoaks, Kent TN13 1XR (GB)**

(56) References cited:
**CN-A- 114 369 108 KR-A- 20220 073 182**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 335 855 B1

## Description

FIELD OF THE INVENTION

The disclosure relates to a heterocyclic compound, an organic light-emitting device including the same, and a diagnostic composition including the heterocyclic compound.

BACKGROUND OF THE INVENTION

Organic light-emitting devices are self-emissive devices, which have improved characteristics in terms of viewing angles, response time, luminance, driving voltage, and response speed, and produce full-color images.

An organic light-emitting device may include an anode, a cathode, and an organic layer that is located between the anode and the cathode and includes an emission layer. A hole transport region may be arranged between the anode and the emission layer, and an electron transport region may be arranged between the emission layer and the cathode. Holes provided from the anode move toward the emission layer through the hole transport region, and electrons provided from the cathode move toward the emission layer through the electron transport region. The holes and the electrons recombine in the emission layer to produce excitons. These excitons transition from an excited state to the ground state to thereby generate light. Organic light-emitting devices are known from KR 2022 0073182 A or CN 114 369 108 A, for instance.

SUMMARY OF THE INVENTION

Provided are a heterocyclic compound, an organic light-emitting device including the same, and a diagnostic composition including the heterocyclic compound.

Additional aspects will be set forth in part in the description which follows and, in part, will be apparent from the description, or may be learned by practice of the presented embodiments of the disclosure.

According to one or more embodiments, there is provided a heterocyclic compound represented by Formula 1

Formula 1

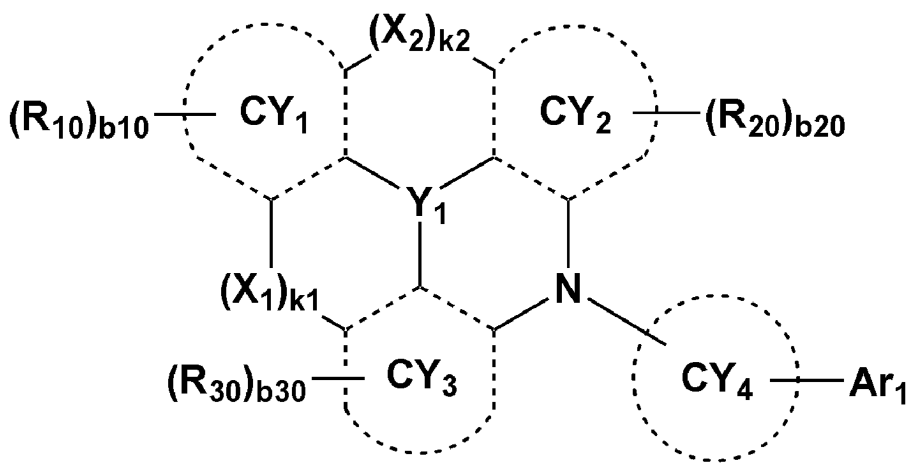

Formula 2

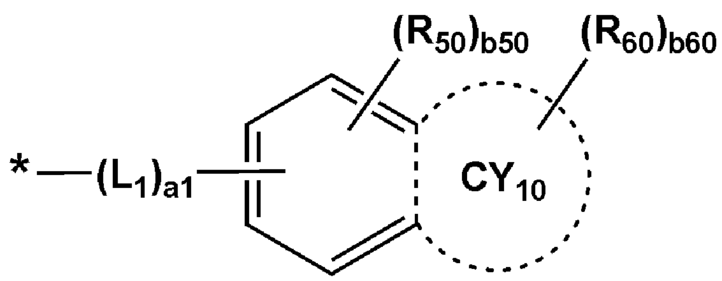

In Formulae 1 and 2,

$Y_1$ is B, N, P, P(=O), or P(=S),
$X_1$ and $X_2$ are each independently a single bond, O, S, Se, N[($CY_5$)-$Ar_2$], N($R_1$), C($R_1$)($R_2$), Si($R_1$)($R_2$), Ge($R_1$)($R_2$), B($R_1$), P($R_1$), P(=O)($R_1$), $S(=O)_2$, or C(=O),
k1 is 0 or 1,
k2 is 0 or 1,
the sum of k1 and k2 is greater than or equal to 1,
$CY_1$ to $CY_3$ are each independently a $C_5$-$C_{30}$ carbocyclic group or a substituted or unsubstituted $C_1$-$C_{30}$ heterocyclic group,
$CY_4$ and $CY_5$ are each independently a $C_5$-$C_{30}$ carbocyclic group that is unsubstituted or substituted with at least one $R_{40}$ or a $C_1$-$C_{30}$ heterocyclic group that is unsubstituted or substituted with at least one $R_{40}$,
$Ar_1$ and $Ar_2$ are each independently a group represented by Formula 2,
$L_1$ is a single bond, a substituted or unsubstituted $C_5$-$C_{30}$ carbocyclic group, or a substituted or unsubstituted $C_1$-$C_{30}$ heterocyclic group,
a1 is 1, 2, or 3,
$CY_{10}$ is a $C_3$-$C_{10}$ non-aromatic carbocyclic group or a $C_1$-$C_{10}$ non-aromatic heterocyclic group,
$R_1$, $R_2$, $R_{10}$, $R_{20}$, $R_{30}$, and $R_{40}$ are each independently a group represented by Formula 2, hydrogen, deuterium, -F, -Cl, -Br, -I, $-SF_5$, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_1$)($Q_2$)($Q_3$), -Ge($Q_1$)($Q_2$)($Q_3$),-C(=O)($Q_1$), -S(=O)($Q_1$), $-S(=O)_2(Q_1)$, -B($Q_1$)($Q_2$), -P($Q_1$)($Q_2$), -P(=O)($Q_1$)($Q_2$),-P(=S)($Q_1$)($Q_2$), or a group represented by Formula 3,
$R_{50}$ and $R_{60}$ are each independently hydrogen, deuterium, -F, -Cl, -Br, -I, $-SF_5$, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_1$)($Q_2$)($Q_3$), -Ge($Q_1$)($Q_2$)($Q_3$),-C(=O)($Q_1$), -S(=O)($Q_1$), $-S(=O)_2(Q_1)$, -B($Q_1$)($Q_2$), -P($Q_1$)($Q_2$), -P(=O)($Q_1$)($Q_2$),-P(=S)($Q_1$)($Q_2$), or a group represented by Formula 3, , wherein at least one of R10, at least one of R20, and/or at least one of R30 is a group represented by Formula 3;

Formula 3

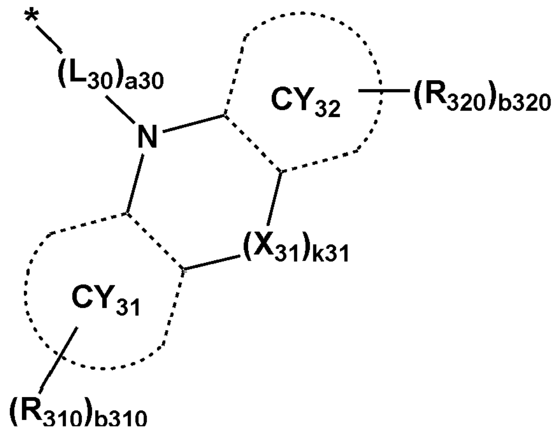

wherein, in Formula 3,

$X_{31}$ is a single bond, O, S, $N(R_{301})$, or $C(R_{301})(R_{302})$,
k31 is 1,
$CY_{31}$ and $CY_{32}$ are each independently a $C_5$-$C_{30}$ carbocyclic group or a $C_1$-$C_{30}$ heterocyclic group,
$L_{30}$ is a single bond, a substituted or unsubstituted $C_5$-$C_{30}$ carbocyclic group, or a substituted or unsubstituted $C_1$-$C_{30}$ heterocyclic group,
a30 is 0, 1, 2, or 3,
$R_{301}$, $R_{302}$, $R_{310}$, and $R_{320}$ are each independently hydrogen, deuterium, -F, -Cl, -Br, -I, $-SF_5$, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_1)(Q_2)(Q_3)$, $-Ge(Q_1)(Q_2)(Q_3)$,$-C(=O)(Q_1)$, $-S(=O)(Q_1)$, $-S(=O)_2(Q_1)$, $-B(Q_1)(Q_2)$, $-P(Q_1)(Q_2)$, $-P(=O)(Q_1)(Q_2)$, or $-P(=S)(Q_1)(Q_2)$,
b310 and b320 are each independently 1, 2, 3, 4, 5, 6, 7, or 8,
b10, b20, b30, b40, and b60 are each independently 1, 2, 3, 4, 5, 6, 7, or 8,
b50 is 1, 2, or 3,
a substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkylaryl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkylheteroaryl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group,
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$,$-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a

carboxylic acid or a salt thereof, a sulfonic acid or a salt thereof, a phosphoric acid or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{11})(Q_{12})$, $-Si(Q_{11})(Q_{12})(Q_{13})$, $-Ge(Q_{11})(Q_{12})(Q_{13})$,$-C(=O)(Q_{11})$, $-S(=O)(Q_{11})$, $-S(=O)_2(Q_{11})$, $-B(Q_{11})(Q_{12})$, $-P(Q_{11})(Q_{12})$,$-P(=O)(Q_{11})(Q_{12})$, $-P(=S)(Q_{11})(Q_{12})$, or any combination thereof,

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{21})(Q_{22})$, $-Si(Q_{21})(Q_{22})(Q_{23})$,$-Ge(Q_{21})(Q_{22})(Q_{23})$, $-C(=O)(Q_{21})$, $-S(=O)(Q_{21})$, $-S(=O)_2(Q_{21})$, $-B(Q_{21})(Q_{22})$,$-P(Q_{21})(Q_{22})$, $-P(=O)(Q_{21})(Q_{22})$, $-P(=S)(Q_{21})(Q_{22})$, or any combination thereof,

$-N(Q_{31})(Q_{32})$, $-Si(Q_{31})(Q_{32})(Q_{33})$, $-Ge(Q_{31})(Q_{32})(Q_{33})$, $-C(=O)(Q_{31})$,$-S(=O)(Q_{31})$, $-S(=O)_2(Q_{31})$, $-B(Q_{31})(Q_{32})$, $-P(Q_{31})(Q_{32})$, $-P(=O)(Q_{31})(Q_{32})$, or$-P(Q_{31})(Q_{32})$,

$Q_1$ to $Q_3$, $Q_{11}$ to $Q_{13}$, $Q_{21}$ to $Q_{23}$, and $Q_{31}$ to $Q_{33}$ are each independently hydrogen, deuterium, -F, -Cl, -Br, -I, a hydroxyl group, a cyano group, a nitro group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group that is unsubstituted or substituted with deuterium, a $C_1$-$C_{60}$ alkyl group, a $C_6$-$C_{60}$ aryl group, or any combination thereof, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group that is unsubstituted or substituted with deuterium, a $C_1$-$C_{60}$ alkyl group, a $C_6$-$C_{60}$ aryl group, or any combination thereof, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, and

* may indicate a binding site to a neighboring atom.

According to one or more embodiments, an organic light-emitting device includes the heterocyclic compound.

According to one or more embodiments, a diagnostic composition includes the heterocyclic compound.

BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 is a schematic cross-sectional view of an organic light-emitting device according to an exemplary embodiment;
FIG. 2 shows energy transfer of an organic light-emitting device according to an exemplary embodiment;
FIG. 3 shows energy transfer of an organic light-emitting device according to an exemplary embodiment;
FIG. 4 shows energy transfer of an organic light-emitting device according to an exemplary embodiment;
FIG. 5 shows energy transfer of an organic light-emitting device according to an exemplary embodiment; and
FIG. 6 shows energy transfer of an organic light-emitting device according to an exemplary embodiment.

DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly on the other element or intervening elements may be present therebetween. In contrast, when an element is referred to as being

"directly on" another element, there are no intervening elements present

It will be understood that, although the terms "first," "second," "third" etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, "a first element," "component," "region," "layer" or "section" discussed below could be termed a second element, component, region, layer or section without departing from the teachings herein.

The terminology used herein is for the purpose of describing particular embodiments only and is not intended to be limiting. As used herein, "a," "an," "the," and "at least one" do not denote a limitation of quantity, and are intended to cover both the singular and plural, unless the context clearly indicates otherwise. For example, "an element" has the same meaning as "at least one element," unless the context clearly indicates otherwise.

"Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower," can therefore, encompasses both an orientation of "lower" and "upper," depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within $\pm$ 30%, 20%, 10% or 5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded Thus, the regions illustrated in the figures are schematic in nature and their shapes are not intended to illustrate the precise shape of a region and are not intended to limit the scope of the present claims.

A heterocyclic compound according to an embodiment is represented by Formula 1:

Formula 1

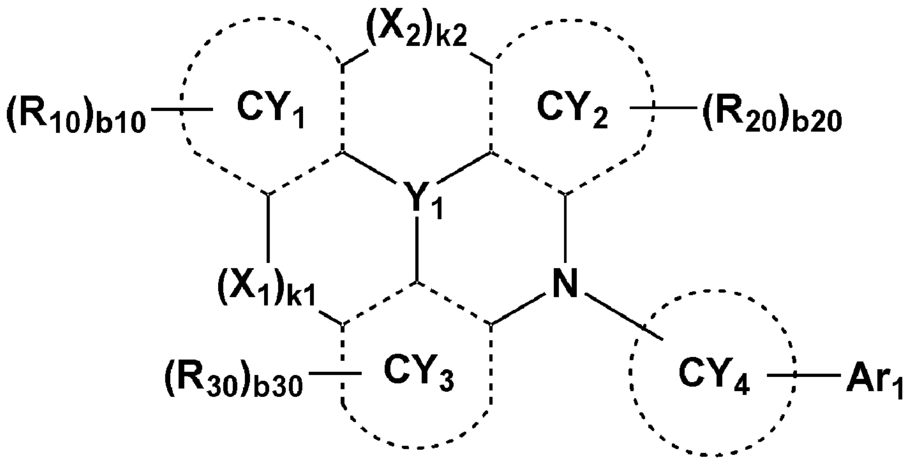

wherein, in Formula 1, $Y_1$ may be B, N, P, P(=O), or P(=S).

In an embodiment, $Y_1$ may be B, N, or P.

For example, $Y_1$ may be B.

In Formula 1, $X_1$ and $X_2$ may each independently be a single bond, O, S, Se, N[($CY_5$)-$Ar_2$], N($R_1$), C($R_1$)($R_2$), Si($R_1$)($R_2$), Ge($R_1$)($R_2$), B($R_1$), P($R_1$), P(=O)($R_1$), $S(=O)_2$, or C(=O).

In an embodiment, $X_1$ and $X_2$ may each independently be a single bond, O, S, Se, N[($CY_5$)-$Ar_2$], N($R_1$), C($R_1$)($R_2$), or Si($R_1$)($R_2$).

In Formula 1, k1 may be 0 or 1. When k1 is 0, $CY_1$ and $CY_3$ are not linked to each other via $X_1$.

In Formula 1, k2 may be 0 or 1. When k2 is 0, $CY_1$ and $CY_2$ are not linked to each other via $X_2$.

In Formula 1, the sum of k1 and k2 may be greater than or equal to 1.

For example, at least one of k1 and k2 may be 1.

In Formula 1, $CY_1$ to $CY_3$ may each independently a $C_5$-$C_{30}$ carbocyclic group or a substituted or unsubstituted $C_1$-$C_{30}$ heterocyclic group.

In an embodiment, $CY_1$ to $CY_3$ may each independently be i) a first ring, ii) a second ring, iii) a condensed ring in which two or more first rings are condensed with each other, iv) a condensed ring in which two or more second rings are condensed with each other, or v) a condensed ring in which at least one first ring is condensed with at least one second ring.

The first ring may be a cyclopentane group, a cyclopentadiene group, a furan group, a thiophene group, a pyrrole group, a silole group, an indene group, a benzofuran group, a benzothiophene group, an indole group, a benzosilole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group, and

the second ring may be an adamantane group, a norbornene group, a bicyclo[1.1.1]pentane group, a bicyclo[2.1.1]hexane group, a bicyclo[2.2.1]heptane group(norbornane group), a bicyclo[2.2.2]octane group, a cyclohexane group, a cyclohexene group, a benzene group, a pyridine group, a pyrimidine group, a pyrazine group, a pyridazine group, or a triazine group.

In an embodiment, $CY_1$ to $CY_3$ may each independently be a $C_6$-$C_{30}$ aromatic carbocyclic group or a $C_1$-$C_{30}$ aromatic heterocyclic group.

In an embodiment, $CY_1$ to $CY_3$ may each independently be a benzene group, a naphthalene group, an anthracene group, a phenanthrene group, a triphenylene group, a pyrene group, a chrysene group, a 1,2,3,4-tetrahydronaphthalene group, a benzothiophene group, a benzofuran group, an indole group, an indene group, a benzosilole group, a benzoborole group, a benzophosphole group, a benzoselenophene group, a benzogermole group, a dibenzothiophene group, a dibenzofuran group, a carbazole group, a fluorene group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridine group,

a pyrimidine group, a pyrazine group, a pyridazine group, a triazine group, a quinoline group, an isoquinoline group, a quinoxaline group, a quinazoline group, a phenanthroline group, a 5,6,7,8-tetrahydroisoquinoline group, a 5,6,7,8-tetrahydroquinoline group, adamantane group, a norbornane group, or a norbornene group.

In an embodiment, $CY_1$ to $CY_3$ may each independently be a benzene group, a naphthalene group, a phenanthrene group, a pyridine group, a pyrimidine group, a quinoline group, an isoquinoline group, a phthalazine group, a naphthyridine group, a quinoxaline group, or a quinazoline group.

In Formula 1, $CY_4$ and $CY_5$ may each independently be a $C_5$-$C_{30}$ carbocyclic group that is unsubstituted or substituted with at least one $R_{40}$ or a $C_1$-$C_{30}$ heterocyclic group that is unsubstituted or substituted with at least one $R_{40}$.

In an embodiment, $CY_4$ and $CY_5$ may each independently be a benzene group, a naphthalene group, an anthracene group, a phenanthrene group, a triphenylene group, a pyrene group, a chrysene group, a 1,2,3,4-tetrahydronaphthalene group, a benzothiophene group, a benzofuran group, an indole group, an indene group, a benzosilole group, a benzoborole group, a benzophosphole group, a benzoselenophene group, a benzogermole group, a dibenzothiophene group, a dibenzofuran group, a carbazole group, a fluorene group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a 5,6,7,8-tetrahydroisoquinoline group, a 5,6,7,8-tetrahydroquinoline group, an adamantane group, a norbornane group, or a norbornene group.

In an embodiment, $CY_4$ and $CY_5$ may each independently be a benzene group, a naphthalene group, a phenanthrene group, a pyridine group, a pyrimidine group, a quinoline group, an isoquinoline group, a phthalazine group, a naphthyridine group, a quinoxaline group, or a quinazoline group, each unsubstituted or substituted with at least one $R_{40}$.

In an embodiment, $CY_4$ and $CY_5$ may each independently be a benzene group that is unsubstituted or substituted with at least one $R_{40}$.

In an embodiment, $CY_4$ and $CY_5$ may each independently be of Formulae 4-1 to 4-3:

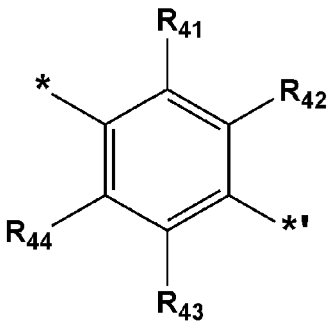

4-1

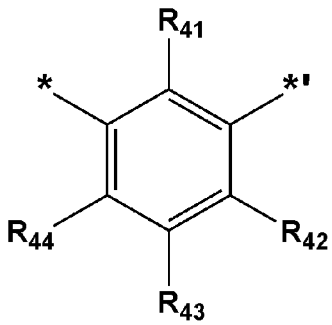

4-2

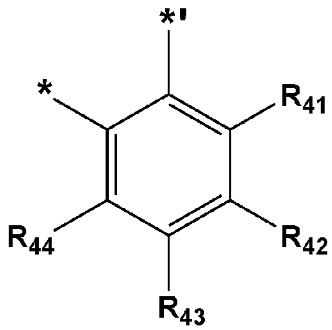

4-3

wherein, in Formulae 4-1 to 4-3,

$R_{41}$ to $R_{44}$ may each independently be the same as described in connection with $R_{40}$, and
* and *' may each indicate a binding site to a neighboring atom.

In Formula 1, $Ar_1$ and $Ar_2$ may each independently be a group represented by Formula 2.

Formula 2

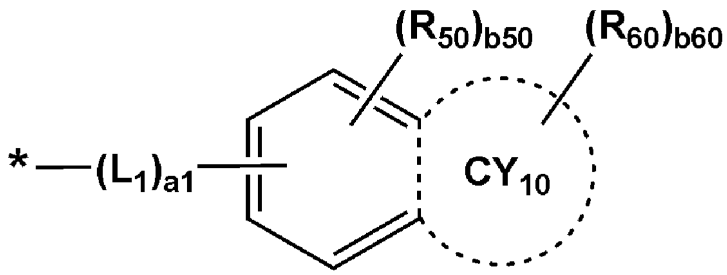

In Formula 2, $L_1$ may be a single bond, a substituted or unsubstituted $C_5$-$C_{30}$ carbocyclic group or a substituted or unsubstituted $C_1$-$C_{30}$ heterocyclic group.

In an embodiment, $L_1$ may be a single bond.

In Formula 2, a1 may be 1, 2, or 3.

In an embodiment, a1 may be 1.

In Formula 2, $CY_{10}$ may be a $C_3$-$C_{10}$ non-aromatic carbocyclic group or a $C_1$-$C_{10}$ non-aromatic heterocyclic group.

In an embodiment, $CY_{10}$ may be a cyclopentane group, a cyclohexane group, a cycloheptane group, a cyclopentene group, a cyclohexene group, or a cycloheptene group.

In Formula 2, * may indicate a binding site to a neighboring atom.

In an embodiment, $Ar_1$ and $Ar_2$ may each independently be a group represented by Formula 2A or 2B.

Formula 2A

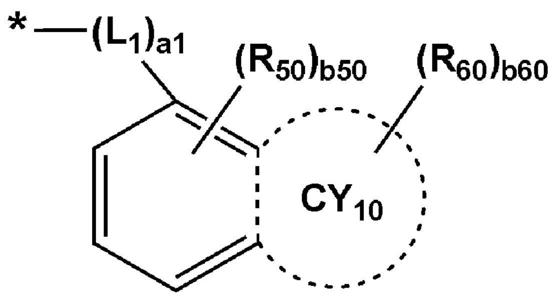

Formula 2B

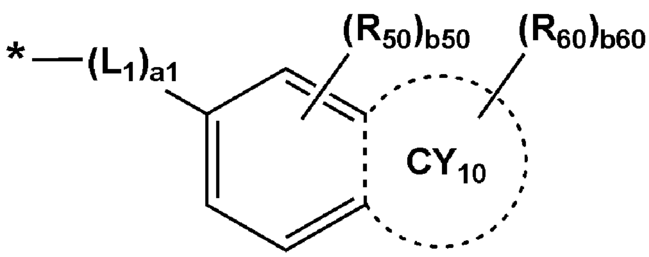

wherein, in Formulae 2A and 2B,

$L_1$, a1, $CY_{10}$, $R_{50}$, $R_{60}$, b50, and b60 may each be the same as described in the present specification, and
* may indicate a binding site to a neighboring atom.

In an embodiment, $Ar_1$ and $Ar_2$ may each independently be a group represented by one of Formulae 5-1 to 5-4:

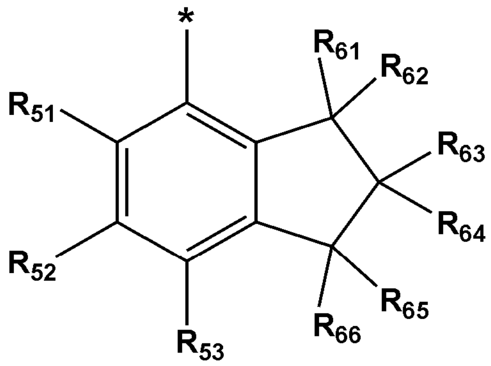

**5-1**

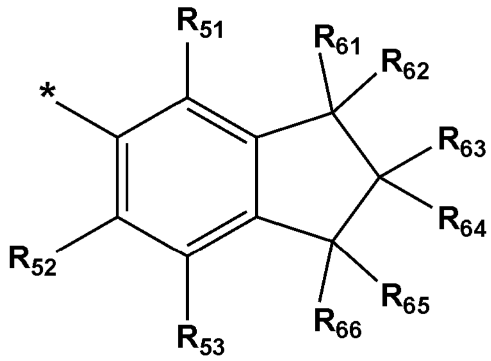

**5-2**

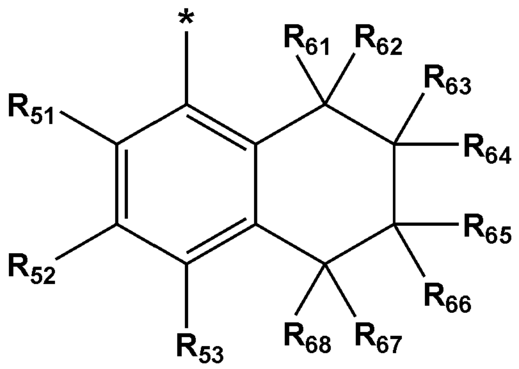

**5-3**

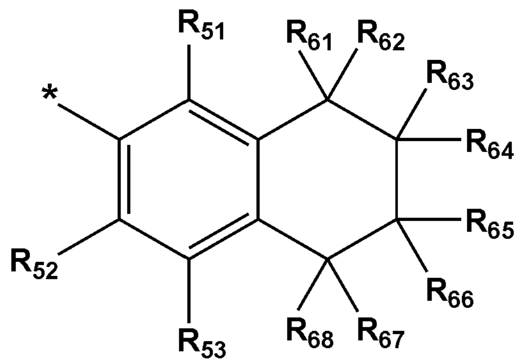

**5-4**

wherein, in Formulae 5-1 to 5-4,

$R_{51}$ to $R_{53}$ may each be the same as described in connection with $R_{50}$,
$R_{61}$ to $R_{68}$ may each be the same as described in connection with $R_{60}$, and
* may indicate a binding site to a neighboring atom.

In an embodiment, $Ar_1$ and $Ar_2$ may each independently be a group represented by one of Formulae 5-11 to 5-26:

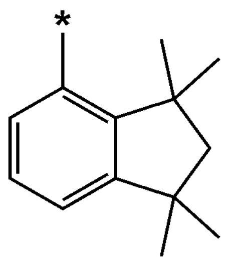

**5-11**

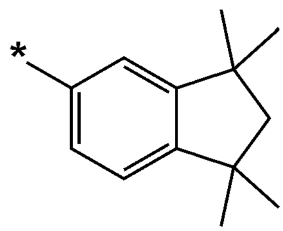

**5-12**

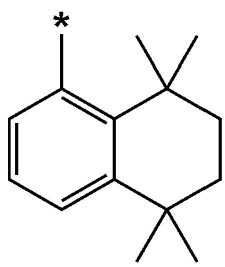

**5-13**

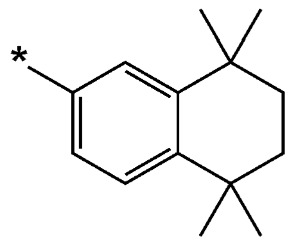

**5-14**

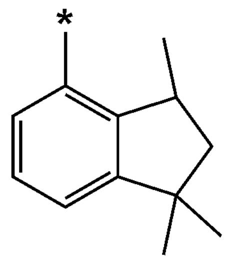

5-15

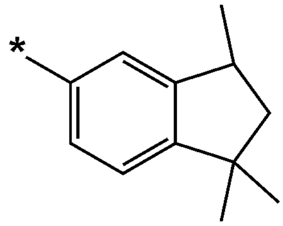

5-16

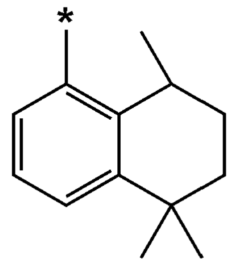

5-17

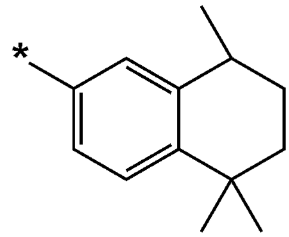

5-18

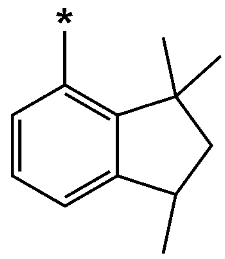

5-19

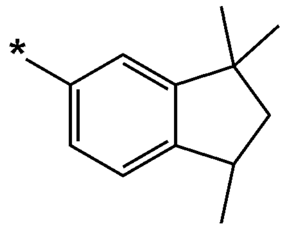

5-20

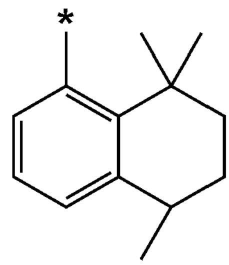

5-21

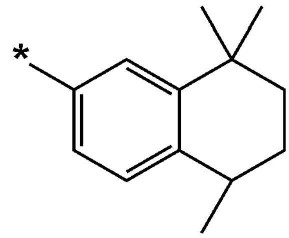

5-22

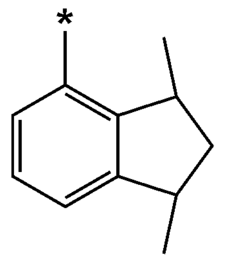

5-23

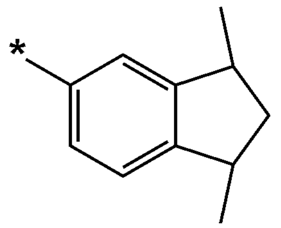

5-24

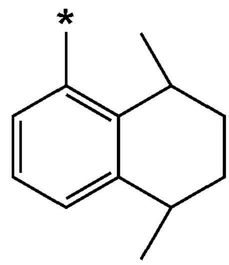

5-25

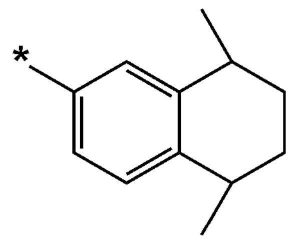

5-26

wherein, in Formulae 5-11 to 5-26,

* may indicate a binding site to a neighboring atom.

In an embodiment, $Ar_1$ and $Ar_2$ may have the same structure.

In an embodiment, $Ar_1$ and $Ar_2$ may have different structures.

In Formula 1, $R_1$, $R_2$, $R_{10}$, $R_{20}$, $R_{30}$, and $R_{40}$ may each independently be a group represented by Formula 2, hydrogen, deuterium, -F, -Cl, -Br, -I, $-SF_5$, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$,$-Si(Q_1)(Q_2)(Q_3)$, $-Ge(Q_1)(Q_2)(Q_3)$, $-C(=O)(Q_1)$, $-S(=O)(Q_1)$, $-S(=O)_2(Q_1)$, $-B(Q_1)(Q_2)$,$-P(Q_1)(Q_2)$, $-P(=O)(Q_1)(Q_2)$, $-P(=S)(Q_1)(Q_2)$, or a group represented by Formula 3.

In Formula 2, $R_{50}$ and $R_{60}$ may each independently be hydrogen, deuterium,-F, -Cl, -Br, -I, $-SF_5$, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a

substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_1)(Q_2)(Q_3)$, $-Ge(Q_1)(Q_2)(Q_3)$,$-C(=O)(Q_1)$, $-S(=O)(Q_1)$, $-S(=O)_2(Q_1)$, $-B(Q_1)(Q_2)$, $-P(Q_1)(Q_2)$, $-P(=O)(Q_1)(Q_2)$,$-P(=S)(Q_1)(Q_2)$, or a group represented by Formula 3:

Formula 3

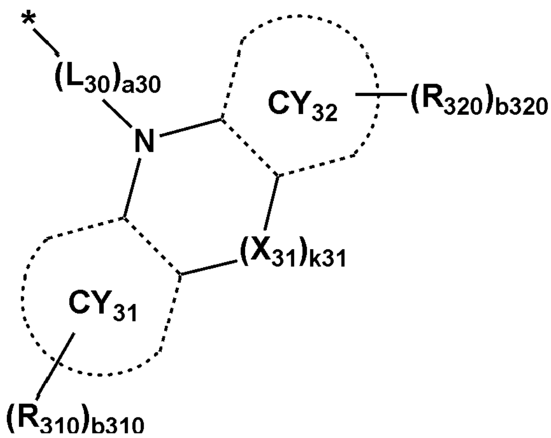

In Formula 3, $X_{31}$ may be a single bond, O, S, $N(R_{301})$, or $C(R_{301})(R_{302})$.

In Formula 3, k31 is 1.

In Formula 3, $CY_{31}$ and $CY_{32}$ may each independently be a $C_5$-$C_{30}$ carbocyclic group or a $C_1$-$C_{30}$ heterocyclic group.

In an embodiment, $CY_{31}$ and $CY_{32}$ may each independently be a benzene group, a naphthalene group, a 1,2,3,4-tetrahydronaphthalene group, a thiophene group, a furan group, a pyrrole group, a cyclopentadiene group, a silole group, a benzothiophene group, a benzofuran group, an indole group, an indene group, a benzosilole group, a dibenzothiophene group, a dibenzofuran group, a carbazole group, a fluorene group, or a dibenzosilole group.

In Formula 3, $L_{30}$ may be a single bond, a substituted or unsubstituted $C_5$-$C_{30}$ carbocyclic group, or a substituted or unsubstituted $C_1$-$C_{30}$ heterocyclic group.

In Formula 3, a30 may be 0, 1, 2, or 3.

In an embodiment, a30 may be 0 or 1.

In Formula 3, $R_{301}$, $R_{302}$, $R_{310}$, and $R_{320}$ may each independently be hydrogen, deuterium, -F, -Cl, -Br, -I, $-SF_5$, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$,$-Si(Q_1)(Q_2)(Q_3)$, $-Ge(Q_1)(Q_2)(Q_3)$, $-C(=O)(Q_1)$, $-S(=O)(Q_1)$, $-S(=O)_2(Q_1)$, $-B(Q_1)(Q_2)$,$-P(Q_1)(Q_2)$, $-P(=O)(Q_1)(Q_2)$, or $-P(=S)(Q_1)(Q_2)$.

In an embodiment, $R_{301}$, $R_{302}$, $R_{310}$, and $R_{320}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, $-SF_5$, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_1$-$C_{20}$ alkyl group, a fluorinated $C_1$-$C_{20}$ alkyl

group, a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group(a norbornanyl group), a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl) cyclooctyl group, a ($C_1$-$C_{20}$ alkyl)adamantanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1] pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl) bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a pyridinyl group, a pyrimidinyl group, or any combination thereof;

a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_1$-$C_{20}$ alkyl group, a fluorinated $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl) cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or $-N(Q_1)(Q_2)$, $-Si(Q_1)(Q_2)(Q_3)$, $-Ge(Q_1)(Q_2)(Q_3)$, $-C(=O)(Q_1)$, $-S(=O)(Q_1)$,$-S(=O)_2(Q_1)$, $-B(Q_1)(Q_2)$, $-P(Q_1)(Q_2)$, $-P(=O)(Q_1)(Q_2)$, or $-P(=S)(Q_1)(Q_2)$.

In an embodiment, $R_{301}$, $R_{302}$, $R_{310}$, and $R_{320}$ may each independently be hydrogen, deuterium, a methyl group, an ethyl group, an n-propyl group, an iso-propyl group, an n-butyl group, an isobutyl group, a sec-butyl group, a tert-butyl group, an n-pentyl group, an isopentyl group, a 2-methylbutyl group, a sec-pentyl group, a tert-pentyl group, a neo-pentyl group, a 3-pentyl group, a 3-methyl-2-butyl group, a phenyl group, a biphenyl group, a $C_1$-$C_{20}$ alkylphenyl group, or a naphthyl group.

In Formula 3, b310 and b320 may each independently be 1, 2, 3, 4, 5, 6, 7, or 8.

In an embodiment, $R_1$, $R_2$, $R_{10}$, $R_{20}$, $R_{30}$, and $R_{40}$ may each independently be: a group represented by Formula 2, a group represented by Formula 3, hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic

acid group or a salt thereof, a phosphoric acid group or a salt thereof, $-SF_5$, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_1$-$C_{20}$ alkyl group, a fluorinated $C_1$-$C_{20}$ alkyl group, a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group(a norbornanyl group), a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl) cyclooctyl group, a ($C_1$-$C_{20}$ alkyl)adamantanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1] pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl) bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a pyridinyl group, a pyrimidinyl group, or any combination thereof;

a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_1$-$C_{20}$ alkyl group, a fluorinated $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl) cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or

$-N(Q_1)(Q_2)$, $-Si(Q_1)(Q_2)(Q_3)$, $-Ge(Q_1)(Q_2)(Q_3)$, $-C(=O)(Q_1)$, $-S(=O)(Q_1)$,$-S(=O)_2(Q_1)$, $-B(Q_1)(Q_2)$, $-P(Q_1)(Q_2)$, $-P(=O)(Q_1)(Q_2)$, $-P(=S)(Q_1)(Q_2)$, or any combination thereof.

In an embodiment, $R_{50}$ and $R_{60}$ may each independently be: a group represented by Formula 3, hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid

group or a salt thereof, $-SF_5$, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_1$-$C_{20}$ alkyl group, a fluorinated $C_1$-$C_{20}$ alkyl group, a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group(a norbornanyl group), a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a pyridinyl group, a pyrimidinyl group, or any combination thereof;

a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_1$-$C_{20}$ alkyl group, a fluorinated $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a silolanyl group, a phenyl group, a ($C_1$-$C_{20}$ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or

$-N(Q_1)(Q_2)$, $-Si(Q_1)(Q_2)(Q_3)$, $-Ge(Q_1)(Q_2)(Q_3)$, $-C(=O)(Q_1)$, $-S(=O)(Q_1)$,$-S(=O)_2(Q_1)$, $-B(Q_1)(Q_2)$, $-P(Q_1)(Q_2)$, $-P(=O)(Q_1)(Q_2)$, or $-P(=S)(Q_1)(Q_2)$.

In an embodiment, $R_1$, $R_2$, $R_{10}$, $R_{20}$, $R_{30}$, and $R_{40}$ may each independently be:

a group represented by Formula 2, a group represented by Formula 3, hydrogen, deuterium, -F, -Cl, -Br, -I, $-CD_3$,

$-CD_2H$, $-CDH_2$, $-CF_3$, $-CF_2H$, $-CFH_2$, a $C_1$-$C_{60}$ alkyl group, a $C_2$-$C_{60}$ alkenyl group, a $C_2$-$C_{60}$ alkynyl group, or a $C_1$-$C_{60}$ alkoxy group; or

a group represented by one of Formulae 9-1 to 9-61, 9-201 to 9-244, 10-1 to 10-154, and 10-201 to 10-350:

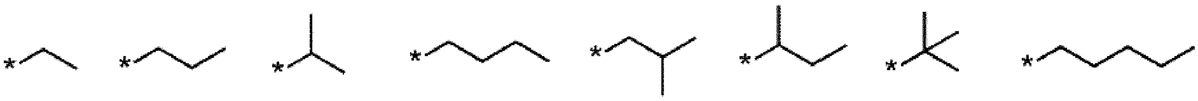

9-1 9-2 9-3 9-4 9-5 9-6 9-7 9-8

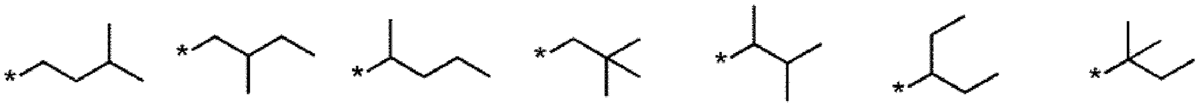

9-9 9-10 9-11 9-12 9-13 9-14 9-15

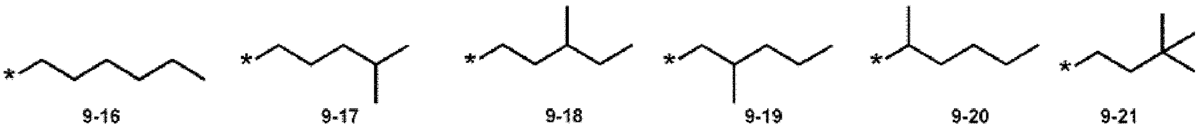

9-16 9-17 9-18 9-19 9-20 9-21

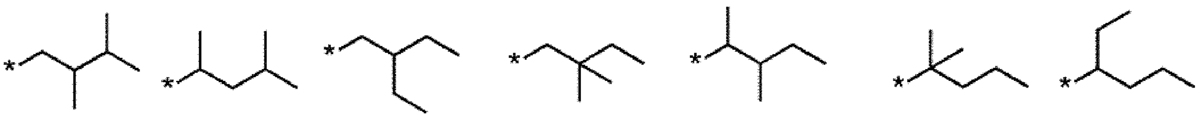

9-22 9-23 9-24 9-25 9-26 9-27 9-28

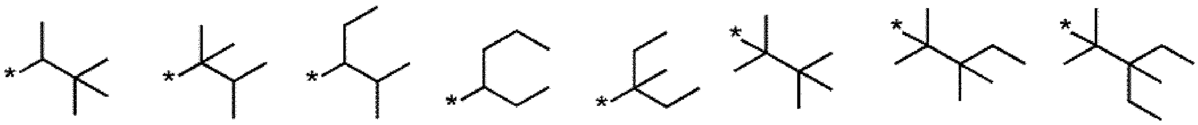

9-29 9-30 9-31 9-32 9-33 9-34 9-35 9-36

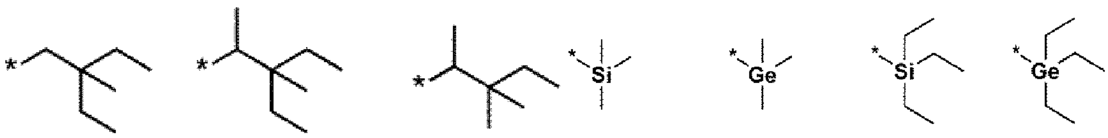

9-37 9-38 9-39 9-40 9-41 9-42 9-43

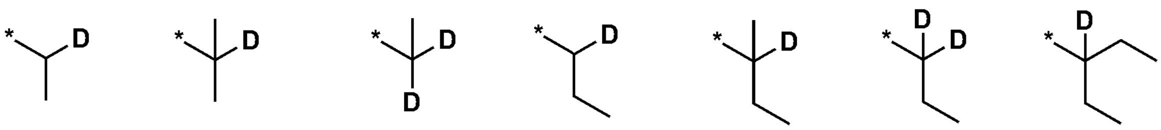

9-44 9-45 9-46 9-47 9-48 9-49 9-50

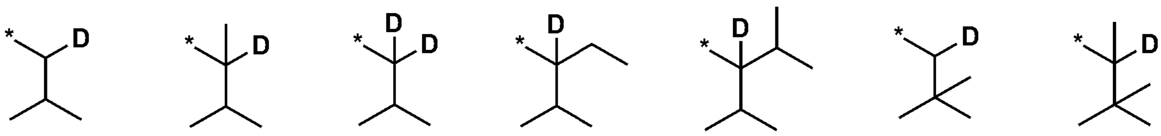

9-51 9-52 9-53 9-54 9-55 9-56 9-57

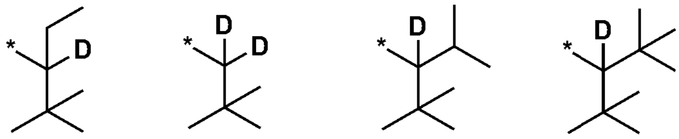
9-58 9-59 9-60 9-61

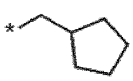
9-201

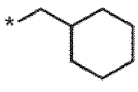
9-202

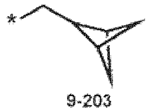
9-203

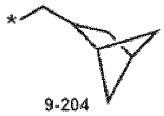
9-204

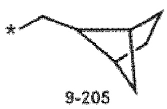
9-205

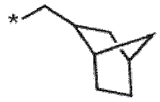
9-206

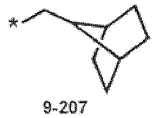
9-207

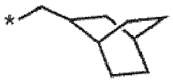
9-208

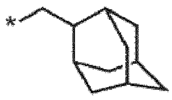
9-209

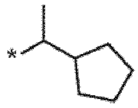
9-210

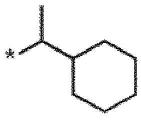
9-211

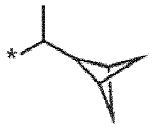
9-212

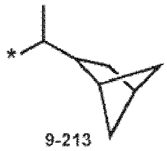
9-213

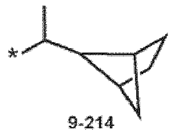
9-214

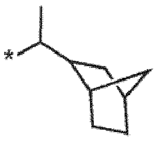
9-215

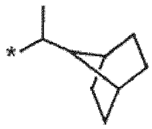
9-216

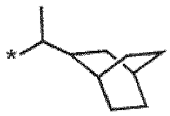
9-217

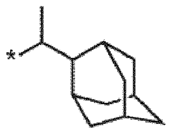
9-218

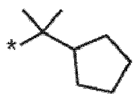
9-219

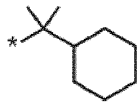
9-220

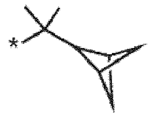
9-221

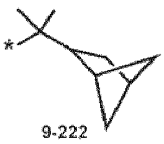
9-222

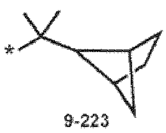
9-223

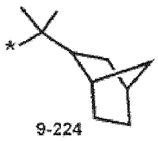
9-224

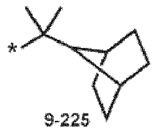
9-225

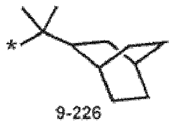
9-226

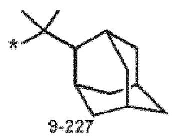
9-227

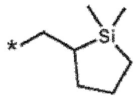
9-228

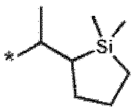
9-229

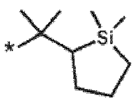
9-230

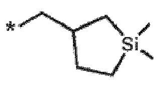
9-231

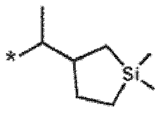
9-232

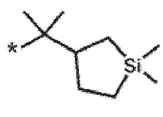
9-233

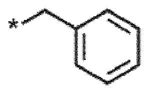
9-234

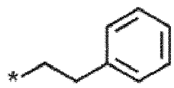
9-235

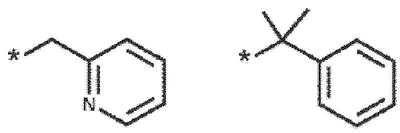
9-236 9-237

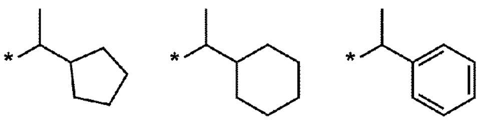
9-238 9-239 9-240

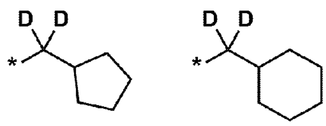
9-241 9-242

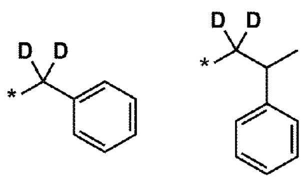
9-243 9-244

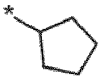
10-1
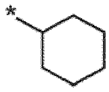
10-2
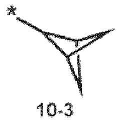
10-3
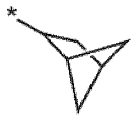
10-4
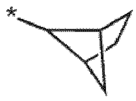
10-5
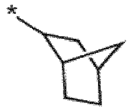
10-6
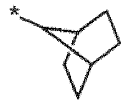
10-7
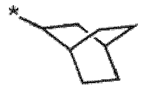
10-8

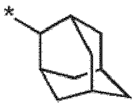
10-9
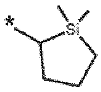
10-10
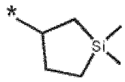
10-11
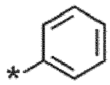
10-12
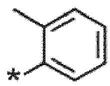
10-13
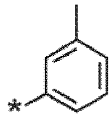
10-14
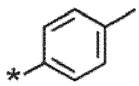
10-15
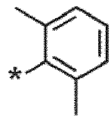
10-16

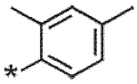
10-17
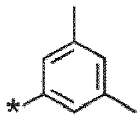
10-18
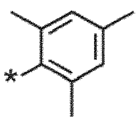
10-19
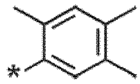
10-20
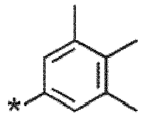
10-21
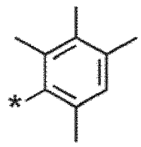
10-22
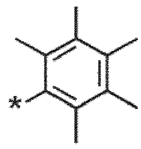
10-23

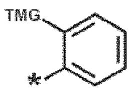
10-24
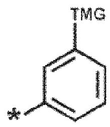
10-25
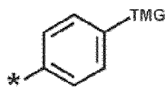
10-26
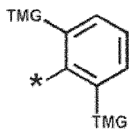
10-27
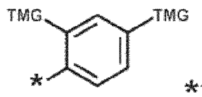
10-28
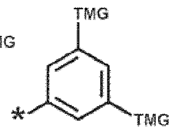
10-29
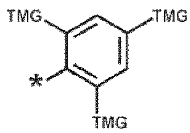
10-30

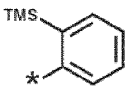
10-31
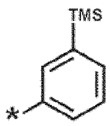
10-32
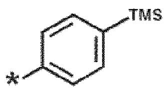
10-33
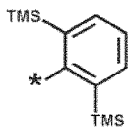
10-34
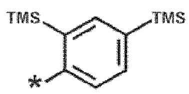
10-35
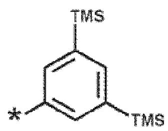
10-36
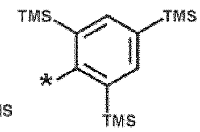
10-37

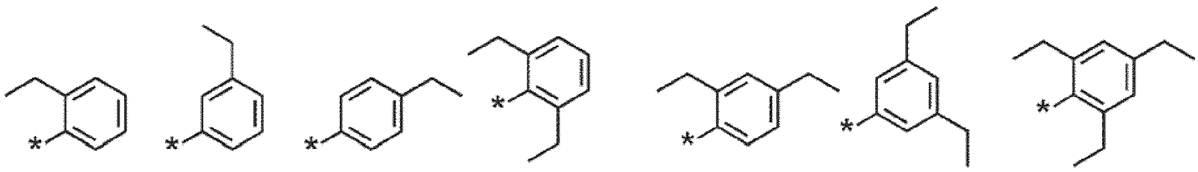
10-38 10-39 10-40 10-41 10-42 10-43 10-44

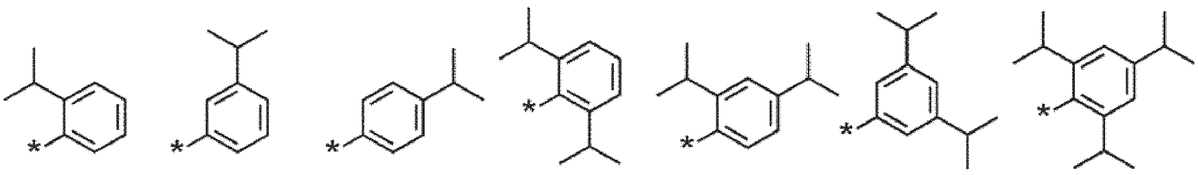
10-45 10-46 10-47 10-48 10-49 10-50 10-51

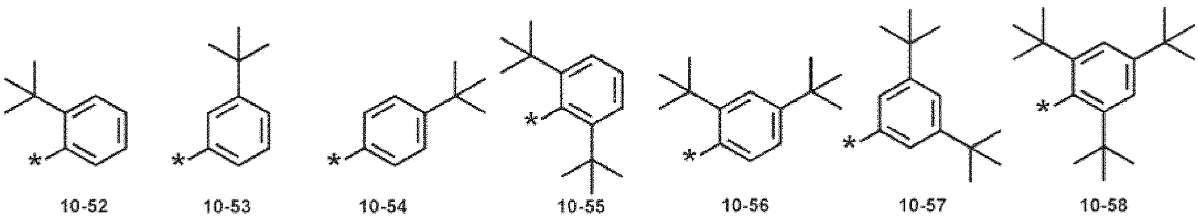
10-52 10-53 10-54 10-55 10-56 10-57 10-58

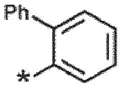
10-59

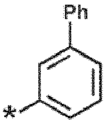
10-60

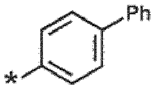
10-61

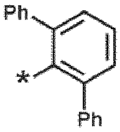
10-62

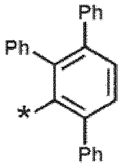
10-63

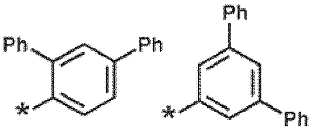
10-64
10-65

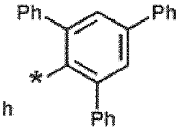
10-66

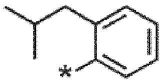
10-67

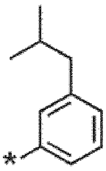
10-68

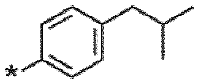
10-69

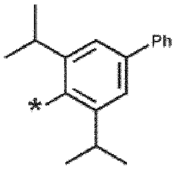
10-70

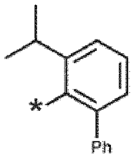
10-71

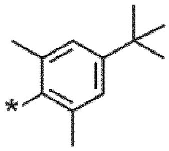
10-72

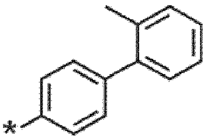
10-73

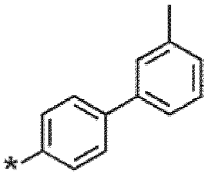
10-74

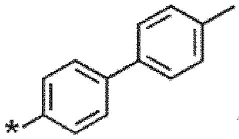
10-75

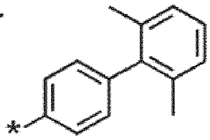
10-76

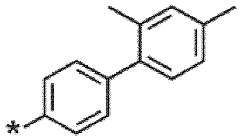
10-77

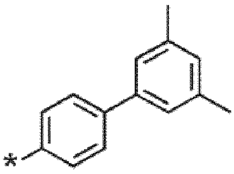
10-78

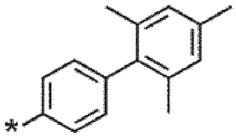
10-79

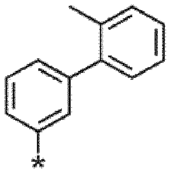
10-80

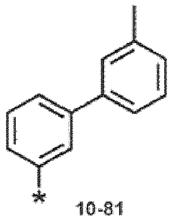
10-81

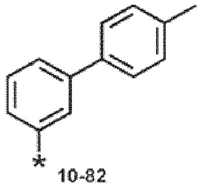
10-82

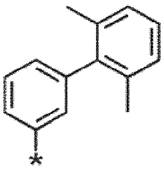
10-83

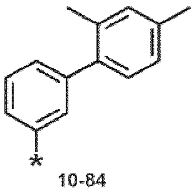
10-84

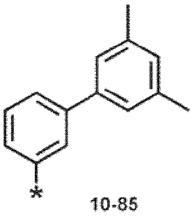
10-85

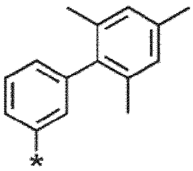
10-86

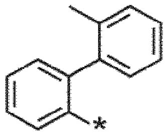
10-87

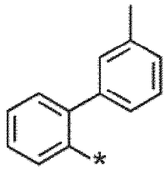
10-88

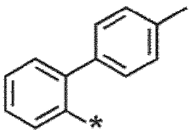
10-89

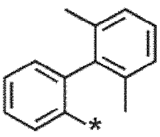
10-90

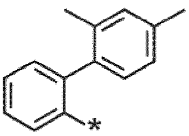
10-91

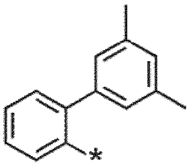
10-92

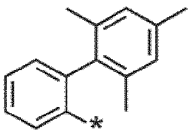
10-93

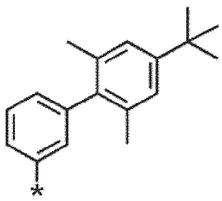
10-94

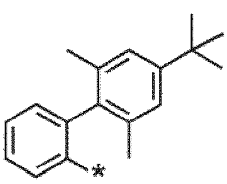
10-95

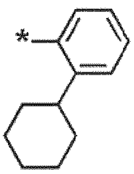
10-96

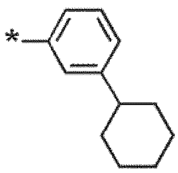
10-97

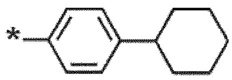
10-98

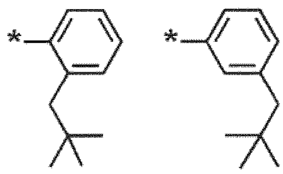
10-99
10-100

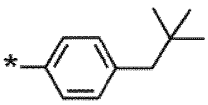
10-101

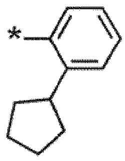
10-102

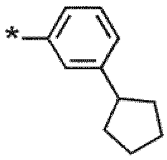
10-103

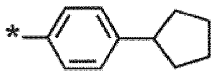
10-104

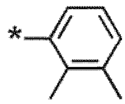
10-105

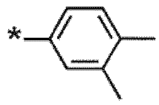
10-106

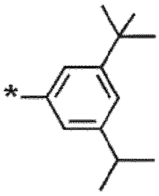
10-107

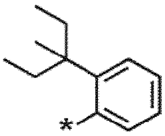
10-108

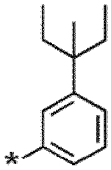
10-109

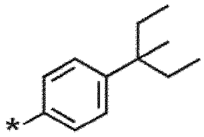
10-110

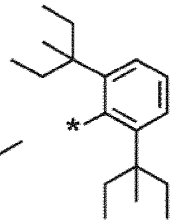
10-111

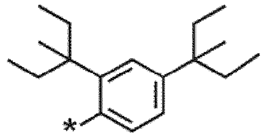
10-112

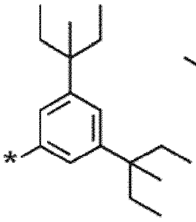
10-113

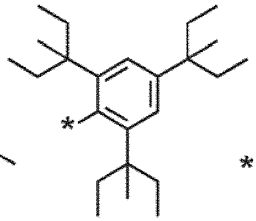
10-114

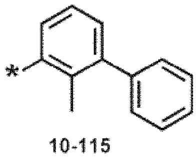
10-115

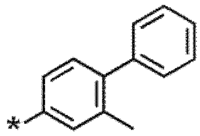
10-116

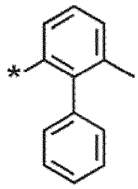
10-117

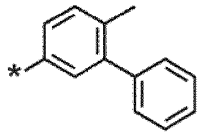
10-118

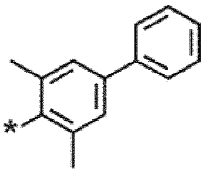
10-119

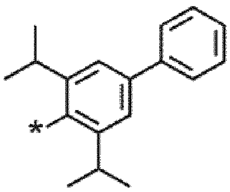
10-120

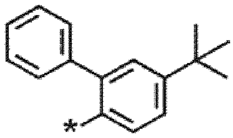
10-121

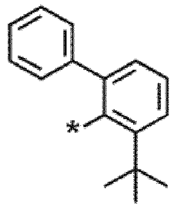
10-122

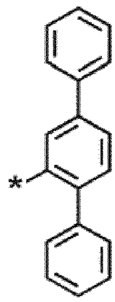
10-123

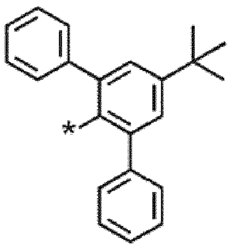
10-124

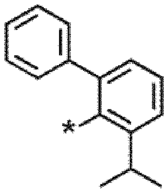
10-125

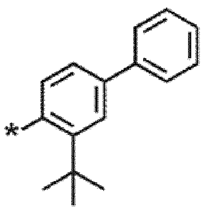
10-126

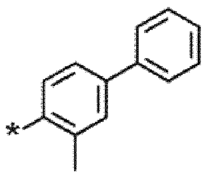
10-127

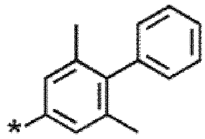
10-128

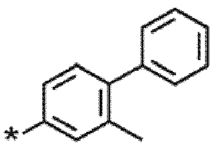
10-129

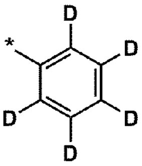
10-130

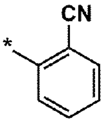
10-131

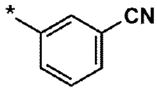
10-132

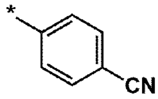
10-133

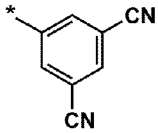
10-134

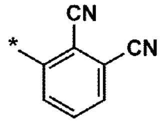
10-135

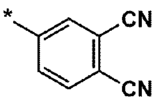
10-136

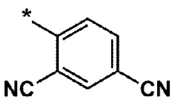
10-137

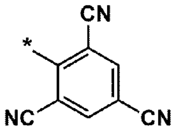
10-138

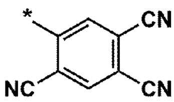
10-139

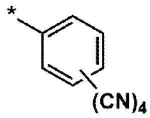
10-140

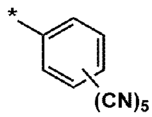
10-141

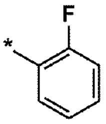
10-142

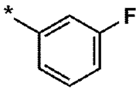
10-143

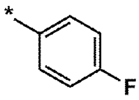
10-144

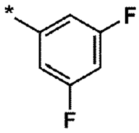
10-145

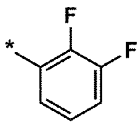
10-146

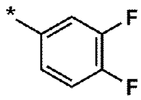
10-147

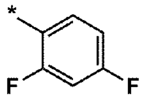
10-148

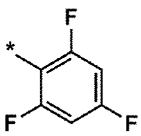
10-149

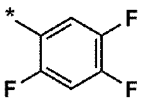
10-150

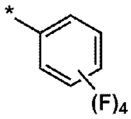
10-151

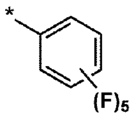
10-152

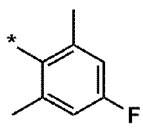
10-153

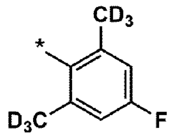
10-154

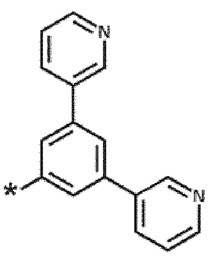
10-201

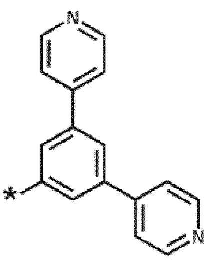
10-202

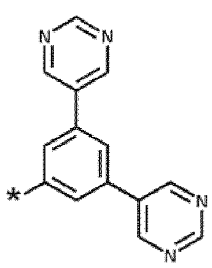
10-203

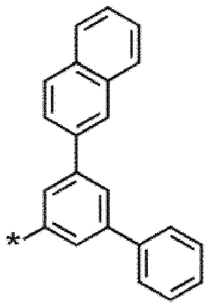
10-204

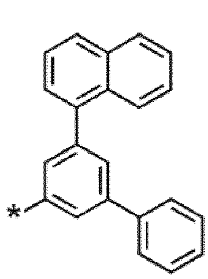
10-205

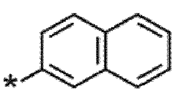
10-206

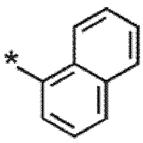
10-207

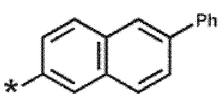
10-208

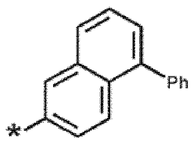
10-209

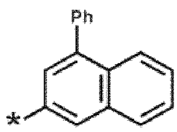
10-210

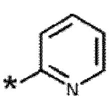
10-211

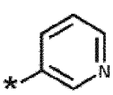
10-212

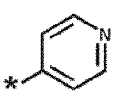
10-213

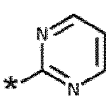
10-214

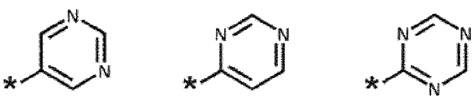
10-215

10-216

10-217

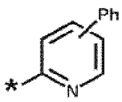
10-218
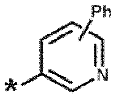
10-219
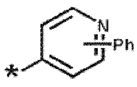
10-220
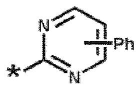
10-221
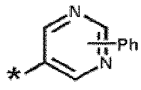
10-222
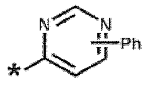
10-223
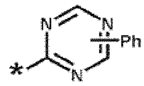
10-224

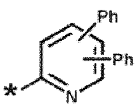
10-225
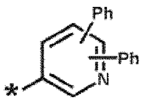
10-226
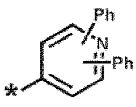
10-227
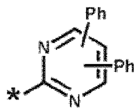
10-228
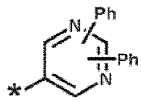
10-229
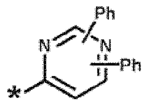
10-230
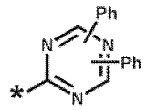
10-231

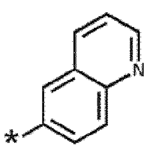
10-232
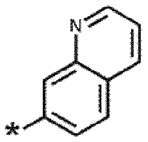
10-233
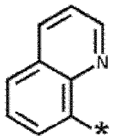
10-234
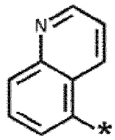
10-235
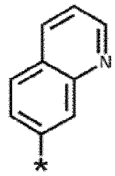
10-236
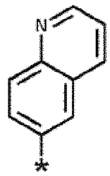
10-237

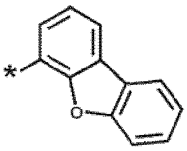
10-238
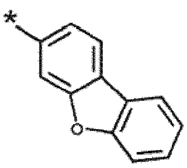
10-239
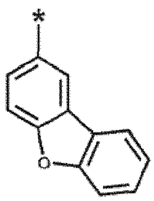
10-240
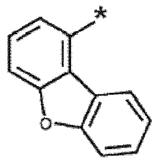
10-241
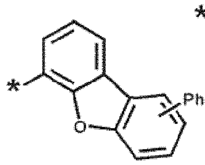
10-242
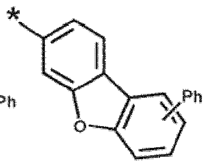
10-243

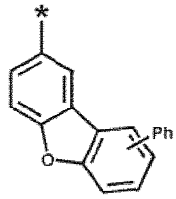
10-244
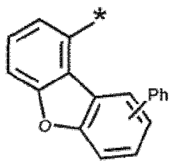
10-245
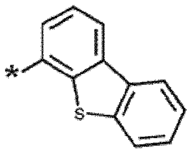
10-246
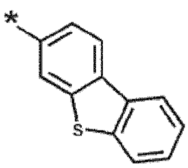
10-247
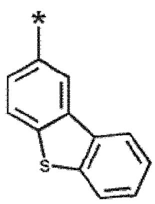
10-248
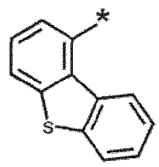
10-249

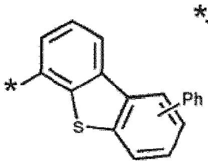
10-250
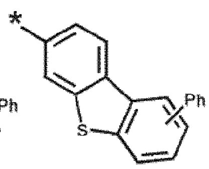
10-251
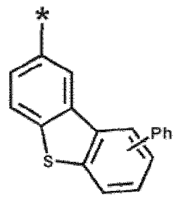
10-252
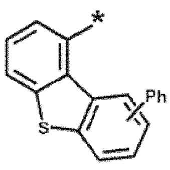
10-253
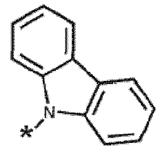
10-254
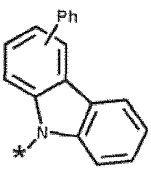
10-255

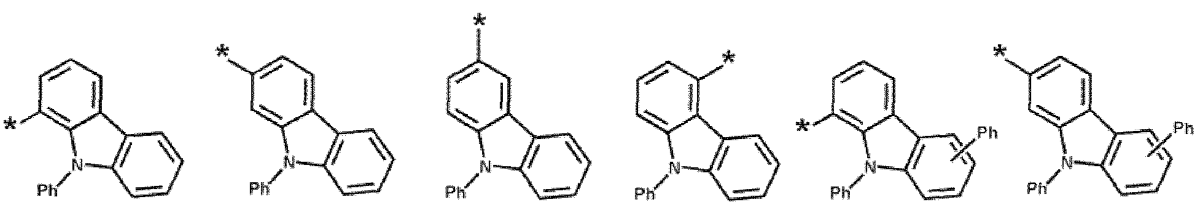
10-256
10-257
10-258
10-259
10-260
10-261

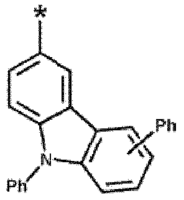
10-262
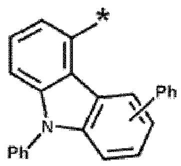
10-263
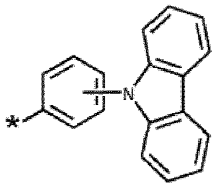
10-264
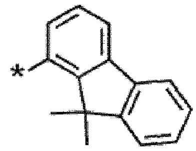
10-265
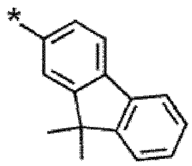
10-266
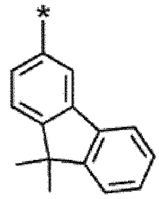
10-267

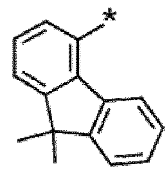
10-268
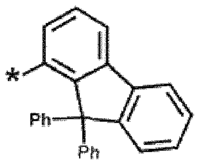
10-269
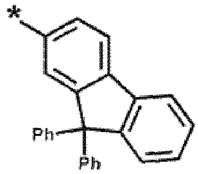
10-270
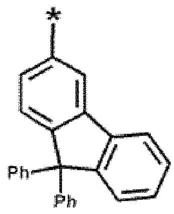
10-271
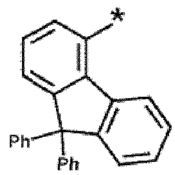
10-272

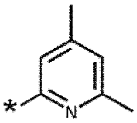
10-273
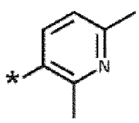
10-274
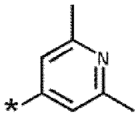
10-275
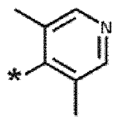
10-276
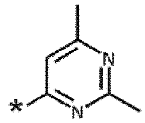
10-277
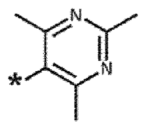
10-278
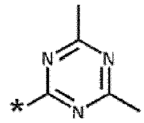
10-279

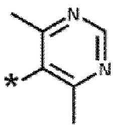
10-280
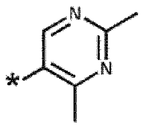
10-281
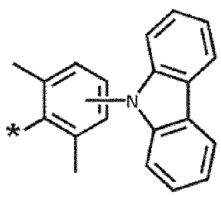
10-282
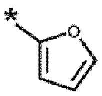
10-283
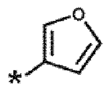
10-284
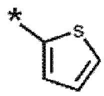
10-285
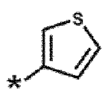
10-286

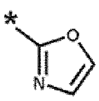
10-287
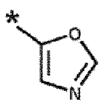
10-288
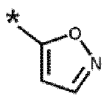
10-289
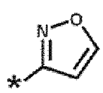
10-290
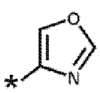
10-291
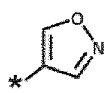
10-292
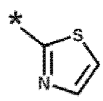
10-293
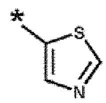
10-294
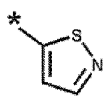
10-295

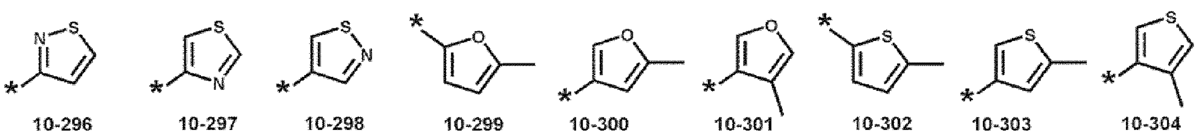
10-296 10-297 10-298 10-299 10-300 10-301 10-302 10-303 10-304

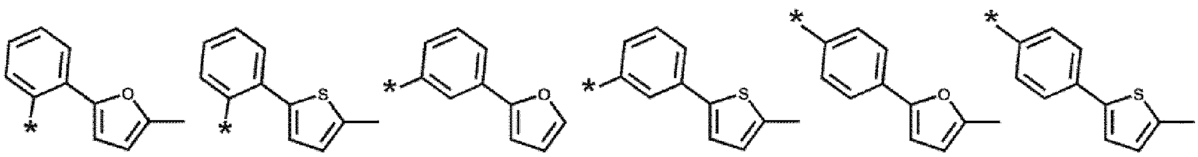
10-305 10-306 10-307 10-308 10-309 10-310

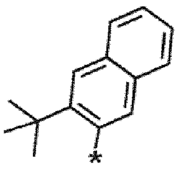
10-311
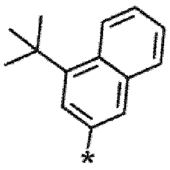
10-312
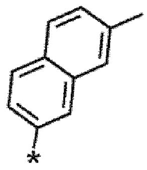
10-313
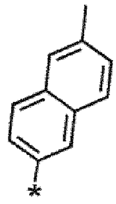
10-314
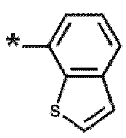
10-315
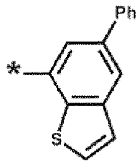
10-316
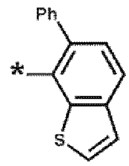
10-317

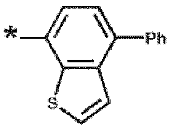
10-318
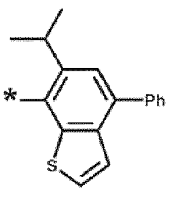
10-319
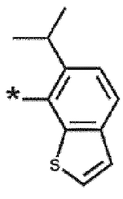
10-320
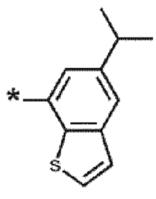
10-321
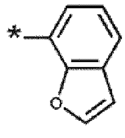
10-322
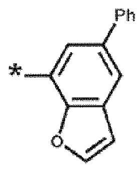
10-323
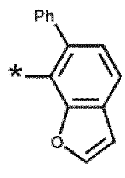
10-324

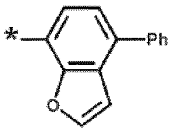
10-325
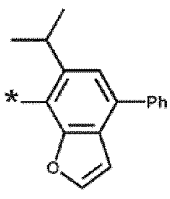
10-326
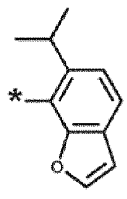
10-327
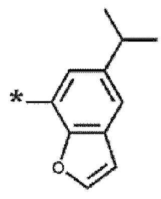
10-328
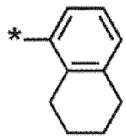
10-329
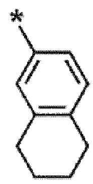
10-330
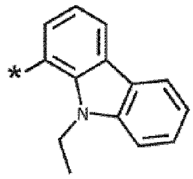
10-331

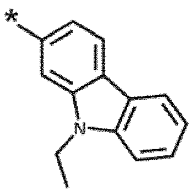
10-332
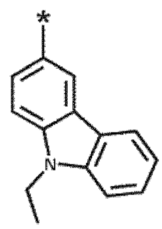
10-333
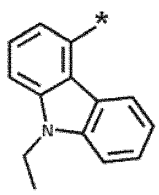
10-334
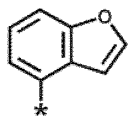
10-335
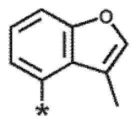
10-336
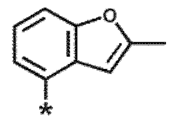
10-337

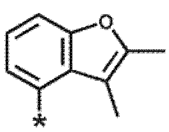
10-338
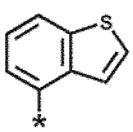
10-339
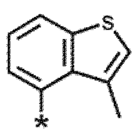
10-340
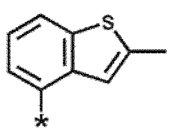
10-341
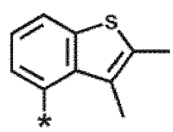
10-342
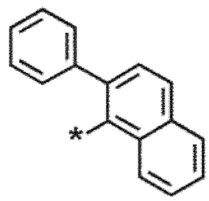
10-343

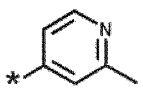
10-344
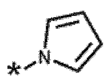
10-345
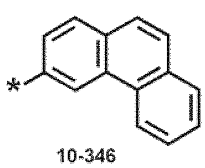
10-346
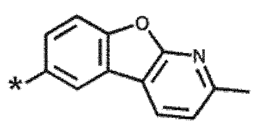
10-347
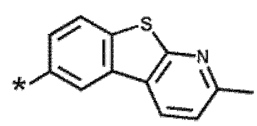
10-348

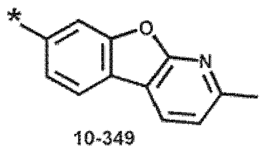
10-349
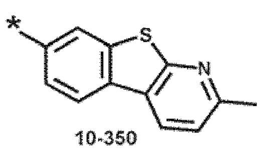
10-350

wherein, in Formulae 9-1 to 9-61, 9-201 to 9-244, 10-1 to 10-154, and 10-201 to 10-350* indicates a binding site to a

neighboring atom, Ph is a phenyl group, TMS is a trimethylsilyl group, and TMG is a trimethylgermyl group.

In an embodiment, $R_{50}$ and $R_{60}$ may each independently be:

a group represented by Formula 3, hydrogen, deuterium, -F, -Cl, -Br,-I, $-CD_3$, $-CD_2H$, $-CDH_2$, $-CF_3$, $-CF_2H$, $-CFH_2$, a $C_1$-$C_{60}$ alkyl group, a $C_2$-$C_{60}$ alkenyl group, a $C_2$-$C_{60}$ alkynyl group, or a $C_1$-$C_{60}$ alkoxy group; or
a group represented by one of Formulae 9-1 to 9-61, 9-201 to 9-244, 10-1 to 10-154, and 10-201 to 10-350.

In an embodiment, at least one instance of $R_{10}$ may be a group represented by Formula 3.
In an embodiment, at least one instance of $R_{20}$ may be a group represented by Formula 3.
In an embodiment, at least one instance of $R_{30}$ may be a group represented by Formula 3.
In an embodiment, at least one instance of $R_{10}$, at least one instance of $R_{20}$, and at least one instance of $R_{30}$ may be a group represented by Formula 3. In an embodiment, one of $R_{30}$ may be a group represented by Formula 3.
In Formulae 1 and 2, b10, b20, b30, b40, and b60 may each independently be 1, 2, 3, 4, 5, 6, 7, or 8.
In Formula 2, b50 may be 1, 2, or 3.
In an embodiment, a group represented by Formula 3 may be a group represented by one of Formulae 3-2 or 3-3:

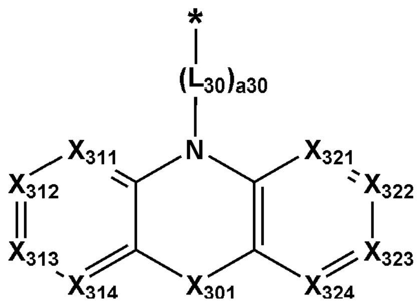

**3-3**

wherein, in Formulae 3-2 or 3-3,

$L_{30}$ and a30 may each be the same as described in the present specification,
$X_{301}$ may be the same as described in connection with $X_{31}$,
$X_{311}$ may be $C(R_{311})$ or N, $X_{312}$ may be $C(R_{312})$ or N, $X_{313}$ may be $C(R_{313})$ or N, $X_{314}$ may be $C(R_{314})$ or N,
$X_{321}$ may be $C(R_{321})$ or N, $X_{322}$ may be $C(R_{322})$ or N, $X_{323}$ may be $C(R_{323})$ or N, $X_{324}$ may be $C(R_{324})$ or N,
$R_{311}$ to $R_{314}$ may each independently be the same as described in connection with $R_{310}$,
$R_{321}$ to $R_{324}$ may each independently be the same as described in connection with $R_{320}$, and
* may indicate a binding site to a neighboring atom.

In an embodiment, the heterocyclic compound may be represented by Formula 1A or 1B:

Formula 1A

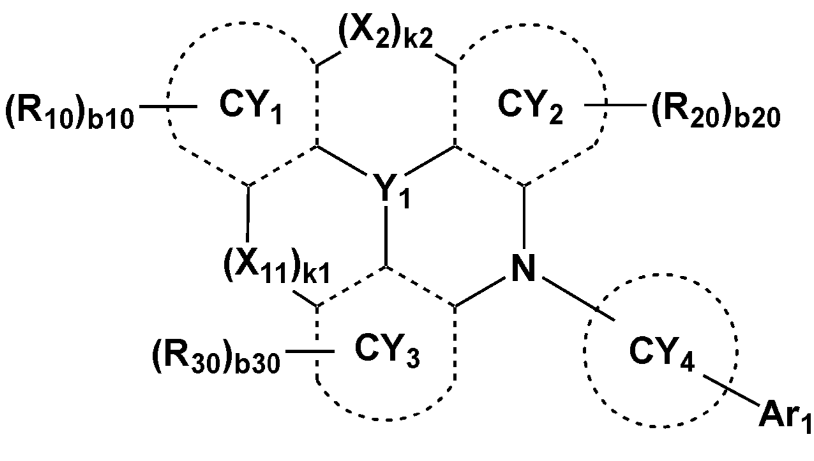

Formula 1B

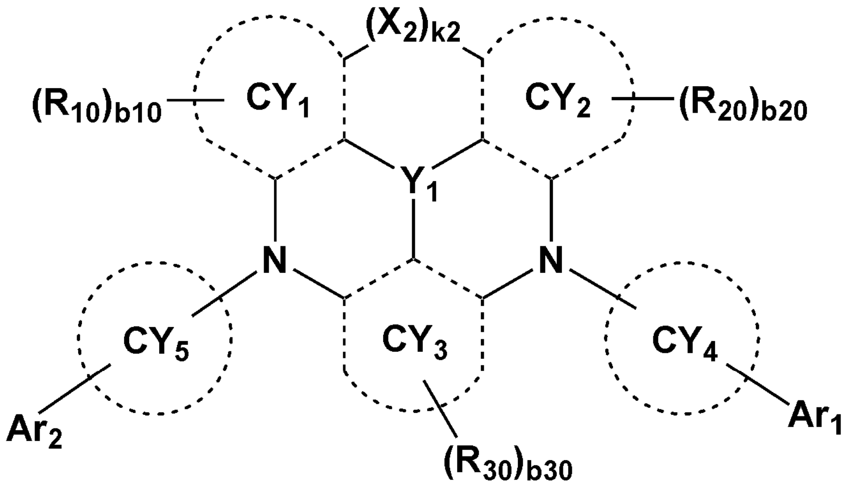

wherein, in Formulae 1A and 1B,

$Y_1$, $X_2$, k1, k2, $CY_1$ to $CY_5$, $CY_{11}$, $R_1$, $R_2$, $R_{10}$, $R_{20}$, $R_{30}$, b10, b20, and b30 may each be the same as described in the present specification, and

$X_{11}$ may be a single bond, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$, or C(=O).

In an embodiment, the heterocyclic compound may be represented by one of Formulae 11-1 to 11-9:

Formula 11-1

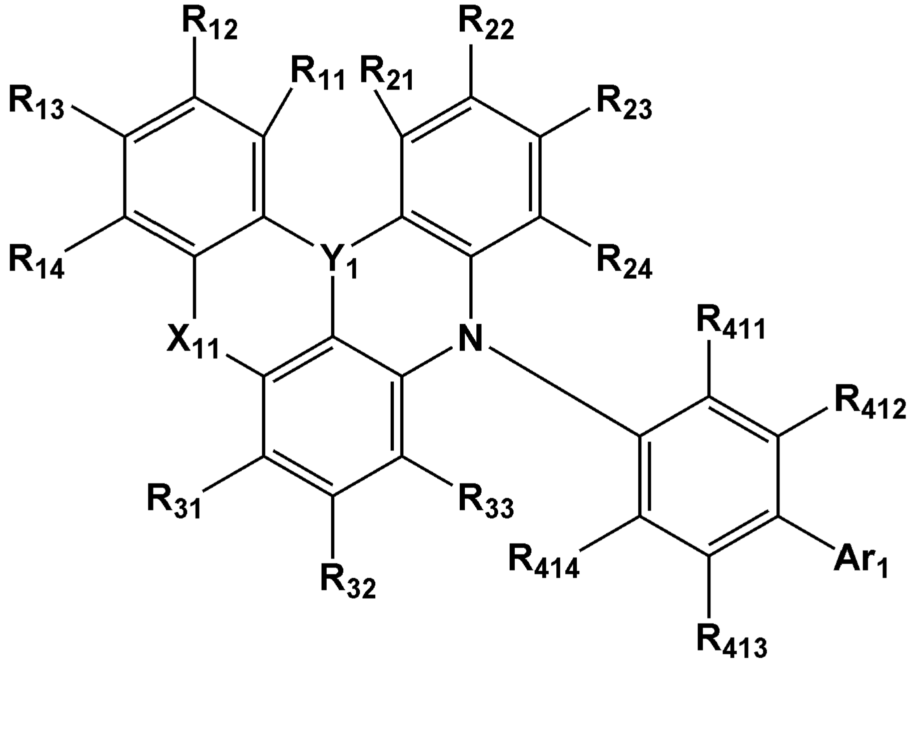

Formula 11-2

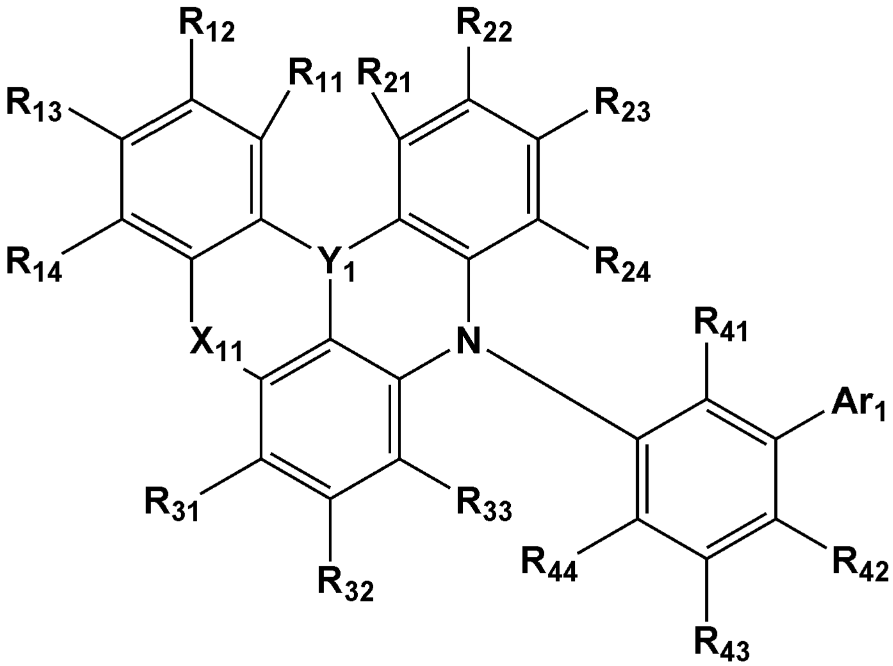

Formula 11-3

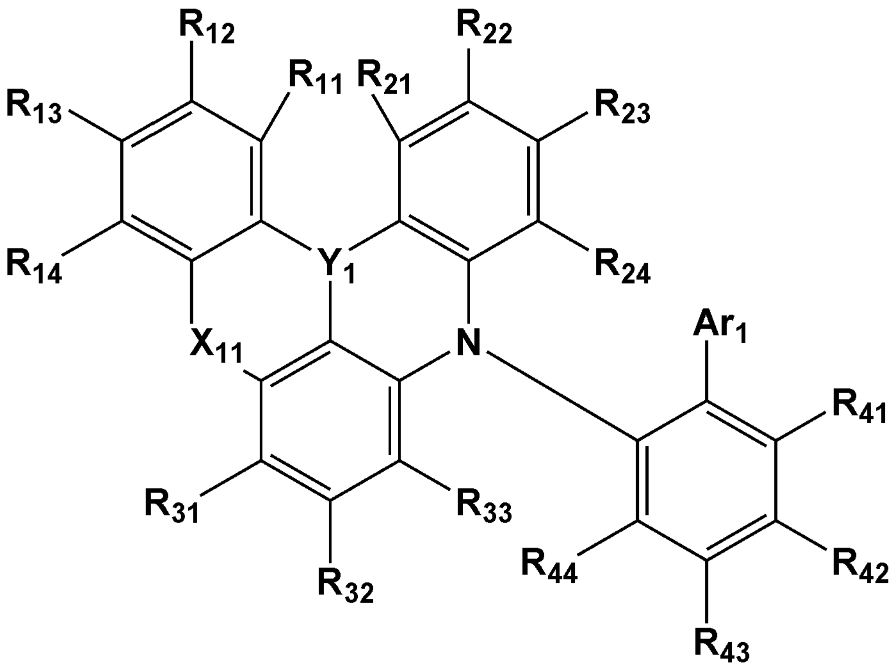

Formula 11-4

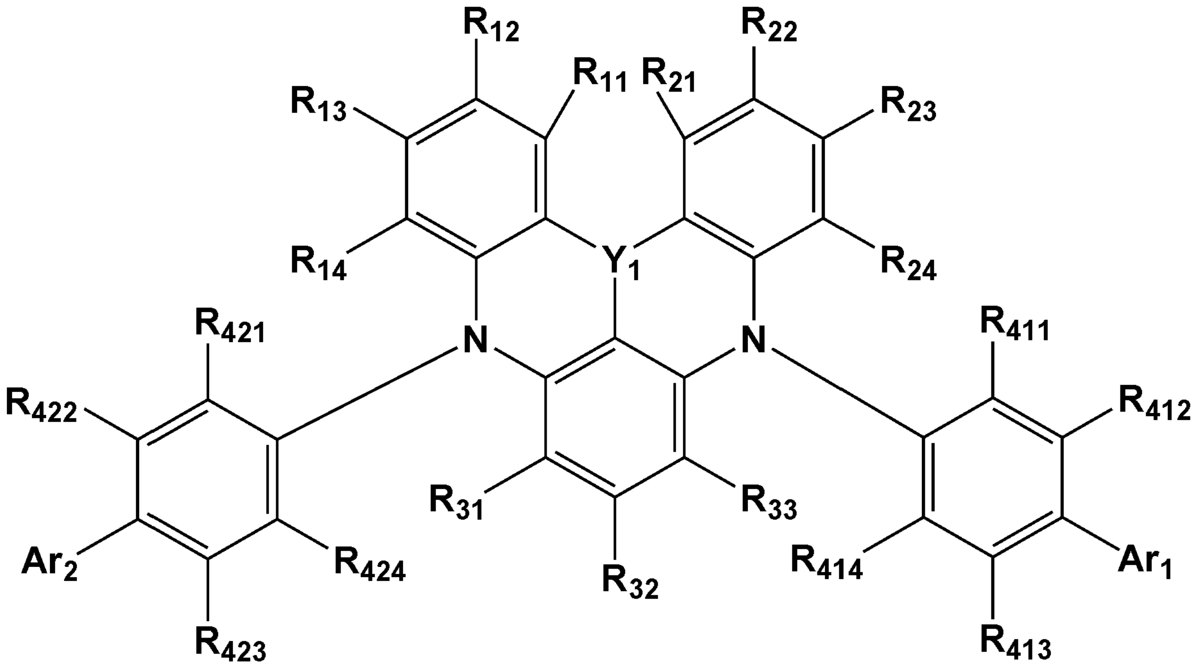

Formula 11-5

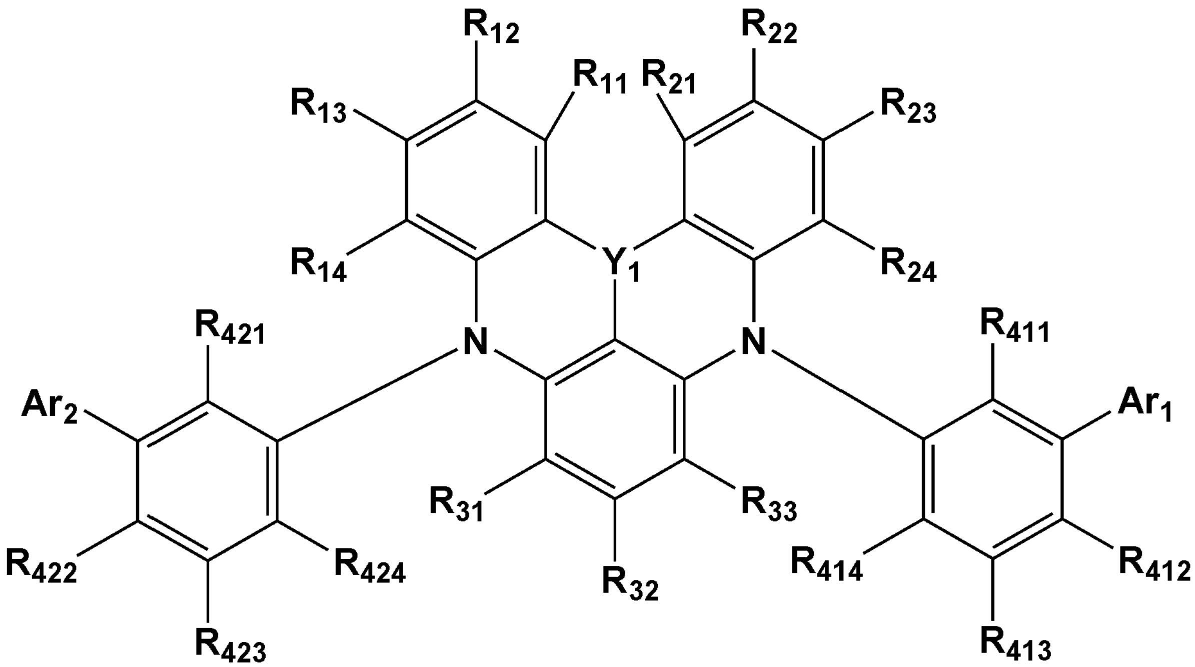

Formula 11-6

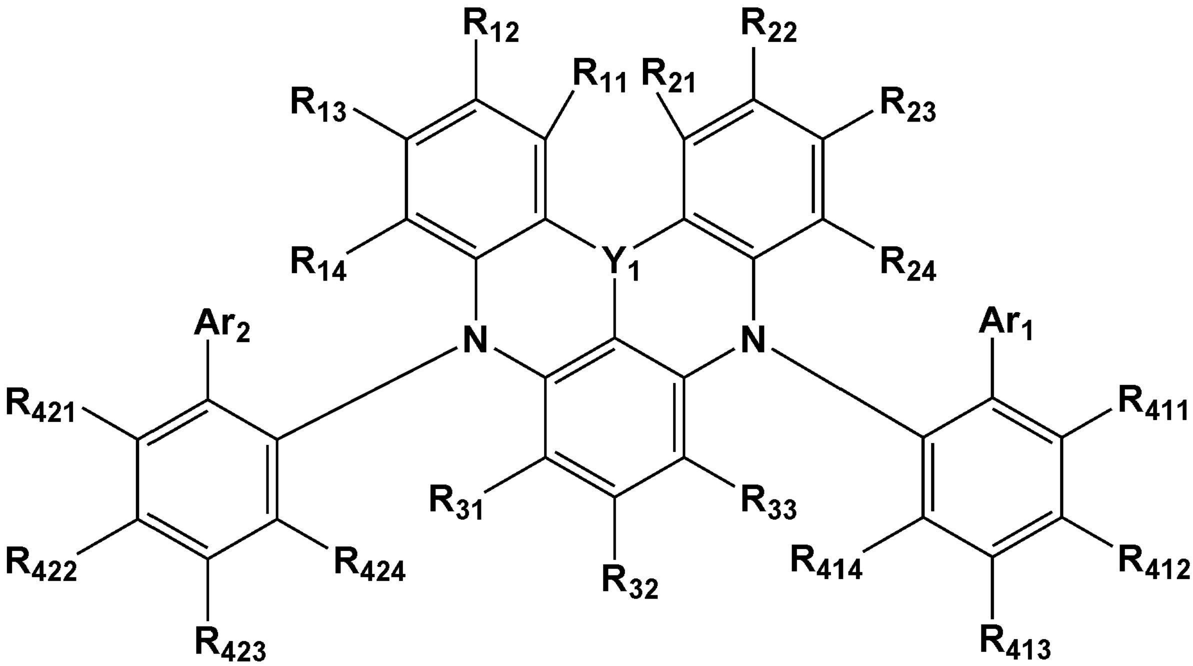

Formula 11-7

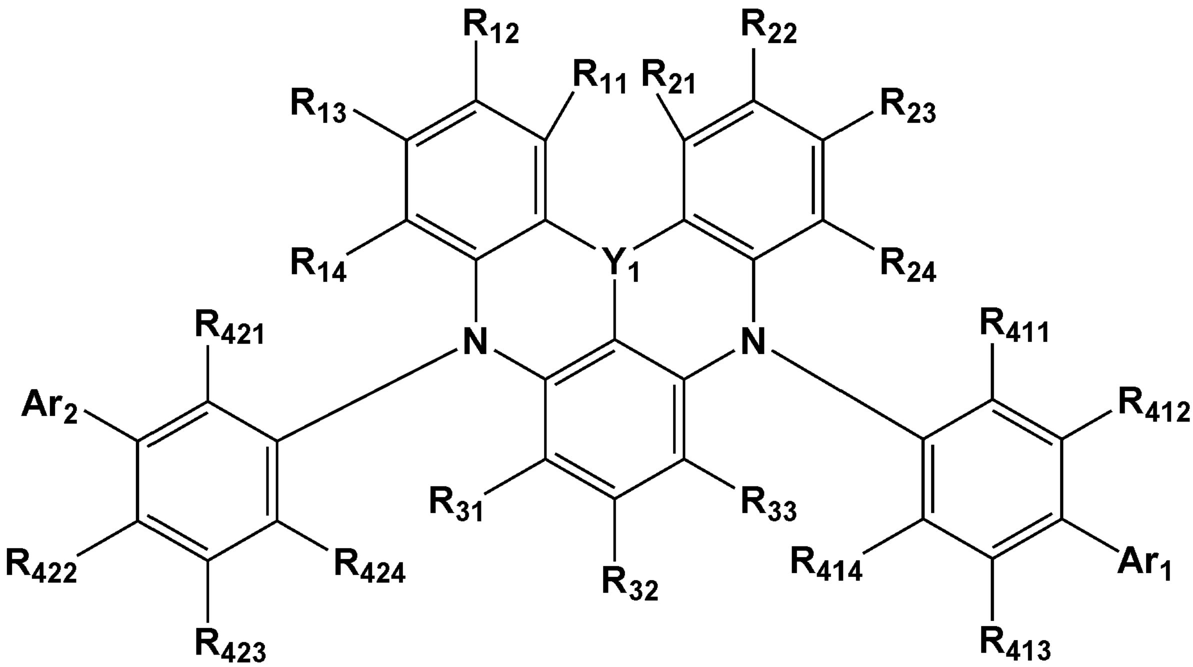

Formula 11-8

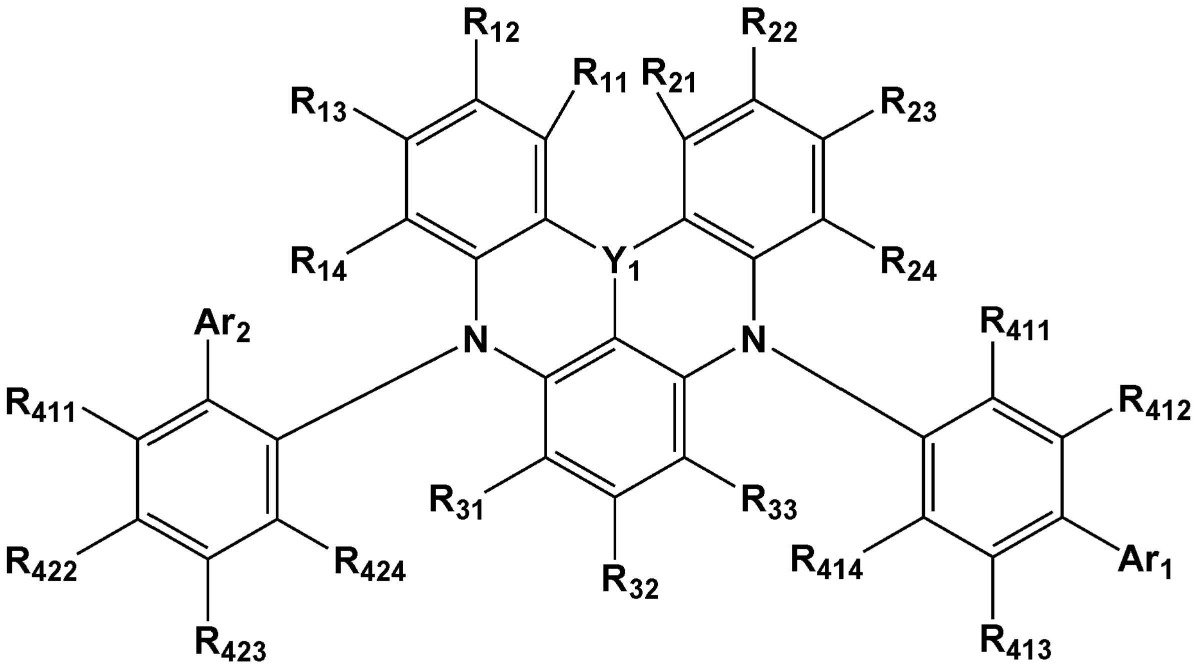

Formula 11-9

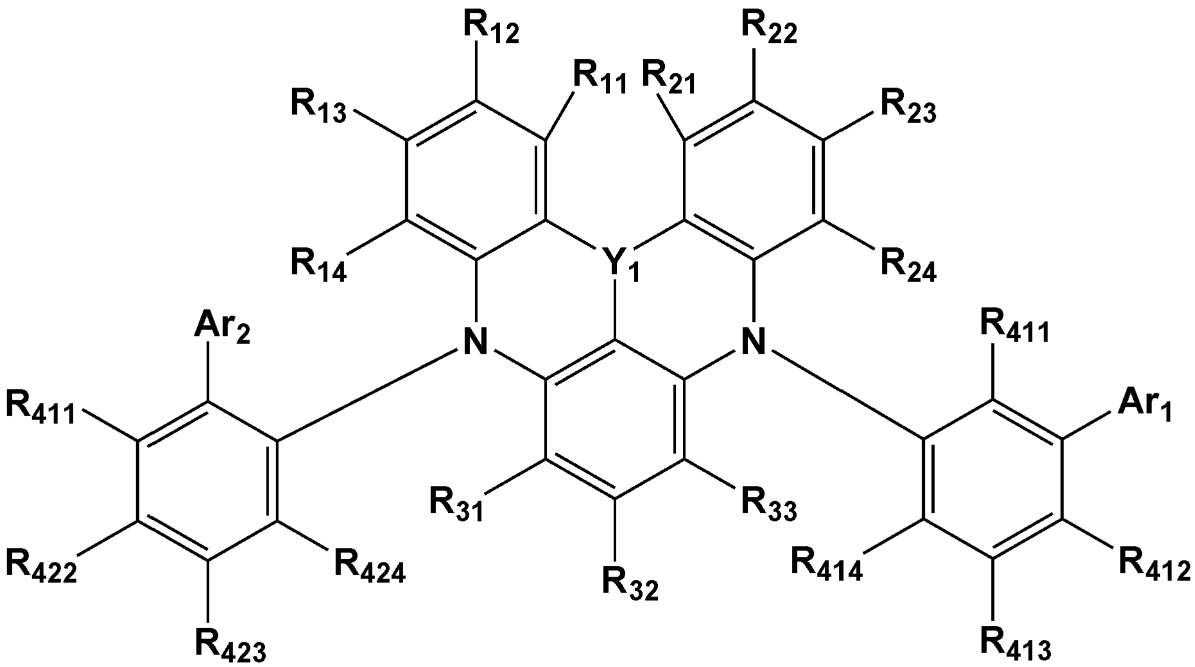

wherein, in Formulae 11-1 to 11-9,

$Y_1$ and $X_1$ may each be the same as described in the present specification,
$X_{11}$ may be a single bond, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$, or C(=O),
$R_{11}$ to $R_{14}$ may each independently be the same as described in connection with $R_{10}$,
$R_{21}$ to $R_{24}$ may each independently be the same as described in connection with $R_{20}$,
$R_{31}$ to $R_{33}$ may each independently be the same as described in connection with $R_{30}$, and
$R_{411}$ to $R_{414}$ and $R_{421}$ to $R_{424}$ may each independently be the same as described in connection with $R_{40}$.

In an embodiment, at least one of $R_{11}$ to $R_{14}$ may be a group represented by Formula 3.
In an embodiment, at least one of $R_{21}$ to $R_{24}$ may be a group represented by Formula 3.
In an embodiment, at least one of $R_{31}$ to $R_{33}$ may be a group represented by Formula 3.
In an embodiment, at least one of $R_{11}$ to $R_{14}$, at least one of $R_{21}$ to $R_{24}$, and at least one of $R_{31}$ to $R_{33}$ may be a group represented by Formula 3.
In an embodiment, the heterocyclic compound may have a symmetric structure or an asymmetric structure.
For example, the heterocyclic compound may have a symmetric structure.
As another example, the heterocyclic compound may have an asymmetric structure.
As used herein, "a symmetric structure" means that when is drawn down the middle of the structure (i.e., through the B and center of the $CY_3$ ring Formula I), each side is the same. For example, Compound 1 has a symmetric structure, whereas Compound 25 has an asymmetric structure.
A substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and the substituted monovalent non-aromatic condensed heteropolycyclic group may be:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$,$-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{11})(Q_{12})$, $-Si(Q_{11})(Q_{12})(Q_{13})$, $-Ge(Q_{11})(Q_{12})(Q_{13})$,$-C(=O)(Q_{11})$, $-S(=O)(Q_{11})$, $-S(=O)_2(Q_{11})$, $-B(Q_{11})(Q_{12})$, $-P(Q_{11})(Q_{12})$,$-P(=O)(Q_{11})(Q_{12})$, $-P(=S)(Q_{11})(Q_{12})$, or any combination thereof;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{21})(Q_{22})$, $-Si(Q_{21})(Q_{22})(Q_{23})$,$-Ge(Q_{21})(Q_{22})(Q_{23})$, $-C(=O)(Q_{21})$, $-S(=O)(Q_{21})$, $-S(=O)_2(Q_{21})$, $-B(Q_{21})(Q_{22})$,$-P(Q_{21})(Q_{22})$, $-P(=O)(Q_{21})(Q_{22})$, $-P(=S)(Q_{21})(Q_{22})$, or any combination thereof; $-N(Q_{31})(Q_{32})$, $-Si(Q_{31})(Q_{32})(Q_{33})$, $-Ge(Q_{31})(Q_{32})(Q_{33})$, $-C(=O)(Q_{31})$, $-S(=O)(Q_{31})$, $-S(=O)_2(Q_{31})$, $-B(Q_{31})(Q_{32})$, $-P(Q_{31})(Q_{32})$, $-P(=O)(Q_{31})(Q_{32})$, or $-P(Q_{31})(Q_{32})$.

$Q_1$ to $Q_3$, $Q_{11}$ to $Q_{13}$, $Q_{21}$ to $Q_{23}$, and $Q_{31}$ to $Q_{33}$ may each independently be: hydrogen; deuterium; -F; -Cl; -Br; -I; a hydroxyl group; a cyano group; a nitro group; an amidino group; a hydrazine group; a hydrazone group; a carboxylic acid or a salt thereof; a sulfonic acid or a salt thereof; a phosphoric acid or a salt thereof; a $C_1$-$C_{60}$ alkyl group that is unsubstituted or substituted with deuterium, a $C_1$-$C_{60}$ alkyl group, a $C_6$-$C_{60}$ aryl group, or any combination thereof; a $C_2$-$C_{60}$ alkenyl group; a $C_2$-$C_{60}$ alkynyl group; a $C_1$-$C_{60}$ alkoxy group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group that is unsubstituted or substituted with deuterium, a $C_1$-$C_{60}$ alkyl group, a $C_6$-$C_{60}$ aryl group, or any combination thereof; a $C_6$-$C_{60}$ aryloxy group; a $C_6$-$C_{60}$ arylthio group; a $C_1$-$C_{60}$ heteroaryl group; a monovalent non-aromatic condensed polycyclic group; or a monovalent non-aromatic condensed heteropolycyclic group.
For example, $Q_1$ to $Q_3$, $Q_{11}$ to $Q_{13}$, $Q_{21}$ to $Q_{23}$, and $Q_{31}$ to $Q_{33}$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$,

$-CHDCD_3$, $-CD_2CD_3$, $-CD_2CD_2H$, or - $CD_2CDH_2$; or

an n-propyl group, an iso-propyl group, an n-butyl group, a sec-butyl group, an isobutyl group, a tert-butyl group, an n-pentyl group, a tert-pentyl group, a neo-pentyl group, an isopentyl group, a sec-pentyl group, a 3-pentyl group, a sec-isopentyl group, a phenyl group, a biphenyl group, or a naphthyl group, each unsubstituted or substituted with deuterium, a $C_1$-$C_{10}$ alkyl group, a phenyl group, or any combination thereof.

In an embodiment, the heterocyclic compound may be at least one of Compounds 1 to 792, but embodiments are not limited thereto:

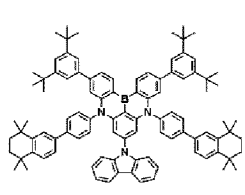
1
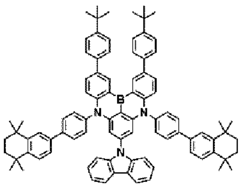
2
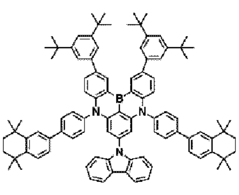
3
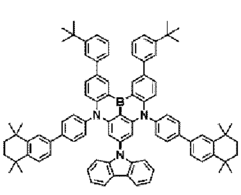
4
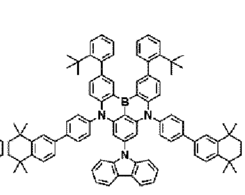
5

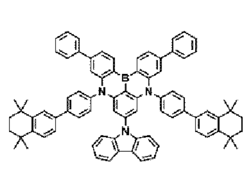
6
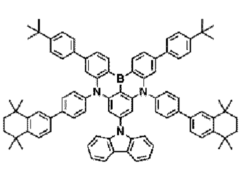
7
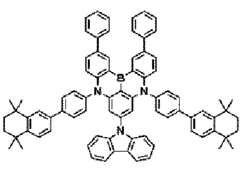
8
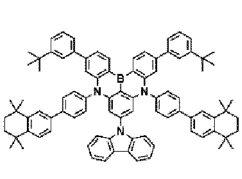
9
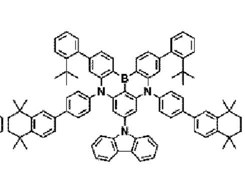
10

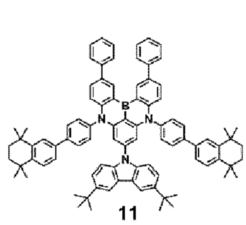
11
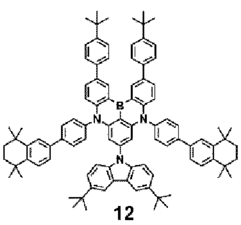
12
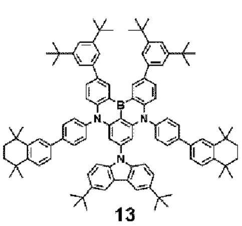
13
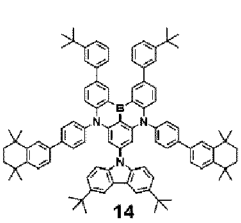
14
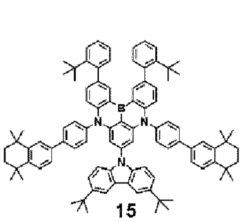
15

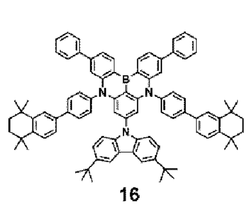
16
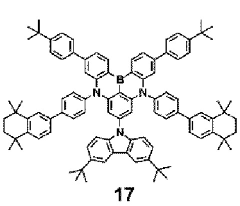
17
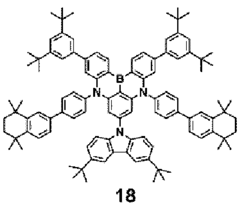
18
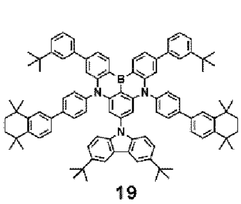
19
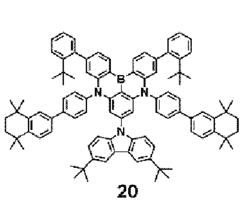
20

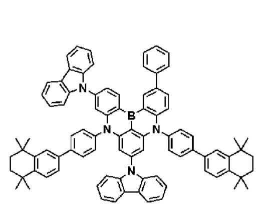
21
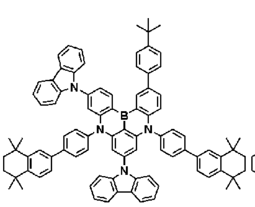
22
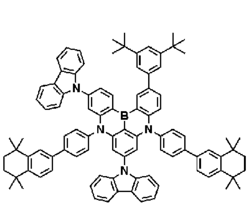
23
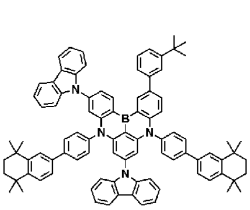
24
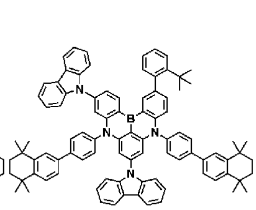
25

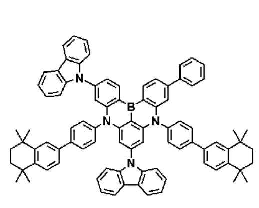
26
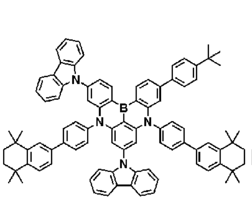
27
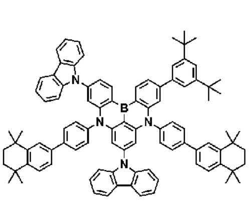
28
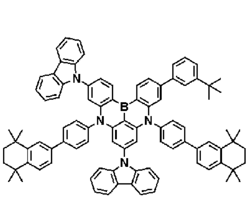
29
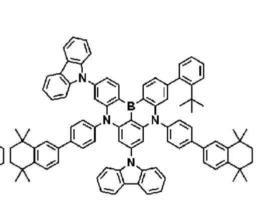
30

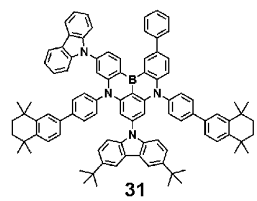
31

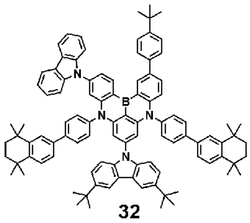
32

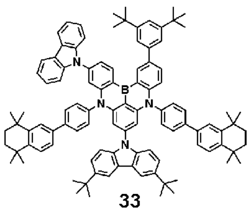
33

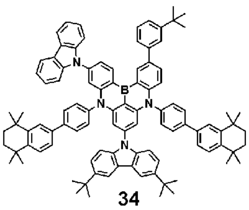
34

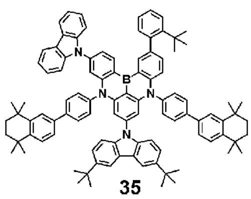
35

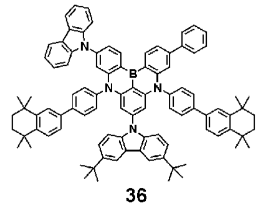
36

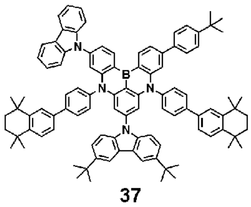
37

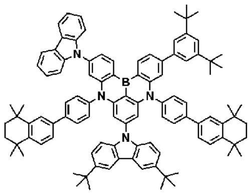
38

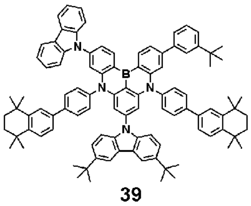
39

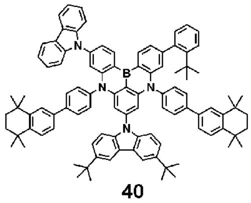
40

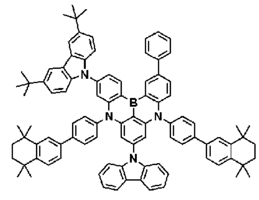
41

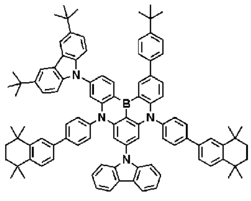
42

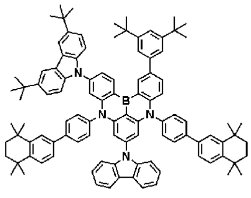
43

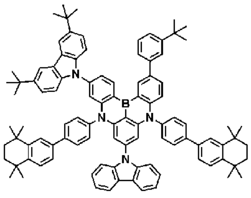
44

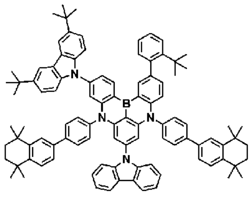
45

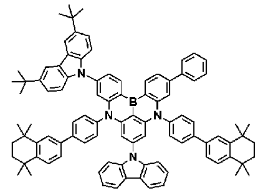
46

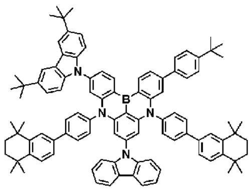
47

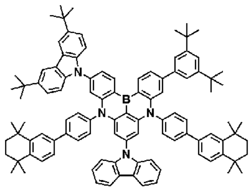
48

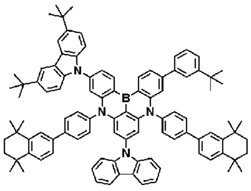
49

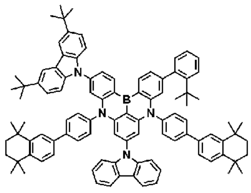
50

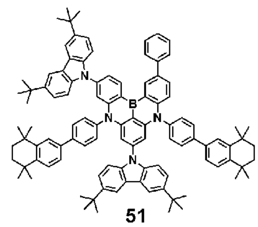
51

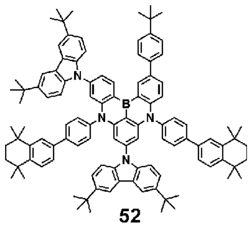
52

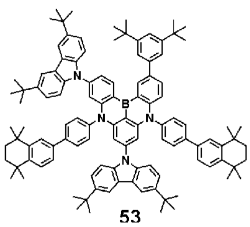
53

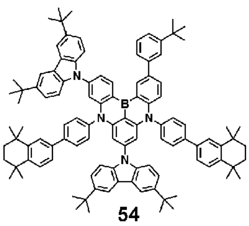
54

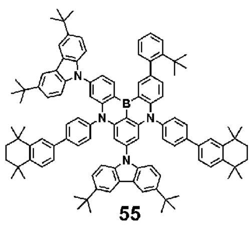
55

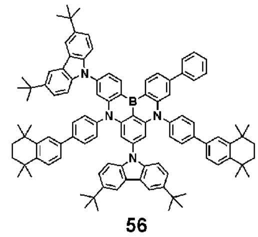
56

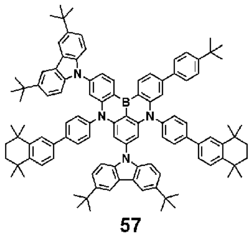
57

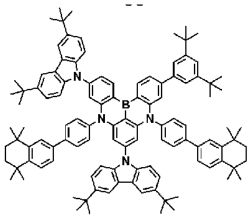
58

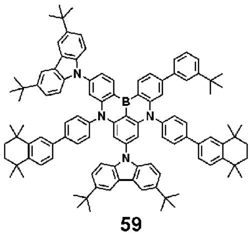
59

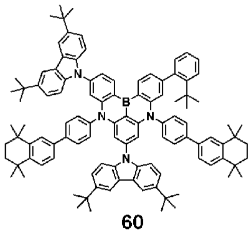
60

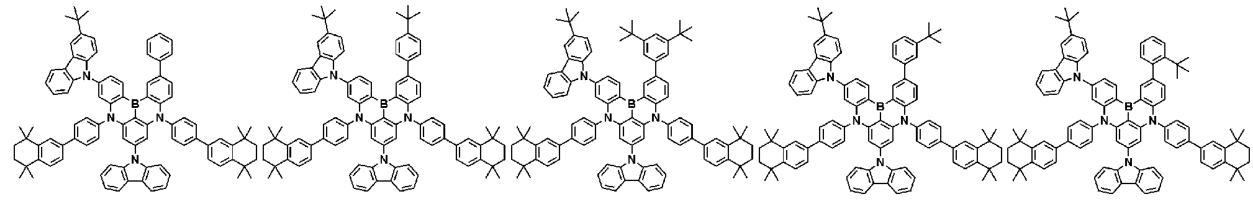

61 62 63 64 65

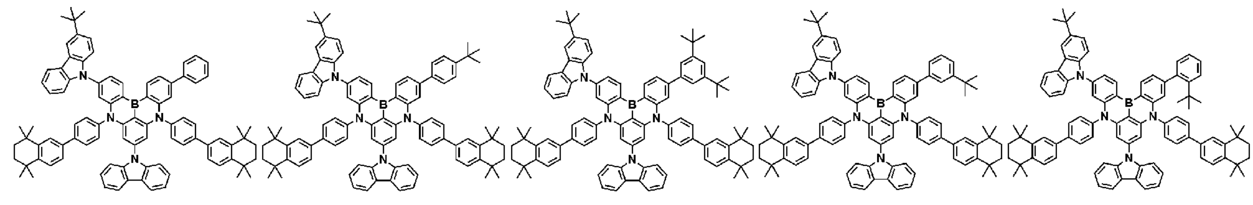

66 67 68 69 70

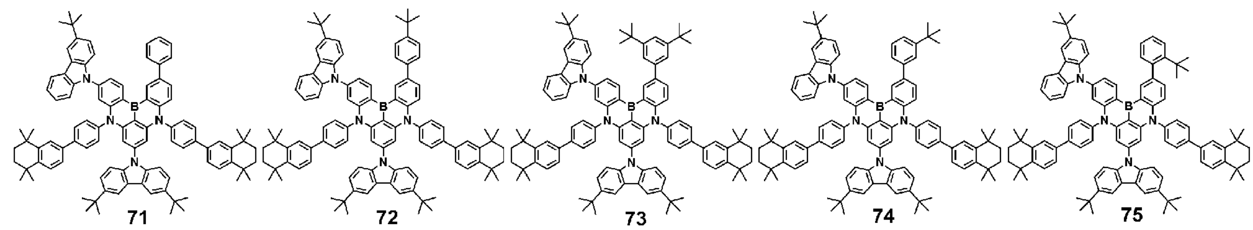

71 72 73 74 75

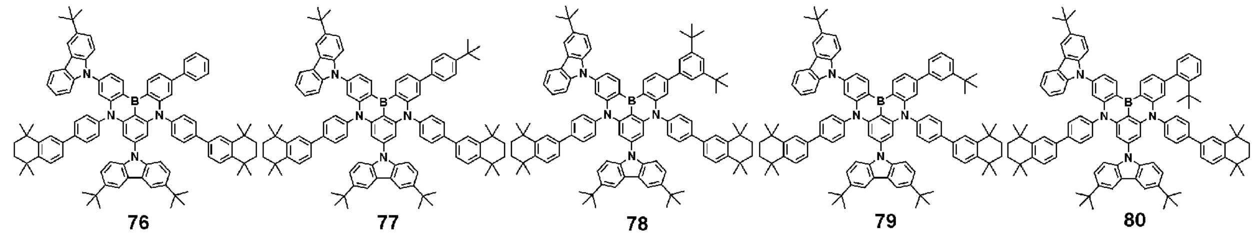

76 77 78 79 80

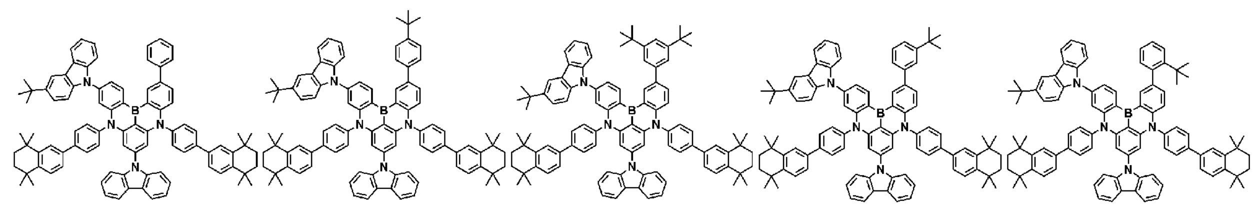

81 82 83 84 85

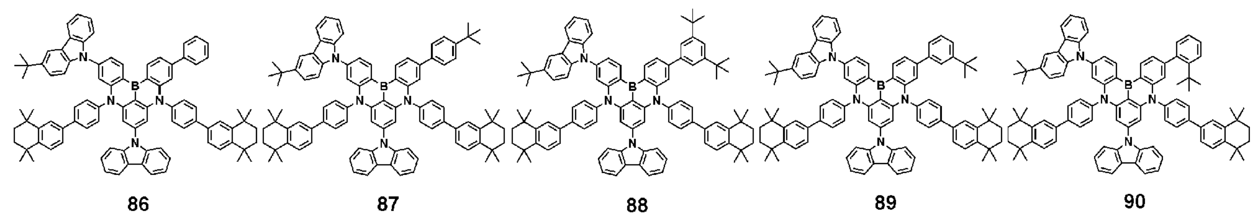

86 87 88 89 90

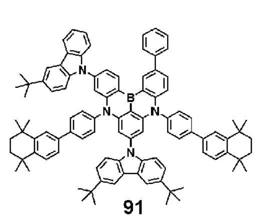
91
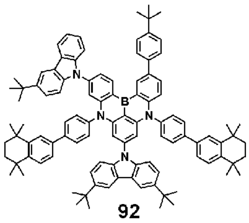
92
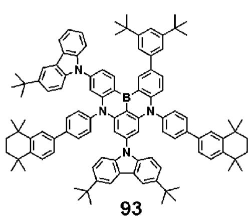
93
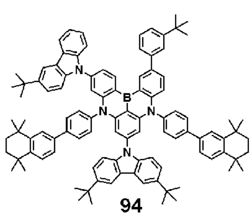
94
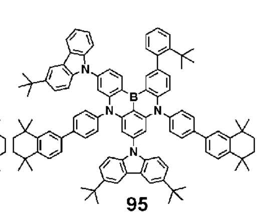
95

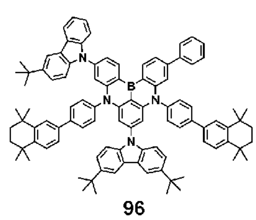
96
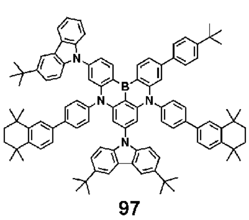
97
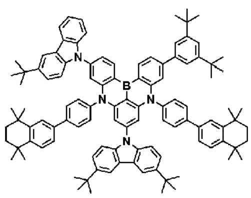
98
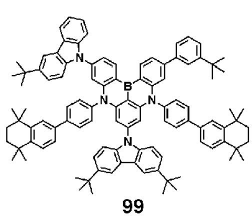
99
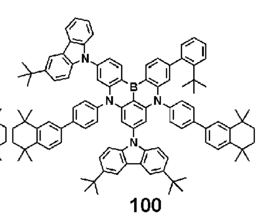
100

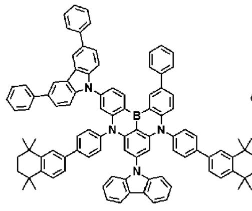
101
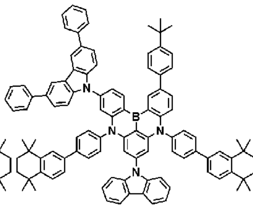
102
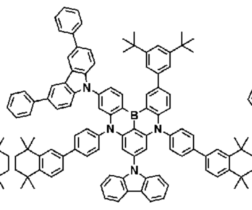
103
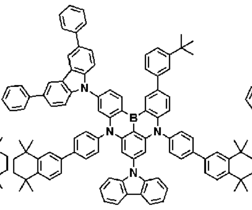
104
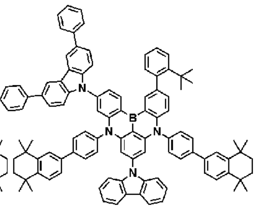
105

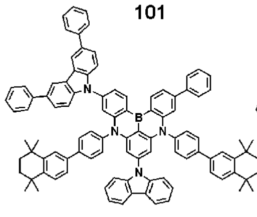
106
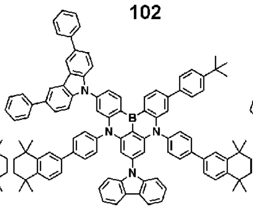
107
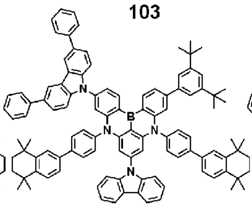
108
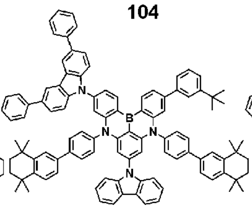
109
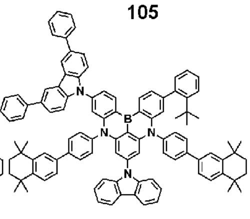
110

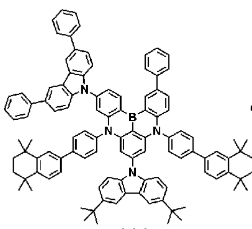
111
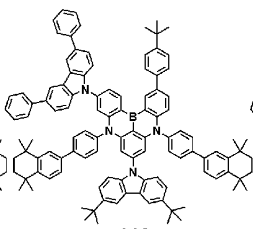
112
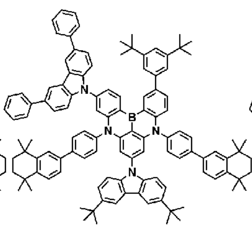
113
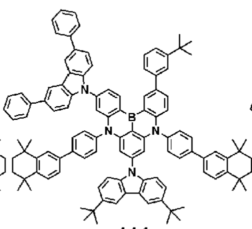
114
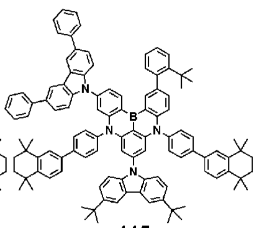
115

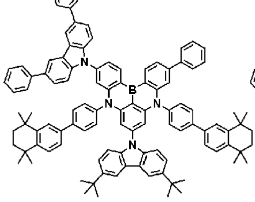
116
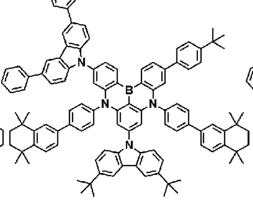
117
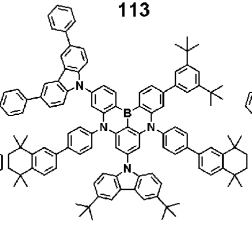
118
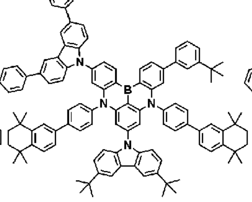
119
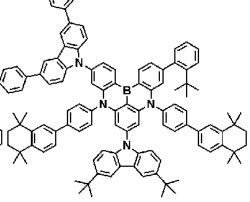
120

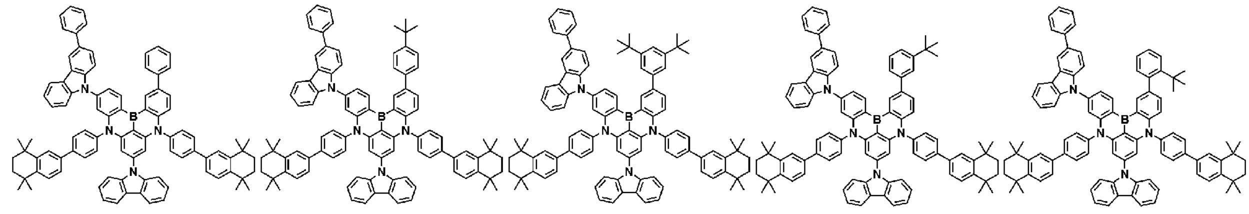

121 122 123 124 125

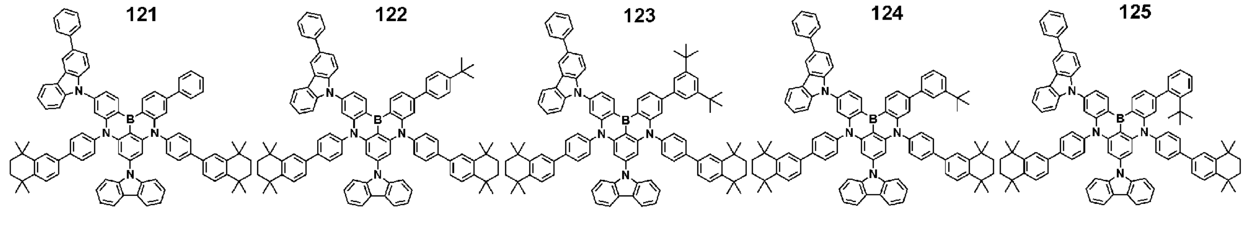

126 127 128 129 130

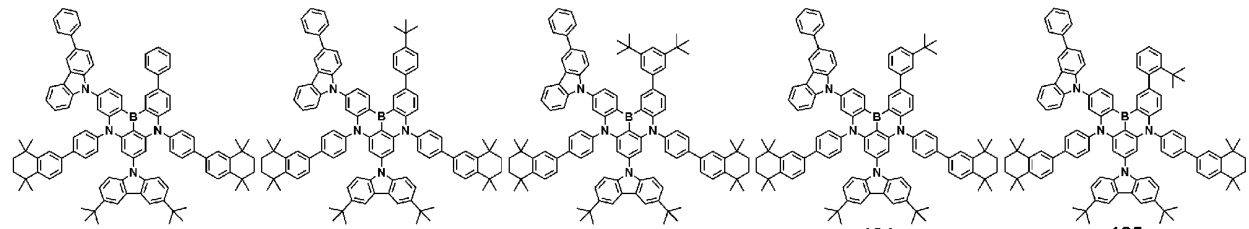

131 132 133 134 135

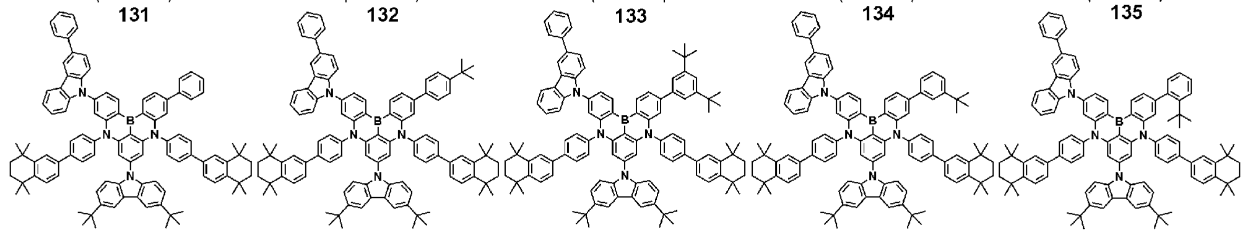

136 137 138 139 140

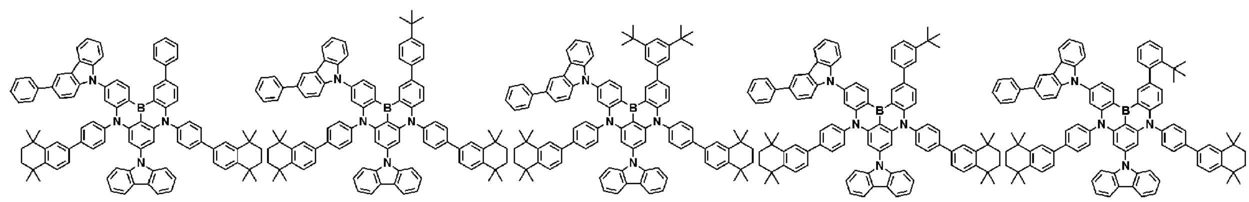

141 142 143 144 145

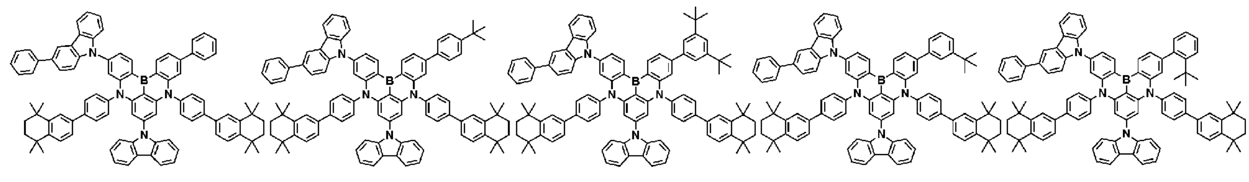

146 147 148 149 150

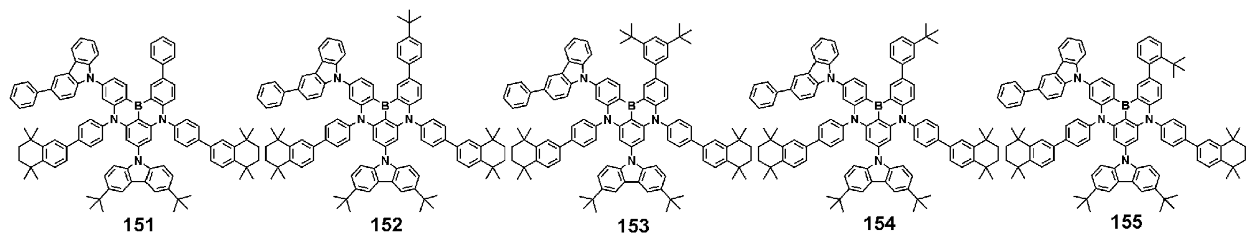

151 152 153 154 155

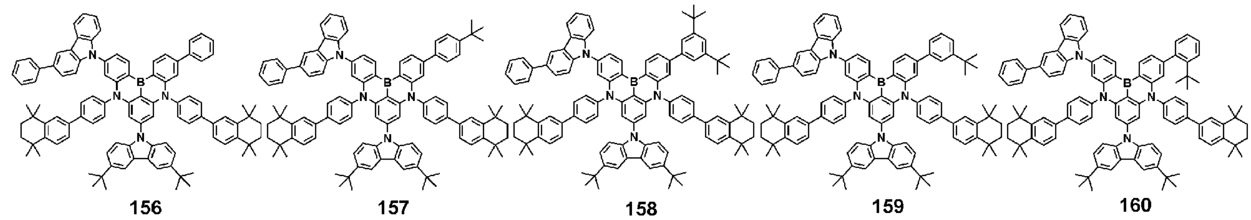

156 157 158 159 160

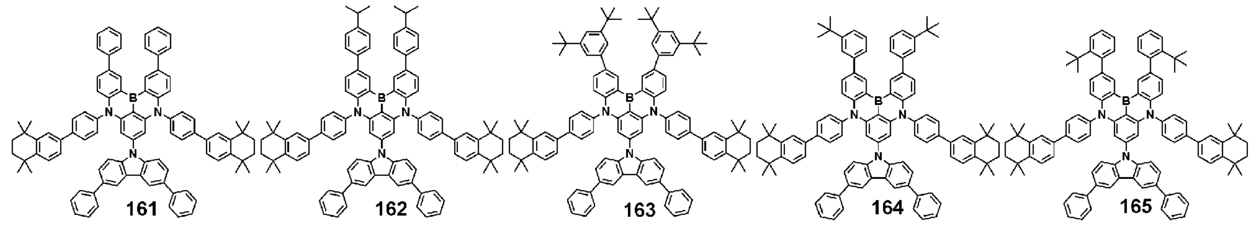

161 162 163 164 165

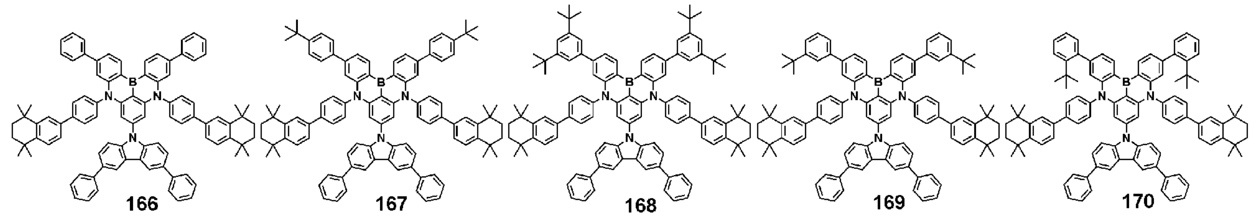

166 167 168 169 170

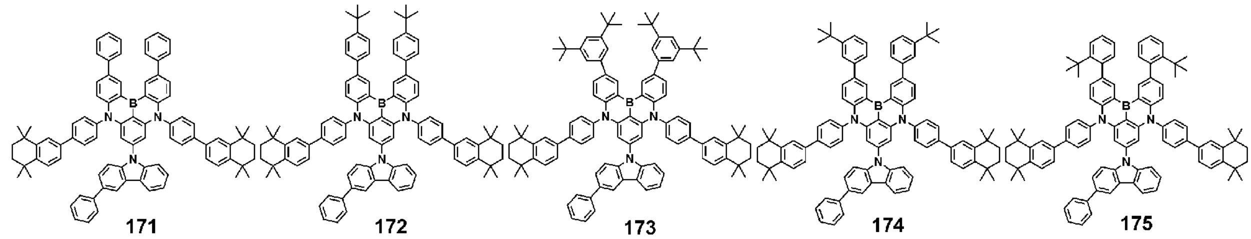

171 172 173 174 175

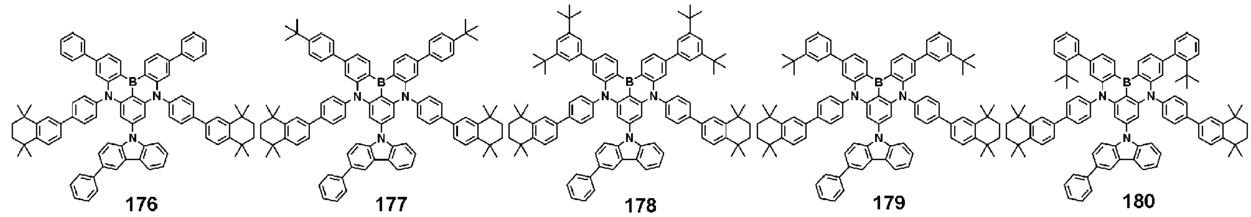

176 177 178 179 180

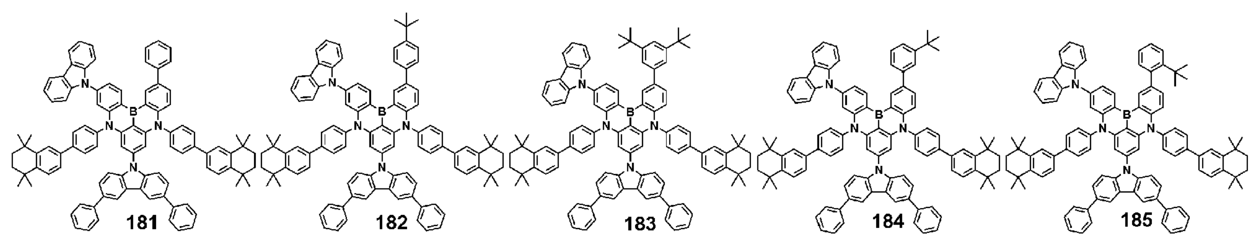

181 182 183 184 185

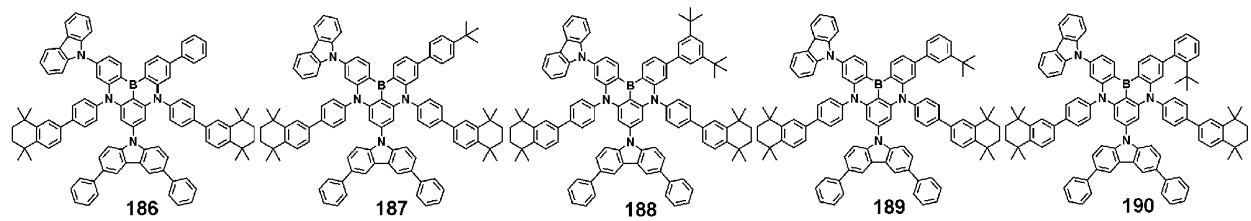

186 187 188 189 190

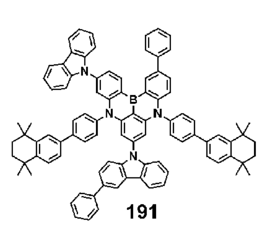
191
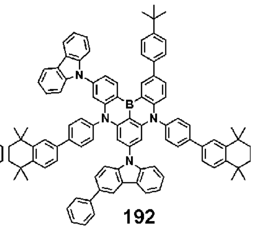
192
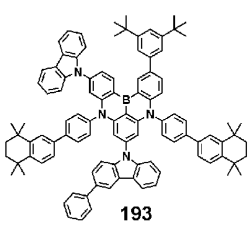
193
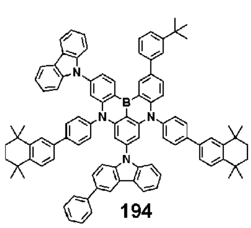
194
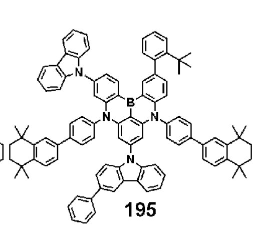
195

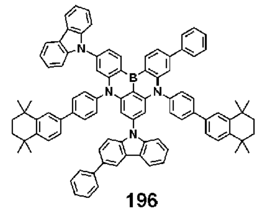
196
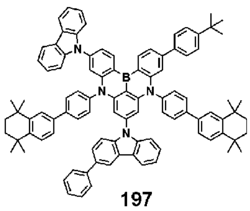
197
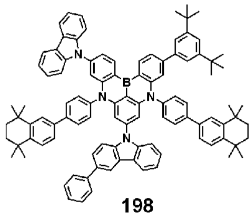
198
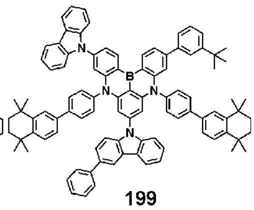
199
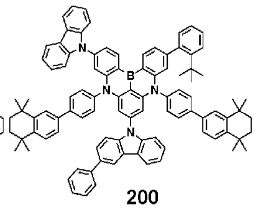
200

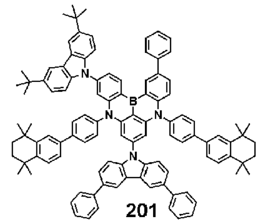
201
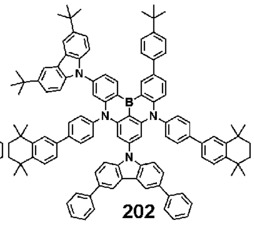
202
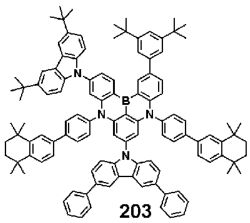
203
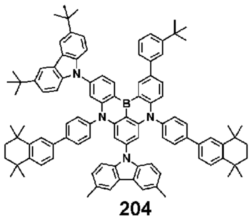
204
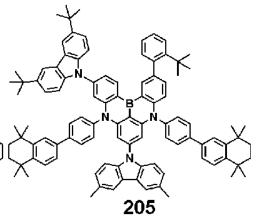
205

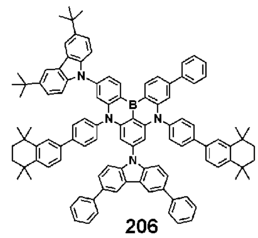
206
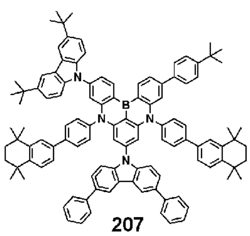
207
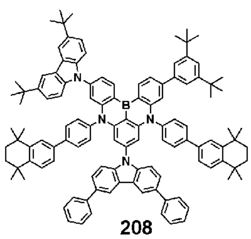
208
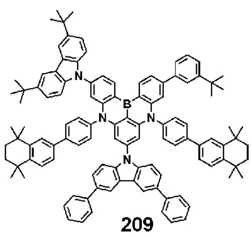
209
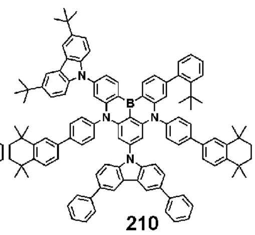
210

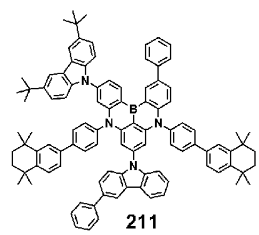
211
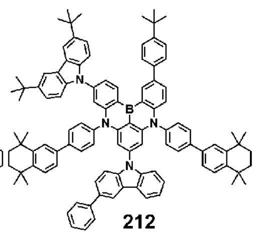
212
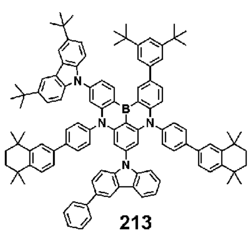
213
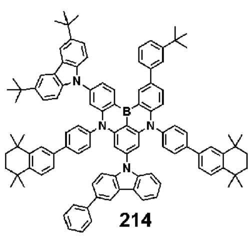
214
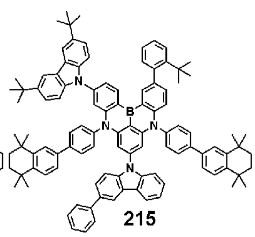
215

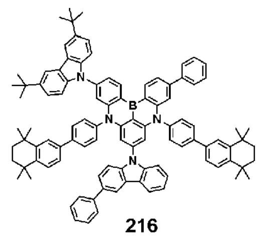
216
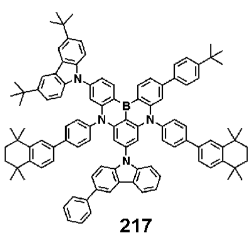
217
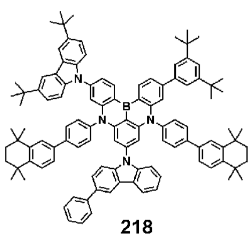
218
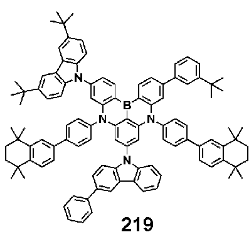
219
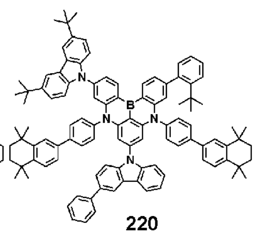
220

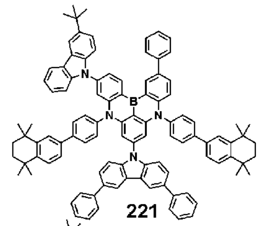
221
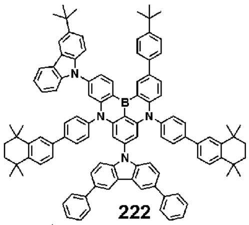
222
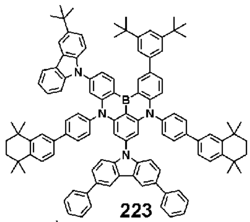
223
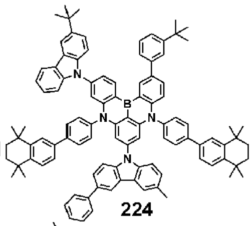
224
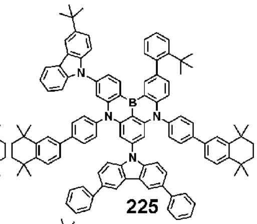
225
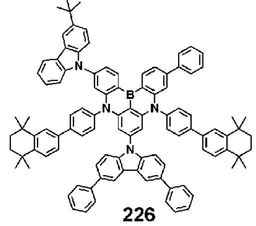
226
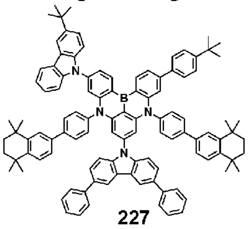
227
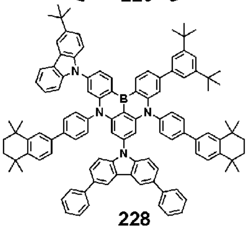
228
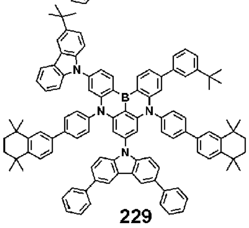
229
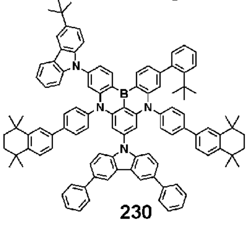
230
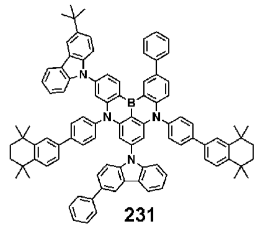
231
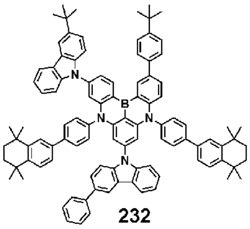
232
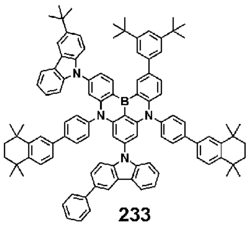
233
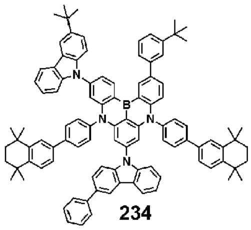
234
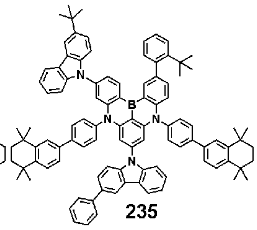
235
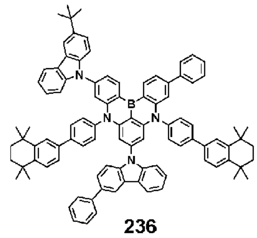
236
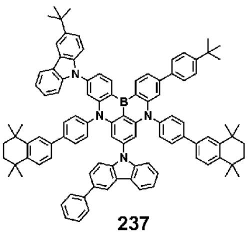
237
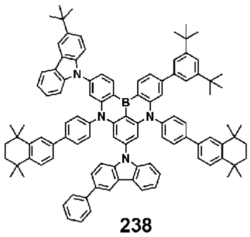
238
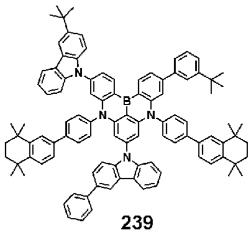
239
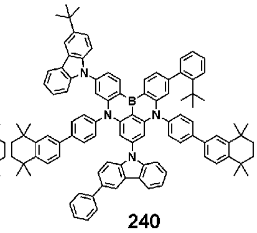
240
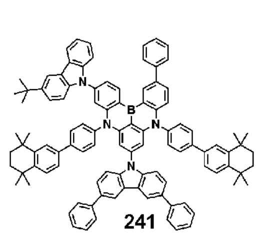
241
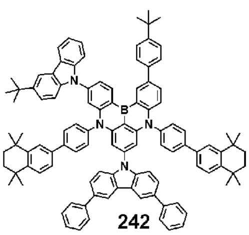
242
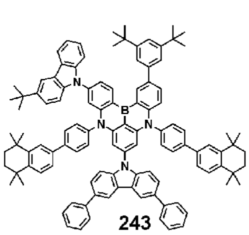
243
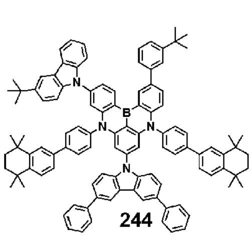
244
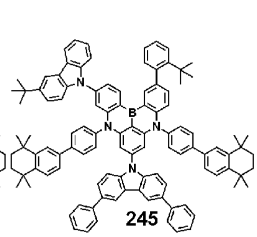
245
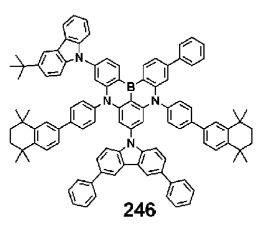
246
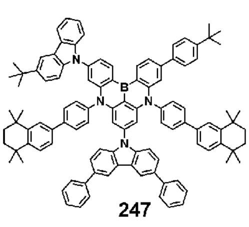
247
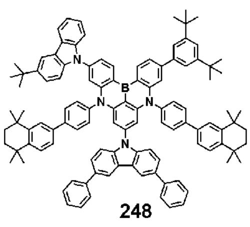
248
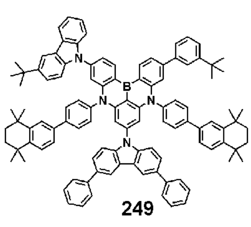
249
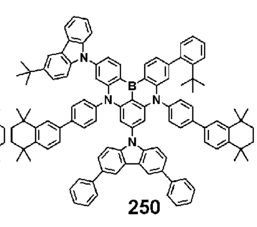
250
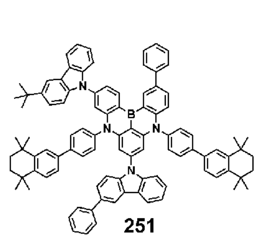
251
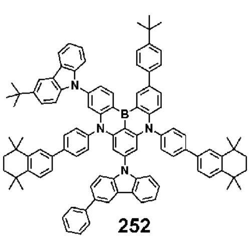
252
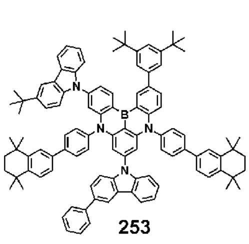
253
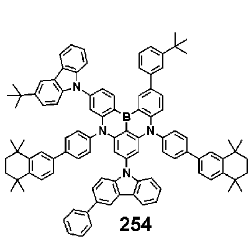
254
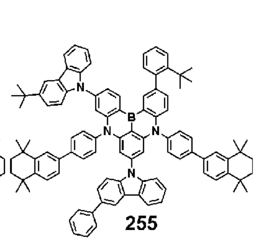
255

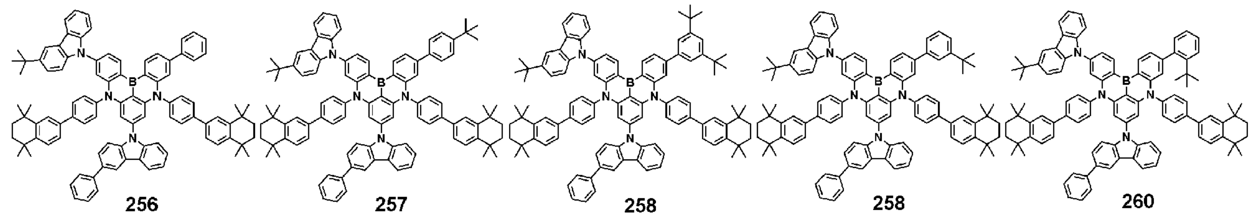

256 257 258 258 260

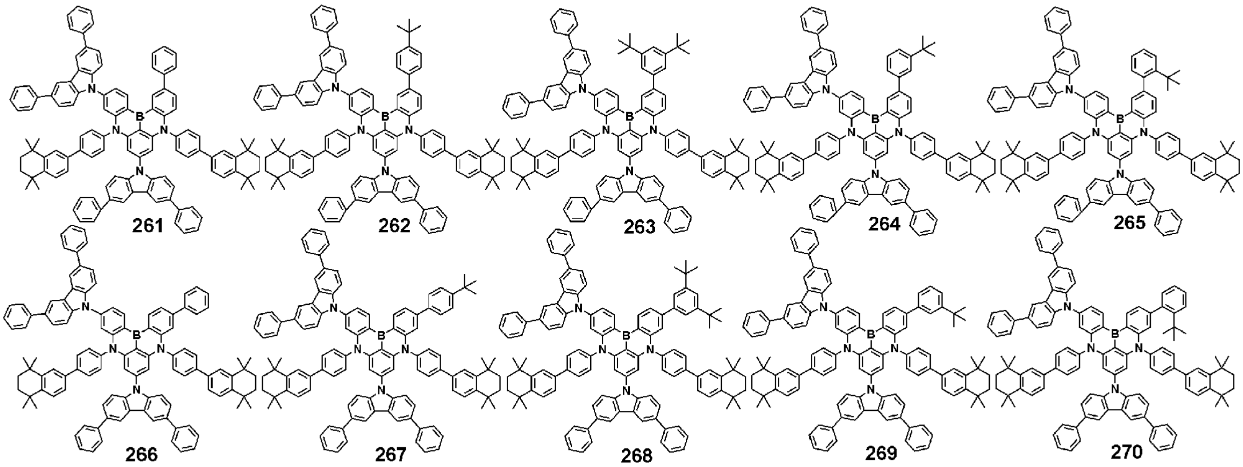

261 262 263 264 265

266 267 268 269 270

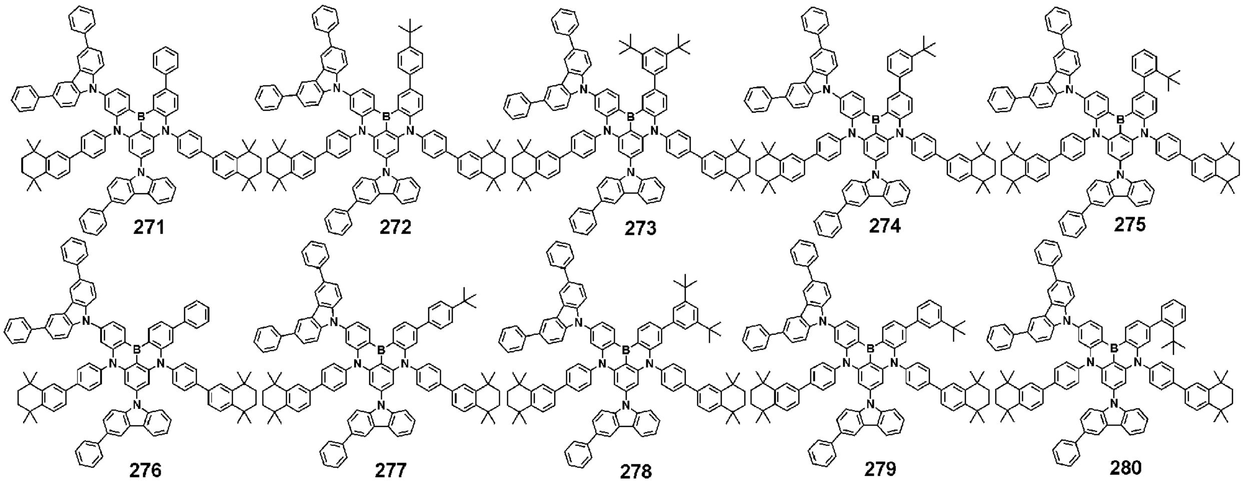

271 272 273 274 275

276 277 278 279 280

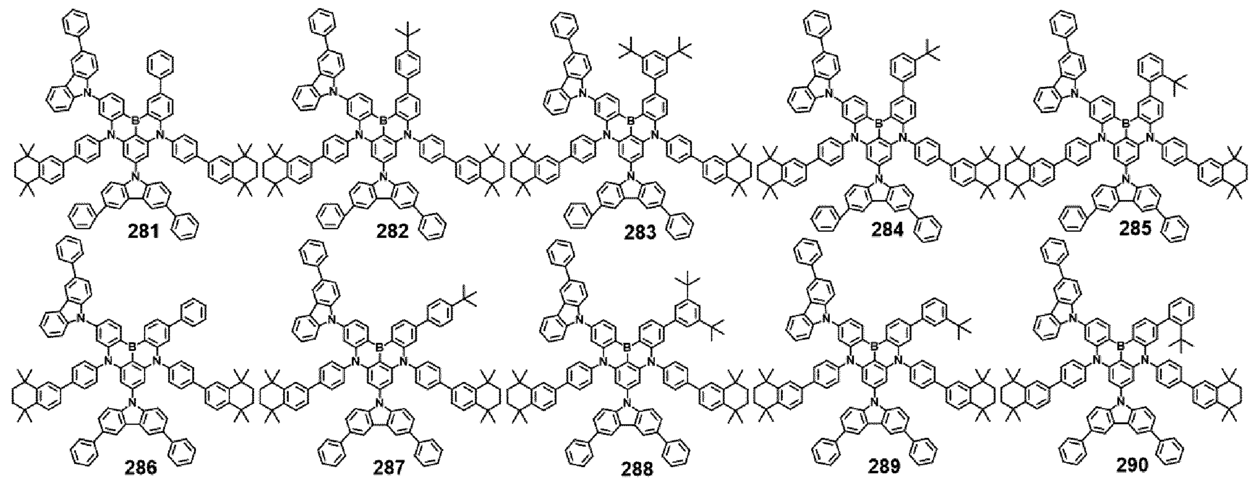

281 282 283 284 285

286 287 288 289 290

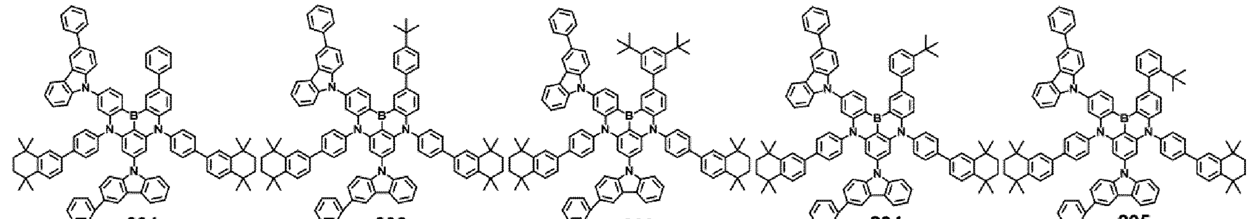

291 292 293 294 295

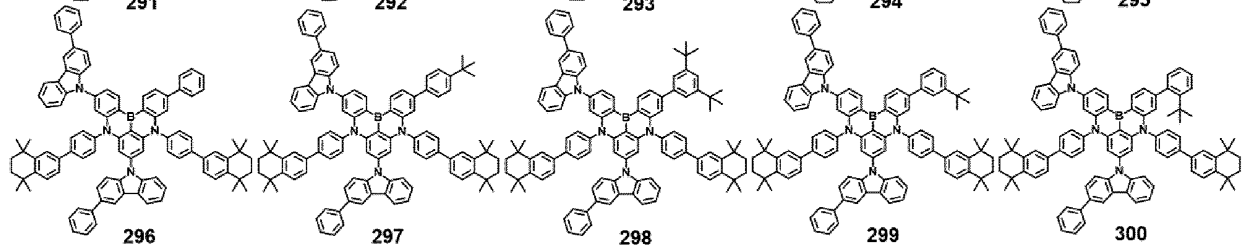

296 297 298 299 300

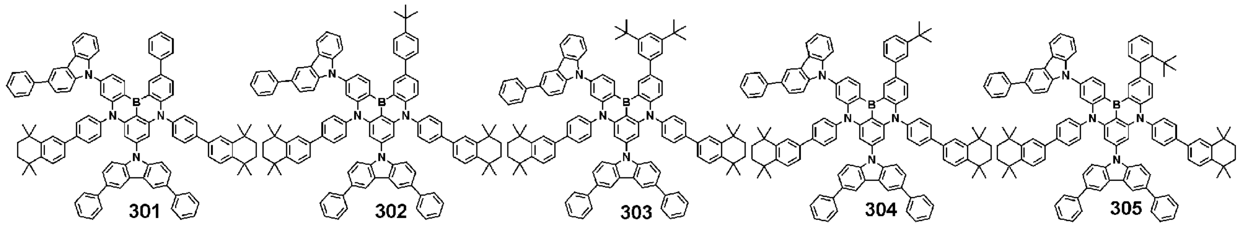

301 302 303 304 305

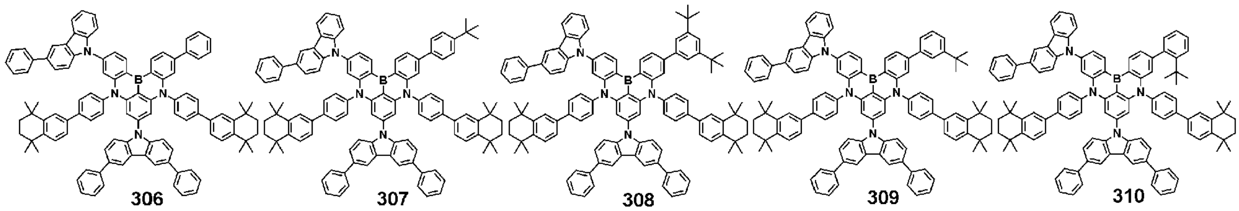

306 307 308 309 310

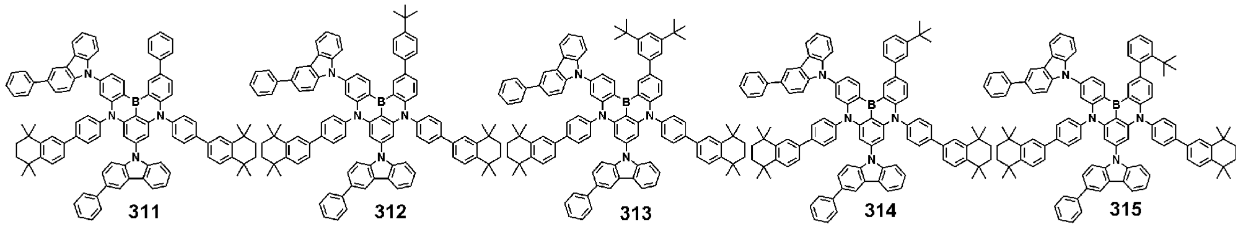

311 312 313 314 315

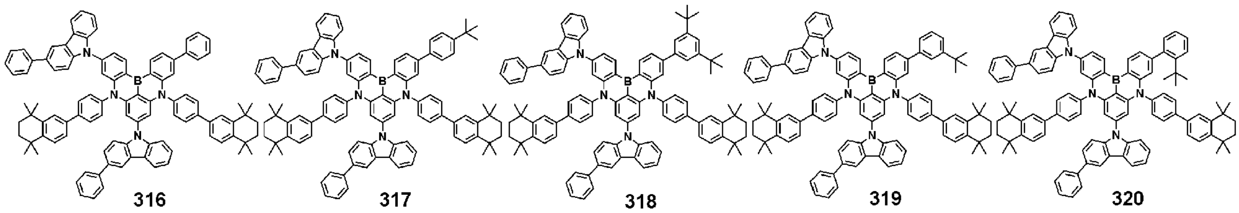

316 317 318 319 320

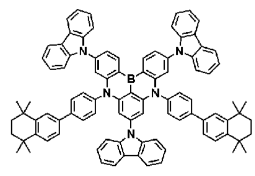
321
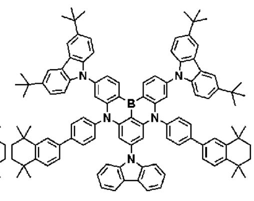
322
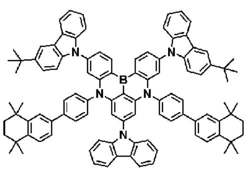
323
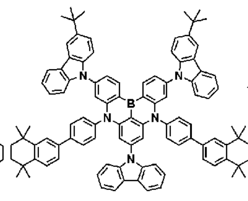
324
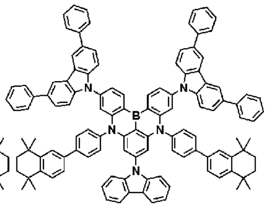
325
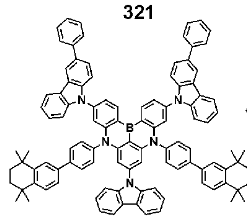
326
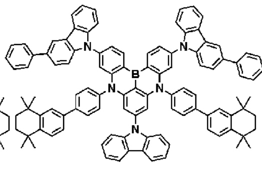
327
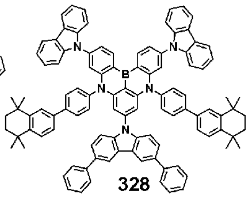
328
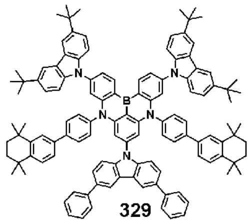
329
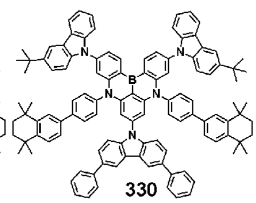
330
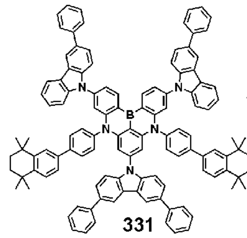
331
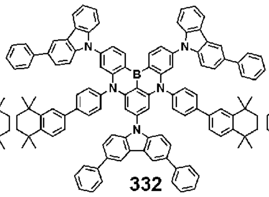
332
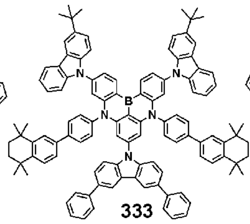
333
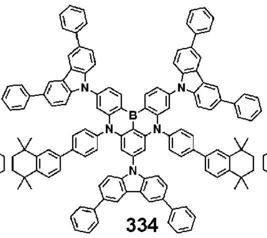
334
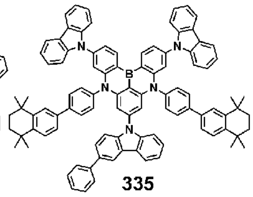
335
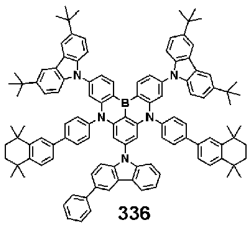
336
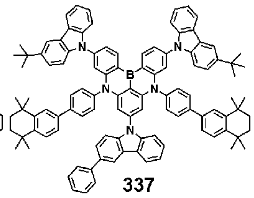
337
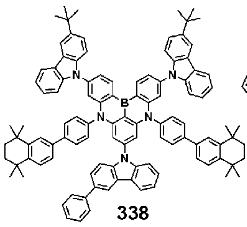
338
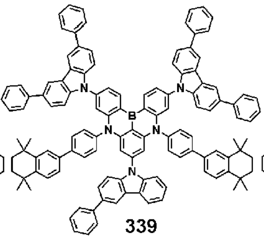
339
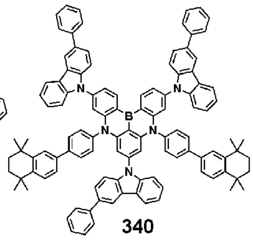
340
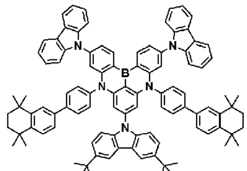
341
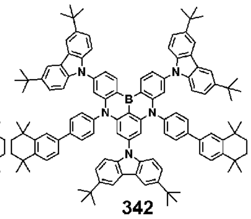
342
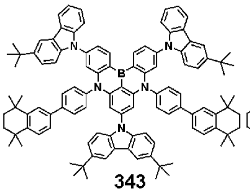
343
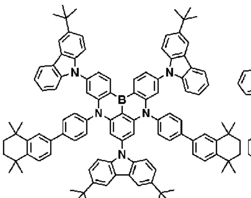
344
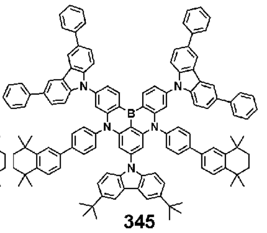
345
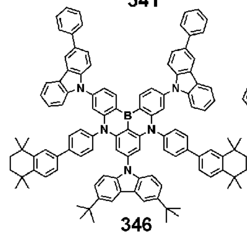
346
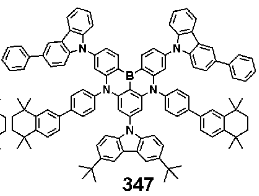
347
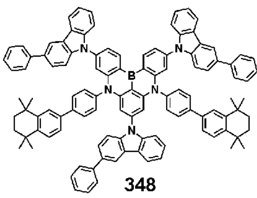
348
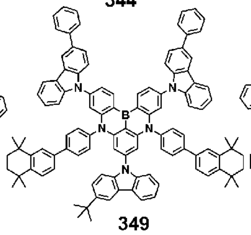
349
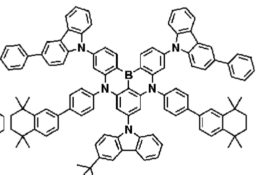
350
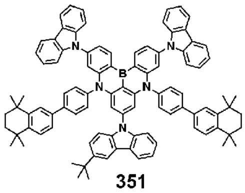
351
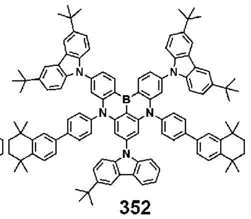
352
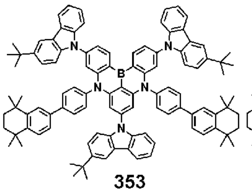
353
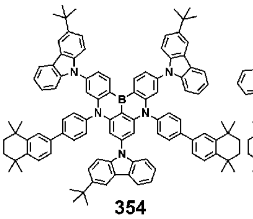
354
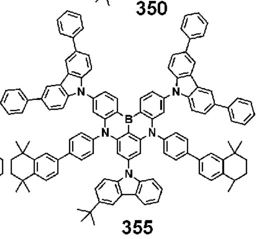
355

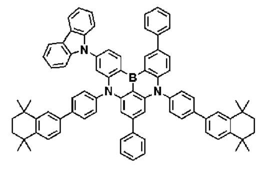
356
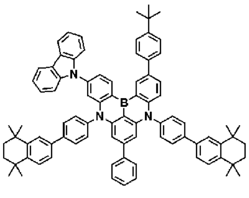
357
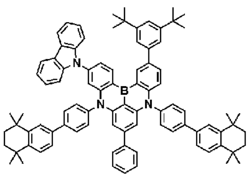
358
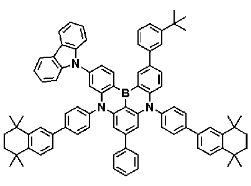
359
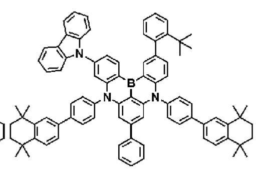
360

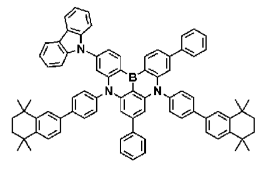
361
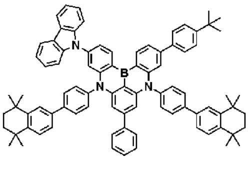
362
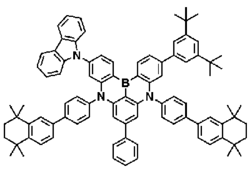
363
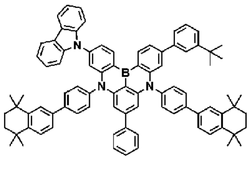
364
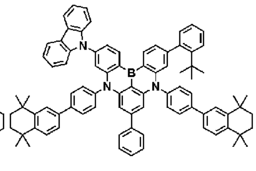
365

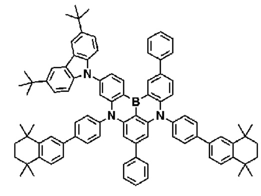
366
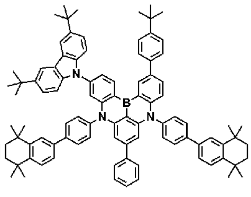
367
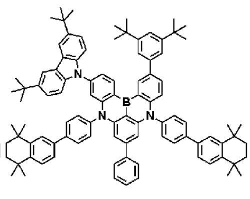
368
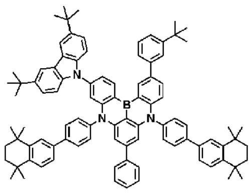
369
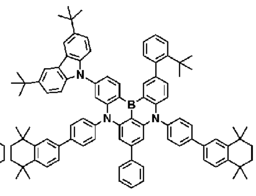
370

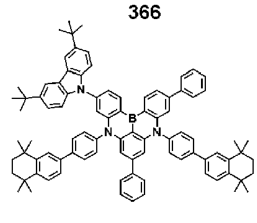
371
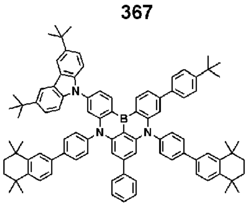
372
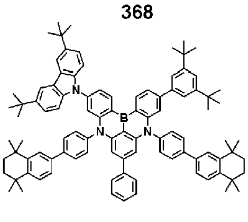
373
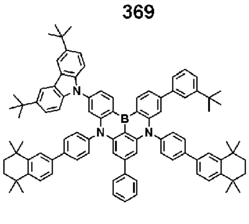
374
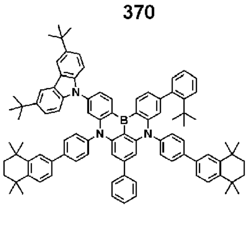
375

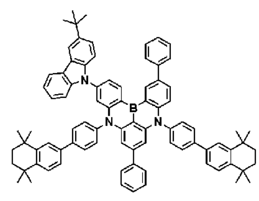
376
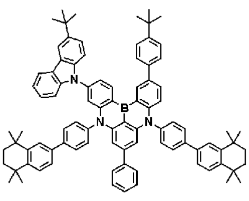
377
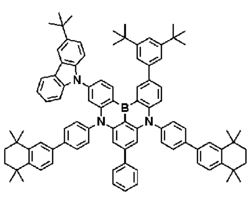
378
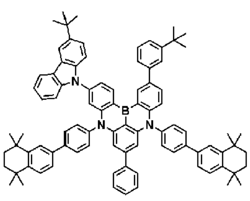
379
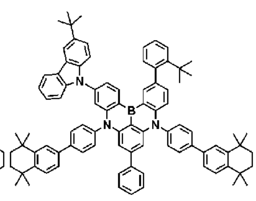
380

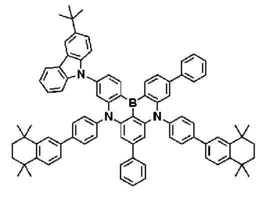
381
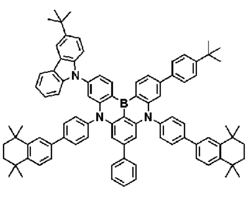
382
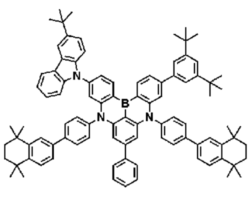
383
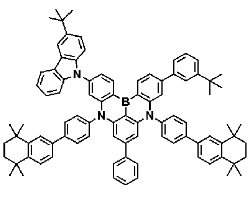
384
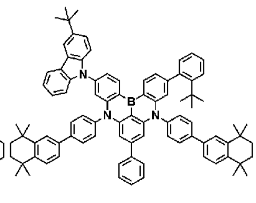
385

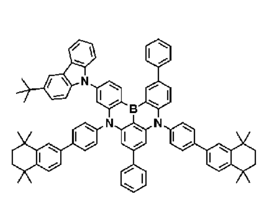
386
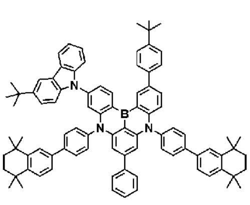
387
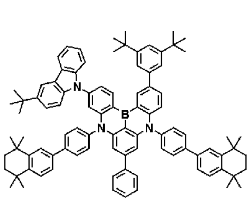
388
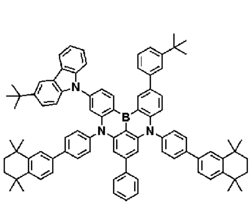
389
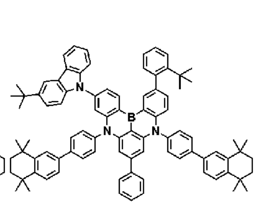
390

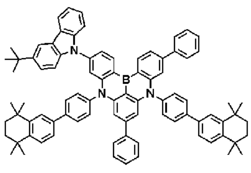
391
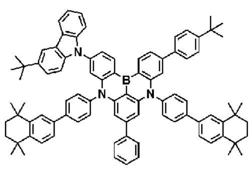
392
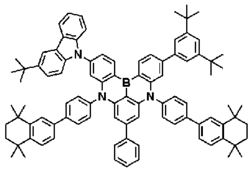
393
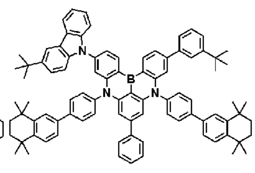
394
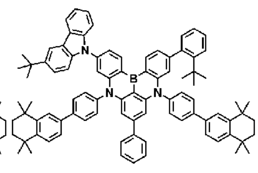
395

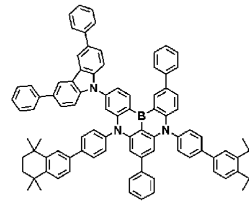
396
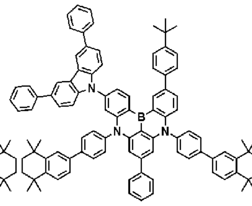
397
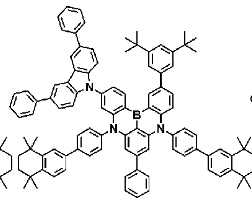
398
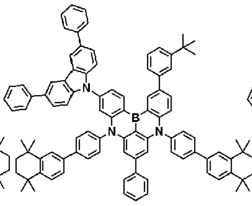
399
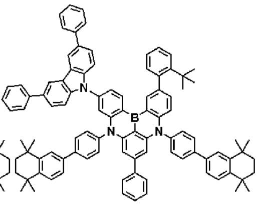
400

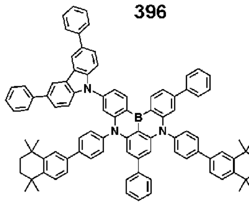
401
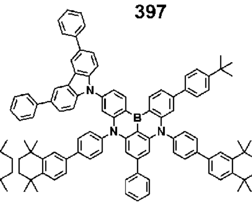
402
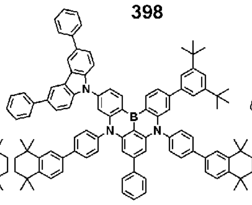
403
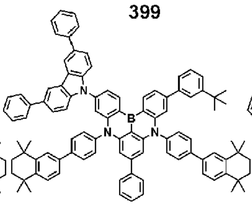
404
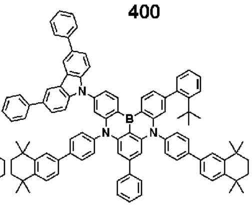
405

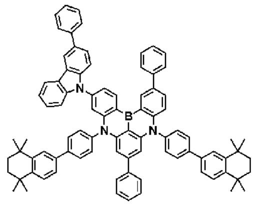
406
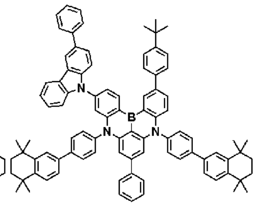
407
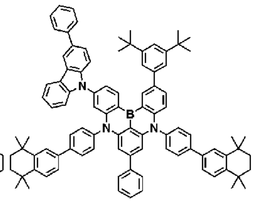
408
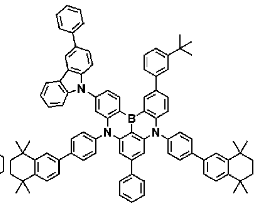
409
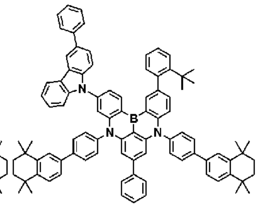
410

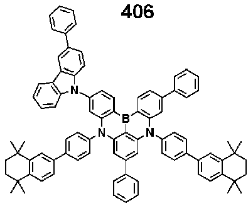
411
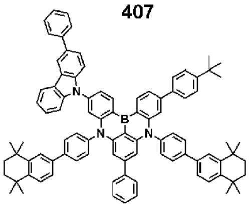
412
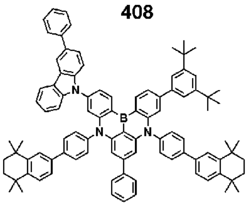
413
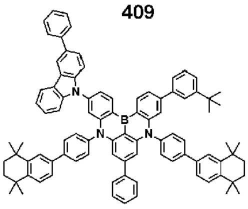
414
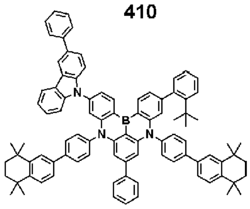
415

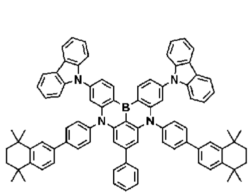
416
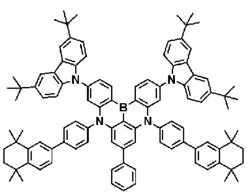
417
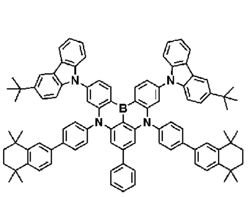
418
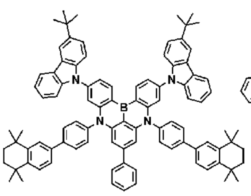
419
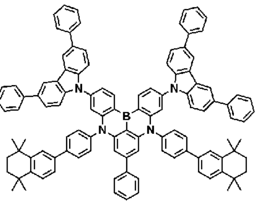
420

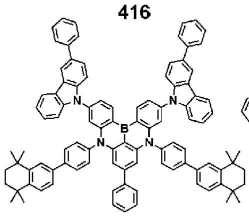
421
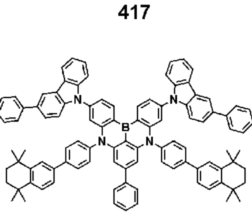
422
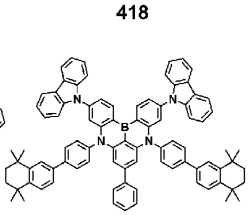
423
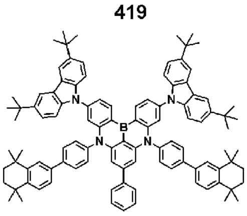
424
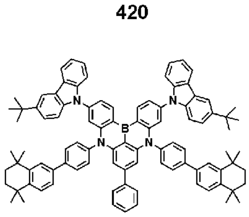
425

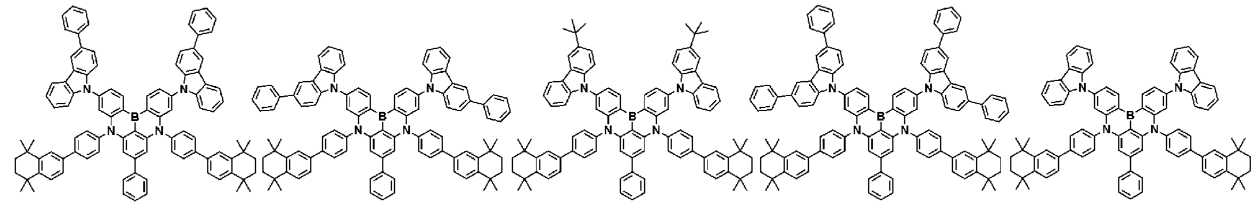

426 427 428 429 430

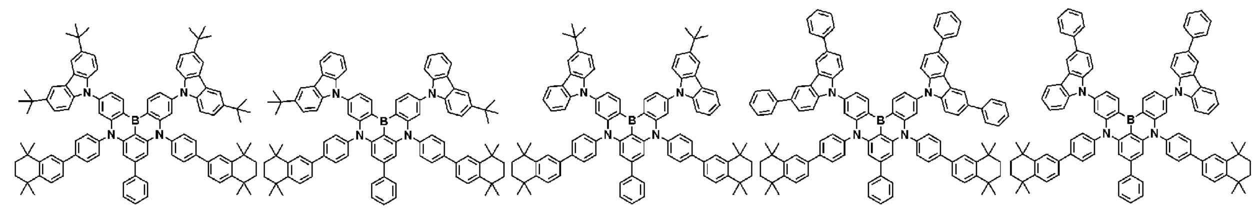

431 432 433 434 435

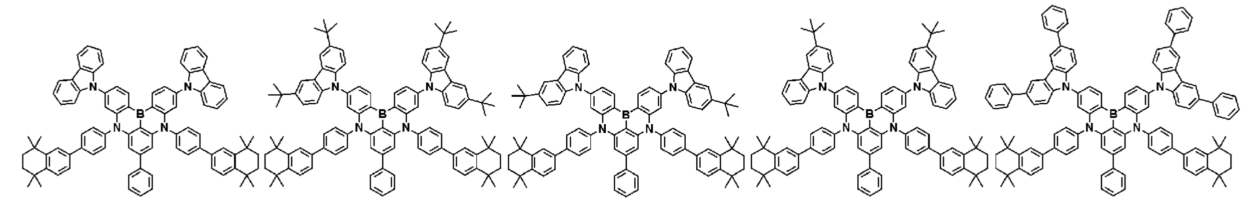

436 437 438 439 440

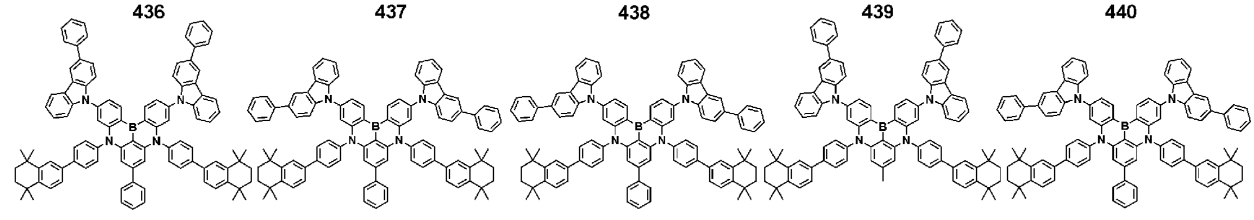

441 442 443 444 445

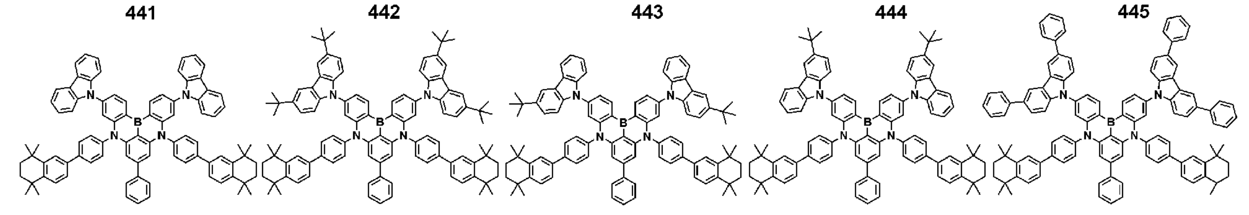

446 447 448 449 450

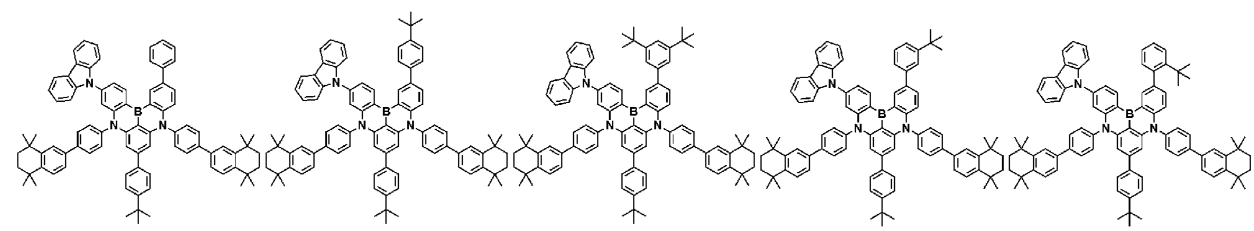

451 452 453 454 455

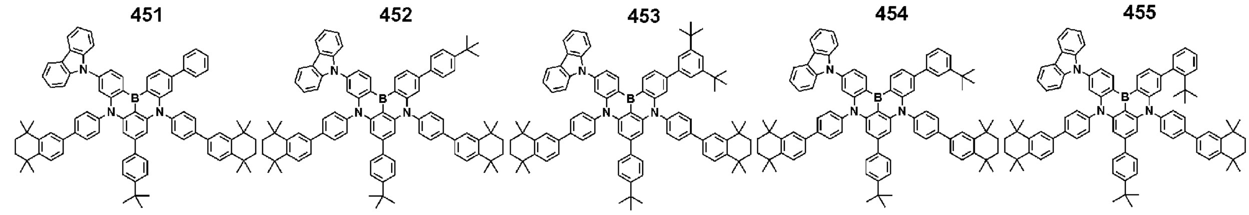

456 457 458 459 460

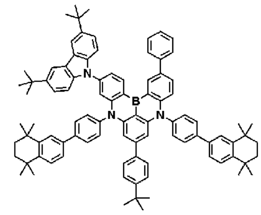
461
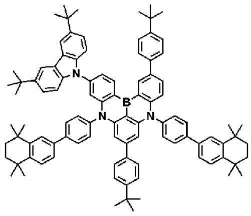
462
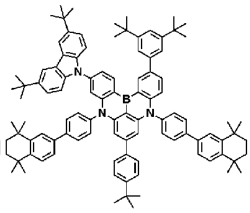
463
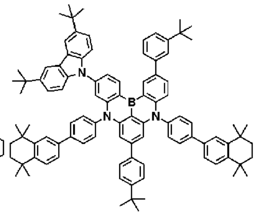
464
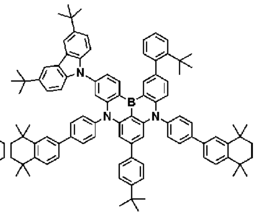
465
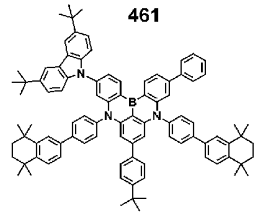
466
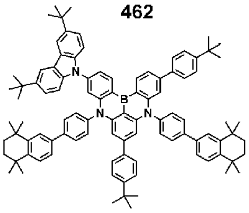
467
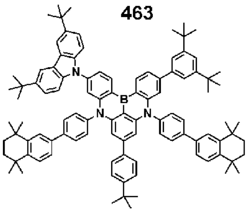
468
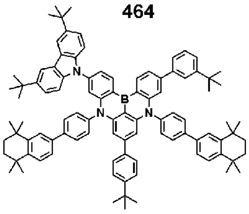
469
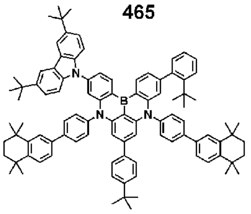
470
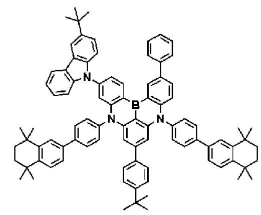
471
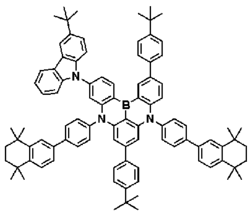
472
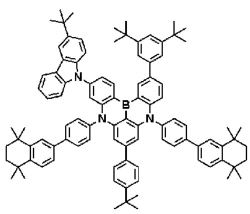
473
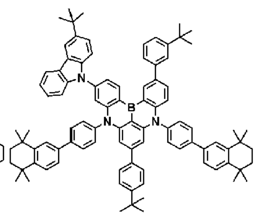
474
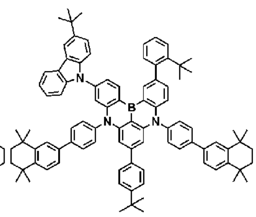
475
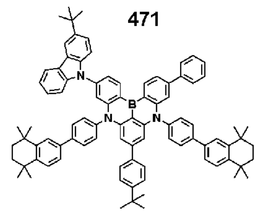
476
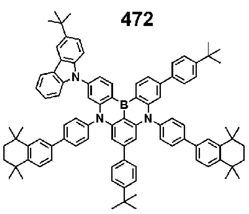
477.
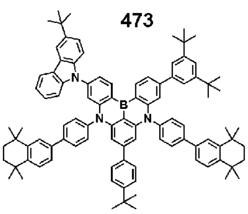
478
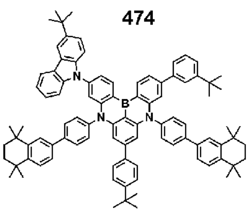
479
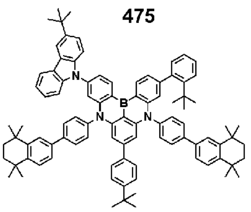
480
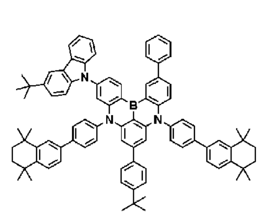
481
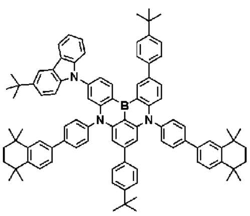
482
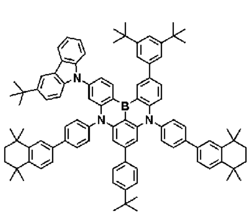
483
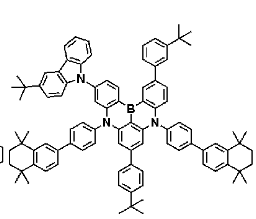
484
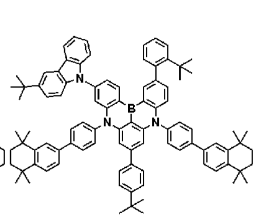
485
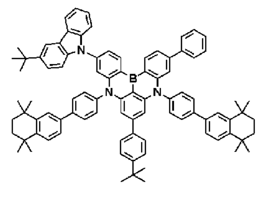
486
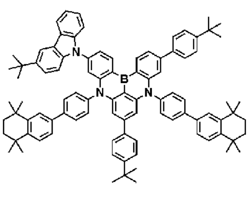
487
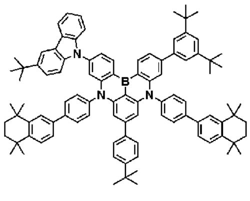
488
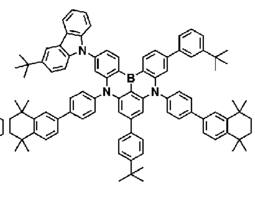
489
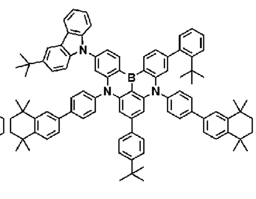
490

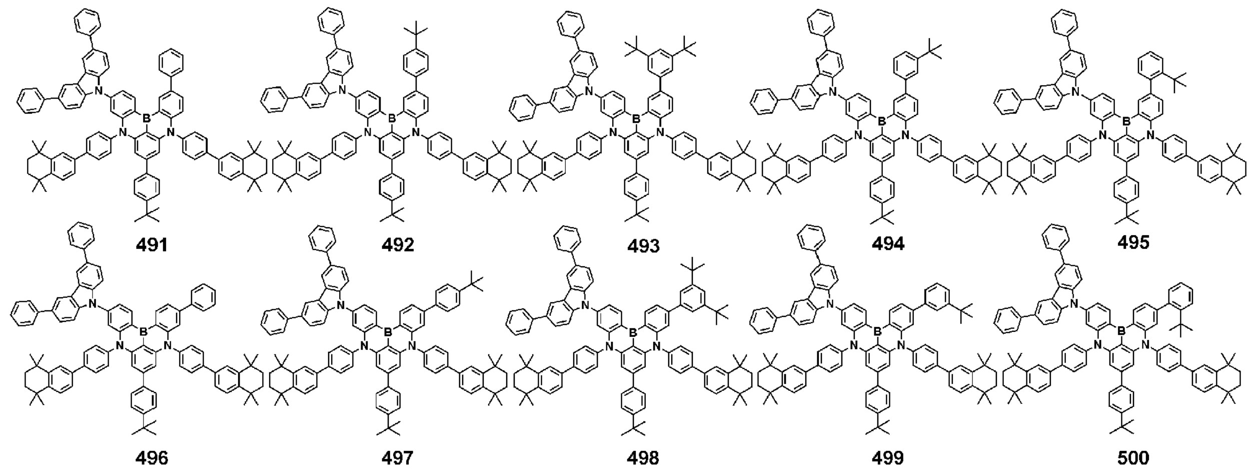

491 492 493 494 495

496 497 498 499 500

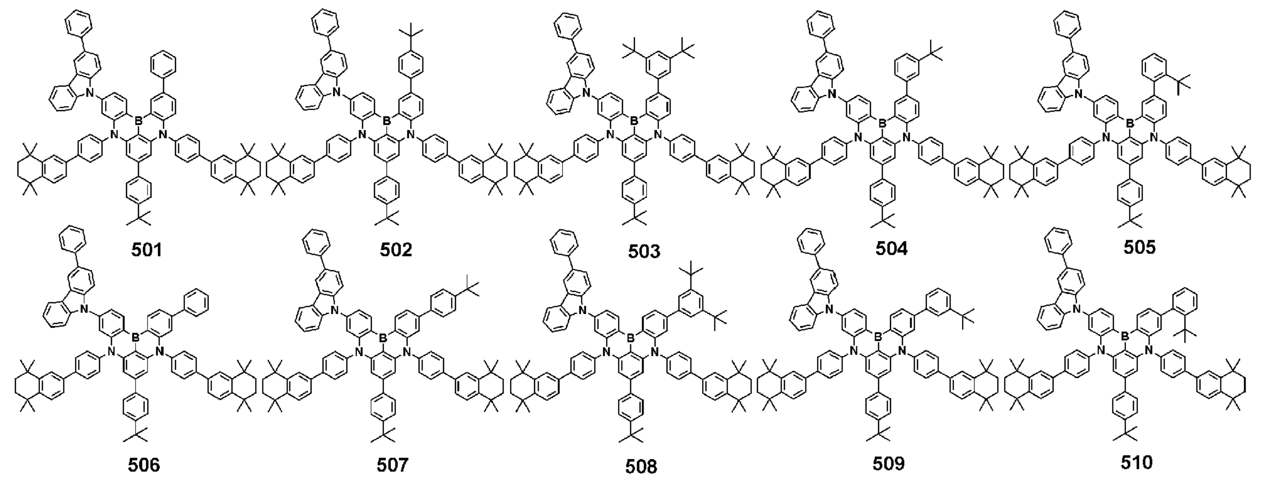

501 502 503 504 505

506 507 508 509 510

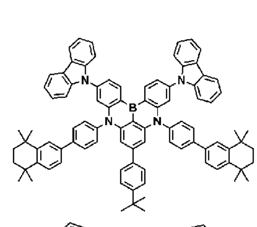
511
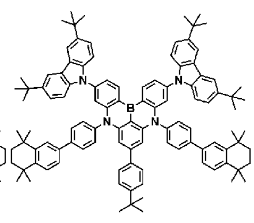
512
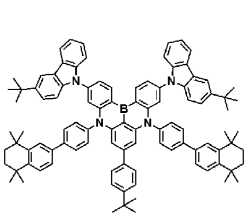
513
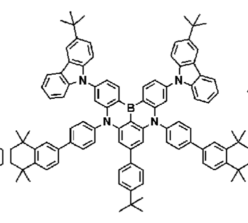
514
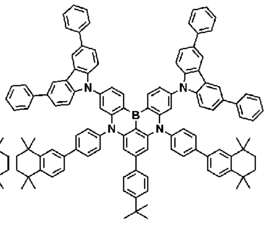
515

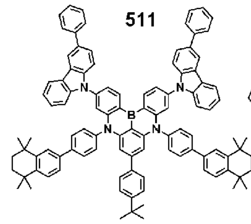
516
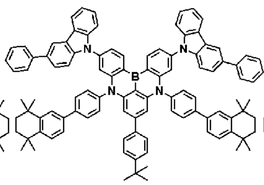
517
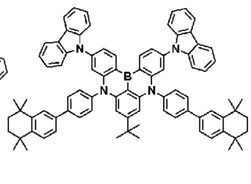
518
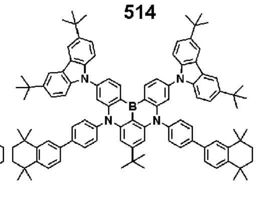
519
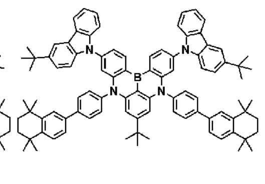
520

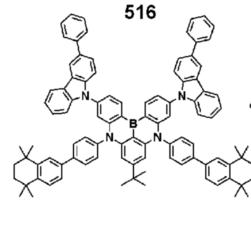
521
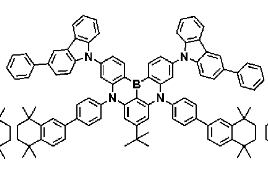
521
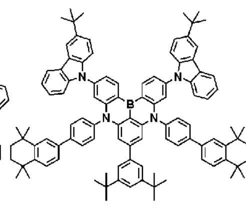
523
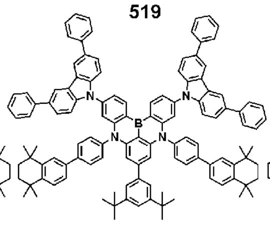
524
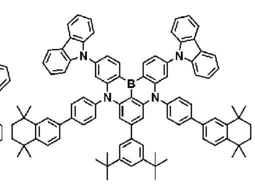
525

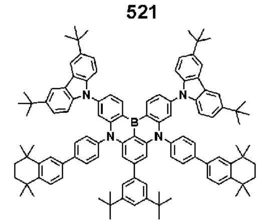
526
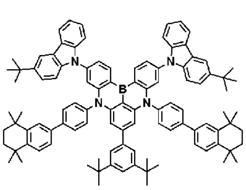
527
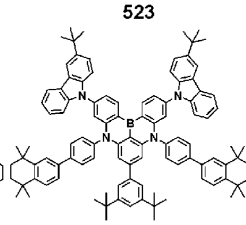
528
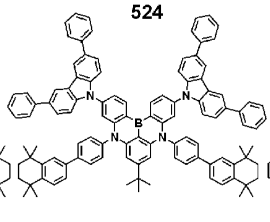
529
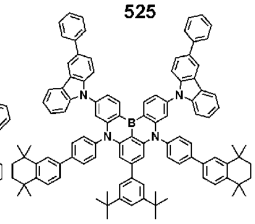
530

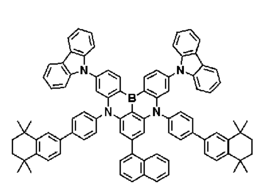
531
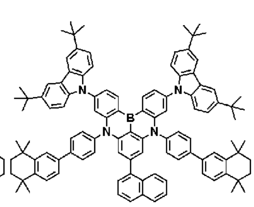
532
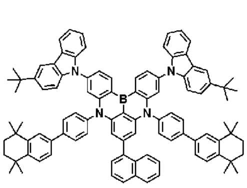
533
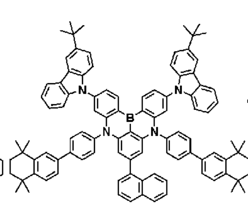
534
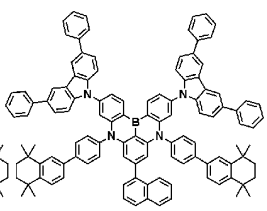
535

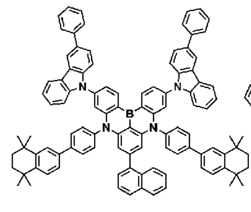
536
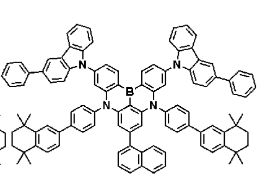
537
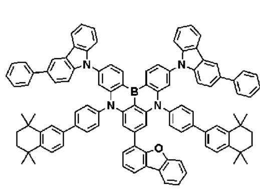
538
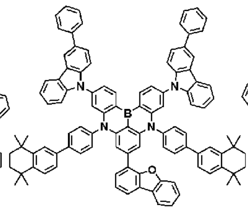
539
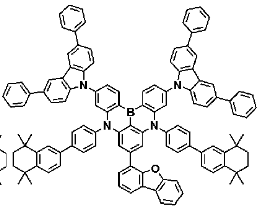
540

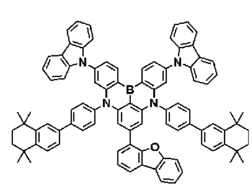
541
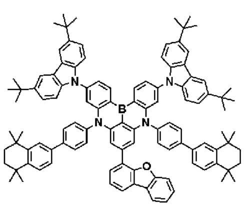
542
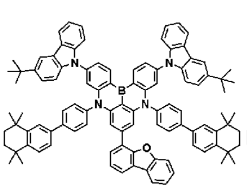
543
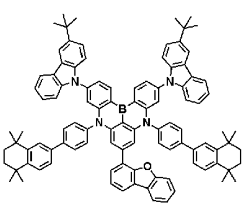
544
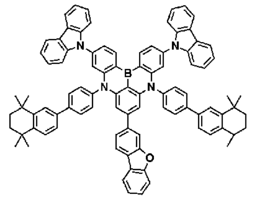
545

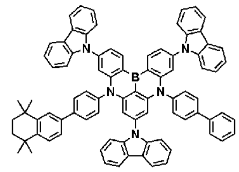
546
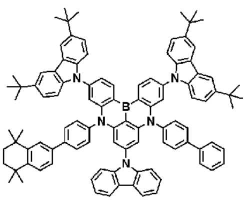
547
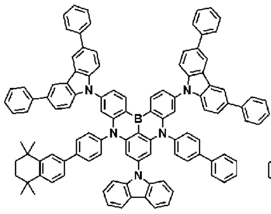
548
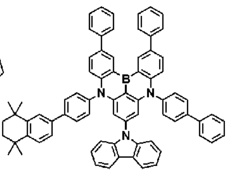
549
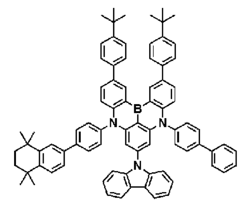
550

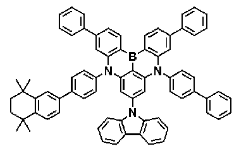
551
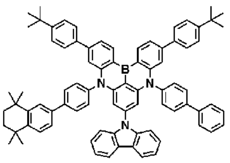
552
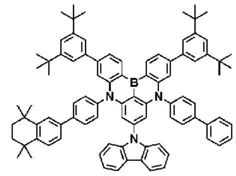
553
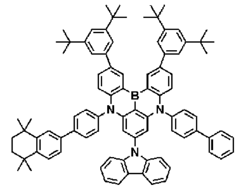
554
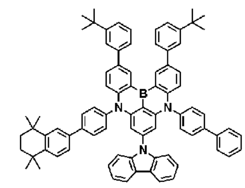
555

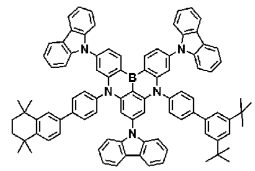
556
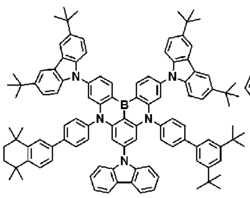
557
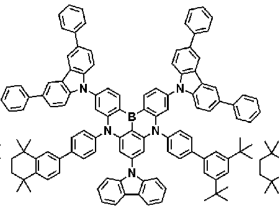
558
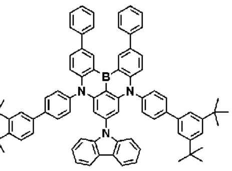
559
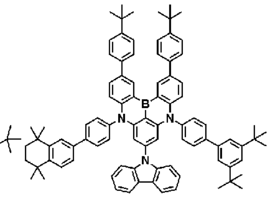
560

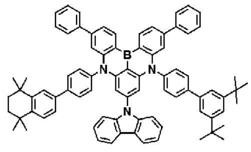
561
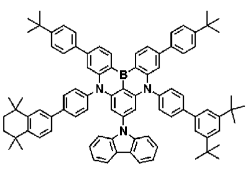
562
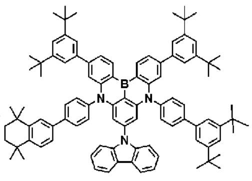
563
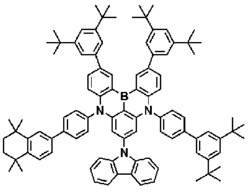
564
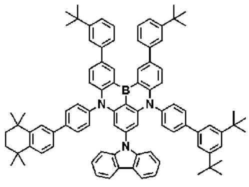
565

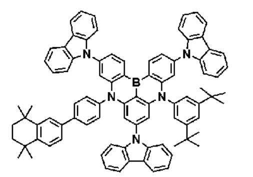
566
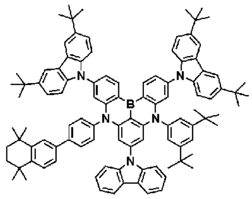
567
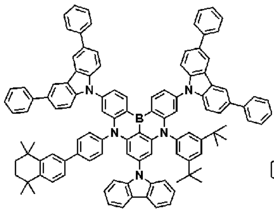
568
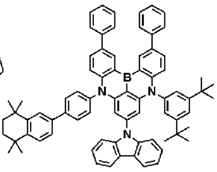
569
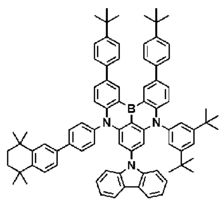
570

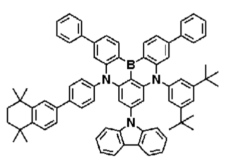
571
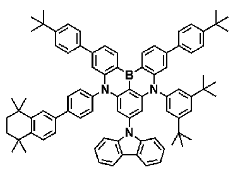
572
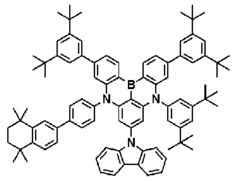
573
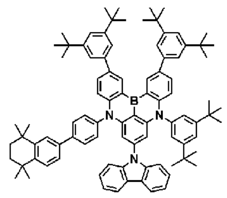
574
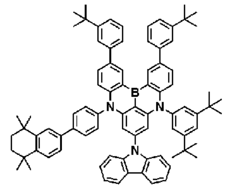
575

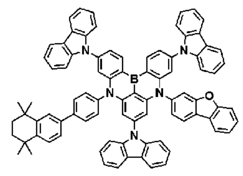
576
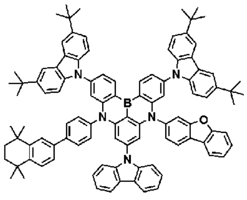
577
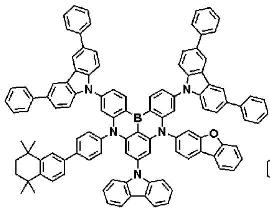
578
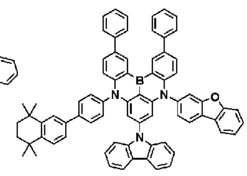
579
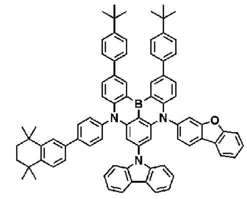
580

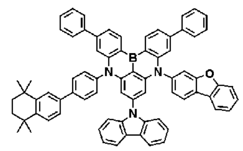
581
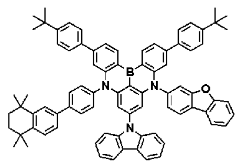
582
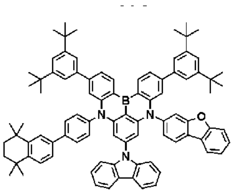
583
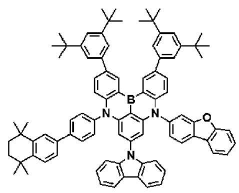
584
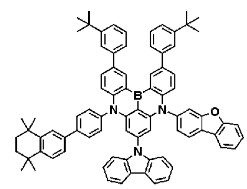
585

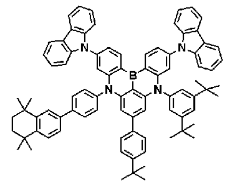
586
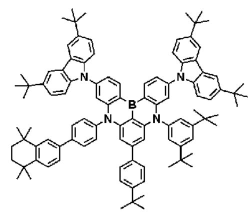
587
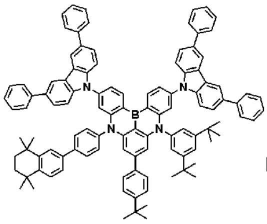
588
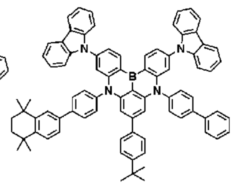
589
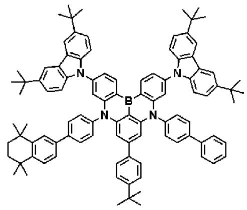
590

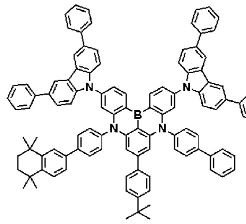
591
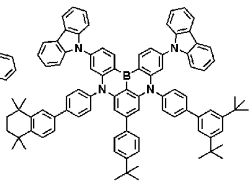
592
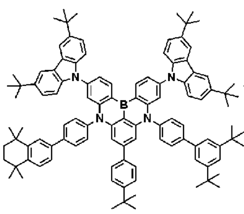
593
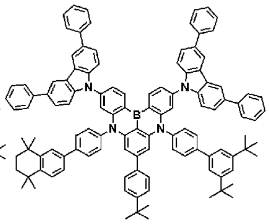
594
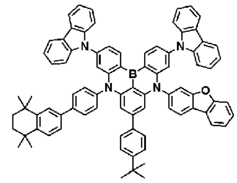
595

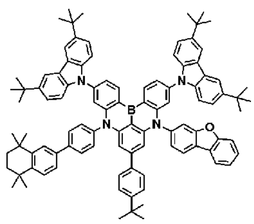
596
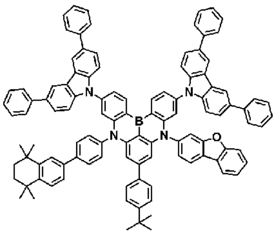
597
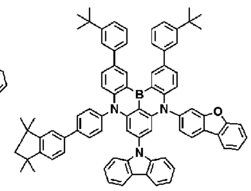
598

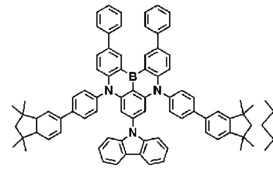
599
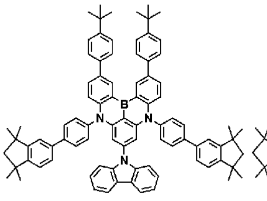
600
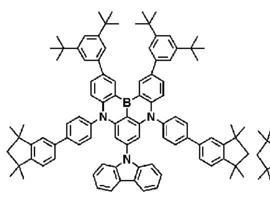
601
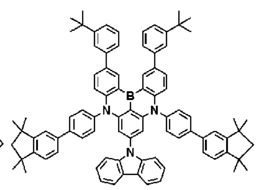
602
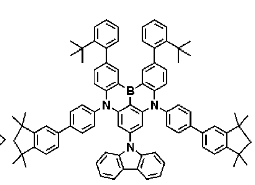
603

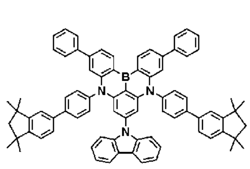
604
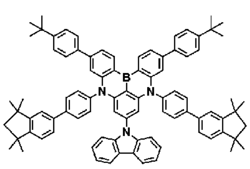
605
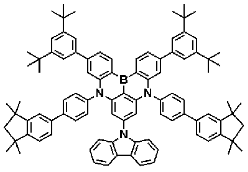
606
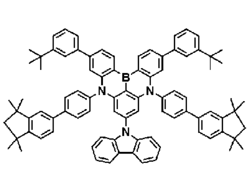
607
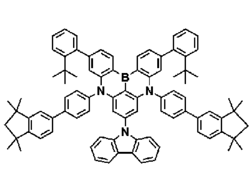
608

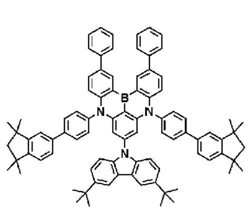
609
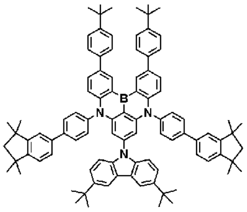
610
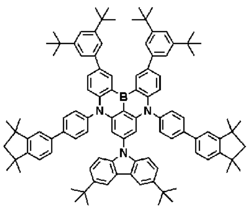
611
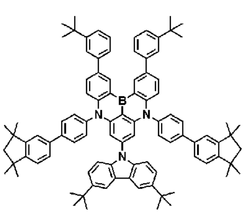
612
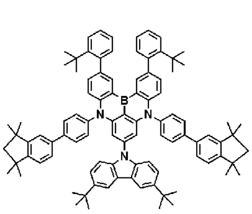
613

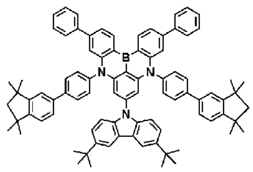
614
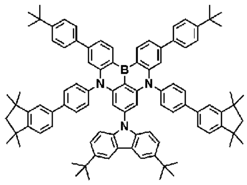
615
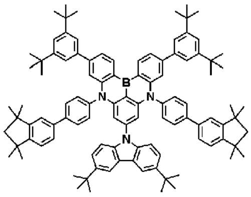
616
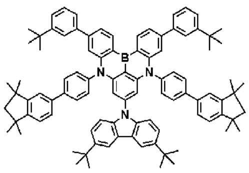
617
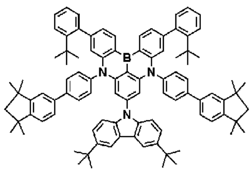
618

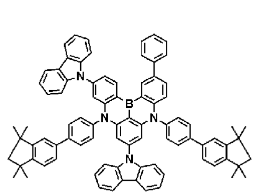
619
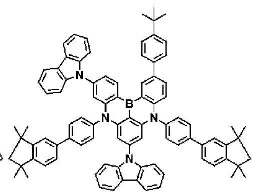
620
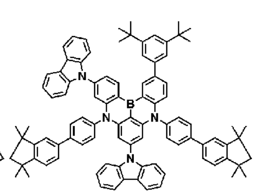
621
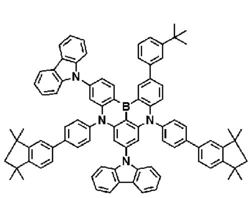
622
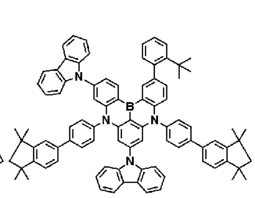
623

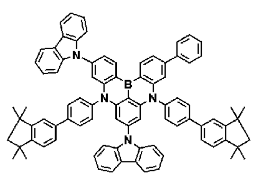
624
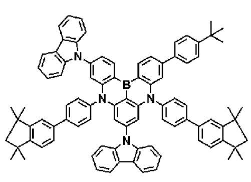
625
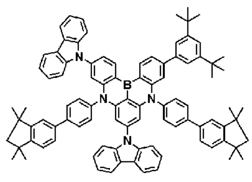
626
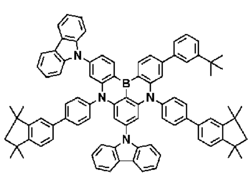
627
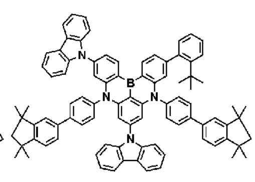
628

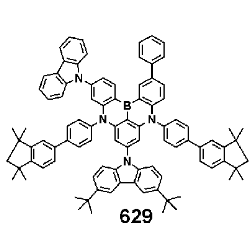
629
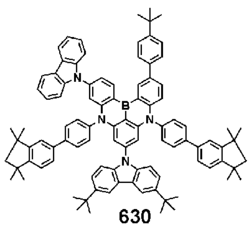
630
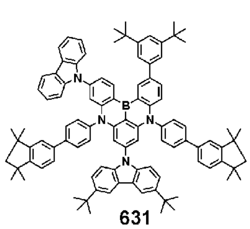
631
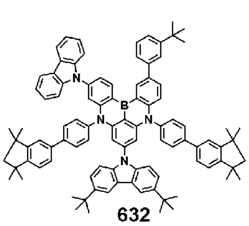
632
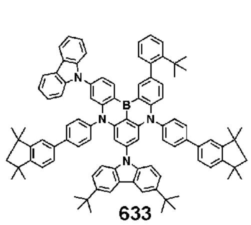
633

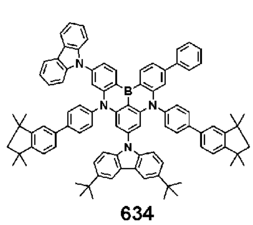
634
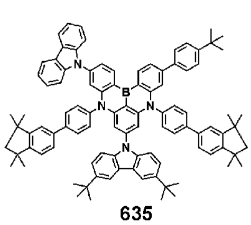
635
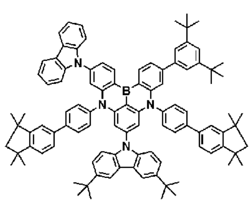
636
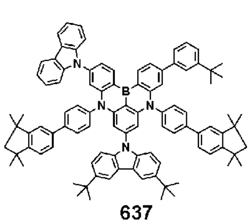
637
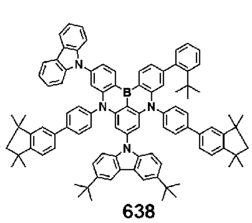
638

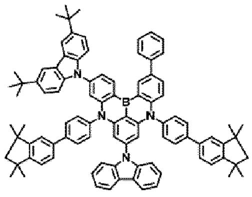
639

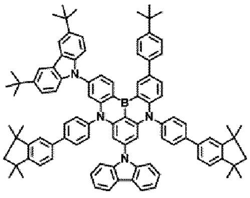
640

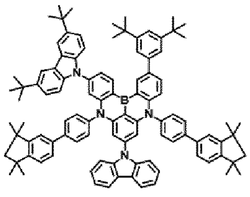
641

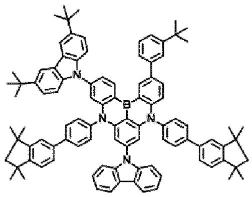
642

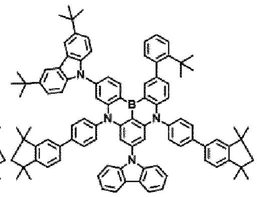
643

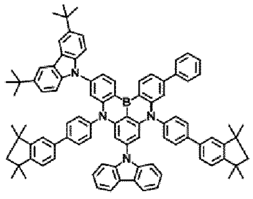
644

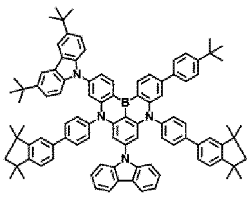
645

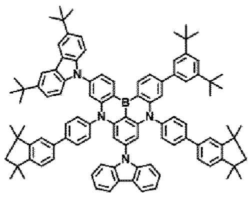
646

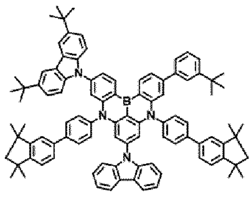
647

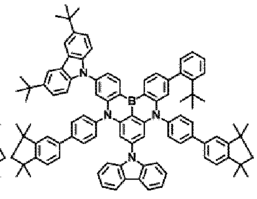
648

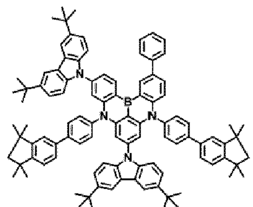
649

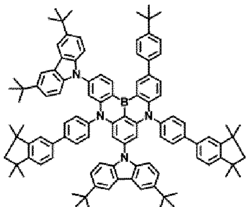
650

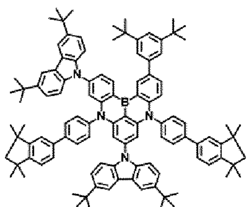
651

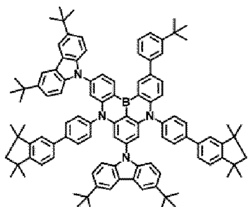
652

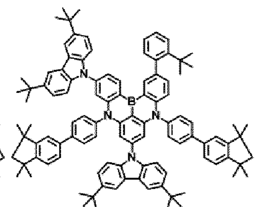
653

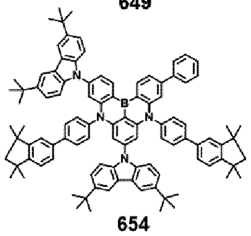
654

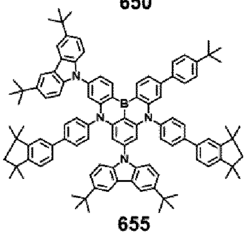
655

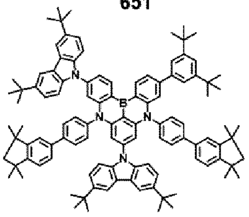
656

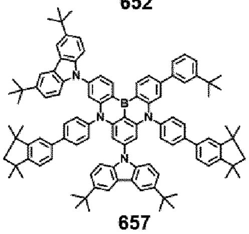
657

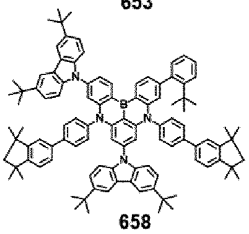
658

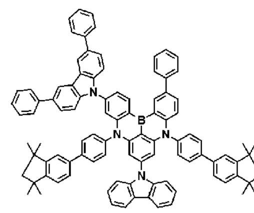
659

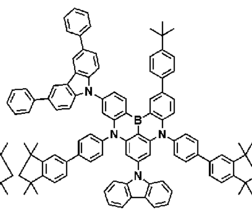
660

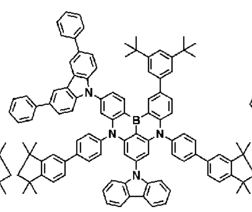
661

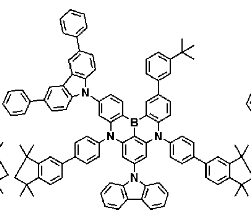
662

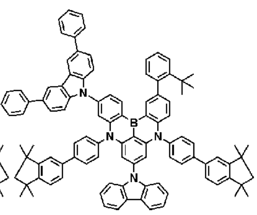
663

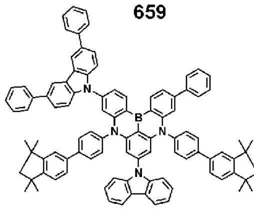
664

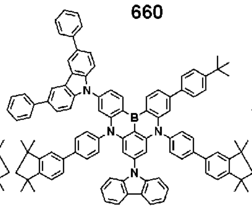
665

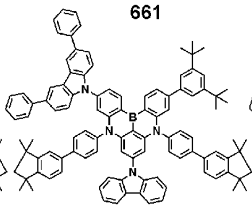
666

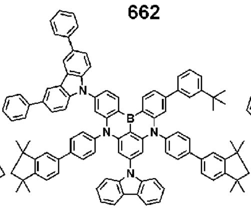
667

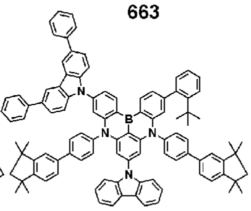
668

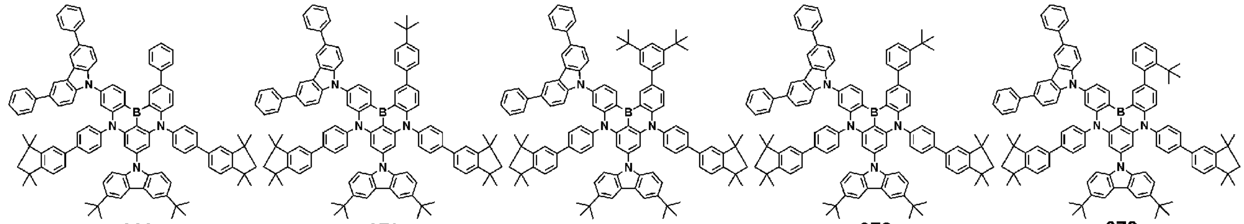

669 670 671 672 673

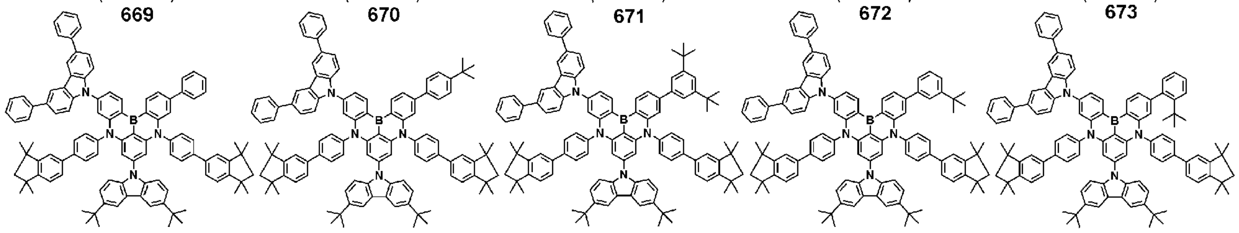

674 675 676 677 678

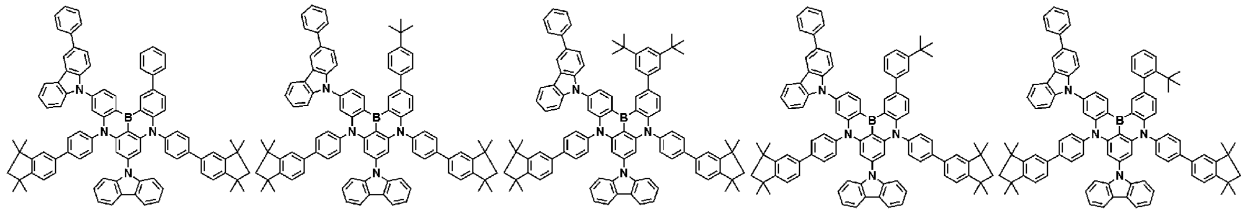

679 680 681 682 683

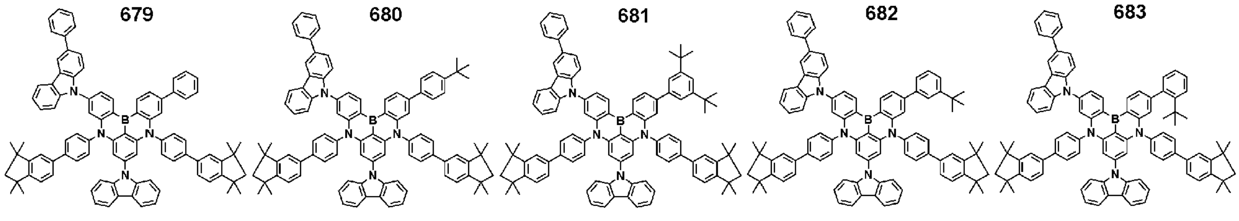

684 685 686 687 688

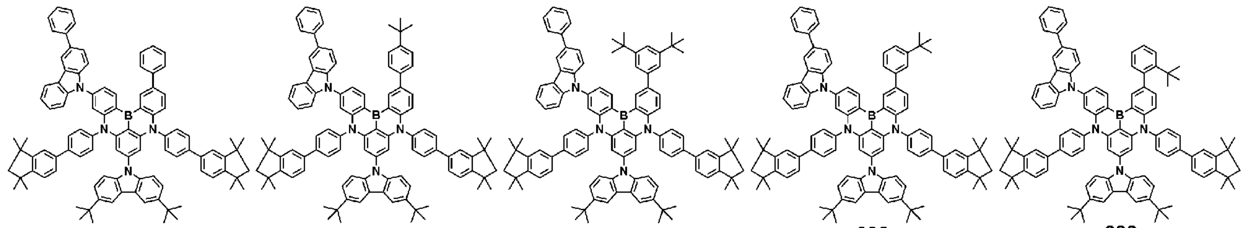

689 690 691 692 693

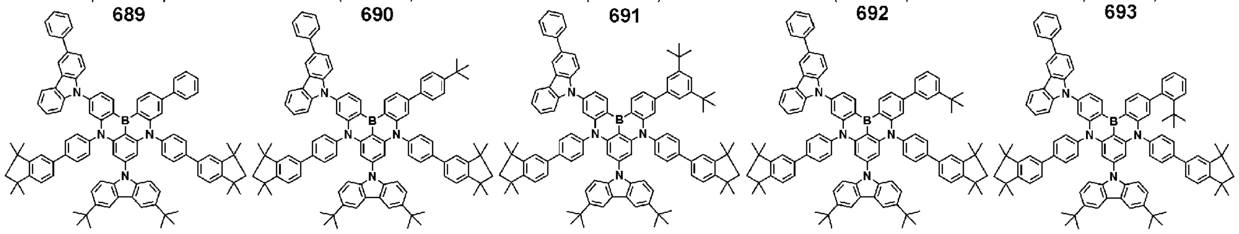

694 695 696 697 698

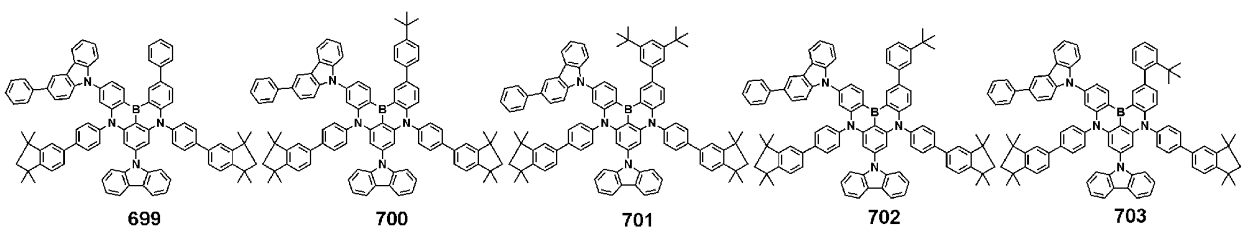

699 700 701 702 703

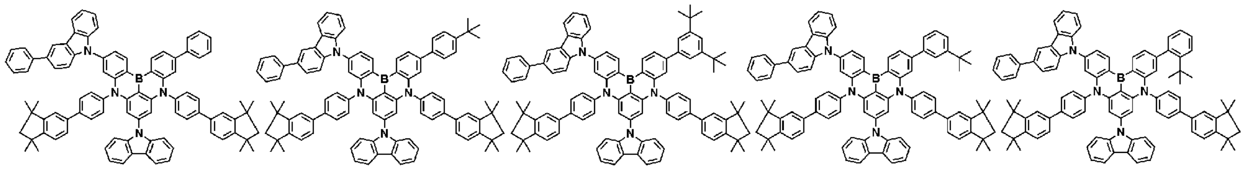

704 705 706 707 708

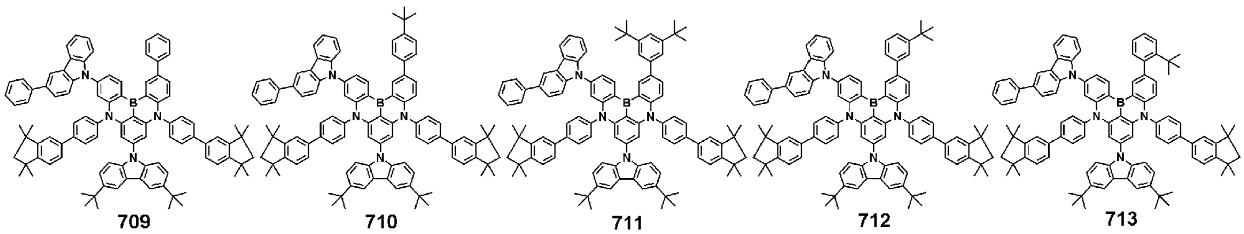

709 710 711 712 713

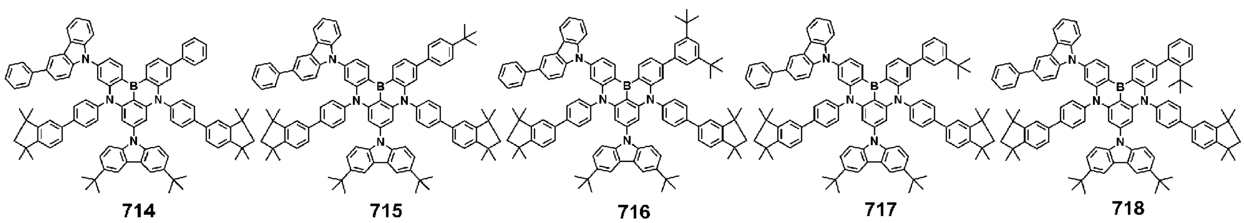

714 715 716 717 718

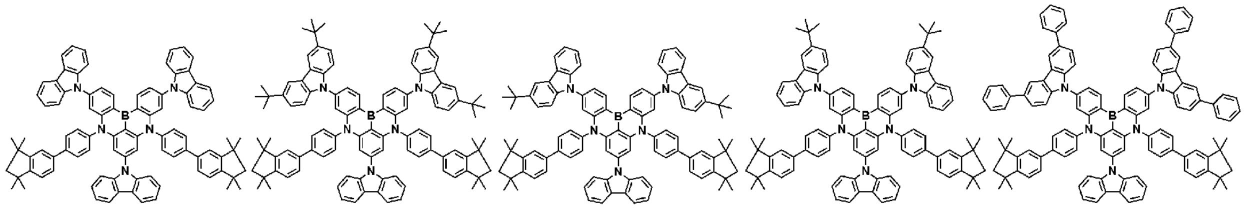

719 720 721 722 723

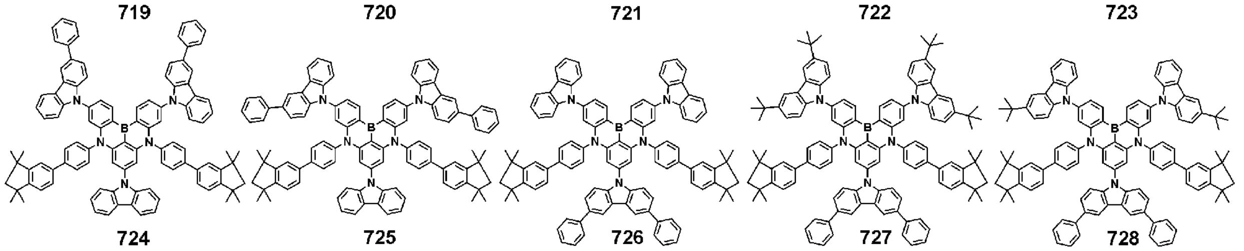

724 725 726 727 728

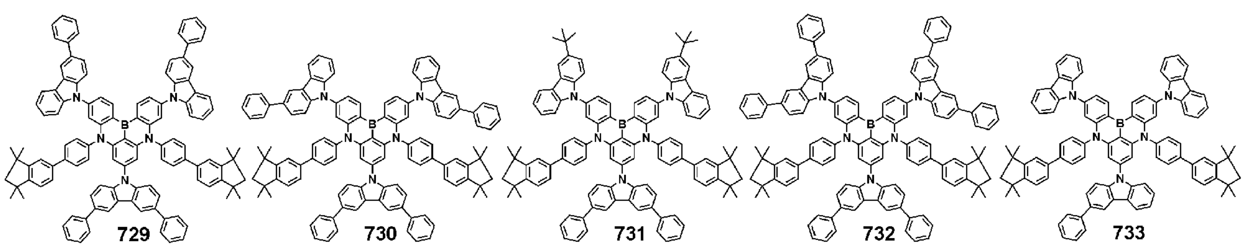

729 730 731 732 733

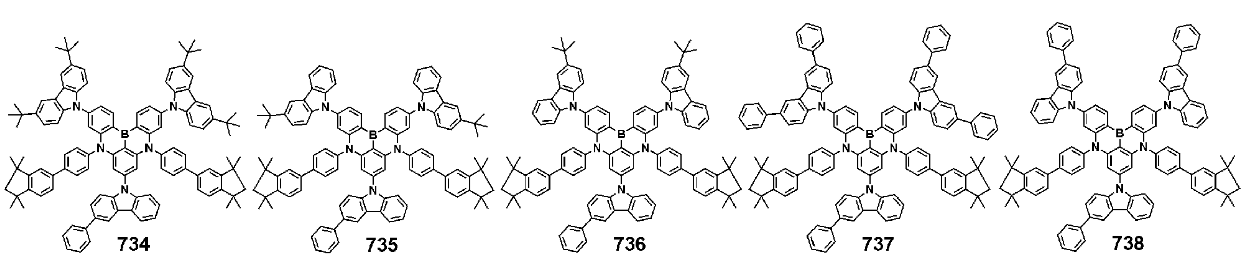

734 735 736 737 738

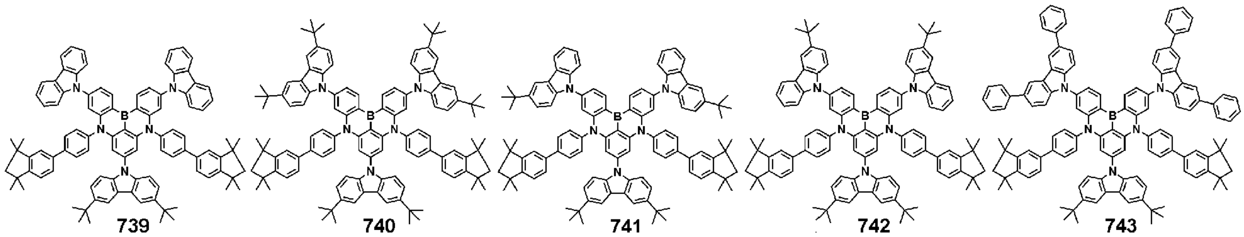

739 740 741 742 743

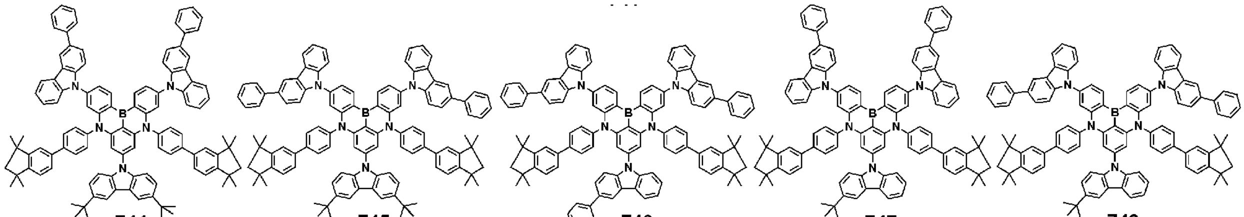

744 745 746 747 748

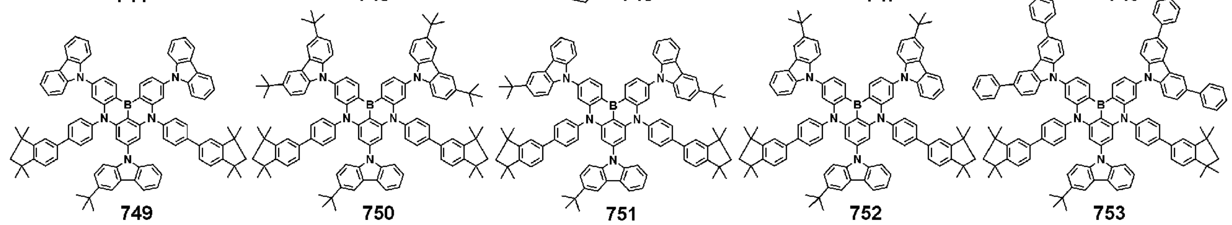

749 750 751 752 753

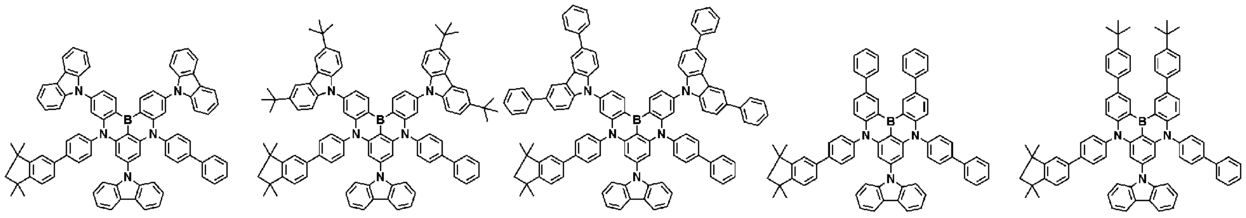

754 755 756 757 758

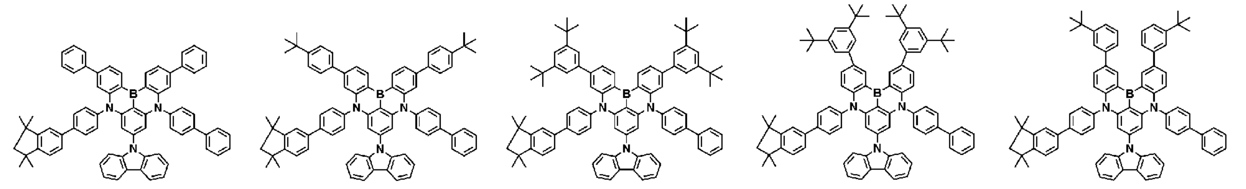

759 760 761 762 763

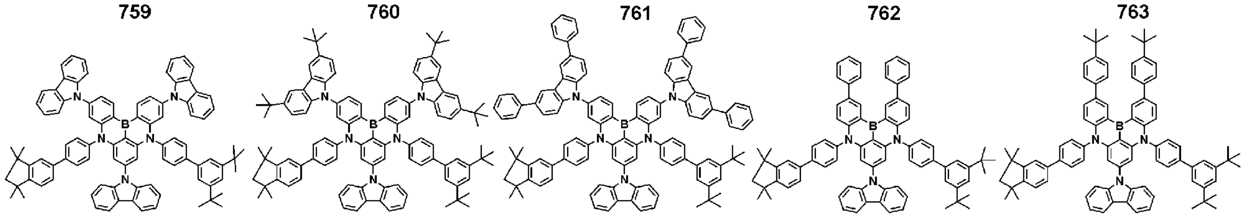

764 765 766 767 768

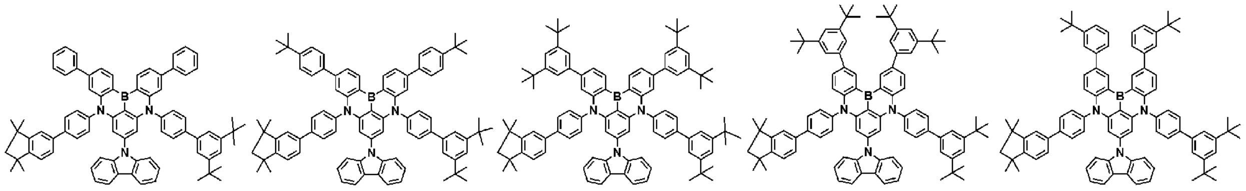

769 770 771 772 773

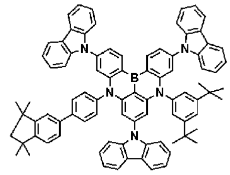
774

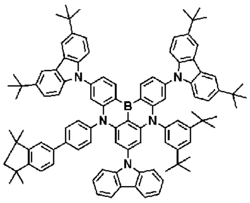
775

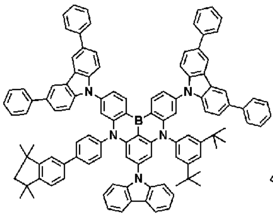
776

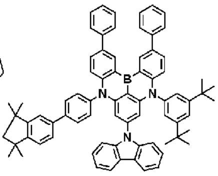
777

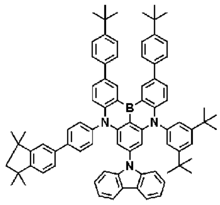
778

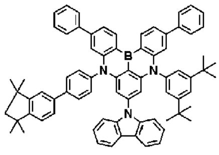
779

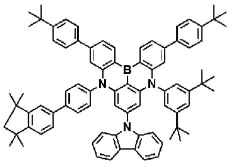
780

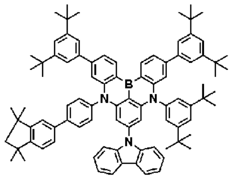
781

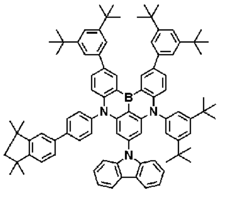
782

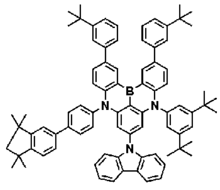
783

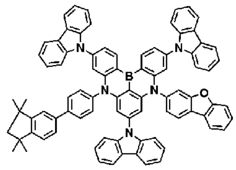
784

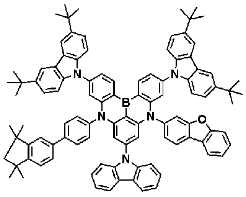
785

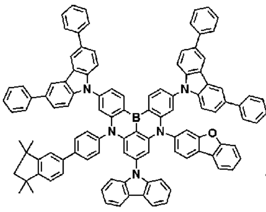
786

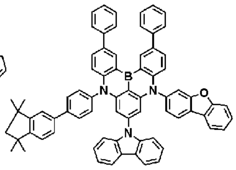
787

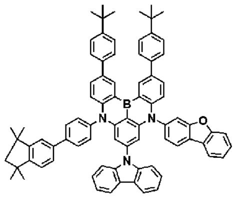
788

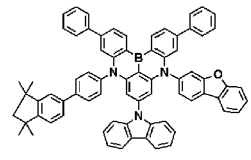
789

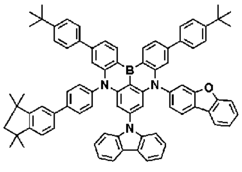
790

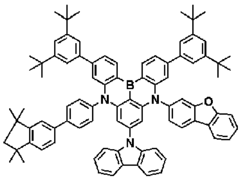
791

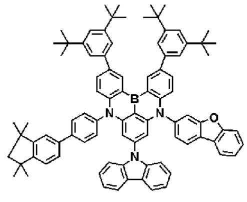
792

The heterocyclic compound represented by Formula 1 satisfies the structure of Formula 1 described above, and includes a structure in which a group represented by Formula 2 is substituted in ring $CY_4$ of Formula 1. Due to this structure, the heterocyclic compound represented by Formula 1 has excellent luminescent characteristics, and in particular, may implement deep blue light emission having a short wavelength.

Although not limited by a particular theory, the heterocyclic compound that satisfies the structure as described above may have high oscillator strength (f), and thus, when the heterocyclic compound is applied to an electronic device, efficiency of the electronic device may increase. In addition, since the heterocyclic compound includes a substituent having a bulky structure represented by Formula 2 as described above, the heterocyclic compound may have a high $S_1$ energy level and a high photoalignment. Accordingly, when the heterocyclic compound is used as a luminescent material, Dexter energy transfer is suppressed and excellent lifespan characteristics may be exhibited.

Therefore, an electronic device, for example, an organic light-emitting device, including the heterocyclic compound represented by Formula 1 may have high efficiency and long lifespan.

The highest occupied molecular orbital (HOMO) energy level, lowest unoccupied molecular orbital (LUMO) energy level, $S_1$ energy level, $T_1$ energy level, and oscillator strength (f) of some of the heterocyclic compounds represented by Formula 1 were evaluated using the Gaussian 09 program with the molecular structure optimization obtained by B3LYP-based density functional theory (DFT), and results thereof are shown in Table 1.

Table 1

| | HOMO (eV) | LUMO (eV) | $S_1$ (eV) | $T_1$ (eV) | f |
|---|---|---|---|---|---|
| Compound 1 | -4.873 | -1.351 | 2.684 | 2.541 | 0.588 |
| Compound A | -4.754 | -1.092 | 2.737 | 2.567 | 0.309 |

(continued)

| | HOMO (eV) | LUMO (eV) | $S_1$ (eV) | $T_1$ (eV) | f |
|---|---|---|---|---|---|
| Compound B | -4.642 | -0.997 | 2.678 | 2.546 | 0.334 |
| Compound C | -4.70 | -1.12 | 2.74 | 2.61 | 0.355 |
| Compound D | -4.66 | -1.05 | 2.76 | 2.64 | 0.335 |

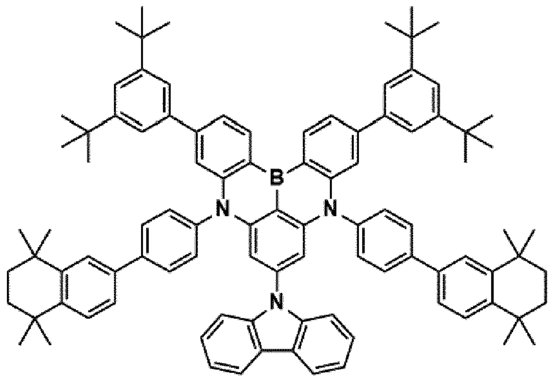

1

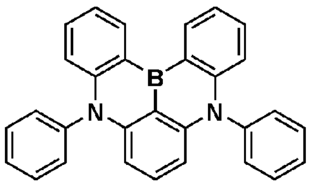

A

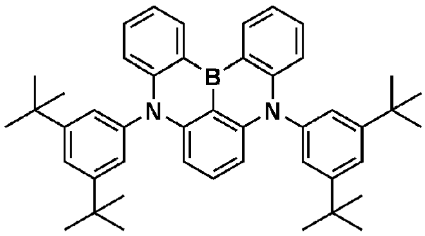

B

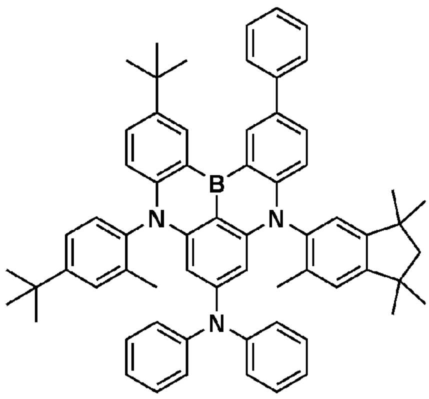

C

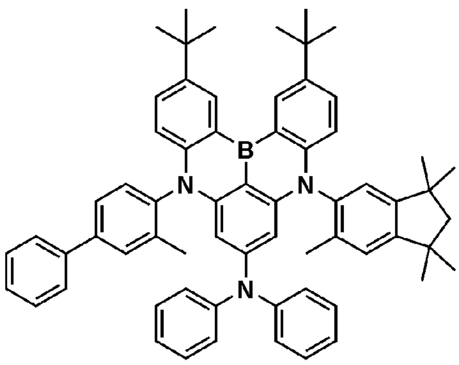

D

From the Table 1, it may be seen that the heterocyclic compound represented by Formula 1 has electric characteristics suitable for use as a dopant (for example, an emitter or a sensitizer) in an electronic device, for example, an organic light-emitting device. For example, the heterocyclic compound has a deep HOMO value and a significantly high f value, as compared to Compounds A to D as comparative compounds.

In an embodiment, a full width at half maximum (FWHM) of an emission peak of an emission spectrum or an electroluminescence (EL) spectrum of the heterocyclic compound may be 60 nm or less. For example, an FWHM of an emission peak of an emission spectrum or an EL spectrum of the heterocyclic compound may be 5 nm to 50 nm, 7 nm to 40 nm, or 10 nm to 30 nm.

A synthesis method of the heterocyclic compound represented by Formula 1 may be recognized by those skilled

in the art with reference to the following Synthesis Examples.

A method of identifying a structure of the heterocyclic compound represented by Formula 1 is not particularly limited. In an embodiment, the structure of the heterocyclic compound may be identified by a known method (for example, nuclear magnetic resonance (NMR) or liquid chromatography-mass spectrometry (LC-MS)).

[Organic light-emitting device]

According to one or more embodiments, an organic light-emitting device includes the heterocyclic compound.

In an embodiment, the organic light-emitting device may include: a first electrode; a second electrode; and an organic layer arranged between the first electrode and the second electrode and including an emission layer, and the organic layer may include the heterocyclic compound represented by Formula 1.

In an embodiment, the emission layer may include the heterocyclic compound.

In an embodiment, the emission layer may include a host and an emitter, and the emitter may include the heterocyclic compound.

In an embodiment, in the emission layer, an amount of the host may be greater than an amount of the heterocyclic compound based on the weight.

In an embodiment, the emission layer may further include a sensitizer.

In an embodiment, the sensitizer may include a phosphorescent compound, a delayed fluorescence compound, or any combination thereof.

The host, the emitter, and the sensitizer are each the same as described in the present specification.

Since the organic light-emitting device includes the emission layer including the heterocyclic compound represented by Formula 1 as described above, the organic light-emitting device may have a relatively narrow FWHM of an emission peak of an EL spectrum, excellent efficiency, and excellent lifespan characteristics.

In an embodiment, the heterocyclic compound may act as a dopant (for example, an emitter or a sensitizer) in the emission layer, and the emission layer may further include a host (in other words, in the emission layer, an amount of the heterocyclic compound represented by Formula 1 may be less than an amount of the host).

In an embodiment, the emission layer may emit blue light. For example, the emission layer may emit blue light having a maximum emission wavelength of about 410 nm to about 490 nm.

The wording "(emission layer) includes at least one heterocyclic compound" as used herein may be to mean that the (emission layer) may include one kind of heterocyclic compound represented by Formula 1 or two or more different kinds of heterocyclic compounds, each represented by Formula 1.

For example, the emission layer may include, as the heterocyclic compound, only Compound 1. In this regard, Compound 1 may be included in the emission layer of the organic light-emitting device. In an embodiment, the emission layer may include, as the heterocyclic compound, Compound 1 and Compound 2.

[Description of FIG. 1]

FIG. 1 is a schematic cross-sectional view of an organic light-emitting device 10 according to an embodiment. Hereinafter, referring to FIG. 1, a structure and manufacturing method of an organic light-emitting device according to an embodiment are described as follows.

In FIG. 1, the organic light-emitting device 10 includes a first electrode 11, a second electrode 19 facing the first electrode 11, and an organic layer 15 between the first electrode 11 and the second electrode 19.

The organic layer 15 includes an emission layer, a hole transport region may be arranged between the first electrode 11 and the emission layer, and an electron transport region may be arranged between the emission layer and the second electrode 19.

A substrate may be additionally disposed under the first electrode 11 or on the second electrode 19. The substrate may be a conventional substrate used in organic light-emitting devices, e.g., a glass substrate or a transparent plastic substrate, each having excellent mechanical strength, thermal stability, transparency, surface smoothness, ease of handling, and water repellency.

[First electrode 11]

The first electrode 11 may be produced by depositing or sputtering, onto the substrate, a material for forming the first electrode 11. The first electrode 11 may be an anode. The material for forming the first electrode 11 may be selected from materials with a high work function for easy hole injection.

The first electrode 11 may be a reflective electrode, a semi-transmissive electrode, or a transmissive electrode. When the first electrode 11 is a transmissive electrode, a material for forming the first electrode 11 may be indium tin oxide (ITO), indium zinc oxide (IZO), tin oxide ($SnO_2$), zinc oxide (ZnO), or any combination thereof, but embodiments are not

limited thereto. In some embodiments, when the first electrode 11 is a semi-transmissive electrode or a reflective electrode, as a material for forming the first electrode 11, at least one of magnesium (Mg), silver (Ag), aluminum (Al), aluminum-lithium (Al-Li), calcium (Ca), magnesium-indium (Mg-In), magnesium-silver (Mg-Ag), or any combination thereof may be used, but embodiments are not limited thereto.

The first electrode 11 may have a single-layered structure or a multilayer structure including a plurality of layers.

[Emission layer]

The emission layer may include the heterocyclic compound.

A thickness of the emission layer may be in a range of about 100 Å to about 1,000 Å, for example, about 200 Å to about 600 Å. When the thickness of the emission layer is within the range, excellent light-emission characteristics may be obtained without a substantial increase in driving voltage.

[Description of FIG. 2]

In an embodiment, the heterocyclic compound may be a fluorescent emitter.

In an embodiment, the emission layer may further include a host (hereinafter, referred to as 'Host A', and Host A may not be identical to the heterocyclic compound). Host A may be understood by referring to the description of a host material described below, but embodiments are not limited thereto. Host A may be a fluorescent host.

Referring to FIG. 2, energy transfer according to an embodiment is described as follows.

Singlet excitons are formed in Host A in the emission layer, and the singlet excitons formed in Host A are transferred to a fluorescent emitter through Förster energy transfer (or, Förster resonance energy transfer (FRET)).

A proportion of the singlet excitons formed in Host A may be only 25 %, and thus, 75 % of triplet excitons formed in Host A may be fused to each other to be converted into singlet excitons. Thus, efficiency of the organic light-emitting device may be further improved. In other words, the efficiency of the organic light-emitting device may be further improved using a triplet-triplet fusion (TTF) mechanism.

In an embodiment, among total emission components emitted from the emission layer, a proportion of emission components emitted from the heterocyclic compound may be greater than or equal to 80 %, for example, greater than or equal to 90 %. For example, among total emission components emitted from the emission layer, a proportion of emission components emitted from the heterocyclic compound may be greater than or equal to 95 %.

The heterocyclic compound may emit fluorescence, and the host may not emit light.

In an embodiment, when the emission layer further includes Host A, in addition to the heterocyclic compound, an amount of the heterocyclic compound may be about 50 parts by weight or less, for example, about 30 parts by weight or less, about 10 parts by weight or less, or about 1 part by weight to about 5 parts by weight, based on 100 parts by weight of the emission layer, and an amount of Host A in the emission layer may be about 50 parts by weight or greater, for example, about 70 parts by weight or greater, about 90 parts by weight or greater, or about 95 part by weight to about 99 parts by weight, based on 100 parts by weight of the emission layer, but embodiments are not limited thereto.

In an embodiment, when the emission layer further includes Host A, in addition to the heterocyclic compound, Host A and the heterocyclic compound may satisfy Condition A:

Condition A

$$E(H_A)_{S1} > E_{S1}$$

wherein, in Condition A,

$E(H_A)_{S1}$ indicates a lowest excited singlet energy level of Host A, and
$E_{S1}$ indicates a lowest excited singlet energy level of the heterocyclic compound.

Here, $E(H_A)_{S1}$ and $E_{S1}$ are evaluated using the DFT method of the Gaussian program, wherein structure optimization is performed at B3LYP/6-31G(d,p) level.

[Description For FIG. 3]

In an embodiment, the heterocyclic compound may be a delayed fluorescence emitter.

In an embodiment, the emission layer may further include a host (hereinafter, referred to as 'Host B', and Host B is not identical to the heterocyclic compound). Host B may be understood by referring to the description of a host material described below, but embodiments are not limited thereto.

Referring to FIG. 3, energy transfer according to an embodiment is described as follows.

25 % of singlet excitons formed in Host B in the emission layer are transferred to a delayed fluorescence emitter through FRET. In addition, 75 % of triplet excitons formed in Host B in the emission layer are transferred to a delayed fluorescence emitter through Dexter energy transfer. At least a portion of energy of a singlet state of the delayed fluorescence emitter may be transferred to a triplet state by intersystem crossing (ISC). The energy transferred to the triplet state of the delayed fluorescence emitter may be transferred to the singlet state by reverse intersystem crossing (RISC). Accordingly, by transferring all the singlet excitons and triplet excitons generated in the emission layer to the heterocyclic compound, the organic light-emitting device having improved efficiency may be obtained.

Therefore, in an embodiment, among total emission components emitted from the emission layer, a proportion of emission components emitted from the heterocyclic compound may be greater than or equal to 80 %, for example, greater than or equal to 90 %. For example, among total emission components emitted from the emission layer, a proportion of emission components emitted from the heterocyclic compound may be greater than or equal to 95 %.

Here, the heterocyclic compound may emit fluorescence and/or delayed fluorescence, and the emission components of the heterocyclic compound may be a total of prompt emission components of the heterocyclic compound and delayed fluorescence components by RISC of the heterocyclic compound. In addition, Host B may not emit light.

In an embodiment, when the emission layer further includes Host B, in addition to the heterocyclic compound, an amount of the heterocyclic compound may be about 50 parts by weight or less, for example, about 30 parts by weight or less, based on 100 parts by weight of the emission layer, and an amount of Host B in the emission layer may be about 50 parts by weight or greater, for example, about 70 parts by weight or greater, based on 100 parts by weight of the emission layer, but embodiments are not limited thereto.

In an embodiment, when the emission layer further includes Host B, in addition to the heterocyclic compound, Host B and the heterocyclic compound may satisfy Condition B:

Condition B

$$E(H_B)_{S1} > E_{S1}$$

wherein, in Condition B,

$E(H_B)_{S1}$ indicates a lowest excited singlet energy level of Host B, and
$E_{S1}$ indicates a lowest excited singlet energy level of the heterocyclic compound.

Here, $E(H_B)_{S1}$ and $E_{S1}$ are evaluated using the DFT method of the Gaussian program, wherein structure optimization is performed at B3LYP/6-31G(d,p) level.

[Description of FIG. 4]

In an embodiment, the heterocyclic compound may be used as a fluorescent emitter, and the emission layer may further include a sensitizer, for example, a delayed fluorescence sensitizer. In an embodiment, the emission layer may further include a host (hereinafter, referred to as 'Host C', and Host C is not identical to the heterocyclic compound and the sensitizer) and a sensitizer (hereinafter, referred to as 'Sensitizer A', and Sensitizer A is not identical to Host C and the heterocyclic compound). Host C and Sensitizer A may respectively be understood by referring to the descriptions of a host material and a sensitizer material described below, but embodiments are not limited thereto.

In an embodiment, among total emission components emitted from the emission layer, a proportion of emission components of the heterocyclic compound may be greater than or equal to 80 %, for example, greater than or equal to 90 % (or for example, greater than or equal to 95 %). For example, the heterocyclic compound may emit fluorescence. In addition, Host C and Sensitizer A may not each emit light.

Referring to FIG. 4, energy transfer according to an embodiment is described as follows.

Singlet and triplet excitons are formed in Host C in the emission layer, and the singlet and triplet excitons formed in Host C may be transferred to Sensitizer A and then to the heterocyclic compound through FRET. 25 % of the singlet excitons are formed in Host C and are transferred to Sensitizer A through FRET, and 75 % of the triplet excitons are formed in Host C and energy thereof is transferred to a singlet state and triplet state of Sensitizer A. At least a portion of energy of the singlet state of Sensitizer A may be transferred to the triplet state by ISC. The energy transferred to the triplet state of Sensitizer A is transferred to the singlet state by RISC, and then, the singlet state energy of Sensitizer A is transferred to the heterocyclic compound through FRET.

Accordingly, by transferring all the singlet excitons and triplet excitons generated in the emission layer to a dopant (for example, an emitter), the organic light-emitting device having improved efficiency may be obtained. In addition, since the organic light-emitting device with significantly reduced energy loss may be obtained, lifespan characteristics of the

organic light-emitting device may be improved.

Referring to FIG. 4, when the emission layer further includes Host C and Sensitizer A, in addition to the heterocyclic compound, Host C, Sensitizer A and the heterocyclic compound may satisfy Condition C-1 and/or C-2:

Condition C-1

$$S_1(H_C) \geq S_1(S_A)$$

Condition C-2

$$S_1(S_A) \geq S_1(HC)$$

wherein, in Conditions C-1 and C-2,

$S_1(H_C)$ indicates a lowest excited singlet energy level of Host C,
$S_1(S_A)$ indicates a lowest excited singlet energy level of Sensitizer A, and
$S_1(HC)$ indicates a lowest excited singlet energy level of the heterocyclic compound.

$S_1(H_C)$, $S_1(S_A)$, and $S_1(HC)$ are evaluated using the DFT method of the Gaussian program, wherein structure optimization is performed at B3LYP/6-31G(d,p) level.

When Host C, Sensitizer A, and the heterocyclic compound satisfy Condition C-1 and/or C-2, FRET from Sensitizer A to the heterocyclic compound may be facilitated, and accordingly, the organic light-emitting device may have improved emission efficiency.

[Description of FIG. 5]

In an embodiment, the heterocyclic compound may be used as a fluorescent emitter, and the emission layer may further include a sensitizer, for example, a phosphorescent sensitizer.

In an embodiment, the emission layer may further include a host (hereinafter, referred to as 'Host D', and Host D is not identical to the heterocyclic compound and the sensitizer) and a sensitizer (hereinafter, referred to as 'Sensitizer B', and Sensitizer B is not identical to Host D and the heterocyclic compound). Host D and Sensitizer B may respectively be understood by referring to the descriptions of a host material and a sensitizer material described below, but embodiments are not limited thereto.

In an embodiment, among total emission components emitted from the emission layer, a proportion of emission components of the heterocyclic compound may be greater than or equal to 80 %, for example, greater than or equal to 90 % (or for example, greater than or equal to 95 %). For example, the heterocyclic compound may emit fluorescence. In addition, Host D and Sensitizer B may not each emit light.

Referring to FIG. 5, energy transfer according to an embodiment is described as follows.

75 % of triplet excitons are formed in Host D in the emission layer and are transferred to Sensitizer B through Dexter energy transfer, and 25 % of singlet excitons are formed in Host D and energy thereof is transferred to a singlet state and triplet state of Sensitizer B. The energy transferred to the singlet state of Sensitizer B is transferred to the triplet state by ISC, and then, the triplet energy of Sensitizer B is transferred to the heterocyclic compound through FRET.

Accordingly, by transferring all the singlet excitons and triplet excitons generated in the emission layer to a dopant (for example, an emitter), the organic light-emitting device having improved efficiency may be obtained. In addition, since the organic light-emitting device with significantly reduced energy loss may be obtained, lifespan characteristics of the organic light-emitting device may be improved.

In an embodiment, when the emission layer further includes Host D and Sensitizer B, in addition to the heterocyclic compound, Host D, Sensitizer B, and the heterocyclic compound may satisfy Condition D-1 and/or D-2:

Condition D-1

$$T_1(H_D) \geq T_1(S_B)$$

Condition D-2

$$T_1(S_B) \geq S_1(HC)$$

wherein, in Conditions D-1 and D-2,

$T_1(H_D)$ indicates a lowest excited triplet energy level of Host D,
$T_1(S_B)$ indicates a lowest excited triplet energy level of Sensitizer B, and
$S_1(HC)$ indicates a lowest excited singlet energy level of the heterocyclic compound.

$T_1(H_D)$, $T_1(S_B)$, and $S_1(HC)$ are evaluated using the DFT method of the Gaussian program, wherein structure optimization is performed at B3LYP/6-31G(d,p) level.
When Host D, Sensitizer B, and the heterocyclic compound satisfy Condition D-1 and/or D-2, FRET from Sensitizer B to the heterocyclic compound may be facilitated, and accordingly, the organic light-emitting device may have improved emission efficiency.
In an embodiment, an amount of Sensitizer B in the emission layer may be about 5 wt% to about 50 wt%, for example, about 10 wt% to about 30 wt%. When the amount is within the range described above, effective energy transfer in the emission layer may be achieved. Thus, the organic light-emitting device may have high efficiency and long lifespan.
In an embodiment, an amount of the heterocyclic compound in the emission layer may be about 0.01 wt% to about 15 wt%, for example, about 0.05 wt% to about 3 wt%, but embodiments are not limited thereto.
In an embodiment, the heterocyclic compound may further satisfy Condition 5:

Condition 5

$$0\ \mu s < T_{decay}(HC) < 5\ \mu s$$

wherein, in Condition 5,
$T_{decay}(HC)$ indicates a decay time of the heterocyclic compound.
The decay time of the heterocyclic compound is a value calculated from a time-resolved photoluminescence (TRPL) spectrum at room temperature of a film (hereinafter, referred to as "Film (HC)") having a thickness of about 40 nm, which is obtained by vacuum-codepositing the host and the heterocyclic compound included in the emission layer on a quartz substrate at a weight ratio of 90:10 at a vacuum pressure of $10^{-7}$ torr.

[Description of FIG. 6]

In an embodiment, the heterocyclic compound may be used as a delayed fluorescence emitter, and the emission layer may further include a sensitizer, for example, a delayed fluorescence sensitizer.
In an embodiment, the emission layer may further include a host (hereinafter, referred to as 'Host E', and Host E is not identical to the heterocyclic compound and a sensitizer) and a sensitizer (hereinafter, referred to as 'Sensitizer C', and Sensitizer C is not identical to Host E and the heterocyclic compound). Host E and Sensitizer C may respectively be understood by referring to the description of a host material and a sensitizer material described below, but embodiments are not limited thereto.
In an embodiment, among total emission components emitted from the emission layer, a proportion of emission components of the heterocyclic compound may be greater than or equal to about 80 %, for example, greater than or equal to about 90 % (or for example, greater than or equal to about 95 %). For example, the heterocyclic compound may emit fluorescence and/or delayed fluorescence. In addition, Host E and Sensitizer C may not each emit light.
Here, the heterocyclic compound emits fluorescence and/or delayed fluorescence, and the emission components of the heterocyclic compound are a total of prompt emission components of the heterocyclic compound and delayed fluorescence components of the heterocyclic compound by RISC.
Referring to FIG. 6, energy transfer according to an embodiment is described as follows.
25 % of singlet excitons are formed in Host E in the emission layer and are transferred to a singlet of Sensitizer C through FRET, and 75 % of triplet excitons are formed in Host E and energy thereof is transferred to a triplet of Sensitizer C. Then, the singlet energy of Sensitizer C is transferred to the heterocyclic compound through FRET, and the triplet energy of Sensitizer C is transferred to the heterocyclic compound through Dexter energy transfer. The energy transferred to the triplet of Sensitizer C may be transferred to the singlet by RISC. In addition, in the case of Sensitizer C, the energy of the triplet state formed in Sensitizer C is reversely transferred to Host E (triplet exciton distributing (TED)), and then transferred to the heterocyclic compound to emit light through RISC.
Accordingly, by transferring all the singlet excitons and triplet excitons generated in the emission layer to a dopant (for example, an emitter), the organic light-emitting device having improved efficiency may be obtained. In addition, since the organic light-emitting device with significantly reduced energy loss is obtained, lifespan characteristics of the organic light-emitting device may be improved.
In an embodiment, when the emission layer further includes Host E and Sensitizer C, in addition to the

heterocyclic compound, Host E, Sensitizer C, and the heterocyclic compound may satisfy Condition E-1, E-2, and/or E-3:

Condition E-1

$$S_1(H_E) \geq S_1(S_C)$$

Condition E-2

$$S_1(S_C) \geq S_1(HC)$$

Condition E-3

$$T_1(S_C) \geq T_1(HC)$$

wherein, in Conditions E-1, E-2, and E-3,

$S_1(H_E)$ indicates a lowest excited singlet energy level of Host E,
$S_1(S_C)$ indicates a lowest excited singlet energy level of Sensitizer C,
$S_1(HC)$ indicates a lowest excited singlet energy level of the heterocyclic compound,

$T_1(Sc)$ indicates a lowest excited triplet energy level of Sensitizer C, and $T_1(HC)$ indicates a lowest excited triplet energy level of the heterocyclic compound.

$S_1(H_E)$, $S_1(S_C)$, $S_1(HC)$, $T_1(Sc)$, and $T_1(HC)$ are evaluated using the DFT method of the Gaussian program, wherein structure optimization is performed at B3LYP/6-31G(d,p) level.

When Host E, Sensitizer C, and the heterocyclic compound satisfy Condition E-1, E-2, and/or E-3, Dexter energy transfer and FRET from Sensitizer C to the heterocyclic compound may be facilitated, and accordingly, the organic light-emitting device may have improved emission efficiency.

In an embodiment, an amount of Sensitizer C in the emission layer may be about 5 wt% to about 50 wt%, for example, about 10 wt% to about 30 wt%. When the amount is within the range described above, effective energy transfer in the emission layer may be achieved. Thus, the organic light-emitting device may have high efficiency and long lifespan.

In an embodiment, an amount of the heterocyclic compound in the emission layer may be about 0.01 wt% to about 15 wt%, for example, about 0.05 wt% to about 3 wt%, but embodiments are not limited thereto.

[Host in emission layer]

In an embodiment, the host may not include a metal atom.

In an embodiment, the host may include at least one compound of a fluorene-containing compound, a carbazole-containing compound, a dibenzofuran-containing compound, a dibenzothiophene-containing compound, an indenocarbazole-containing compound, an indolocarbazole-containing compound, a benzofurocarbazole-containing compound, a benzothienocarbazole-containing compound, an acridine-containing compound, a dihydroacridine-containing compound, a triindolobenzene-containing compound, a pyridine-containing compound, a pyrimidine-containing compound, a triazine-containing compound, a silicon-containing compound, a cyano group-containing compound, a phosphine oxide-containing compound, a sulfoxide-containing compound, a sulfonyl-containing compound, or any combination thereof.

For example, the host may be a compound including at least one carbazole ring and at least one cyano group, or a phosphine oxide-containing compound.

In an embodiment, the host may consist of one kind of host. When the host consists of one kind of host, the one kind of host may be a bipolar host, an electron-transporting host, or a hole-transporting host, which will be described later.

In an embodiment, the host may be a mixture of two or more different kinds of hosts. For example, the host may be a mixture of an electron-transporting host and a hole-transporting host, a mixture of two different kinds of electron-transporting hosts, or a mixture of two different kinds of hole-transporting hosts. The electron-transporting host and the hole-transporting host may respectively be the same as those described below.

In an embodiment, the host may include an electron-transporting host including at least one electron-transporting moiety and a hole-transporting host that does not include an electron-transporting moiety.

The electron-transporting moiety used herein may be a cyano group, a $\pi$ electron-deficient nitrogen-containing cyclic group, a group represented by one of the following Formulae, or a combination thereof:

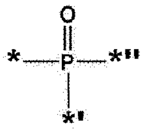 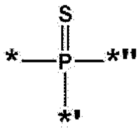 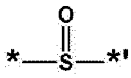 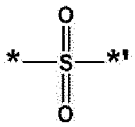

wherein, in Formulae, *, *', and *" may each indicate a binding site to any neighboring atom.

In an embodiment, the electron-transporting host in the emission layer may include at least one of a cyano group, a π electron-deficient nitrogen-containing cyclic group, or a combination thereof.

In an embodiment, the electron-transporting host in the emission layer may include at least one cyano group.

In an embodiment, the electron-transporting host in the emission layer may include at least one cyano group and at least one π electron-deficient nitrogen-containing cyclic group.

In an embodiment, the host may include an electron-transporting host and a hole-transporting host, wherein the electron-transporting host may include at least one π electron-deficient nitrogen-free cyclic group and at least one electron-transporting moiety, and the hole-transporting host may include at least one π electron-deficient nitrogen-free cyclic group and may not include an electron-transporting moiety.

The term "π electron-deficient nitrogen-containing cyclic group" used herein refers to a cyclic group having at least one *-N=*' moiety, and for example, may be selected from: an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and a condensed ring in which two or more π electron-deficient nitrogen-containing cyclic groups are condensed with each other.

Meanwhile, the π electron-deficient nitrogen-free cyclic group may be: a benzene group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a triindolobenzene group, a condensed ring in which two or more π electron-deficient nitrogen-free cyclic groups are condensed with each other, or any combination thereof, but embodiments are not limited thereto.

In an embodiment, when the host is a mixture of an electron-transporting host and a hole-transporting host, then then a weight ratio of the electron-transporting host and the hole-transporting host may be 1:9 to 9:1, for example, 2:8 to 8:2, for example, 4:6 to 6:4, for example, 5:5. When the weight ratio of the electron-transporting host and the hole-transporting host satisfies the range described above, then a balance of hole and electron transport in the emission layer may be achieved.

The host may include at least one of TPBi, TBADN, ADN (also referred to as "DNA"), CBP, CDBP, TCP, mCP, Compound H50, Compound H51, or any combination thereof:

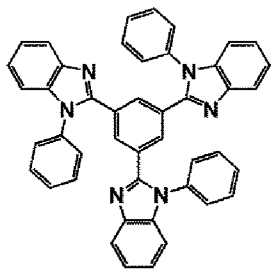

TPBi

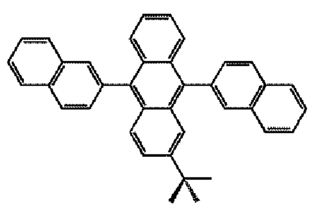

TBADN

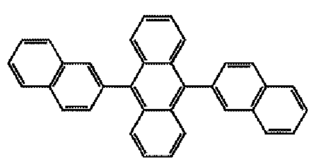

ADN

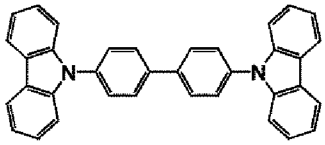

CBP

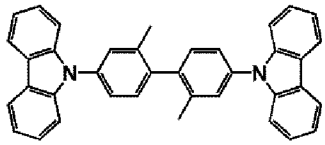

CDBP

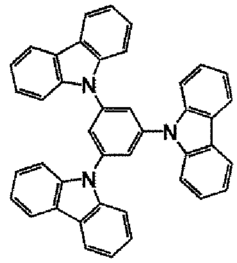

TCP

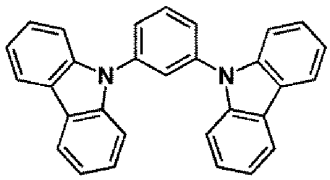

mCP

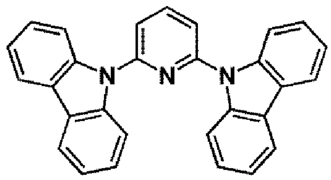

H50

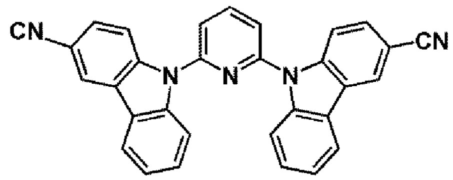

H51

.

In an embodiment, the host may further include a compound represented by Formula 301:

Formula 301

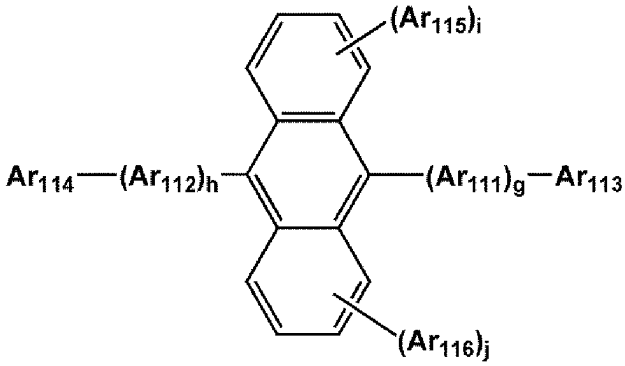

wherein, in Formula 301, $Ar_{111}$ and $Ar_{112}$ may each independently be:

a phenylene group, a naphthalene group, a phenanthrenylene group, or a pyrenylene group; or
a phenylene group, a naphthalene group, a phenanthrenylene group, or a pyrenylene group, each substituted with at least one phenyl group, naphthyl group, anthracenyl group, or any combination thereof.

$Ar_{113}$ to $Ar_{116}$ in Formula 301 may each independently be:

a $C_1$-$C_{10}$ alkyl group, a phenyl group, a naphthyl group, a phenanthrenyl group, or a pyrenyl group; or
a phenyl group, a naphthyl group, a phenanthrenyl group, and a pyrenyl group, each substituted with at least one phenyl group, naphthyl group, anthracenyl group, or any combination thereof.

The designations g, h, i, and j in Formula 301 may each independently be an integer from 0 to 4, and may be, for example, 0, 1, or 2.

In Formula 301, $Ar_{113}$ to $Ar_{116}$ may each independently be:

a $C_1$-$C_{10}$ alkyl group that is substituted with at least one of a phenyl group, a naphthyl group, an anthracenyl group, or any combination thereof;
a phenyl group, a naphthyl group, an anthracenyl group, a pyrenyl, a phenanthrenyl group, or a fluorenyl group;
a phenyl group, a naphthyl group, an anthracenyl group, a pyrenyl group, a phenanthrenyl group, or a fluorenyl group, each substituted with at least one deuterium, -F, -Cl, -Br, -I, a hydroxyl group, a cyano group, a nitro group, an amino

group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthracenyl group, a pyrenyl group, a phenanthrenyl group, a fluorenyl group; a group shown below, or a combination thereof, but embodiments are not limited thereto.

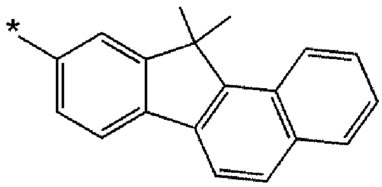

,

In an embodiment, the host may include a compound represented by Formula 302:

Formula 302

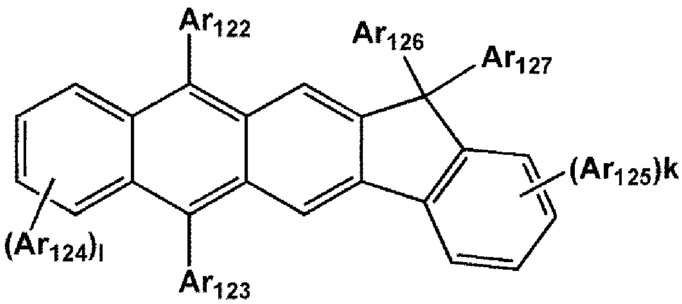

.

$Ar_{122}$ to $Ar_{125}$ in Formula 302 are the same as described in detail in connection with $Ar_{113}$ in Formula 301.

$Ar_{126}$ and $Ar_{127}$ in Formula 302 may each independently be a $C_1$-$C_{10}$ alkyl group (for example, a methyl group, an ethyl group, or a propyl group).

The designations k and l in Formula 302 may each independently be an integer from 0 to 4. For example, k and l may be 0, 1, or 2.

In an embodiment, the host may include at least one of Compounds H1 to H25:

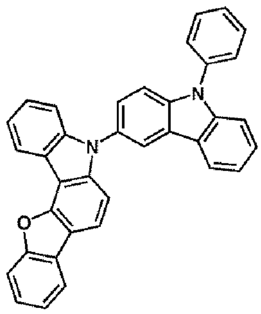

H1

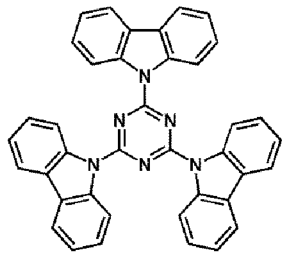

H2

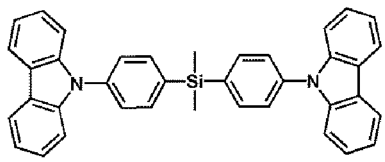

H3

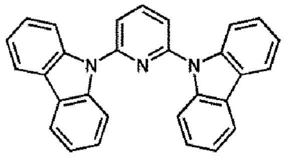

H4

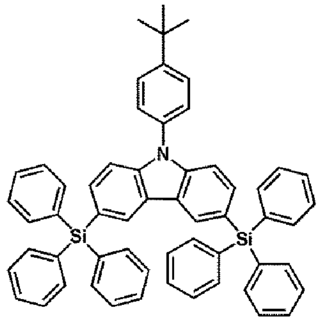

H5

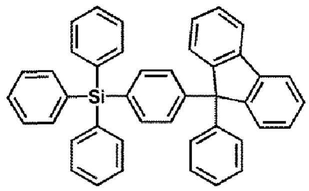

H6

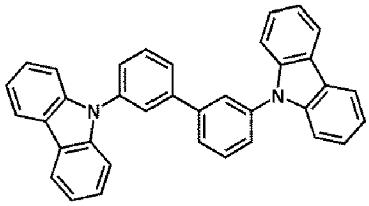

H7

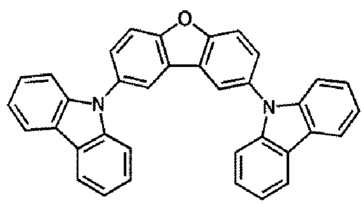

H8

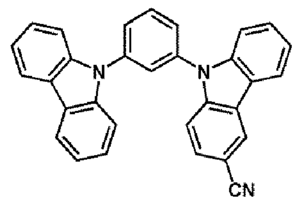

H9

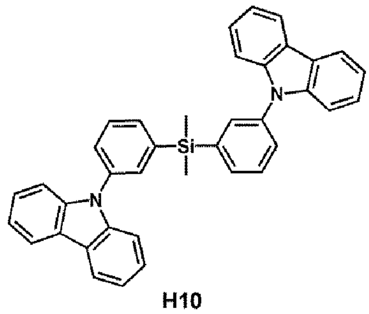

H10

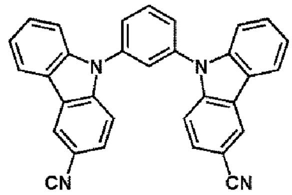

H11

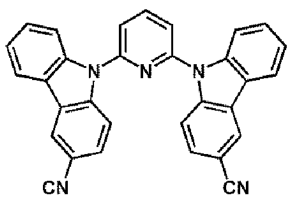

H12

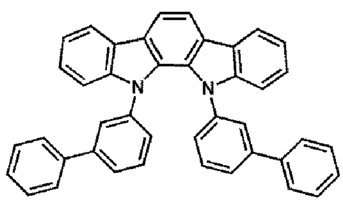

H13

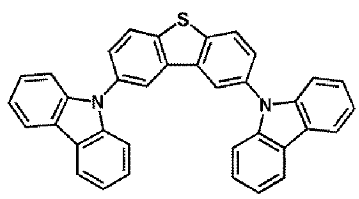

H14

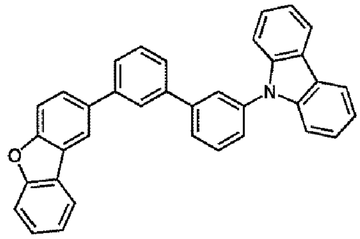

H15

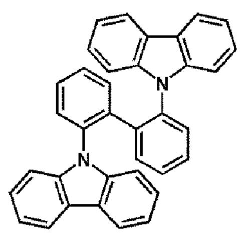

H16

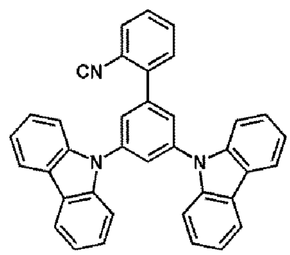

H17

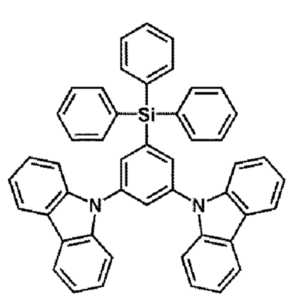

H18

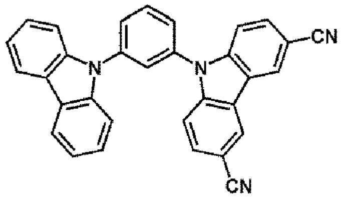

H19

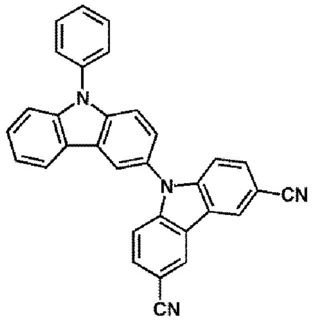

H20

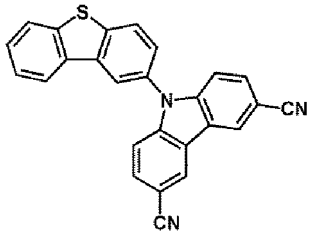

H21

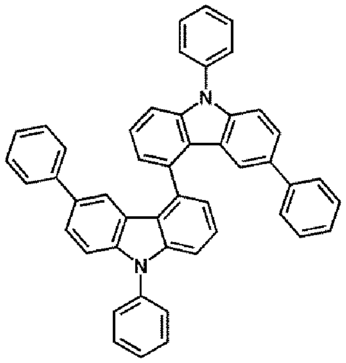

H22

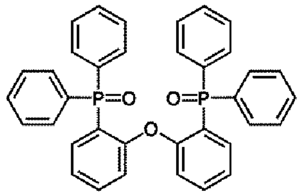

H23

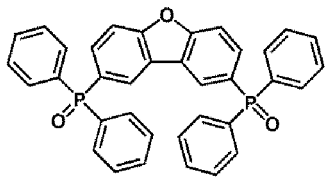

H24

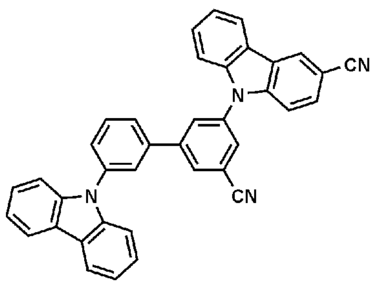

H25

In an embodiment, the host may consist of one kind of compound. For example, the one kind of compound may be a first material (hole-transporting host), a second material (electron-transporting host) as described above, or any combination thereof.

In an embodiment, the host may include two or more kinds of compounds. For example, the host may include two or more different kinds of hole-transporting hosts, or two or more different kinds of electron-transporting hosts, or a combination of at least one hole-transporting host and at least one electron-transporting host.

[Emitter in emission layer]

The emitter includes the heterocyclic compound.

[Sensitizer in emission layer]

In an embodiment, the sensitizer may include a phosphorescent compound.

In an embodiment, the phosphorescent compound may include an organometallic compound including at least one metal.

In an embodiment, the organometallic compound may include: at least one transition metal ($M_{11}$); and an organic ligand ($L_{11}$), wherein $L_{11}$ and $M_{11}$ may form one cyclometalated ring, two cyclometalated rings, three cyclometalated rings, or four cyclometalated rings.

In an embodiment, the organometallic compound may be represented by Formula 101:

Formula 101 $M_{11}(L_{11})n_{11}(L_{12})n_{12}$.

wherein, in Formula 101,

$M_{11}$ may be a transition metal, and
$L_{11}$ may be a ligand represented by one of Formulae 1-1 to 1-4; and
$L_{12}$ may be a monodentate ligand or a bidentate ligand,
n11 may be 1, and
n12 may be selected from 0, 1, and 2,

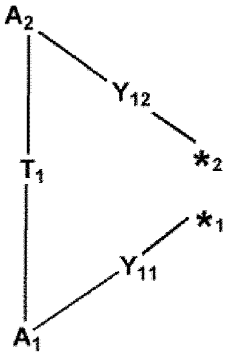

1-1

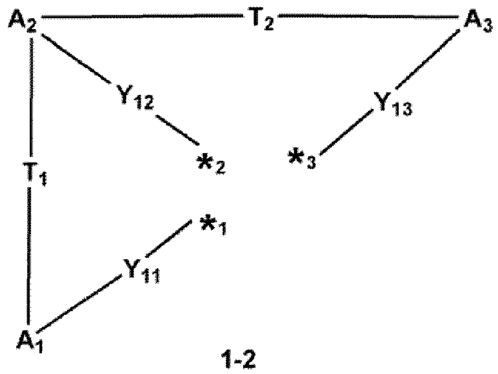

1-2

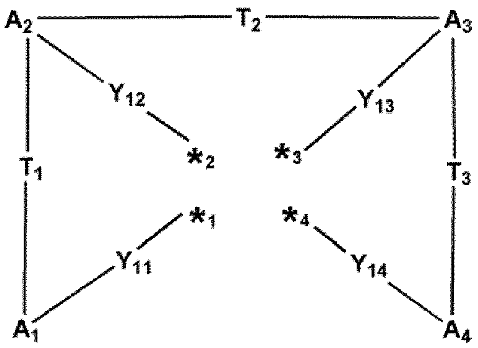

1-3

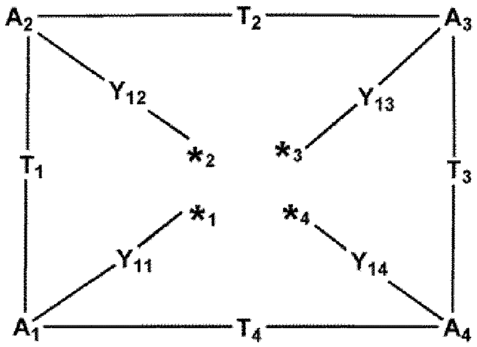

1-4

wherein, in Formulae 1-1 to 1-4,

$A_1$ to $A_4$ may each independently be a substituted or unsubstituted $C_5$-$C_{30}$ carbocyclic group, a substituted or unsubstituted $C_1$-$C_{30}$ heterocyclic group, or a non-cyclic group,
$Y_{11}$ to $Y_{14}$ may each independently be a chemical bond, O, S, $N(R_{91})$, $B(R_{91})$, $P(R_{91})$, or $C(R_{91})(R_{92})$,
$T_1$ to $T_4$ may each independently be a single bond, a double bond, *-$N(R_{93})$-*', *-$B(R_{93})$-*', *-$P(R_{93})$-*', *-$C(R_{93})(R_{94})$-*', *-$Si(R_{93})(R_{94})$-*', *-$Ge(R_{93})(R_{94})$-*', *-S-*', *-Se-*', *-O-*', *-C(=O)-*', *-S(=O)-*', *-$S(=O)_2$-*', *-$C(R_{93})$=*', *=$C(R_{93})$-*', *-$C(R_{93})=C(R_{94})$-*', *-C(=S)-*', or *-C≡C-*',
a substituent of the substituted $C_5$-$C_{30}$ carbocyclic group, a substituent of the substituted $C_1$-$C_{30}$ heterocyclic group, and $R_{91}$ to $R_{94}$ may each independently be hydrogen, deuterium, -F, -Cl, -Br, -I, -$SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent aromatic condensed polycyclic group, a substituted or unsubstituted monovalent

aromatic condensed heteropolycyclic group, a substituted or unsubstituted monovalent non-aromatic condensed polycyclic group, a substituted or unsubstituted monovalent non-aromatic condensed heteropolycyclic group, $-Si(Q_1)(Q_2)(Q_3)$, $-Ge(Q_1)(Q_2)(Q_3)$, $-C(Q_1)(Q_2)(Q_3)$, $-B(Q_1)(Q_2)$, $-N(Q_1)(Q_2)$, $-P(Q_1)(Q_2)$, $-C(=O)(Q_1)$, $-S(=O)(Q_1)$, $-S(=O)_2(Q_1)$, $-P(=O)(Q_1)(Q_2)$, or $-P(=S)(Q_1)(Q_2)$, wherein the substituent of the substituted $C_5$-$C_{30}$ carbocyclic group and the substituent of the substituted $C_1$-$C_{30}$ heterocyclic group are not hydrogen,

*1, *2, *3, and *4 each indicate a binding site to $M_{11}$, and

$Q_1$ to $Q_3$ may each independently be hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkylheteroaryl group, a $C_1$-$C_{60}$ heteroaryloxy group, a $C_1$-$C_{60}$ heteroarylthio group, a monovalent aromatic condensed polycyclic group, a monovalent aromatic condensed heteropolycyclic group, a monovalent non-aromatic condensed polycyclic group, a monovalent non-aromatic condensed heteropolycyclic group, a $C_1$-$C_{60}$ alkyl group that is substituted with at least one deuterium, -F, a cyano group, a $C_1$-$C_{60}$ alkyl group, a $C_6$-$C_{60}$ aryl group, or any combination thereof, or a $C_6$-$C_{60}$ aryl group that is substituted with at least one deuterium, -F, a cyano group, a $C_1$-$C_{60}$ alkyl group, a $C_6$-$C_{60}$ aryl group, or any combination thereof.

In an embodiment, the transition metal may be platinum (Pt), palladium (Pd), gold (Au), iridium (Ir), osmium (Os), titanium (Ti), zirconium (Zr), hafnium (Hf), europium (Eu), terbium (Tb), thulium (Tm), or rhodium (Rh).

In an embodiment, the sensitizer may include a delayed fluorescence compound.

In an embodiment, the delayed fluorescence compound may be represented by Formula 101 or 102.

Formula 101

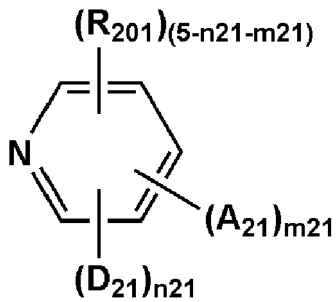

Formula 102

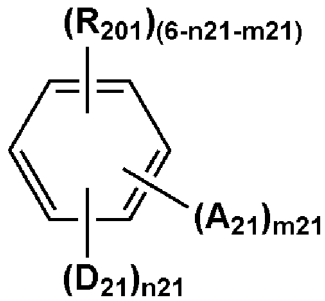

wherein, in Formulae 101 and 102,

$A_{21}$ may be an acceptor group,

$D_{21}$ may be a donor group,

m21 may be 1, 2, or 3, n21 may be 1, 2, or, 3, and

the sum of n21 and m21 in Formula 101 may be 5 or less, and the sum of n21 and m21 in Formula 102 may be 6 or less;

$R_{201}$ may be hydrogen, deuterium, -F, -Cl, -Br, -I, $-SF_5$, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-Si(Q_1)(Q_2)(Q_3)$, $-Ge(Q_1)(Q_2)(Q_3)$, $-C(Q_1)(Q_2)(Q_3)$, $-B(Q_1)(Q_2)$, $-N(Q_1)(Q_2)$, $-P(Q_1)(Q_2)$, $-C(=O)(Q_1)$, $-S(=O)(Q_1)$, $-S(=O)_2(Q_1)$, $-P(=O)(Q_1)(Q_2)$, or $-P(=S)(Q_1)(Q_2)$, and a plurality of $R_{201}$(s) may optionally be bonded together to form a substituted or unsubstituted $C_5$-$C_{30}$ carbocyclic group or a substituted or unsubstituted $C_1$-$C_{30}$ heterocyclic group,

wherein $Q_1$ to $Q_3$ may each independently be hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a

$C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkylheteroaryl group, a $C_1$-$C_{60}$ heteroaryloxy group, a $C_1$-$C_{60}$ heteroarylthio group, a monovalent aromatic condensed polycyclic group, a monovalent aromatic condensed heteropolycyclic group, a monovalent non-aromatic condensed polycyclic group, a monovalent non-aromatic condensed heteropolycyclic group, a $C_1$-$C_{60}$ alkyl group that is substituted with at least one deuterium, -F, a cyano group, a $C_1$-$C_{60}$ alkyl group, a $C_6$-$C_{60}$ aryl group, or any combination thereof, or a $C_6$-$C_{60}$ aryl group that is substituted with at least one deuterium, -F, a cyano group, a $C_1$-$C_{60}$ alkyl group, a $C_6$-$C_{60}$ aryl group, or any combination thereof.

In an embodiment, in Formulae 101 and 102, $A_{21}$ may be a substituted or unsubstituted $\pi$ electron-deficient nitrogen-free cyclic group.

In detail, the $\pi$ electron-deficient nitrogen-free cyclic group may be: a benzene group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, or a triindolobenzene group; or a condensed ring in which two or more $\pi$ electron-deficient nitrogen-free cyclic groups are condensed with each other, but embodiments are not limited thereto.

For example, in Formulae 101 and 102, $D_{21}$ may be: -F, a cyano group, or a $\pi$ electron-deficient nitrogen-containing cyclic group;

a $C_1$-$C_{60}$ alkyl group, a $\pi$ electron-deficient nitrogen-containing cyclic group, or a $\pi$ electron-deficient nitrogen-free cyclic group, each substituted with at least one -F, a cyano group, or any combination thereof; or
a $\pi$ electron-deficient nitrogen-containing cyclic group, each substituted with at least one of deuterium, a $C_1$-$C_{60}$ alkyl group, a $\pi$ electron-deficient nitrogen-containing cyclic group, or a $\pi$ electron-deficient nitrogen-free cyclic group.

In detail, the $\pi$ electron-deficient nitrogen-free cyclic group is the same as described above.

In detail, the $\pi$ electron-deficient nitrogen-containing cyclic group refers to a cyclic group having at least one *-N=*' moiety, and, for example, may be: an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azacarbazole group, or a benzimidazolobenzimidazole group; or a condensed ring in which two or more $\pi$ electron-deficient nitrogen-containing cyclic groups are condensed with each other.

In an embodiment, in the organic layer, an amount of the sensitizer may be greater than an amount of the emitter. For example, a volume ratio of the sensitizer and the emitter may be about 30:0.1 to about 10:3, or about 10:0.1 to about 20:5. As another example, a weight ratio of the sensitizer and the emitter may be about 10:0.1 to about 20:5. Meanwhile, a volume ratio of the host and the sensitizer in the organic layer may be about 60:40 to about 95:5 or about 70:30 to about 90:10. Alternatively, a weight ratio of the host and the sensitizer may be about 60:40 to about 95:5. When the amount is within the range described above, the organic light-emitting device may have improved emission efficiency and/or lifespan characteristics.

FIG. 1 is a schematic cross-sectional view of the organic light-emitting device 10 according to an embodiment. Hereinafter, referring to FIG. 1, the structure and manufacturing method of the organic light-emitting device 10 according to an embodiment are described as follows. The organic light-emitting device 10 includes a first electrode 11, an organic layer 15, and a second electrode 19, which are sequentially stacked.

A substrate may be additionally disposed under the first electrode 11 or on the second electrode 19. For use as the substrate, any substrate that is used in organic light-emitting devices available in the art may be used, and for example, a glass substrate or a transparent plastic substrate, each having excellent mechanical strength, thermal stability, transparency, surface smoothness, ease of handling, and water resistance, may be used.

The first electrode 11 may be formed by depositing or sputtering, for example, onto the substrate, a material for

forming the first electrode 11. The first electrode 11 may be an anode. The material for forming the first electrode 11 may be a material with a high work function for easy hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide ($SnO_2$), or zinc oxide (ZnO). Alternatively, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multilayer structure including two or more layers. For example, the first electrode 11 may have a three-layered structure of ITO/Ag/ITO, but embodiments are not limited thereto.

The organic layer 15 is disposed above the first electrode 11.

The organic layer 15 may include a hole transport region, an emission layer, or an electron transport region.

The hole transport region may be between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. Alternatively, the hole transport region may have a hole injection layer/hole transport layer structure, a hole injection layer/hole transport layer/electron blocking layer structure, or a hole injection layer/a first hole transport layer/a second hole transport layer/an electron blocking layer structure, wherein, for each structure, respective layers are sequentially stacked in this stated order from the first electrode 11.

When the hole transport region includes a hole injection layer, the hole injection layer may be formed on the first electrode 11 using one or more suitable methods, for example, vacuum deposition, spin coating, casting, and/or Langmuir-Blodgett (LB) deposition.

When a hole injection layer is formed by vacuum deposition, the deposition condition may vary according to a compound used as a material to form the hole injection layer, and the structure and thermal characteristics of the hole injection layer. For example, the deposition condition may include a deposition temperature of about 100 °C to about 500 °C, a vacuum pressure of about $10^{-8}$ torr to about $10^{-3}$ torr, and a deposition rate of about 0.01 Å/sec to about 100 Å/sec. However, the deposition condition are not limited thereto.

When the hole injection layer is formed by spin coating, the coating condition may vary according to a compound used as a material to form the hole injection layer, and the structure and thermal characteristics of the hole injection layer. For example, the coating condition may include a coating speed of about 2,000 rpm to about 5,000 rpm, and a heat treatment temperature of about 80 °C to about 200 °C for removing a solvent after coating. However, the coating condition are not limited thereto.

The conditions for forming the hole transport layer and the electron blocking layer may be the same as the conditions for forming the hole injection layer.

The hole transport region may include at least one of m-MTDATA, TDATA, 2-TNATA, NPB, β-NPB, TPD, spiro-TPD, spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201, a compound represented by Formula 202, or any combination thereof:

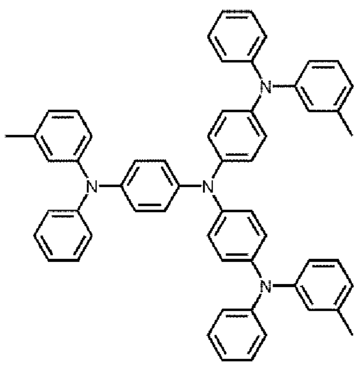

m-MTDATA

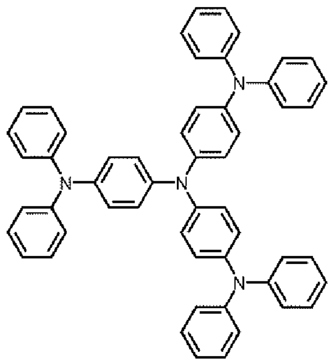

TDATA

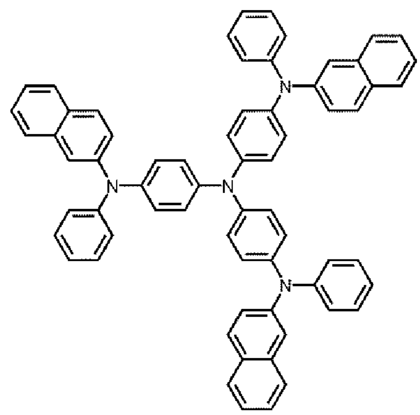

2-TNATA

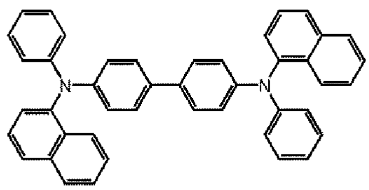

NPB

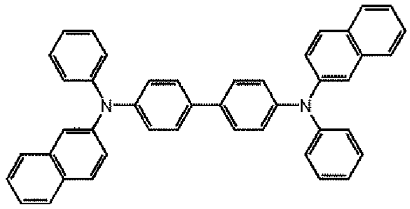

β-NPB

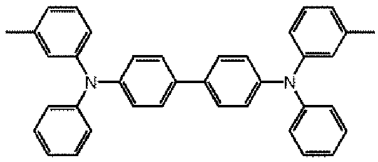

TPD

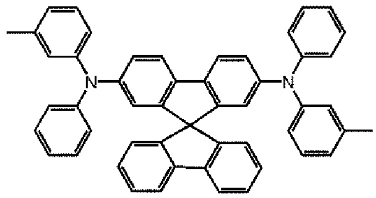

Spiro-TPD

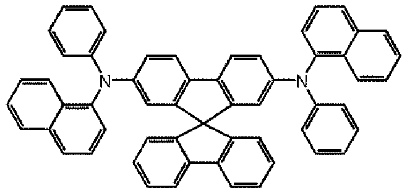

Spiro-NPB

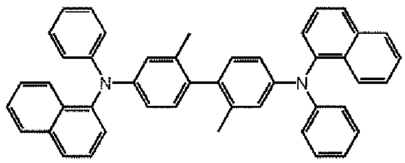

methylated NPB

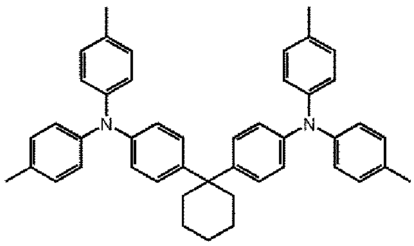

TAPC

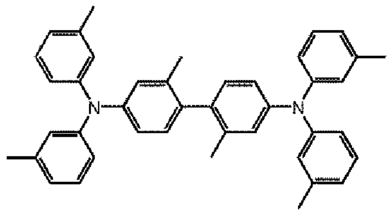

HMTPD

Formula 201

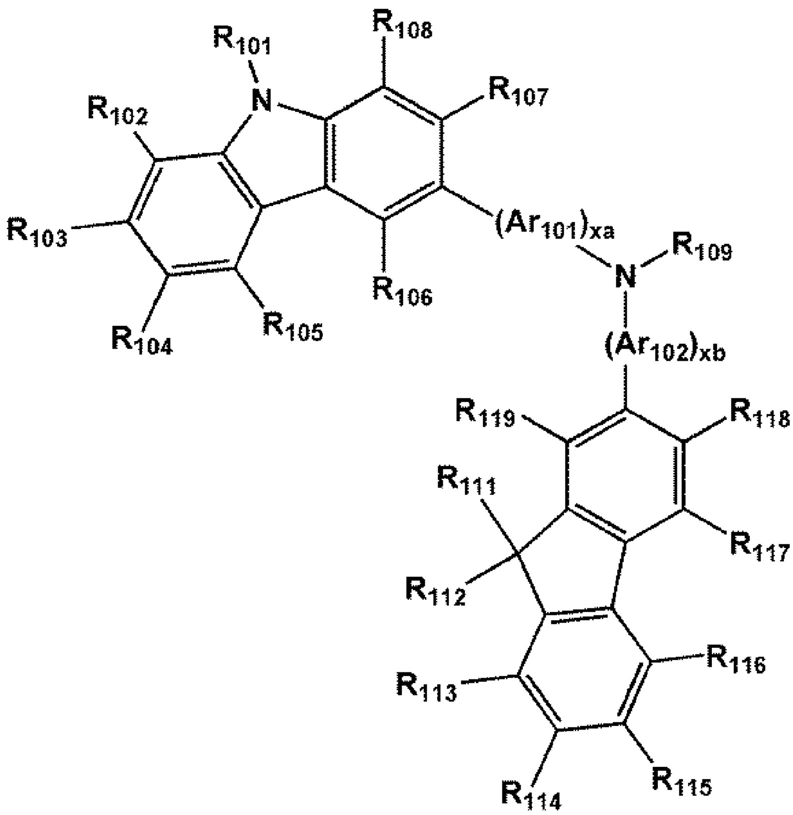

Formula 202

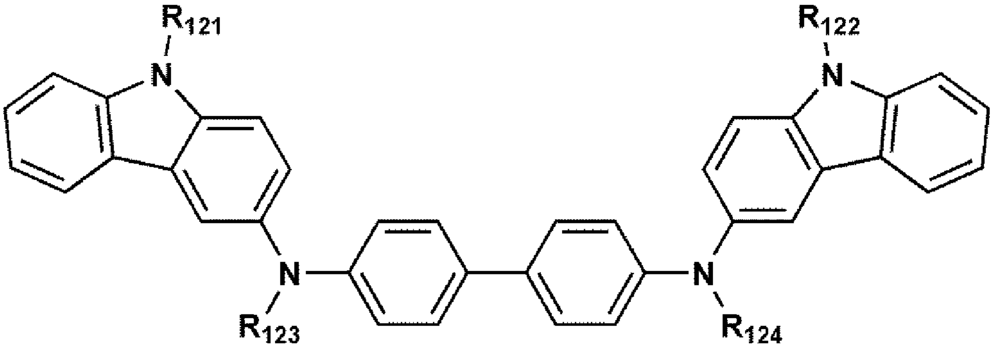

wherein, in Formula 201, $Ar_{101}$ and $Ar_{102}$ may each independently be:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, or a pentacenylene group; or

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, or a pentacenylene group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof.

In Formula 201, xa and xb may each independently be an integer from 0 to 5, may each independently be or 0, 1, or 2. For example, xa may be 1 and xb may be 0, but xa and xb are not limited thereto.

In Formulae 201 and 202, $R_{101}$ to $R_{108}$, $R_{111}$ to $R_{119}$, and $R_{121}$ to $R_{124}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (for example, a methyl group, an ethyl group, a propyl group, a butyl group, a pentyl group, or a hexyl group), or a $C_1$-$C_{10}$ alkoxy group (for example, a methoxy group, an ethoxy group, a propoxy group, a butoxy group, or a pentoxy group);

a $C_1$-$C_{10}$ alkyl group or a $C_1$-$C_{10}$ alkoxy group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, or a combination thereof;

a phenyl group, a naphthyl group, an anthracenyl group, a fluorenyl group, or a pyrenyl group; or

a phenyl group, a naphthyl group, an anthracenyl group, a fluorenyl group, or a pyrenyl group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{10}$ alkyl group, a $C_1$-$C_{10}$ alkoxy group, or a combination thereof, but embodiments are not limited thereto.

In Formula 201, $R_{109}$ may be:

a phenyl group, a naphthyl group, an anthracenyl group, or a pyridinyl group; or

a phenyl group, a naphthyl group, an anthracenyl group, or a pyridinyl group, each substituted with deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof,

a $C_1$-$C_{20}$ alkyl group, a $C_1$-$C_{20}$ alkoxy group, a phenyl group, a naphthyl group, an anthracenyl group, a pyridinyl group, or any combination thereof.

In an embodiment, the compound represented by Formula 201 may be represented by Formula 201A, but embodiments are not limited thereto:

Formula 201A

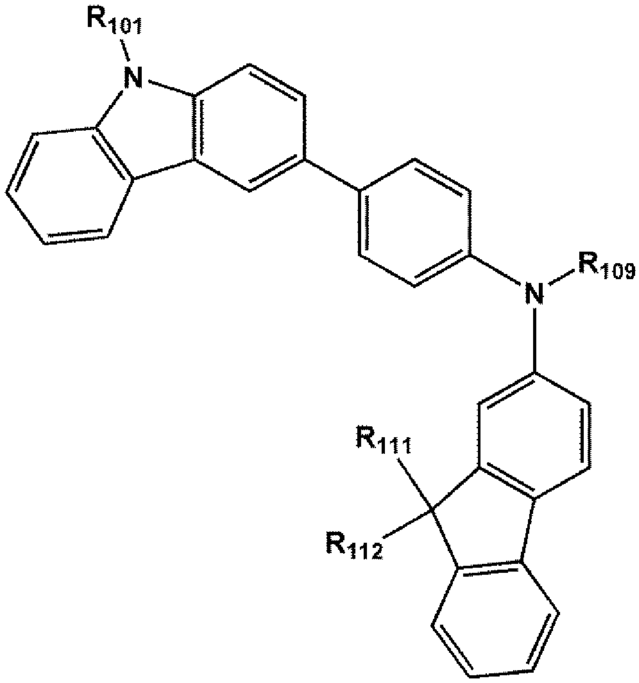

wherein, in Formula 201A, $R_{101}$, $R_{111}$, $R_{112}$, and $R_{109}$ are each the same as described above.

For example, the compound represented by Formula 201 and the compound represented by Formula 202 may include Compounds HT1 to HT20, but embodiments are not limited thereto:

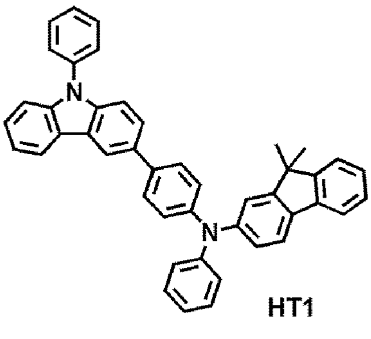

HT1

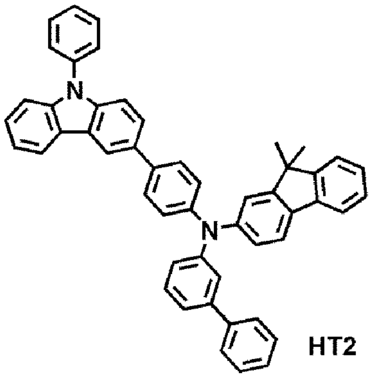

HT2

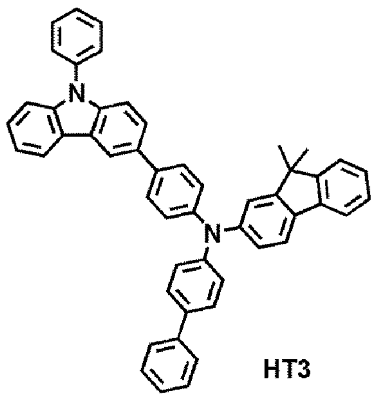

HT3

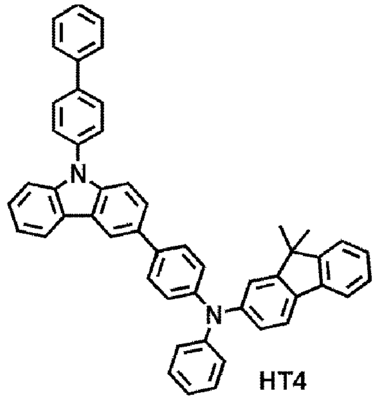
HT4

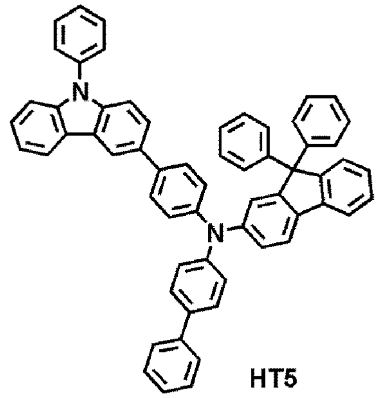
HT5

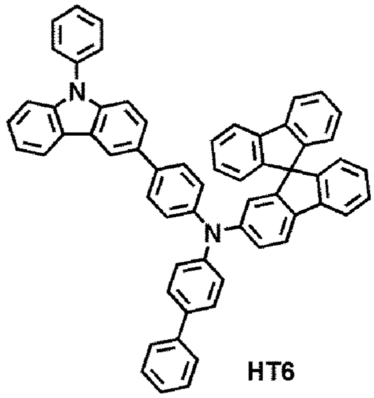
HT6

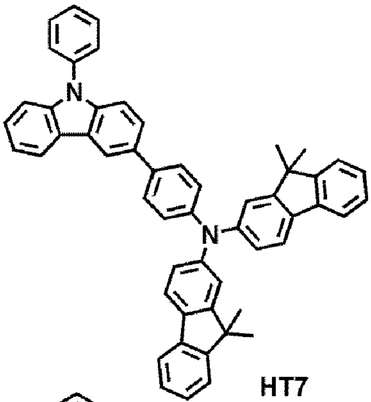
HT7

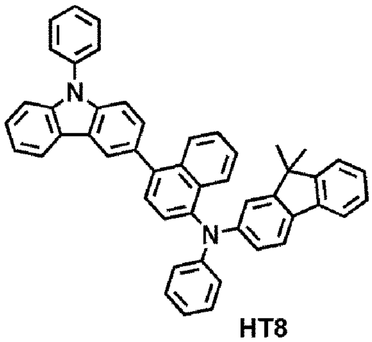
HT8

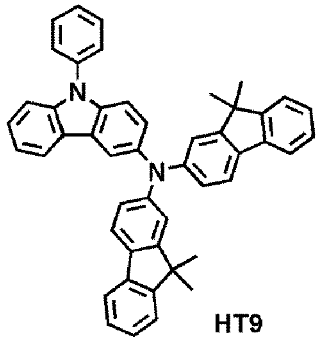
HT9

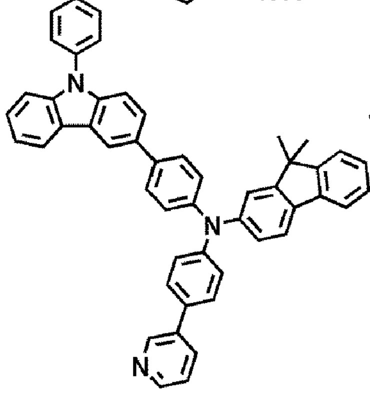
HT10

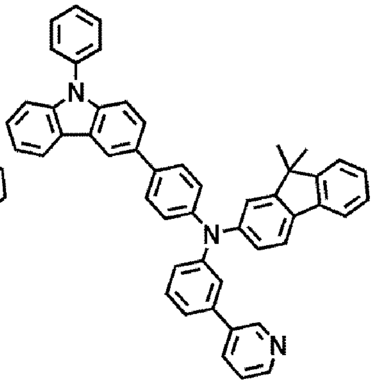
HT11

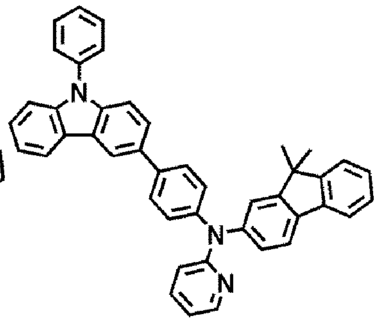
HT12

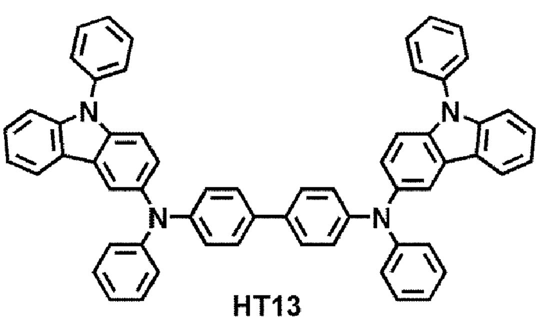
HT13

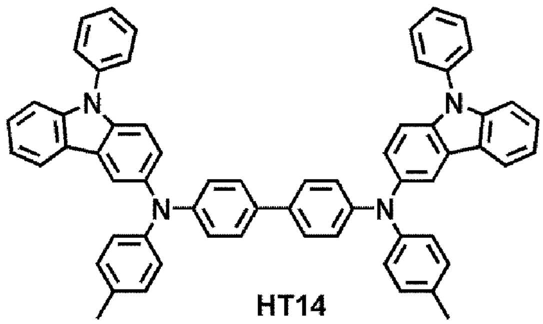
HT14

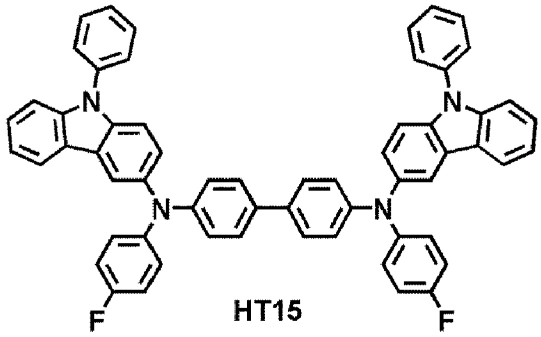

HT15

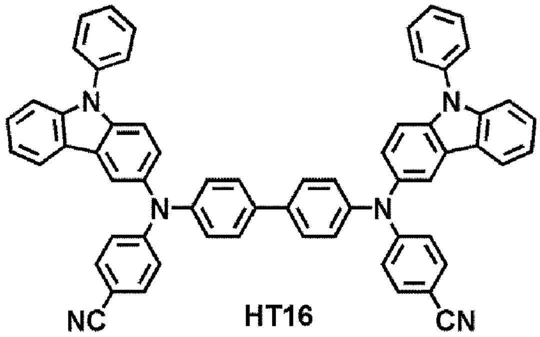

HT16

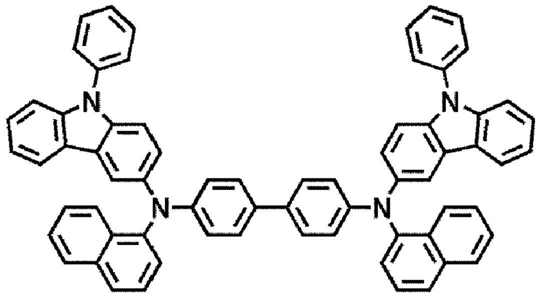

HT17

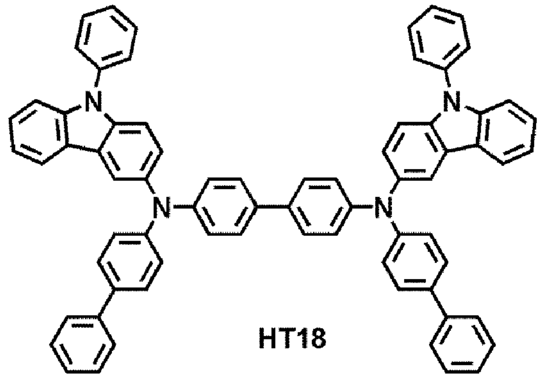

HT18

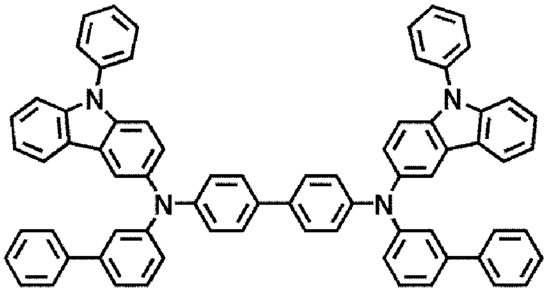

HT19

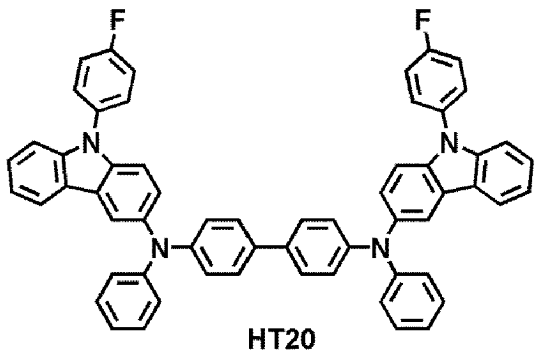

HT20

A thickness of the hole transport region may be about 100 Å to about 10,000 Å, for example, about 100 Å to about 1,000 Å. When the hole transport region includes at least one of a hole injection layer and a hole transport layer, a thickness of the hole injection layer may be about 100 Å to about 10,000 Å, for example, about 100 Å to about 1,000 Å, and a thickness of the hole transport layer may be about 50 Å to about 2,000 Å, for example, about 100 Å to about 1,500 Å. When the thicknesses of the hole transport region, the hole injection layer, and the hole transport layer are within these ranges as described above, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one of a quinone derivative, a metal oxide, and a cyano group-containing compound, but embodiments are not limited thereto. For example, examples of the p-dopant are: a quinone derivative, such as tetracyanoquinonedimethane (TCNQ) and 2,3,5,6-tetrafluoro-tetracyano-1,4-benzoquinonedimethane (F4-TCNQ); a metal oxide, such as a tungsten oxide or a molybdenum oxide; or a cyano group-containing compound, such as Compound HT-D1 or F12, but embodiments are not limited thereto:

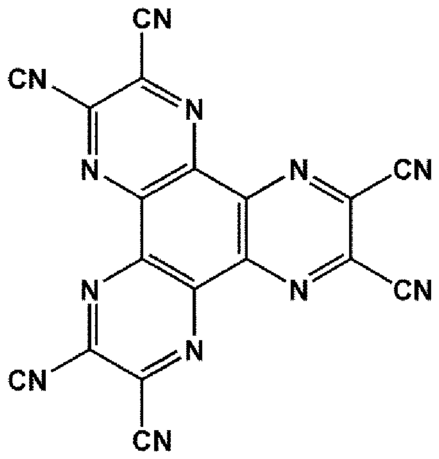

HT-D1

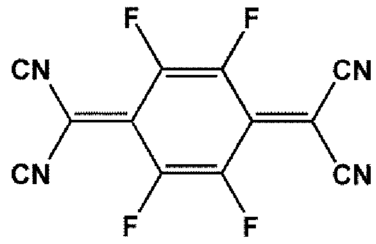

F4-TCNQ

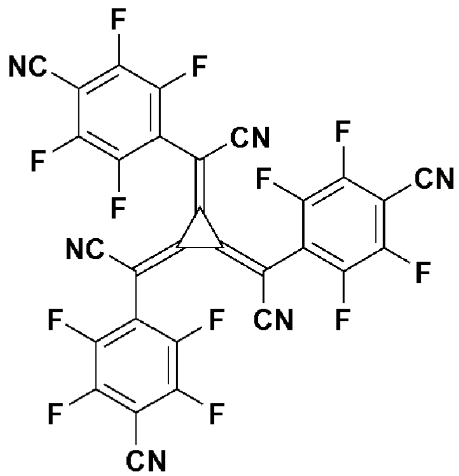

F12

The hole transport region may include a buffer layer.

The buffer layer may compensate for an optical resonance distance according to a wavelength of light emitted from the emission layer, and thus, efficiency of the organic light-emitting device may be improved.

Meanwhile, when the hole transport region includes an electron blocking layer, a material for the electron blocking layer may be selected from materials for the hole transport region described above and materials for a host described above. However, the material for the electron blocking layer is not limited thereto. For example, when the hole transport region includes an electron blocking layer, examples of a material for the electron blocking layer may include mCP described below.

Then, an emission layer may be formed on the hole transport region using vacuum deposition, spin coating, casting, LB deposition, or the like. When the emission layer is formed by vacuum deposition or spin coating, the deposition condition and the coating condition may vary according to a compound used as a material to form the emission layer, but the deposition condition and the coating condition may be similar to those applied in forming the hole injection layer.

When the organic light-emitting device is a full-color organic light-emitting device, the emission layer may be patterned into a red emission layer, a green emission layer, and a blue emission layer. Alternatively, the emission layer has a structure in which a red emission layer, a green emission layer, and/or a blue emission layer are stacked, and thus, may emit white light, and various modifications may be made.

When the emission layer includes a host and a dopant, an amount of the dopant may be about 0.01 parts by weight to about 15 parts by weight based on 100 parts by weight of the host, but embodiments are not limited thereto.

A thickness of the emission layer may be about 100 Å to about 1,000 Å, for example, about 200 Å to about 600 Å. When the thickness of the emission layer is within the range, excellent light-emission characteristics may be obtained without a substantial increase in driving voltage.

Next, an electron transport region may be disposed above the emission layer.

The electron transport region may include a hole blocking layer, an electron transport layer, an electron injection layer, or a combination thereof.

For example, the electron transport region may have a hole blocking layer/electron transport layer/electron injection layer structure or an electron transport layer/electron injection layer structure, but embodiments are not limited thereto. The electron transport layer may have a single-layered structure or a multilayer structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP, Bphen, BAlq, or any combination thereof, but embodiments are not limited thereto:

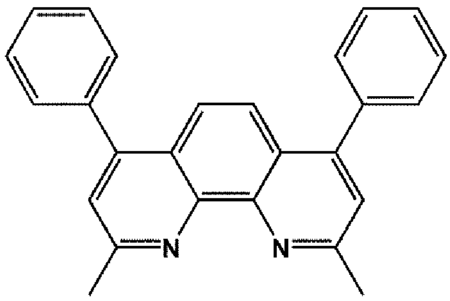
BCP

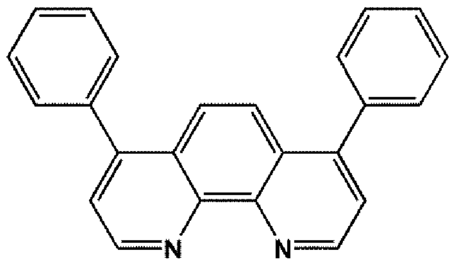
Bphen

A thickness of the hole blocking layer may be about 20 Å to about 1,000 Å, for example, about 30 Å to about 300 Å. When the thickness of the hole blocking layer is within the range as described above, excellent hole blocking characteristics may be obtained without a substantial increase in driving voltage.

The electron transport layer may include at least one of BCP, Bphen, $Alq_3$, BAlq, TAZ, NTAZ, or any combination thereof:

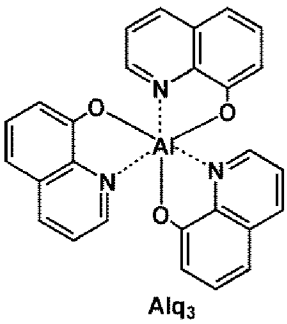
$Alq_3$

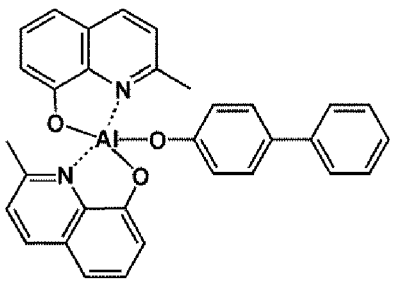
BAlq

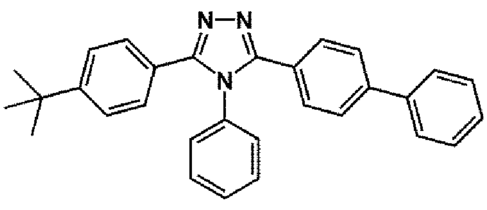
TAZ

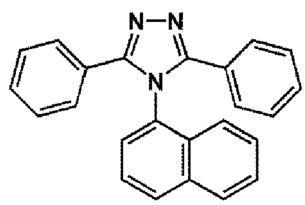
NTAZ

Alternatively, the electron transport layer may include at least one of Compounds ET1 to ET25, but embodiments are not limited thereto:

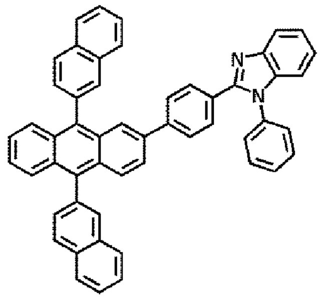

ET1

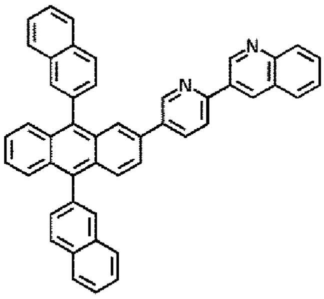

ET2

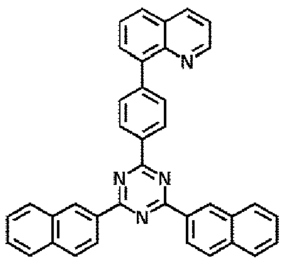

ET3

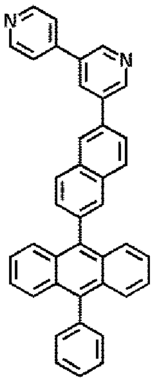

ET4

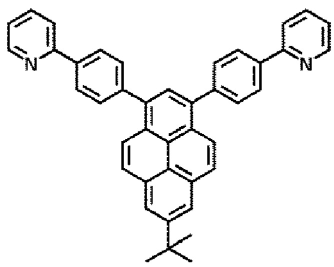

ET5

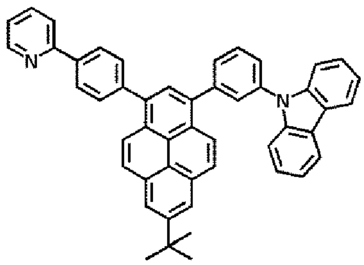

ET6

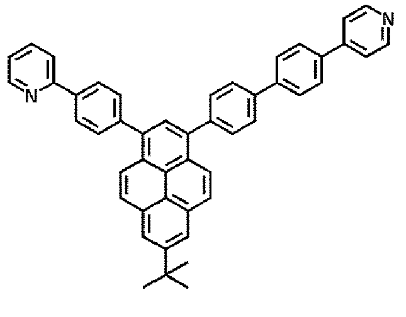

ET7

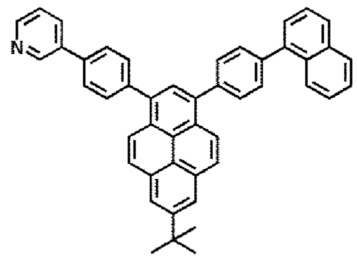

ET8

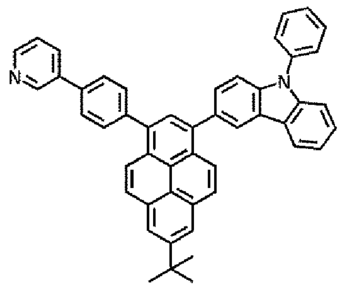

ET9

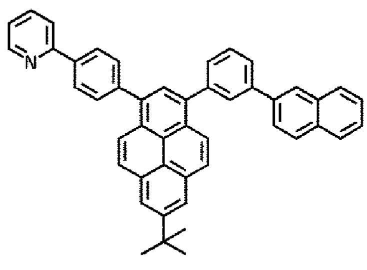

ET10

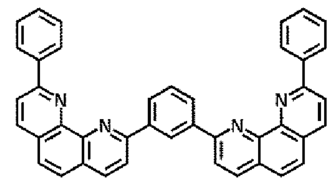

ET11

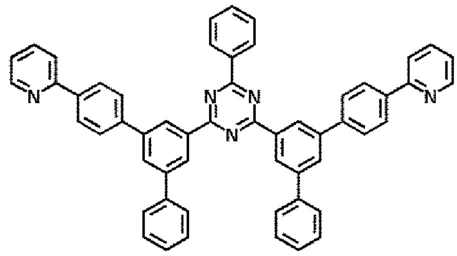

ET12

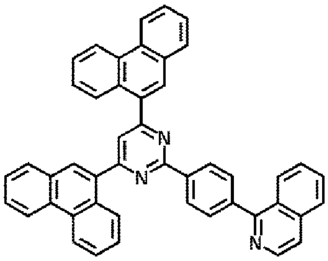

ET13

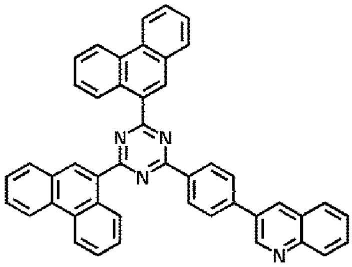

ET14

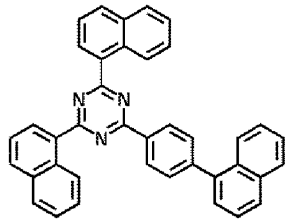

ET15

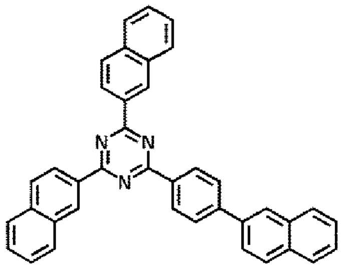

ET16

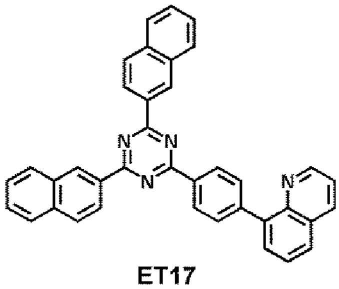

ET17

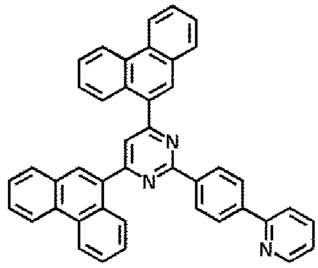

ET18

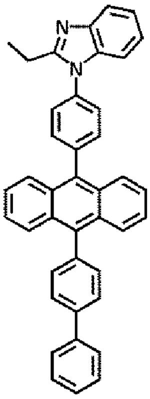

ET19

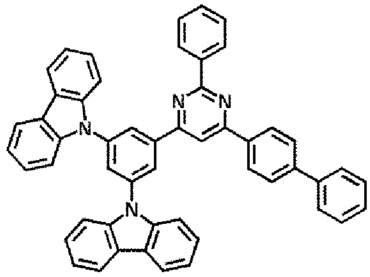

ET20

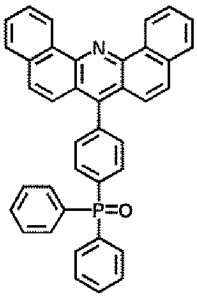

ET21

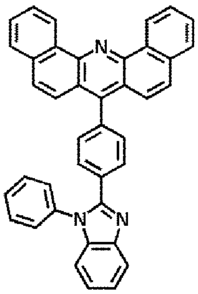

ET22

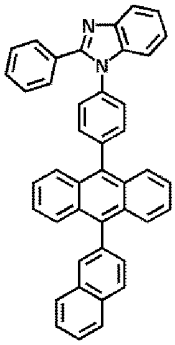

ET23

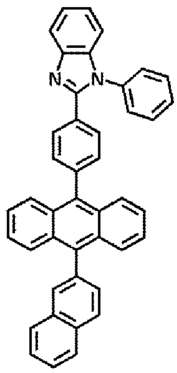

ET24

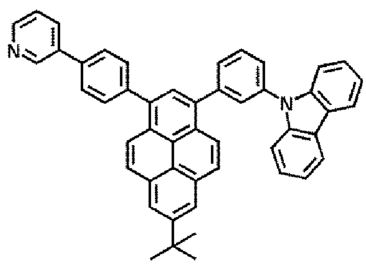

ET25

A thickness of the electron transport layer may be about 100 Å to about 1,000 Å, for example, about 150 Å to about 500 Å. When the thickness of the electron transport layer is within the range as described above, satisfactory electron transporting characteristics may be obtained without a substantial increase in driving voltage.

The electron transport layer may include a metal-containing material in addition to the material as described above.

The metal-containing material may include a Li complex. The Li complex may include, for example, Compound

ET-D1 (lithium quinolate, LiQ) or ET-D2:

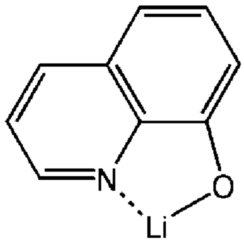

ET-D1

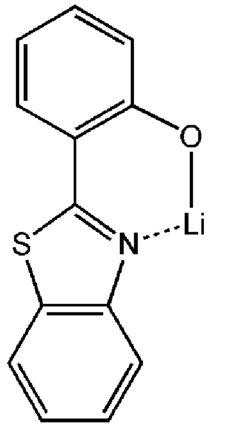

ET-D2

In addition, the electron transport region may include an electron injection layer that facilitates injection of electrons from the second electrode 19 thereinto.

The electron injection layer may include LiF, NaCl, CsF, $Li_2O$, BaO, or a combination thereof.

A thickness of the electron injection layer may be about 1 Å to about 100 Å, for example, about 3 Å to about 90 Å. When the thickness of the electron injection layer is within the range as described above, satisfactory electron injection characteristics may be obtained without a substantial increase in driving voltage.

The second electrode 19 is disposed above the organic layer 15. The second electrode 19 may be a cathode. A material for forming the second electrode 19 may include a metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as the material for forming the second electrode 19. Alternatively, a transmissive formed using ITO or IZO may be used as the second electrode 19 in order to manufacture a top-emission type light-emitting device,

Hereinbefore, the organic light-emitting device has been described with reference to FIG. 1, but embodiments are not limited thereto.

According to one or more embodiments, there is provided an electronic apparatus including the organic light-emitting device.

The electronic apparatus may further include a thin-film transistor in addition to the organic light-emitting device as described above. The thin-film transistor may include a source electrode, a drain electrode, and an activation layer, wherein any one of the source electrode and the drain electrode may be electrically connected to one of the first electrode and the second electrode of the organic light-emitting device.

According to one or more embodiments, there is provided a diagnostic composition including the heterocyclic compound represented by Formula 1.

The diagnostic composition may include at least one heterocyclic compound represented by Formula 1.

The heterocyclic compound represented by Formula 1 provides high emission efficiency, and accordingly, the diagnostic composition including the heterocyclic compound may have high diagnostic efficiency.

The diagnostic composition may be used in various applications including a diagnosis kit, a diagnosis reagent, a biosensor, and a biomarker.

The $C_1$-$C_{60}$ alkyl group as used herein refers to a linear or branched saturated aliphatic hydrocarbon monovalent group having 1 to 60 carbon atoms, and examples thereof include a methyl group, an ethyl group, a propyl group, an isobutyl group, a sec-butyl group, a tert-butyl group, a pentyl group, an isoamyl group, and a hexyl group. The $C_1$-$C_{60}$ alkylene group as used herein refers to a divalent group having the same structure as the $C_1$-$C_{60}$ alkyl group.

Examples of the $C_1$-$C_{60}$ alkyl group, the $C_1$-$C_{20}$ alkyl group, and/or the $C_1$-$C_{10}$ alkyl group as used herein may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group, each unsubstituted or substituted with a methyl group, an ethyl group, an n-propyl group, an isopropyl group, an n-butyl group, a sec-butyl group, an isobutyl group, a tert-butyl

group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group. For example, Formula 9-33 is a branched $C_6$ alkyl group, for example, a tert-butyl group that is substituted with two methyl groups.

The $C_1$-$C_{60}$ alkoxy group used herein refers to a monovalent group represented by $-OA_{101}$ (wherein $A_{101}$ is the $C_1$-$C_{60}$ alkyl group), and examples thereof include a methoxy group, an ethoxy group, and an isopropyloxy group.

Examples of the $C_1$-$C_{60}$ alkoxy group, the $C_1$-$C_{20}$ alkoxy group, or the $C_1$-$C_{10}$ alkoxy group as used herein may include a methoxy group, an ethoxy group, a propoxy group, a butoxy group, and a pentoxy group.

The $C_2$-$C_{60}$ alkenyl group as used herein has a structure including at least one carbon-carbon double bond in the middle or at the terminus of the $C_2$-$C_{60}$ alkyl group, and examples thereof include an ethenyl group, a propenyl group, and a butenyl group. The $C_2$-$C_{60}$ alkenylene group as used herein refers to a divalent group having the same structure as the $C_2$-$C_{60}$ alkenyl group.

The $C_2$-$C_{60}$ alkynyl group as used herein has a structure including at least one carbon-carbon triple bond in the middle or at the terminus of the $C_2$-$C_{60}$ alkyl group, and examples thereof include an ethynyl group and a propynyl group. The $C_2$-$C_{60}$ alkynylene group as used herein refers to a divalent group having the same structure as the $C_2$-$C_{60}$ alkynyl group.

The $C_3$-$C_{10}$ cycloalkyl group as used herein refers to a monovalent saturated hydrocarbon cyclic group having 3 to 10 carbon atoms, and examples thereof include a cyclopropyl group, a cyclobutyl group, a cyclopentyl group, a cyclohexyl group, and a cycloheptyl group. The $C_3$-$C_{10}$ cycloalkylene group as used herein refers to a divalent group having the same structure as the $C_3$-$C_{10}$ cycloalkyl group.

The $C_3$-$C_{10}$ cycloalkyl group as used herein may include a cyclopropyl group, a cyclobutyl group, a cyclopentyl group, a cyclohexyl group, a cycloheptyl group, a cyclooctyl group, an adamantanyl group, a bicyclo[1.1.1]pentyl group, a bicyclo[2.1.1]hexyl group, a bicyclo[2.2.1]heptyl group (norbornanyl group), a bicyclo[2.2.2]octyl group, and the like.

The $C_1$-$C_{10}$ heterocycloalkyl group as used herein refers to a monovalent saturated cyclic group having at least one heteroatom, for example, N, O, P, Si, B, Ti, S, Se, Te, Ge, or any combination thereof as a ring-forming atom and 1 to 10 carbon atoms, and examples thereof include a tetrahydrofuranyl group and a tetrahydrothiophenyl group. The $C_1$-$C_{10}$ heterocycloalkylene group as used herein refers to a divalent group having the same structure as the $C_1$-$C_{10}$ heterocycloalkyl group.

Examples of the $C_1$-$C_{10}$ heterocycloalkyl group as used herein may include a silolanyl group, a silinanyl group, a tetrahydrofuranyl group, a tetrahydro-2H-pyranyl group, and a tetrahydrothiophenyl group.

The $C_3$-$C_{10}$ cycloalkenyl group as used herein refers to a monovalent cyclic group that has 3 to 10 carbon atoms, at least one carbon-carbon double bond in its ring, and no aromaticity, and examples thereof include a cyclopentenyl group, a cyclohexenyl group, and a cycloheptenyl group. The $C_3$-$C_{10}$ cycloalkenylene group as used herein refers to a divalent group having the same structure as the $C_3$-$C_{10}$ cycloalkenyl group.

The $C_1$-$C_{10}$ heterocycloalkenyl group as used herein refers to a monovalent cyclic group that has at least one hetero atom, for example, N, O, P, Si, B, Ti, S, Se, Te, Ge, or any combination thereof as a ring-forming atom, 2 to 10 carbon atoms, and at least one carbon-carbon double bond in its ring. Examples of the $C_1$-$C_{10}$ heterocycloalkenyl group include a 2,3-dihydrofuranyl group and a 2,3-dihydrothiophenyl group. The $C_2$-$C_{10}$ heterocycloalkenylene group as used herein refers to a divalent group having the same structure as the $C_2$-$C_{10}$ heterocycloalkenyl group.

The $C_6$-$C_{60}$ aryl group as used herein refers to a monovalent group having a carbocyclic aromatic system having 6 to 60 carbon atoms, and the $C_6$-$C_{60}$ arylene group as used herein refers to a divalent group having a carbocyclic aromatic system having 6 to 60 carbon atoms. Examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the two or more rings may be fused to each other.

The $C_7$-$C_{60}$ alkylaryl group as used herein refers to a $C_6$-$C_{60}$ aryl group that is substituted with at least one $C_1$-$C_{60}$ alkyl group.

The $C_1$-$C_{60}$ heteroaryl group as used herein refers to a monovalent group having a cyclic aromatic system that has at least one heteroatom, for example, **N, O,** P, Si, B, Ti, S, Se, Te, Ge, or any combination thereof as a ring-forming atom, and 1 to 60 carbon atoms. The $C_1$-$C_{60}$ heteroarylene group as used herein refers to a divalent group having a carbocyclic aromatic system that has at least one heteroatom, for example, N**,** O, P, Si, B, Ti, S, Se, Te, Ge, or any combination thereof as a ring-forming atom, and 1 to 60 carbon atoms. Examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_6$-$C_{60}$ heteroaryl group and the $C_6$-$C_{60}$ heteroarylene group each include two or more rings, the two or more rings may be fused to each other.

The $C_2$-$C_{60}$ alkylheteroaryl group as used herein refers to a $C_1$-$C_{60}$ heteroaryl group that is substituted with at least one $C_1$-$C_{60}$ alkyl group.

The $C_6$-$C_{60}$ aryloxy group as used herein indicates -$OA_{102}$ (wherein $A_{102}$ is the $C_6$-$C_{60}$ aryl group), and the $C_6$-$C_{60}$ arylthio group as used herein indicates -$SA_{103}$ (wherein $A_{103}$ is the $C_6$-$C_{60}$ aryl group).

The $C_1$-$C_{60}$ heteroaryloxy group used herein indicates -$OA_{104}$ (wherein $A_{104}$ is a $C_1$-$C_{60}$ heteroaryl group), and the $C_1$-$C_{60}$ heteroarylthio group indicates -$SA_{105}$ (wherein $A_{105}$ is the $C_1$-$C_{60}$ heteroaryl group).

The monovalent non-aromatic condensed polycyclic group as used herein refers to a monovalent group having two or more rings condensed with each other, only carbon atoms (for example, having 8 to 60 carbon atoms) as ring-forming atoms, and no aromaticity in its molecular structure when considered as a whole. Examples of the monovalent non-aromatic condensed polycyclic group include a fluorenyl group. The divalent non-aromatic condensed polycyclic group as used herein refers to a divalent group having the same structure as a monovalent non-aromatic condensed polycyclic group.

The monovalent non-aromatic condensed heteropolycyclic group as used herein refers to a monovalent group having two or more rings condensed with each other, a heteroatom, for example, N, O, P, Si, B, Ti, S, Se, Te, Ge, or any combination thereof, other than carbon atoms (for example, having 2 to 60 carbon atoms), as a ring-forming atom, and no aromaticity in its molecular structure when considered as a whole. Examples of the monovalent non-aromatic condensed heteropolycyclic group include a carbazolyl group. The divalent non-aromatic condensed heteropolycyclic group as used herein refers to a divalent group having the same structure as a monovalent non-aromatic condensed heteropolycyclic group.

The $C_5$-$C_{30}$ carbocyclic group as used herein refers to a saturated or unsaturated cyclic group having, as a ring-forming atom, 5 to 30 carbon atoms only. The $C_5$-$C_{30}$ carbocyclic group may be a monocyclic group or a polycyclic group. Examples of the "$C_5$-$C_{30}$ carbocyclic group (that is unsubstituted or substituted with at least one $R_{1a}$)" may include an adamantane group, a norbornene group, a bicyclo[1.1.1]pentane group, a bicyclo[2.1.1]hexane group, a bicyclo[2.2.1]heptane group (norbornane group), a bicyclo[2.2.2]octane group, a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, a cyclopentadiene group, a silole group, and a fluorene group (each unsubstituted or substituted with at least one $R_{1a}$).

The $C_1$-$C_{30}$ heterocyclic group as used herein refers to a saturated or unsaturated cyclic group having at least one heteroatom, for example, N, O, Si, P, B, Ti, S, Se, Te, Ge, or any combination thereof, other than 1 to 30 carbon atoms, as a ring-forming atom. The $C_1$-$C_{30}$ heterocyclic group may be a monocyclic group or a polycyclic group. Examples of the "$C_1$-$C_{30}$ heterocyclic group (that is unsubstituted or substituted with at least one $R_{1a}$)" may include a thiophene group, a furan group, a pyrrole group, a silole group, a borole group, a phosphole group, a selenophene group, a germole group, a benzothiophene group, a benzofuran group, an indole group, an indene group, a benzosilole group, a benzoborole group, a benzophosphole group, a benzoselenophene group, a benzogermole group, a dibenzothiophene group, a dibenzofuran group, a carbazole group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group (each unsubstituted or substituted with at least one $R_{1a}$).

Herein, TMS represents *-$Si(CH_3)_3$, and TMG represents *-$Ge(CH_3)_3$.

As used herein, the number of carbons in each group that is substituted (e.g., $C_1$-$C_{60}$) excludes the number of carbons in the substituent. For example, a $C_1$-$C_{60}$ alkyl group can be substituted with a $C_1$-$C_{60}$ alkyl group. The total number of carbons included in the $C_1$-$C_{60}$ alkyl group substituted with the $C_1$-$C_{60}$ alkyl group is not limited to 60 carbons. In addition, more than one $C_1$-$C_{60}$ alkyl substituent may be present on the $C_1$-$C_{60}$ alkyl group. This definition is not limited to the $C_1$-$C_{60}$ alkyl group and applies to all substituted groups that recite a carbon range.

At least one substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_1$-$C_{60}$ hetero-

aryloxy group, the substituted $C_1$-$C_{60}$ heteroarylthio group, the substituted monovalent non-aromatic condensed polycyclic group, and the substituted monovalent non-aromatic condensed heteropolycyclic group may be:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkyl aryl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-Si(Q_{11})(Q_{12})(Q_{13})$, $-Ge(Q_{11})(Q_{12})(Q_{13})$, $-N(Q_{14})(Q_{13})$, $-B(Q_{16})(Q_{17})$, $-P(=O)(Q_{18})(Q_{19})$, or any combination thereof;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, or a monovalent non-aromatic condensed heteropolycyclic group;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-Si(Q_{21})(Q_{22})(Q_{23})$, $-Ge(Q_{21})(Q_{22})(Q_{23})$, $-N(Q_{24})(Q_{25})$, $-B(Q_{26})(Q_{27})$, $-P(=O)(Q_{28})(Q_{29})$, or any combination thereof, or
$-Si(Q_{31})(Q_{32})(Q_{33})$, $-Ge(Q_{31})(Q_{32})(Q_{33})$, $-N(Q_{34})(Q_{35})$, $-B(Q_{36})(Q_{37})$, or $-P(=O)(Q_{38})(Q3_9)$,
wherein $Q_1$ to $Q_9$, $Q_{11}$ to $Q_{19}$, $Q_{21}$ to $Q_{29}$, and $Q_{31}$ to $Q_{39}$ may each independently be hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a $C_1$-$C_{60}$ heteroaryloxy group, a $C_1$-$C_{60}$ heteroarylthio group, a substituted or unsubstituted monovalent non-aromatic condensed polycyclic group, or a substituted or unsubstituted monovalent non-aromatic condensed heteropolycyclic group.

Hereinafter, a compound and an organic light-emitting device according to an embodiment are described in detail with reference to Synthesis Examples and Examples. However, the disclosure is not limited to the following Synthesis Examples and Examples. The wording "B was used instead of A" used in describing Synthesis Examples means that an amount of B used was identical to an amount of A used based on molar equivalence.

EXAMPLES

[Synthesis Example]

Synthesis Example 1: Synthesis of Compound 1

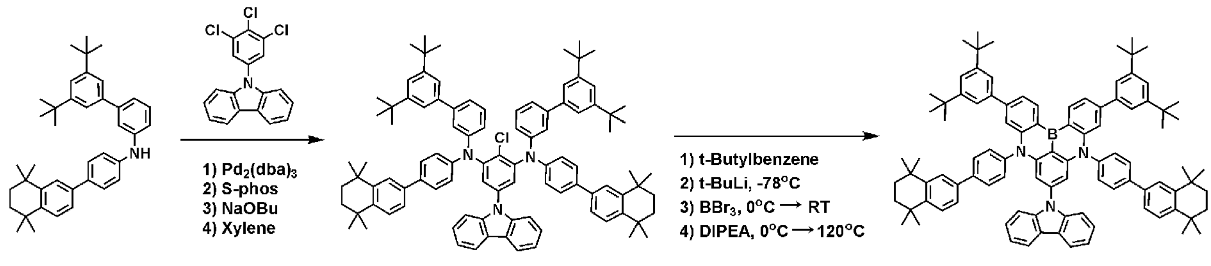


1-1

1

(1) Synthesis of Compound 1-1

Under nitrogen, 9-(3,4,5-trichlorophenyl)-9H-carbazole (3 g, 8.65 mmol), 3',5'-di-tert-butyl-N-(4-(5,5,8,8-tetramethyl-5,6,7,8-tetrahydronaphthalen-2-yl)phenyl)-[1,1'-biphenyl]-3-amine (9.69 g, 17.31 mmol), $Pd_2(dba)_3$ (0.79 g, 0.86 mmol), S-phos (0.710 g, 1.73 mmol), and Nat-OBu (2.91 g, 30.28 mmol) were added to 300 ml of xylene and refluxed for 30 minutes, and then a saturated ammonium chloride solution was used to terminate the reaction. An organic layer was extracted using ethyl acetate and then dried using $MgSO_4$. The dried organic layer was concentrated in vacuo to remove a solvent therefrom, followed by purification through column chromatography using methylene chloride (MC):hexane (1:3), to thereby obtain 10 g of Compound 1-1 which was a white solid.
LCMS (m/z) calculated: 1361.63 g/mol, found: [M+] 1359.81 g/mol

(2) Synthesis of Compound 1

Under nitrogen, Compound 1-1 (5 g, 3.6 mmol) was dissolved in 90 ml of t-butylbenzene, the mixture was cooled to -78 °C, t-BuLi (6.2 ml, 8.99 mmol) was added thereto, and then the mixture was stirred at 60 °C for 1 hour and 30 minutes. The mixture was cooled to 0 °C, boron tribromide (0.69 ml, 7.20 mmol) was added thereto, and then the mixture was stirred at room temperature for an hour and 30 minutes. The mixture was cooled to 0 °C, N,N-diisopropylethyl amine (1.22 ml, 7.20 mmol) was added thereto, the mixture was heated at 120 °C for 4 hours, and then a sodium acetate solution (1.0 M in water) was used to terminate the reaction. An organic layer was extracted and dried using $MgSO_4$. The dried organic layer was concentrated in vacuo to remove a solvent therefrom, followed by purification through column chromatography using MC:hexane (1:4), to thereby obtain 1 g of Compound 1 which was a solid. (yield: 25 %)
LCMS (m/z) calculated: 1334.694 g/mol, found: [M+] 1333.74 g/mol

[Examples]

Example 1

An ITO glass substrate was cut to a size of 50 mm x 50 mm x 0.5 mm and then, sonicated in acetone isopropyl alcohol and pure water, each for 15 minutes, and then, cleaned by exposure to UV ozone for 30 minutes.

Then, HAT-CN was deposited on the ITO electrode (anode) on the glass substrate to form a hole injection layer having a thickness of 100 Å, NPB was deposited on the hole injection layer to form a first hole transport layer having a thickness of 500 Å, TCTA was deposited on the first hole transport layer to form a second hole transport layer having a thickness of 50 Å, and mCP was deposited on the second hole transport layer to form an electron blocking layer having a thickness of 50 Å.

A first host (H16), a second host (H25), and an emitter (Compound 1) were co-deposited on the electron blocking layer to form an emission layer having a thickness of 400 Å. In this regard, the first host and the second host were mixed at a ratio of 60:40, and the emitter was adjusted to be 3 wt% based on the total weight of the first host, the second host, and the emitter.

DBFPO was deposited on the emission layer to form a hole blocking layer having a thickness of 100 Å, and DBFPO and LiQ were co-deposited thereon at a weight ratio of 5:5 to form an electron transport layer having a thickness of 300 Å, LiQ was deposited on the electron transport layer to form an electron injection layer having a thickness of 10 Å, and then Al was deposited on the electron injection layer to form a cathode having a thickness of 1,000 Å, thereby completing the manufacture of an organic light-emitting device.

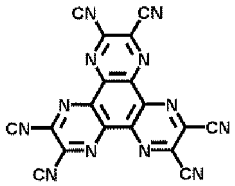

HT-D1(HAT-CN)

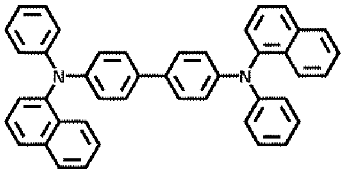

NPB

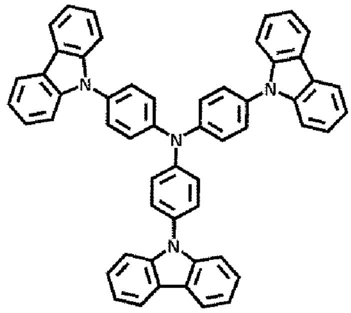

TCTA

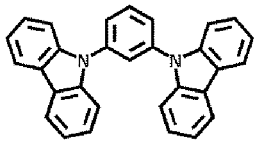

mCP

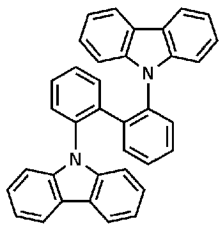

H16

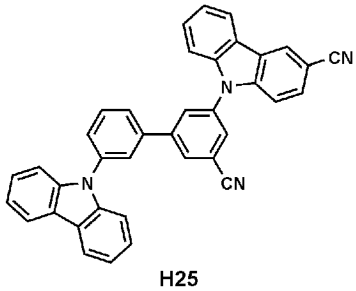

H25

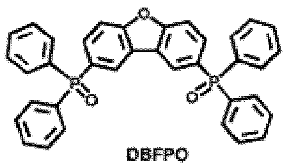

DBFPO

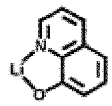

LiQ

Comparative Examples 1 and 2

Organic light-emitting devices were manufactured in the same manner as in Example 1, except that compounds shown in Table 2 were each used instead of the emitter in forming the emission layer.

The lifespan $T_{95}$ (at 1,200 cd/m$^2$, hr) FWHM (nm) of EL spectrum, and external quantum efficiency (EQE, %) of the organic light-emitting devices manufactured in Example 1 and Comparative Examples 1 and 2 were measured and evaluated using a current-voltage meter (Keithley 2400) and a luminance meter (Minolta Cs-1000A). The lifespan $T_{95}$ (at 1,200 cd/m$^2$, hr) is a time taken for the luminance of the organic light-emitting devices to reduce from initial luminance of 100% to 95% thereof. The results are shown in Table 2.

Table 2

| | Emitter in emission layer | FWHM (nm) | EQE (%) | $T_{95}$ (hr) |
|---|---|---|---|---|
| Example 1 | Compound 1 | 21 | 21.1 | 48 |
| Comparative Example 1 | Compound A | 22 | 16.3 | 6.1 |
| Comparative Example 2 | Compound B | 23 | 20.7 | 7.1 |

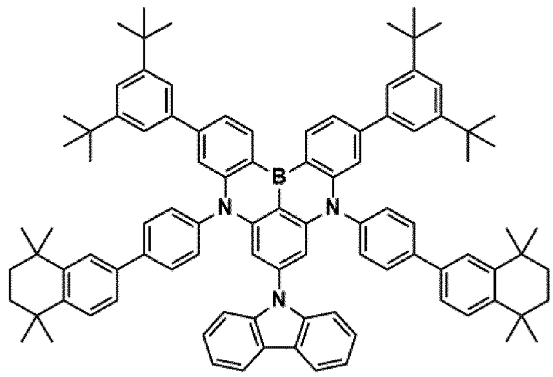

1

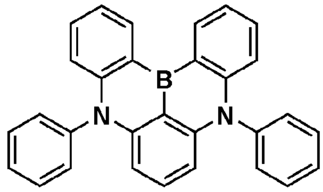

A

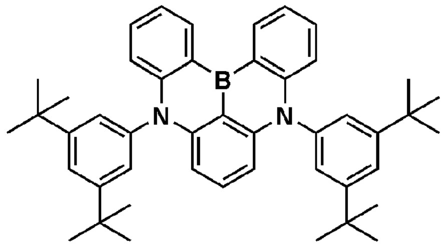

B

From Table 2, it could be seen that the organic light-emitting device according to an embodiment had excellent emission efficiency, FWHM, and lifespan characteristics. In addition, it could be seen that the organic light-emitting device of Example 1 had higher external quantum efficiency, narrower FWHM, and significantly longer lifespan than those of the organic light-emitting devices of Comparative Examples 1 and 2.

According to the one or more embodiments, a heterocyclic compound has excellent luminescent characteristics and charge mobility characteristics, and thus, an electronic device, for example, an organic light-emitting device, including the heterocyclic compound may have low driving voltage, high efficiency, and long lifespan characteristics. Therefore, a high-quality organic light-emitting device may be implemented using the heterocyclic compound.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. A heterocyclic compound represented by Formula 1:

Formula 1

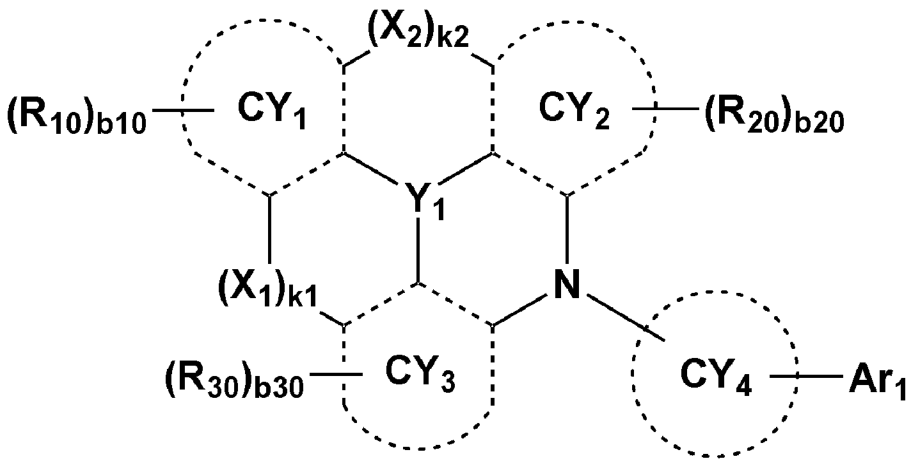

Formula 2

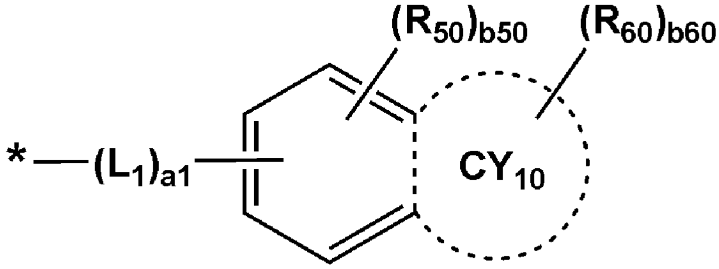

wherein, in Formulae 1 and 2,

$Y_1$ is B, N, P, P(=O), or P(=S),
$X_1$ and $X_2$ are each independently a single bond, O, S, Se, N[($CY_5$)-$Ar_2$], N($R_1$), C($R_1$)($R_2$), Si($R_1$)($R_2$), Ge($R_1$)($R_2$), B($R_1$), P($R_1$), P(=O)($R_1$), S(=O)$_2$, or C(=O),
k1 is 0 or 1,
k2 is 0 or 1,
the sum of k1 and k2 is greater than or equal to 1,
$CY_1$ to $CY_3$ are each independently a $C_5$-$C_{30}$ carbocyclic group or a substituted or unsubstituted $C_1$-$C_{30}$ heterocyclic group,
$CY_4$ and $CY_5$ are each independently a $C_5$-$C_{30}$ carbocyclic group that is unsubstituted or substituted with at least one $R_{40}$ or a $C_1$-$C_{30}$ heterocyclic group that is unsubstituted or substituted with at least one $R_{40}$,
$Ar_1$ and $Ar_2$ are each independently a group represented by Formula 2,
$L_1$ is a single bond, a substituted or unsubstituted $C_5$-$C_{30}$ carbocyclic group or a substituted or unsubstituted $C_1$-$C_{30}$ heterocyclic group,
a1 is 1, 2, or 3,
$CY_{10}$ is a $C_3$-$C_{10}$ non-aromatic carbocyclic group or a $C_1$-$C_{10}$ non-aromatic heterocyclic group,
$R_1$, $R_2$, $R_{10}$, $R_{20}$, $R_{30}$, and $R_{40}$ are each independently a group represented by Formula 2, hydrogen, deuterium, -F, -Cl, -Br, -I, -$SF_5$, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_1$)($Q_2$)($Q_3$), -Ge($Q_1$)($Q_2$)($Q_3$), - C(=O)($Q_1$), -S(=O)($Q_1$), -S(=O)$_2$($Q_1$), -B($Q_1$)($Q_2$), -P($Q_1$)($Q_2$), -P(=O)($Q_1$)($Q_2$), - P(=S)($Q_1$)($Q_2$), or a group represented by Formula 3,
$R_{50}$ and $R_{60}$ are each independently hydrogen, deuterium, -F, -Cl, -Br, -I, -$SF_5$, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_1$)($Q_2$)($Q_3$), -Ge($Q_1$)($Q_2$)($Q_3$), - C(=O)($Q_1$), -S(=O)($Q_1$), -S(=O)$_2$($Q_1$), -B($Q_1$)($Q_2$), -P($Q_1$)($Q_2$), -P(=O)($Q_1$)($Q_2$), - P(=S)($Q_1$)($Q_2$), or a group represented by Formula 3,
wherein at least one of $R_{10}$, at least one of $R_{20}$, and/or at least one of $R_{30}$ is a group represented by Formula 3;

Formula 3

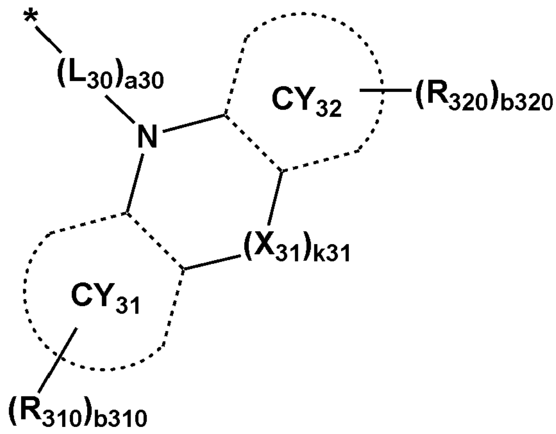

wherein, in Formula 3,

$X_{31}$ is a single bond, O, S, $N(R_{301})$, or $C(R_{301})(R_{302})$,
k31 is 1,
$CY_{31}$ and $CY_{32}$ are each independently a $C_5$-$C_{30}$ carbocyclic group or a $C_1$-$C_{30}$ heterocyclic group,
$L_{30}$ is a single bond, a substituted or unsubstituted $C_5$-$C_{30}$ carbocyclic group or a substituted or unsubstituted $C_1$-$C_{30}$ heterocyclic group,
a30 is 0, 1, 2, or 3,
$R_{301}$, $R_{302}$, $R_{310}$, and $R_{320}$ are each independently hydrogen, deuterium, -F, -Cl, -Br, -I, -SFs, a hydroxyl group, a cyano group, a nitro group, an amidino group, a hydrazino group, a hydrazono group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkylaryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkylheteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_1)(Q_2)(Q_3)$, $-Ge(Q_1)(Q_2)(Q_3)$, $-C(=O)(Q_1)$, $-S(=O)(Q_1)$, $-S(=O)_2(Q_1)$, $-B(Q_1)(Q_2)$, $-P(Q_1)(Q_2)$, $-P(=O)(Q_1)(Q_2)$, or $-P(=S)(Q_1)(Q_2)$,
b310 and b320 are each independently 1, 2, 3, 4, 5, 6, 7, or 8,
b10, b20, b30, b40, and b60 are each independently 1, 2, 3, 4, 5, 6, 7, or 8,
b50 is 1, 2, or 3,
a substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkylaryl group, the substituted $C_6$-$C_{60}$ aryloxy group, the substituted $C_6$-$C_{60}$ arylthio group, the substituted $C_1$-$C_{60}$ heteroaryl group, the $C_2$-$C_{60}$ alkylheteroaryl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each

substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, - $N(Q_{11})(Q_{12})$, $-Si(Q_{11})(Q_{12})(Q_{13})$, $-Ge(Q_{11})(Q_{12})(Q_{13})$, $-C(=O)(Q_{11})$, $-S(=O)(Q_{11})$, - $S(=O)_2(Q_{11})$, $-B(Q_{11})(Q_{12})$, $-P(Q_{11})(Q_{12})$, $-P(=O)(Q_{11})(Q_{12})$, $-P(=S)(Q_{11})(Q_{12})$, or any combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{21})(Q_{22})$, $-Si(Q_{21})(Q_{22})(Q_{23})$, $-Ge(Q_{21})(Q_{22})(Q_{23})$, - $C(=O)(Q_{21})$, $-S(=O)(Q_{21})$, $-S(=O)_2(Q_{21})$, $-B(Q_{21})(Q_{22})$, $-P(Q_{21})(Q_{22})$, $-P(=O)(Q_{21})(Q_{22})$, - $P(=S)(Q_{21})(Q_{22})$, or any combination thereof;

$-N(Q_{31})(Q_{32})$, $-Si(Q_{31})(Q_{32})(Q_{33})$, $-Ge(Q_{31})(Q_{32})(Q_{33})$, $-C(=O)(Q_{31})$, $-S(=O)(Q_{31})$, $-S(=O)_2(Q_{31})$, $-B(Q_{31})(Q_{32})$, $-P(Q_{31})(Q_{32})$, $-P(=O)(Q_{31})(Q_{32})$, or $-P(Q_{31})(Q_{32})$;

$Q_1$ to $Q_3$, $Q_{11}$ to $Q_{13}$, $Q_{21}$ to $Q_{23}$, and $Q_{31}$ to $Q_{33}$ are each independently: hydrogen; deuterium; -F; -Cl; -Br; -I; a hydroxyl group; a cyano group; a nitro group; an amidino group; a hydrazine group; a hydrazone group; a carboxylic acid or a salt thereof; a sulfonic acid or a salt thereof; a phosphoric acid or a salt thereof; a $C_1$-$C_{60}$ alkyl group that is unsubstituted or substituted with deuterium, a $C_1$-$C_{60}$ alkyl group, a $C_6$-$C_{60}$ aryl group, or any combination thereof; a $C_2$-$C_{60}$ alkenyl group; a $C_2$-$C_{60}$ alkynyl group; a $C_1$-$C_{60}$ alkoxy group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group that is unsubstituted or substituted with deuterium, a $C_1$-$C_{60}$ alkyl group, a $C_6$-$C_{60}$ aryl group, or any combination thereof; a $C_6$-$C_{60}$ aryloxy group; a $C_6$-$C_{60}$ arylthio group; a $C_1$-$C_{60}$ heteroaryl group; a monovalent non-aromatic condensed polycyclic group; or a monovalent non-aromatic condensed heteropolycyclic group, and

* indicates a binding site to a neighboring atom.

2. The heterocyclic compound of claim 1, wherein the heterocyclic compound is represented by Formula 1A or 1B:

Formula 1A

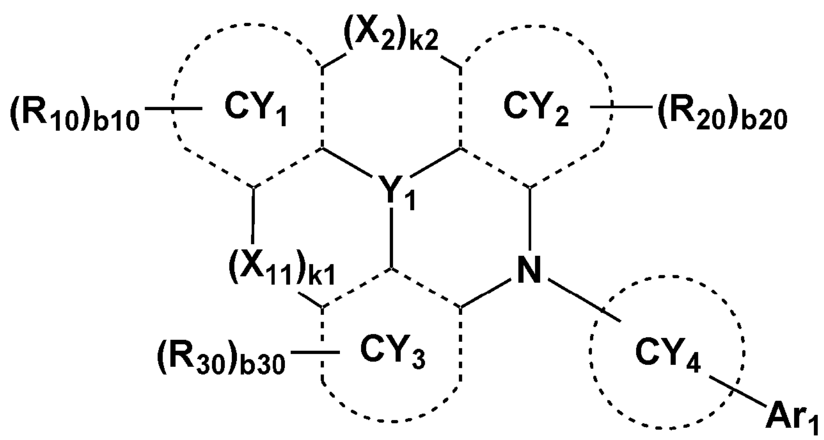

Formula 1B

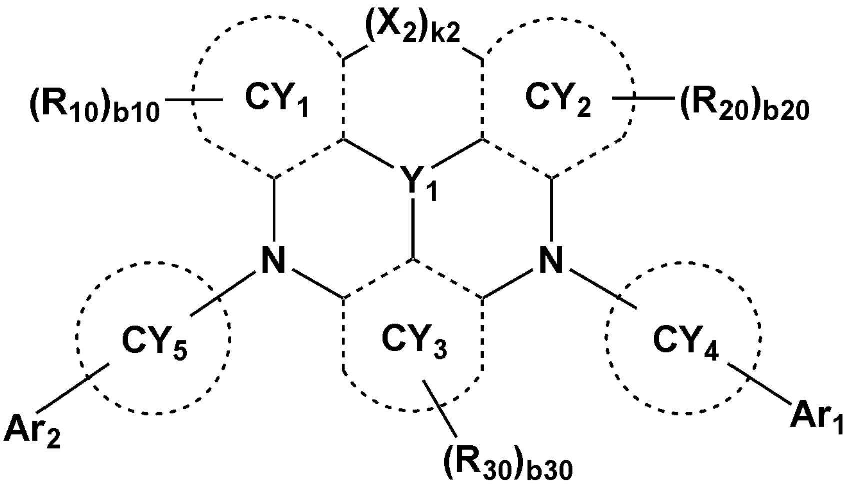

wherein, in Formulae 1A and 1B,

$X_{11}$ is a single bond, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$, or C(=O), and
$Y_1$, $X_2$, k1, k2, $CY_1$ to CYs, $Ar_1$, $Ar_2$, $R_1$, $R_2$, $R_{10}$, $R_{20}$, $R_{30}$, b10, b20, and b30 may each be the same as described in claim 1.

**3.** The heterocyclic compound of claims 1 or 2, wherein $CY_1$ to $CY_3$ are each independently a $C_6$-$C_{30}$ aromatic carbocyclic group or a $C_1$-$C_{30}$ aromatic heterocyclic group; and/or

wherein $CY_4$ and CYs are each independently represented by one of Formulae 4-1 to 4-3:

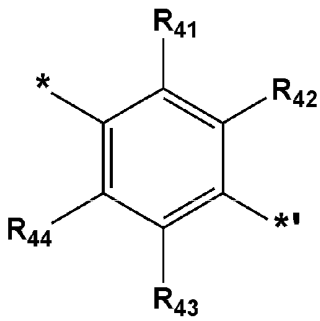

4-1

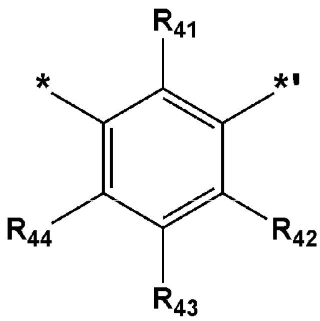

4-2

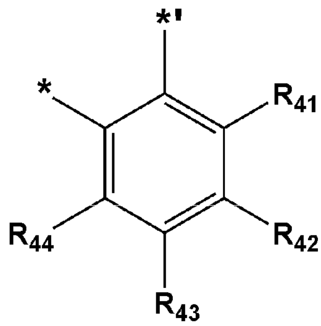

4-3

wherein, in Formulae 4-1 to 4-3,
$R_{41}$ to $R_{44}$ are each independently the same as described in connection with $R_{40}$ in claim 1, and
* and *' each indicate a binding site to a neighboring atom.

**4.** The heterocyclic compound of any of claims 1-3, wherein $CY_{10}$ is a cyclopentane group, a cyclohexane group, a cycloheptane group, a cyclopentene group, a cyclohexene group, or a cycloheptene group; and/or

wherein $Ar_1$ and $Ar_2$ are each independently a group represented by Formula 2A or 2B:

Formula 2A

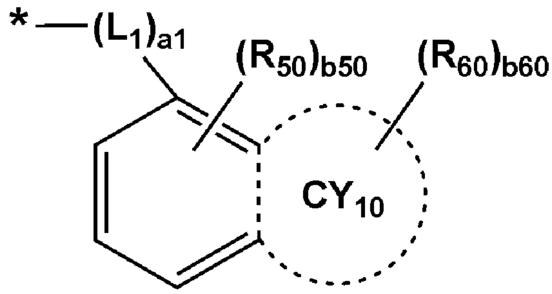

Formula 2B

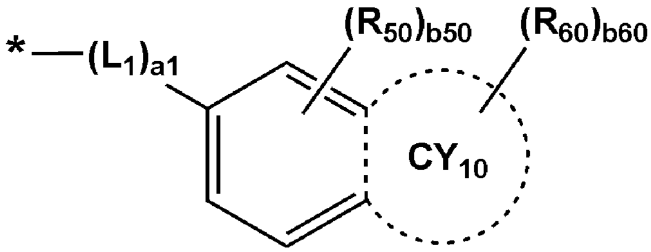

wherein, in Formulae 2A and 2B,
each of $L_1$, a1, $CY_{10}$, $R_{50}$, $R_{60}$, b50, and b60 is the same as described in claim 1, and
* indicates a binding site to a neighboring atom.

**5.** The heterocyclic compound of any of claims 1-4, wherein $Ar_1$ and $Ar_2$ are each independently a group represented by one of Formulae 5-1 to 5-4:

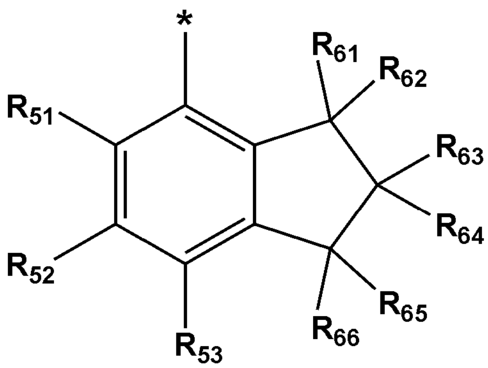

5-1

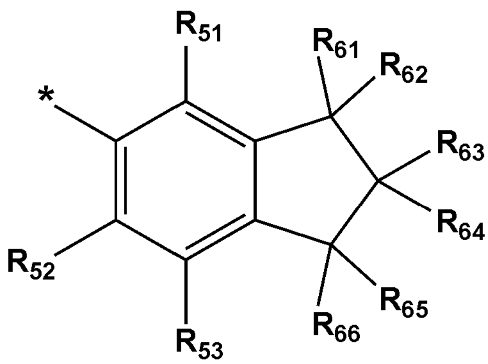

5-2

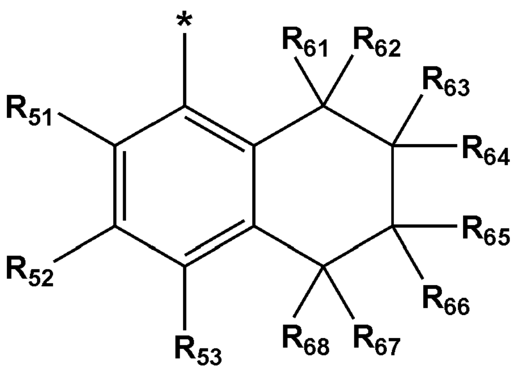

5-3

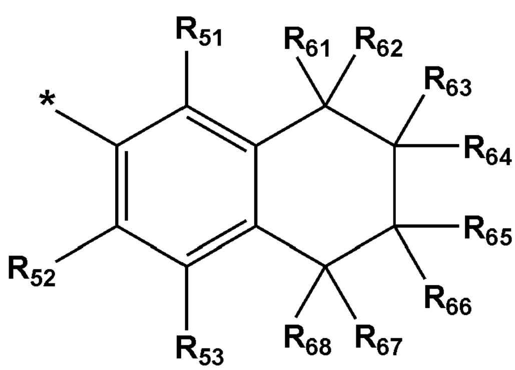

5-4

wherein, in Formulae 5-1 to 5-4,

$R_{51}$ to $R_{53}$ are each the same as described in connection with $R_{50}$ in claim 1,
$R_{61}$ to $R_{68}$ are each the same as described in connection with $R_{60}$ in claim 1, and
* indicates a binding site to a neighboring atom.

**6.** The heterocyclic compound of any of claims 1-5, wherein a group represented by Formula 3 is a group represented by one of Formulae 3-2 or 3-3:

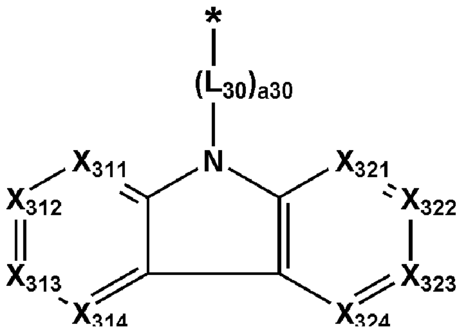

3-2

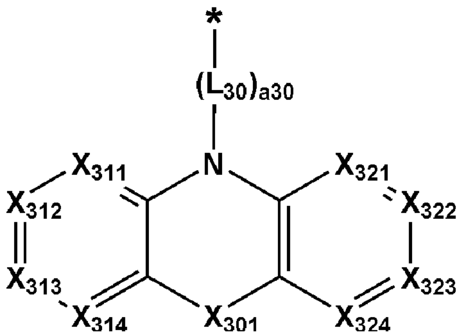

3-3

wherein, in Formulae 3-2 or 3-3,

$L_{30}$ and a30 are each the same as described in claim 1, $X_{301}$ is the same as described in connection with X31 in claim 1,
$X_{311}$ is C($R_{311}$) or N, $X_{312}$ is C($R_{312}$) or N, $X_{313}$ is C($R_{313}$) or N, $X_{314}$ is C($R_{314}$) or N,
$X_{321}$ is C($R_{321}$) or N, $X_{322}$ is C($R_{322}$) or N, $X_{323}$ is C($R_{323}$) or N, $X_{324}$ is C($R_{324}$) or N,
$R_{311}$ to $R_{314}$ are each independently the same as described in connection with $R_{310}$ in claim 1,
$R_{321}$ to $R_{324}$ are each independently the same as described in connection with $R_{320}$ in claim 1, and
* indicates a binding site to a neighboring atom.

**7.** The heterocyclic compound of any of claims 1-6, wherein the heterocyclic compound is represented by one of Formulae 11-1 to 11-9:

Formula 11-1

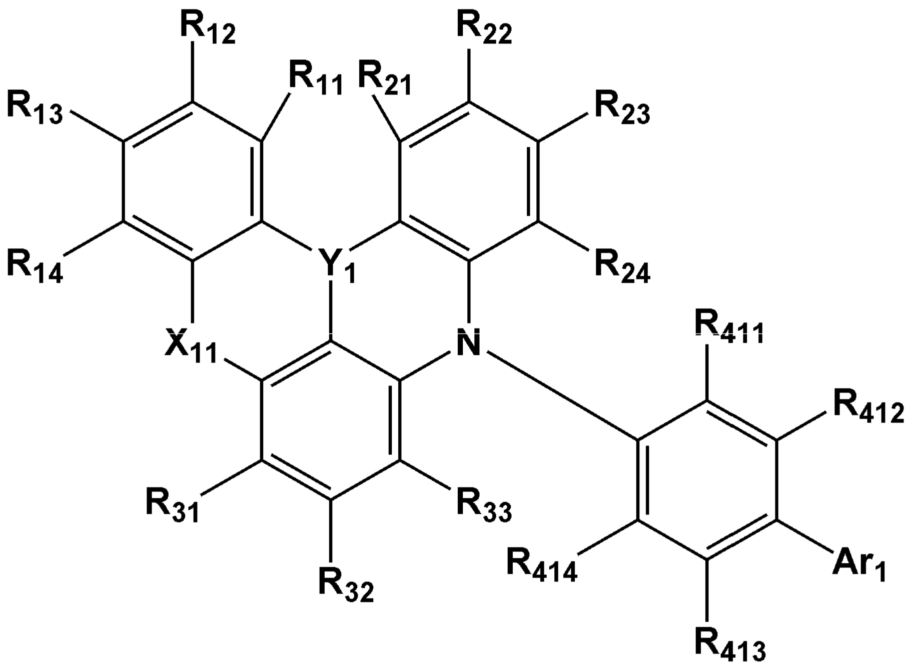

Formula 11-2

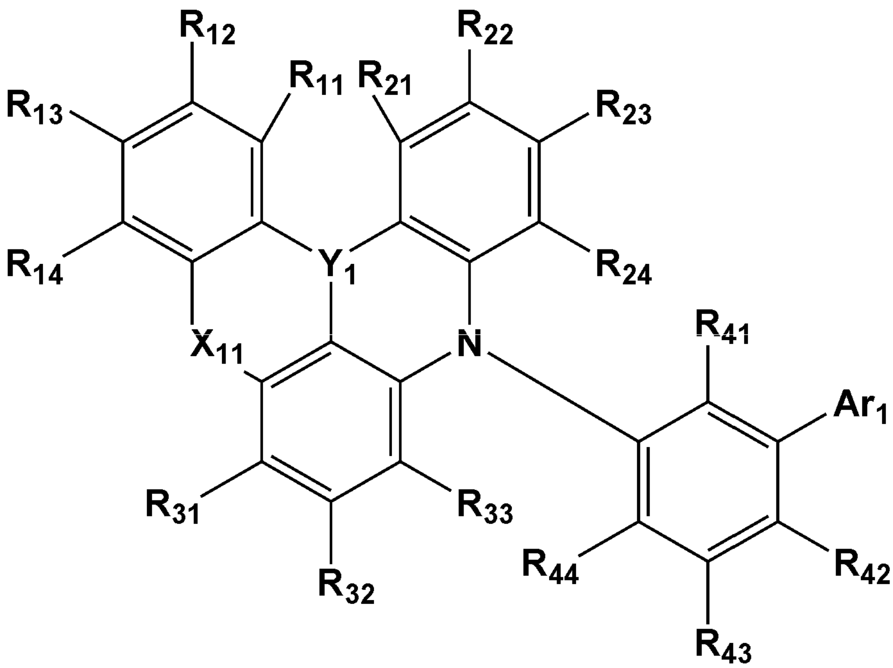

Formula 11-3

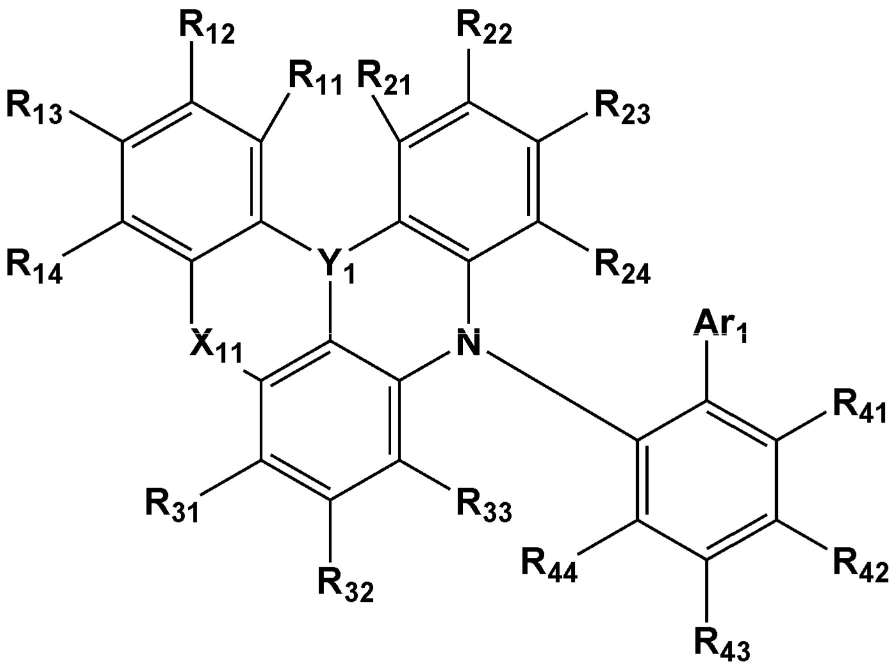

Formula 11-4

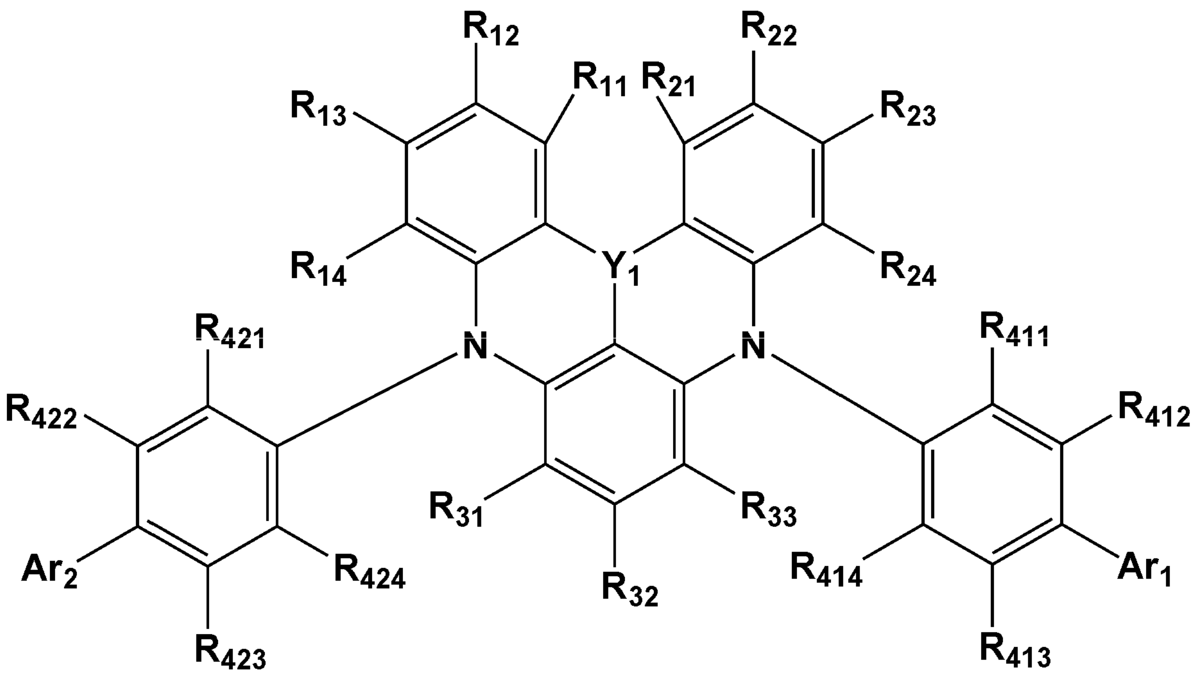

Formula 11-5

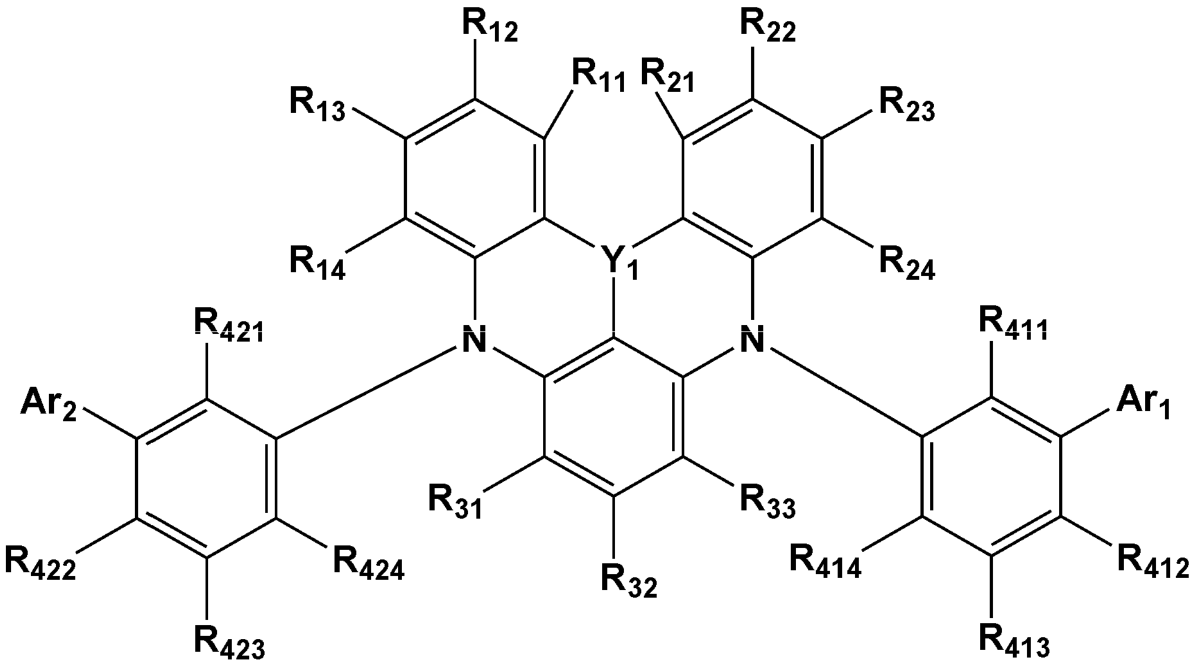

Formula 11-6

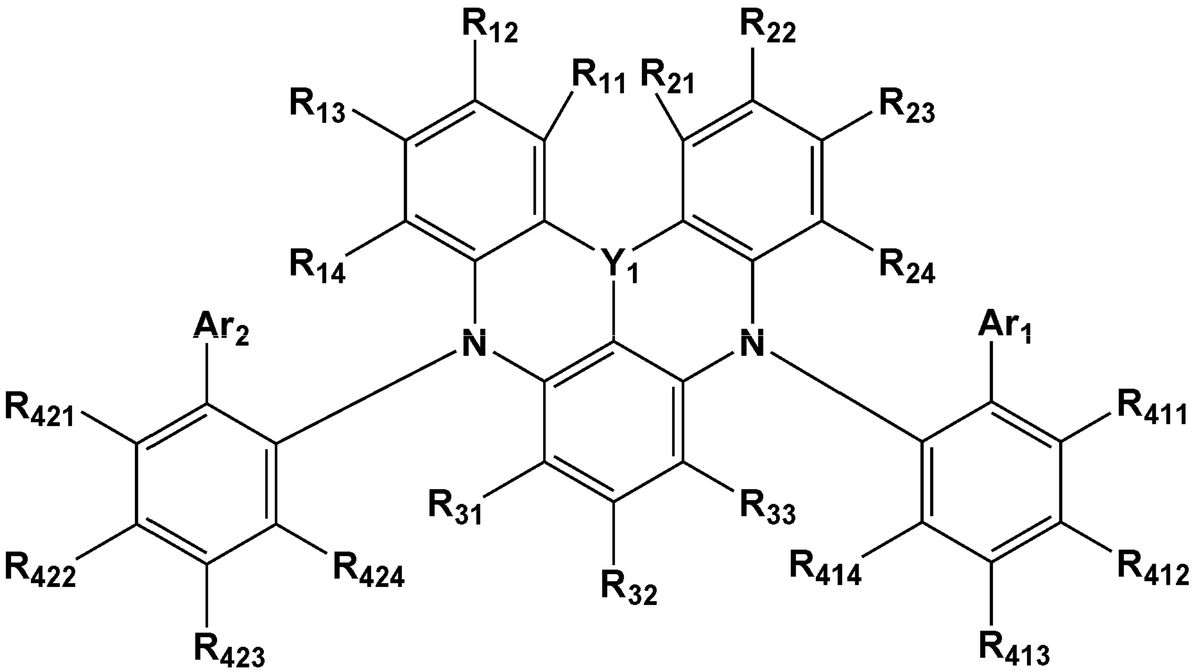

Formula 11-7

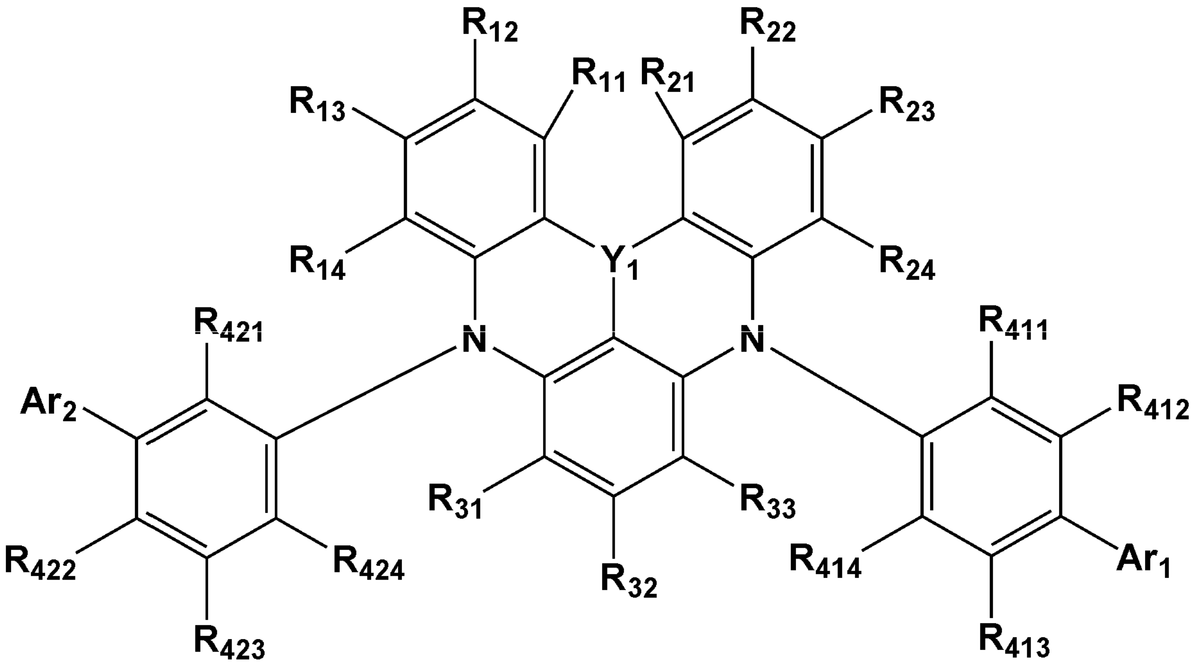

Formula 11-8

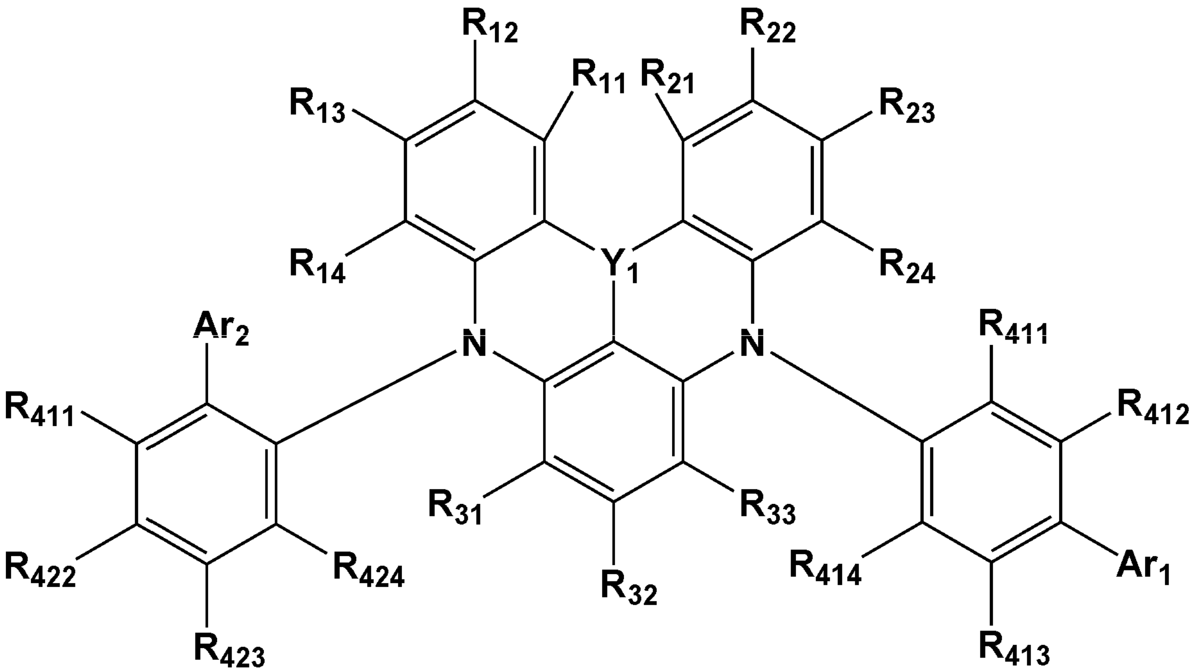

Formula 11-9

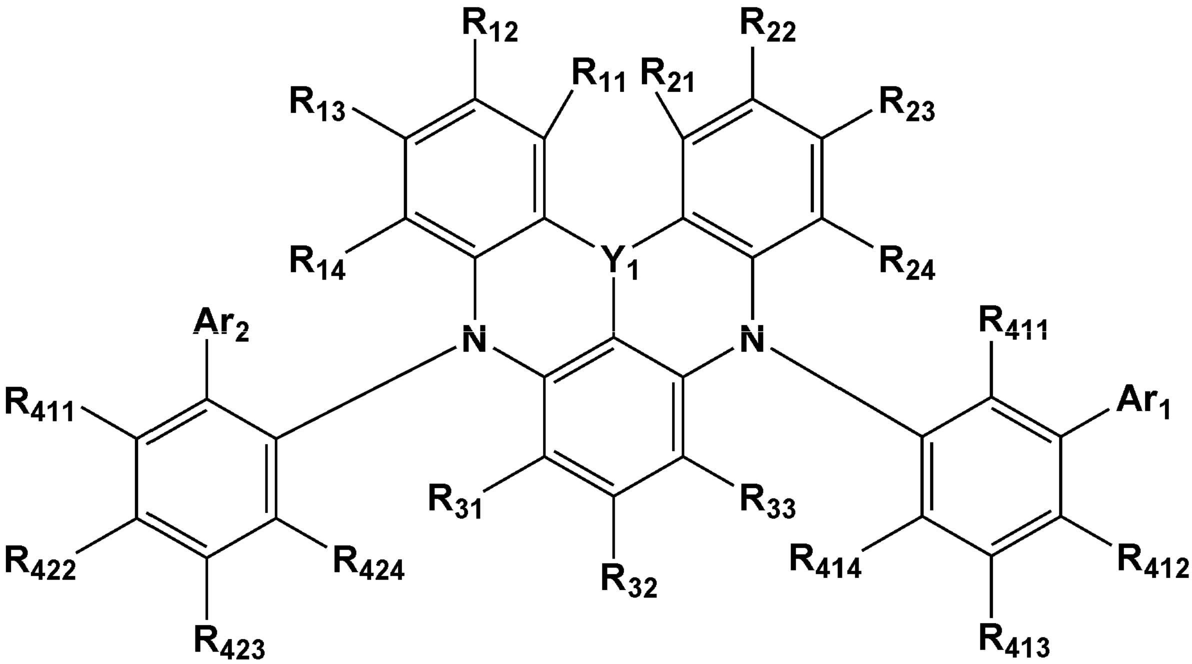

wherein, in Formulae 11-1 to 11-9,

$Y_1$, $Ar_1$, and $Ar_2$ are each the same as described in claim 1,
$X_{11}$ is a single bond, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$, or $C(=O)$, $R_1$ and $R_2$ are the same as described in claim 1,
$R_{11}$ to $R_{14}$ are each independently the same as described in connection with $R_{10}$ in claim 1,
$R_{21}$ to $R_{24}$ are each independently the same as described in connection with $R_{20}$ in claim 1,
$R_{31}$ to $R_{33}$ are each independently the same as described in connection with $R_{30}$ in claim 1, and
$R_{411}$ to $R_{414}$ and $R_{421}$ to $R_{424}$ are each independently the same as described in connection with $R_{40}$ in claim 1.

**8.** The heterocyclic compound of any of claims 1-7, wherein the heterocyclic compound has a symmetric structure or an asymmetric structure.

**9.** The heterocyclic compound of any of claims 1-8, wherein the heterocyclic compound is one of Compounds 1 to 792:

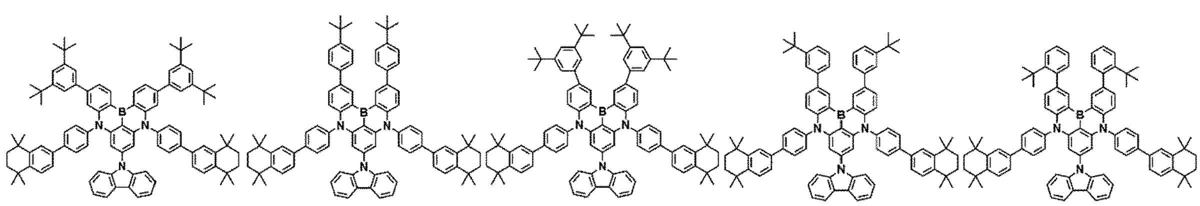

1 2 3 4 5

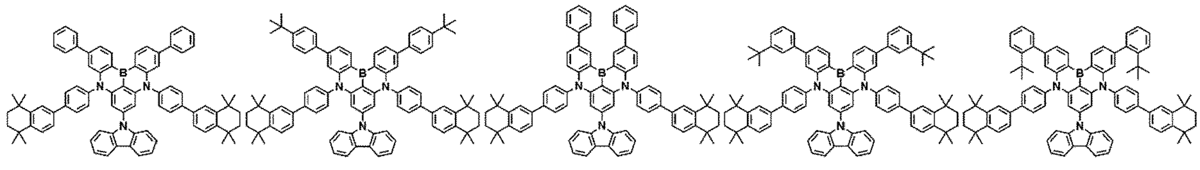

6 7 8 9 10

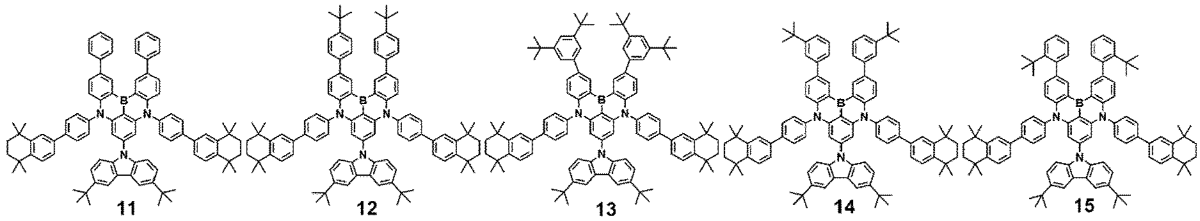

11 12 13 14 15

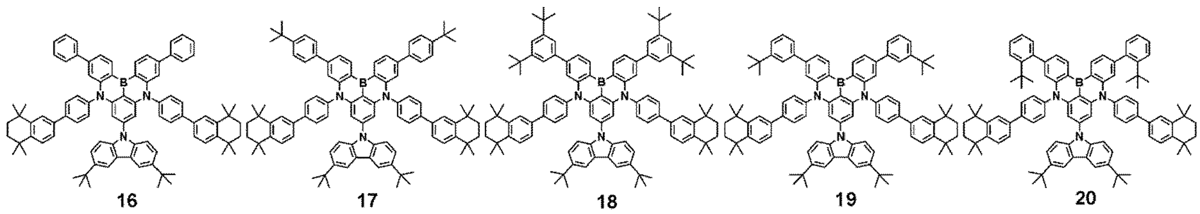

16 17 18 19 20

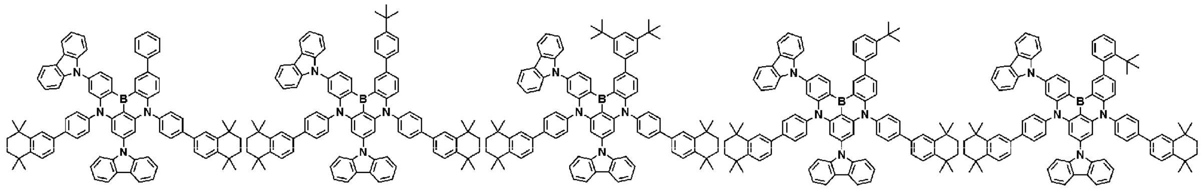

21 22 23 24 25

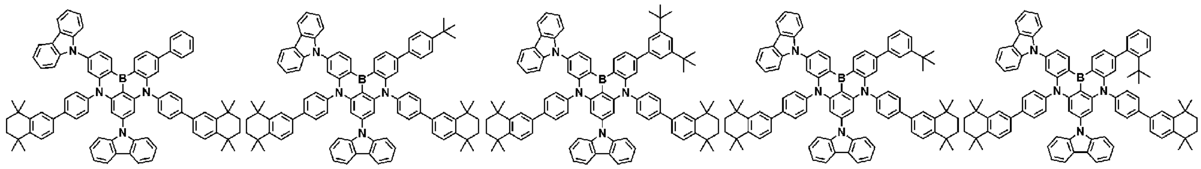

26 27 28 29 30

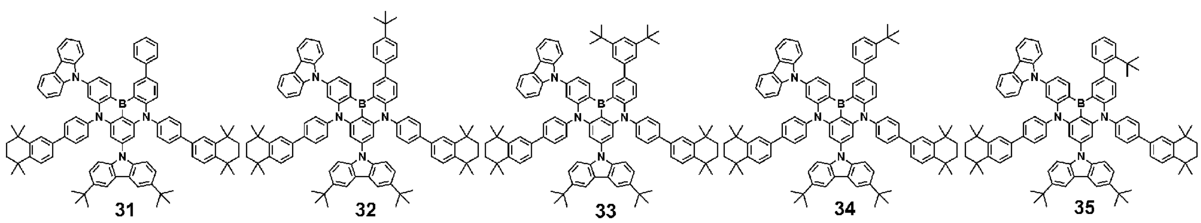

31 32 33 34 35

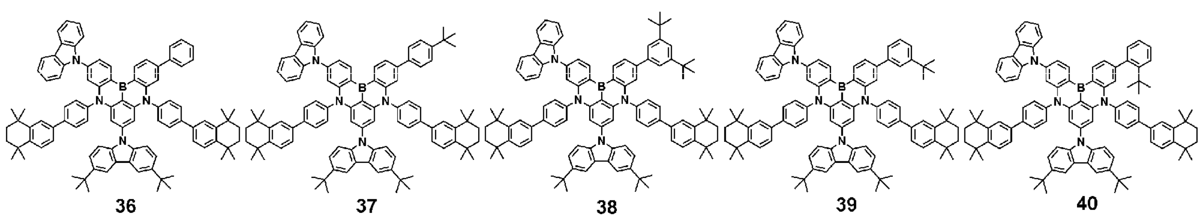

36 37 38 39 40

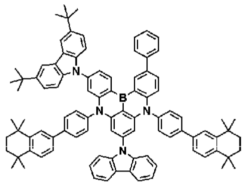
41
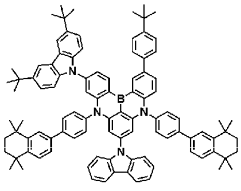
42
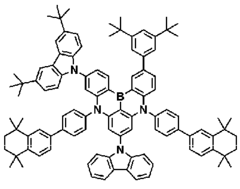
43
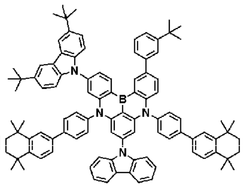
44
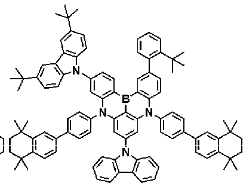
45

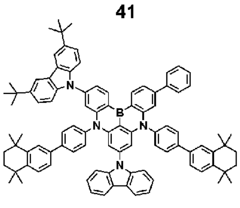
46
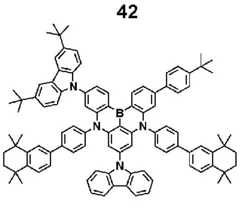
47
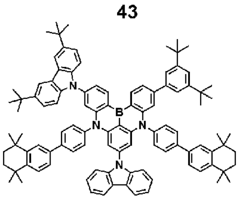
48
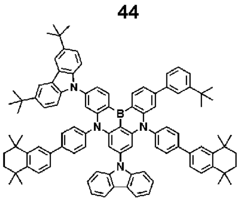
49
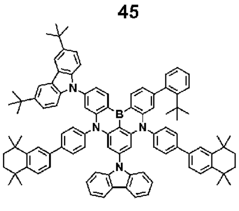
50

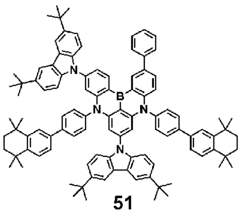
51
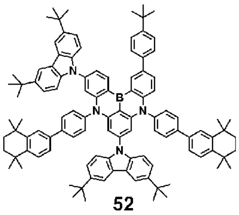
52
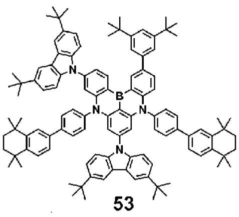
53
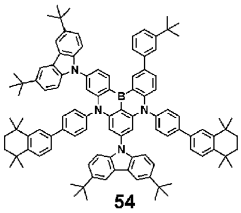
54
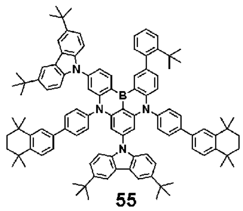
55

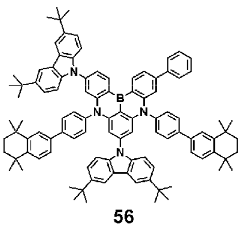
56
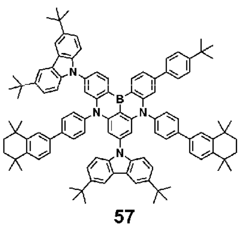
57
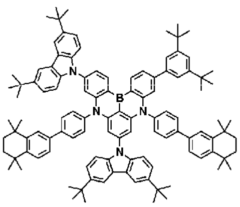
58
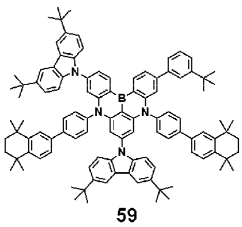
59
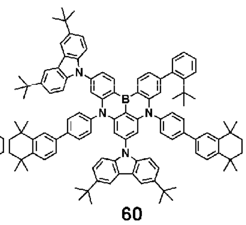
60

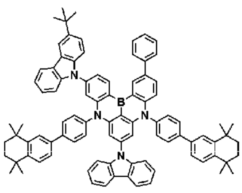
61
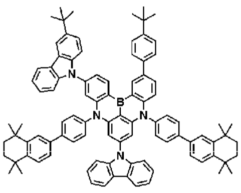
62
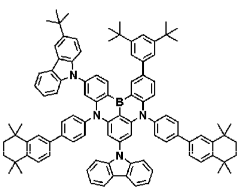
63
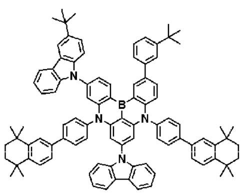
64
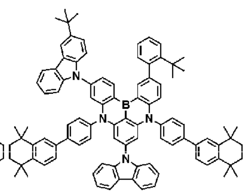
65

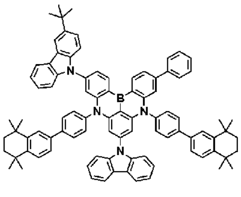
66
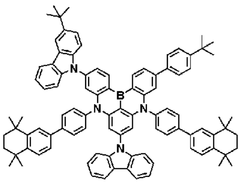
67
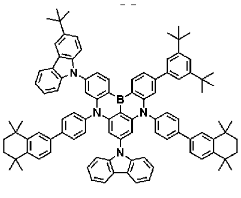
68
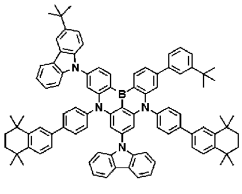
69
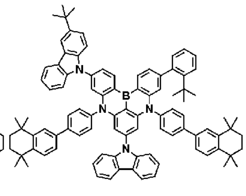
70

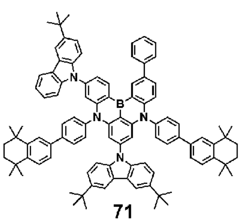
71
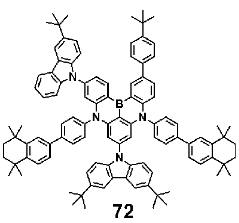
72
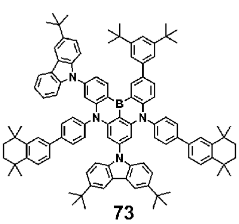
73
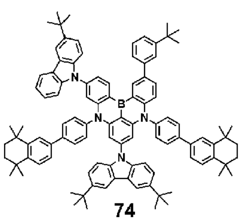
74
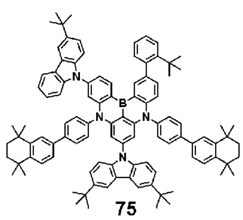
75

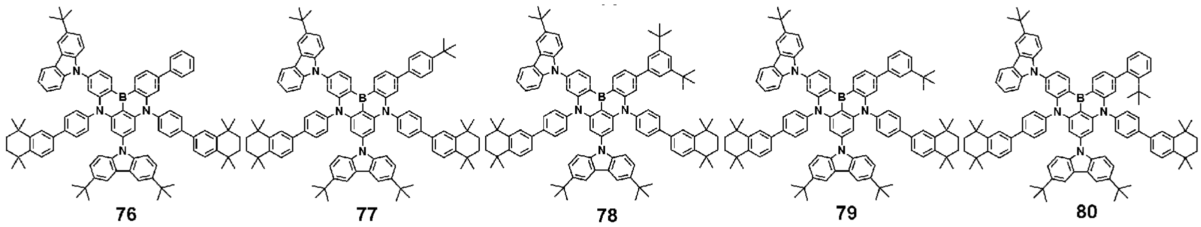

76 77 78 79 80

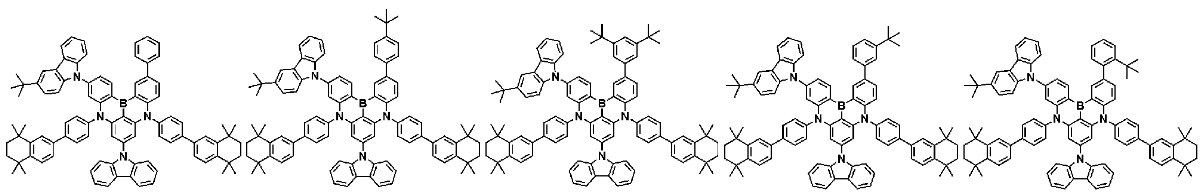

81 82 83 84 85

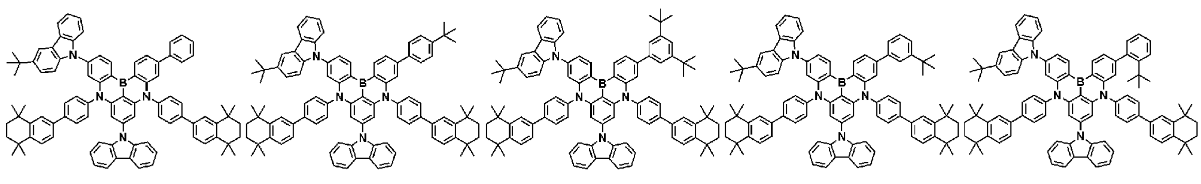

86 87 88 89 90

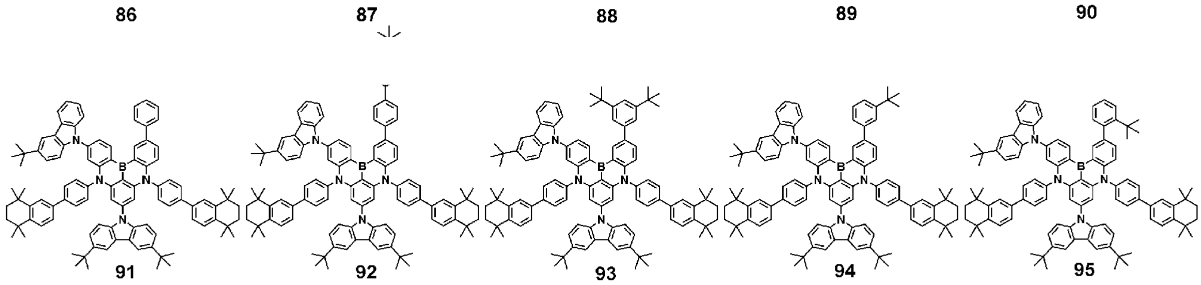

91 92 93 94 95

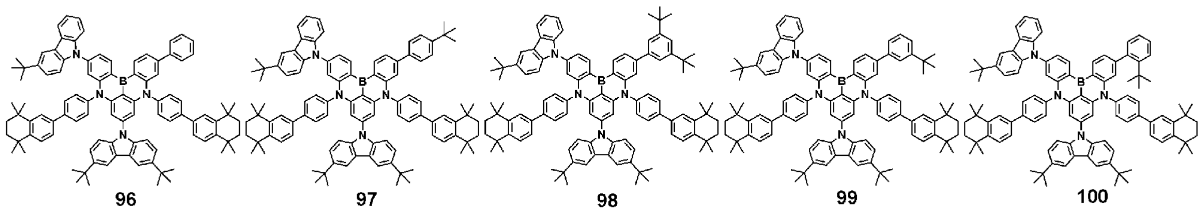

96 97 98 99 100

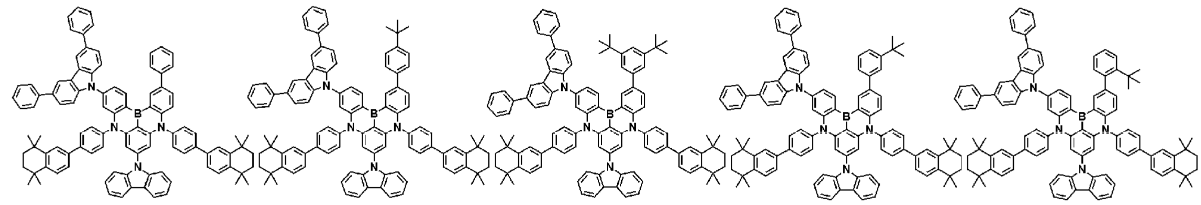

101 102 103 104 105

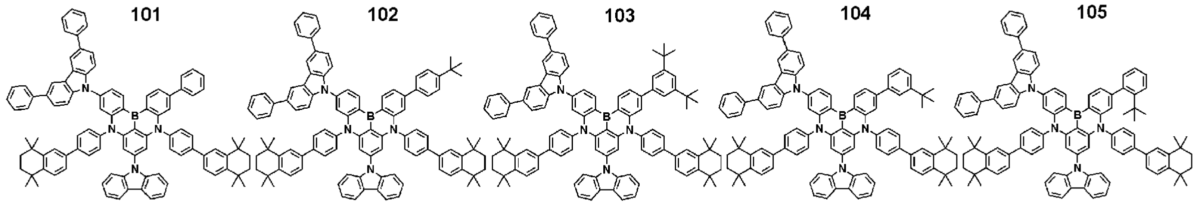

106 107 108 109 110

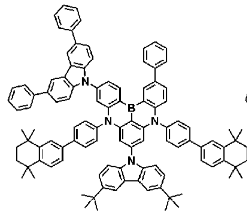
111
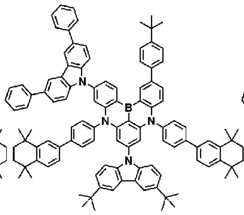
112
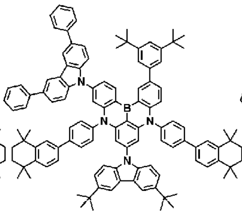
113
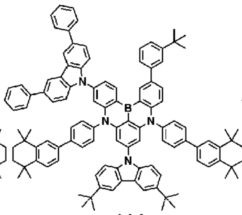
114
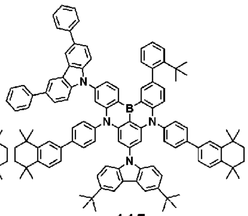
115
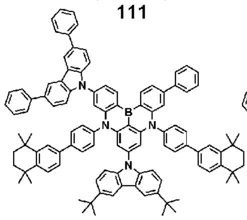
116
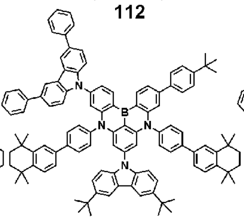
117
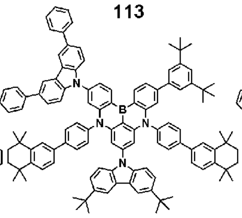
118
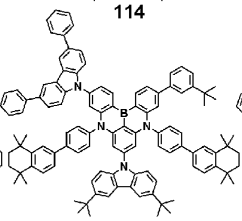
119
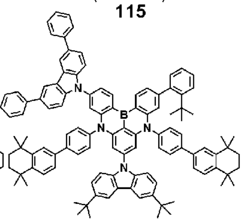
120
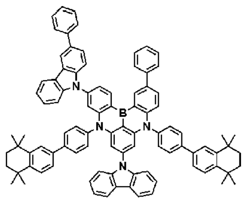
121
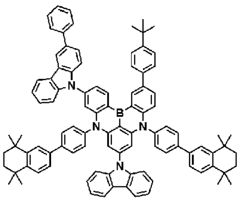
122
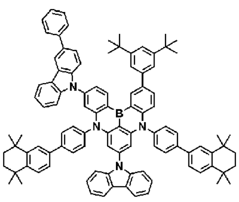
123
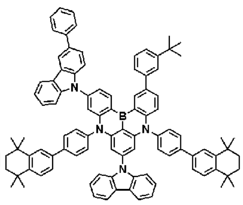
124
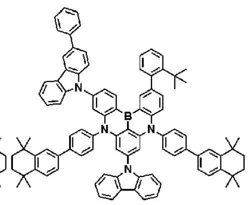
125
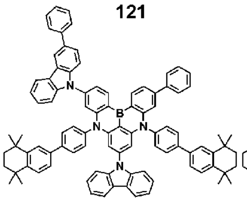
126
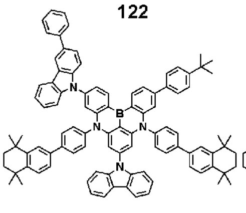
127
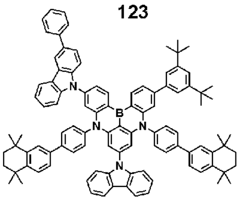
128
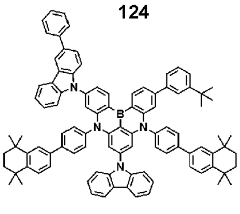
129
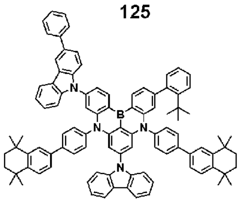
130
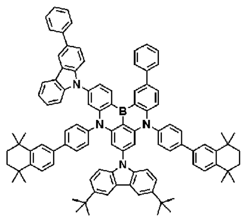
131
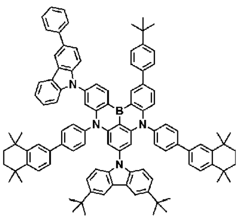
132
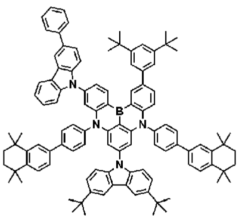
133
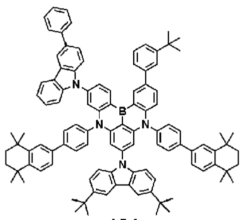
134
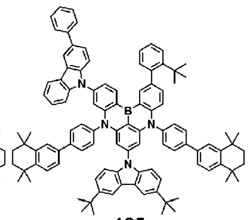
135
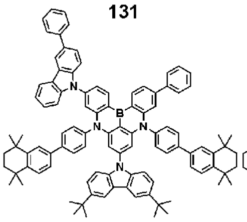
136
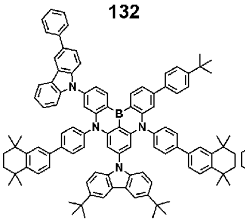
137
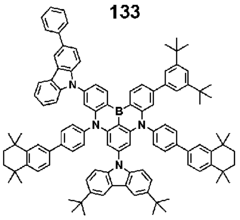
138
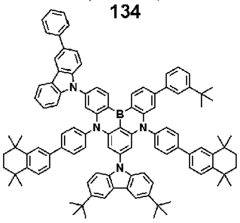
139
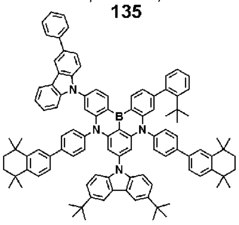
140
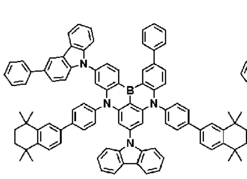
141
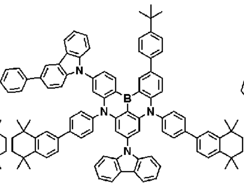
142
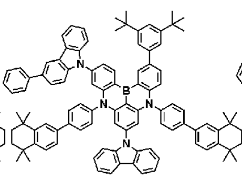
143
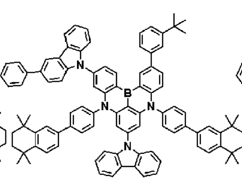
144
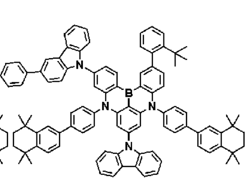
145

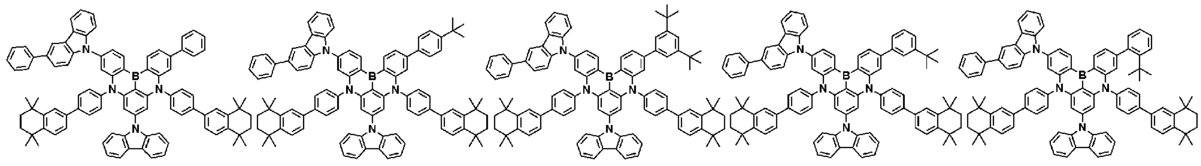

146 147 148 149 150

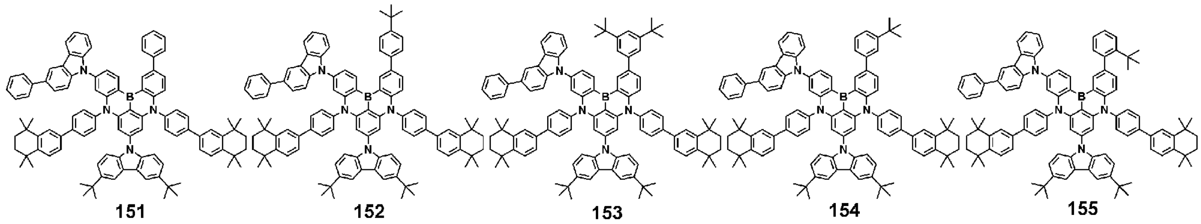

151 152 153 154 155

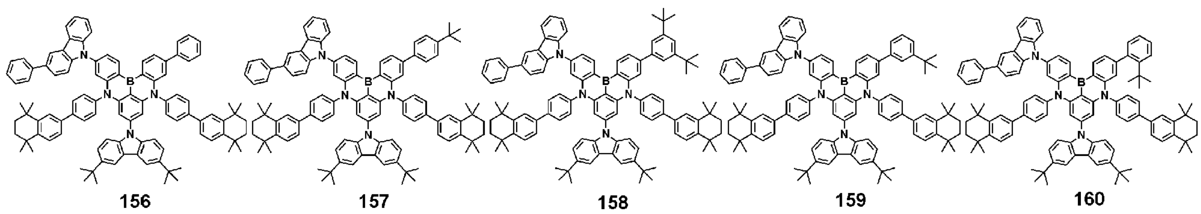

156 157 158 159 160

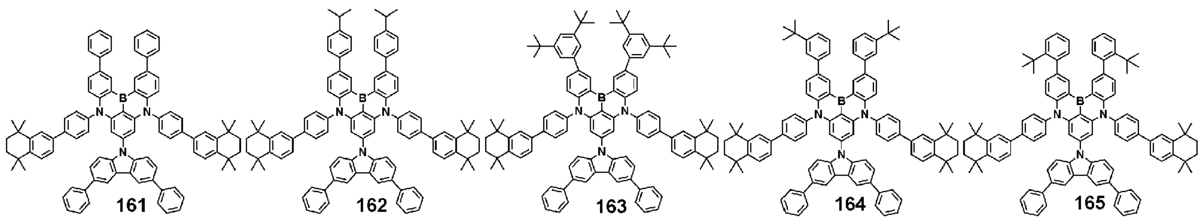

161 162 163 164 165

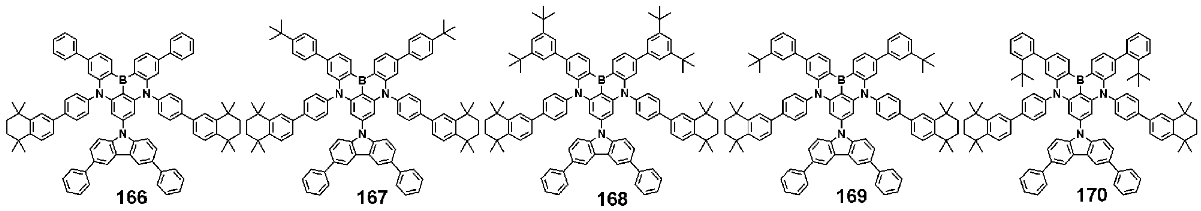

166 167 168 169 170

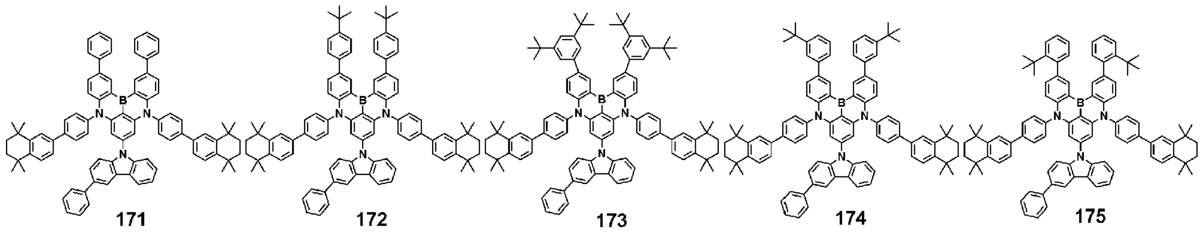

171 172 173 174 175

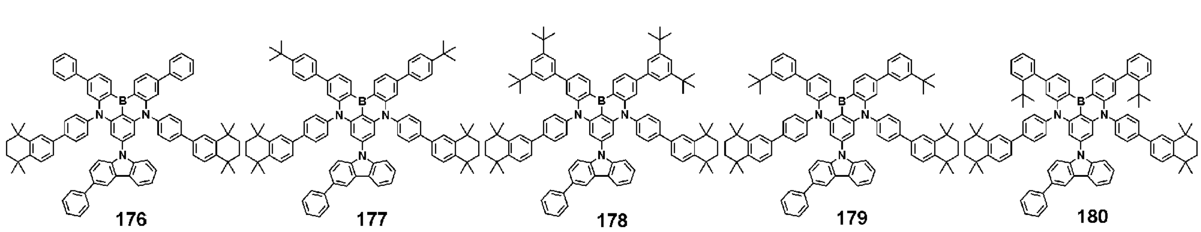

176 177 178 179 180

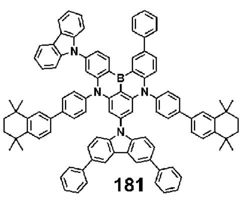
181
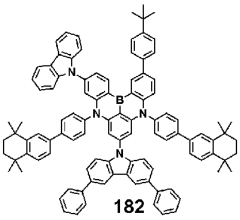
182
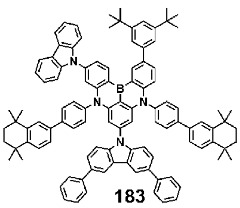
183
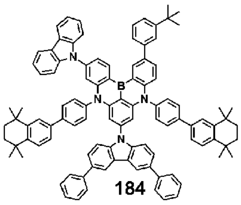
184
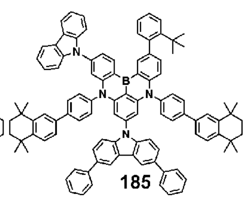
185

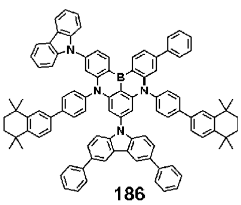
186
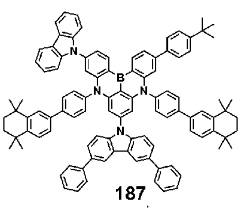
187
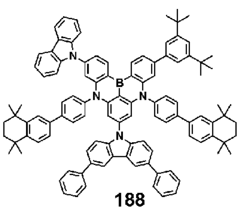
188
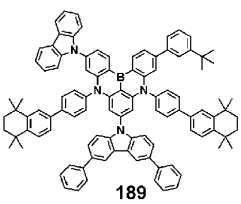
189
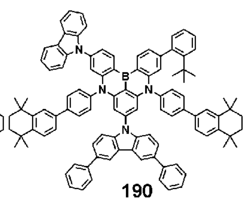
190

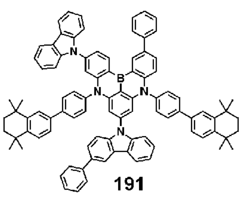
191
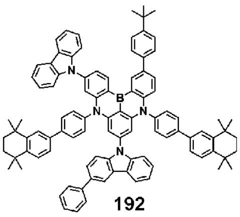
192
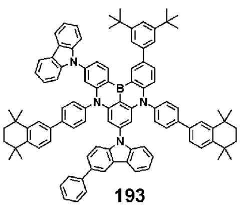
193
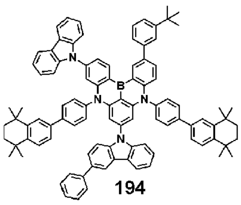
194
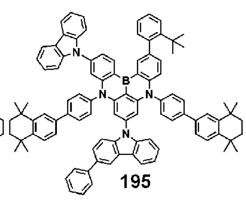
195

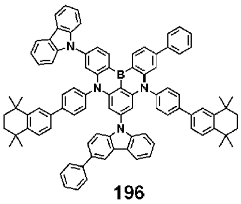
196
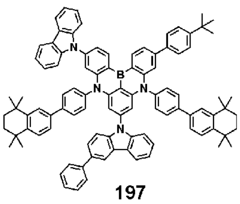
197
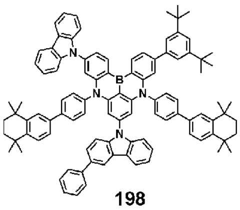
198
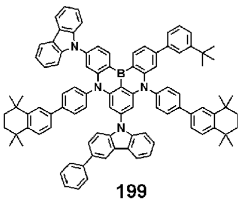
199
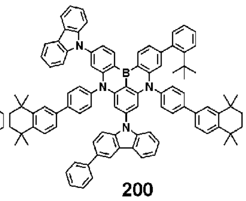
200

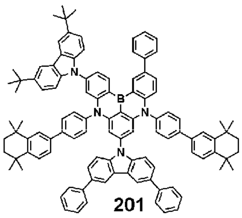
201
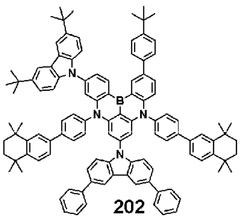
202
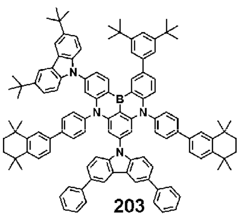
203
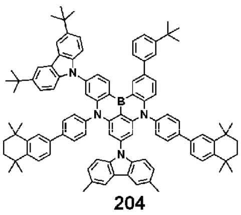
204
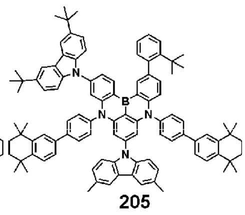
205

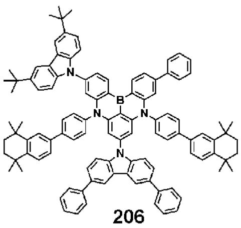
206
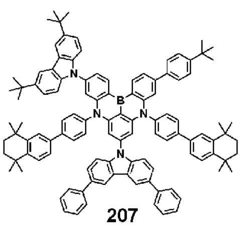
207
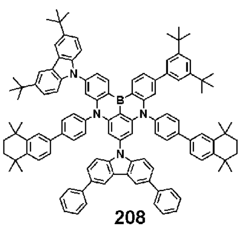
208
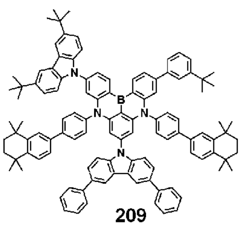
209
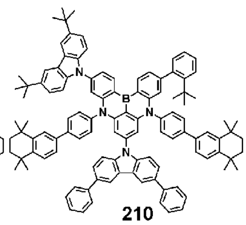
210

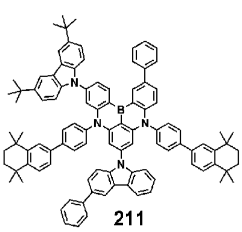
211
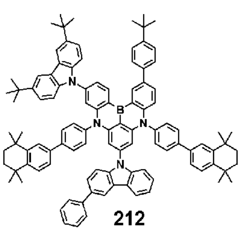
212
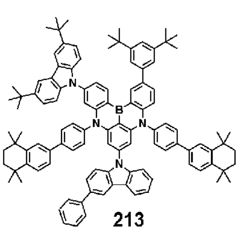
213
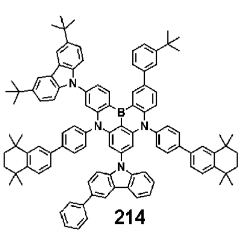
214
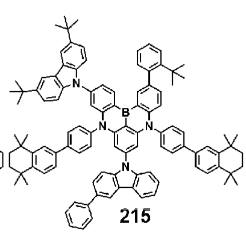
215

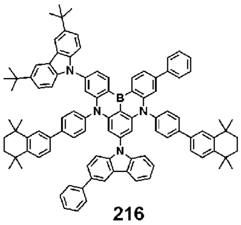
216
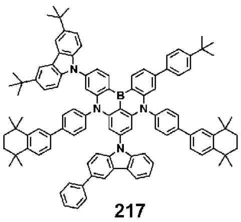
217
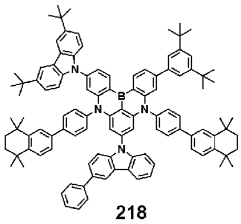
218
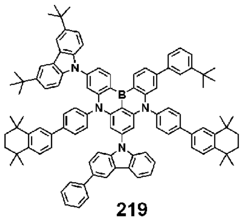
219
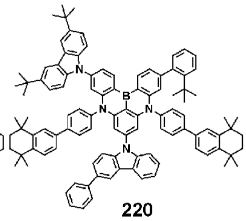
220

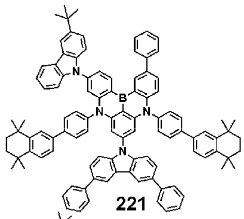
221
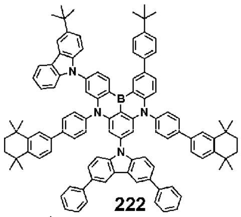
222
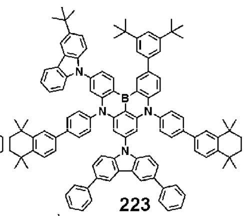
223
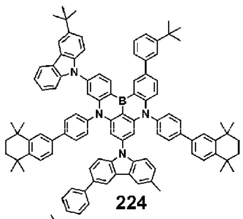
224
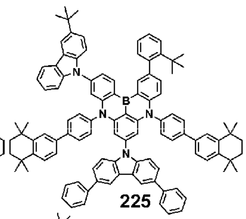
225

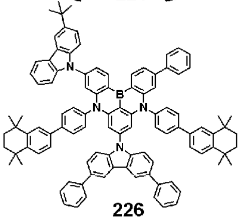
226
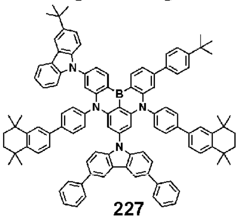
227
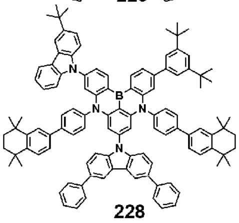
228
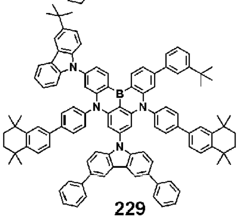
229
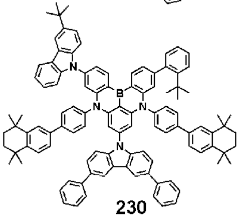
230

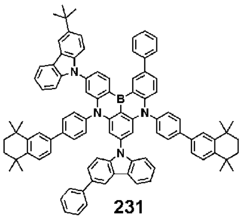
231
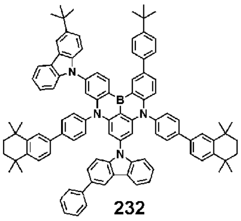
232
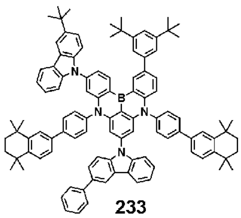
233
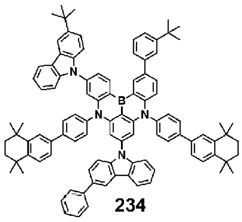
234
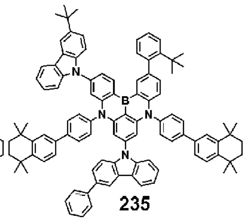
235

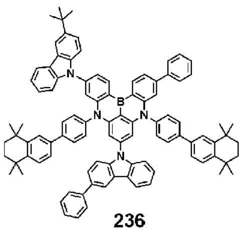
236
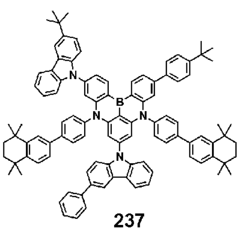
237
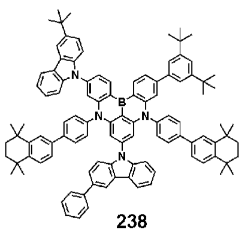
238
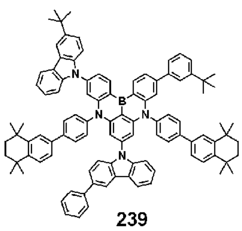
239
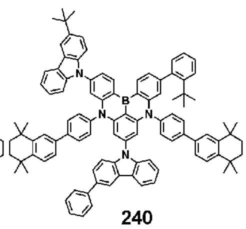
240

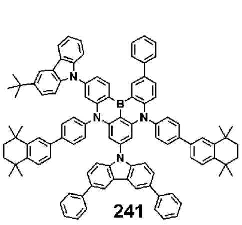
241
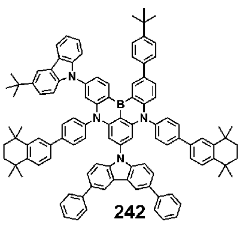
242
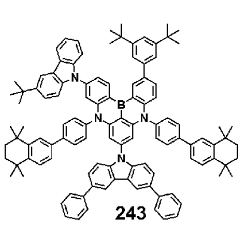
243
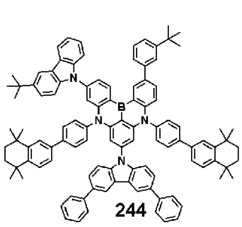
244
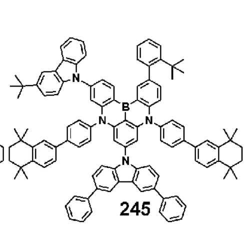
245

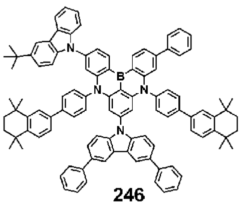
246
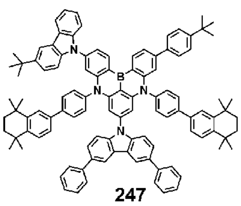
247
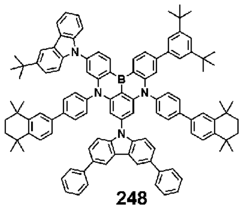
248
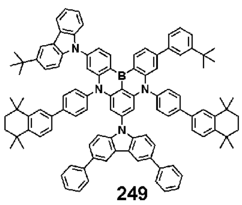
249
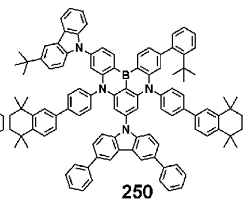
250

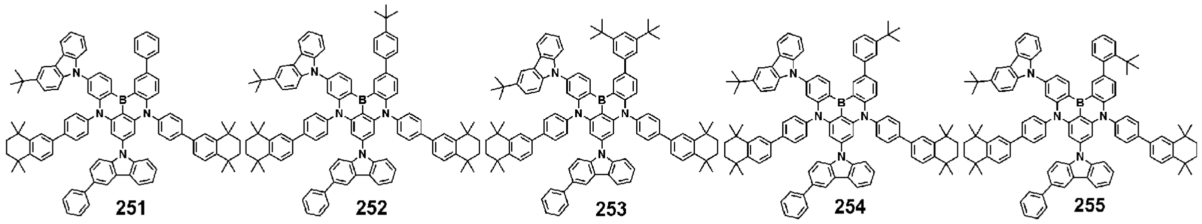

251 252 253 254 255

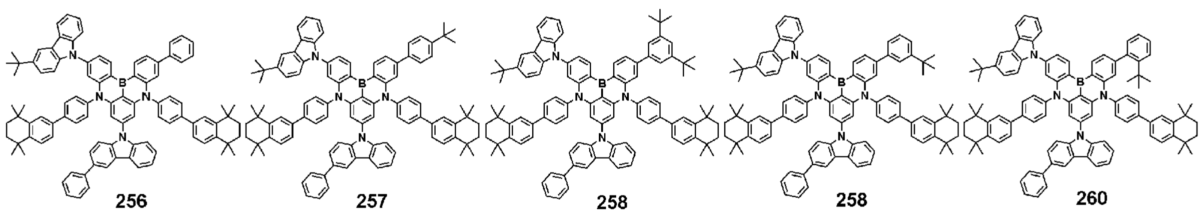

256 257 258 258 260

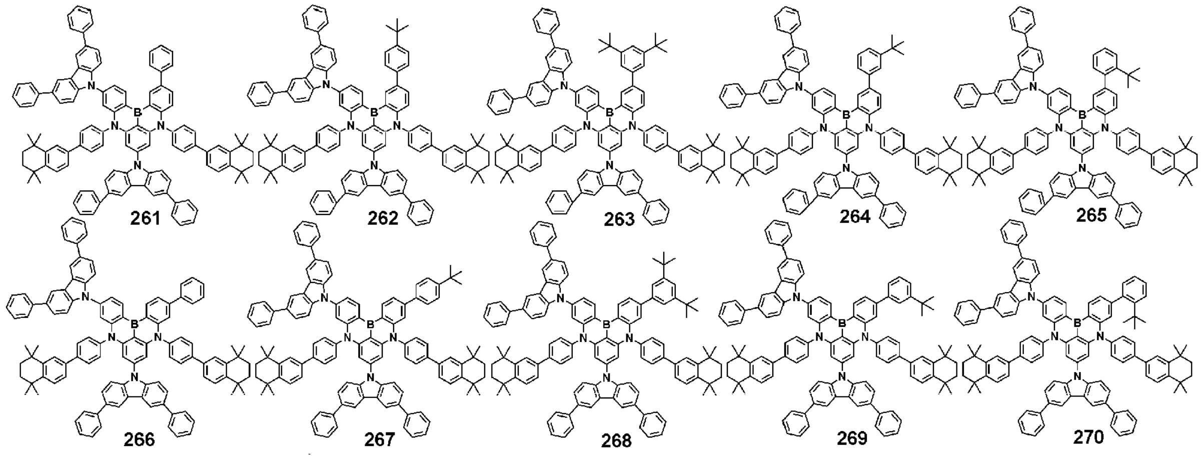

261 262 263 264 265

266 267 268 269 270

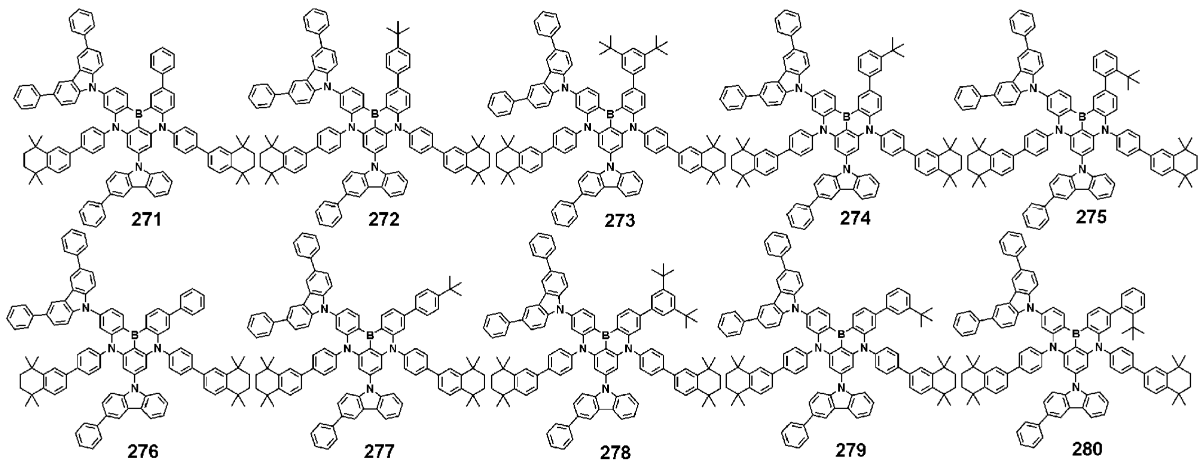

271 272 273 274 275

276 277 278 279 280

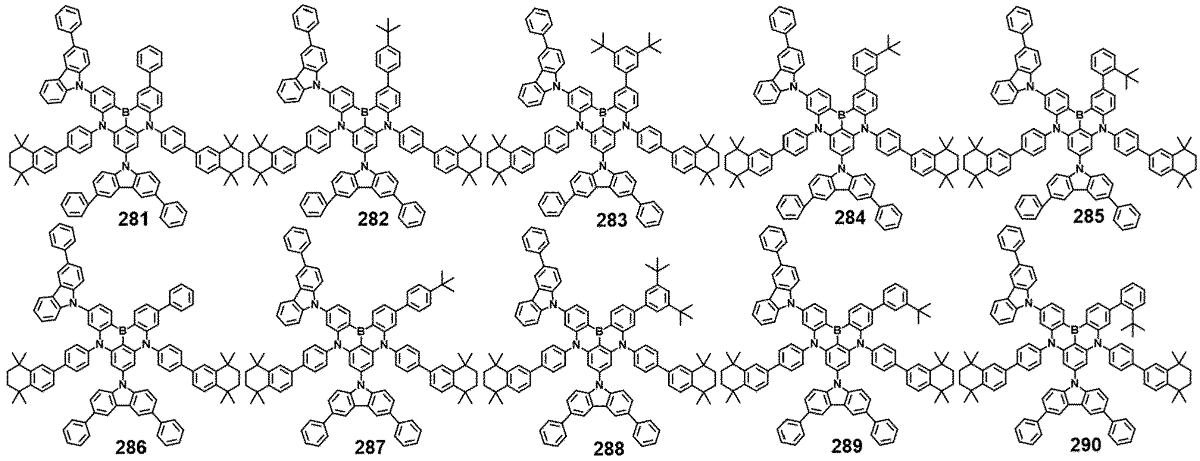

281 282 283 284 285

286 287 288 289 290

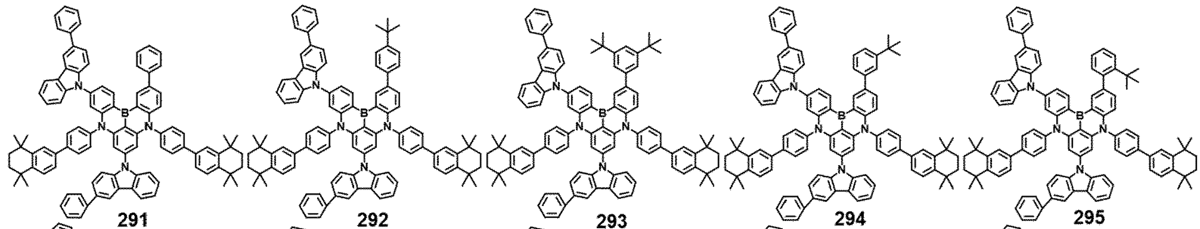

291 292 293 294 295

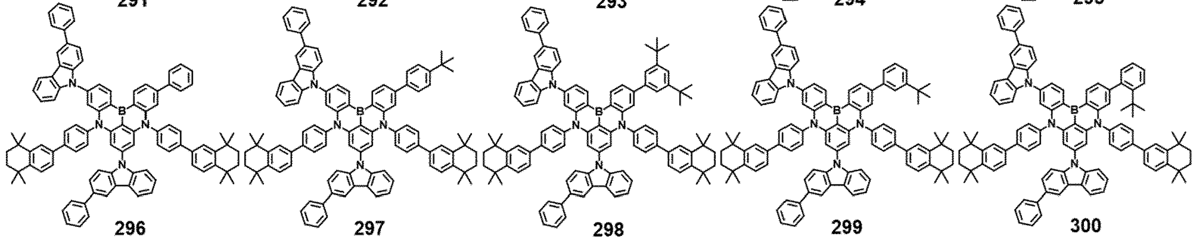

296 297 298 299 300

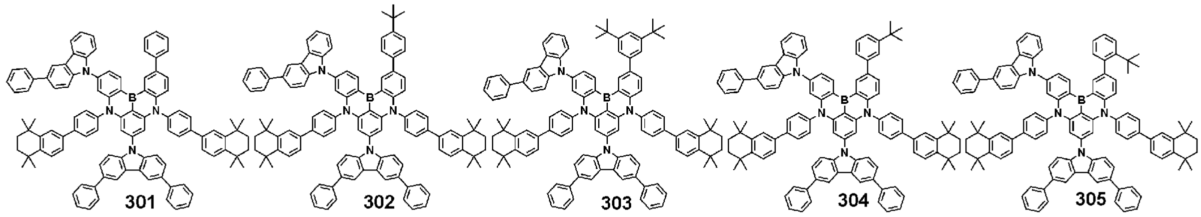

301 302 303 304 305

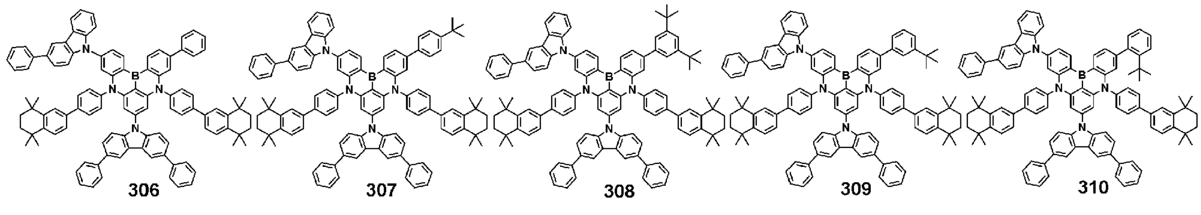

306 307 308 309 310

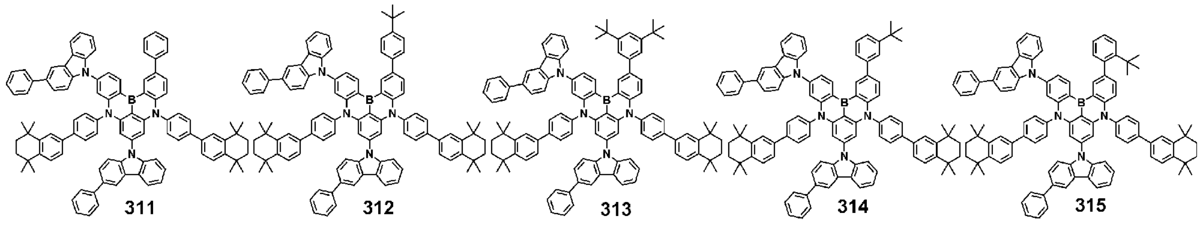

311 312 313 314 315

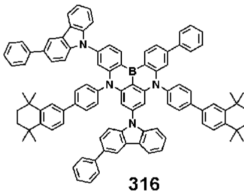
316

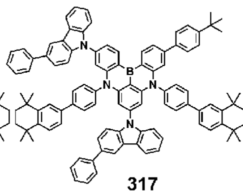
317

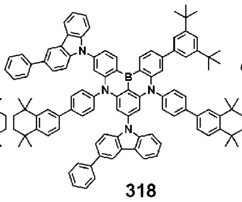
318

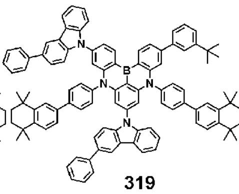
319

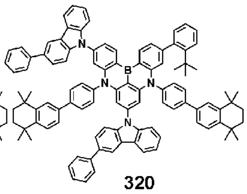
320

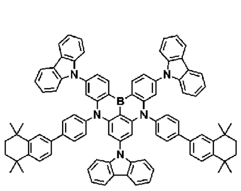
321

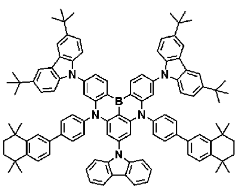
322

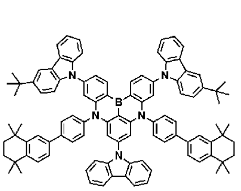
323

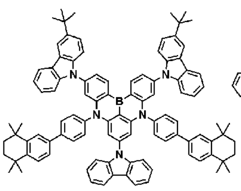
324

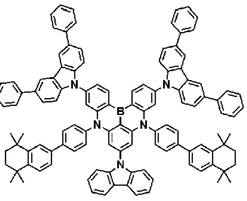
325

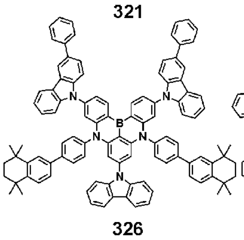
326

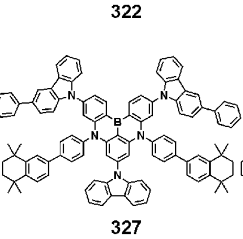
327

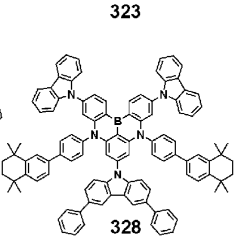
328

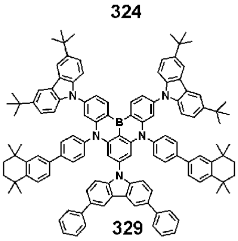
329

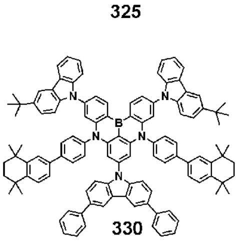
330

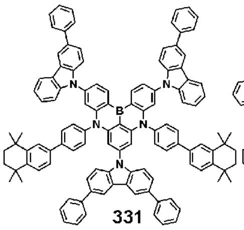
331

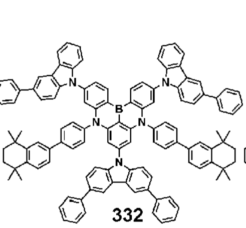
332

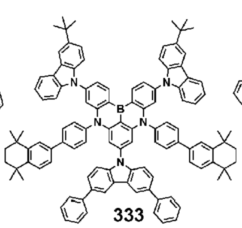
333

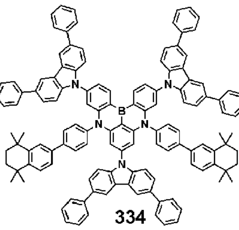
334

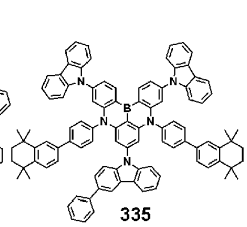
335

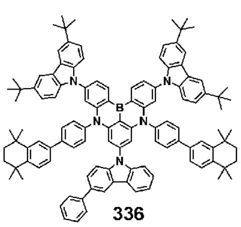
336

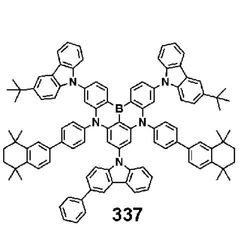
337

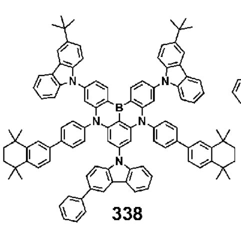
338

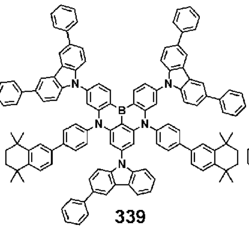
339

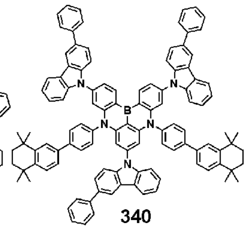
340

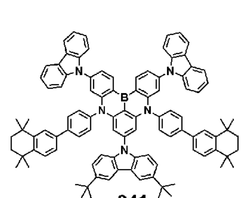
341
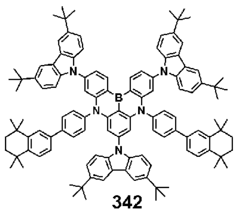
342
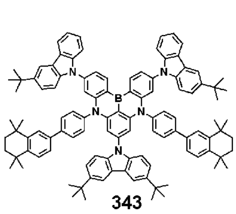
343
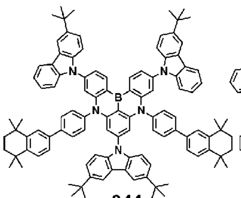
344
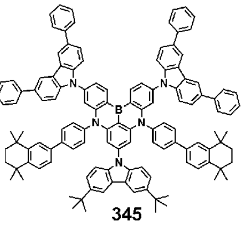
345

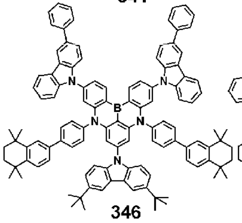
346
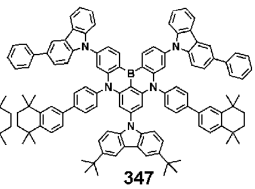
347
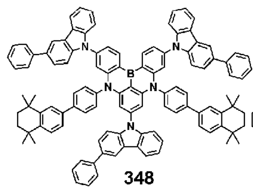
348
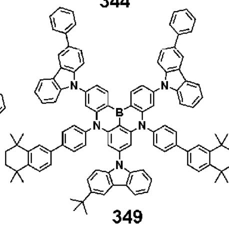
349
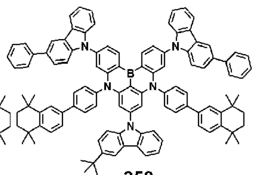
350

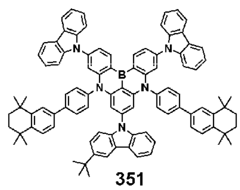
351
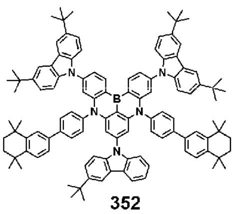
352
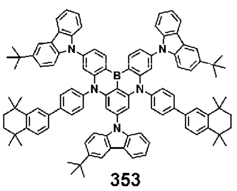
353
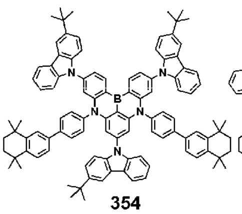
354
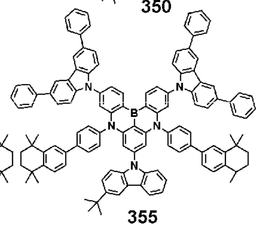
355

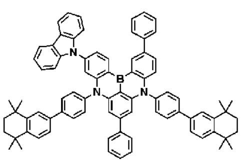
356
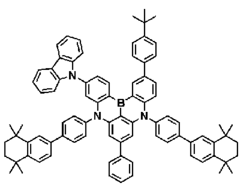
357
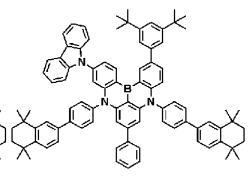
358
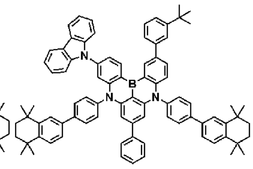
359
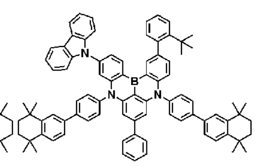
360

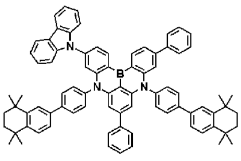
361
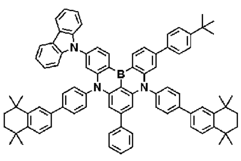
362
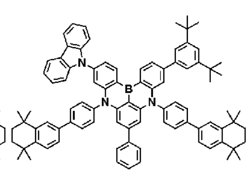
363
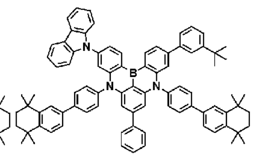
364
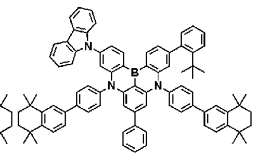
365

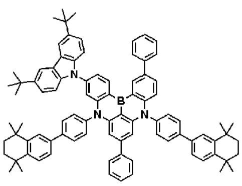
366
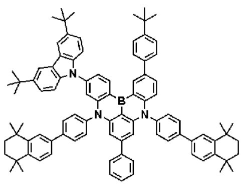
367
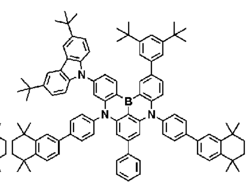
368
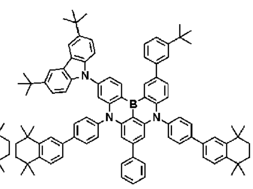
369
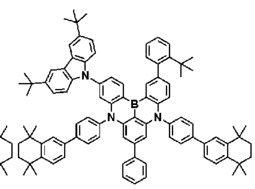
370

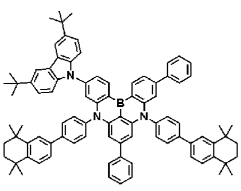
371
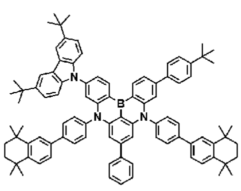
372
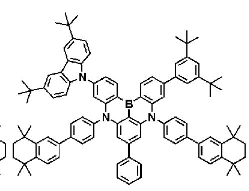
373
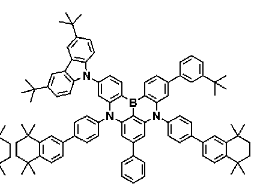
374
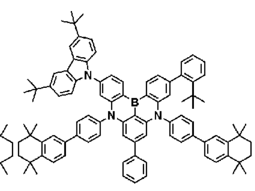
375

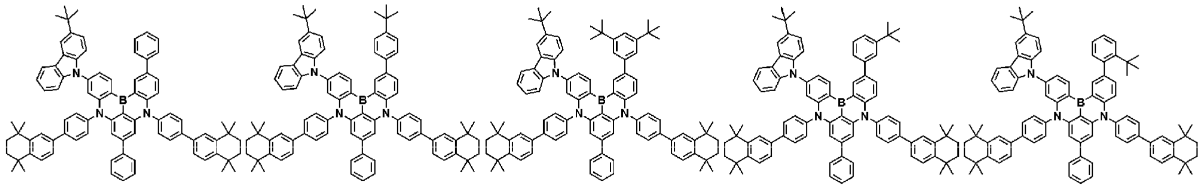

376 377 378 379 380

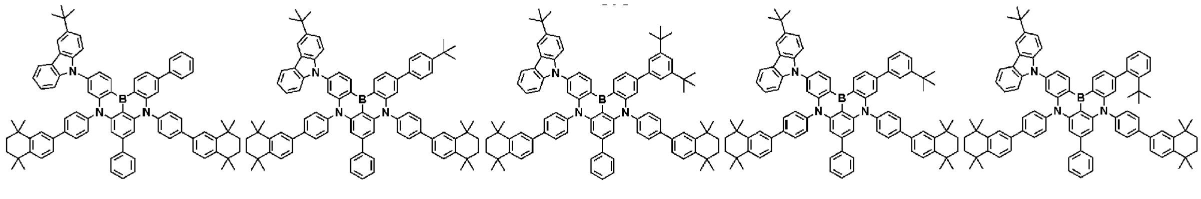

381 382 383 384 385

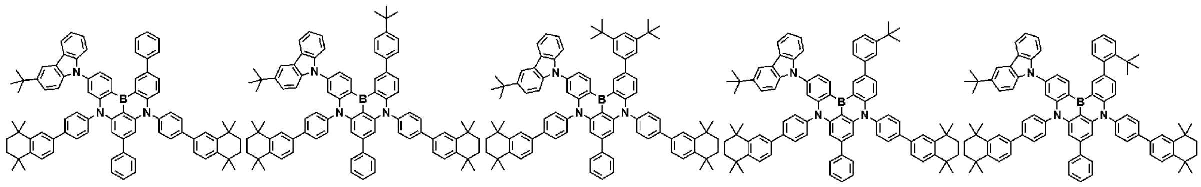

386 387 388 389 390

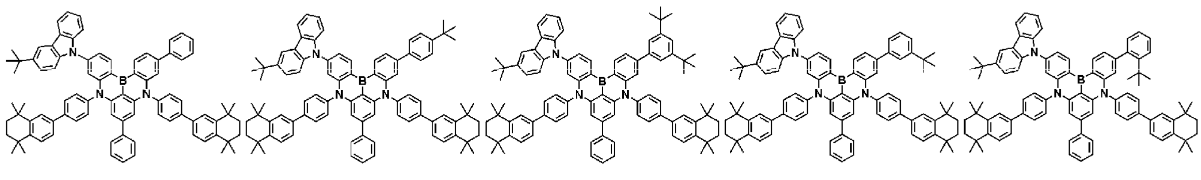

391 392 393 394 395

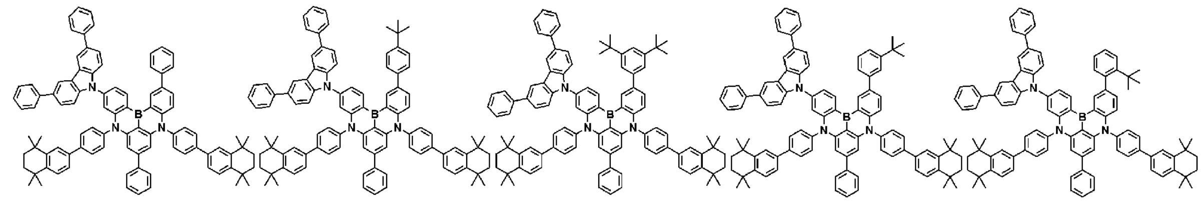

396 397 398 399 400

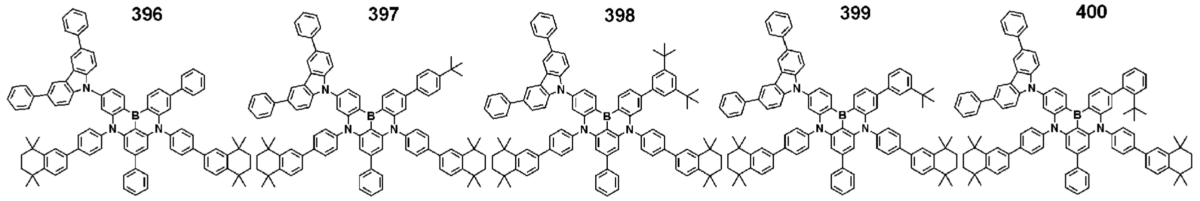

401 402 403 404 405

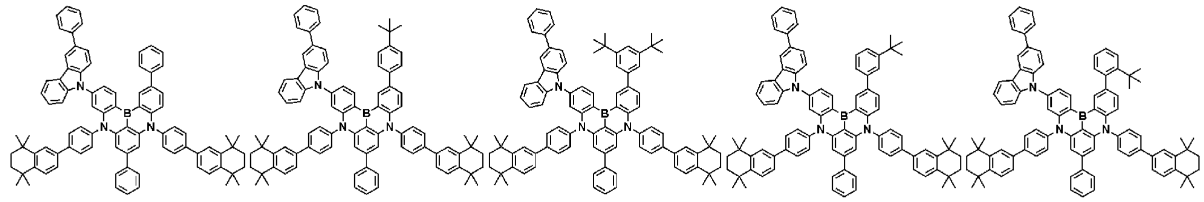

406 407 408 409 410

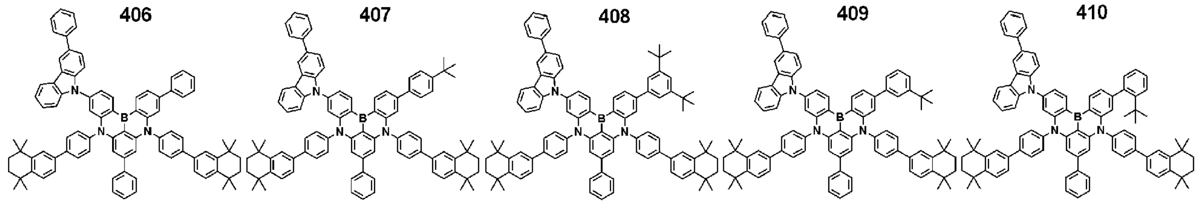

411 412 413 414 415

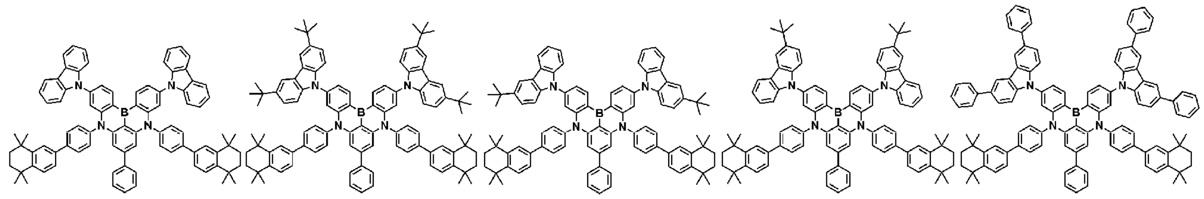

416 417 418 419 420

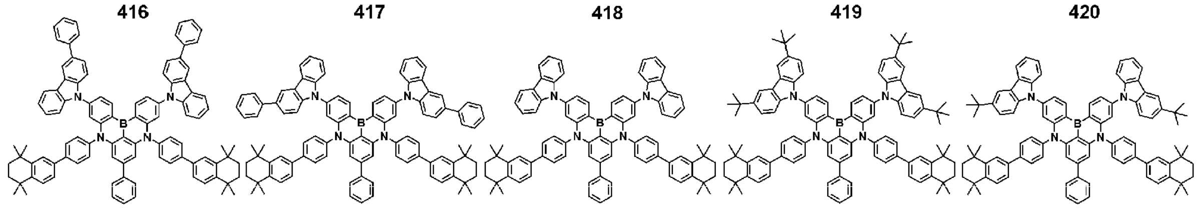

421 422 423 424 425

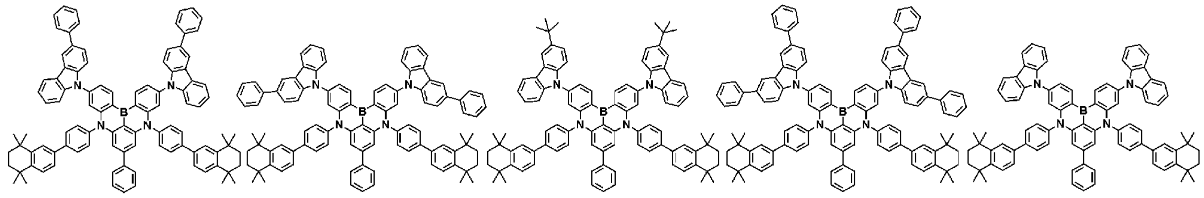

426 427 428 429 430

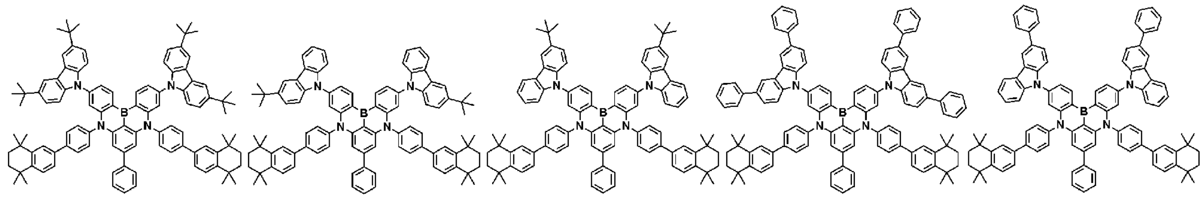

431 432 433 434 435

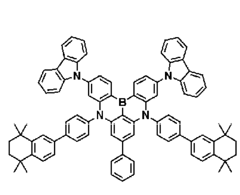
436
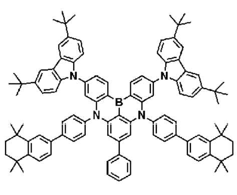
437
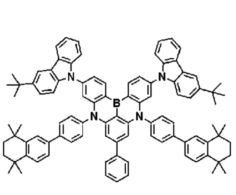
438
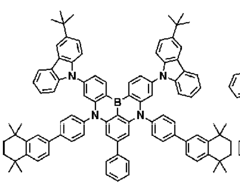
439
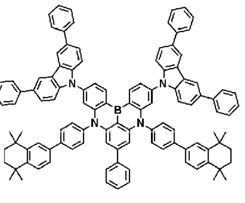
440

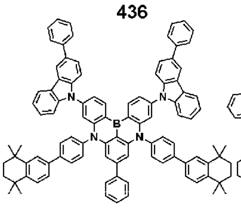
441
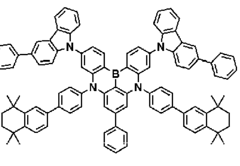
442
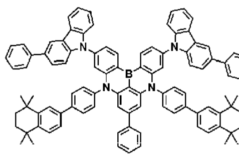
443
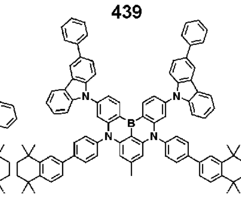
444
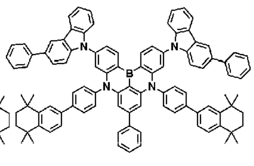
445

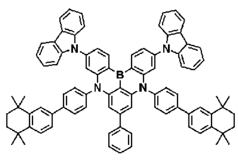
446
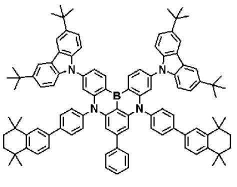
447
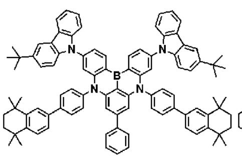
448
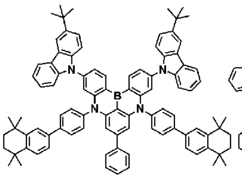
449
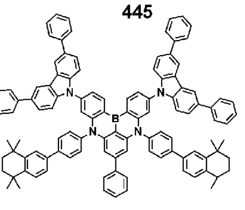
450

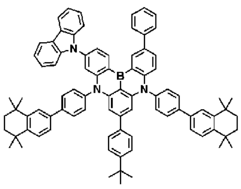
451
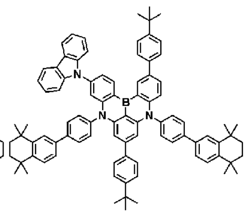
452
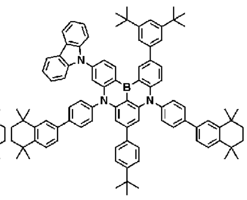
453
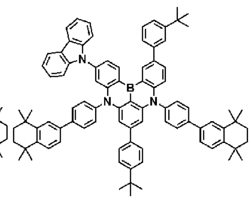
454
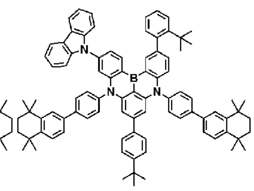
455

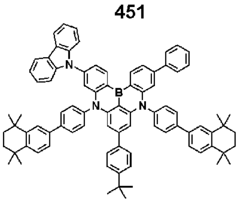
456
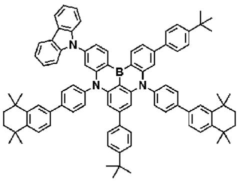
457
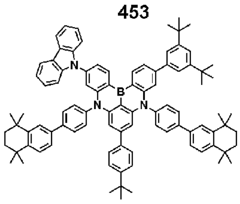
458
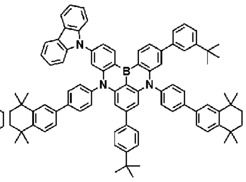
459
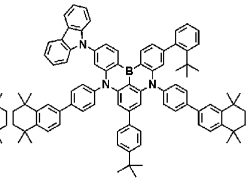
460

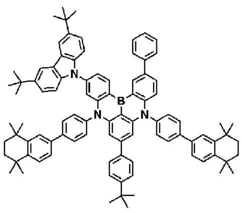
461
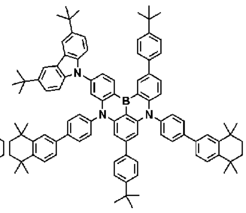
462
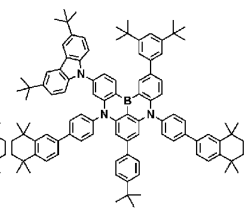
463
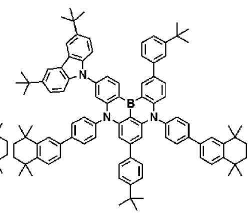
464
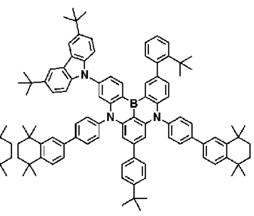
465

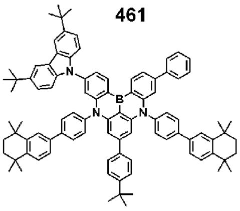
466
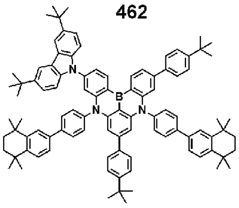
467
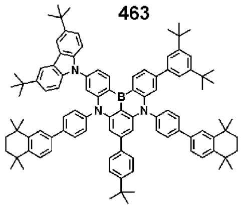
468
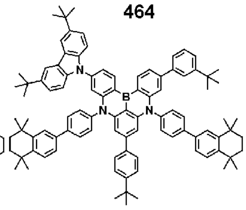
469
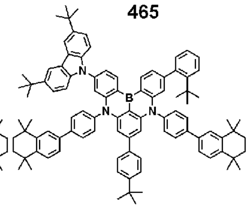
470

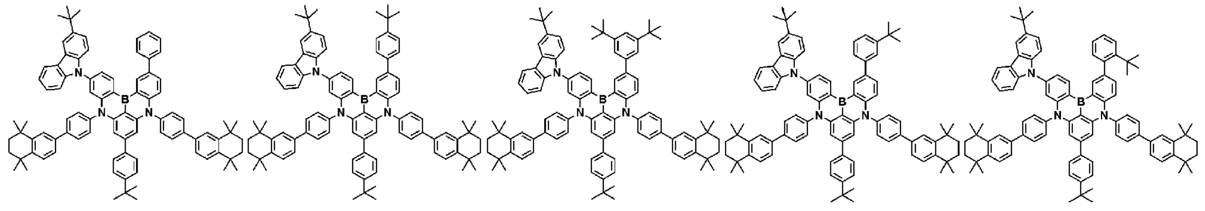

471 472 473 474 475

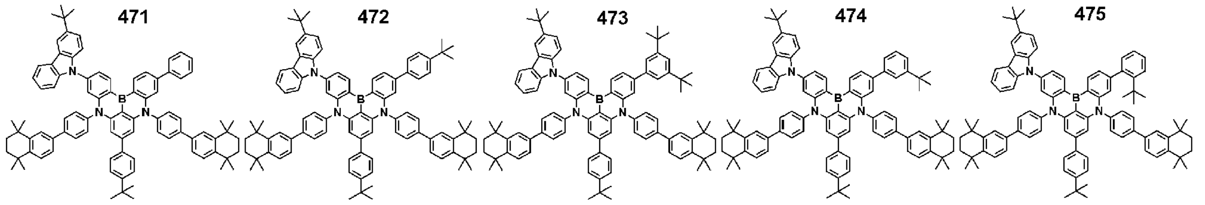

476 477, 478 479 480

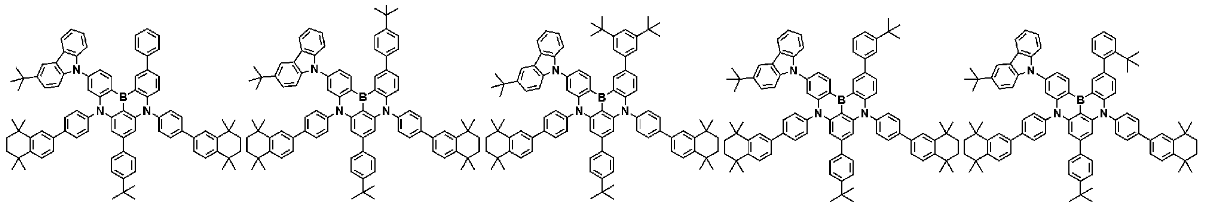

481 482 483 484 485

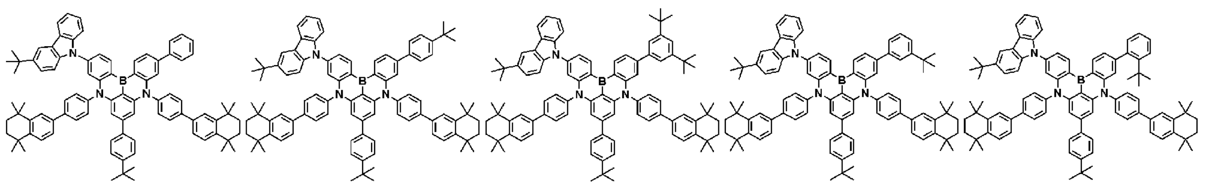

486 487 488 489 490

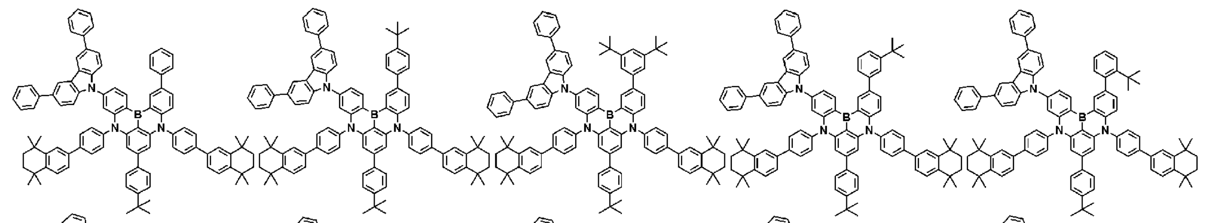

491 492 493 494 495

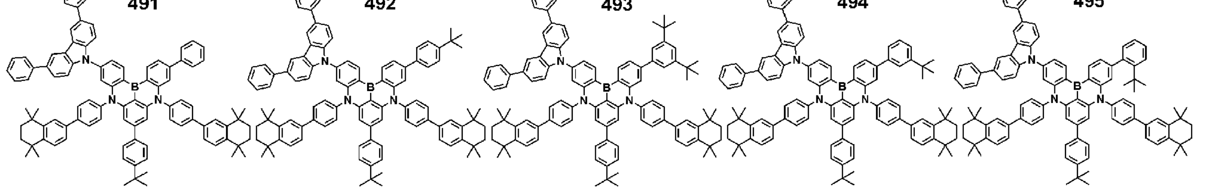

496 497 498 499 500

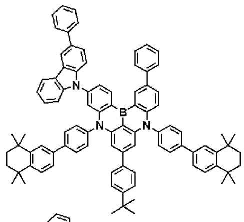
501
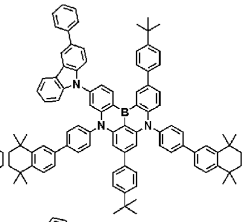
502
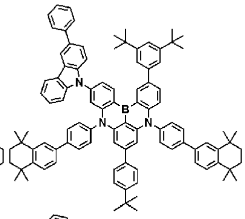
503
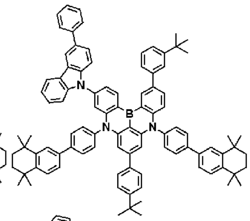
504
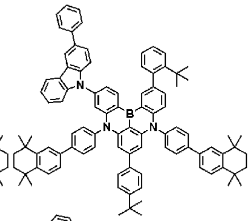
505
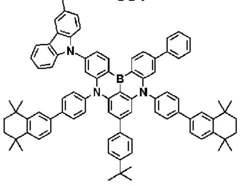
506
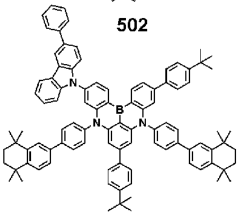
507
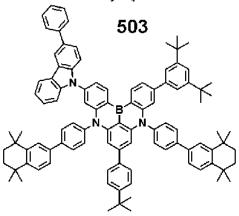
508
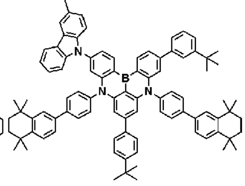
509
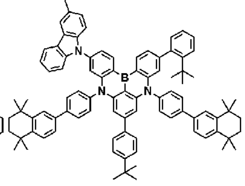
510
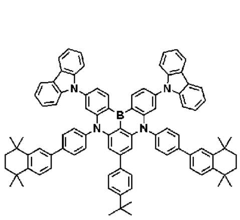
511
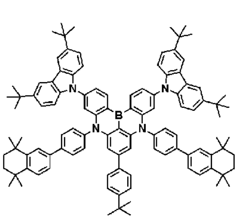
512
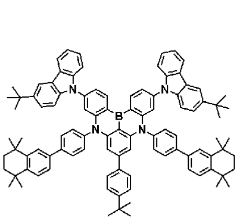
513
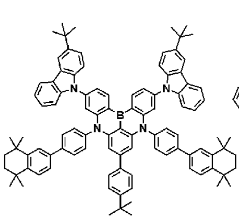
514
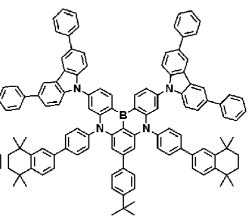
515
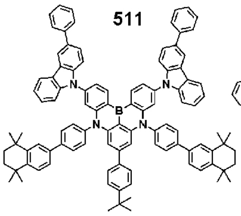
516
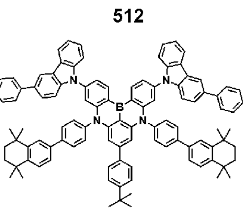
517
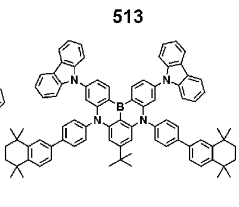
518
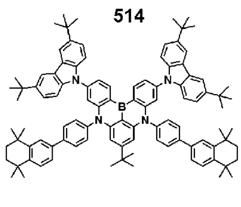
519
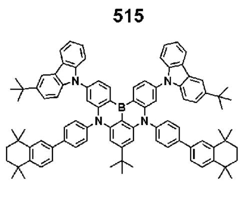
520
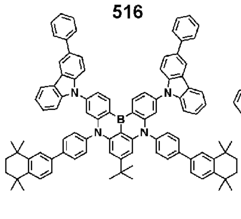
521
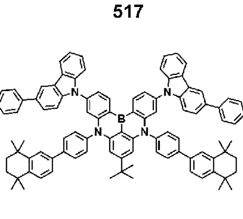
521
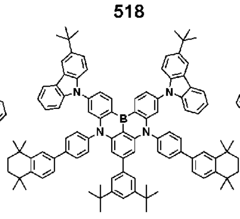
523
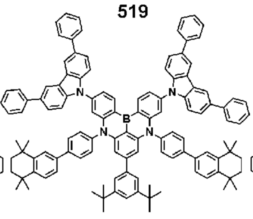
524
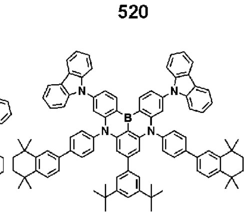
525
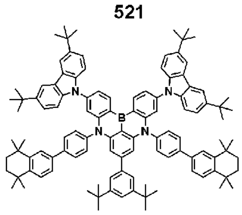
526
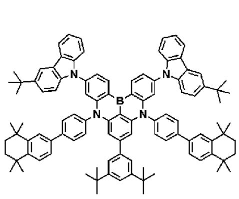
527
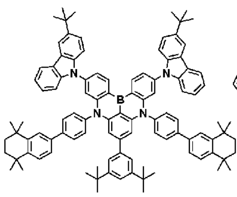
528
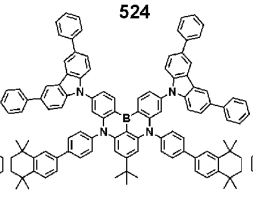
529
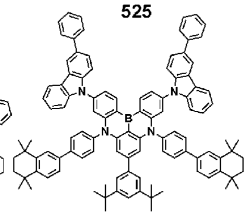
530
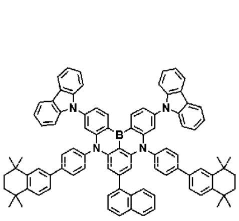
531
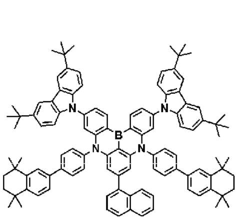
532
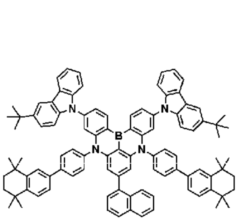
533
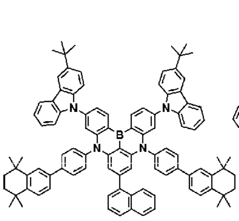
534
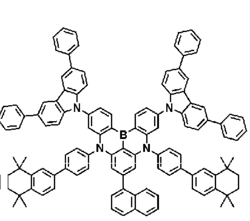
535

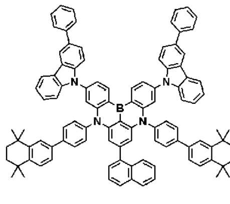
536
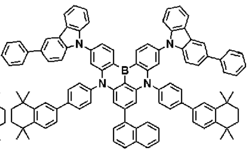
537
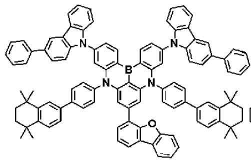
538
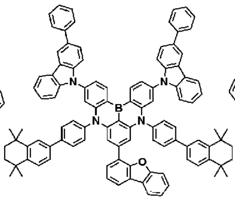
539
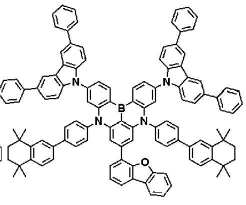
540

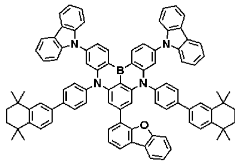
541
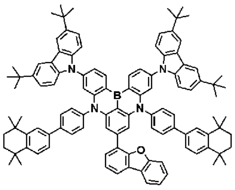
542
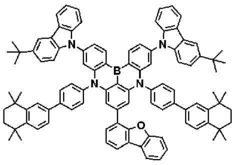
543
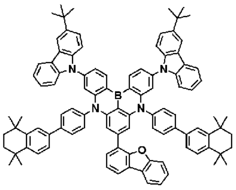
544
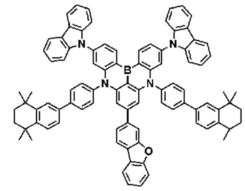
545

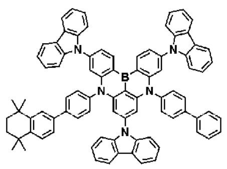
546
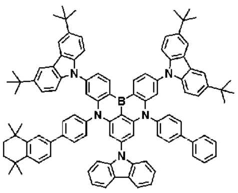
547
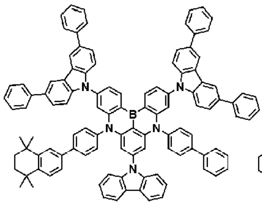
548
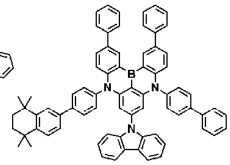
549
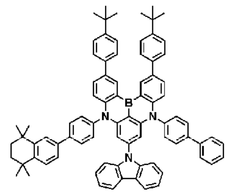
550

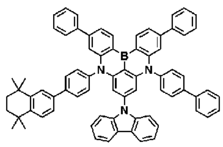
551
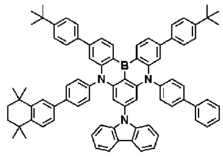
552
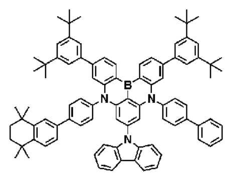
553
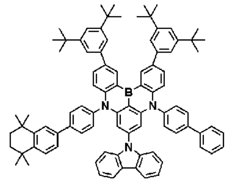
554
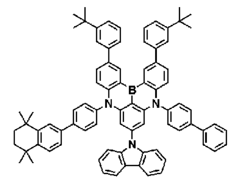
555

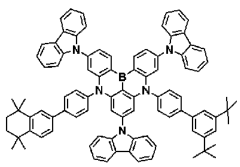
556
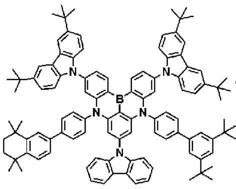
557
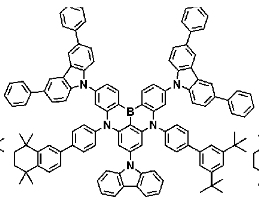
558
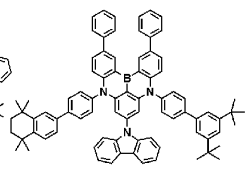
559
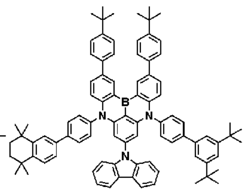
560

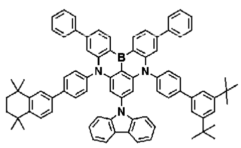
561
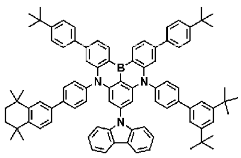
562
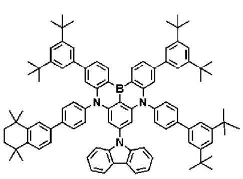
563
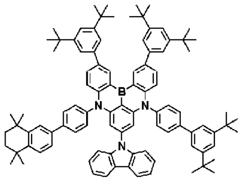
564
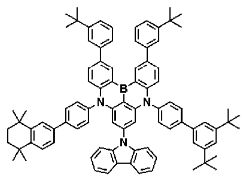
565

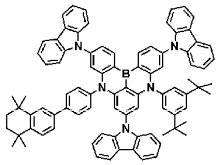
566
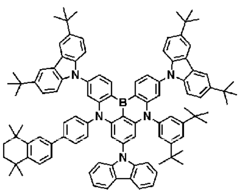
567
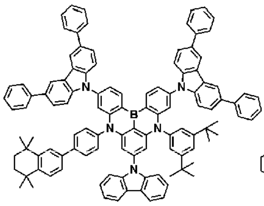
568
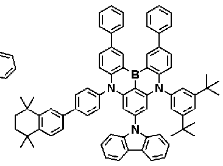
569
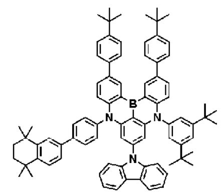
570

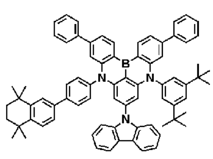
571
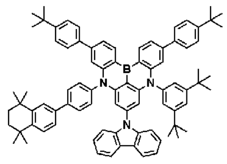
572
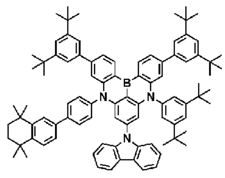
573
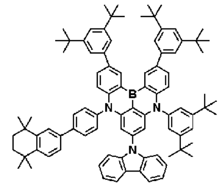
574
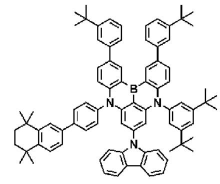
575

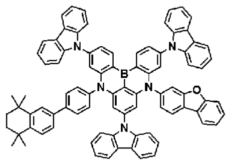
576
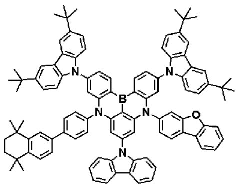
577
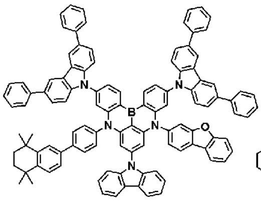
578
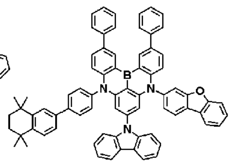
579
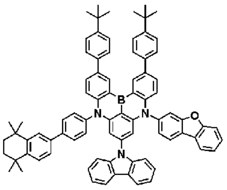
580

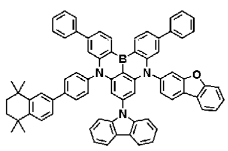
581
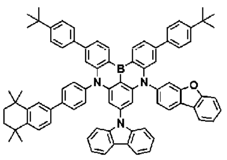
582
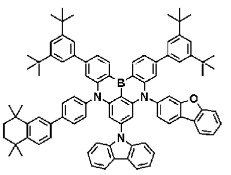
583
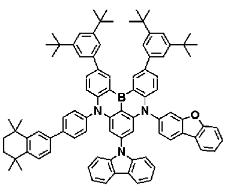
584
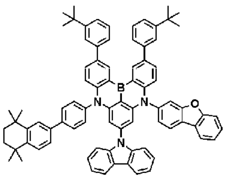
585

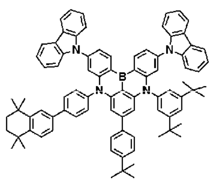
586
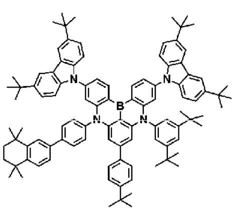
587
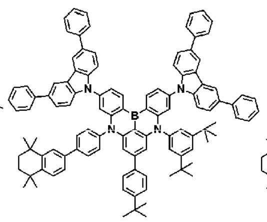
588
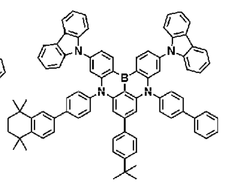
589
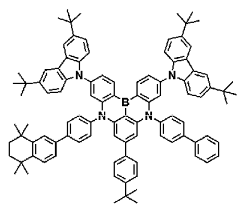
590

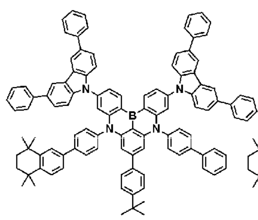
591
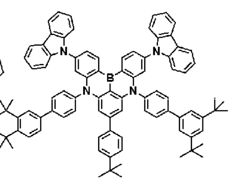
592
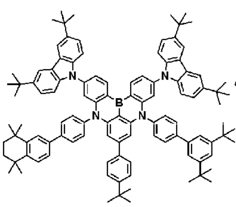
593
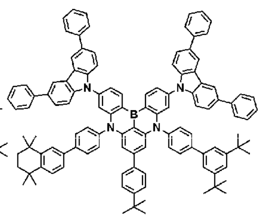
594
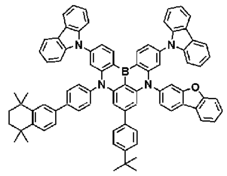
595

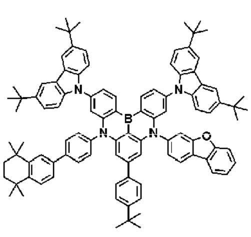
596

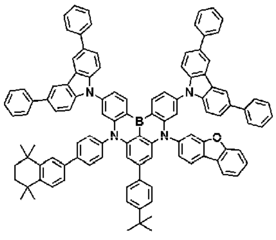
597

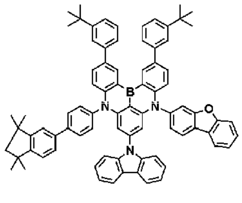
598

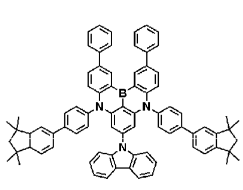
599

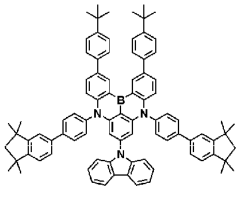
600

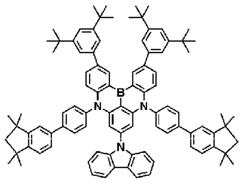
601

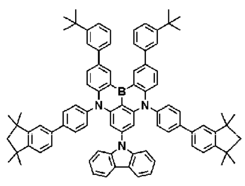
602

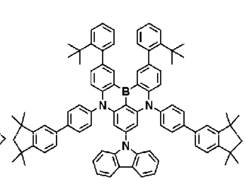
603

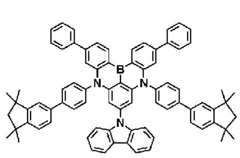
604

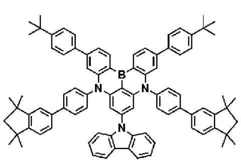
605

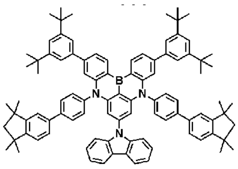
606

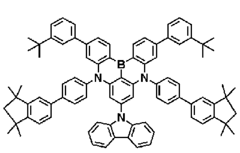
607

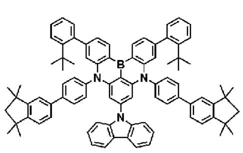
608

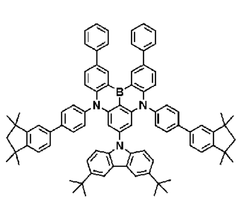
609

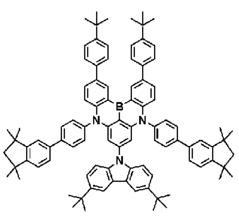
610

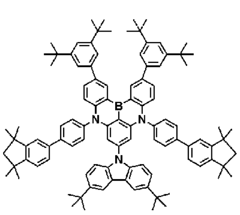
611

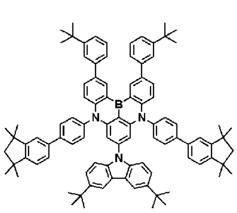
612

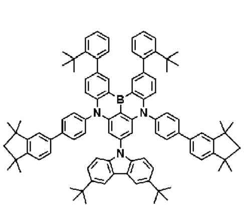
613

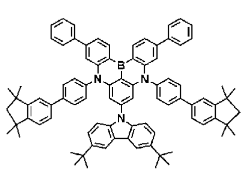
614

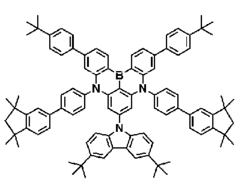
615

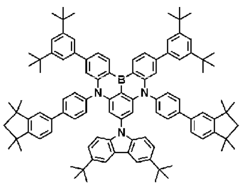
616

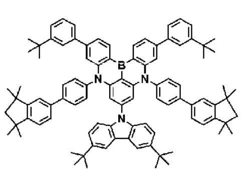
617

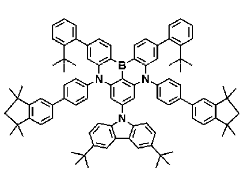
618

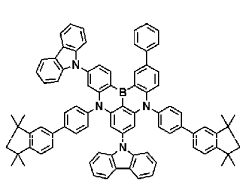
619

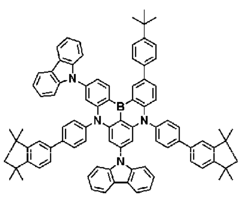
620

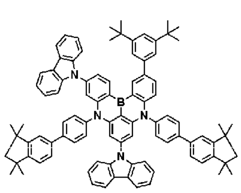
621

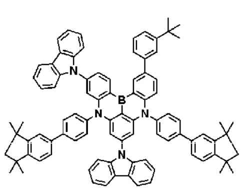
622

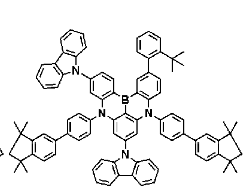
623

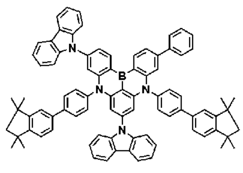
624
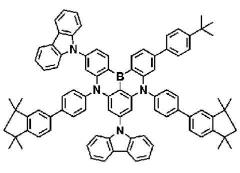
625
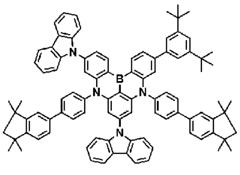
626
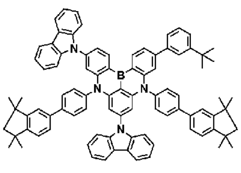
627
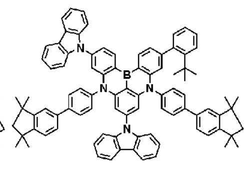
628

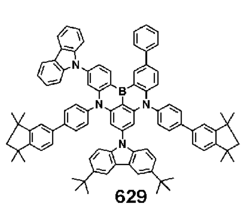
629
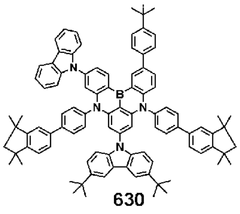
630
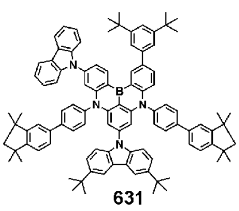
631
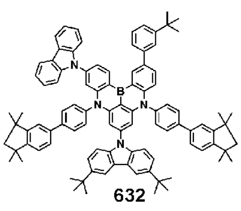
632
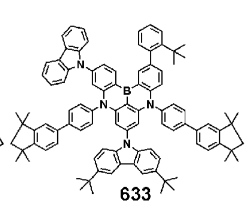
633

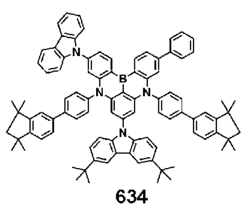
634
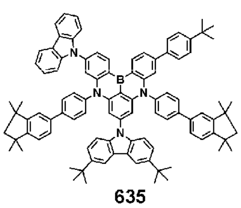
635
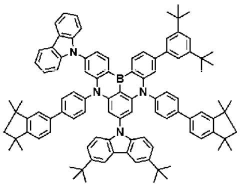
636
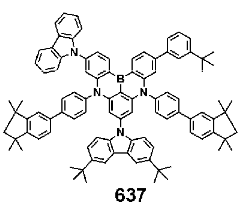
637
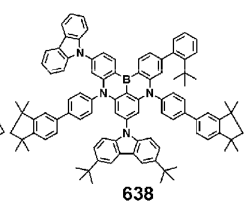
638

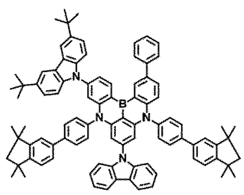
639
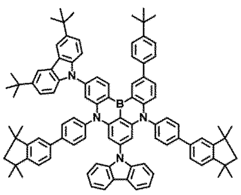
640
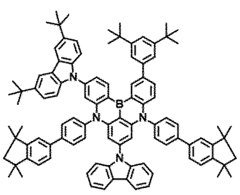
641
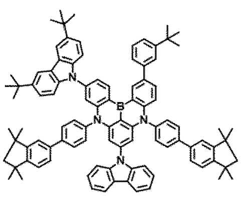
642
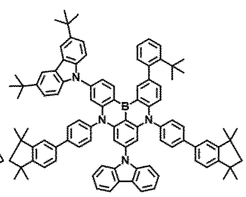
643

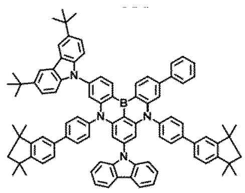
644
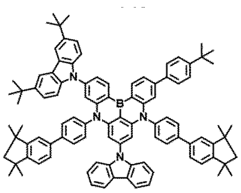
645
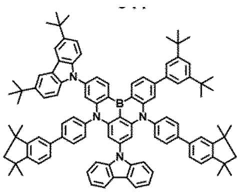
646
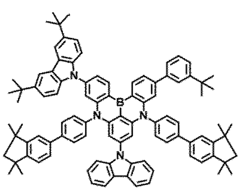
647
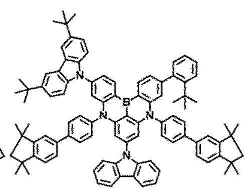
648

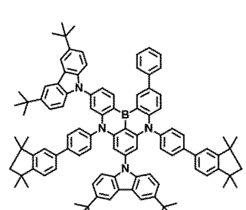
649
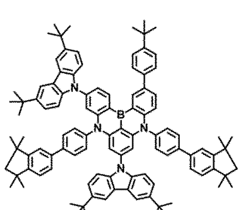
650
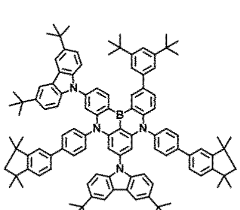
651
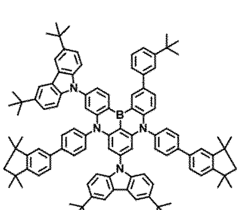
652
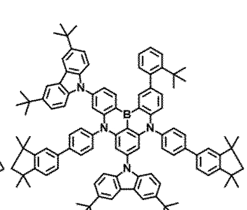
653

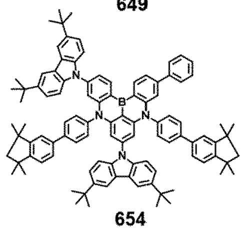
654
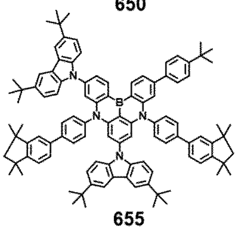
655
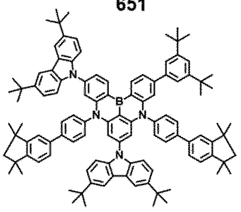
656
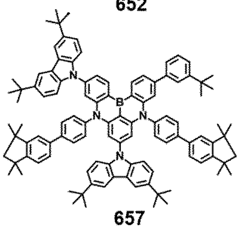
657
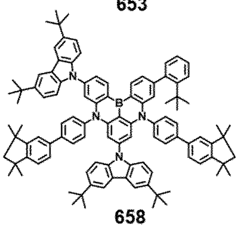
658

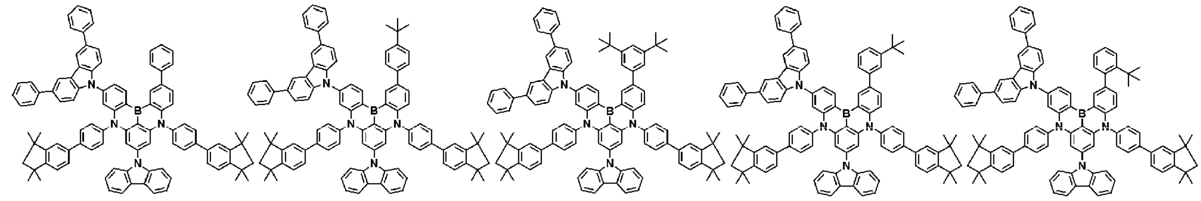

659 660 661 662 663

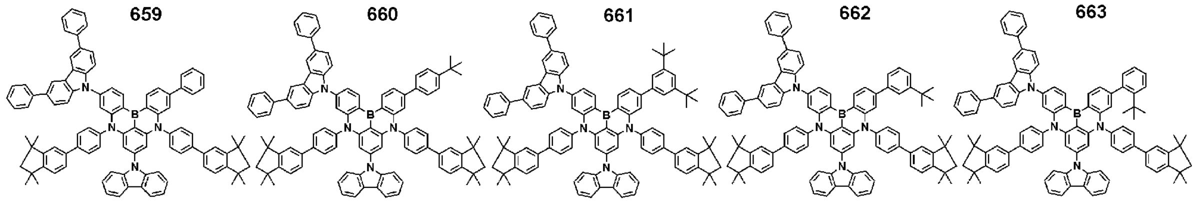

664 665 666 667 668

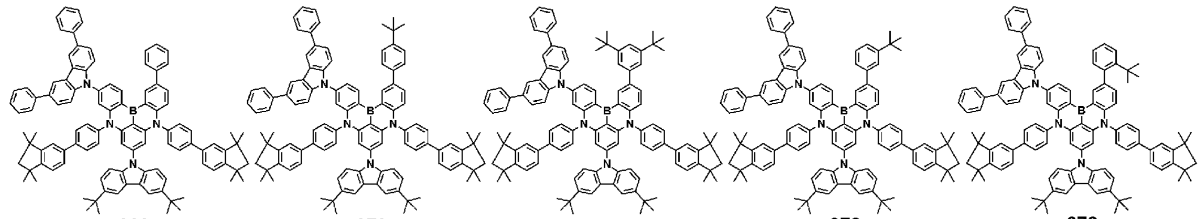

669 670 671 672 673

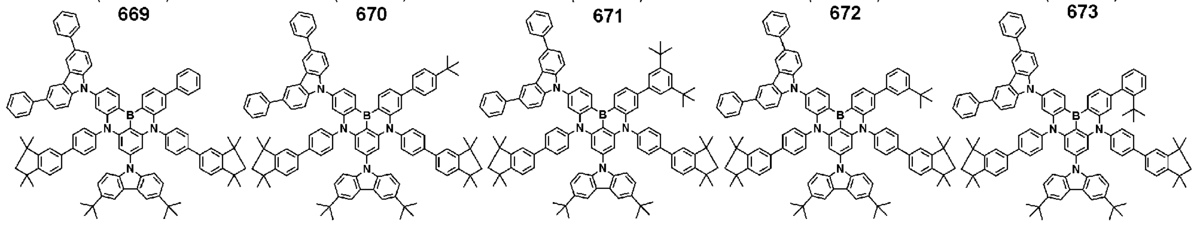

674 675 676 677 678

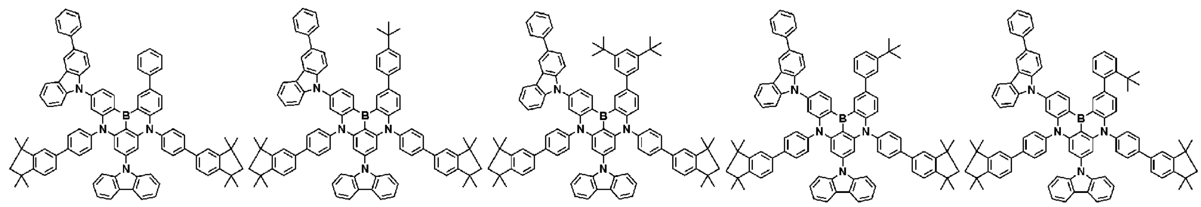

679 680 681 682 683

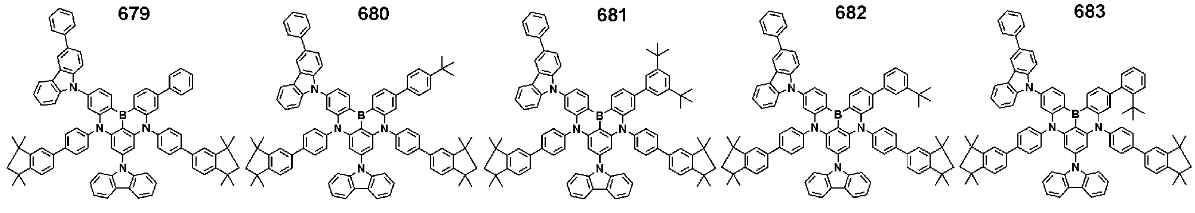

684 685 686 687 688

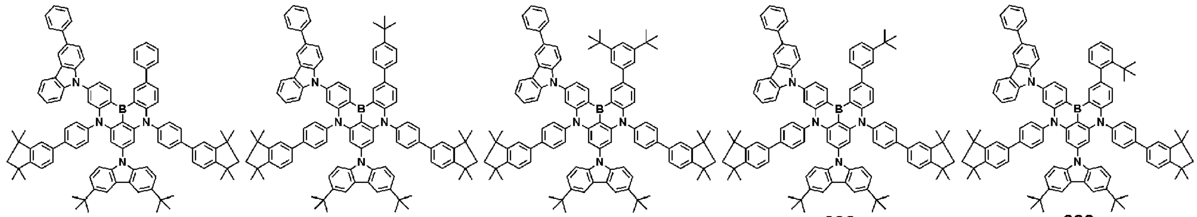

689 690 691 692 693

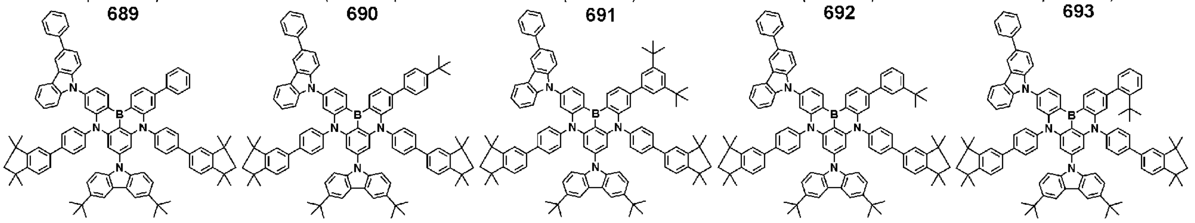

694 695 696 697 698

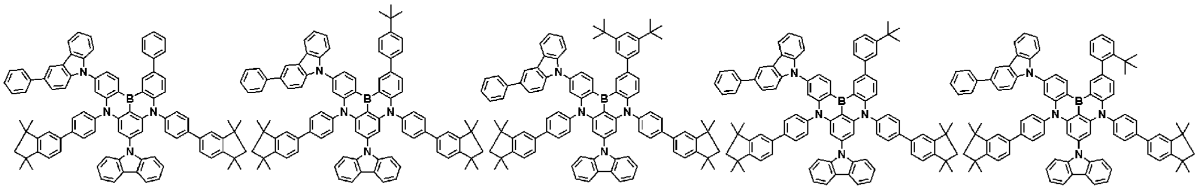

699 700 701 702 703

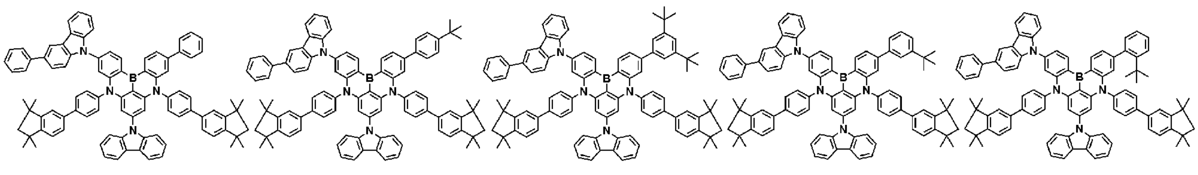

704 705 706 707 708

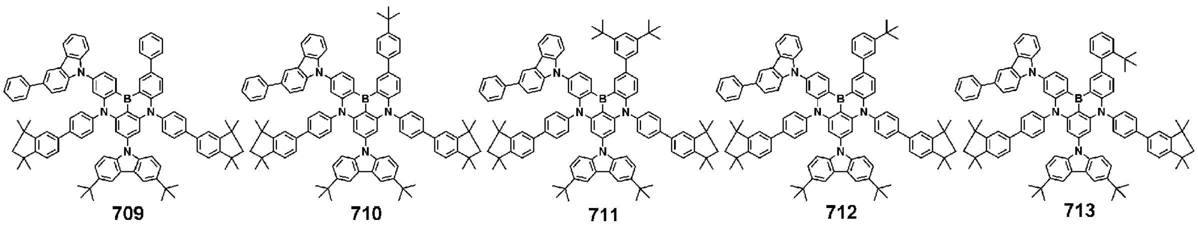

709 710 711 712 713

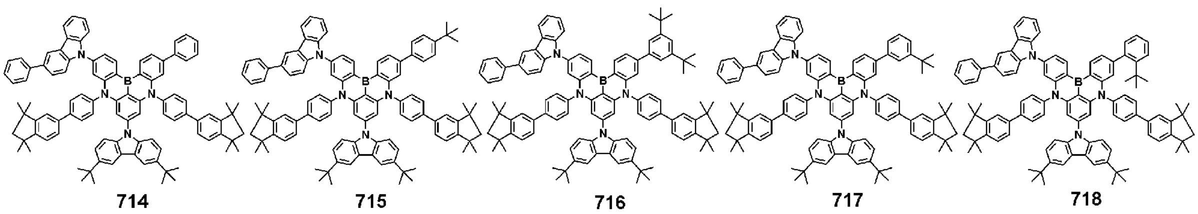

714 715 716 717 718

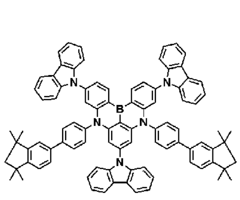
719
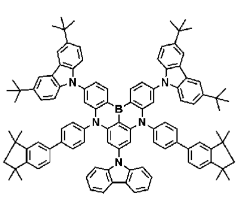
720
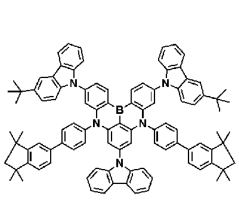
721
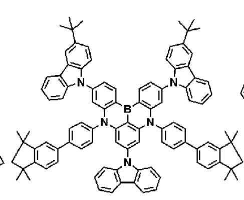
722
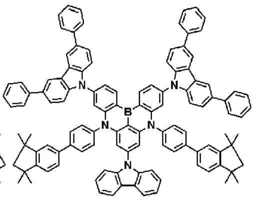
723
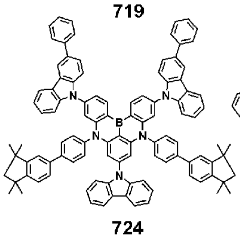
724
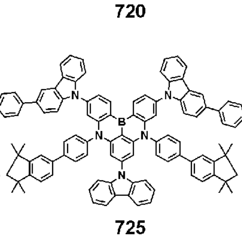
725
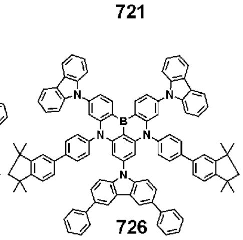
726
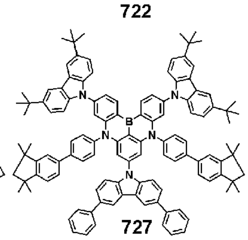
727
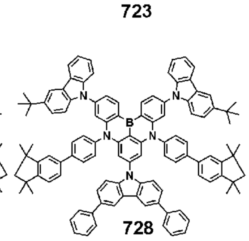
728
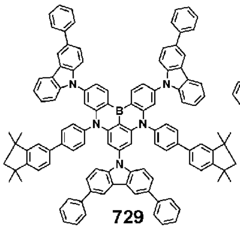
729
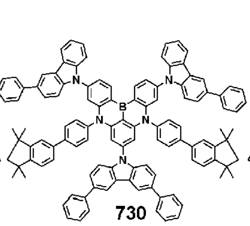
730
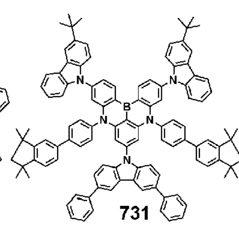
731
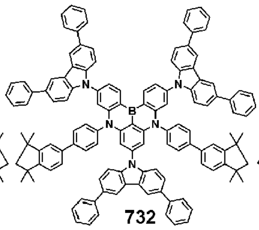
732
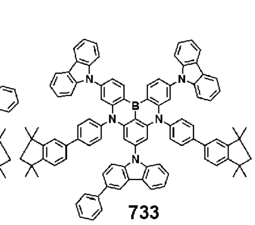
733
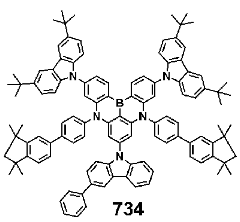
734
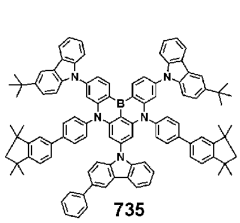
735
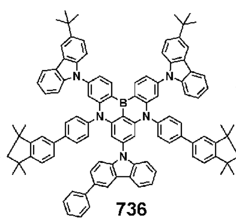
736
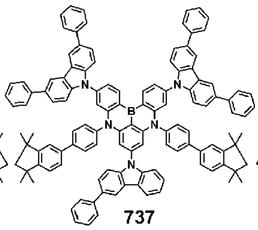
737
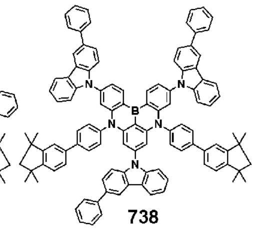
738
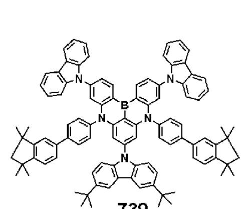
739
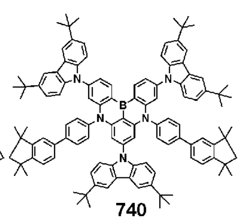
740
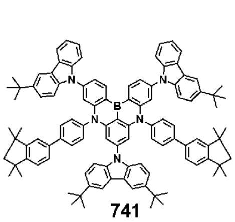
741
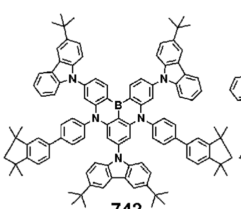
742
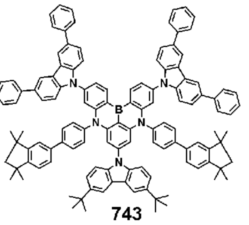
743
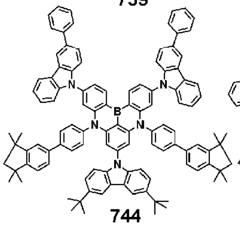
744
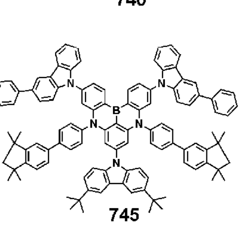
745
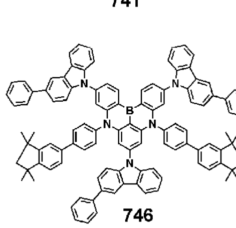
746
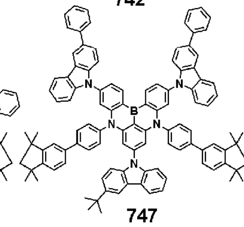
747
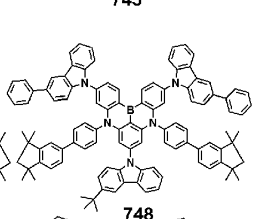
748
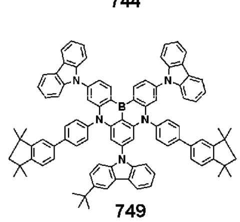
749
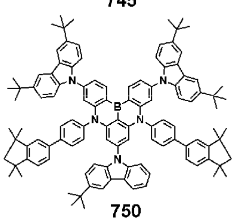
750
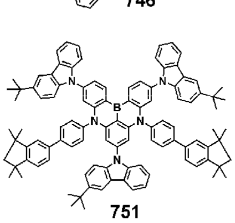
751
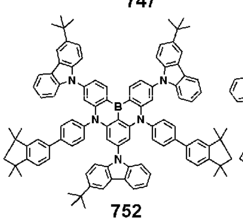
752
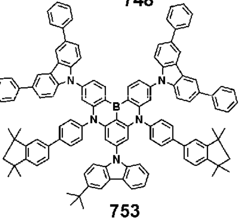
753

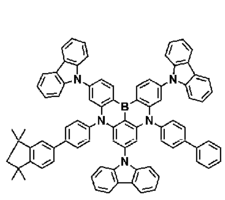
754
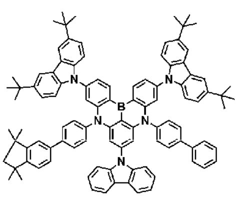
755
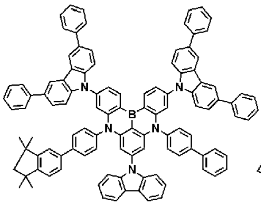
756
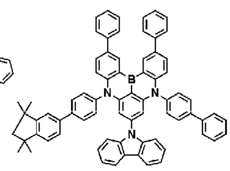
757
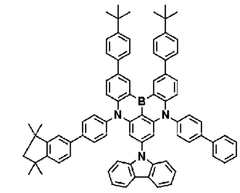
758

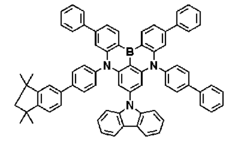
759
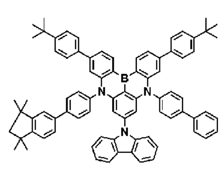
760
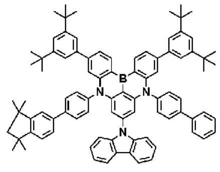
761
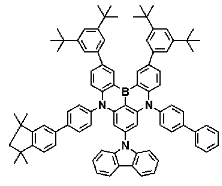
762
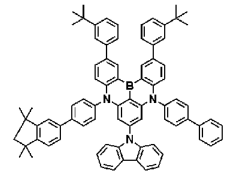
763

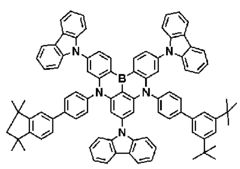
764
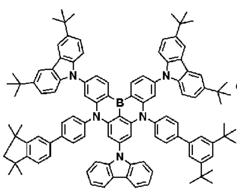
765
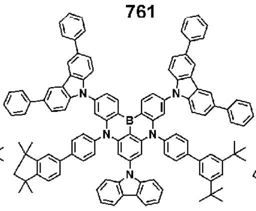
766
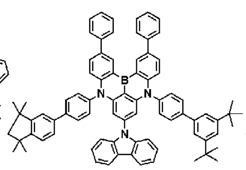
767
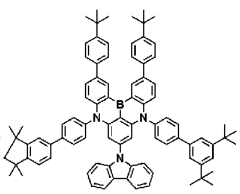
768

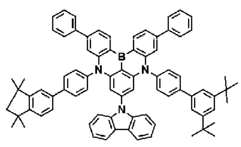
769
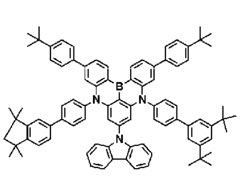
770
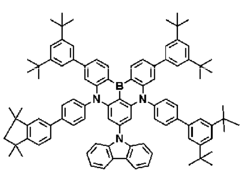
771
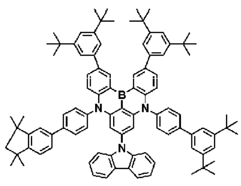
772
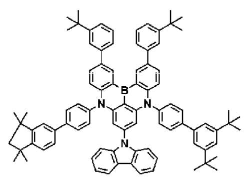
773

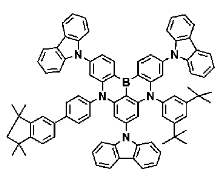
774
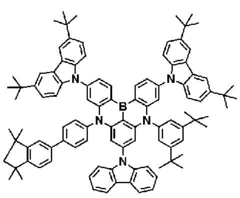
775
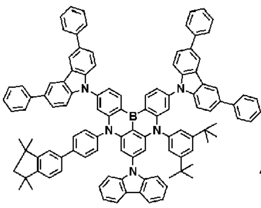
776
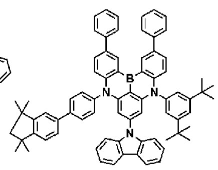
777
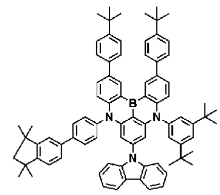
778

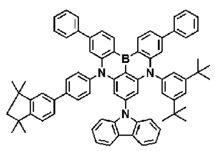
779
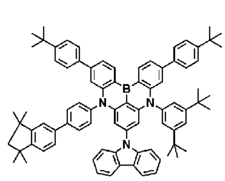
780
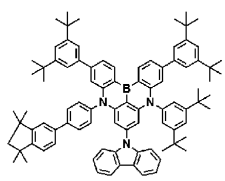
781
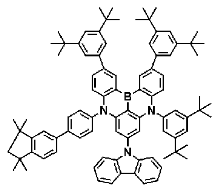
782
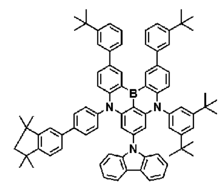
783

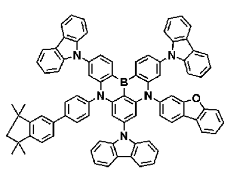
784
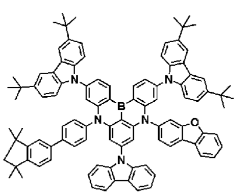
785
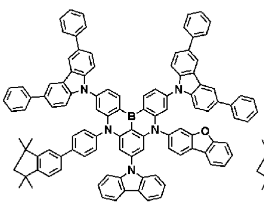
786
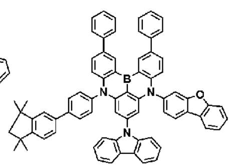
787
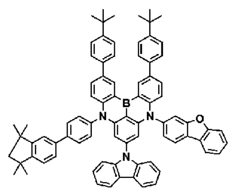
788

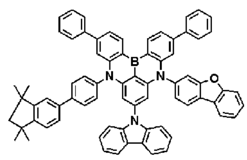
789

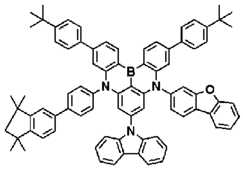
790

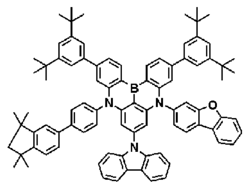
791

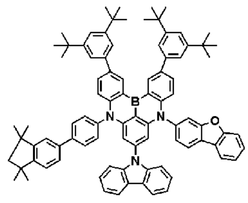
792

.

**10.** An organic light-emitting device comprising:

a first electrode;
a second electrode; and
an organic layer arranged between the first electrode and the second electrode and comprising an emission layer, wherein the organic layer comprises the heterocyclic compound of any of claims 1-9.

**11.** The organic light-emitting device of claim 10, wherein the emission layer comprises the heterocyclic compound.

**12.** The organic light-emitting device of claim 11, wherein the emission layer comprises a host and an emitter, and the emitter comprises the heterocyclic compound.

**13.** The organic light-emitting device of claim 12, wherein an amount of the host is greater than an amount of the heterocyclic compound; and/or
wherein the emission layer further comprises a sensitizer, preferably wherein the sensitizer comprises a phosphorescent compound, a delayed fluorescence compound, or any combination thereof.

**14.** The organic light-emitting device of any of claims 11-13, wherein the emission layer emits blue light having a maximum emission wavelength of 400 nm to 490 nm.

**15.** A diagnostic composition comprising the heterocyclic compound of any of claims 1-9.

**Patentansprüche**

**1.** Heterocyclische Verbindung, dargestellt durch Formel 1:

Formel 1

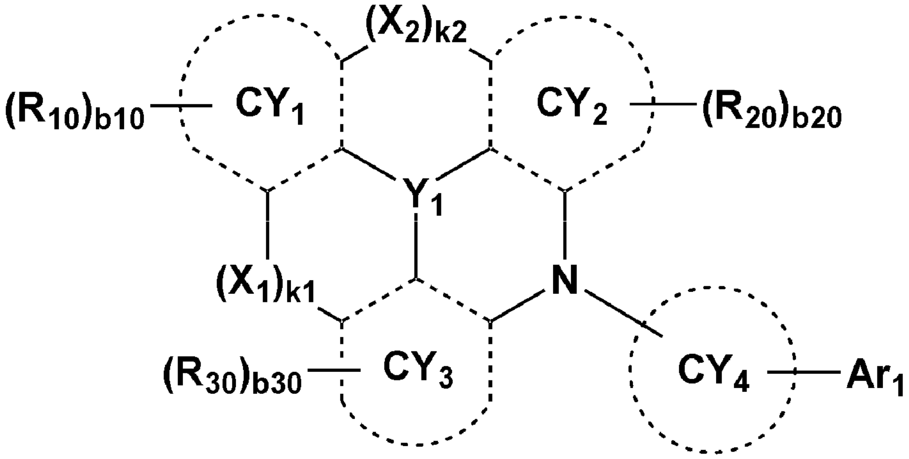

Formel 2

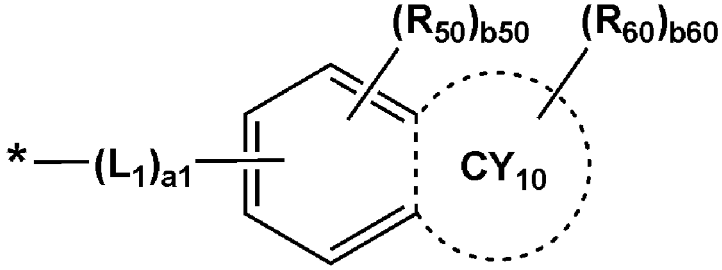

wobei in Formeln 1 und 2

$Y_1$ B, N, P, P(=O) oder P(=S) ist,

$X_1$ und $X_2$ jeweils unabhängig eine Einfachbindung, O, S, Se, N[($CY_5$)-$Ar_2$], N($R_1$), C($R_1$)($R_2$), Si($R_1$)($R_2$), Ge($R_1$)($R_2$), B($R_1$), P($R_1$), P(=O)($R_1$), $S(=O)_2$ oder C(=O) sind,

k1 0 oder 1 ist,

k2 0 oder 1 ist,

die Summe aus k1 und k2 größer als oder gleich 1 ist,

$CY_1$ bis $CY_3$ j eweils unabhängig eine carbocyclische $C_5$-$C_{30}$-Gruppe oder eine substituierte oder unsubstituierte heterocyclische $C_1$-$C_{30}$-Gruppe sind,

$CY_4$ und $CY_5$ jeweils unabhängig eine unsubstituierte oder mit mindestens einem $R_{40}$ substituierte carbocyclische $C_5$-$C_{30}$-Gruppe sind oder eine unsubstituierte oder mit mindestens einem $R_{40}$ substituierte heterocyclische $C_1$-$C_{30}$-Gruppe sind,

$Ar_1$ und $Ar_2$ jeweils unabhängig eine durch Formel 2 dargestellte Gruppe sind,

$L_1$ eine Einfachbindung, eine substituierte oder unsubstituierte carbocyclische $C_5$-$C_{30}$-Gruppe oder eine substituierte oder unsubstituierte heterocyclische $C_1$-$C_{30}$-Gruppe ist,

a1 1, 2 oder 3 ist,

$CY_{10}$ eine nicht-aromatische carbocyclische $C_3$-$C_{10}$-Gruppe oder eine nicht-aromatische heterocyclische $C_1$-$C_{10}$-Gruppe ist,

$R_1$, $R_2$, $R_{10}$, $R_{20}$, $R_{30}$ und $R_{40}$ jeweils unabhängig eine durch Formel 2 dargestellte Gruppe, Wasserstoff, Deuterium, -F, -Cl, -Br, -I, -$SF_5$, eine Hydroxygruppe, eine Cyanogruppe, eine Nitrogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_1$-$C_{10}$-Heterocycloalkylgruppe, eine substituierte oder unsubstituierte $C_3$-$C_{10}$-Cycloalkenylgruppe, eine substituierte oder unsubstituierte $C_1$-$C_{10}$-Heterocycloalkenylgruppe, eine substituierte oder unsubstituierte $C_6$-$C_{60}$-Arylgruppe, eine substituierte oder unsubstituierte $C_7$-$C_{60}$-Alkylarylgruppe, eine substituierte oder unsubstituierte $C_6$-$C_{60}$-Aryloxygruppe, eine substituierte oder unsubstituierte $C_6$-$C_{60}$-Arylthiogruppe, eine substituierte oder unsubstituierte $C_1$-$C_{60}$-Heteroarylgruppe, eine substituierte oder unsubstituierte $C_2$-$C_{60}$-Alkylheteroarylgruppe, eine substituierte oder unsubstituierte $C_1$-$C_{60}$-Heteroaryloxygruppe, eine substituierte oder unsubstituierte $C_1$-$C_{60}$-Heteroarylthiogruppe, eine substituierte oder unsubstituierte einwertige nicht-aromatische kondensierte polycyclische Gruppe, eine substituierte oder unsubstituierte einwertige nicht-aromatische kondensierte heteropolycyclische Gruppe, -N($Q_1$)($Q_2$), -Si($Q_1$)($Q_2$)($Q_3$), -Ge($Q_1$)($Q_2$)($Q_3$), -C(=O)($Q_1$), - S(=O)($Q_1$), -$S(=O)_2$($Q_1$), -B($Q_1$)($Q_2$), -P($Q_1$)($Q_2$), -P(=O)($Q_1$)($Q_2$), -P(=S)($Q_1$)($Q_2$) oder eine durch Formel 3 dargestellte Gruppe sind,

$R_{50}$ und $R_{60}$ jeweils unabhängig Wasserstoff, Deuterium, -F, -Cl, -Br, -I, -$SF_5$, eine Hydroxygruppe, eine Cyanogruppe, eine Nitrogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_1$-$C_{10}$-Heterocycloalkylgruppe, eine substituierte oder unsubstituierte $C_3$-$C_{10}$-Cycloalkenylgruppe, eine substituierte oder unsubstituierte $C_1$-$C_{10}$-Heterocycloalkenylgruppe, eine substituierte oder unsubstituierte $C_6$-$C_{60}$-Arylgruppe, eine substituierte oder unsubstituierte $C_7$-$C_{60}$-Alkylarylgruppe, eine substituierte oder unsubstituierte $C_6$-$C_{60}$-Aryloxygruppe, eine substituierte oder unsubstituierte

$C_6$-$C_{60}$-Arylthiogruppe, eine substituierte oder unsubstituierte $C_1$-$C_{60}$-Heteroarylgruppe, eine substituierte oder unsubstituierte $C_2$-$C_{60}$-Alkylheteroarylgruppe, eine substituierte oder unsubstituierte $C_1$-$C_{60}$-Heteroaryloxygruppe, eine substituierte oder unsubstituierte $C_1$-$C_{60}$-Heteroarylthiogruppe, eine substituierte oder unsubstituierte einwertige nicht-aromatische kondensierte polycyclische Gruppe, eine substituierte oder unsubstituierte einwertige nicht-aromatische kondensierte heteropolycyclische Gruppe, $-N(Q_1)(Q_2)$, $-Si(Q_1)(Q_2)(Q_3)$, $-Ge(Q_1)(Q_2)(Q_3)$, $-C(=O)(Q_1)$, $-S(=O)(Q_1)$, $-S(=O)_2(Q_1)$, $-B(Q_1)(Q_2)$, $-P(Q_1)(Q_2)$, $-P(=O)(Q_1)(Q_2)$, $-P(=S)(Q_1)(Q_2)$ oder eine durch Formel 3 dargestellte Gruppe sind,

wobei mindestens eines von $R_{10}$, mindestens eines von $R_{20}$ und/oder mindestens eines von $R_{30}$ eine durch Formel 3 dargestellte Gruppe ist;

Formel 3

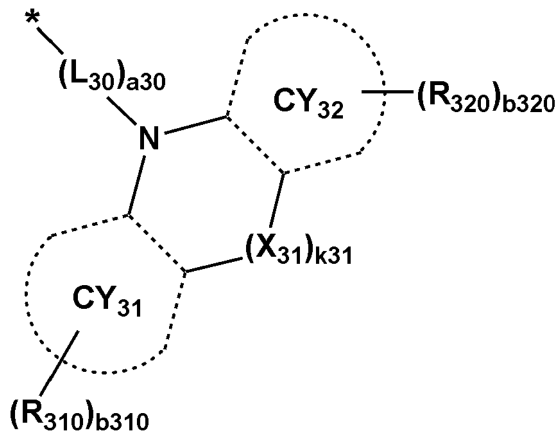

wobei in Formel 3

$X_{31}$ eine Einfachbindung, O, S, $N(R_{301})$ oder $C(R_{301})(R_{302})$ ist,

k31 gleich 1 ist,

$CY_{31}$ and $CY_{32}$ jeweils unabhängig eine carbocyclische $C_5$-$C_{30}$-Gruppe oder eine heterocyclische $C_1$-$C_{30}$-Gruppe sind,

$L_{30}$ eine Einfachbindung, eine substituierte oder unsubstituierte carbocyclische $C_5$-$C_{30}$-Gruppe oder eine substituierte oder unsubstituierte heterocyclische $C_1$-$C_{30}$-Gruppe ist,

a30 0, 1, 2 oder 3 ist,

$R_{301}$, $R_{302}$, $R_{310}$ und $R_{320}$ jeweils unabhängig Wasserstoff, Deuterium, -F, -Cl, -Br, -I, $-SF_5$, eine Hydroxygruppe, eine Cyanogruppe, eine Nitrogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_1$-$C_{10}$-Heterocycloalkylgruppe, eine substituierte oder unsubstituierte $C_3$-$C_{10}$-Cycloalkenylgruppe, eine substituierte oder unsubstituierte $C_1$-$C_{10}$-Heterocycloalkenylgruppe, eine substituierte oder unsubstituierte $C_6$-$C_{60}$-Arylgruppe, eine substituierte oder unsubstituierte $C_7$-$C_{60}$-Alkylarylgruppe, eine substituierte oder unsubstituierte $C_6$-$C_{60}$-Aryloxygruppe, eine substituierte oder unsubstituierte $C_6$-$C_{60}$-Arylthiogruppe, eine substituierte oder unsubstituierte $C_1$-$C_{60}$-Heteroarylgruppe, eine substituierte oder unsubstituierte $C_2$-$C_{60}$-Alkylheteroarylgruppe, eine substituierte oder unsubstituierte $C_1$-$C_{60}$-Heteroaryloxygruppe, eine substituierte oder unsubstituierte $C_1$-$C_{60}$-Heteroarylthiogruppe, eine substituierte oder unsubstituierte einwertige nicht-aromatische kondensierte polycyclische Gruppe, eine substituierte oder unsubstituierte einwertige nicht-aromatische kondensierte heteropolycyclische Gruppe, $-N(Q_1)(Q_2)$, $-Si(Q_1)(Q_2)(Q_3)$, $-Ge(Q_1)(Q_2)(Q_3)$, $-C(=O)(Q_1)$, $-S(=O)(Q_1)$, $-S(=O)_2(Q_1)$, $-B(Q_1)(Q_2)$, $-P(Q_1)(Q_2)$, $-P(=O)(Q_1)(Q_2)$ oder $-P(=S)(Q_1)(Q_2)$ sind,

b310 und b320 jeweils unabhängig 1, 2, 3, 4, 5, 6, 7 oder 8 sind,

b10, b20, b30, b40 und b60 jeweils unabhängig 1, 2, 3, 4, 5, 6, 7 oder 8 sind,

b50 1, 2 oder 3 ist,

ein Substituent der substituierten carbocyclischen $C_5$-$C_{30}$-Gruppe, der substituierten heterocyclischen $C_1$-$C_{30}$-Gruppe, der substituierten $C_1$-$C_{60}$-Alkylgruppe, der substituierten $C_2$-$C_{60}$-Alkenylgruppe, der sub-

stituierten $C_2$-$C_{60}$-Alkinylgruppe, der substituierten $C_1$-$C_{60}$-Alkoxygruppe, der substituierten $C_3$-$C_{10}$-Cycloalkylgruppe, der substituierten $C_1$-$C_{10}$-Heterocycloalkylgruppe, der substituierten $C_3$-$C_{10}$-Cycloalkenylgruppe, der substituierten $C_1$-$C_{10}$-Heterocycloalkenylgruppe, der substituierten $C_6$-$C_{60}$-Arylgruppe, der substituierten $C_7$-$C_{60}$-Alkylarylgruppe, der substituierten $C_6$-$C_{60}$-Aryloxygruppe, der substituierten $C_6$-$C_{60}$-Arylthiogruppe, der substituierten $C_1$-$C_{60}$-Heteroarylgruppe, der $C_2$-$C_{60}$-Alkylheteroarylgruppe, der substituierten $C_1$-$C_{60}$-Heteroaryloxygruppe, der substituierten $C_1$-$C_{60}$-Heteroarylthiogruppe, der substituierten einwertigen nicht-aromatischen kondensierten polycyclischen Gruppe und der substituierten einwertigen nicht-aromatischen kondensierten heteropolycyclischen Gruppe Folgendes ist:

Deuterium, -F, -Cl, -Br, -I, $CD_3$, $-CD_2H$, $-CDH_2$, $-CF_3$, $-CF_2H$, $-CFH_2$, eine Hydroxygruppe, eine Cyanogruppe, eine Nitrogruppe, eine Aminogruppe, eine Amidinogruppe, eine Hydrazingruppe, einer Hydrazongruppe, eine Carbonsäure oder ein Salz davon, eine Sulfonsäure oder ein Salz davon, eine Phosphorsäure oder ein Salz davon, eine $C_1$-$C_{60}$-Alkylgruppe, eine $C_2$-$C_{60}$-Alkenylgruppe, eine $C_2$-$C_{60}$-Alkinylgruppe oder eine $C_1$-$C_{60}$-Alkoxygruppe;
eine $C_1$-$C_{60}$-Alkylgruppe, eine $C_2$-$C_{60}$-Alkenylgruppe, eine $C_2$-$C_{60}$-Alkinylgruppe oder eine $C_1$-$C_{60}$-Alkoxygruppe, jeweils substituiert mit Deuterium, -F, -Cl, -Br, -I, $-CD_3$, $-CD_2H$, - $CDH_2$, $-CF_3$, $-CF_2H$, $-CFH_2$, einer Hydroxygruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer $C_3$-$C_{10}$-Cycloalkylgruppe, einer $C_1$-$C_{10}$-Heterocycloalkylgruppe, einer $C_3$-$C_{10}$-Cycloalkenylgruppe, einer $C_1$-$C_{10}$-Heterocycloalkenylgruppe, einer $C_6$-$C_{60}$-Arylgruppe, einer $C_6$-$C_{60}$-Aryloxygruppe, einer $C_6$-$C_{60}$-Arylthiogruppe, einer $C_1$-$C_{60}$-Heteroarylgruppe, einer einwertigen nicht-aromatischen kondensierten polycyclischen Gruppe, einer einwertigen nicht-aromatischen kondensierten heteropolycyclischen Gruppe, $-N(Q_{11})(Q_{12})$, $-Si(Q_{11})(Q_{12})(Q_{13})$, $-Ge(Q_{11})(Q_{12})(Q_{13})$, $-C(=O)(Q_{11})$, $-S(=O)(Q_{11})$, $-S(=O)_2(Q_{11})$, $-B(Q_{11})(Q_{12})$, $-P(Q_{11})(Q_{12})$, $-P(=O)(Q_{11})(Q_{12})$, $-P(=S)(Q_{11})(Q_{12})$ oder einer beliebigen Kombination davon;
eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_1$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine einwertige nicht-aromatische kondensierte polycyclische Gruppe oder eine einwertige nicht-aromatische kondensierte heteropolycyclische Gruppe, jeweils unsubstituiert oder substituiert mit Deuterium, -F, -Cl, -Br, -I, $-CD_3$, $-CD_2H$, $-CDH_2$, $-CF_3$, $-CF_2H$, $-CFH_2$, einer Hydroxygruppe, einer Cyanogruppe, einer Nitrogruppe, einer Aminogruppe, einer Amidinogruppe, einer Hydrazingruppe, einer Hydrazongruppe, einer Carbonsäure oder einem Salz davon, einer Sulfonsäure oder einem Salz davon, einer Phosphorsäure oder einem Salz davon, einer $C_1$-$C_{60}$-Alkylgruppe, einer $C_2$-$C_{60}$-Alkenylgruppe, einer $C_2$-$C_{60}$-Alkinylgruppe, einer $C_1$-$C_{60}$-Alkoxygruppe, einer $C_3$-$C_{10}$-Cycloalkylgruppe, einer $C_1$-$C_{10}$-Heterocycloalkylgruppe, einer $C_3$-$C_{10}$-Cycloalkenylgruppe, einer $C_1$-$C_{10}$-Heterocycloalkenylgruppe, einer $C_6$-$C_{60}$-Arylgruppe, einer $C_6$-$C_{60}$-Aryloxygruppe, einer $C_6$-$C_{60}$-Arylthiogruppe, einer $C_1$-$C_{60}$-Heteroarylgruppe, einer einwertigen nicht-aromatischen kondensierten polycyclischen Gruppe, einer einwertigen nicht-aromatischen kondensierten heteropolycyclischen Gruppe, $-N(Q_{21})(Q_{22})$, $-Si(Q_{21})(Q_{22})(Q_{23})$, - $Ge(Q_{21})(Q_{22})(Q_{23})$, $-C(=O)(Q_{21})$, $-S(=O)(Q_{21})$, $-S(=O)_2(Q_{21})$, $-B(Q_{21})(Q_{22})$, $-P(Q_{21})(Q_{22})$, - $P(=O)(Q_{21})(Q_{22})$, $-P(=S)(Q_{21})(Q_{22})$ oder einer beliebigen Kombination davon;
$-N(Q_{31})(Q_{32})$, $-Si(Q_{31})(Q_{32})(Q_{33})$, $-Ge(Q_{31})(Q_{32})(Q_{33})$, $-C(=O)(Q_{31})$, $-S(=O)(Q_{31})$, - $S(=O)_2(Q_{31})$, $-B(Q_{31})(Q_{32})$, $-P(Q_{31})(Q_{32})$, $-P(=O)(Q_{31})(Q_{32})$ oder $-P(Q_{31})(Q_{32})$;
$Q_1$ bis $Q_3$, $Q_{11}$ bis $Q_{13}$, $Q_{21}$ bis $Q_{23}$ und $Q_{31}$ bis $Q_{33}$ sind jeweils unabhängig: Wasserstoff, Deuterium, -F, -Cl, -Br, -I, eine Hydroxygruppe, eine Cyanogruppe, eine Nitrogruppe, eine Amidinogruppe, eine Hydrazingruppe, eine Hydrazongruppe, eine Carbonsäure oder ein Salz davon, eine Sulfonsäure oder ein Salz davon, eine Phosphorsäure oder ein Salz davon, eine $C_1$-$C_{60}$-Alkylgruppe, die unsubstituiert oder substitutiert ist mit Deuterium, einer $C_1$-$C_{60}$-Alkylgruppe, einer $C_6$-$C_{60}$-Arylgruppe oder einer beliebigen Kombination davon, eine $C_2$-$C_{60}$-Alkenylgruppe, eine $C_2$-$C_{60}$-Alkinylgruppe, eine $C_1$-$C_{60}$-Alkoxygruppe, eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_1$-$C_{10}$-Heterocycloalkenylgruppe, eine $C_6$-$C_{60}$-Arylgruppe, die unsubstituiert oder substituiert ist mit Deuterium, einer $C_1$-$C_{60}$-Alkylgruppe, einer $C_6$-$C_{60}$-Arylgruppe oder einer beliebigen Kombination davon, eine $C_6$-$C_{60}$-Aryloxygruppe, eine $C_6$-$C_{60}$-Arylthiogruppe, eine $C_1$-$C_{60}$-Heteroarylgruppe, eine einwertige nicht-aromatische kondensierte polycyclische Gruppe, oder eine einwertige nicht-aromatische kondensierte heteropolycyclische Gruppe, und
* zeigt eine Bindungsstelle zu einem benachbarten Atom an.

2. Heterocyclische Verbindung nach Anspruch 1, wobei die heterocyclische Verbindung durch Formel 1A oder 1B dargestellt ist:

Formel 1A

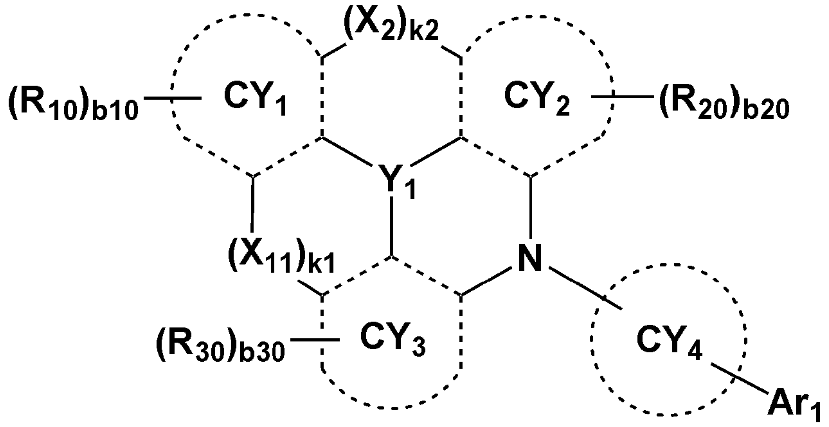

Formel 1B

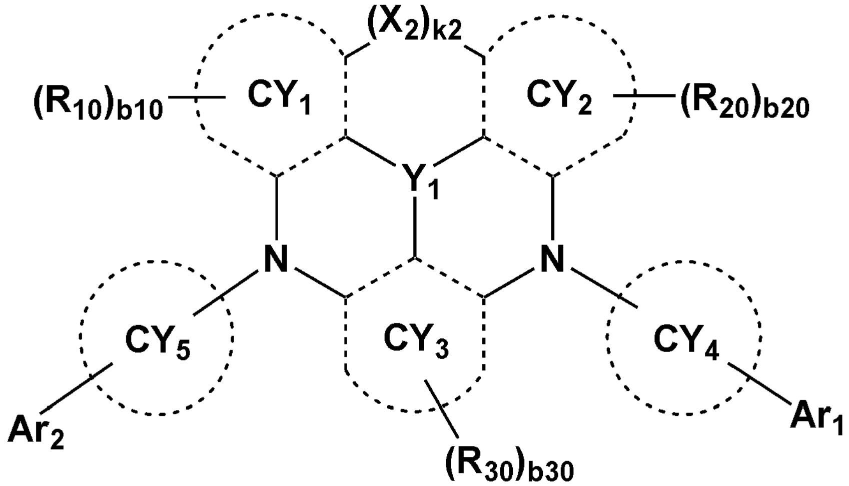

wobei in Formeln 1A und 1B,

$X_{11}$ eine Einfachbindung, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$ oder C(=O) ist, und
$Y_1$, $X_2$, k1, k2, $CY_1$ bis $CY_5$, $Ar_1$, $Ar_2$, $R_1$, $R_2$, $R_{10}$, $R_{20}$, $R_{30}$, b10, b20 und b30 jeweils dieselben wie die in Anspruch 1 beschriebenen sein können.

3. Heterocyclische Verbindung nach Anspruch 1 oder 2, wobei $CY_1$ bis $CY_3$ jeweils unabhängig eine aromatische carbocyclische $C_6$-$C_{30}$-Gruppe oder eine aromatische heterocyclische $C_1$-$C_{30}$-Gruppe sind; und/oder

wobei $CY_4$ und $CY_5$ jeweils unabhängig durch eine von Formeln 4-1 bis 4-3 dargestellt sind:

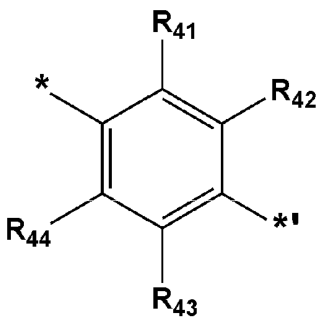

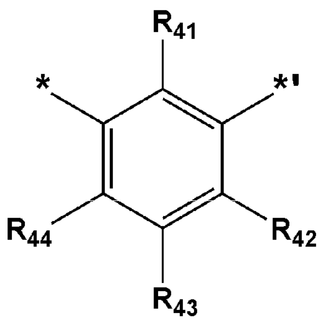

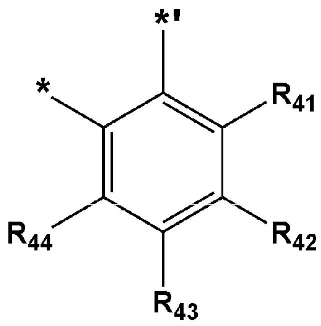

4-1 4-2 4-3

wobei, in Formeln 4-1 bis 4-3,
$R_{41}$ bis $R_{44}$ jeweils unabhängig dieselben wie die in Zusammenhang mit $R_{40}$ in Anspruch 1 beschriebenen sind,
* und *' jeweils eine Bindungsstelle zu einem benachbarten Atom anzeigen.

4. Heterocyclische Verbindung nach einem der Ansprüche 1 bis 3, wobei $CY_{10}$ eine Cyclopentangruppe, eine Cyclohexangruppe, eine Cycloheptangruppe, eine Cyclopentengruppe, eine Cyclohexengruppe oder eine Cycloheptengruppe ist; und/oder

wobei $Ar_1$ und $Ar_2$ jeweils unabhängig eine durch Formel 2A oder 2B dargestellte Gruppe sind:

Formel 2A

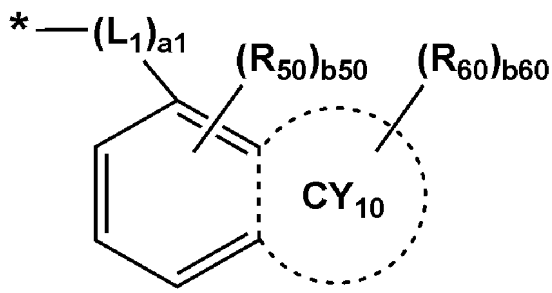

Formel 2B

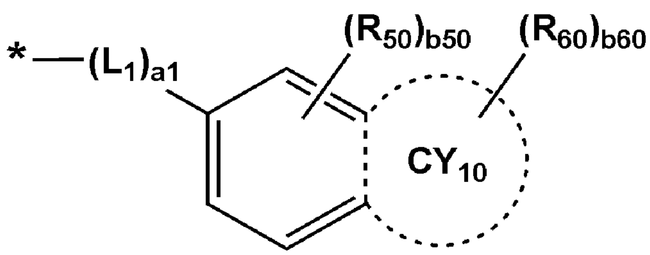

wobei in Formeln 2A und 2B,
$L_1$, a1, $CY_{10}$, $R_{50}$, $R_{60}$, b50 und b60 jeweils dieselben wie die in Anspruch 1 beschriebenen sind, und
* eine Bindungsstelle zu einem benachbarten Atom anzeigt.

5. Heterocyclische Verbindung nach einem der Ansprüche 1 bis 4, wobei $Ar_1$ und $Ar_2$ jeweils unabhängig eine Gruppe sind, die durch eine von Formeln 5-1 bis 5-4 dargestellt ist:

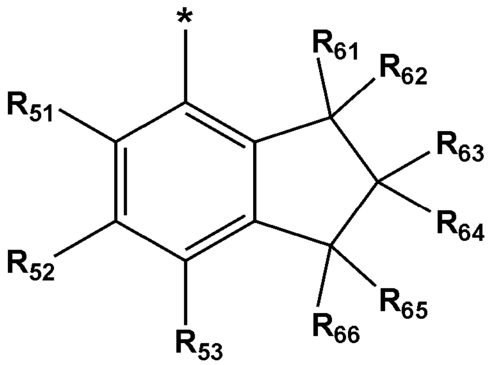

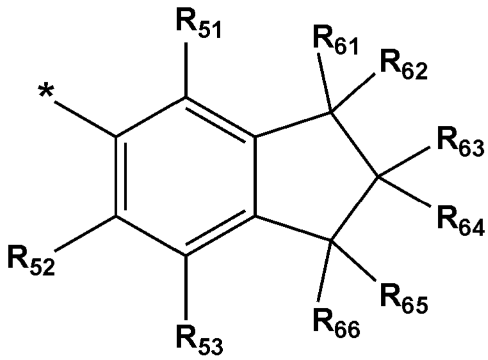

5-1 5-2

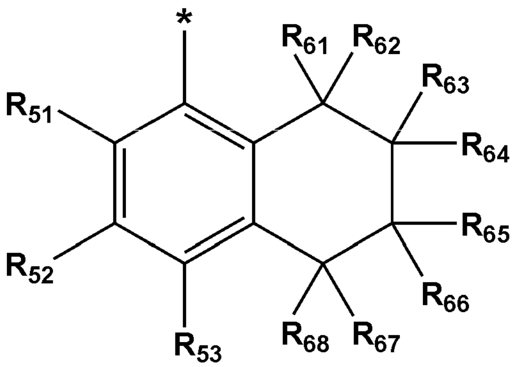

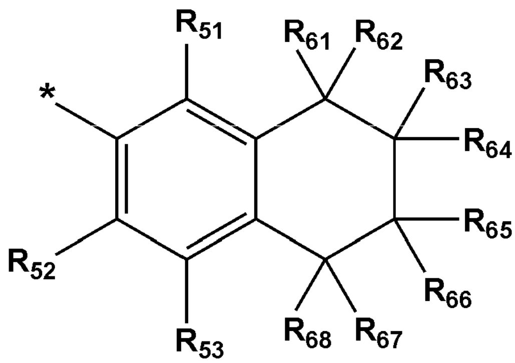

5-3 5-4

wobei, in Formeln 5-1 bis 5-4,

$R_{51}$ bis $R_{53}$ jeweils dieselben wie die in Zusammenhang mit $R_{50}$ in Anspruch 1 beschriebenen sind,
$R_{61}$ bis $R_{68}$ jeweils dieselben wie die in Zusammenhang mit $R_{60}$ in Anspruch 1 beschriebenen sind, und
* eine Bindungsstelle zu einem benachbarten Atom anzeigt.

**6.** Heterocyclische Verbindung nach einem der Ansprüche 1 bis 5, wobei eine durch Formel 3 dargestellte Gruppe eine durch eine von Formeln 3-2 oder 3-3 dargestellte Gruppe ist:

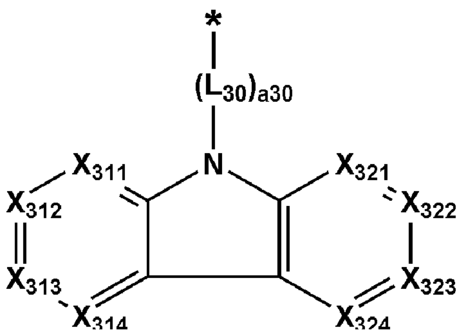

3-2

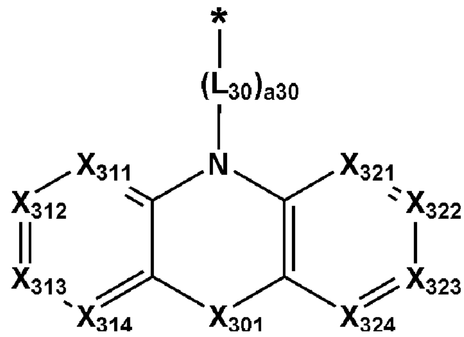

**3-3**

wobei in Formeln 3-2 oder 3-3,

$L_{30}$ und a30 jeweils dieselben wie die in Anspruch 1 beschriebenen sind,
$X_{301}$ dieselbe wie die in Zusammenhang mit X31 in Anspruch 1 beschriebene ist,
$X_{311}$ für $C(R_{311})$ oder N steht, $X_{312}$ für $C(R_{312})$ oder N steht, $X_{313}$ für $C(R_{313})$ oder N steht, $X_{314}$ für $C(R_{314})$ oder N steht,
$X_{321}$ für $C(R_{321})$ oder N steht, $X_{322}$ für $C(R_{322})$ oder N steht, $X_{323}$ für $C(R_{323})$ oder N steht, $X_{324}$ für $C(R_{324})$ oder N steht,
$R_{311}$ bis $R_{314}$ jeweils unabhängig dieselben wie die in Zusammenhang mit $R_{310}$ in Anspruch 1 beschriebenen sind,
$R_{321}$ bis $R_{324}$ jeweils unabhängig dieselben wie die in Zusammenhang mit $R_{320}$ in Anspruch 1 beschriebenen sind, und
* eine Bindungsstelle zu einem benachbarten Atom anzeigt.

**7.** Heterocyclische Verbindung nach einem der Ansprüche 1 bis 6, wobei die heterocyclische Verbindung durch eine von Formeln 11-1 bis 11-9 dargestellt ist:

Formel 11-1

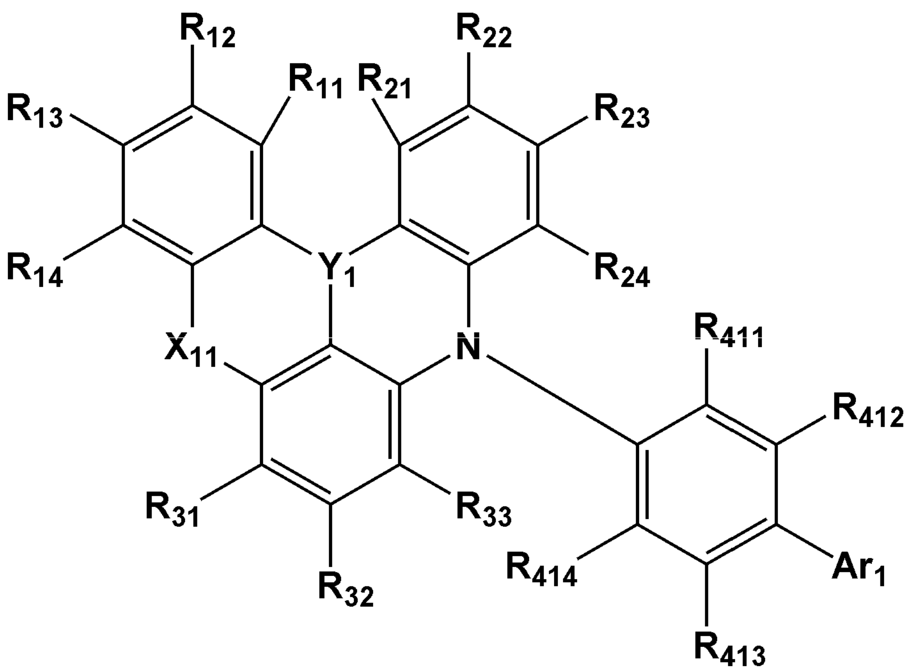

Formel 11-2

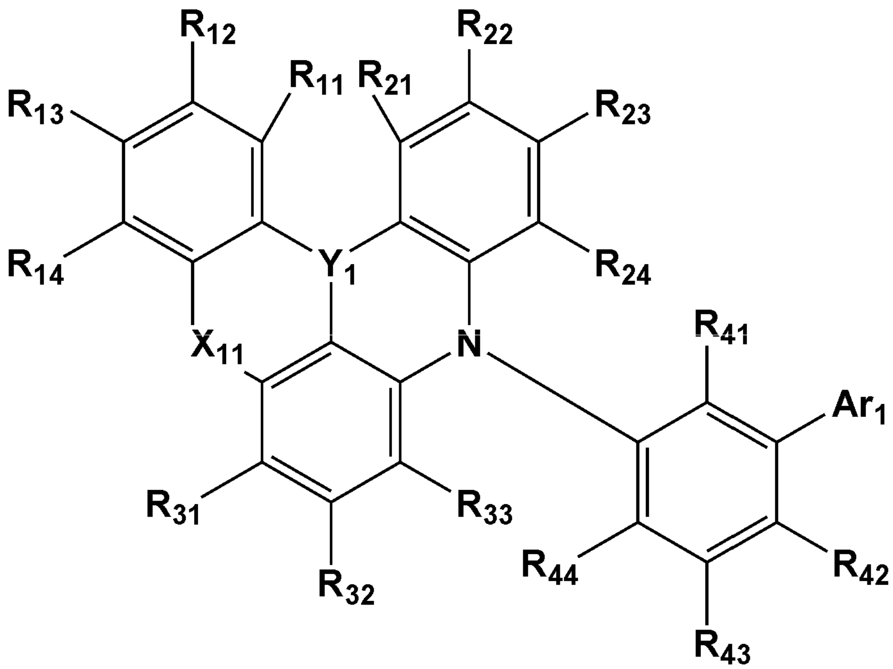

Formel 11-3

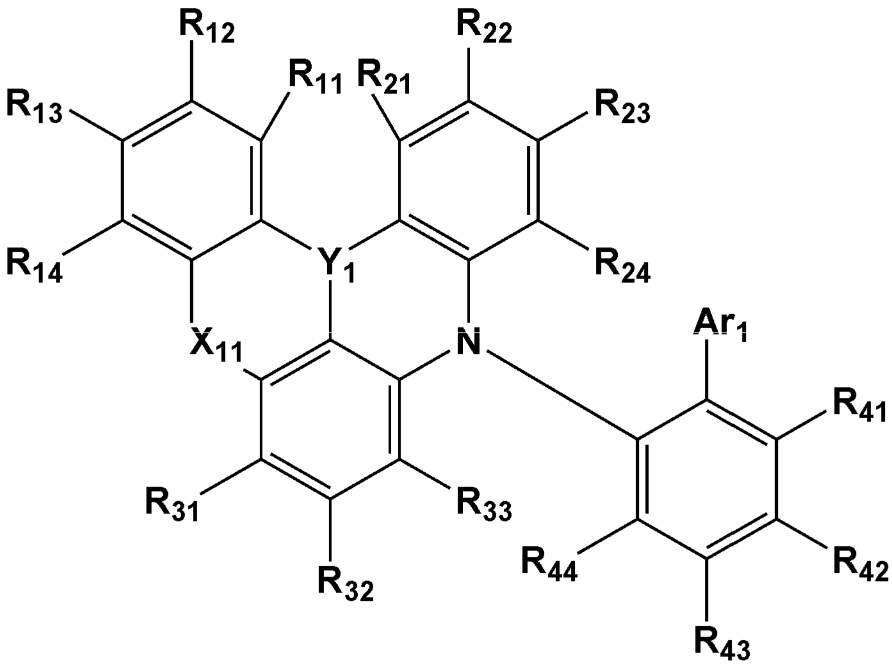

Formel 11-4

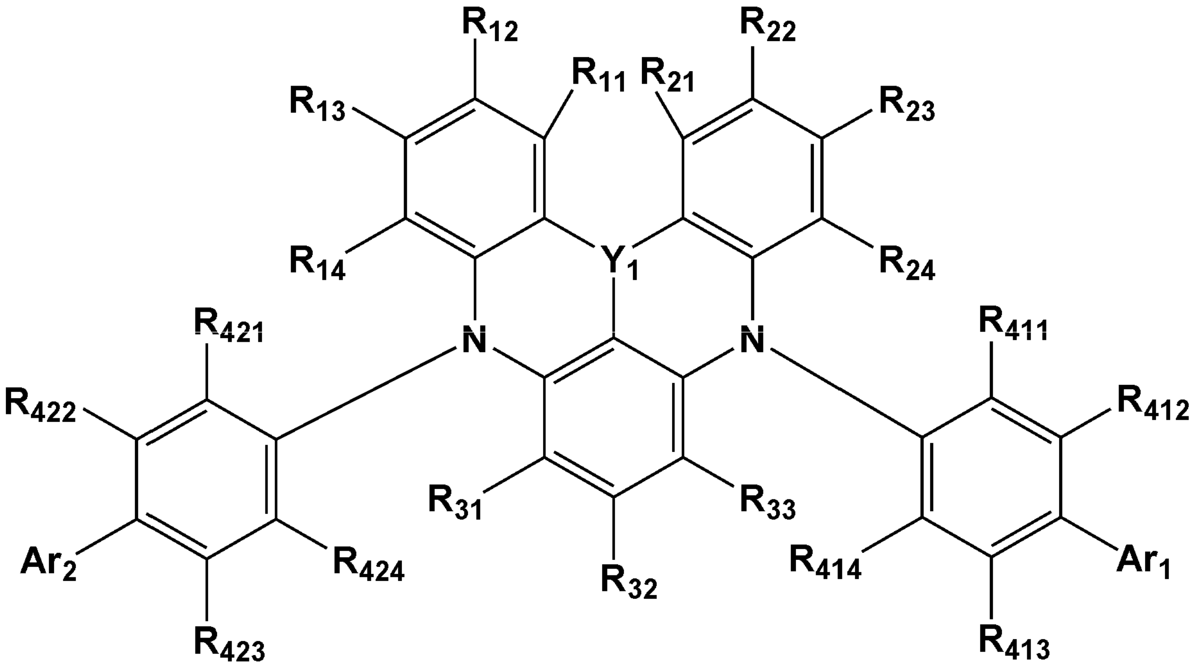

Formel 11-5

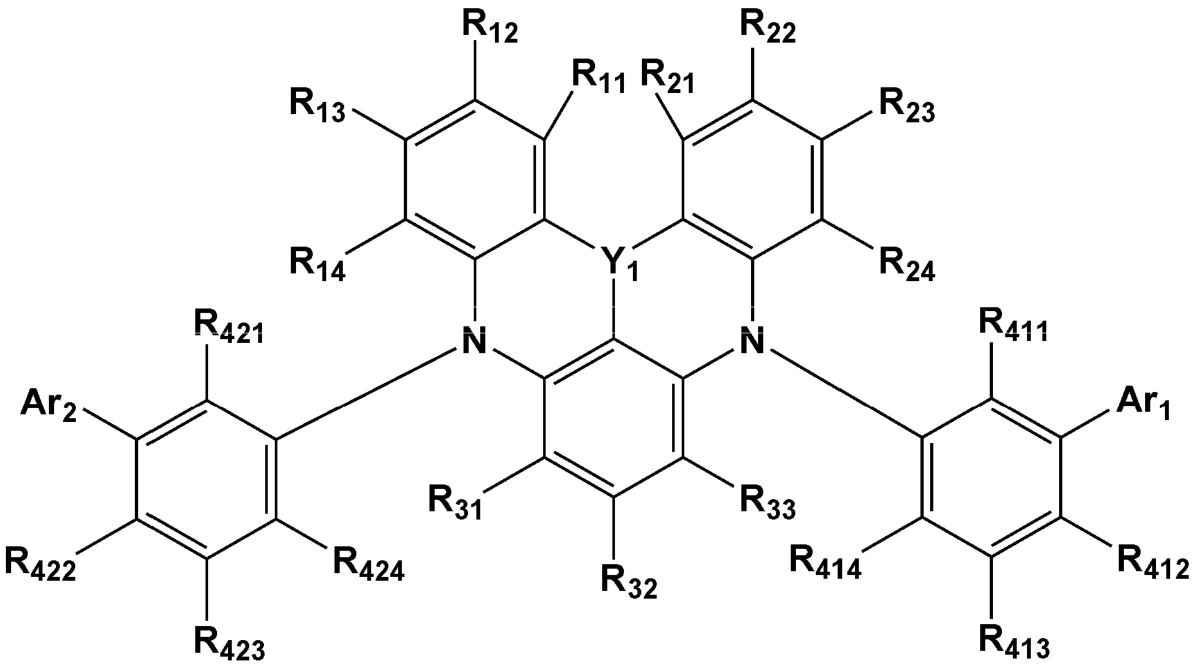

Formel 11-6

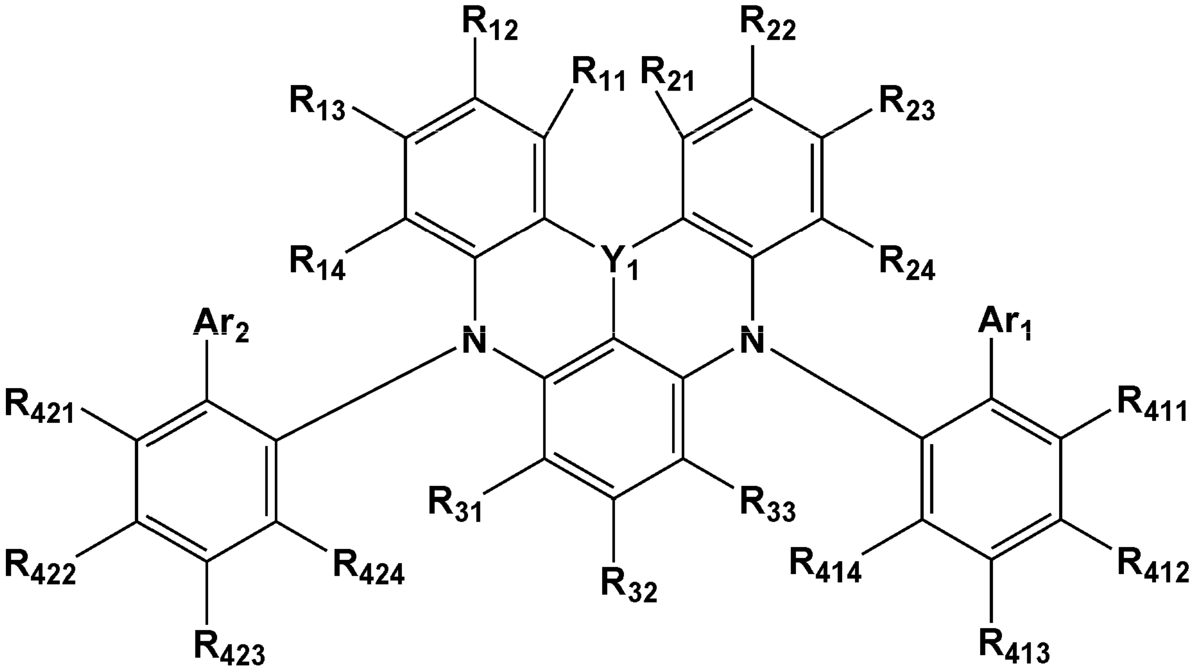

Formel 11-7

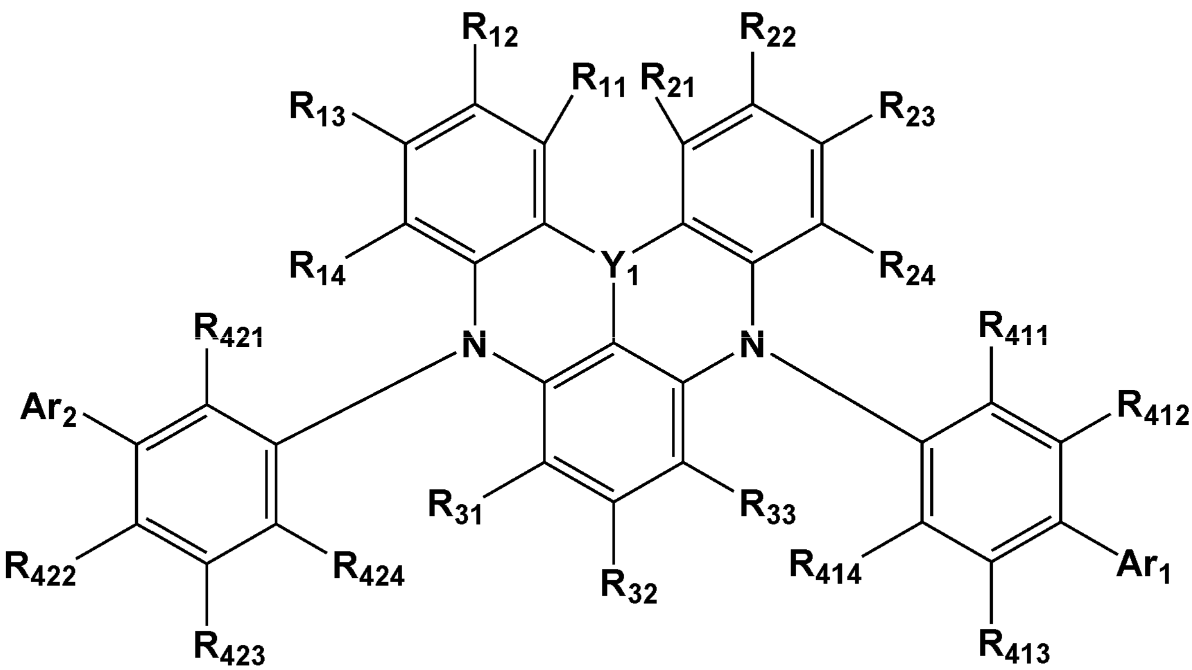

Formel 11-8

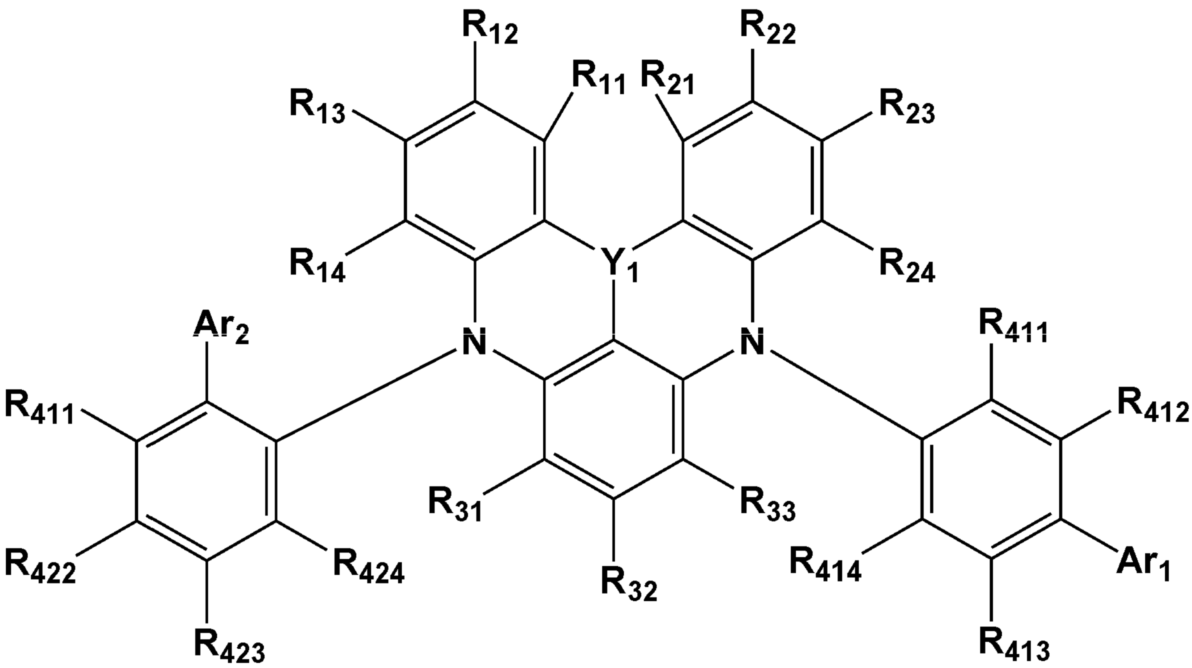

Formel 11-9

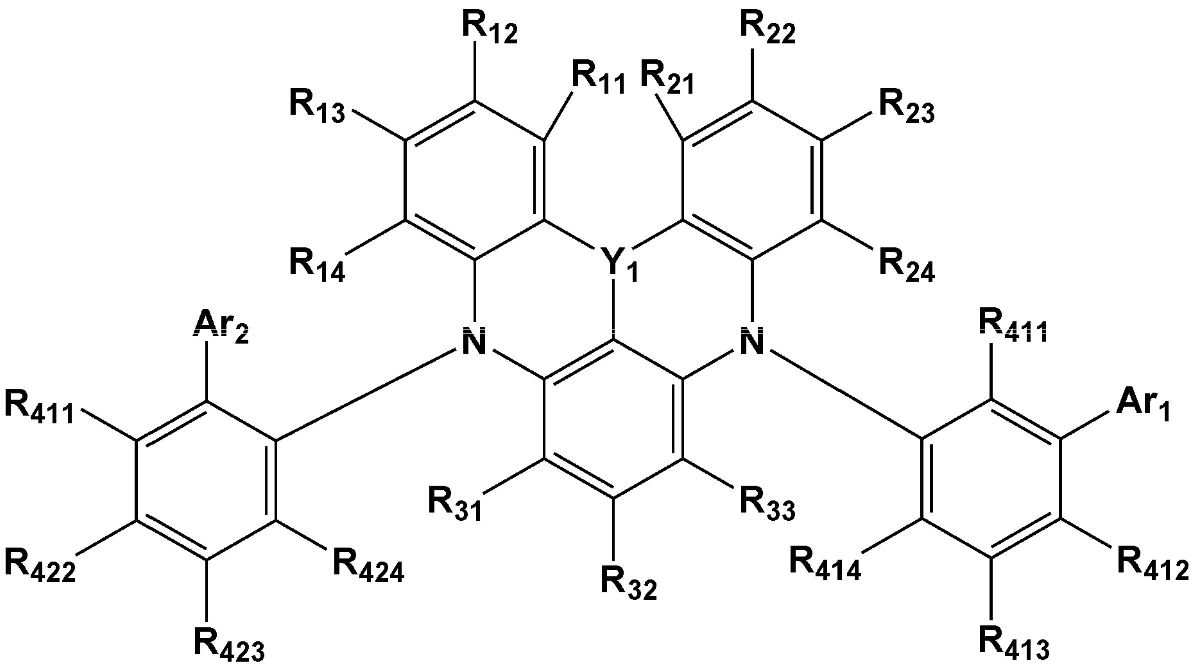

wobei, in Formeln 11-1 bis 11-9,

$Y_1$, $Ar_1$ und $Ar_2$ jeweils dieselben wie die in Anspruch 1 beschriebenen sind,
$X_{11}$ eine Einfachbindung, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$ oder C(=O) ist, $R_1$ und $R_2$ dieselben wie die in Anspruch 1 beschriebenen sind,
$R_{11}$ bis $R_{14}$ jeweils unabhängig dieselben wie die in Zusammenhang mit $R_{10}$ in Anspruch 1 beschriebenen sind,
$R_{21}$ bis $R_{24}$ jeweils unabhängig dieselben wie die in Zusammenhang mit $R_{20}$ in Anspruch 1 beschriebenen sind,

$R_{31}$ bis $R_{33}$ jeweils unabhängig dieselben wie die in Zusammenhang mit $R_{30}$ in Anspruch 1 beschriebenen sind, $R_{411}$ bis $R_{414}$ und $R_{421}$ bis $R_{424}$ jeweils unabhängig dieselben wie die in Zusammenhang mit $R_{40}$ in Anspruch 1 beschriebenen sind.

**8.** Heterocyclische Verbindung nach einem der Ansprüche 1 bis 7, wobei die heterocyclische Verbindung eine symmetrische Struktur oder eine asymmetrische Struktur aufweist.

**9.** Heterocyclische Verbindung nach einem der Ansprüche 1 bis 8, wobei die heterocyclische Verbindung eine von Verbindungen 1 bis 792 ist:

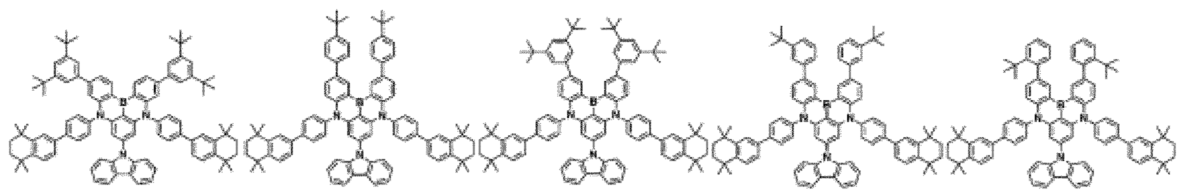

1 2 3 4 5

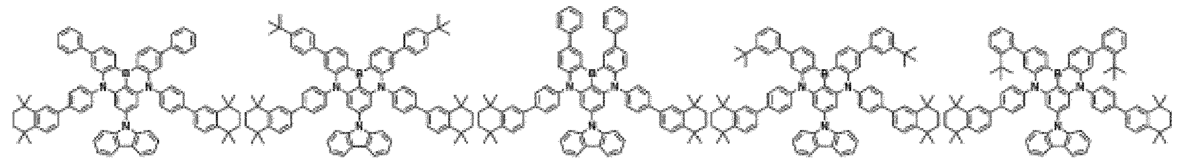

6 7 8 9 10

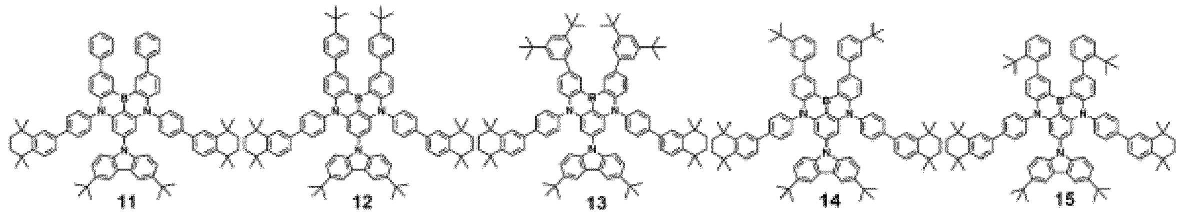

11 12 13 14 15

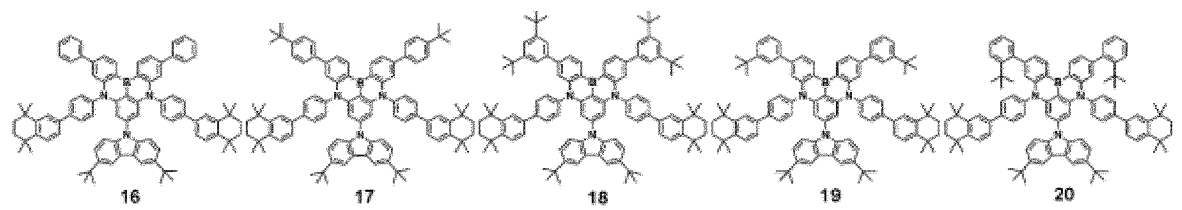

16 17 18 19 20

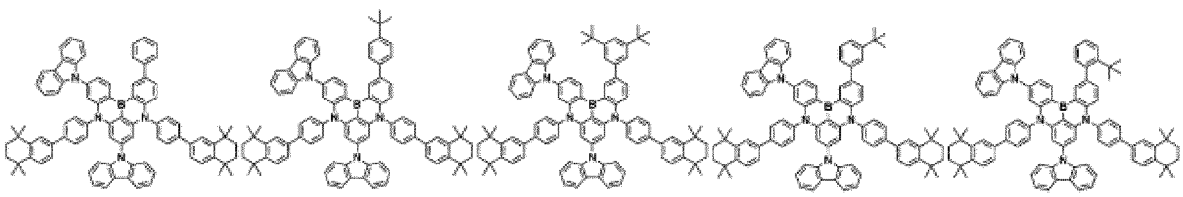

21 22 23 24 25

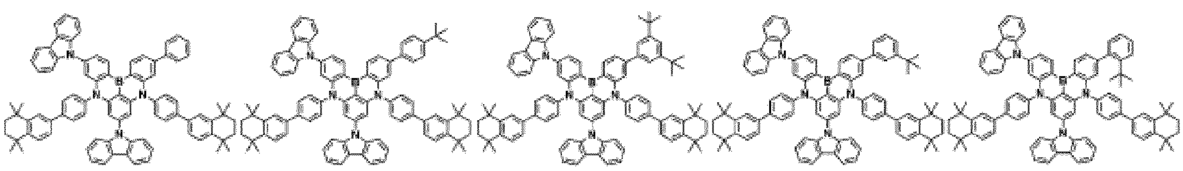

26 27 28 29 30

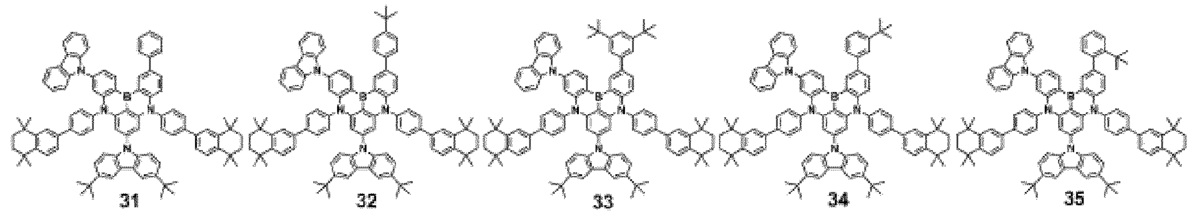

31 32 33 34 35

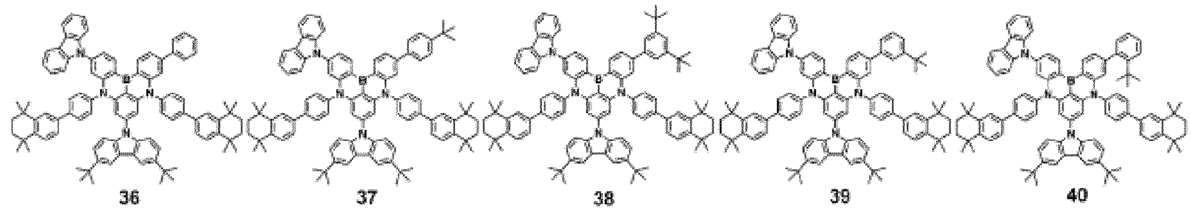

36 37 38 39 40

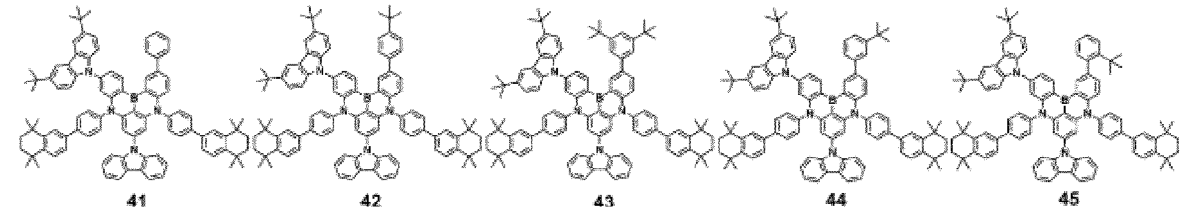

41 42 43 44 45

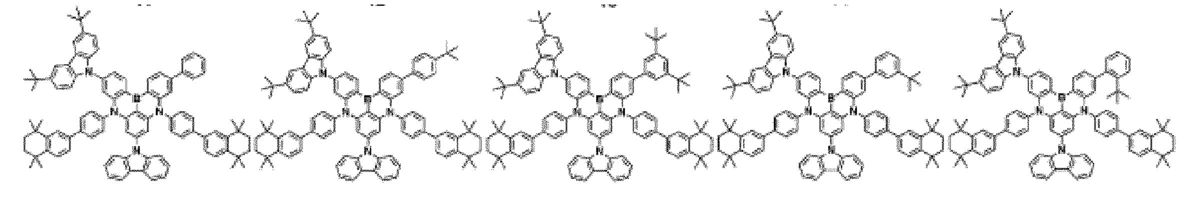

46 47 48 49 50

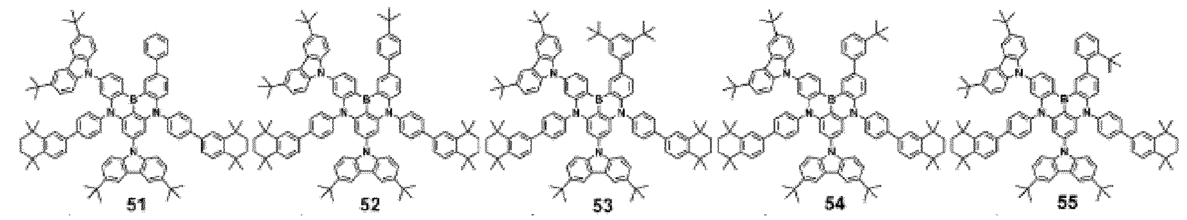

51 52 53 54 55

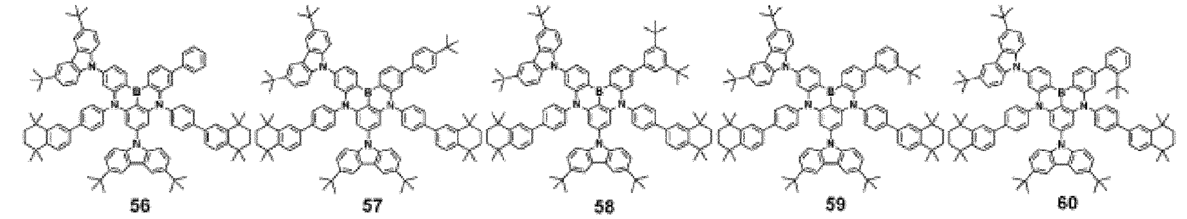

56 57 58 59 60

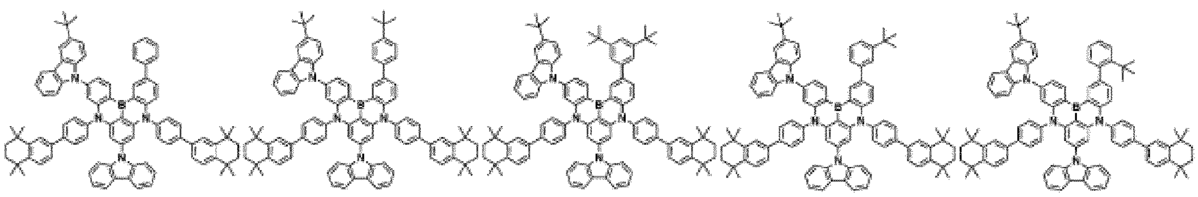

61 62 63 64 65

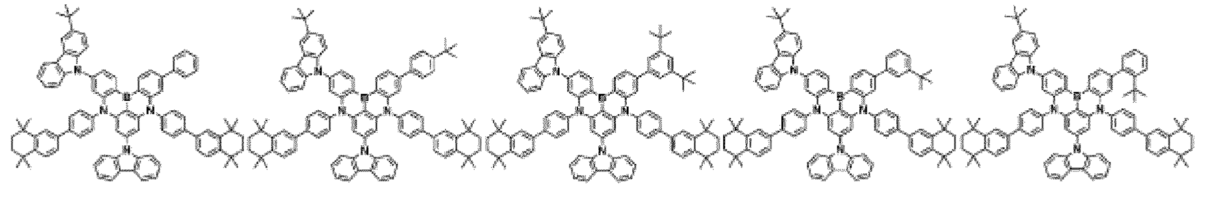

66 67 68 69 70

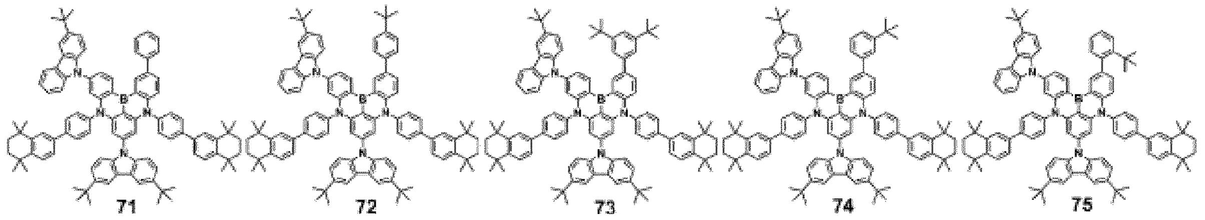

71 72 73 74 75

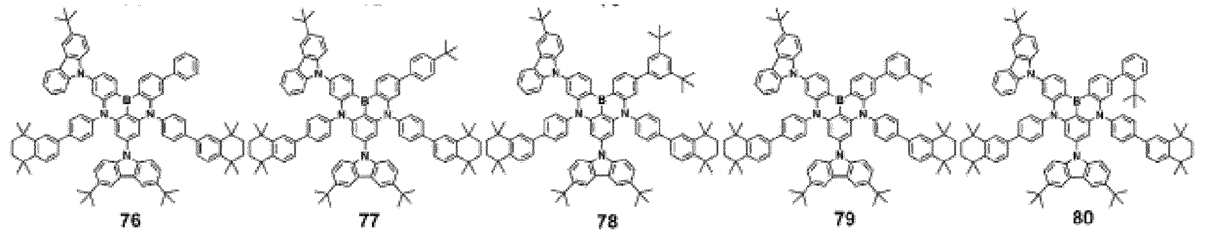

76 77 78 79 80

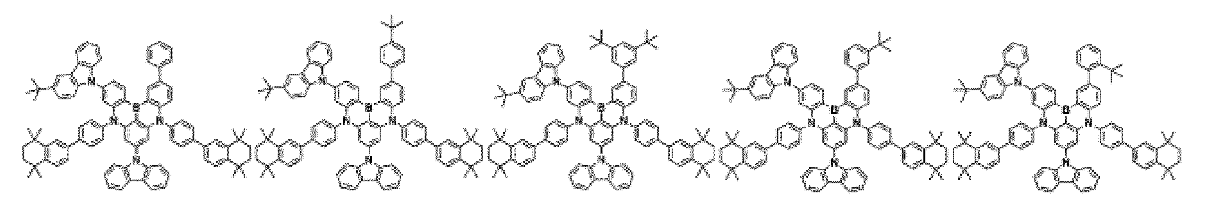

81 82 83 84 85

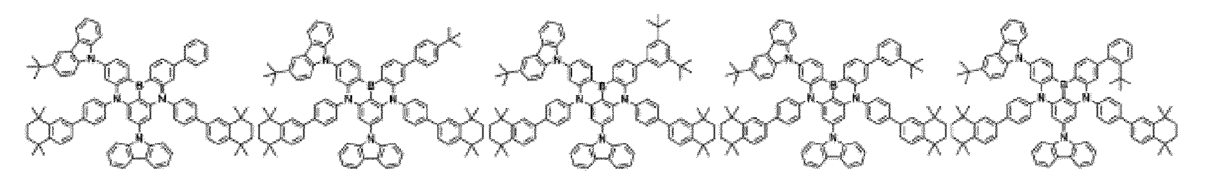

86 87 88 89 90

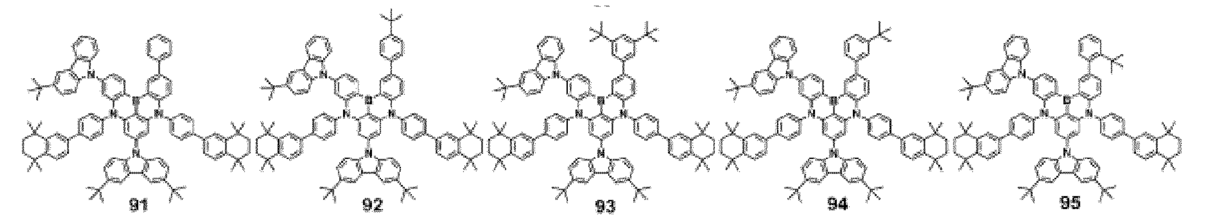

91 92 93 94 95

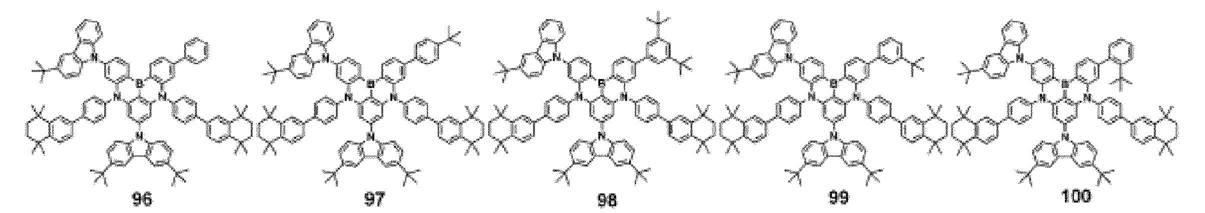

96 97 98 99 100

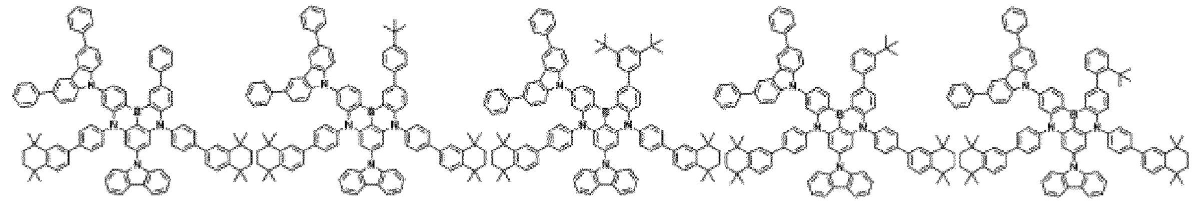

101 102 103 104 105

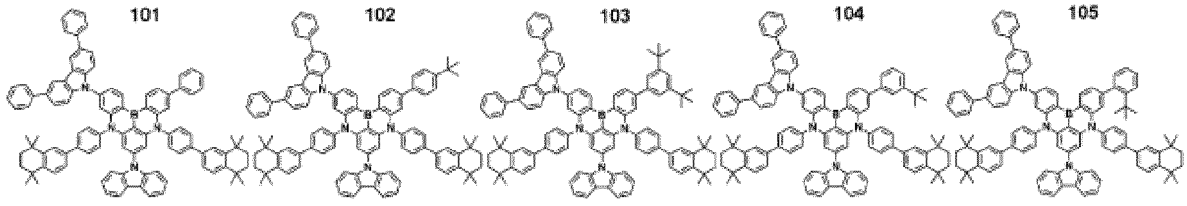

106 107 108 109 110

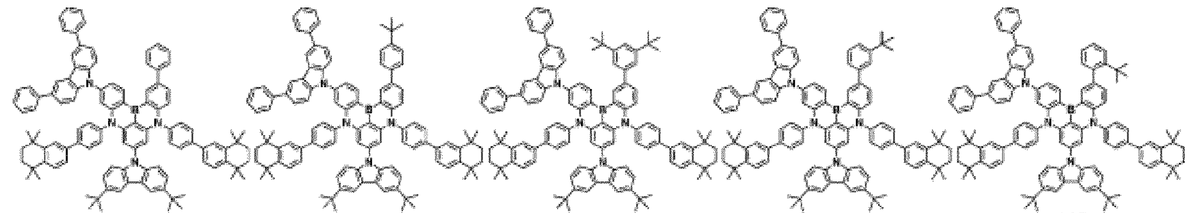

111 112 113 114 115

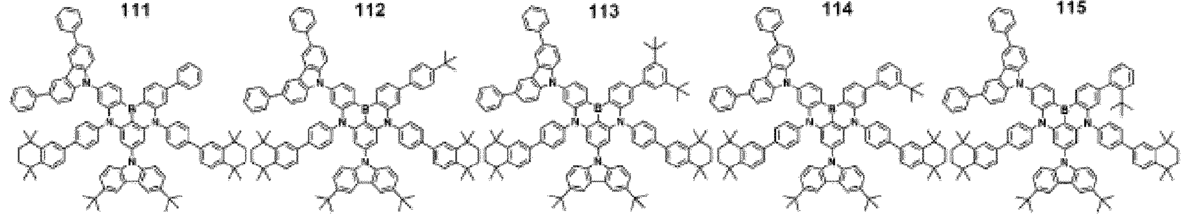

116 117 118 119 120

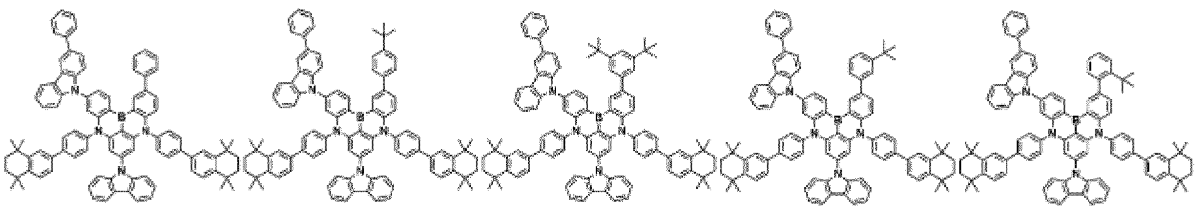

121 122 123 124 125

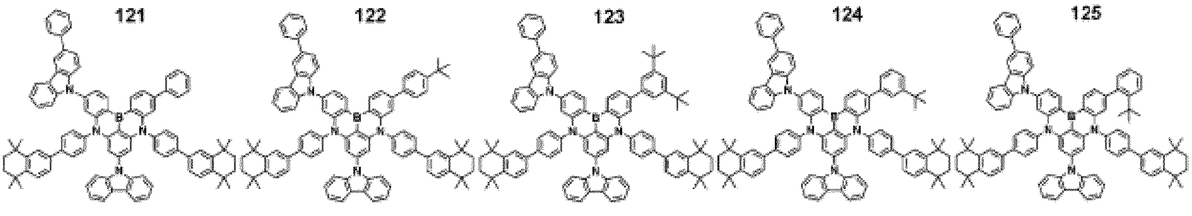

126 127 128 129 130

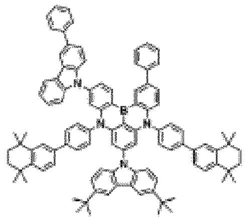
131
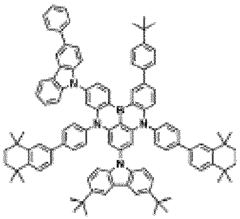
132
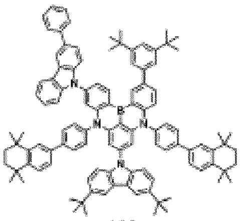
133
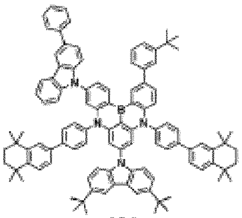
134
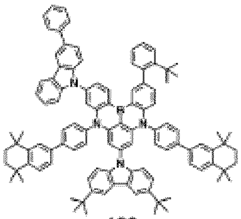
135

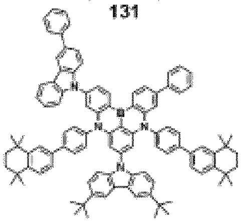
136
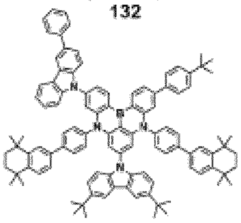
137
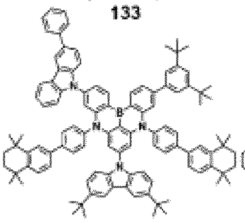
138
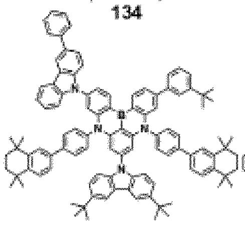
139
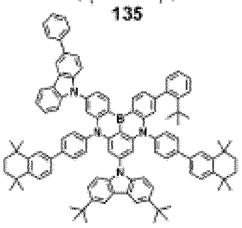
140

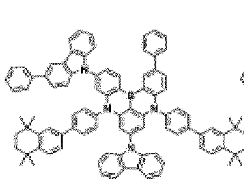
141
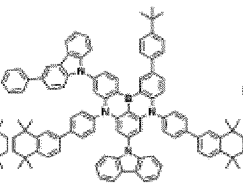
142
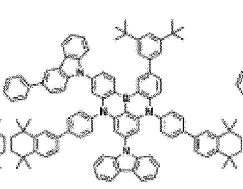
143
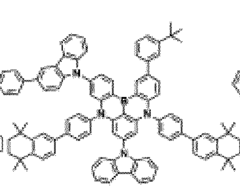
144
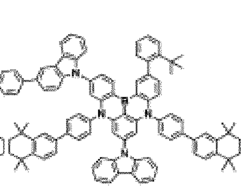
145

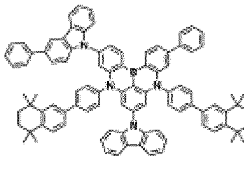
146
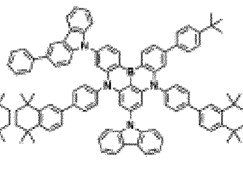
147
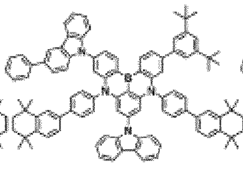
148
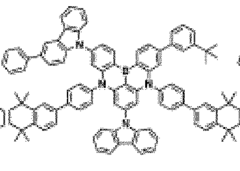
149
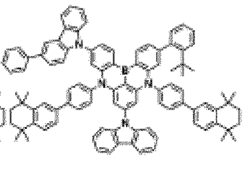
150

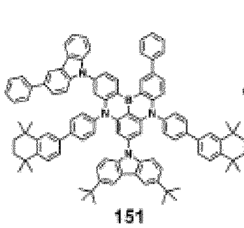
151
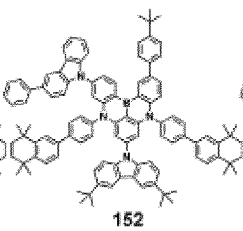
152
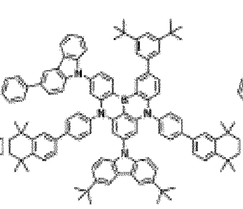
153
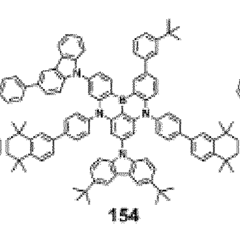
154
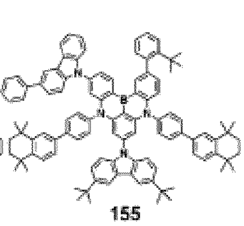
155

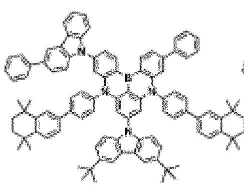
156
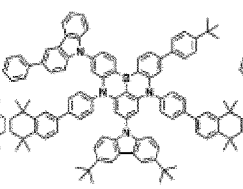
157
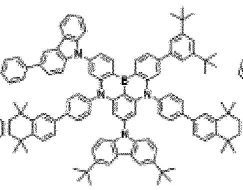
158
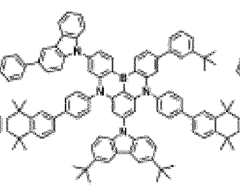
159
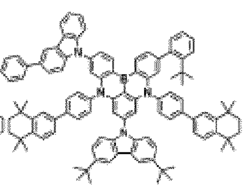
160

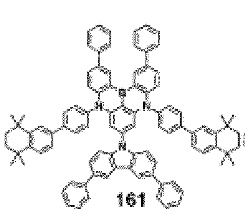
161
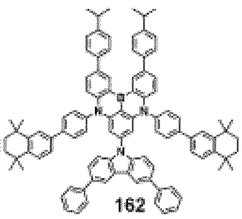
162
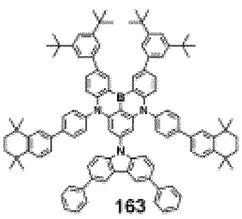
163
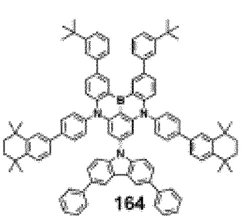
164
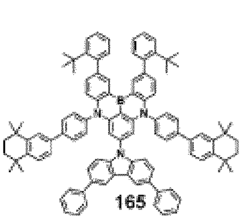
165

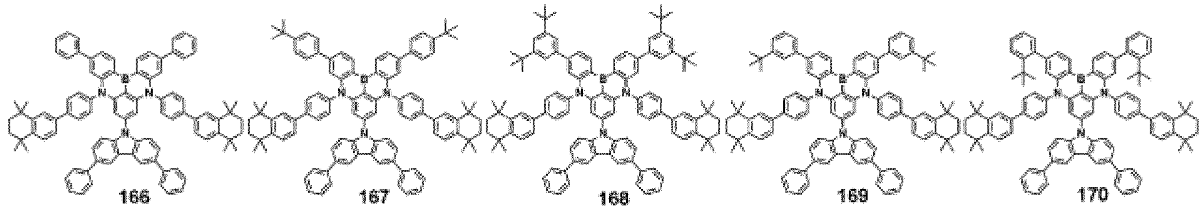

166 167 168 169 170

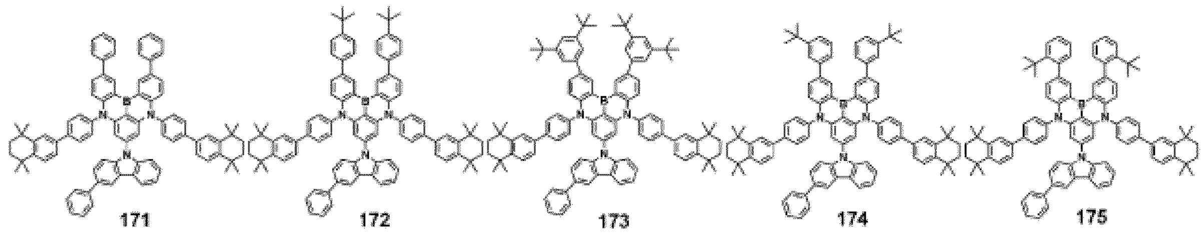

171 172 173 174 175

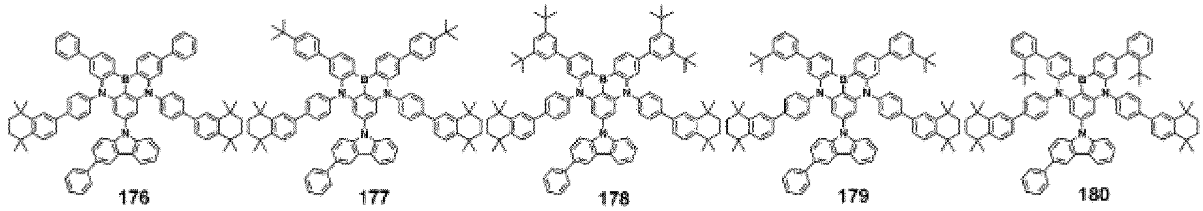

176 177 178 179 180

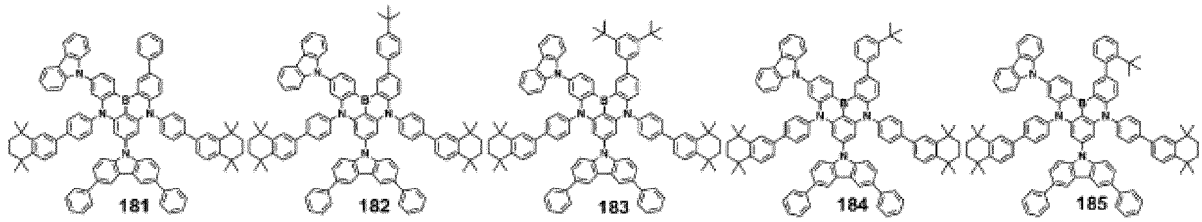

181 182 183 184 185

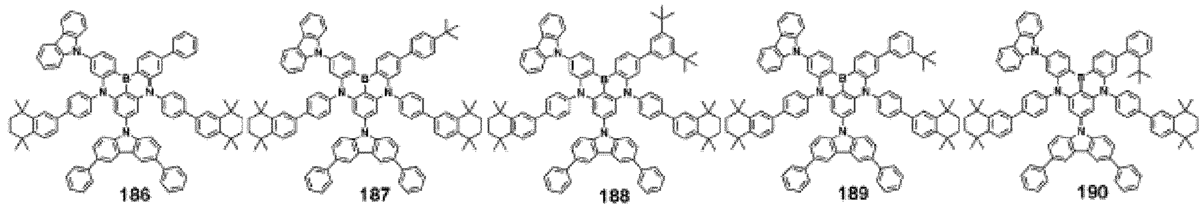

186 187 188 189 190

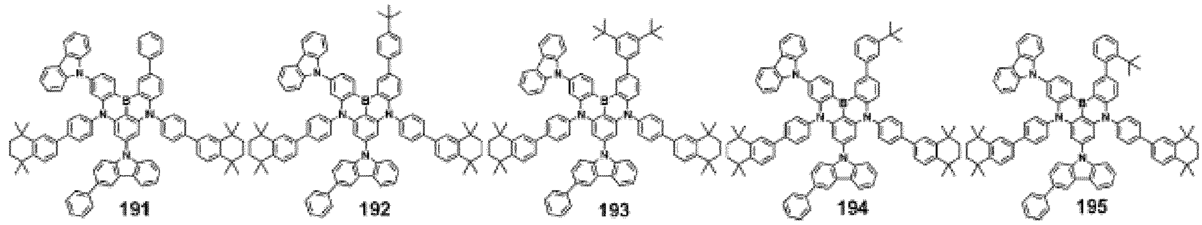

191 192 193 194 195

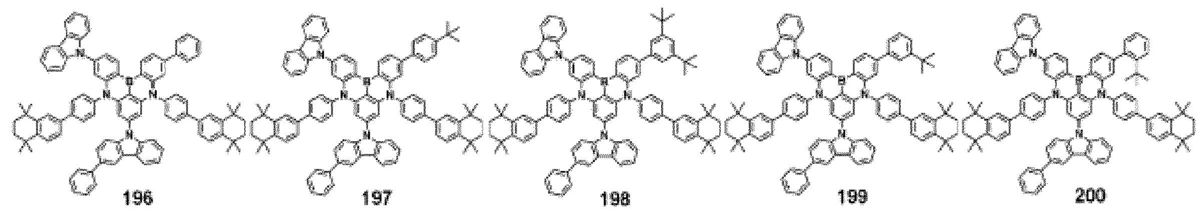

196 197 198 199 200

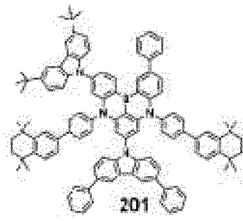
201
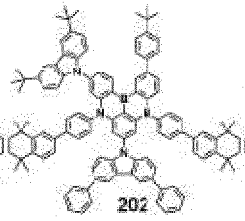
202
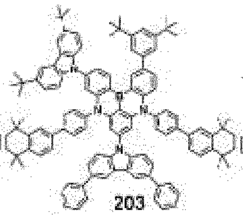
203
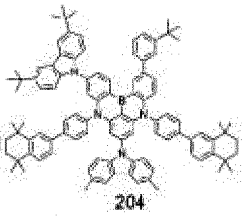
204
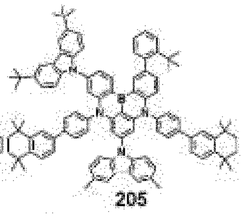
205

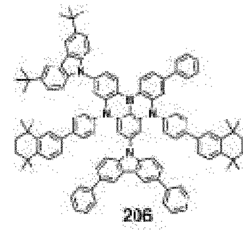
206
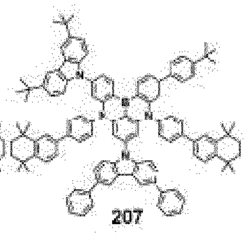
207
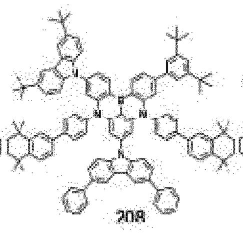
208
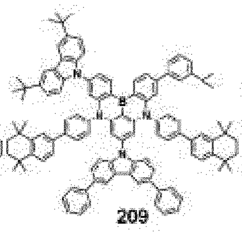
209
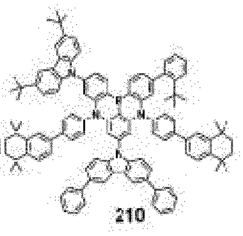
210

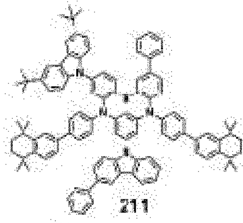
211
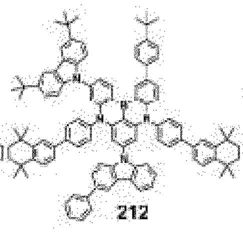
212
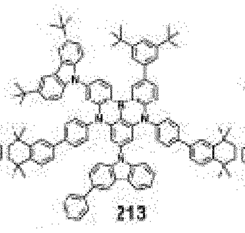
213
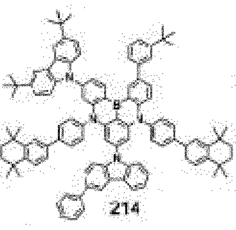
214
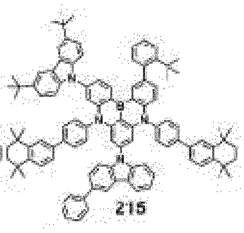
215

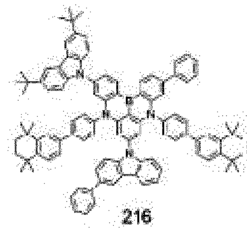
216
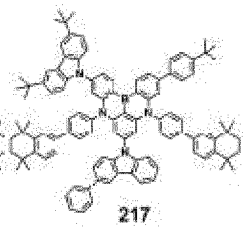
217
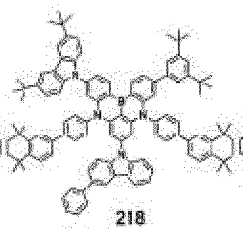
218
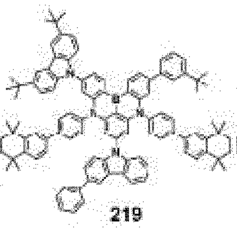
219
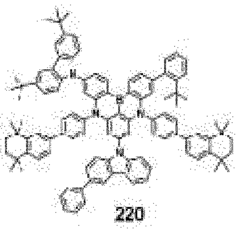
220

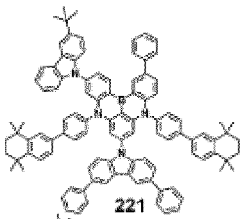
221
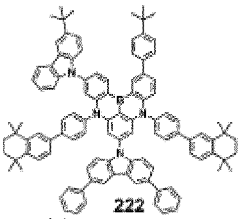
222
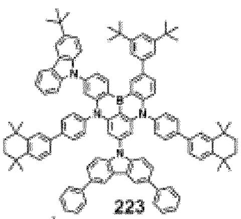
223
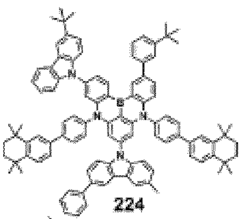
224
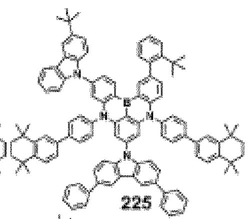
225

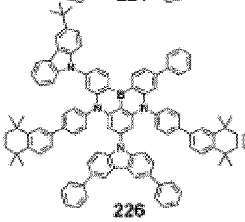
226
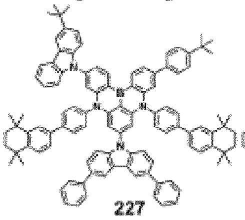
227
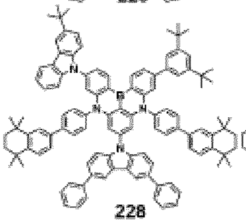
228
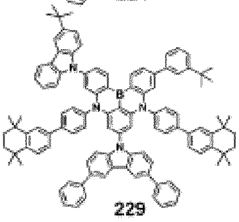
229
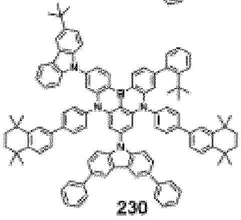
230

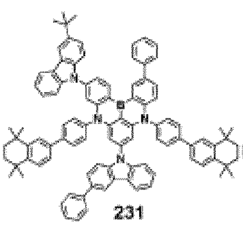
231
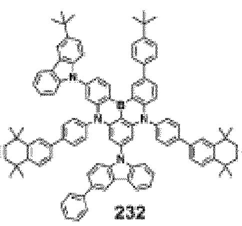
232
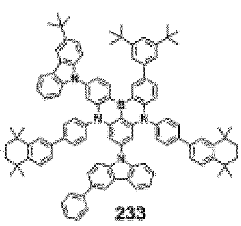
233
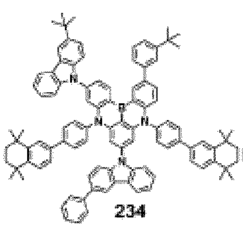
234
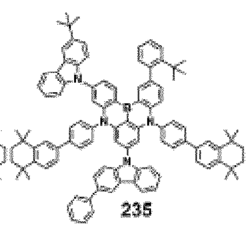
235

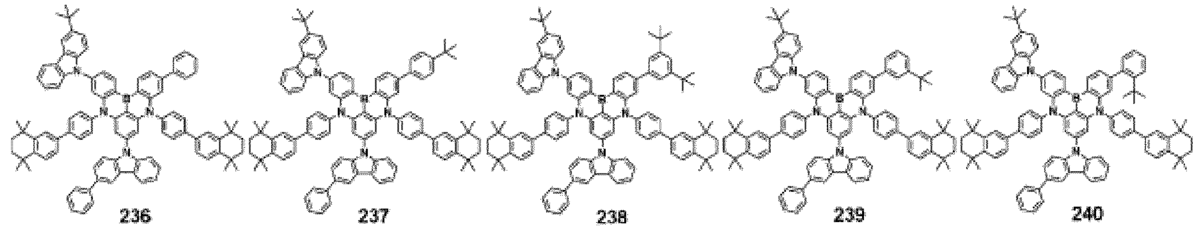

236 237 238 239 240

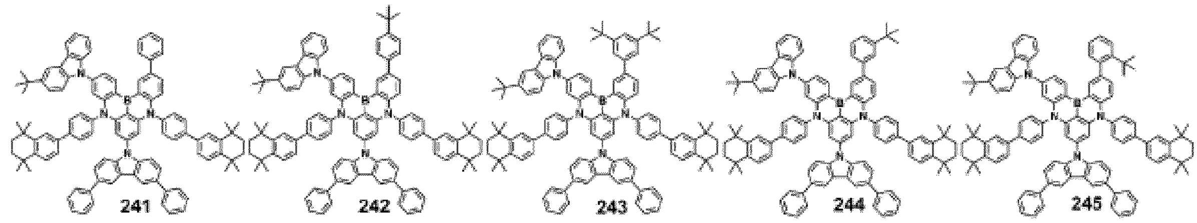

241 242 243 244 245

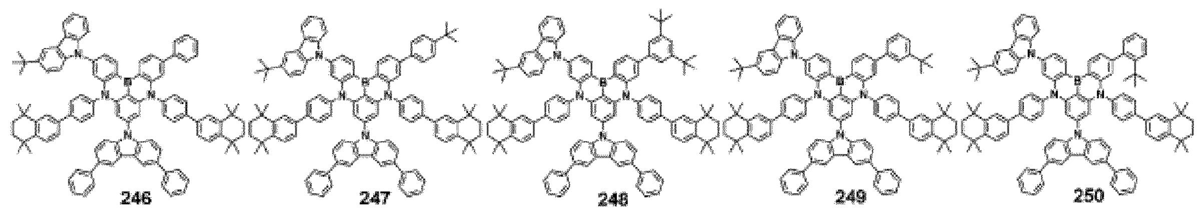

246 247 248 249 250

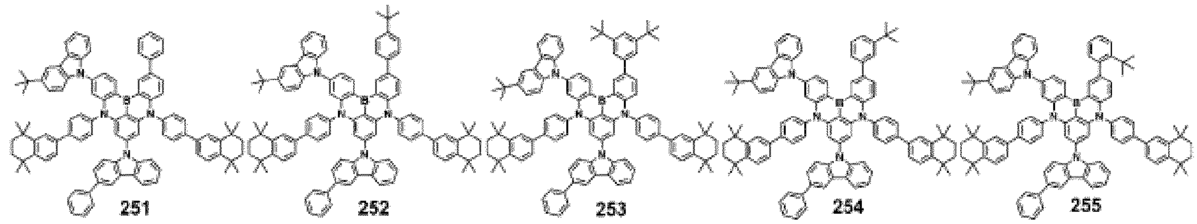

251 252 253 254 255

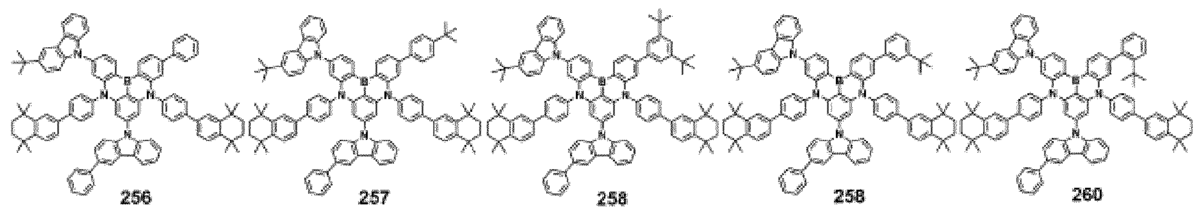

256 257 258 258 260

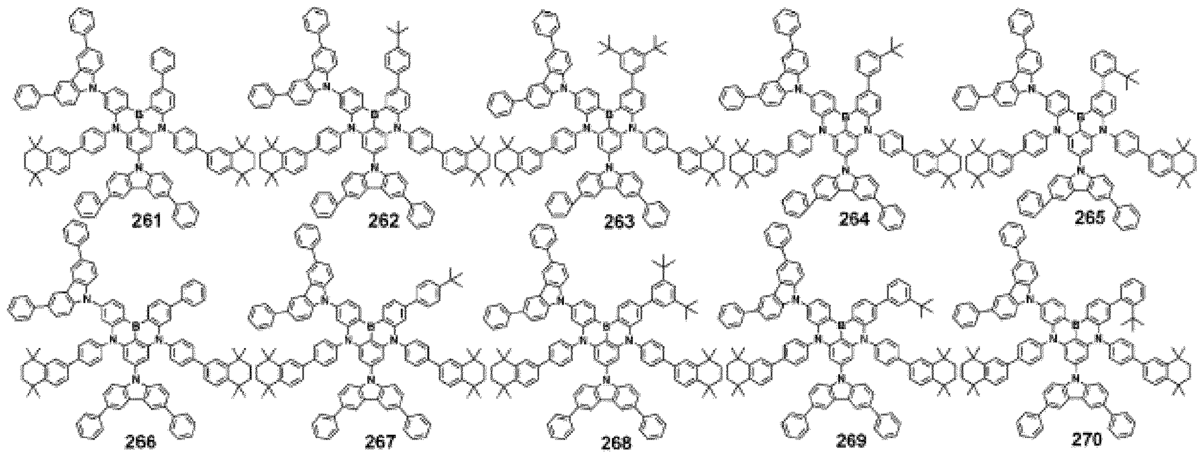

261 262 263 264 265

266 267 268 269 270

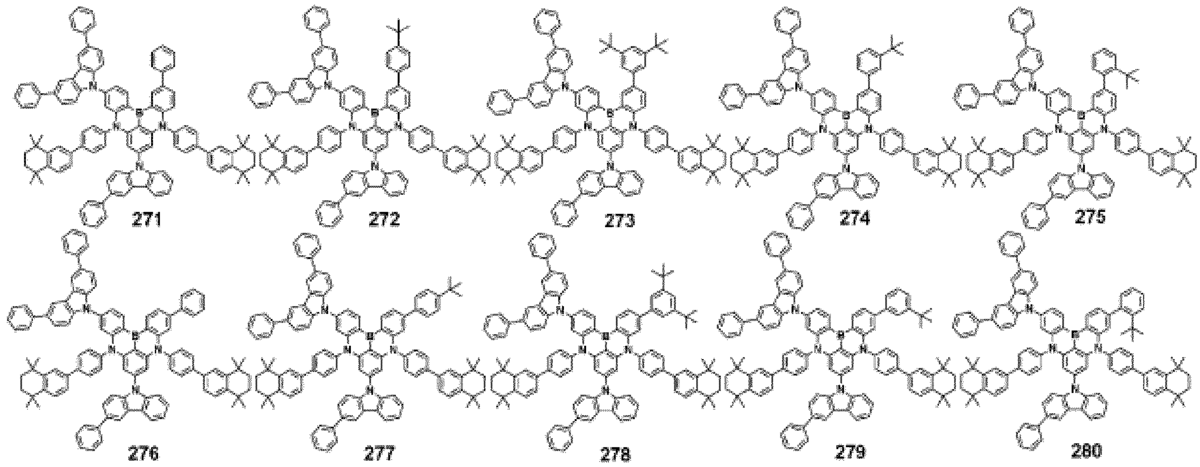

271 272 273 274 275

276 277 278 279 280

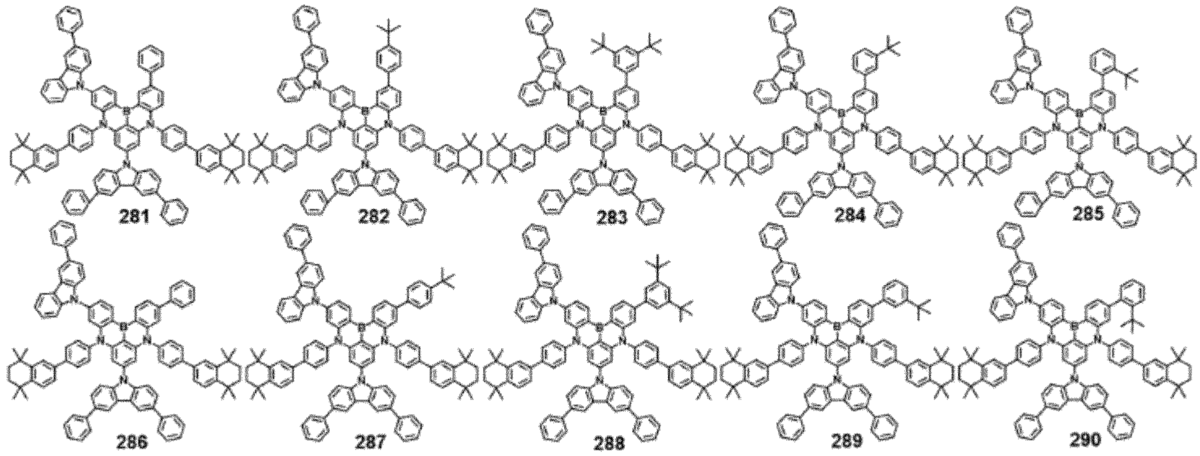

281 282 283 284 285

286 287 288 289 290

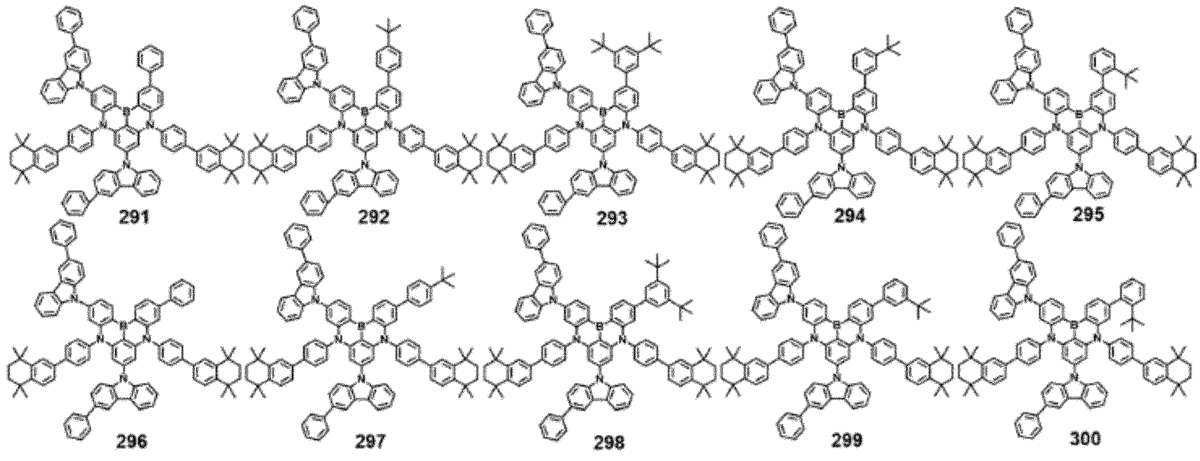

291 292 293 294 295

296 297 298 299 300

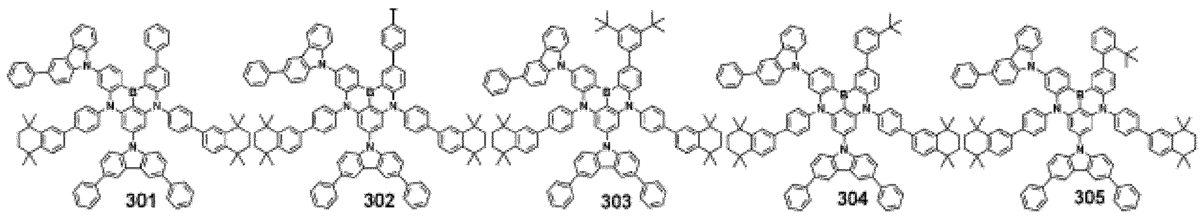

301 302 303 304 305

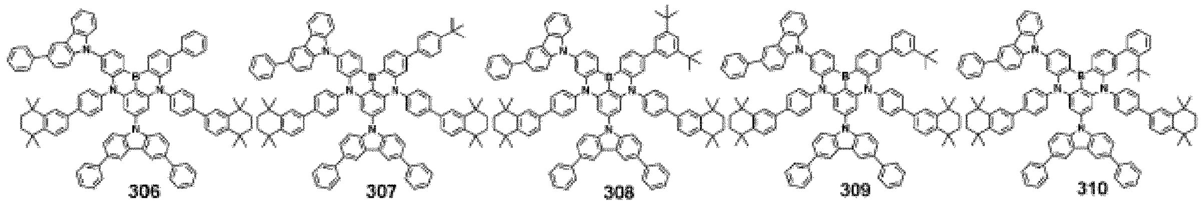

306 307 308 309 310

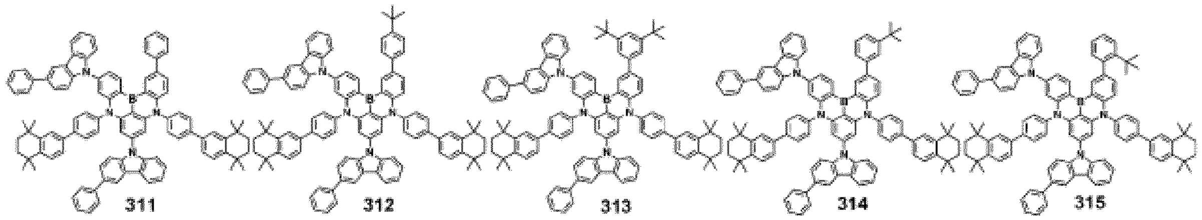

311 312 313 314 315

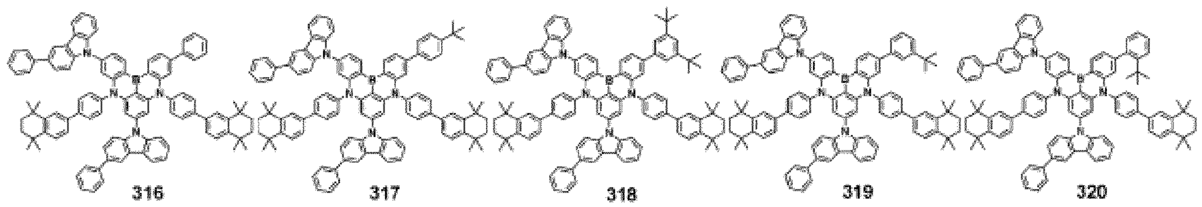

316 317 318 319 320

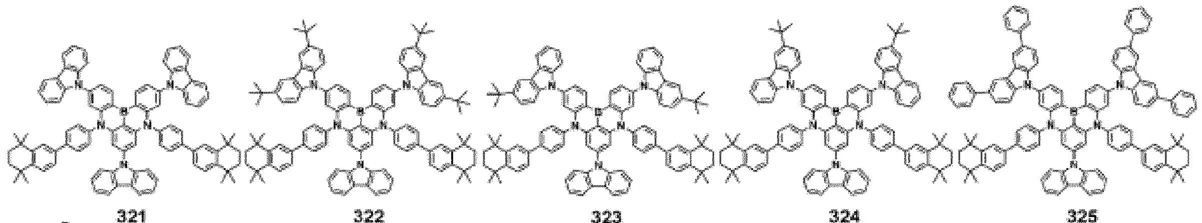

321 322 323 324 325

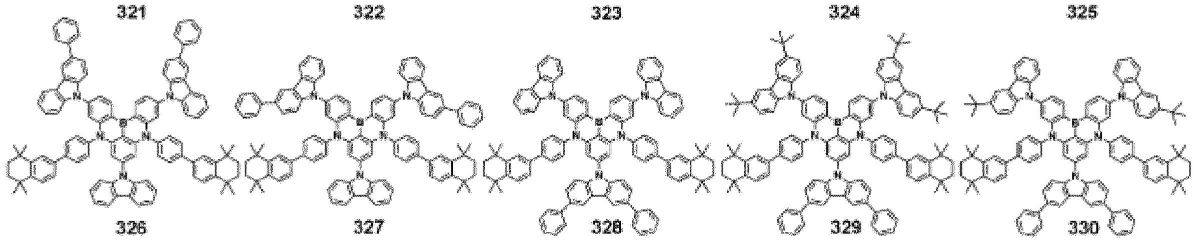

326 327 328 329 330

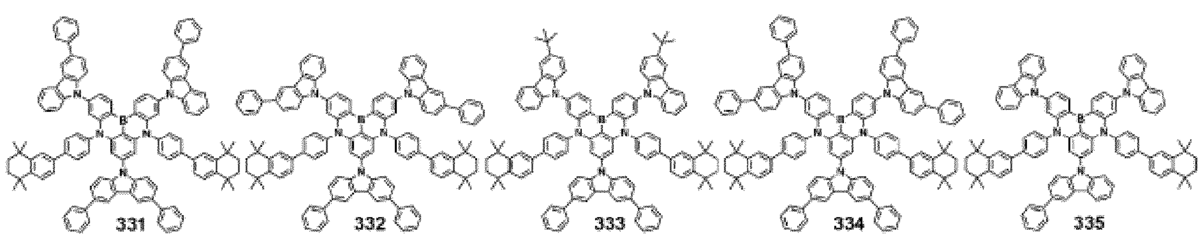

331 332 333 334 335

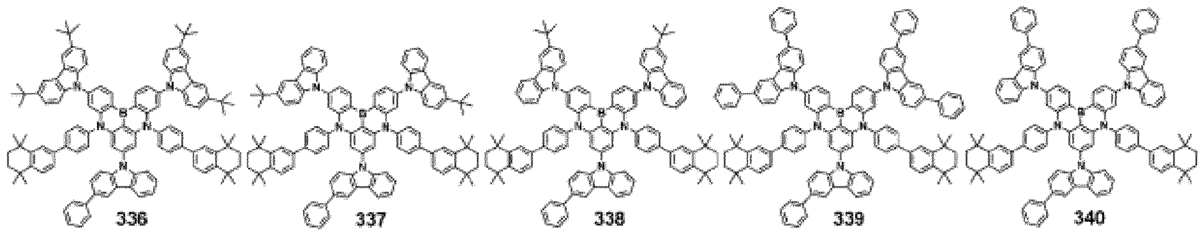

336 337 338 339 340

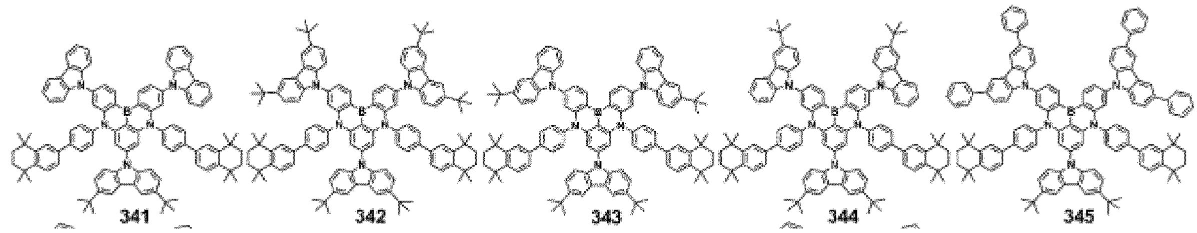

341 342 343 344 345

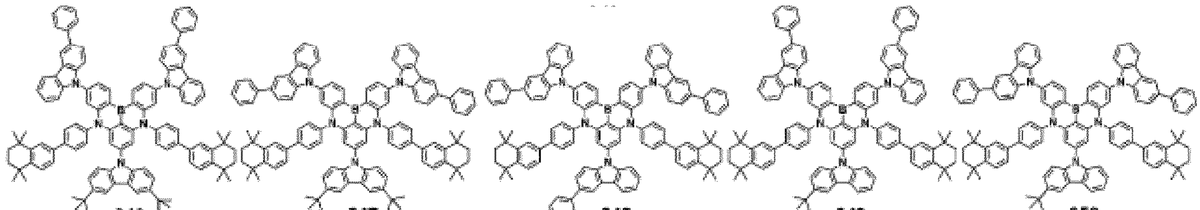

346 347 348 349 350

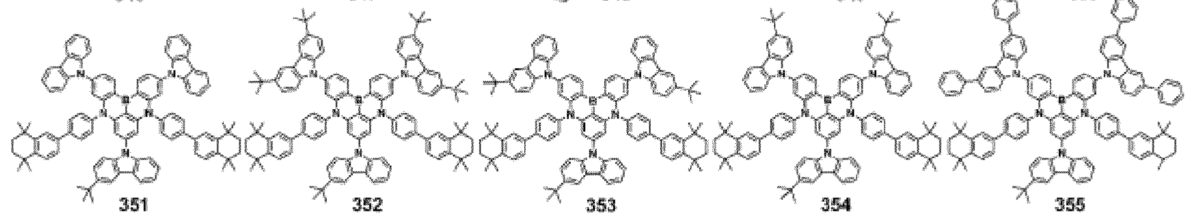

351 352 353 354 355

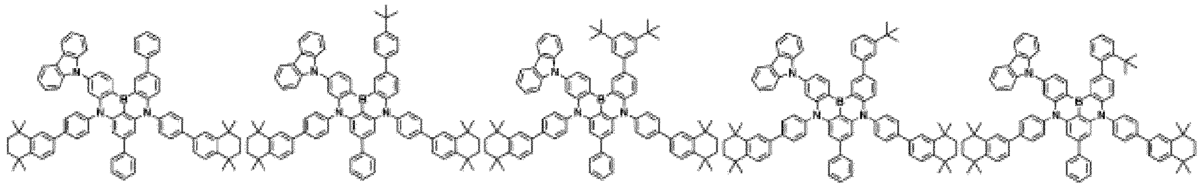

356 357 358 359 360

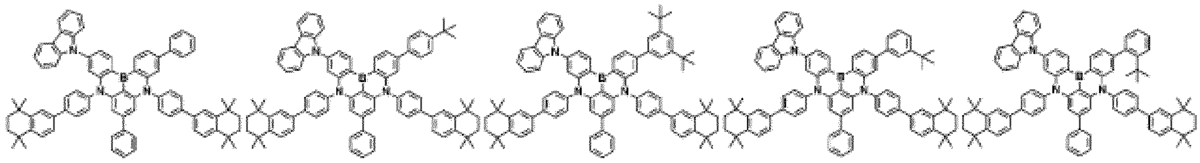

361 362 363 364 365

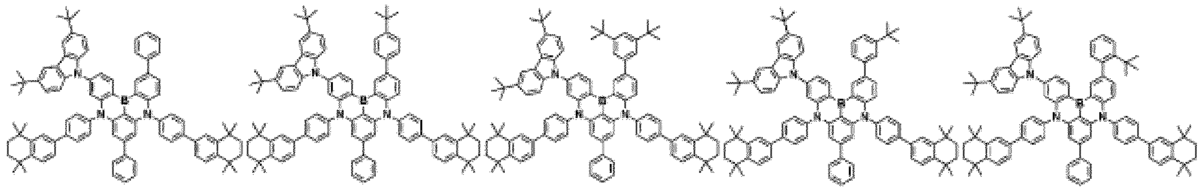

366 367 368 369 370

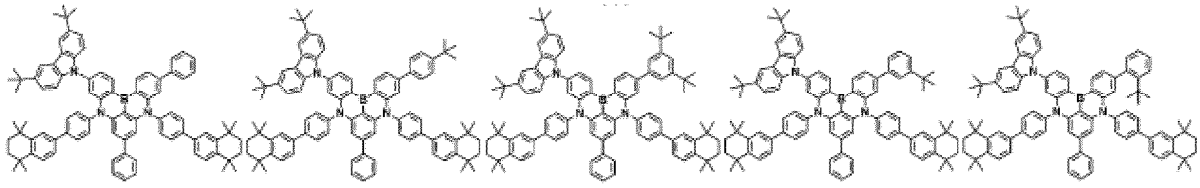

371 372 373 374 375

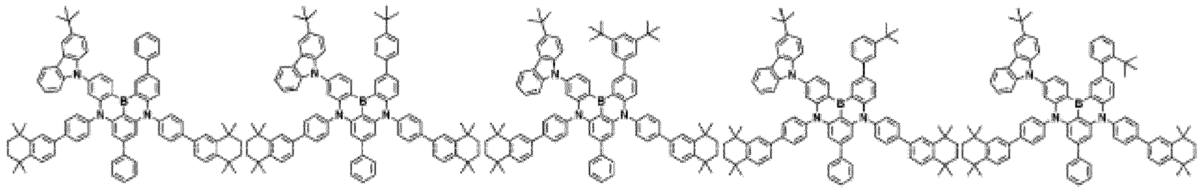

376 377 378 379 380

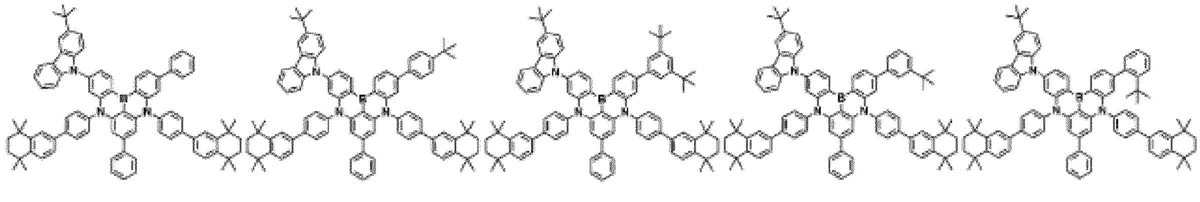

381 382 383 384 385

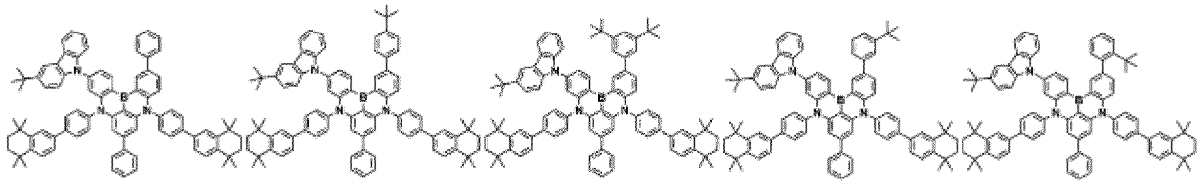

386 387 388 389 390

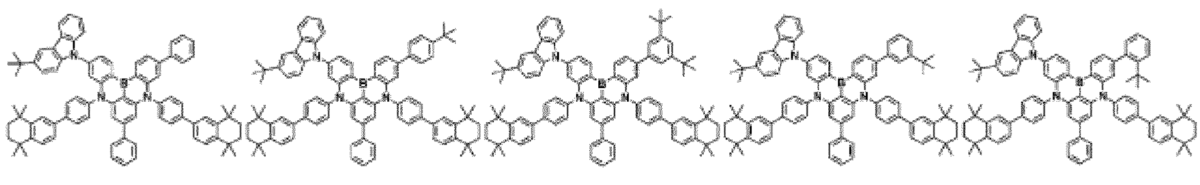

391 392 393 394 395

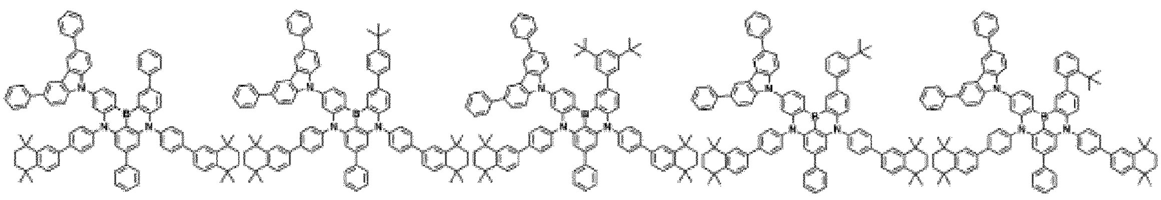

396 397 398 399 400

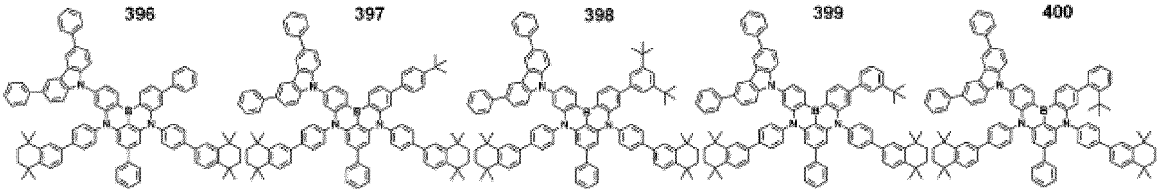

401 402 403 404 405

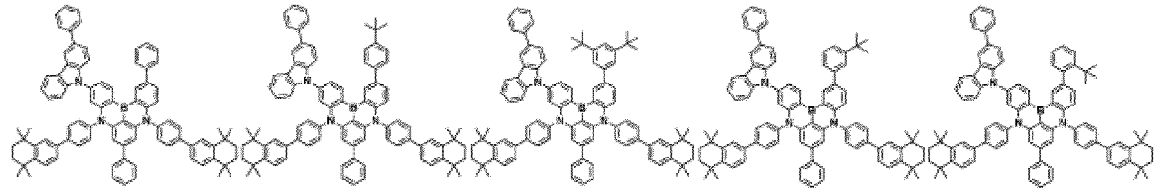

406 407 408 409 410

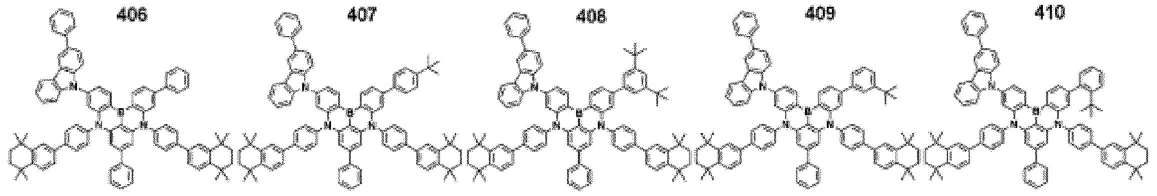

411 412 413 414 415

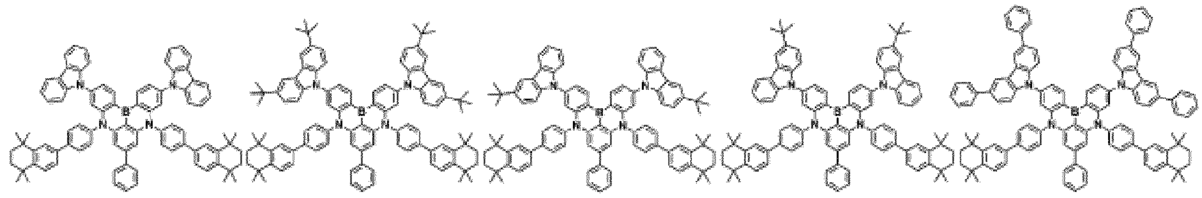

416 417 418 419 420

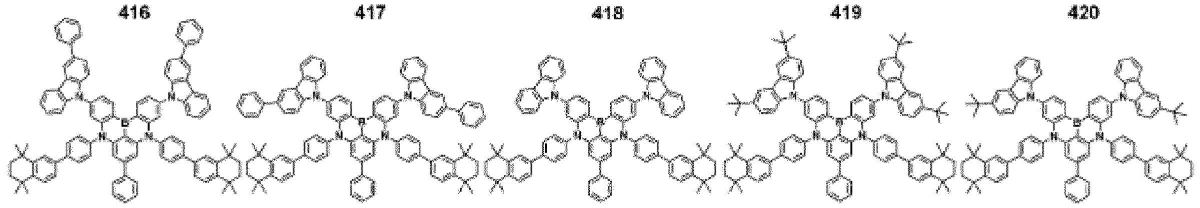

421 422 423 424 425

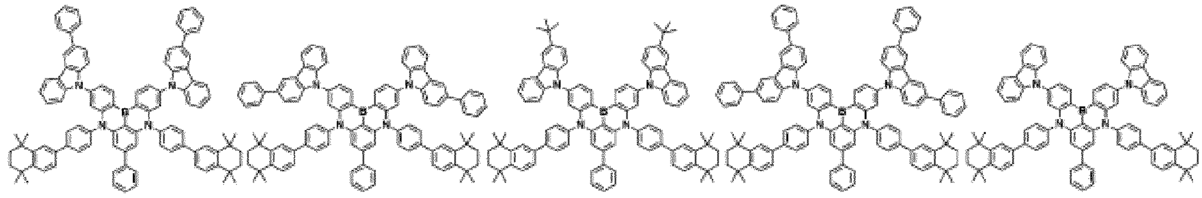

426 427 428 429 430

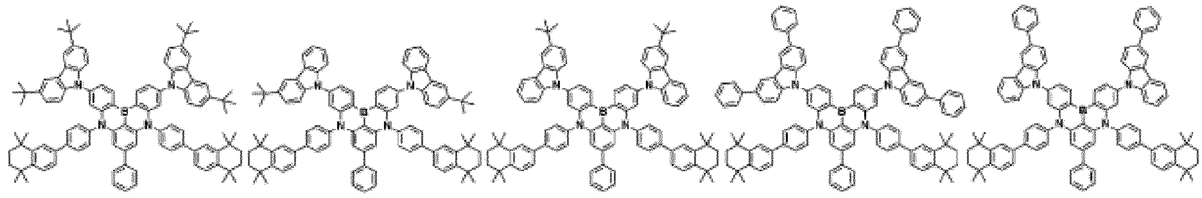

431 432 433 434 435

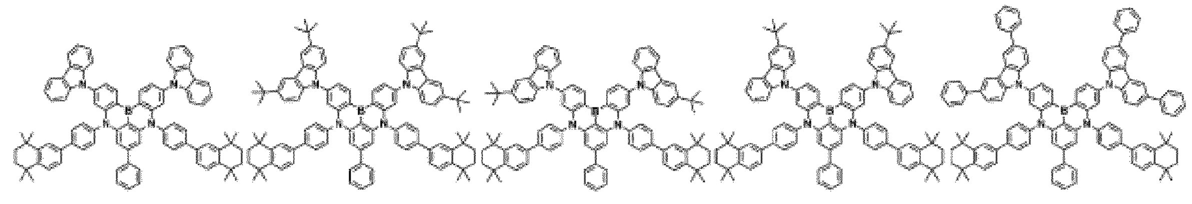

436 437 438 439 440

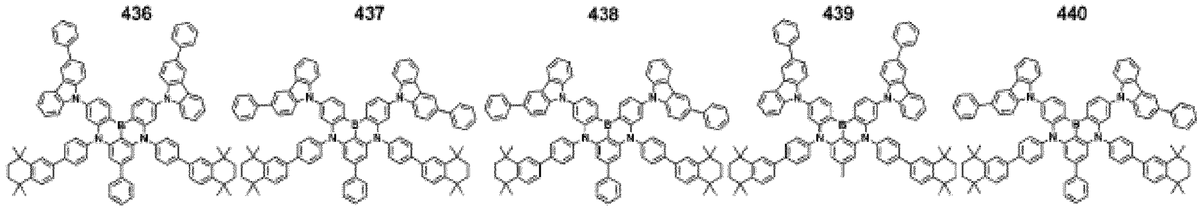

441 442 443 444 445

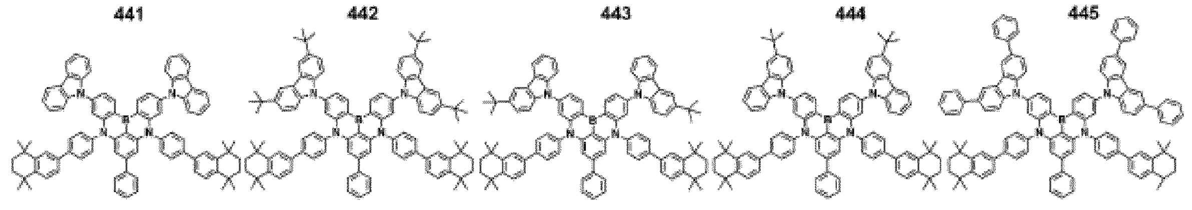

446 447 448 449 450

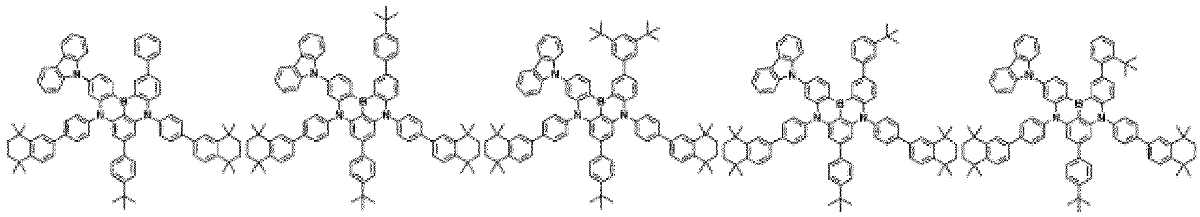

451 452 453 454 455

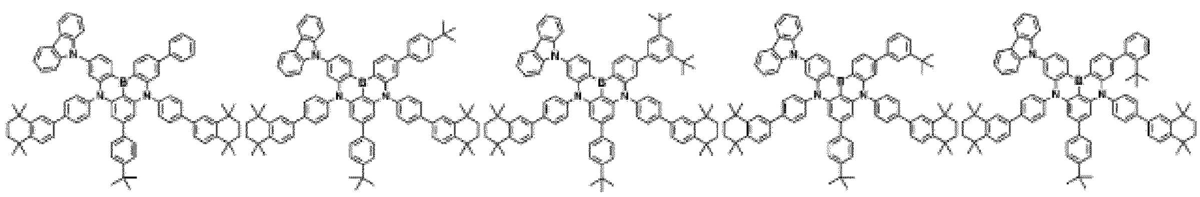

456 457 458 459 460

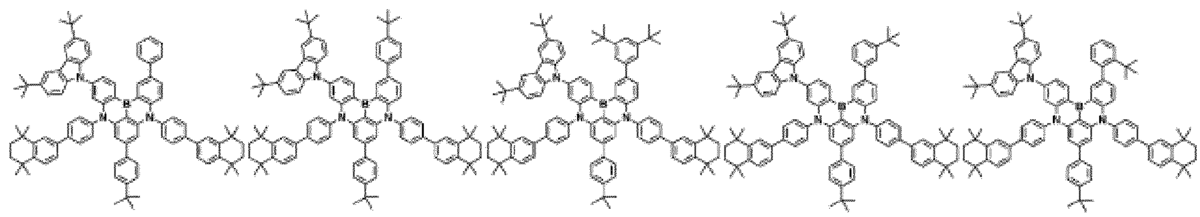

461 462 463 464 465

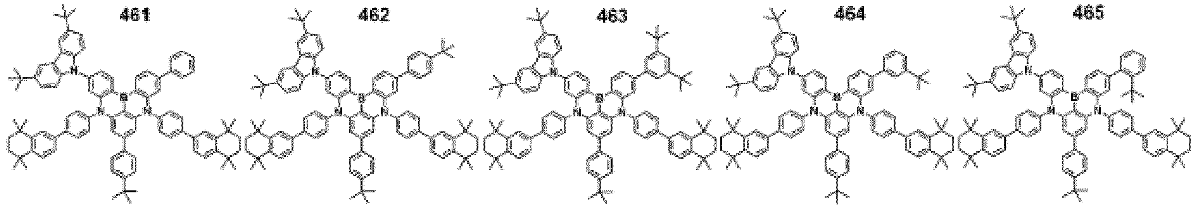

466 467 468 469 470

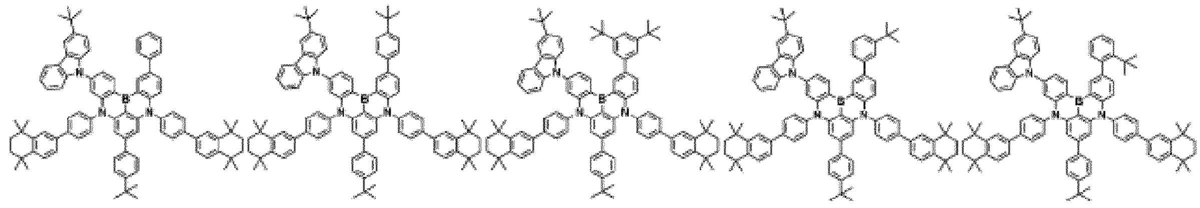

471 472 473 474 475

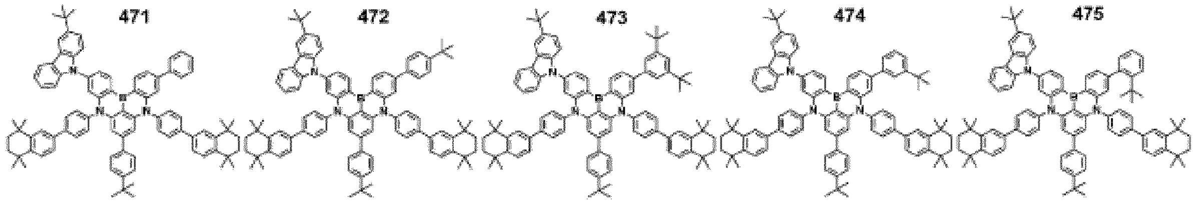

476 477. 478 479 480

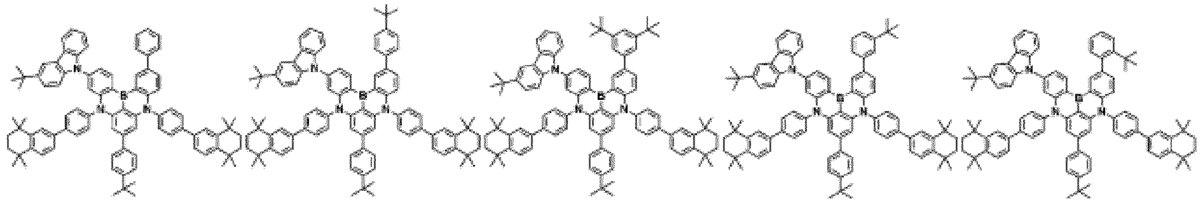

481 482 483 484 485

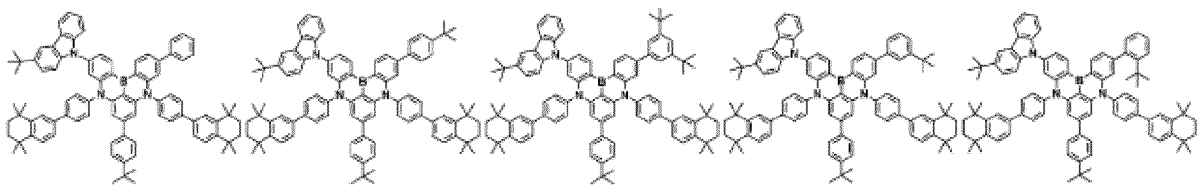

486 487 488 489 490

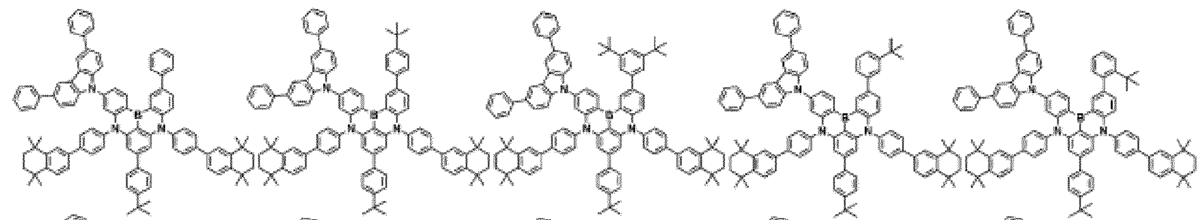

491 492 493 494 495

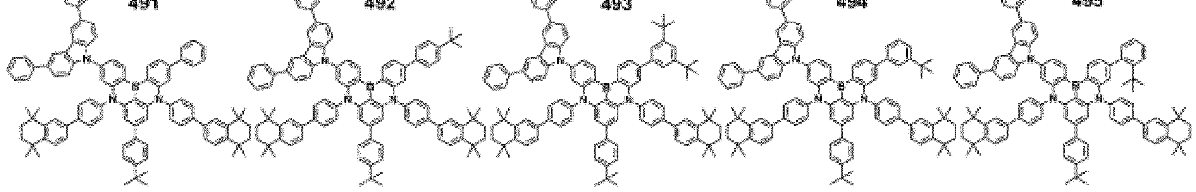

496 497 498 499 500

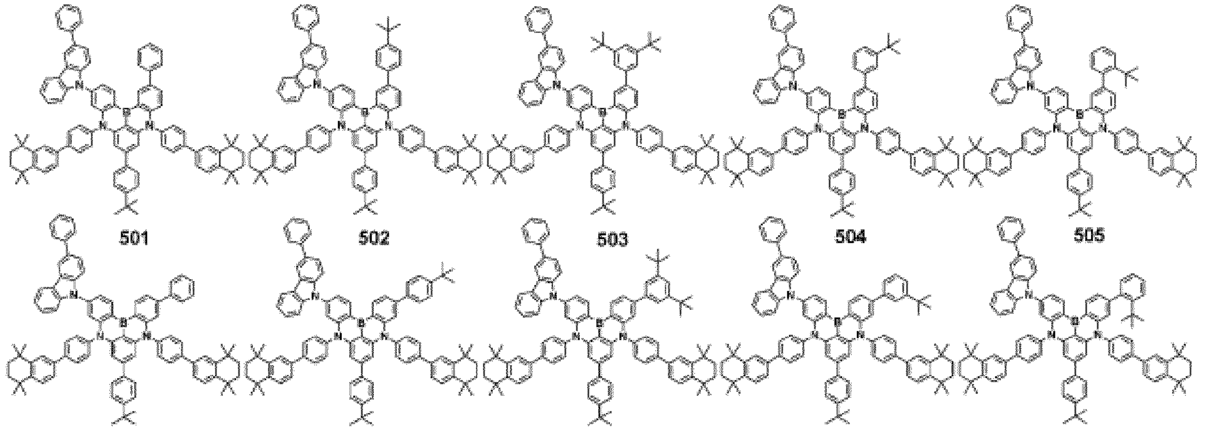

501 502 503 504 505

506 507 508 509 510

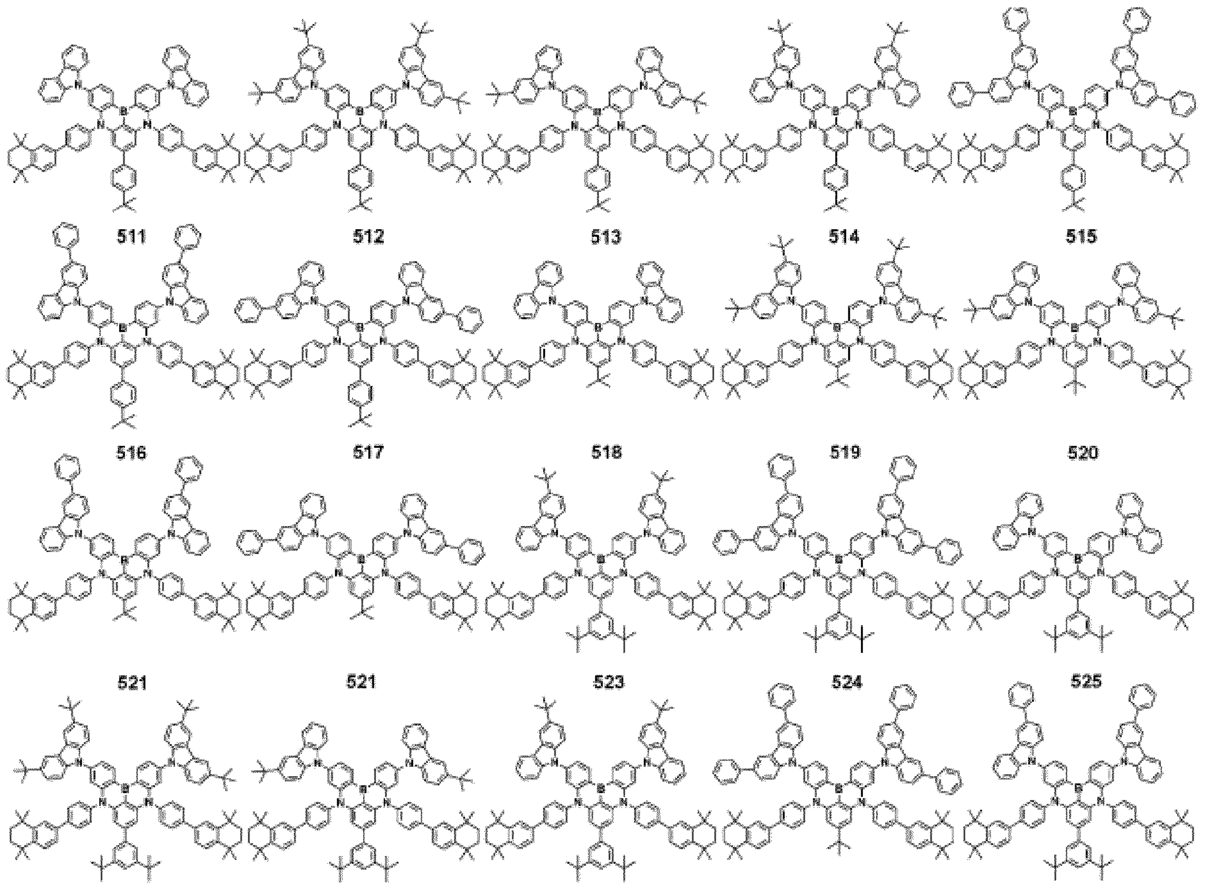

511 512 513 514 515

516 517 518 519 520

521 521 523 524 525

526 527 528 529 530

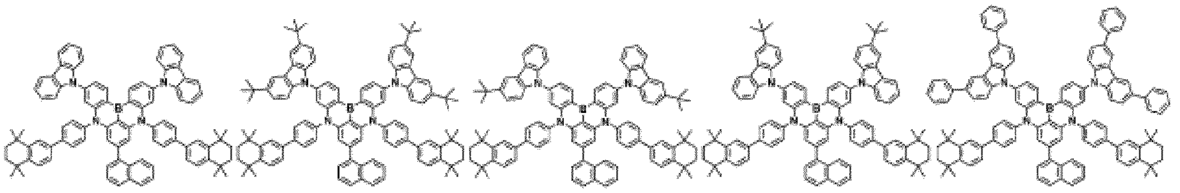

531 532 533 534 535

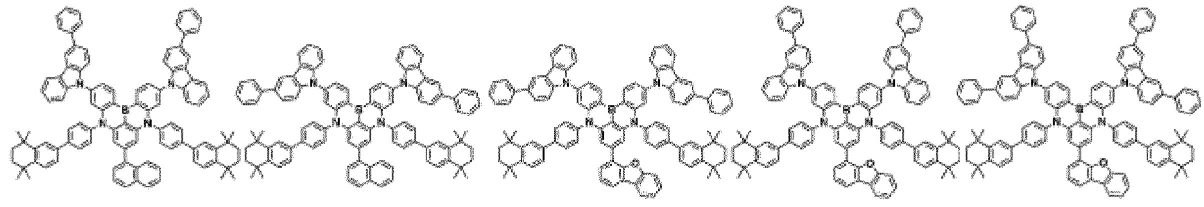

536 537 538 539 540

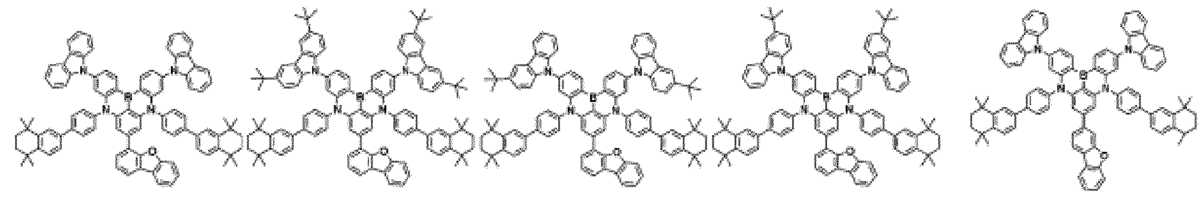

541 542 543 544 545

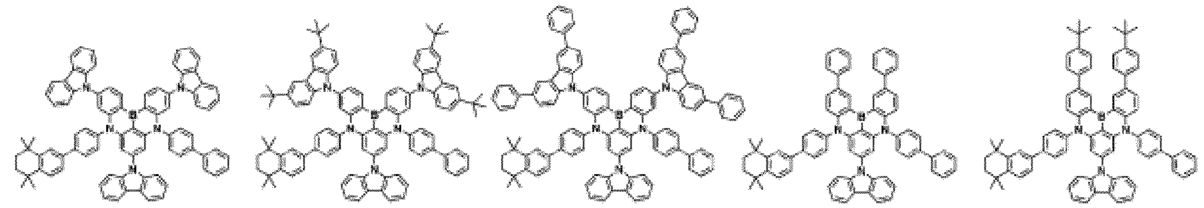

546 547 548 549 550

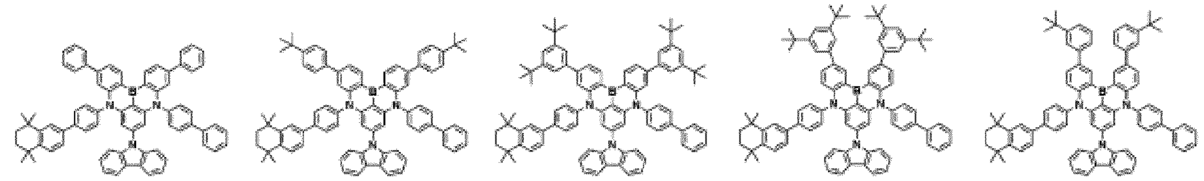

551 552 553 554 555

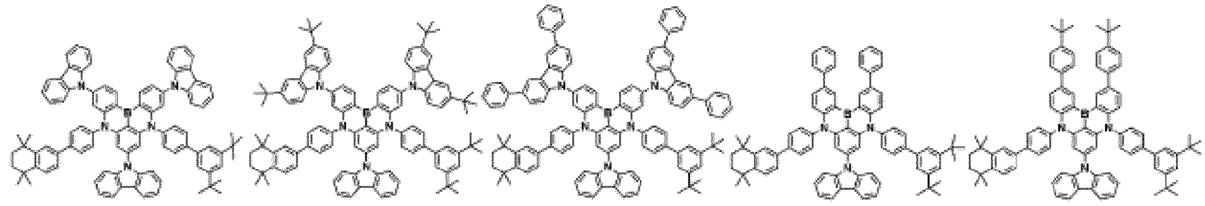

556 557 558 559 560

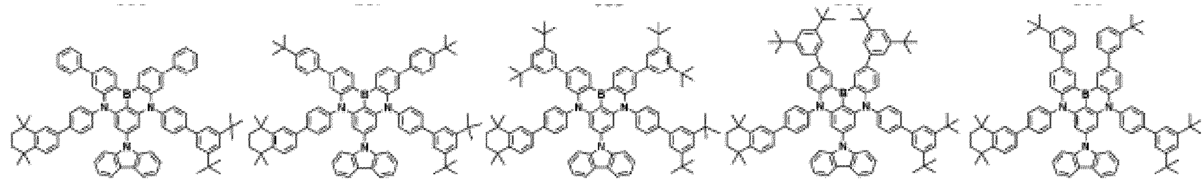

561 562 563 564 565

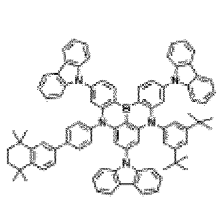
566
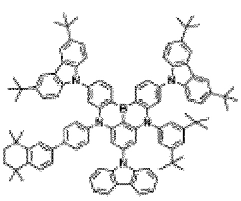
567
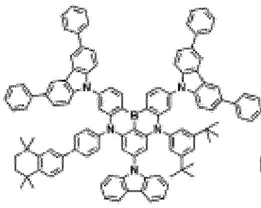
568
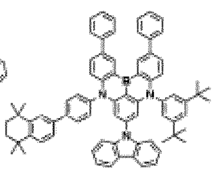
569
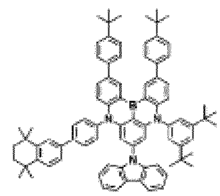
570

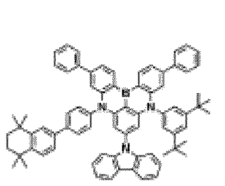
571
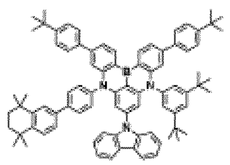
572
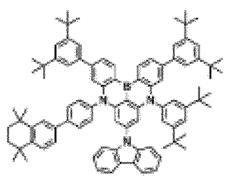
573
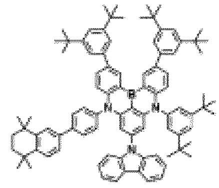
574
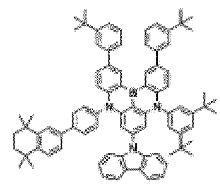
575

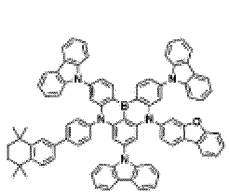
576
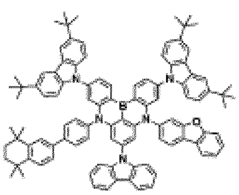
577
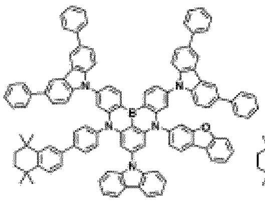
578
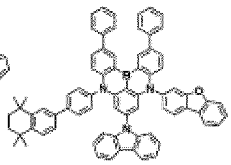
579
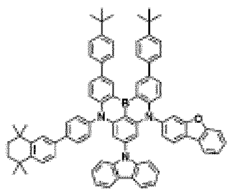
580

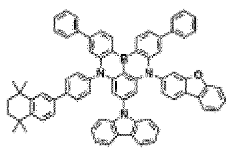
581
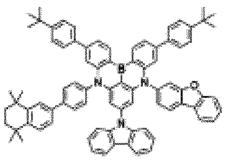
582
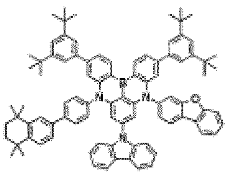
583
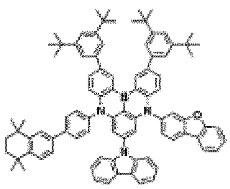
584
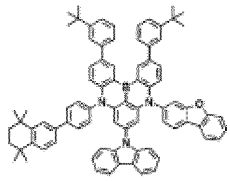
585

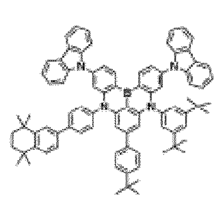
586
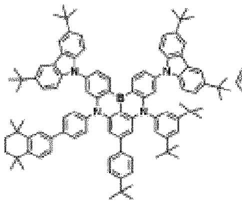
587
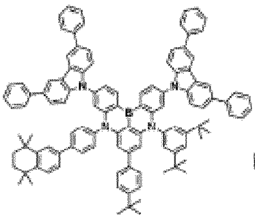
588
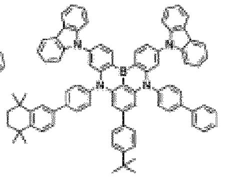
589
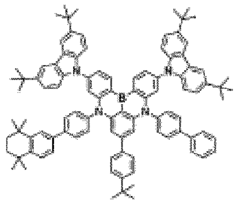
590

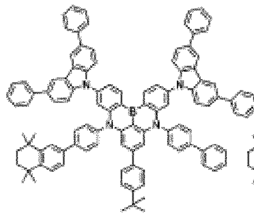
591
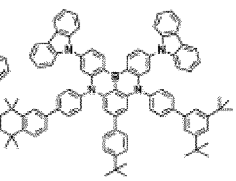
592
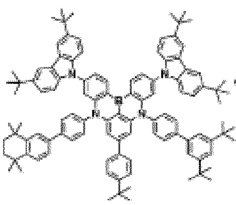
593
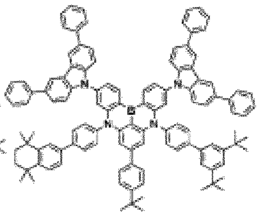
594
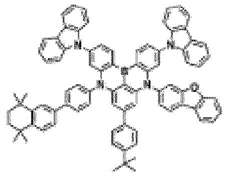
595

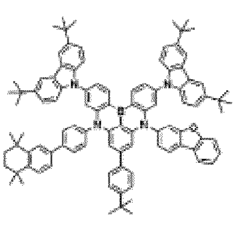
596
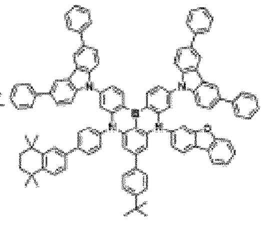
597
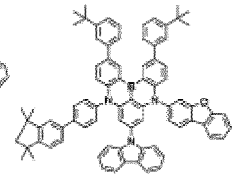
598

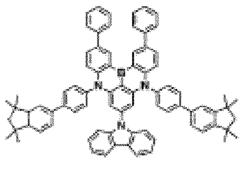
599
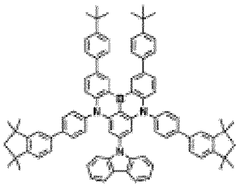
600
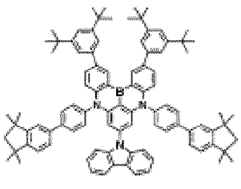
601
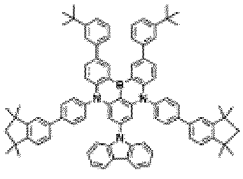
602
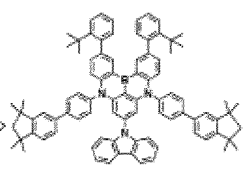
603

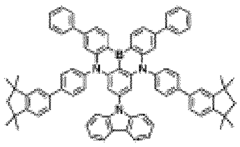
604
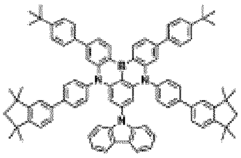
605
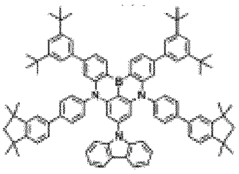
606
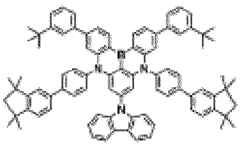
607
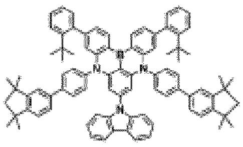
608

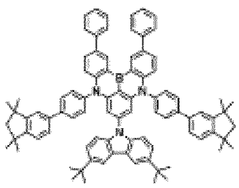
609
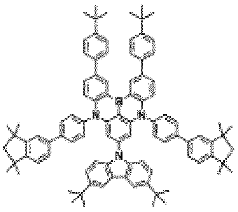
610
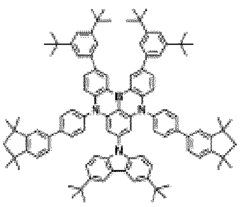
611
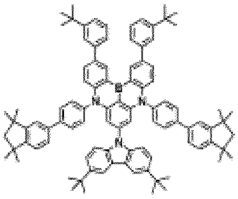
612
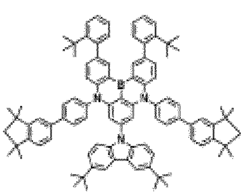
613

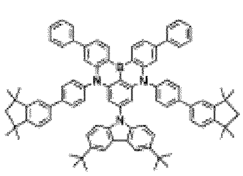
614
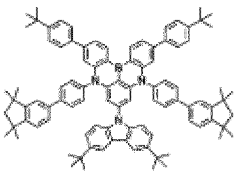
615
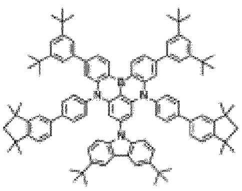
616
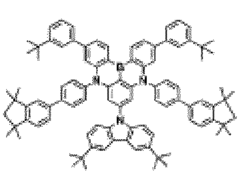
617
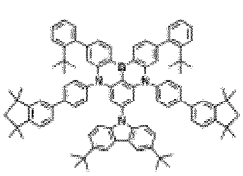
618

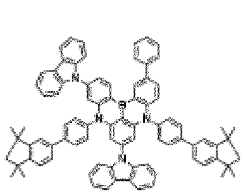
619
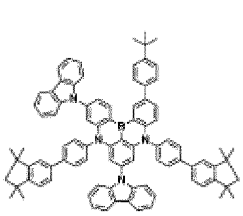
620
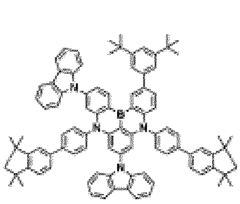
621
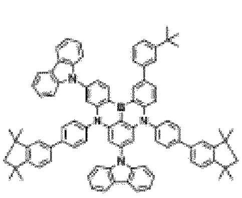
622
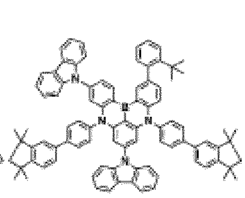
623

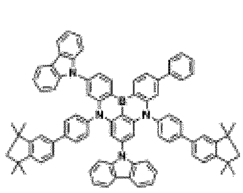
624
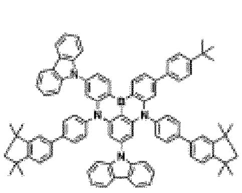
625
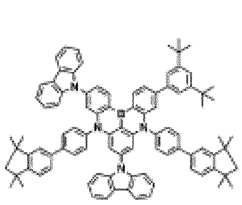
626
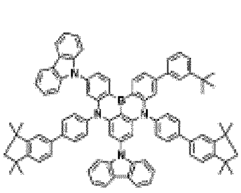
627
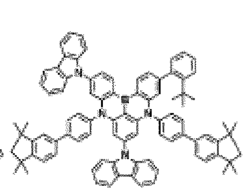
628

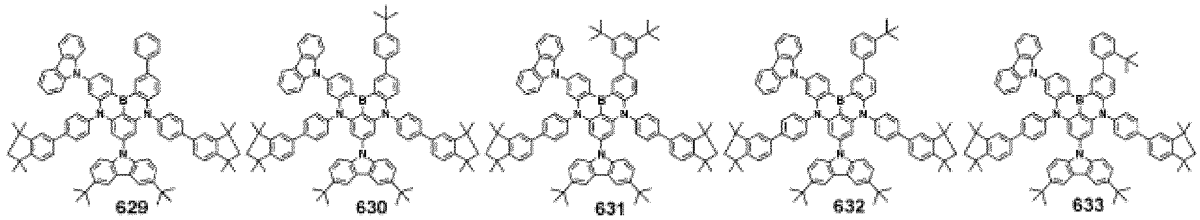

629 630 631 632 633

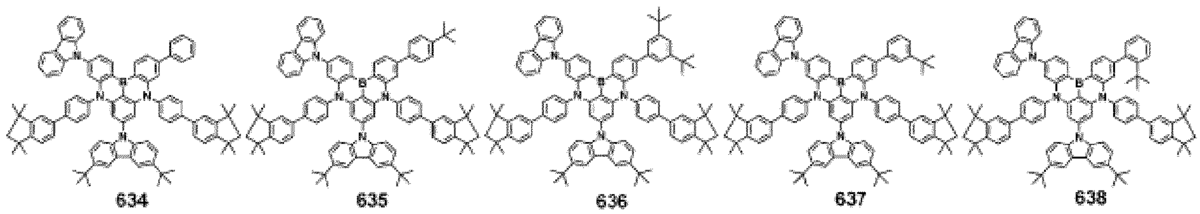

634 635 636 637 638

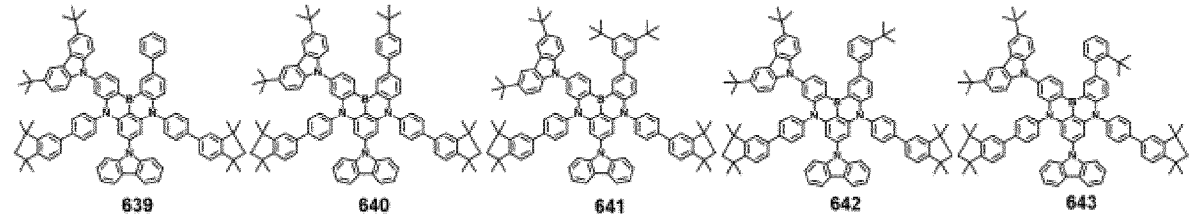

639 640 641 642 643

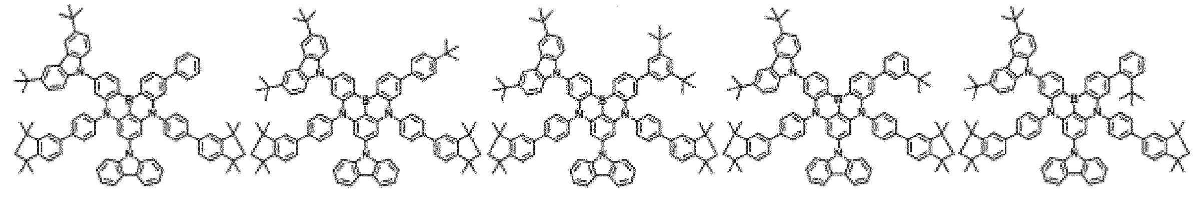

644 645 646 647 648

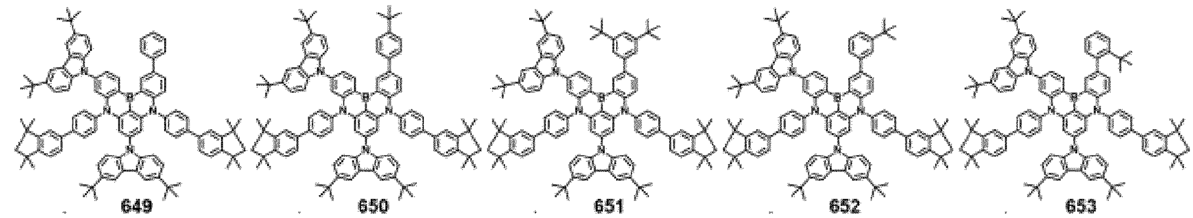

649 650 651 652 653

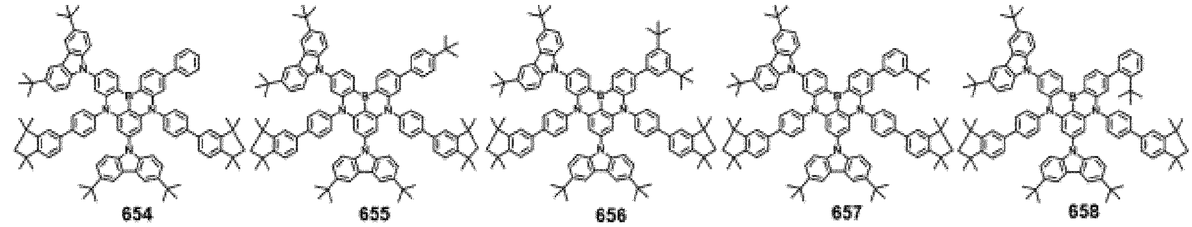

654 655 656 657 658

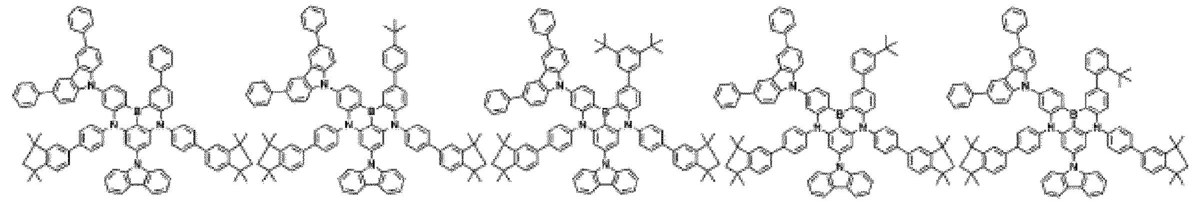

659 660 661 662 663

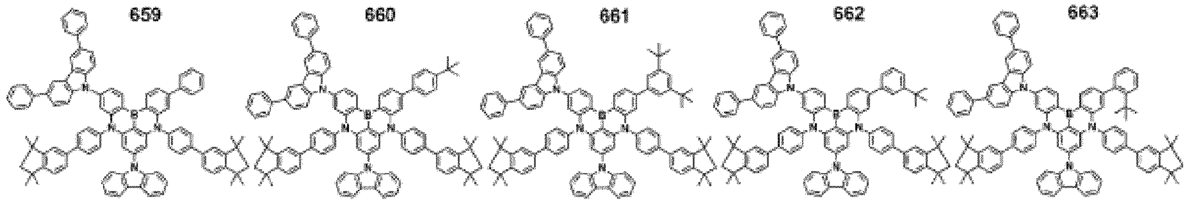

664 665 666 667 668

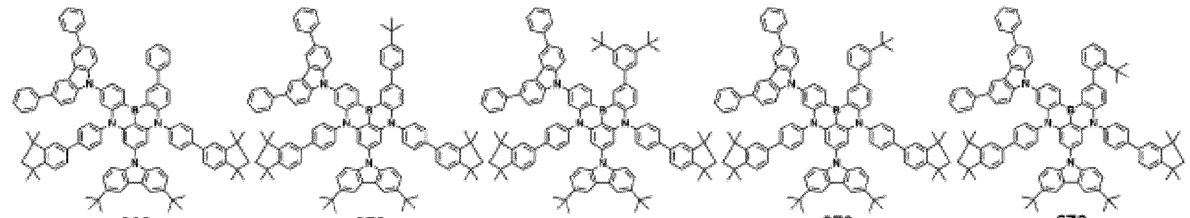

669 670 671 672 673

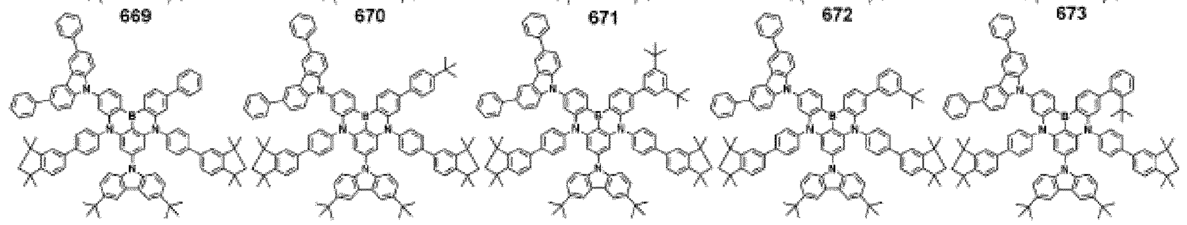

674 675 676 677 678

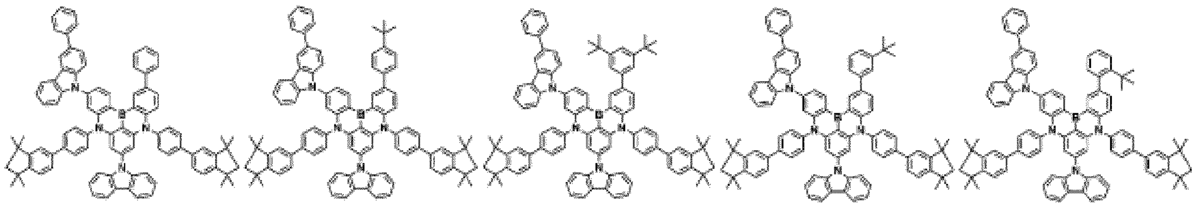

679 680 681 682 683

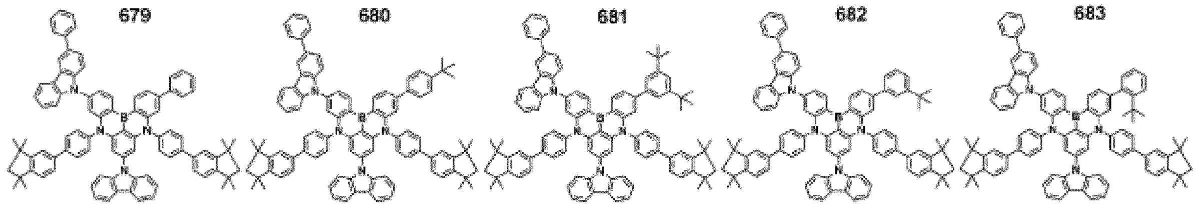

684 685 686 687 688

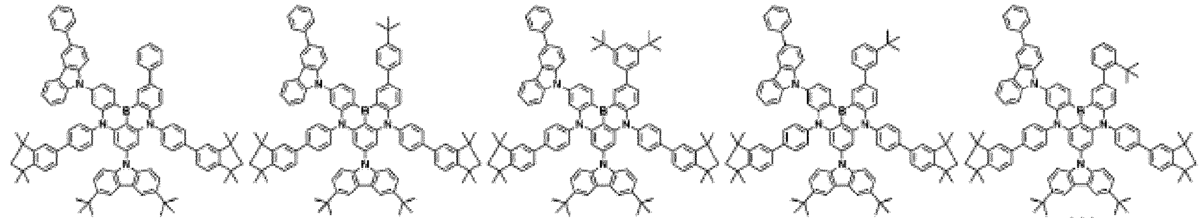

689 690 691 692 693

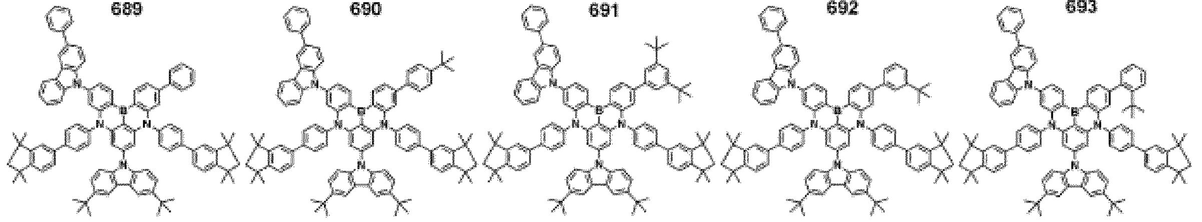

694 695 696 697 698

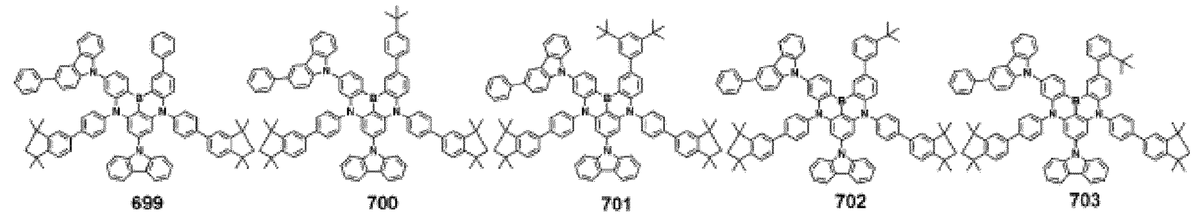

699 700 701 702 703

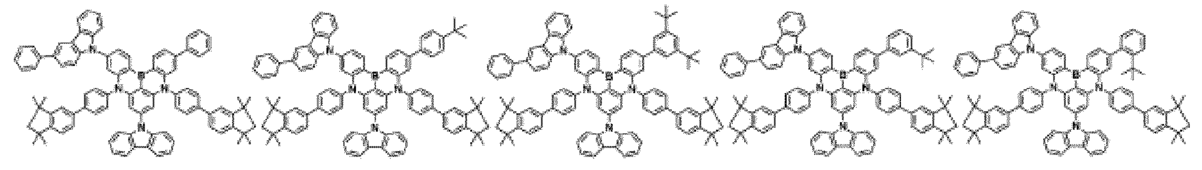

704 705 706 707 708

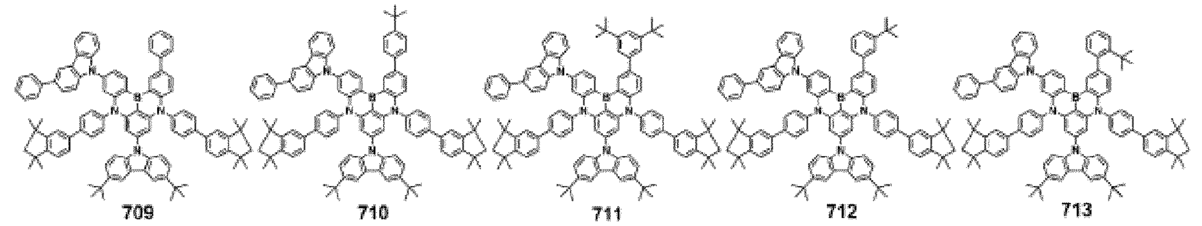

709 710 711 712 713

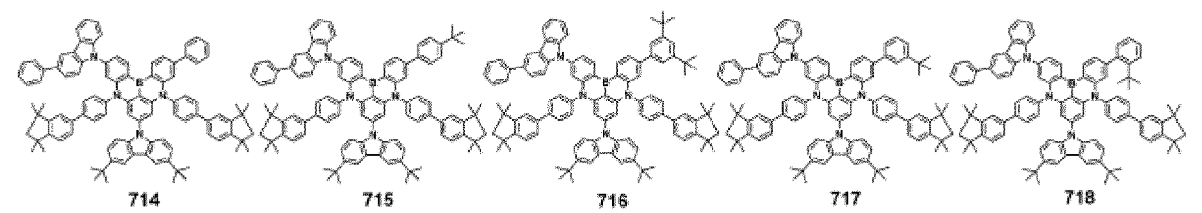

714 715 716 717 718

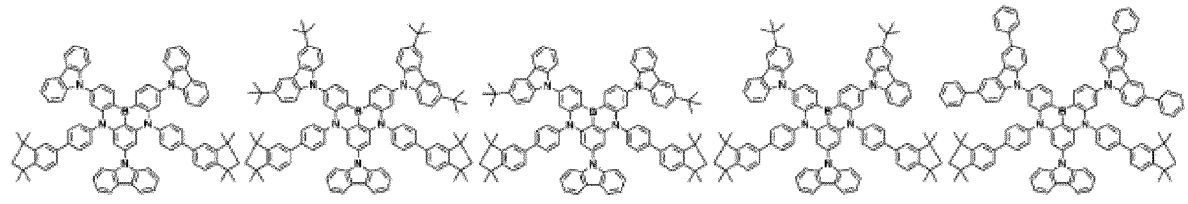

719 720 721 722 723

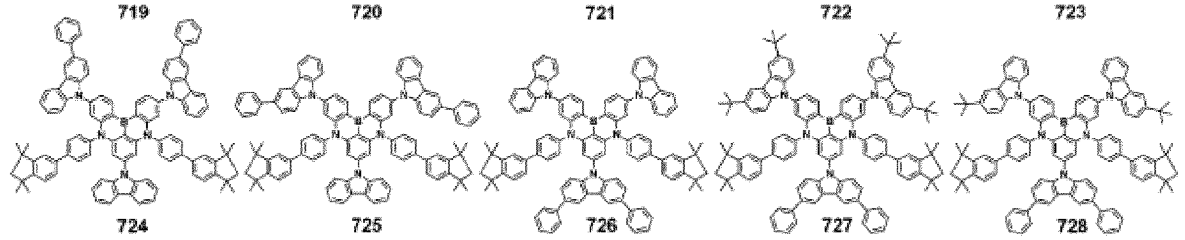

724 725 726 727 728

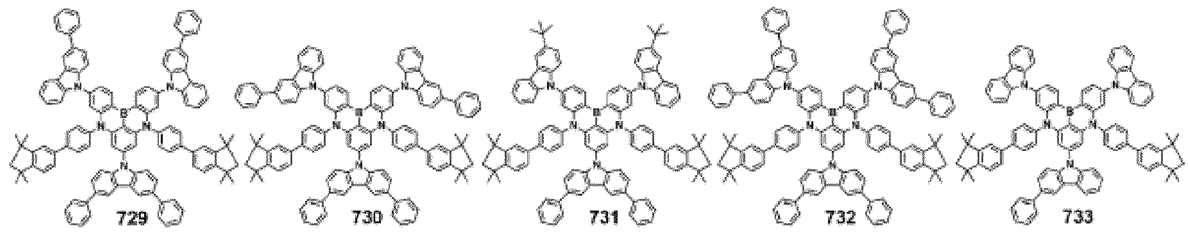

729 730 731 732 733

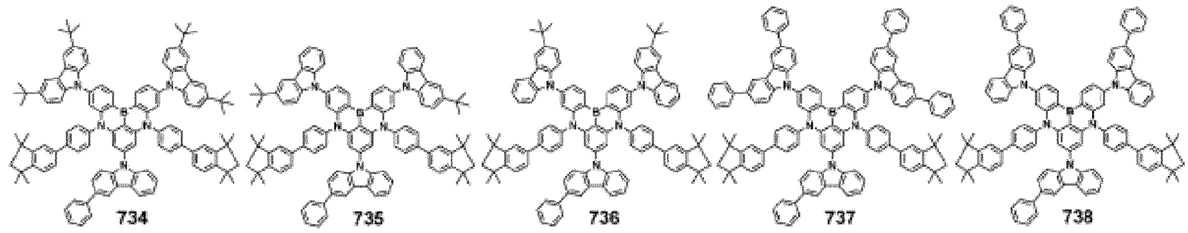

734 735 736 737 738

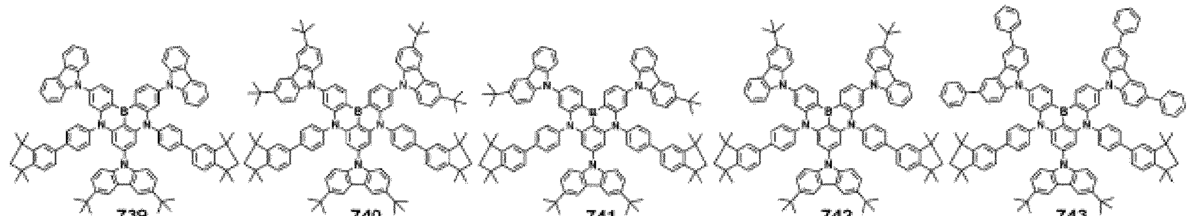

739 740 741 742 743

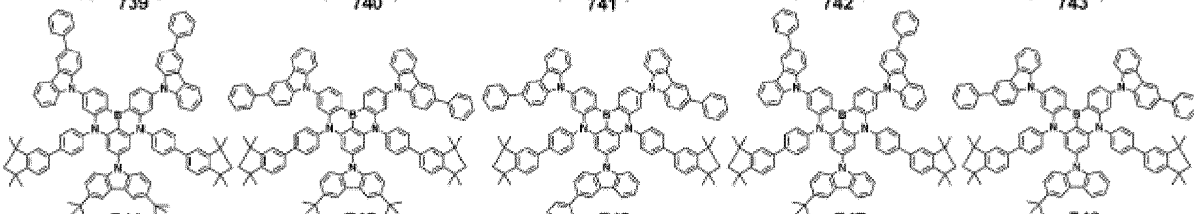

744 745 746 747 748

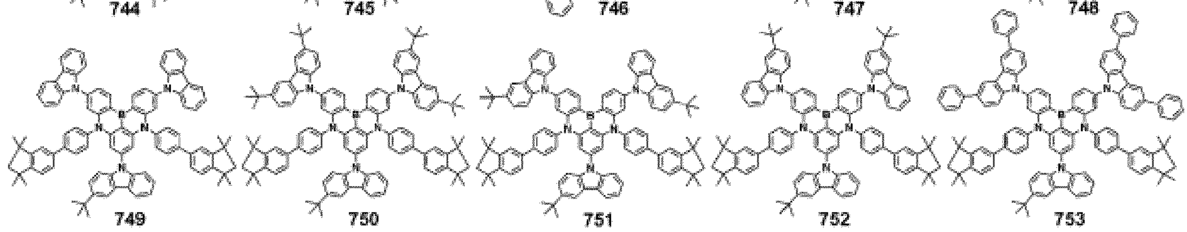

749 750 751 752 753

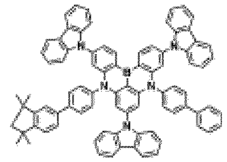
754
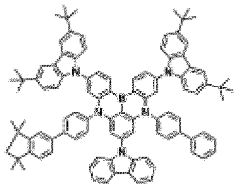
755
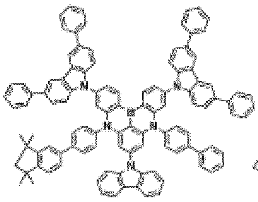
756
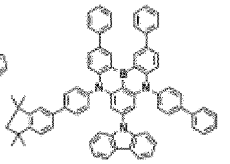
757
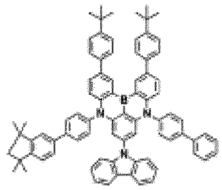
758

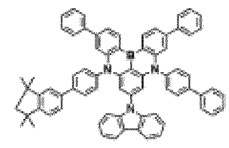
759
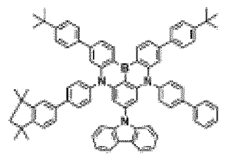
760
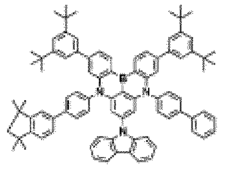
761
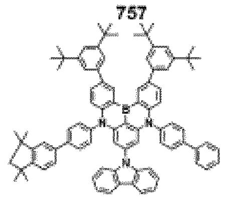
762
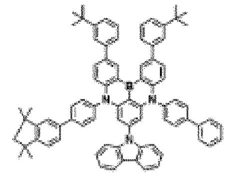
763

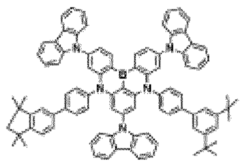
764
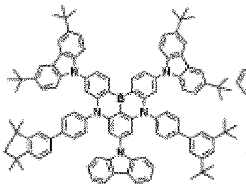
765
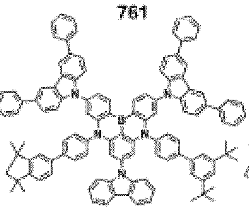
766
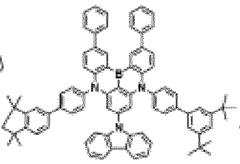
767
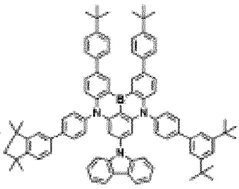
768

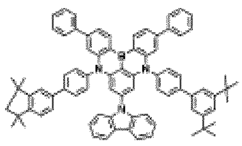
769
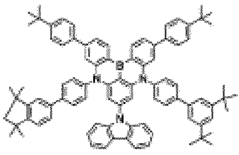
770
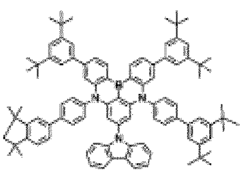
771
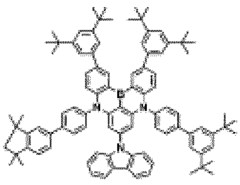
772
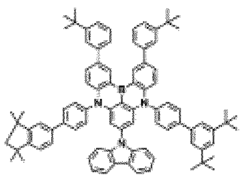
773

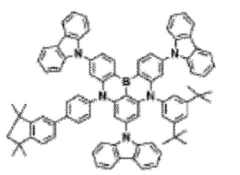
774
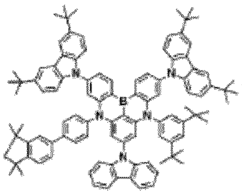
775
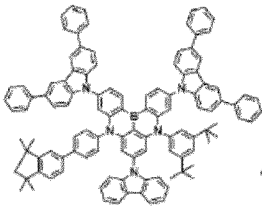
776
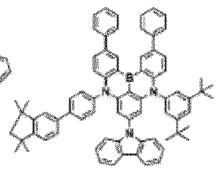
777
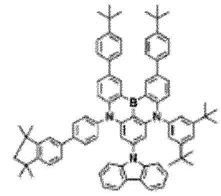
778

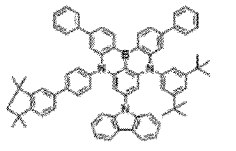
779
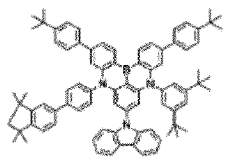
780
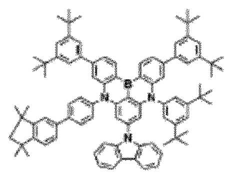
781
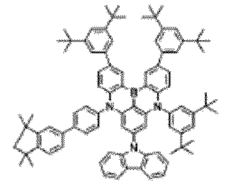
782
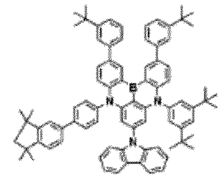
783

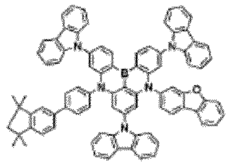
784
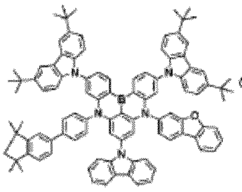
785
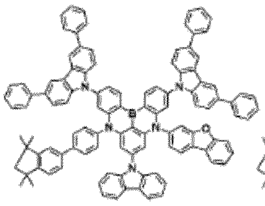
786
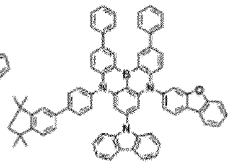
787
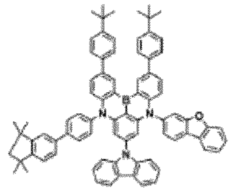
788

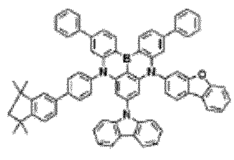
789

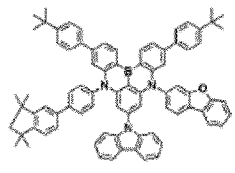
790

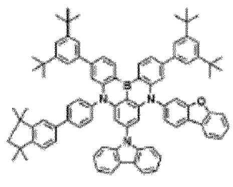
791

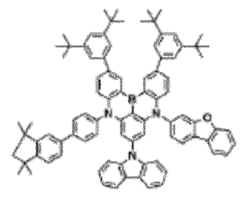
792

**10.** Organische lichtemittierende Vorrichtung, umfassend:

eine erste Elektrode,
eine zweite Elektrode; und
eine organische Schicht, die zwischen der ersten Elektrode und der zweiten Elektrode vorgesehen ist und eine Emissionsschicht umfasst,
wobei die organische Schicht die heterocyclische Verbindung nach einem der Ansprüche 1 bis 9 umfasst.

**11.** Organische lichtemittierende Vorrichtung nach Anspruch 10, wobei die Emissionsschicht die heterocyclische Verbindung umfasst.

**12.** Organische lichtemittierende Vorrichtung nach Anspruch 11, wobei die Emissionsschicht einen Wirt und einen Emitter umfasst, und
der Emitter die heterocyclische Verbindung umfasst.

**13.** Organische lichtemittierende Vorrichtung nach Anspruch 12, wobei eine Menge des Wirts größer als eine Menge der heterocyclischen Verbindung ist; und/oder
wobei die Emissionsschicht ferner einen Sensibilisator umfasst, wobei der Sensibilisator vorzugsweise eine phosphoreszierende Verbindung, eine verzögerte Fluoreszenzverbindung oder eine beliebige Kombination davon umfasst.

**14.** Organische lichtemittierende Vorrichtung nach einem der Ansprüche 11 bis 13, wobei die Emissionsschicht blaues Licht mit einer maximalen Emissionswellenlänge von 400 nm bis 490 nm emittiert.

**15.** Diagnostische Zusammensetzung, umfassend die heterocyclische Verbindung nach einem der Ansprüche 1 bis 9.

**Revendications**

**1.** Composé hétérocyclique représenté par la Formule 1 :

Formule 1

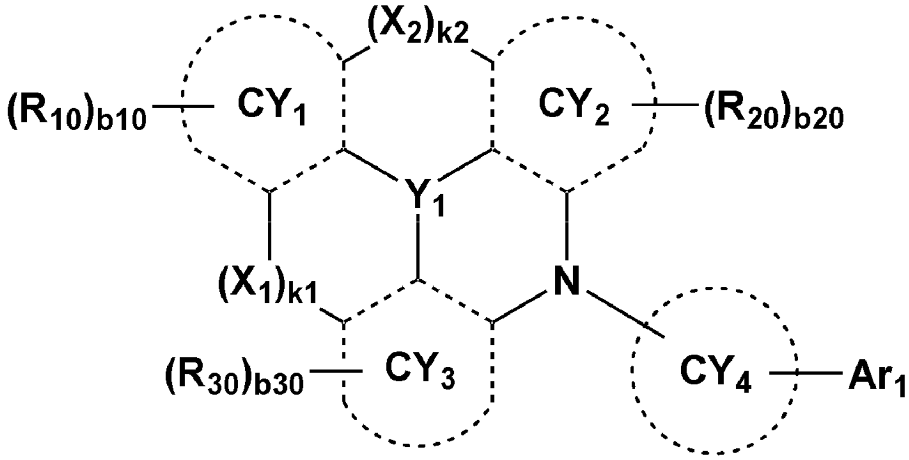

Formule 2

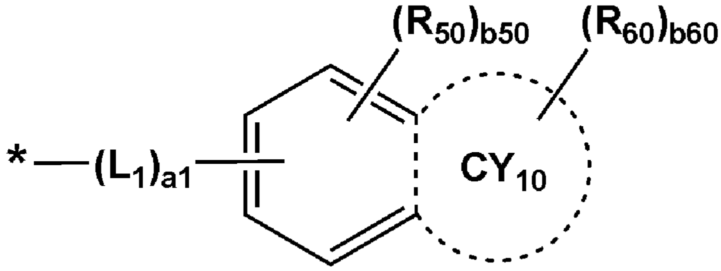

dans lequel, dans les Formules 1 et 2,

$Y_1$ est B, N, P, P(=O), ou P(=S),
$X_1$ et $X_2$ sont chacun indépendamment une liaison simple, O, S, Se, N[($CY_5$)-$Ar_2$], N($R_1$), C($R_1$)($R_2$), Si($R_1$)($R_2$), Ge($R_1$)($R_2$), B($R_1$), P($R_1$), P(=O)($R_1$), S(=O)$_2$ ou C(=O),
k1 vaut 0 ou 1,
k2 vaut 0 ou 1,
la somme de k1 et k2 est supérieure ou égale à 1,
$CY_1$ à $CY_3$ sont chacun indépendamment un groupe carbocyclique en $C_5$-$C_{30}$ ou un groupe hétérocyclique en $C_1$-$C_{30}$ substitué ou non substitué,
$CY_4$ et $CY_5$ sont chacun indépendamment un groupe carbocyclique en $C_5$-$C_{30}$ qui est non substitué ou substitué par au moins un $R_{40}$ ou un groupe hétérocyclique en $C_1$-$C_{30}$ qui est non substitué ou substitué par au moins un $R_{40}$,
$Ar_1$ et $Ar_2$ sont chacun indépendamment un groupe représenté par la Formule 2,
$L_1$ est une liaison simple, un groupe carbocyclique en $C_5$-$C_{30}$ substitué ou non substitué ou un groupe hétérocyclique en $C_1$-$C_{30}$ substitué ou non substitué,
a1 vaut 1, 2 ou 3 ;
$CY_{10}$ est un groupe carbocyclique non aromatique en $C_3$-$C_{10}$ ou un groupe hétérocyclique non aromatique en $C_1$-$C_{10}$,
$R_1$, $R_2$, $R_{10}$, $R_{20}$, $R_{30}$ et $R_{40}$ sont chacun indépendamment un groupe représenté par la Formule 2, un hydrogène, un deutérium, -F, -Cl, -Br, -I, -$SF_5$, un groupe hydroxyle, un groupe cyano, un groupe nitro, un groupe amidino, un groupe hydrazino, un groupe hydrazono, un groupe acide carboxylique ou un sel de celui-ci, un groupe acide sulfonique ou un sel de celui-ci, un groupe acide phosphorique ou un sel de celui-ci, un groupe alkyle en $C_1$-$C_{60}$ substitué ou non substitué, un groupe alcényle en $C_2$-$C_{60}$ substitué ou non substitué, un groupe alcynyle en $C_2$-$C_{60}$ substitué ou non substitué, un groupe alcoxy en $C_1$-$C_{60}$ substitué ou non substitué, un groupe cycloalkyle en $C_3$-$C_{10}$ substitué ou non substitué, un groupe hétérocycloalkyle en $C_1$-$C_{10}$ substitué ou non substitué, un groupe cycloalcényle en $C_3$-$C_{10}$ substitué ou non substitué, un groupe hétérocycloalcényle en $C_1$-$C_{10}$ substitué ou non substitué, un groupe aryle en $C_6$-$C_{60}$ substitué ou non substitué, un groupe alkylaryle en $C_7$-$C_{60}$ substitué ou non substitué, un groupe aryloxy en $C_6$-$C_{60}$ substitué ou non substitué, un groupe arylthio en $C_6$-$C_{60}$ substitué ou non substitué, un groupe hétéroaryle en $C_1$-$C_{60}$ substitué ou non substitué, un groupe alkylhétéroaryle en $C_2$-$C_{60}$ substitué ou non substitué, un groupe hétéroaryloxy en $C_1$-$C_{60}$ substitué ou non substitué, un groupe hétéroarylthio en $C_1$-$C_{60}$ substitué ou non substitué, un groupe polycyclique condensé non aromatique monovalent substitué ou non substitué, un groupe hétéropolycyclique condensé non aromatique monovalent substitué ou non substitué, -N($Q_1$)($Q_2$), -Si($Q_1$)($Q_2$)($Q_3$), -Ge($Q_1$)($Q_2$)($Q_3$), -C(=O)($Q_1$), -S(=O)($Q_1$), -S(=O)$_2$($Q_1$), -B($Q_1$)($Q_2$), -P($Q_1$)($Q_2$), -P(=O)($Q_1$)($Q_2$), -P(=S)($Q_1$)($Q_2$), ou un groupe représenté par la Formule 3,
$R_{50}$ et $R_{60}$ sont chacun indépendamment un hydrogène, un deutérium, -F, -Cl, -Br, -I, -$SF_5$, un groupe hydroxyle, un groupe cyano, un groupe nitro, un groupe amidino, un groupe hydrazino, un groupe hydrazono, un groupe acide carboxylique ou un sel de celui-ci, un groupe acide sulfonique ou un sel de celui-ci, un groupe acide phosphorique ou un sel de celui-ci, un groupe alkyle en $C_1$-$C_{60}$ substitué ou non substitué, un groupe alcényle en $C_2$-$C_{60}$ substitué ou non substitué, un groupe alcynyle en $C_2$-$C_{60}$ substitué ou non substitué, un groupe alcoxy en $C_1$-$C_{60}$ substitué ou non substitué, un groupe cycloalkyle en $C_3$-$C_{10}$ substitué ou non substitué, un groupe hétérocycloalkyle en $C_1$-$C_{10}$ substitué ou non substitué, un groupe cycloalcényle en $C_3$-$C_{10}$ substitué ou non substitué, un groupe hétérocycloalcényle en $C_1$-$C_{10}$ substitué ou non substitué, un groupe aryle en $C_6$-$C_{60}$ substitué ou non substitué, un groupe alkylaryle en $C_7$-$C_{60}$ substitué ou non substitué, un groupe aryloxy en $C_6$-$C_{60}$ substitué ou non substitué, un groupe arylthio en $C_6$-$C_{60}$ substitué ou non substitué, un groupe hétéroaryle en $C_1$-$C_{60}$ substitué ou non substitué, un groupe alkylhétéroaryle en $C_2$-$C_{60}$ substitué ou non

substitué, un groupe hétéroaryloxy en $C_1$-$C_{60}$ substitué ou non substitué, un groupe hétéroarylthio en $C_1$-$C_{60}$ substitué ou non substitué, un groupe polycyclique condensé non aromatique monovalent substitué ou non substitué, un groupe hétéropolycyclique condensé non aromatique monovalent substitué ou non substitué, -N($Q_1$)($Q_2$), -Si($Q_1$)($Q_2$)($Q_3$), -Ge($Q_1$)($Q_2$)($Q_3$), -C(=O)($Q_1$), -S(=O)($Q_1$), -S(=O)$_2$($Q_1$), -B($Q_1$)($Q_2$), -P($Q_1$)($Q_2$), -P(=O)($Q_1$)($Q_2$), -P(=S)($Q_1$)($Q_2$), ou un groupe représenté par la Formule 3,
dans lequel au moins l'un de $R_{10}$, au moins l'un de $R_{20}$ et/ou au moins l'un de $R_{30}$ est un groupe représenté par la Formule 3 ;

Formule 3

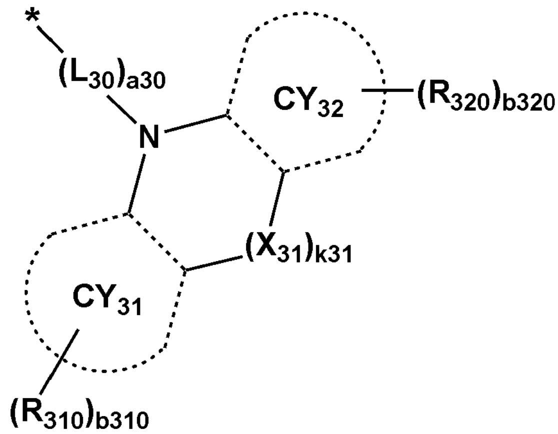

dans lequel, dans la Formule 3,

$X_{31}$ est une liaison simple, O, S, N($R_{301}$), ou C($R_{301}$)($R_{302}$),
k31 vaut 1,
$CY_{31}$ et $CY_{32}$ sont chacun indépendamment un groupe carbocyclique en $C_5$-$C_{30}$ ou un groupe hétérocyclique en $C_1$-$C_{30}$,
$L_{30}$ est une liaison simple, un groupe carbocyclique en $C_5$-$C_{30}$ substitué ou non substitué ou un groupe hétérocyclique en $C_1$-$C_{30}$ substitué ou non substitué,
a30 vaut 0, 1, 2 ou 3,
$R_{301}$, $R_{302}$, $R_{310}$ et $R_{320}$ sont chacun indépendamment un hydrogène, un deutérium, -F, -Cl, -Br, -I, -$SF_5$, un groupe hydroxyle, un groupe cyano, un groupe nitro, un groupe amidino, un groupe hydrazino, un groupe hydrazono, un groupe acide carboxylique ou un sel de celui-ci, un groupe acide sulfonique ou un sel de celui-ci, un groupe acide phosphorique ou un sel de celui-ci, un groupe alkyle en $C_1$-$C_{60}$ substitué ou non substitué, un groupe alcényle en $C_2$-$C_{60}$ substitué ou non substitué, un groupe alcynyle en $C_2$-$C_{60}$ substitué ou non substitué, un groupe alcoxy en $C_1$-$C_{60}$ substitué ou non substitué, un groupe cycloalkyle en $C_3$-$C_{10}$ substitué ou non substitué, un groupe hétérocycloalkyle en $C_1$-$C_{10}$ substitué ou non substitué, un groupe cycloalcényle en $C_3$-$C_{10}$ substitué ou non substitué, un groupe hétérocycloalcényle en $C_1$-$C_{10}$ substitué ou non substitué, un groupe aryle en $C_6$-$C_{60}$ substitué ou non substitué, un groupe alkylaryle en $C_7$-$C_{60}$ substitué ou non substitué, un groupe aryloxy en $C_6$-$C_{60}$ substitué ou non substitué, un groupe arylthio en $C_6$-$C_{60}$ substitué ou non substitué, un groupe hétéroaryle en $C_1$-$C_{60}$ substitué ou non substitué, un groupe alkylhétéroaryle en $C_2$-$C_{60}$ substitué ou non substitué, un groupe hétéroaryloxy en $C_1$-$C_{60}$ substitué ou non substitué, un groupe hétéroarylthio en $C_1$-$C_{60}$ substitué ou non substitué, un groupe polycyclique condensé monovalent non aromatique substitué ou non substitué, un groupe hétéropolycyclique condensé monovalent non aromatique substitué ou non substitué, -N($Q_1$)($Q_2$), -Si($Q_1$)($Q_2$)($Q_3$), -Ge($Q_1$)($Q_2$)($Q_3$), -C(=O)($Q_1$), -S(=O)($Q_1$), -S(=O)$_2$($Q_1$), -B($Q_1$)($Q_2$), -P($Q_1$)($Q_2$), -P(=O)($Q_1$)($Q_2$), ou -P(=S)($Q_1$)($Q_2$),
b310 et b320 valent chacun indépendamment 1, 2, 3, 4, 5, 6, 7 ou 8,
b10, b20, b30, b40 et b60 valent chacun indépendamment 1, 2, 3, 4, 5, 6, 7 ou 8,
b50 vaut 1, 2 ou 3,
un substituant du groupe carbocyclique en $C_5$-$C_{30}$ substitué, du groupe hétérocyclique en $C_1$-$C_{30}$ substitué, du groupe alkyle en $C_1$-$C_{60}$ substitué, du groupe alcényle en $C_2$-$C_{60}$ substitué, du groupe alcynyle en $C_2$-$C_{60}$ substitué, du groupe alcoxy en $C_1$-$C_{60}$ substitué, du groupe cycloalkyle en $C_3$-$C_{10}$ substitué, du groupe hétérocycloalkyle en $C_1$-$C_{10}$ substitué, du groupe cycloalcényle en $C_3$-$C_{10}$ substitué, du groupe hétérocycloalcényle en $C_1$-$C_{10}$ substitué, du groupe aryle en $C_6$-$C_{60}$ substitué, du groupe alkylaryle en $C_7$-$C_{60}$

substitué, du groupe aryloxy en $C_6$-$C_{60}$ substitué, du groupe arylthio en $C_6$-$C_{60}$ substitué, du groupe hétéroaryle en $C_1$-$C_{60}$ substitué, du groupe alkylhétéroaryle en $C_2$-$C_{60}$, du groupe hétéroaryloxy en $C_1$-$C_{60}$ substitué, du groupe hétéroarylthio en $C_1$-$C_{60}$ substitué, du groupe polycyclique condensé non aromatique monovalent substitué et du groupe hétéropolycyclique condensé non aromatique monovalent substitué est :

un deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{60}$, un groupe alcényle en $C_2$-$C_{60}$, un groupe alcynyle en $C_2$-$C_{60}$ ou un groupe alcoxy en $C_1$-$C_{60}$ ;
un groupe alkyle en $C_1$-$C_{60}$, un groupe alcényle en $C_2$-$C_{60}$, un groupe alcynyle en $C_2$-$C_{60}$ ou un groupe alcoxy en $C_1$-$C_{60}$, chacun étant substitué par un deutérium, -F, -Cl, -Br, -I, $-CD_3$, - $CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe cycloalkyle en $C_3$-$C_{10}$, un groupe hétérocycloalkyle en $C_1$-$C_{10}$, un groupe cycloalcényle en $C_3$-$C_{10}$, un groupe hétérocycloalcényle en $C_1$-$C_{10}$, un groupe aryle en $C_6$-$C_{60}$, un groupe aryloxy en $C_6$-$C_{60}$, un groupe arylthio en $C_6$-$C_{60}$, un groupe hétéroaryle en $C_1$-$C_{60}$, un groupe polycyclique condensé non aromatique monovalent, un groupe hétéropolycyclique condensé non aromatique monovalent, $-N(Q_{11})(Q_{12})$, $-Si(Q_{11})(Q_{12})(Q_{13})$, $-Ge(Q_{11})(Q_{12})(Q_{13})$, - $C(=O)(Q_{11})$, $-S(=O)(Q_{11})$, $-S(=O)_2(Q_{11})$, $-B(Q_{11})(Q_{12})$, $-P(Q_{11})(Q_{12})$, $-P(=O)(Q_{11})(Q_{12})$, - $P(=S)(Q_{11})(Q_{12})$ ou toute combinaison de ceux-ci ;
un groupe cycloalkyle en $C_3$-$C_{10}$, un groupe hétérocycloalkyle en $C_1$-$C_{10}$, un groupe cycloalcényle en $C_3$-$C_{10}$, un groupe hétérocycloalcényle en $C_1$-$C_{10}$, un groupe aryle en $C_6$-$C_{60}$, un groupe aryloxy en $C_6$-$C_{60}$, un groupe arylthio en $C_6$-$C_{60}$, un groupe hétéroaryle en $C_1$-$C_{60}$, un groupe polycyclique condensé non aromatique monovalent, ou un groupe hétéropolycyclique condensé non aromatique monovalent, chacun étant non substitué ou substitué par un deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{60}$, un groupe alcényle en $C_2$-$C_{60}$, un groupe alcynyle en $C_2$-$C_{60}$, un groupe alcoxy en $C_1$-$C_{60}$, un groupe cycloalkyle en $C_3$-$C_{10}$, un groupe hétérocycloalkyle en $C_1$-$C_{10}$, un groupe cycloalcényle en $C_3$-$C_{10}$, un groupe hétérocycloalcényle en $C_1$-$C_{10}$, un groupe aryle en $C_6$-$C_{60}$, un groupe aryloxy en $C_6$-$C_{60}$, un groupe arylthio en $C_6$-$C_{60}$, un groupe hétéroaryle en $C_1$-$C_{60}$, un groupe polycyclique condensé non aromatique monovalent, un groupe hétéropolycyclique condensé non aromatique monovalent, - $N(Q_{21})(Q_{22})$, $-Si(Q_{21})(Q_{22})(Q_{23})$, $-Ge(Q_{21})(Q_{22})(Q_{23})$, $-C(=O)(Q_{21})$, $-S(=O)(Q_{21})$, $-S(=O)_2(Q_{21})$, - $B(Q_{21})(Q_{22})$, $-P(Q_{21})(Q_{22})$, $-P(=O)(Q_{21})(Q_{22})$, $-P(=S)(Q_{21})(Q_{22})$, ou toute combinaison de ceux-ci ;
$-N(Q_{31})(Q_{32})$, $-Si(Q_{31})(Q_{32})(Q_{33})$, $-Ge(Q_{31})(Q_{32})(Q_{33})$, $-C(=O)(Q_{31})$, $-S(=O)(Q_{31})$, - $S(=O)_2(Q_{31})$, $-B(Q_{31})(Q_{32})$, $-P(Q_{31})(Q_{32})$, $-P(=O)(Q_{31})(Q_{32})$, ou $-P(Q_{31})(Q_{32})$ ;
$Q_1$ à $Q_3$, $Q_{11}$ à $Q_{13}$, $Q_{21}$ à $Q_{23}$, et $Q_{31}$ à $Q_{33}$ sont chacun indépendamment : un hydrogène ; un deutérium ; -F ; -Cl ; -Br ; -I ; un groupe hydroxyle ; un groupe cyano ; un groupe nitro ; un groupe amidino ; un groupe hydrazine ; un groupe hydrazone ; un acide carboxylique ou un sel de celui-ci ; un acide sulfonique ou un sel de celui-ci ; un acide phosphorique ou un sel de celui-ci ; un groupe alkyle en $C_1$-$C_{60}$ qui est non substitué ou substitué par un deutérium, un groupe alkyle en $C_1$-$C_{60}$, un groupe aryle en $C_6$-$C_{60}$, ou toute combinaison de ceux-ci ; un groupe alcényle en $C_2$-$C_{60}$ ; un groupe alcynyle en $C_2$-$C_{60}$ ; un groupe alcoxy en $C_1$-$C_{60}$ ; un groupe cycloalkyle en $C_3$-$C_{10}$ ; un groupe hétérocycloalkyle en $C_1$-$C_{10}$ ; un groupe cycloalcényle en $C_3$-$C_{10}$, un groupe hétérocycloalcényle en $C_1$-$C_{10}$, un groupe aryle en $C_6$-$C_{60}$ qui est non substitué ou substitué par un deutérium, un groupe alkyle en $C_1$-$C_{60}$, un groupe aryle en $C_6$-$C_{60}$, ou toute combinaison de ceux-ci ; un groupe aryloxy en $C_6$-$C_{60}$ ; un groupe arylthio en $C_6$-$C_{60}$ ; un groupe hétéroaryle en $C_1$-$C_{60}$ ; un groupe polycyclique condensé non aromatique monovalent ; ou un groupe hétéropolycyclique condensé non aromatique monovalent, et
* indique un site de liaison à un atome voisin.

**2.** Composé hétérocyclique de la revendication 1, ledit composé hétérocyclique étant représenté par la Formule 1A ou 1B :

Formule 1A

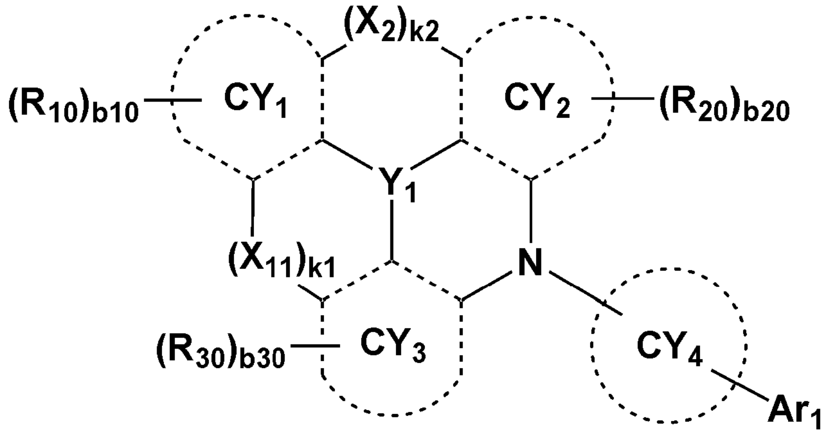

Formule 1B

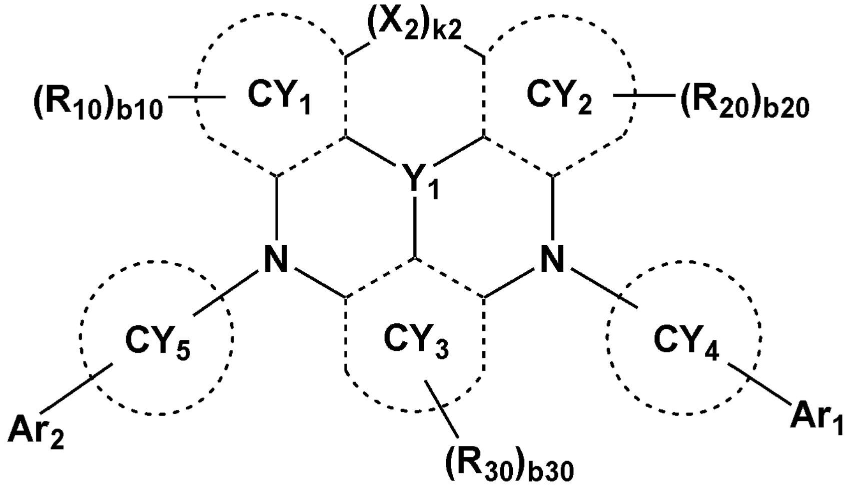

dans lequel, dans les Formules 1A et 1B,

$X_{11}$ est une liaison simple, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$ ou C(=O), et
$Y_1$, $X_2$, k1, k2, $CY_1$ à $CY_5$, $Ar_1$, $Ar_2$, $R_1$, $R_2$, $R_{10}$, $R_{20}$, $R_{30}$, b10, b20 et b30 peuvent chacun être les mêmes que ceux décrits dans la revendication 1.

**3.** Composé hétérocyclique des revendications 1 ou 2, dans lequel $CY_1$ à $CY_3$ sont chacun indépendamment un groupe carbocyclique aromatique en $C_6$-$C_{30}$ ou un groupe hétérocyclique aromatique en $C_1$-$C_{30}$ ; et/ou

dans lequel $CY_4$ et $CY_5$ sont chacun indépendamment représentés par l'une des Formules 4-1 à 4-3 :

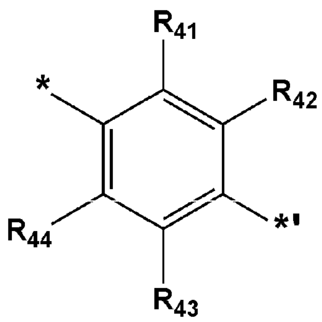

**4-1**

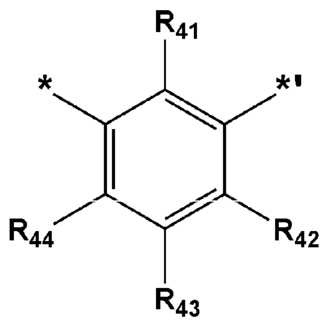

**4-2**

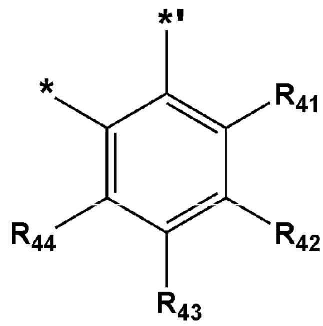

**4-3**

dans lequel, dans les Formules 4-1 à 4-3,
$R_{41}$ à $R_{44}$ sont chacun indépendamment les mêmes que ceux décrits en relation avec $R_{40}$ dans la revendication 1, et
* et *' indiquent chacun un site de liaison à un atome voisin.

**4.** Composé hétérocyclique de l'une quelconque des revendications 1 à 3, dans lequel $CY_{10}$ est un groupe cyclopentane, un groupe cyclohexane, un groupe cycloheptane, un groupe cyclopentène, un groupe cyclohexène ou un groupe cycloheptène ; et/ou

dans lequel $Ar_1$ et $Ar_2$ sont chacun indépendamment un groupe représenté par la Formule 2A ou 2B :

Formule 2A

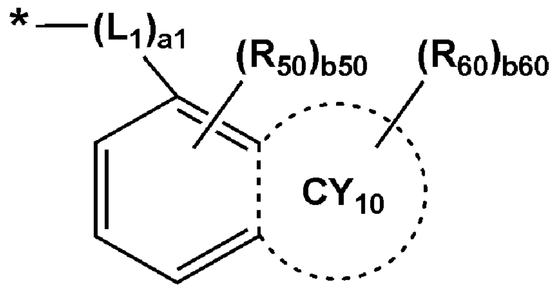

Formule 2B

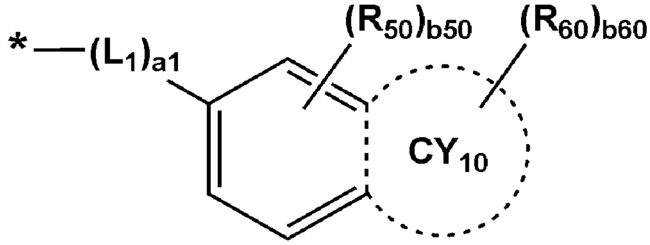

dans lequel, dans les Formules 2A et 2B,
chacun de $L_1$, a1, $CY_{10}$, $R_{50}$, $R_{60}$, b50 et b60 est le même que celui décrit dans la revendication 1, et
* indique un site de liaison à un atome voisin.

**5.** Composé hétérocyclique de l'une quelconque des revendications 1 à 4, dans lequel $Ar_1$ et $Ar_2$ sont chacun indépendamment un groupe représenté par l'une des Formules 5-1 à 5-4 :

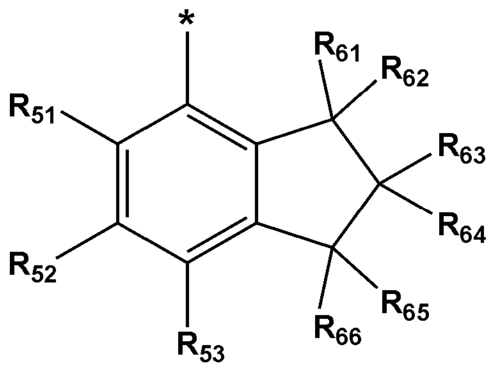

**5-1**

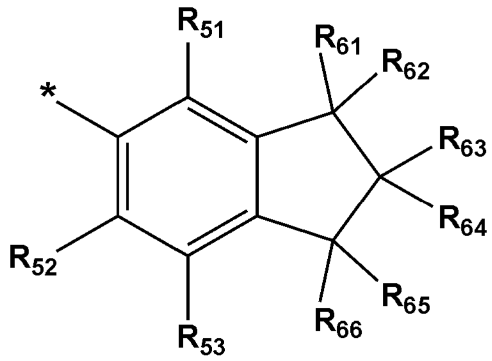

**5-2**

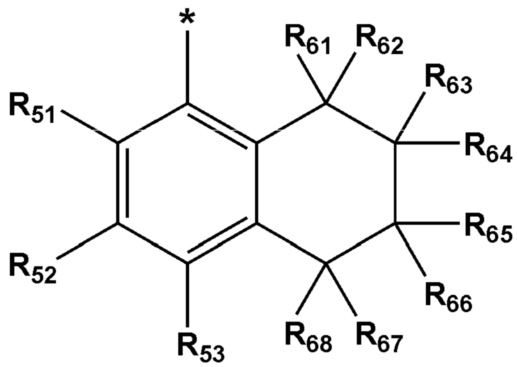

**5-3**

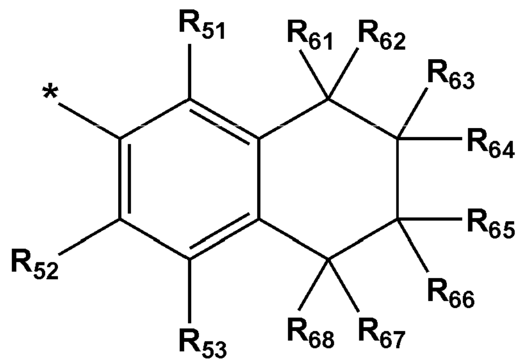

**5-4**

dans lequel, dans les Formules 5-1 à 5-4,

$R_{51}$ à $R_{53}$ sont chacun les mêmes que ceux décrits en relation avec $R_{50}$ dans la revendication 1,
$R_{61}$ à $R_{68}$ sont chacun les mêmes que ceux décrits en relation avec $R_{60}$ dans la revendication 1, et
* indique un site de liaison à un atome voisin.

**6.** Composé hétérocyclique de l'une quelconque des revendications 1 à 5, dans lequel un groupe représenté par la Formule 3 est un groupe représenté par l'une des Formules 3-2 ou 3-3 :

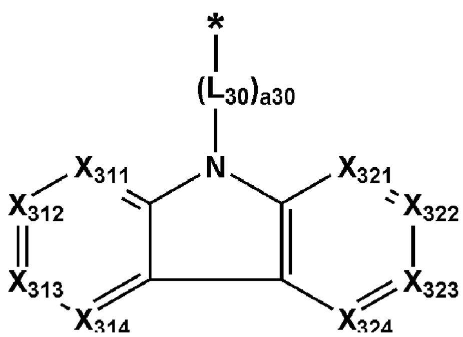

**3-2**

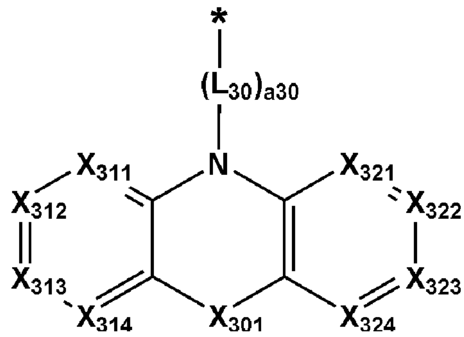

3-3

dans lequel, dans les Formules 3-2 ou 3-3,

$L_{30}$ et a30 sont chacun les mêmes que ceux décrits dans la revendication 1,
$X_{301}$ est le même que celui décrit en relation avec X31 dans la revendication 1,
$X_{311}$ est $C(R_{311})$ ou N, $X_{312}$ est $C(R_{312})$ ou N, $X_{313}$ est $C(R_{313})$ ou N, $X_{314}$ est $C(R_{314})$ ou N,
$X_{321}$ est $C(R_{321})$ ou N, $X_{322}$ est $C(R_{322})$ ou N, $X_{323}$ est $C(R_{323})$ ou N, $X_{324}$ est $C(R_{324})$ ou N,
$R_{311}$ à $R_{314}$ sont chacun indépendamment les mêmes que ceux décrits en relation avec $R_{310}$ dans la revendication 1,
$R_{321}$ à $R_{324}$ sont chacun indépendamment les mêmes que ceux décrits en relation avec $R_{320}$ dans la revendication 1, et
* indique un site de liaison à un atome voisin.

**7.** Composé hétérocyclique de l'une quelconque des revendications 1 à 6, dans lequel le composé hétérocyclique est représenté par l'une des Formules 11-1 à 11-9 :

Formule 11-1

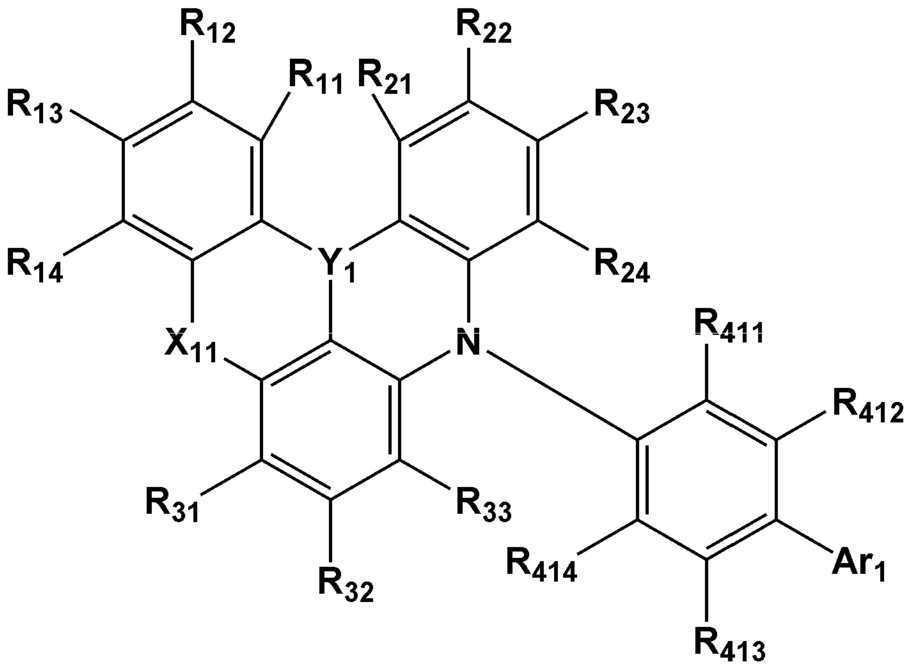

Formule 11-2

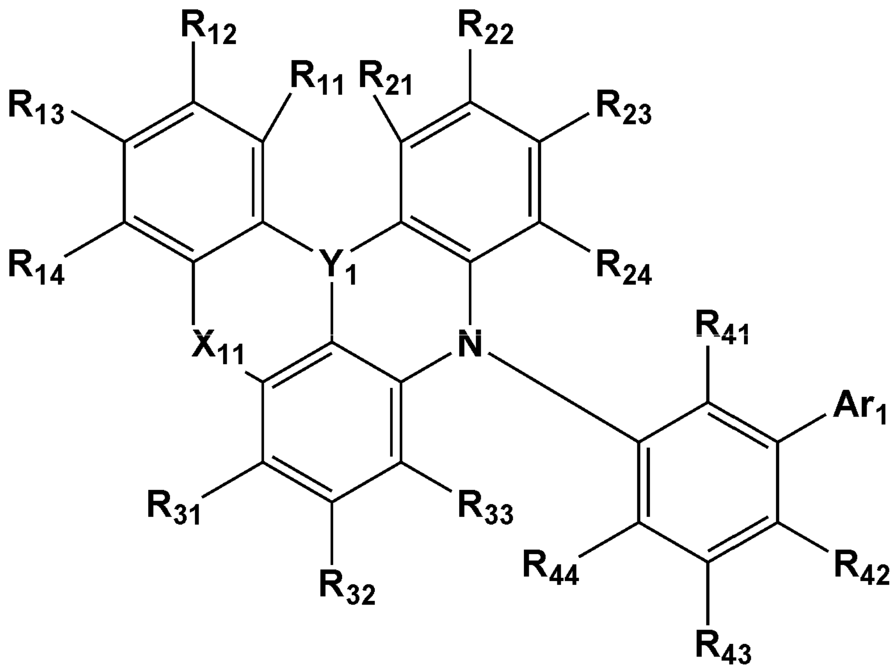

Formule 11-3

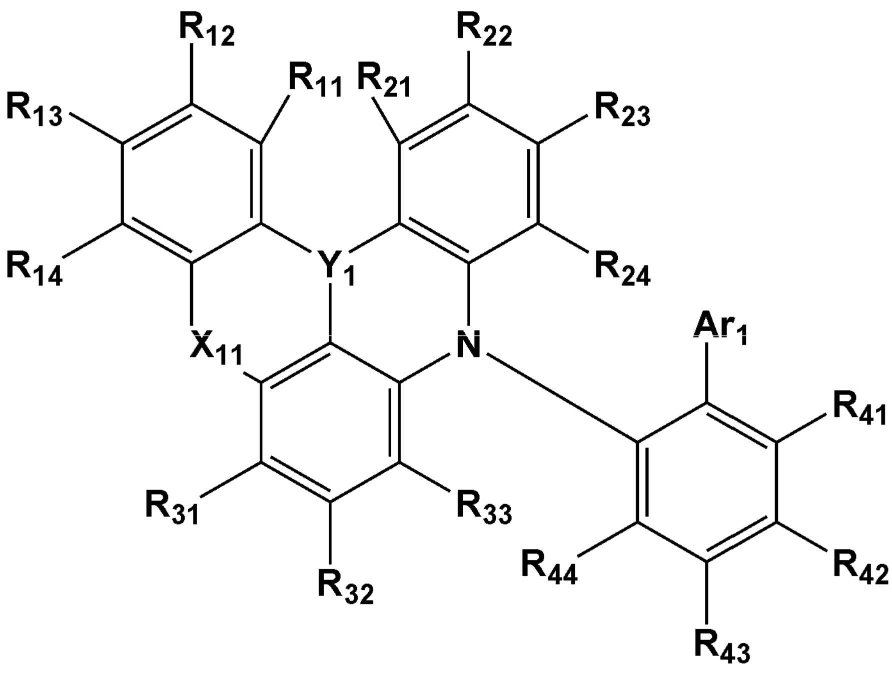

Formule 11-4

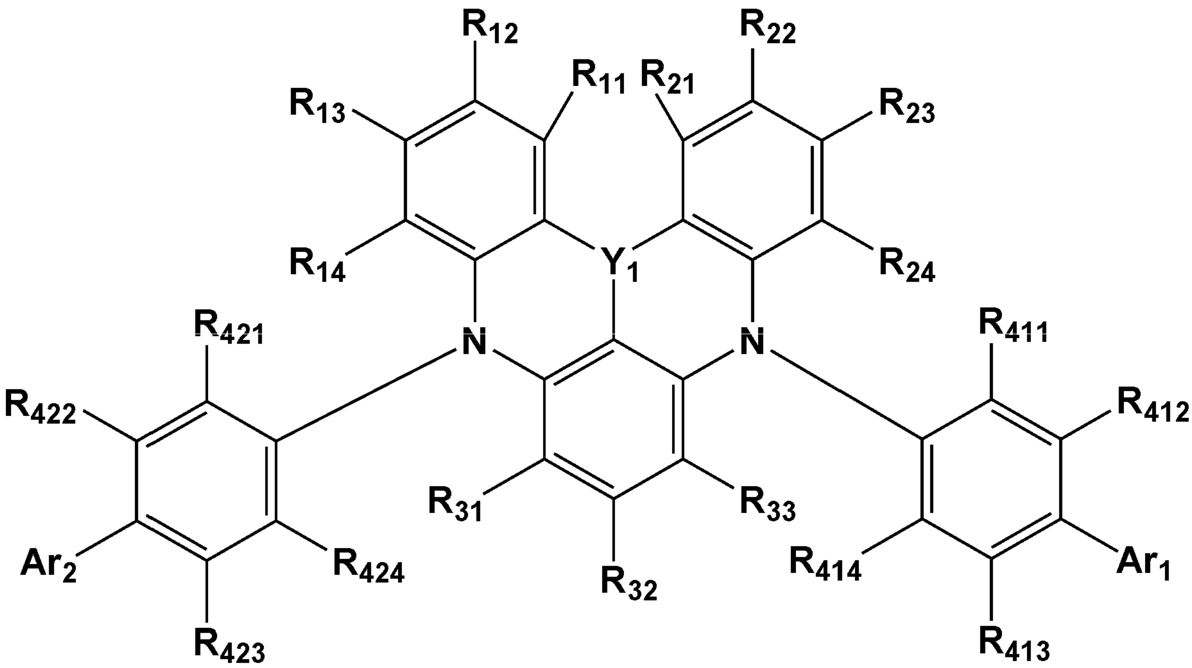

Formule 11-5

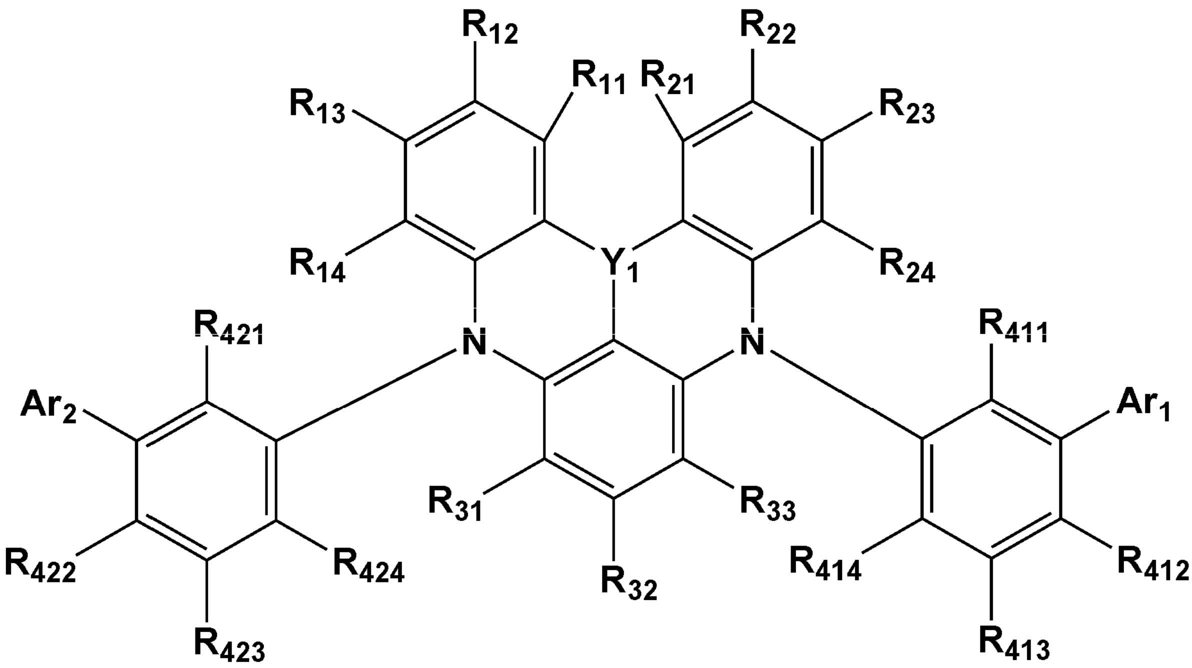

Formule 11-6

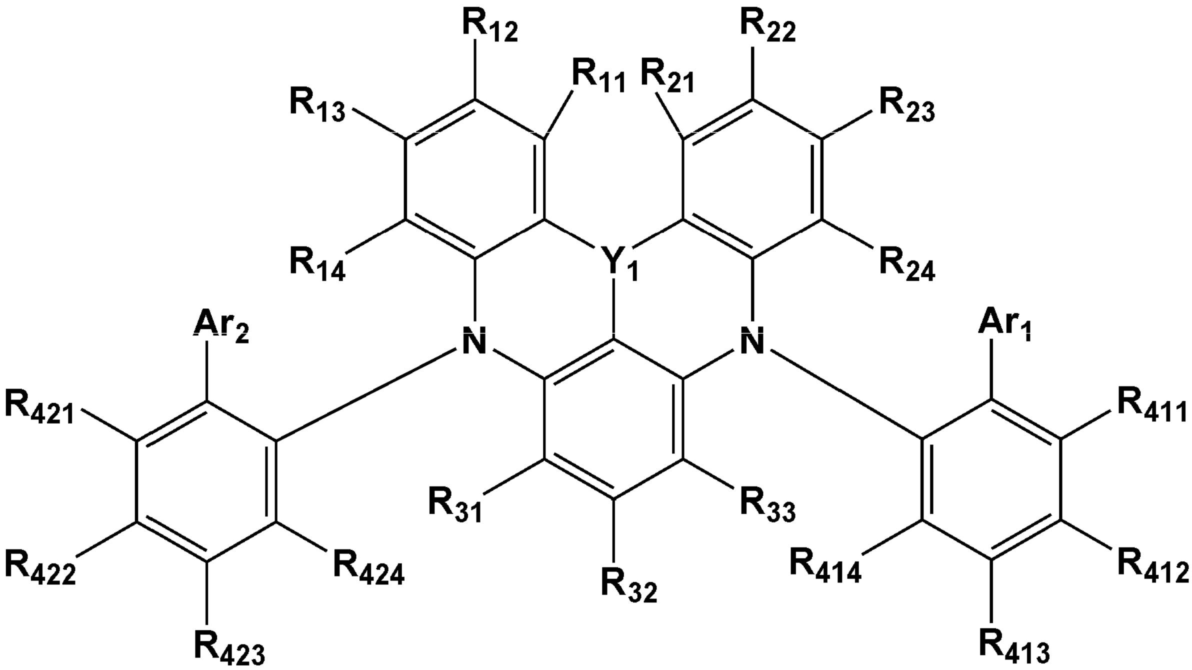

Formule 11-7

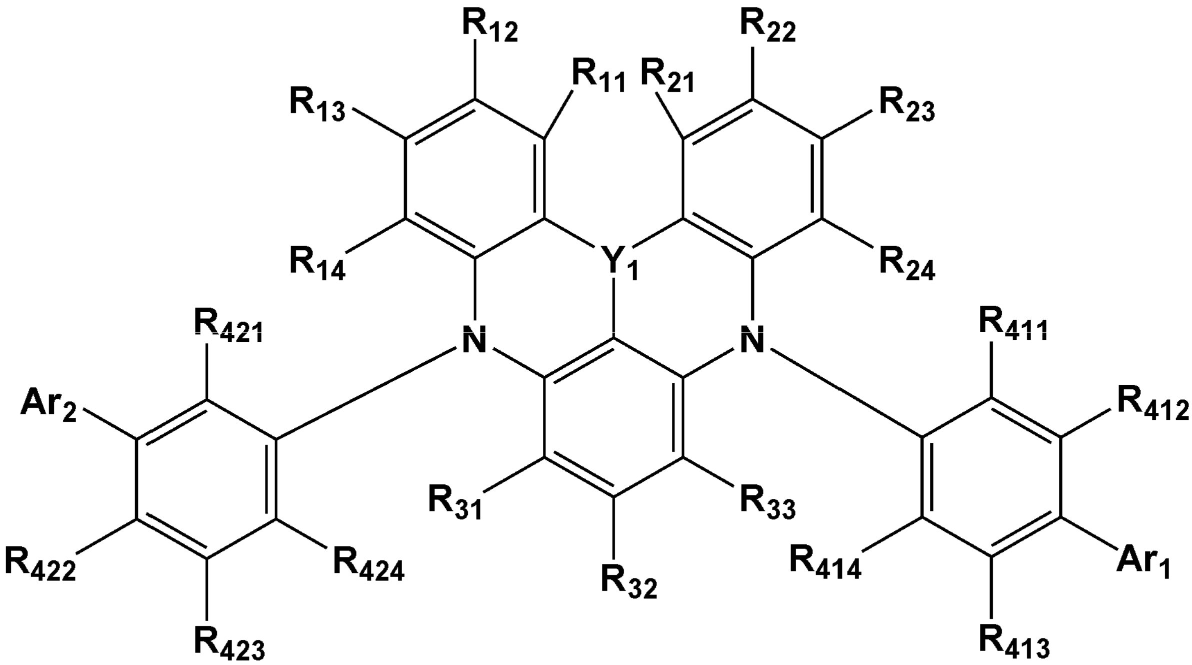

Formule 11-8

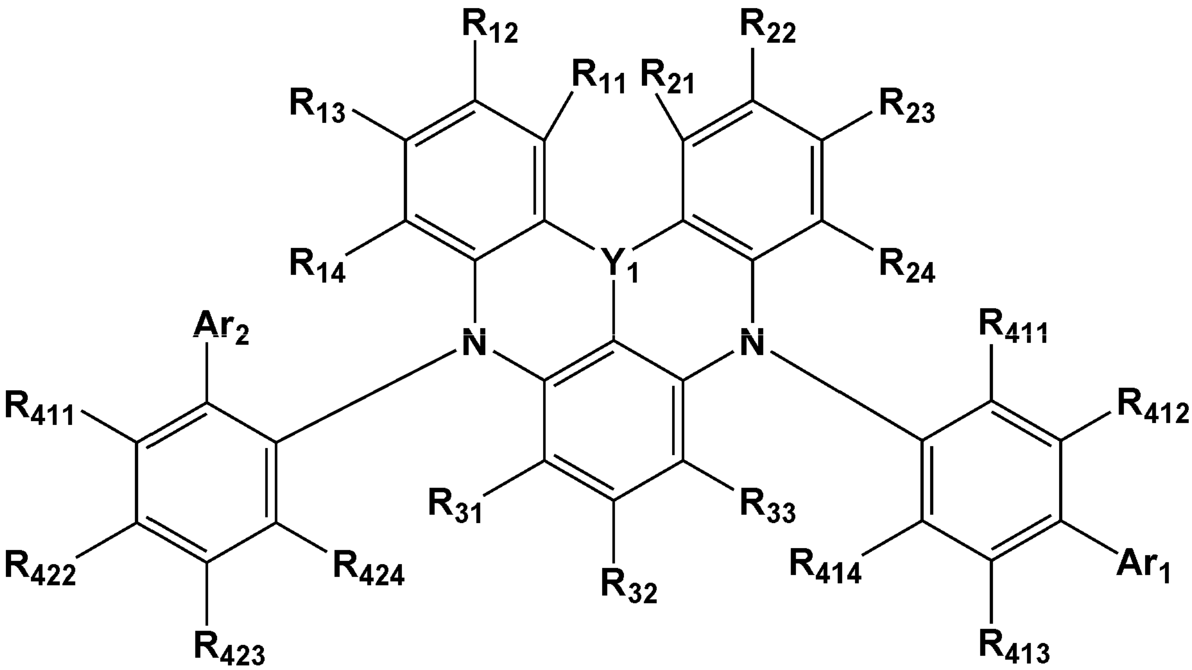

Formule 11-9

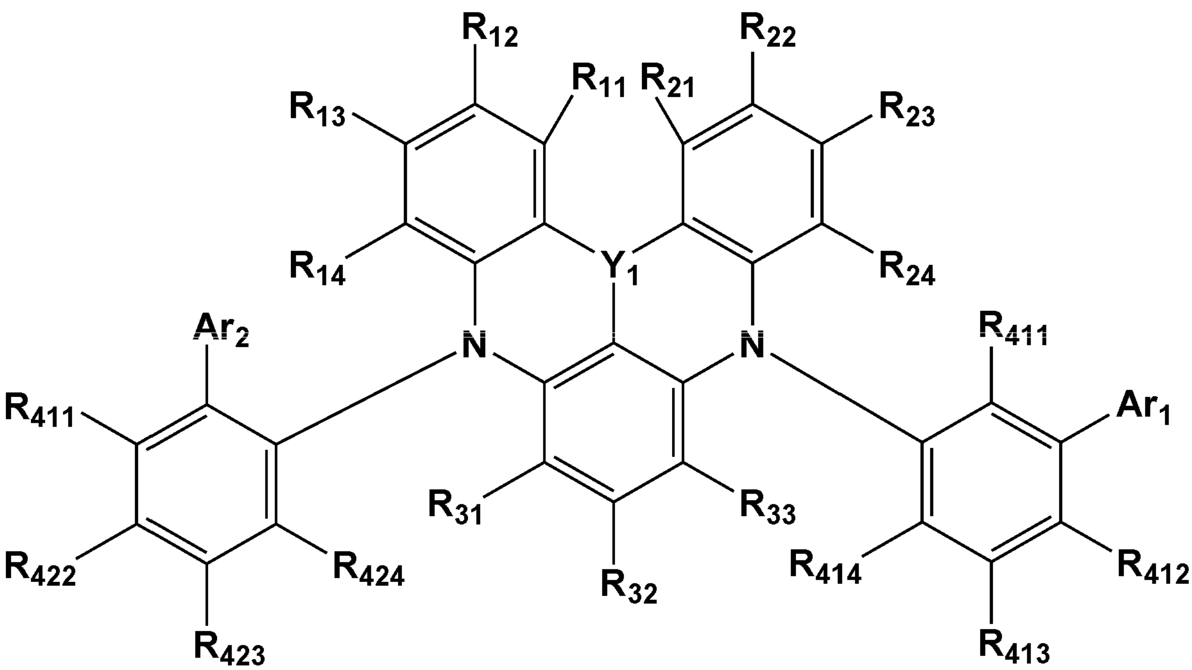

dans lequel, dans les Formules 11-1 à 11-9,

$Y_1$, $Ar_1$ et $Ar_2$ sont chacun les mêmes que ceux décrits dans la revendication 1,
$X_{11}$ est une liaison simple, O, S, Se, $N(R_1)$, $C(R_1)(R_2)$, $Si(R_1)(R_2)$, $Ge(R_1)(R_2)$, $B(R_1)$, $P(R_1)$, $P(=O)(R_1)$, $S(=O)_2$ ou C(=O), $R_1$ et $R_2$ sont les mêmes que ceux décrits dans la revendication 1,
$R_{11}$ à $R_{14}$ sont chacun indépendamment les mêmes que ceux décrits en relation avec $R_{10}$ dans la revendication 1,
$R_{21}$ à $R_{24}$ sont chacun indépendamment les mêmes que ceux décrits en relation avec $R_{20}$ dans la revendication

1,

$R_{31}$ à $R_{33}$ sont chacun indépendamment les mêmes que ceux décrits en relation avec $R_{30}$ dans la revendication 1, et

$R_{411}$ à $R_{414}$ et $R_{421}$ à $R_{424}$ sont chacun indépendamment les mêmes que ceux décrits en relation avec $R_{40}$ dans la revendication 1.

**8.** Composé hétérocyclique de l'une quelconque des revendications 1 à 7, dans lequel le composé hétérocyclique possède une structure symétrique ou une structure asymétrique.

**9.** Composé hétérocyclique de l'une quelconque des revendications 1 à 8, dans lequel le composé hétérocyclique est l'un des composés 1 à 792 :

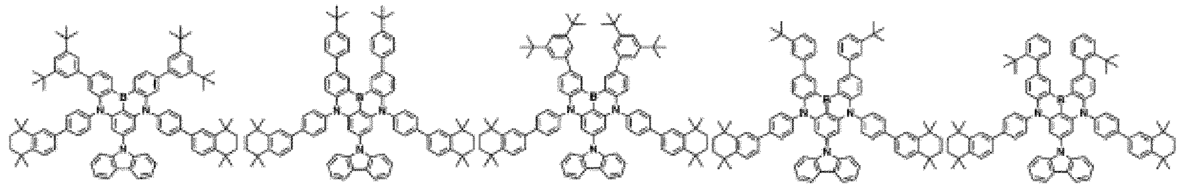

1 2 3 4 5

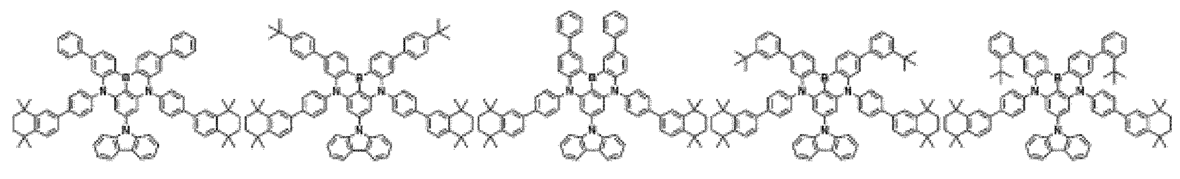

6 7 8 9 10

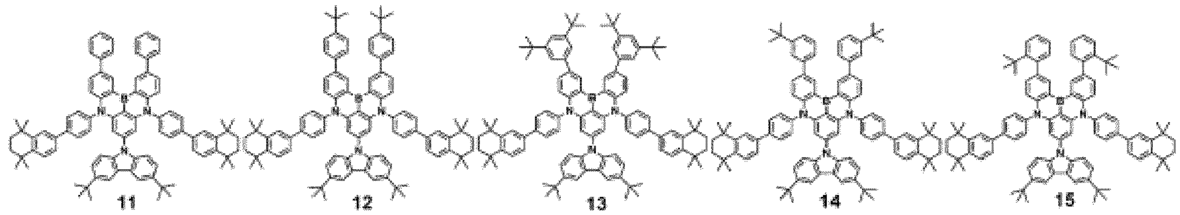

11 12 13 14 15

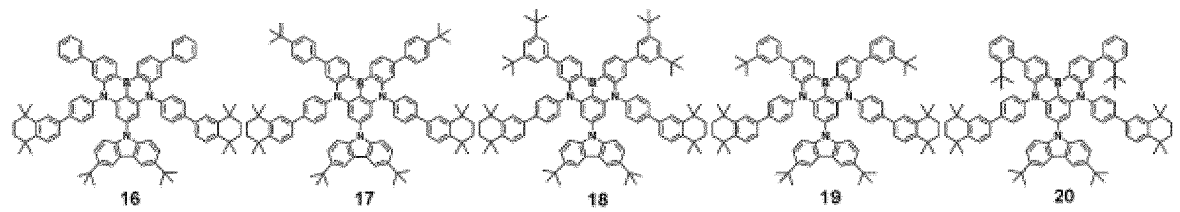

16 17 18 19 20

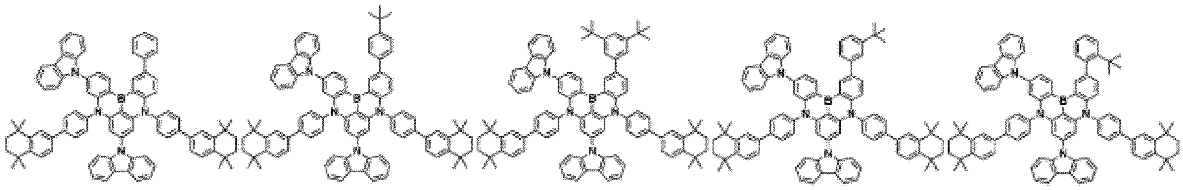

21 22 23 24 25

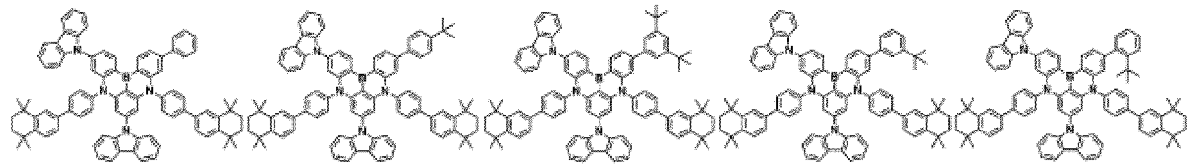

26 27 28 29 30

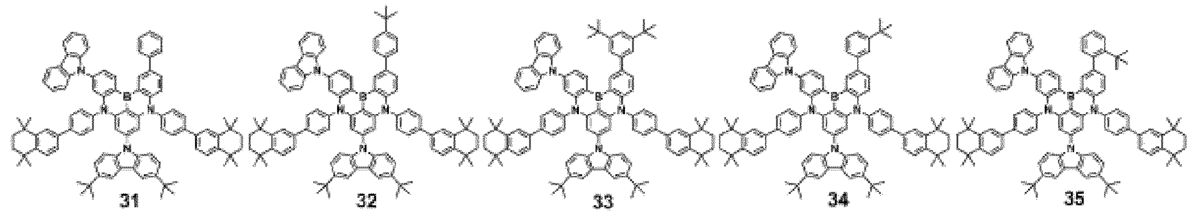

31 32 33 34 35

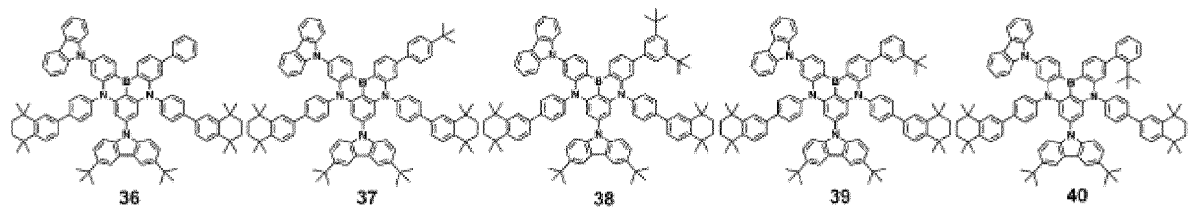

36 37 38 39 40

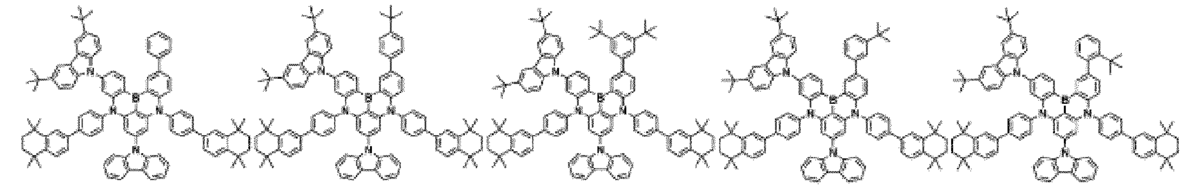

41 42 43 44 45

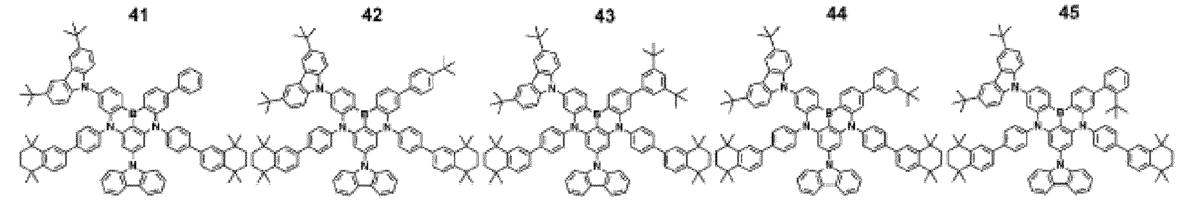

46 47 48 49 50

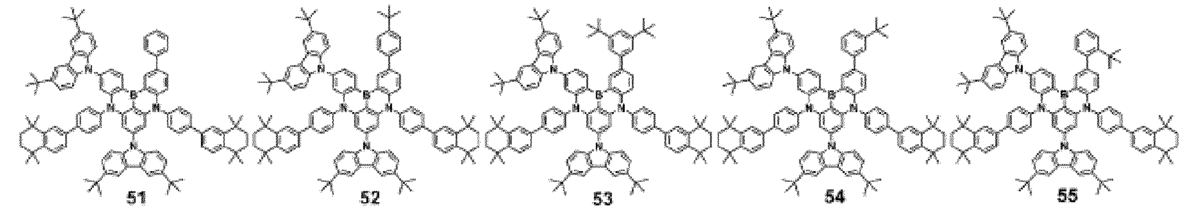

51 52 53 54 55

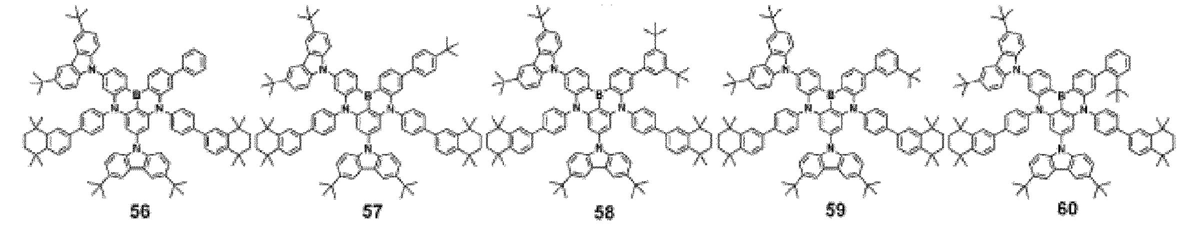

56 57 58 59 60

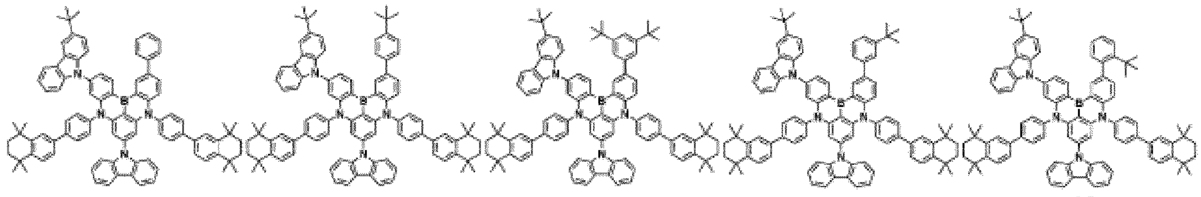

61 62 63 64 65

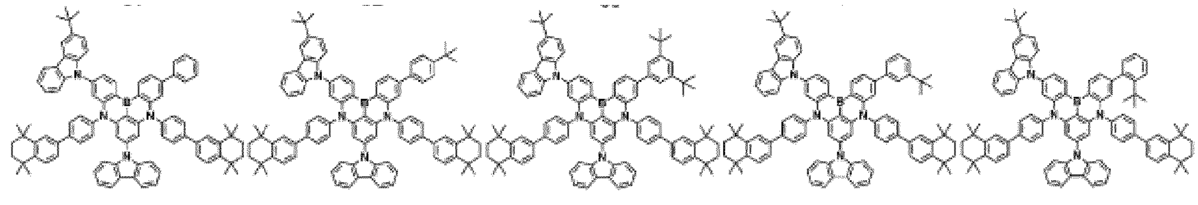

66 67 68 69 70

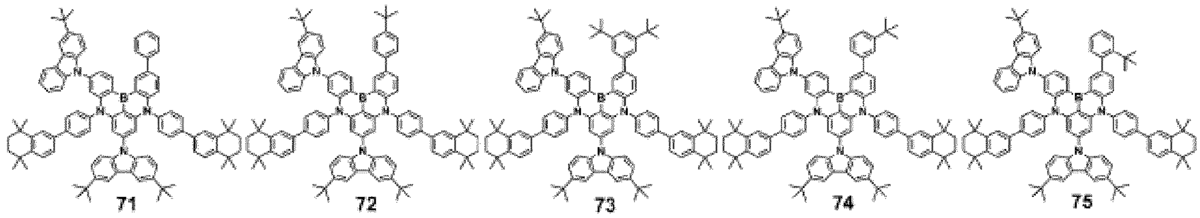

71 72 73 74 75

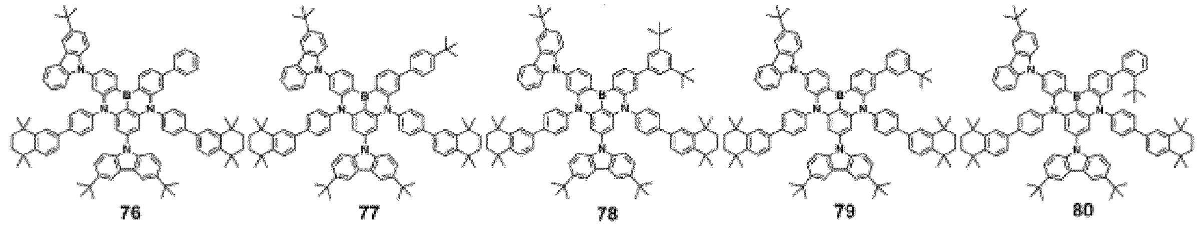

76 77 78 79 80

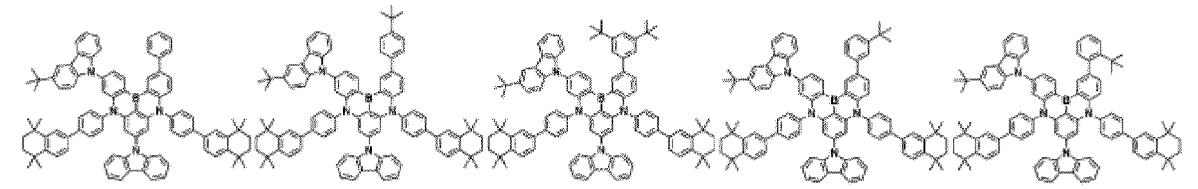

81 82 83 84 85

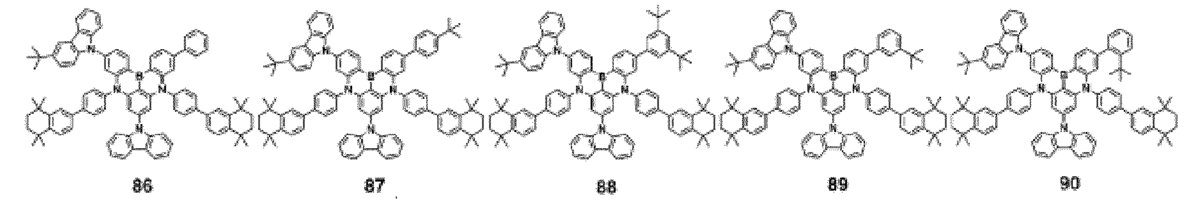

86 87 88 89 90

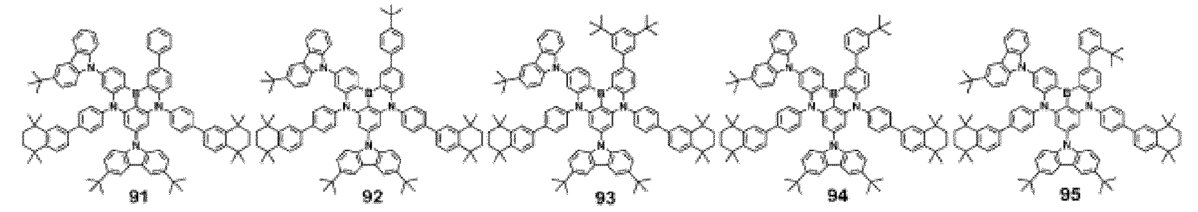

91 92 93 94 95

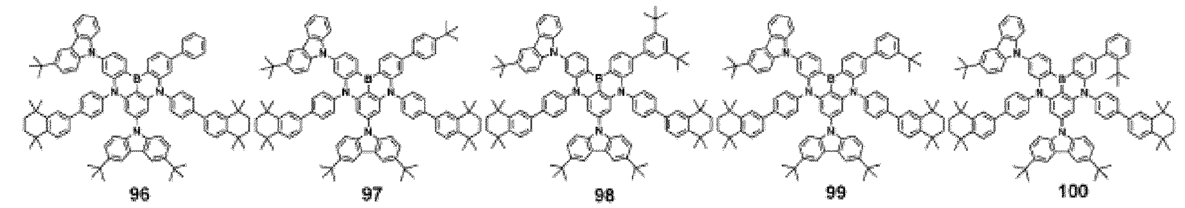

96 97 98 99 100

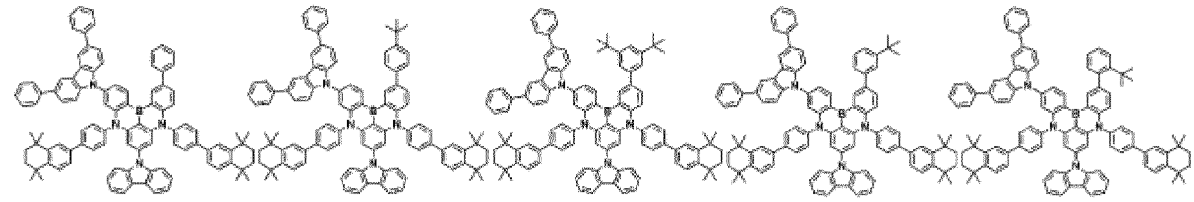

101 102 103 104 105

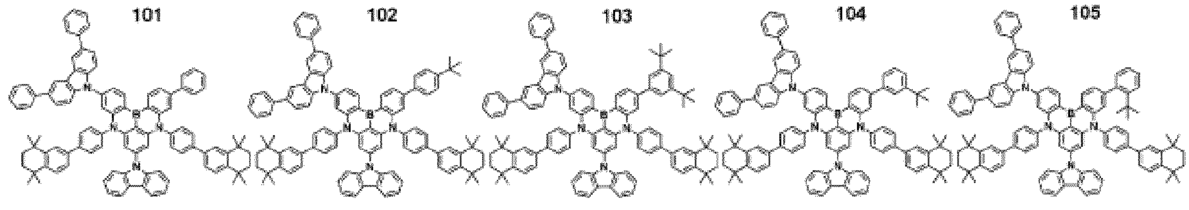

106 107 108 109 110

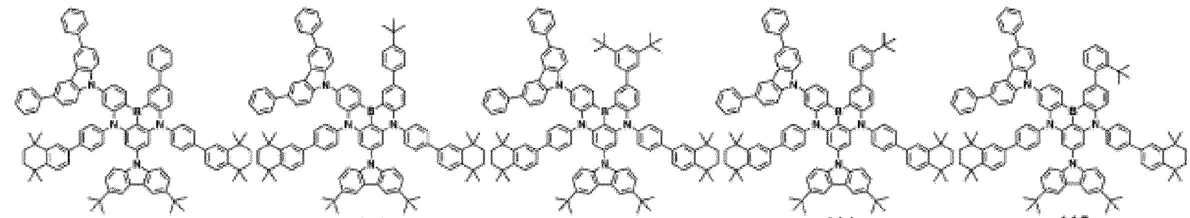

111 112 113 114 115

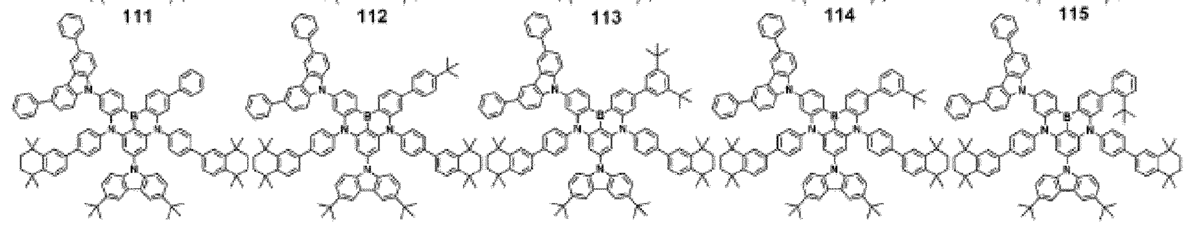

116 117 118 119 120

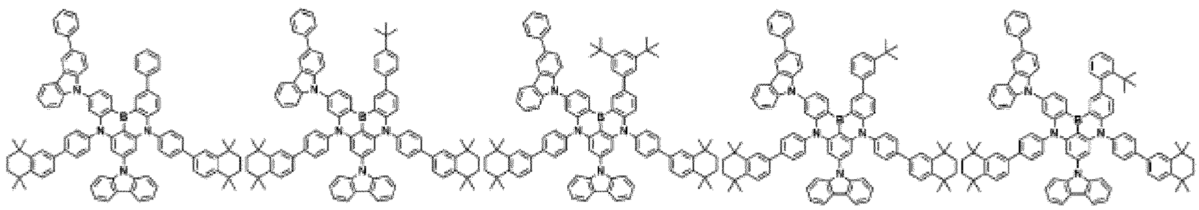

121 122 123 124 125

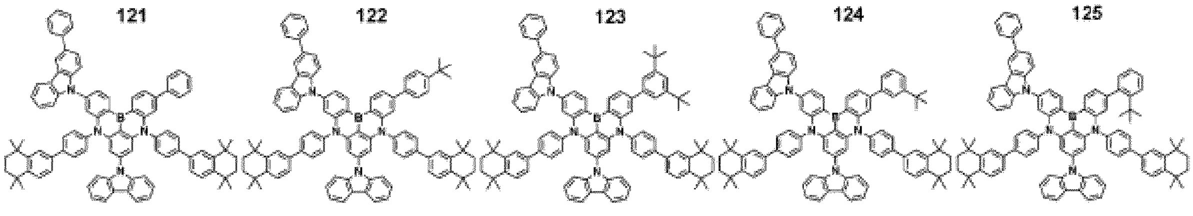

126 127 128 129 130

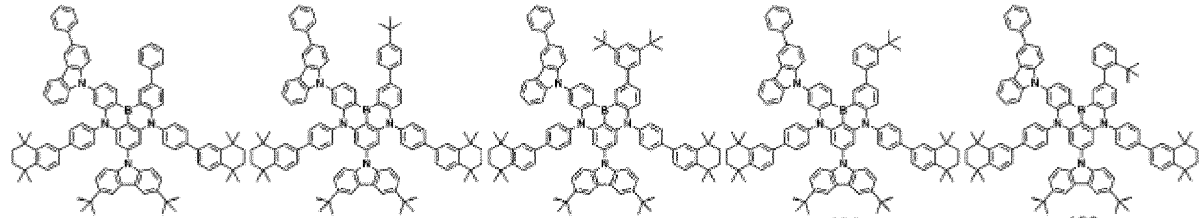

131 132 133 134 135

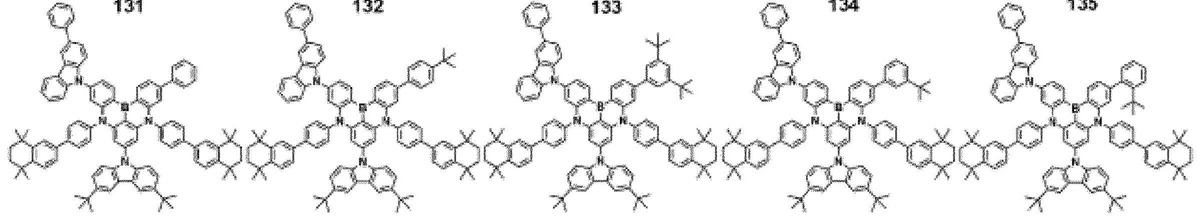

136 137 138 139 140

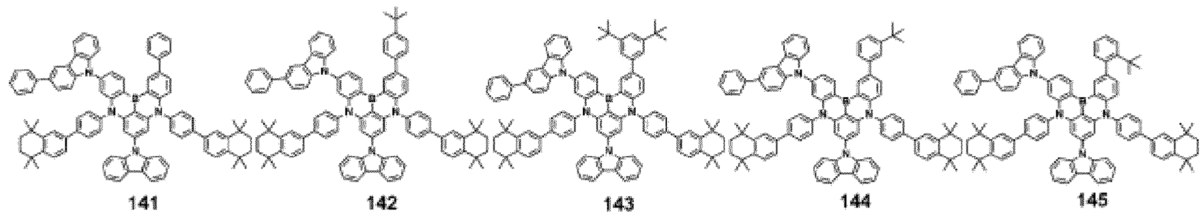

141 142 143 144 145

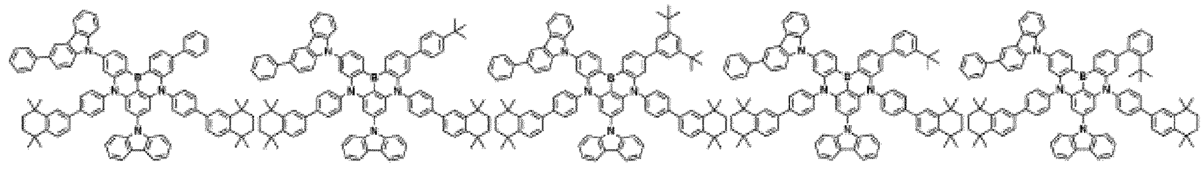

146 147 148 149 150

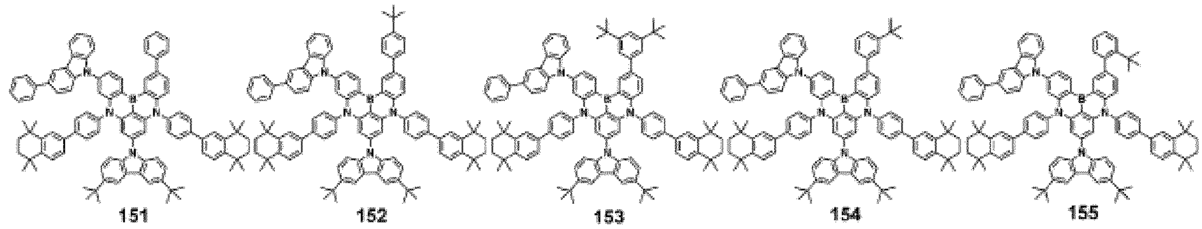

151 152 153 154 155

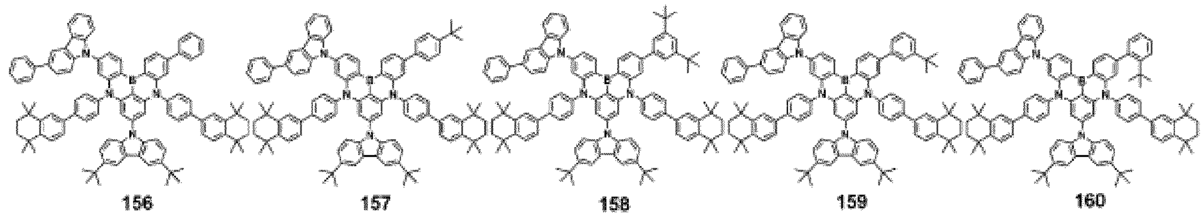

156 157 158 159 160

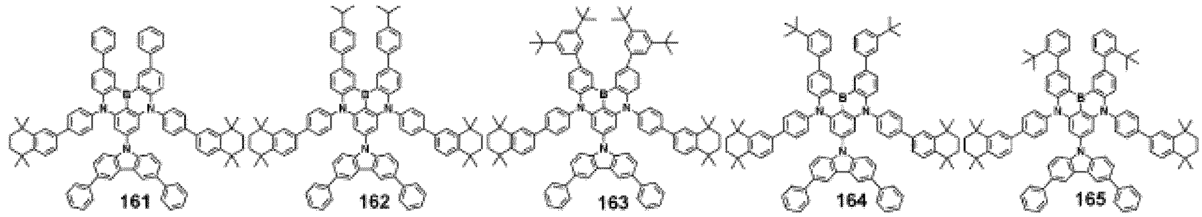

161 162 163 164 165

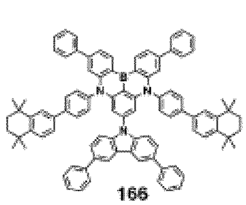
166
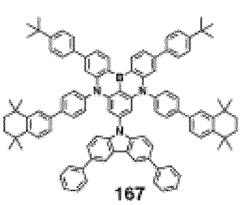
167
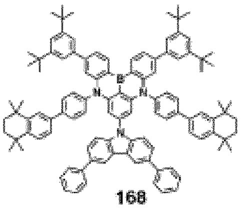
168
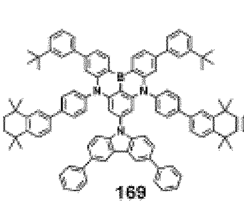
169
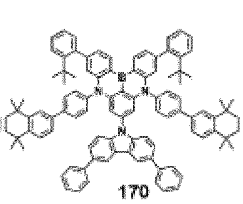
170

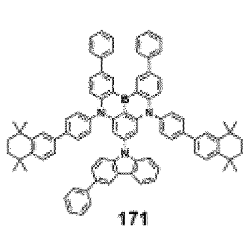
171
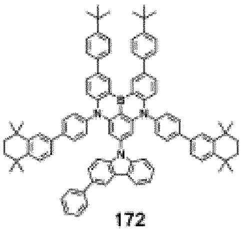
172
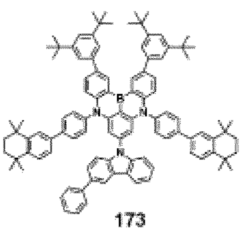
173
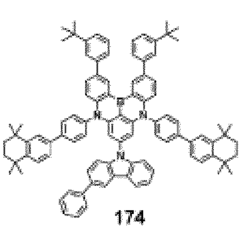
174
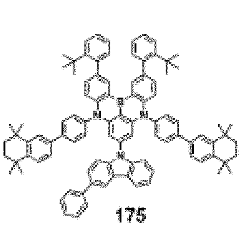
175

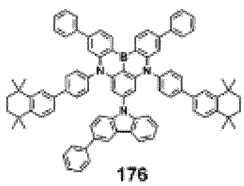
176
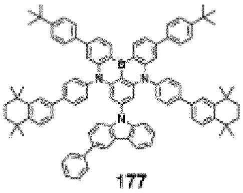
177
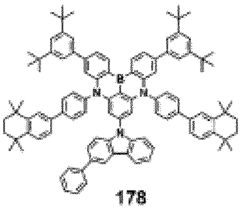
178
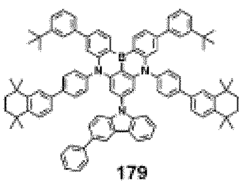
179
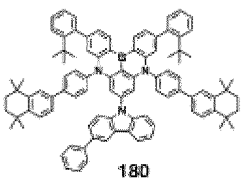
180

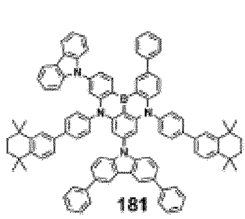
181
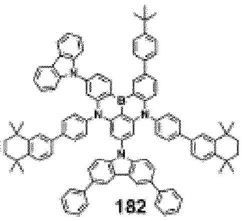
182
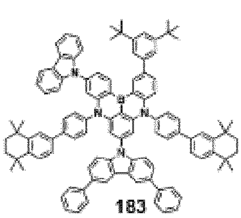
183
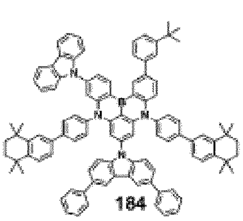
184
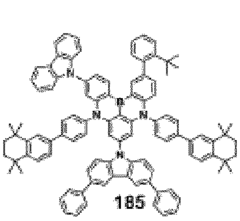
185

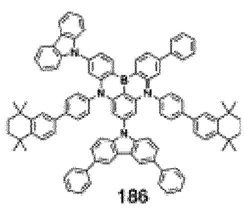
186
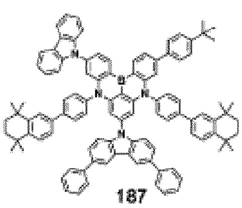
187
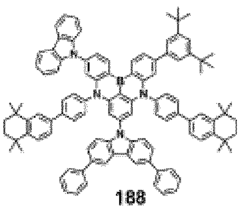
188
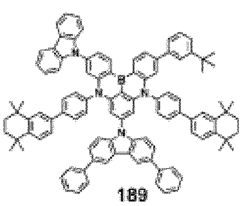
189
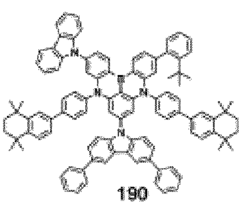
190

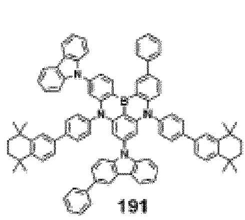
191
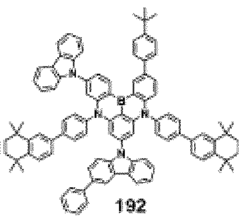
192
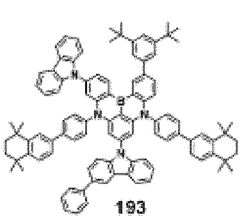
193
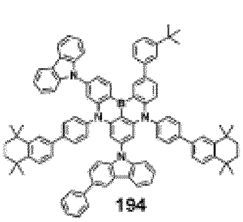
194
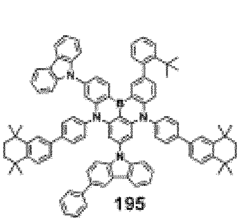
195

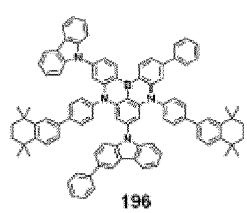
196
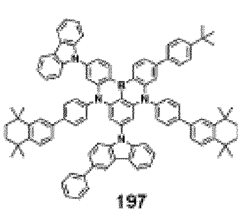
197
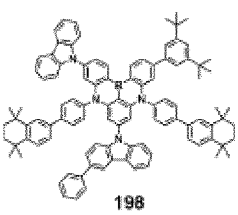
198
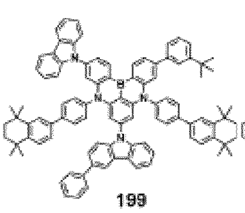
199
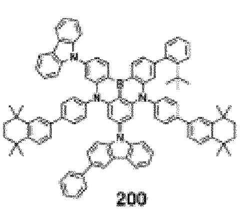
200

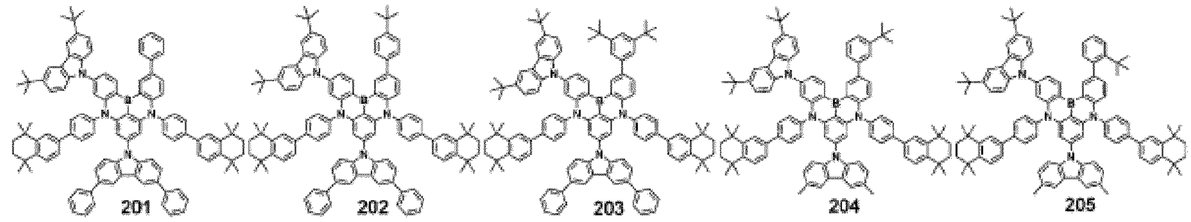

201 202 203 204 205

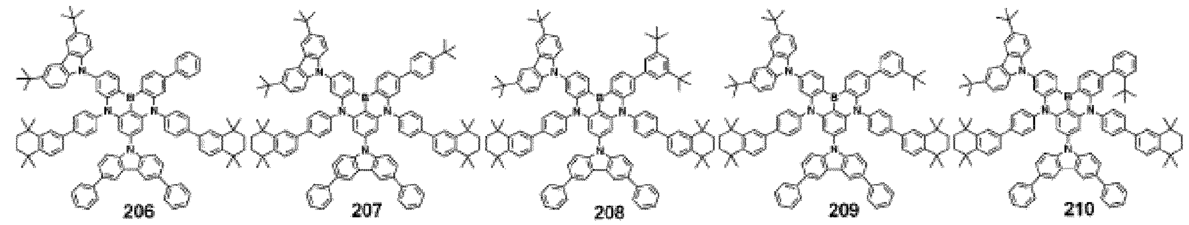

206 207 208 209 210

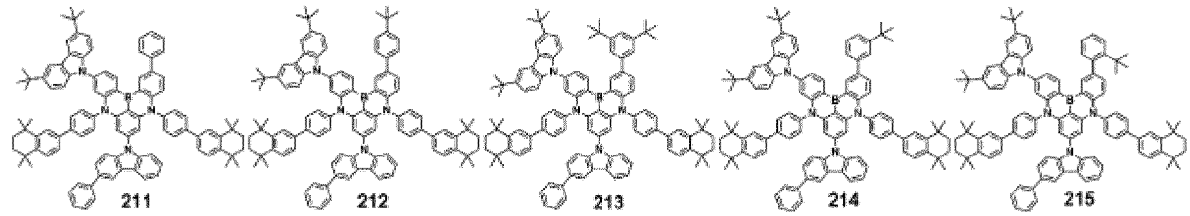

211 212 213 214 215

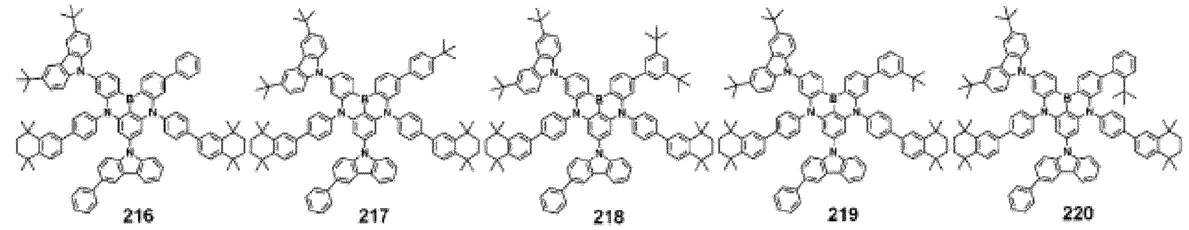

216 217 218 219 220

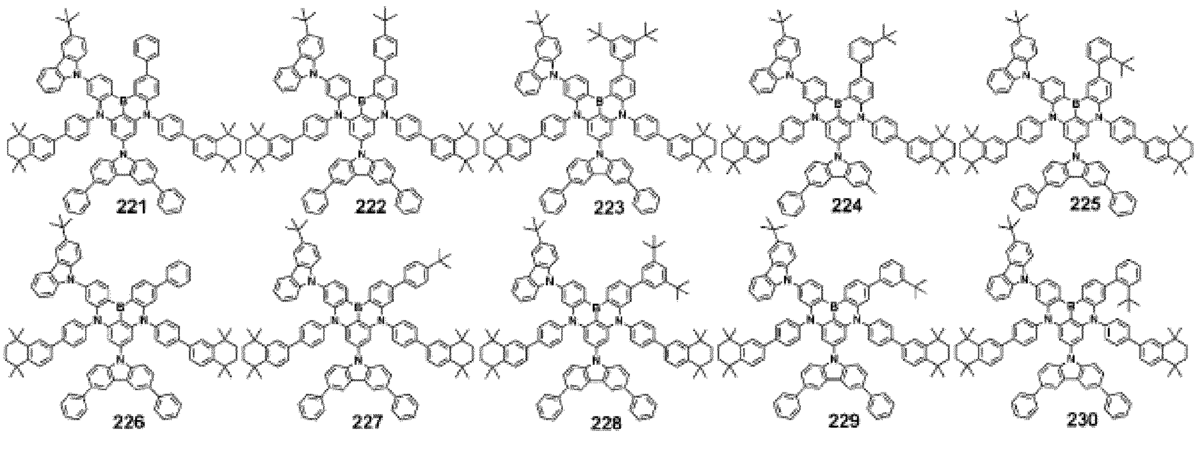

221 222 223 224 225

226 227 228 229 230

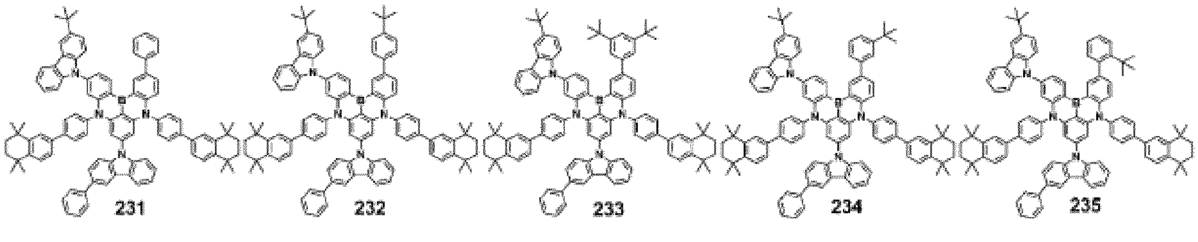

231 232 233 234 235

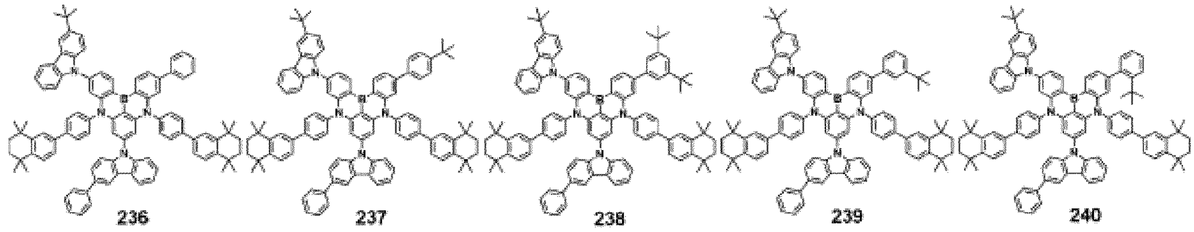

236 237 238 239 240

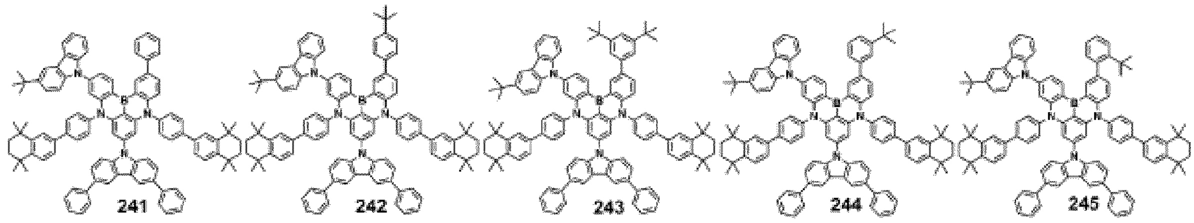

241 242 243 244 245

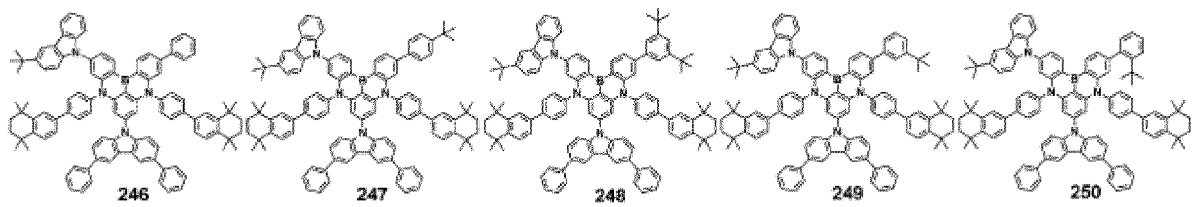

246 247 248 249 250

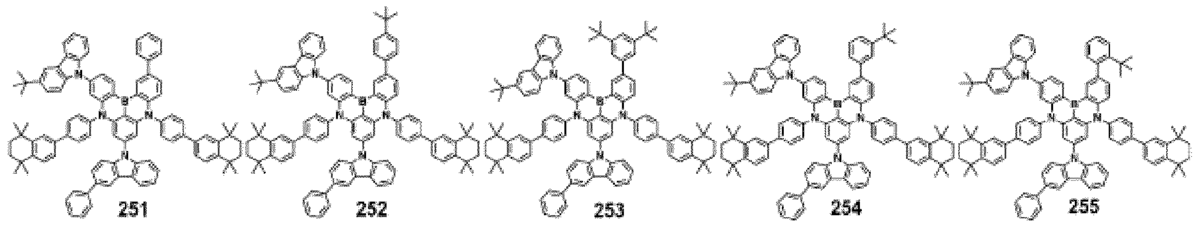

251 252 253 254 255

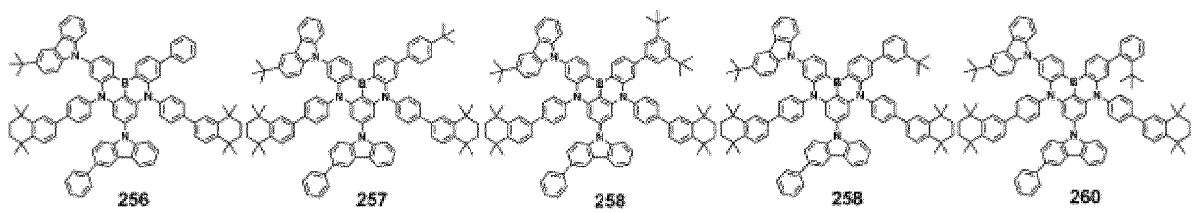

256 257 258 258 260

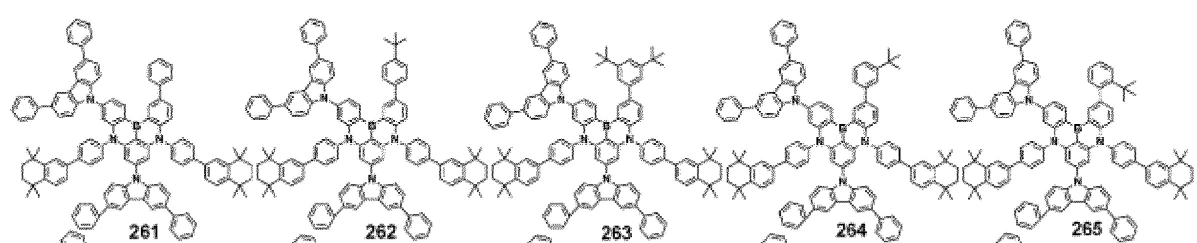

261 262 263 264 265

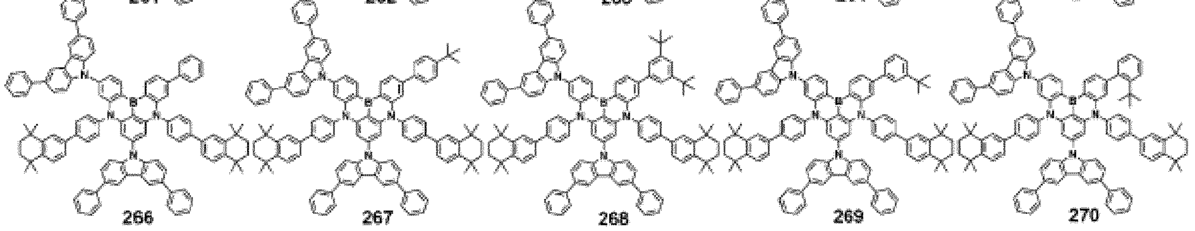

266 267 268 269 270

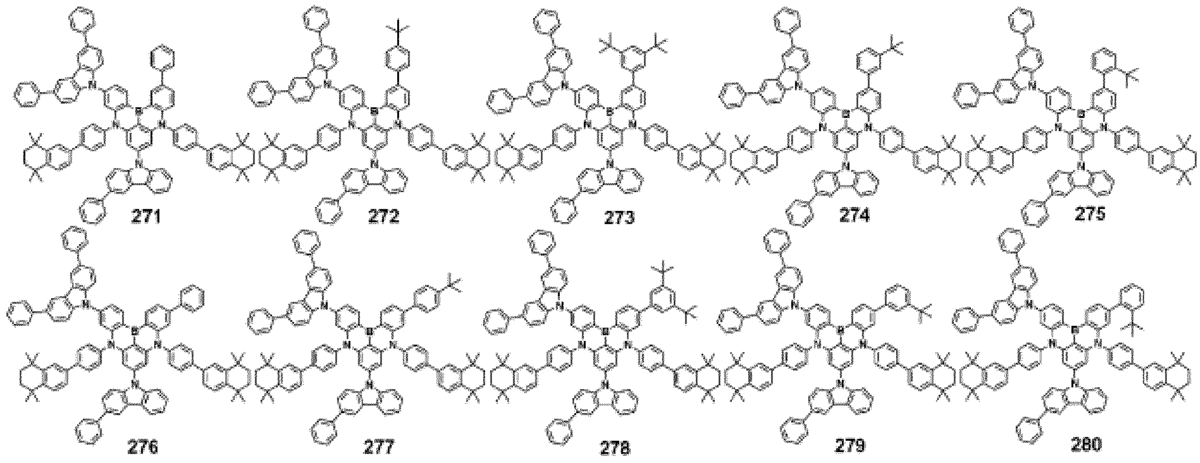

271 272 273 274 275

276 277 278 279 280

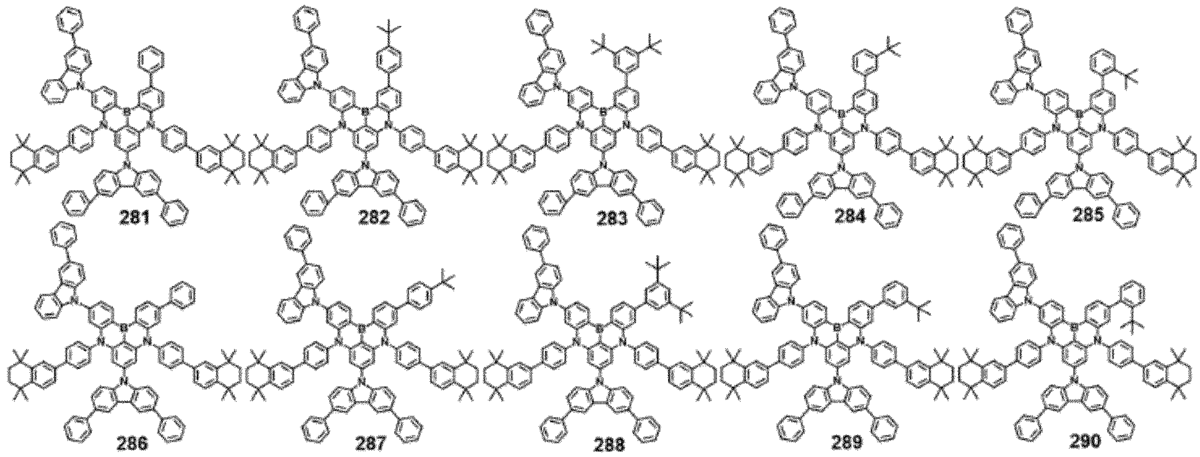

281 282 283 284 285

286 287 288 289 290

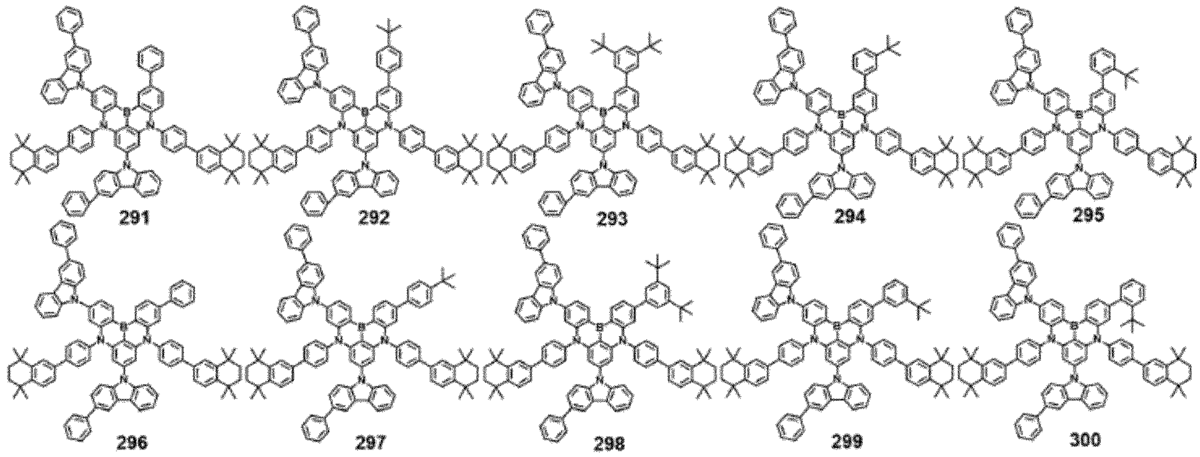

291 292 293 294 295

296 297 298 299 300

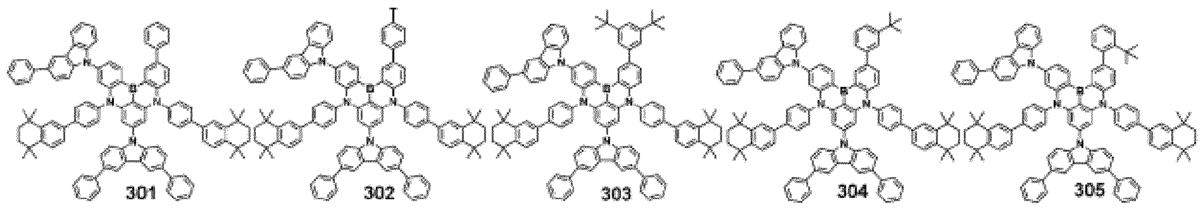

301 302 303 304 305

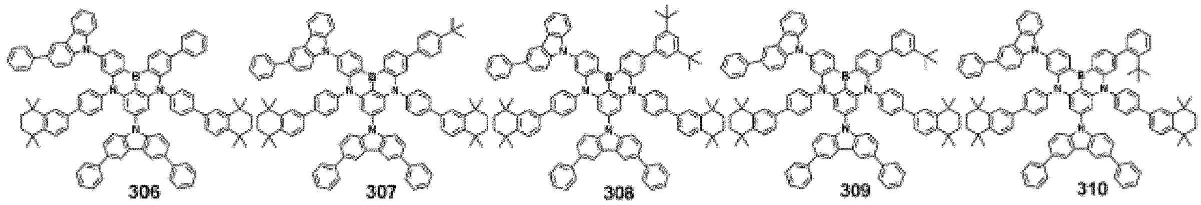

306 307 308 309 310

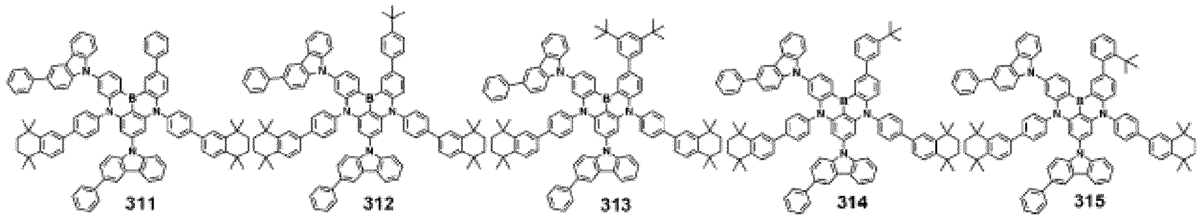

311 312 313 314 315

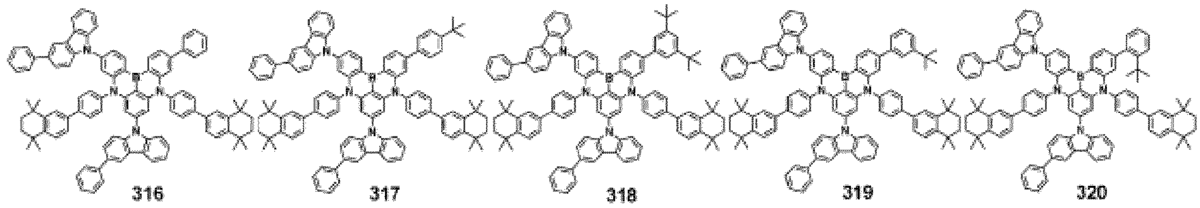

316 317 318 319 320

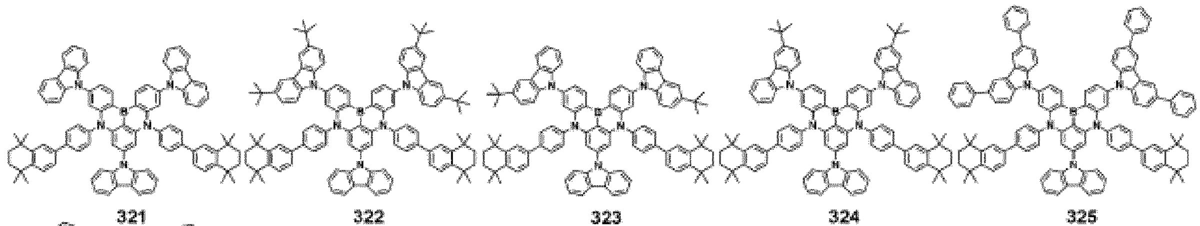

321 322 323 324 325

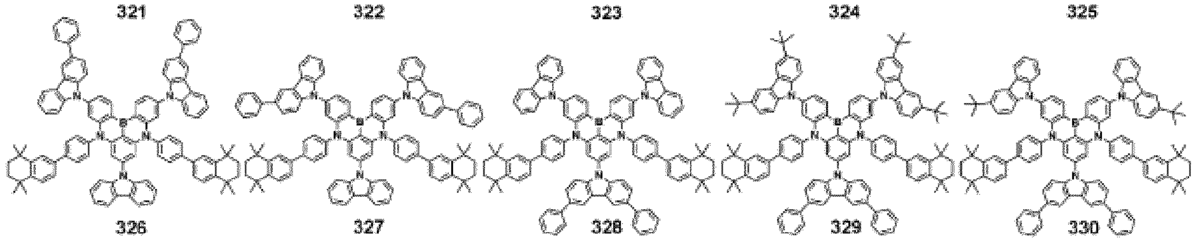

326 327 328 329 330

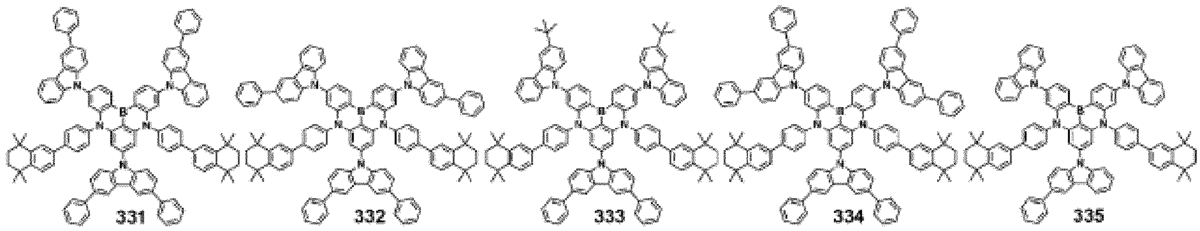

331 332 333 334 335

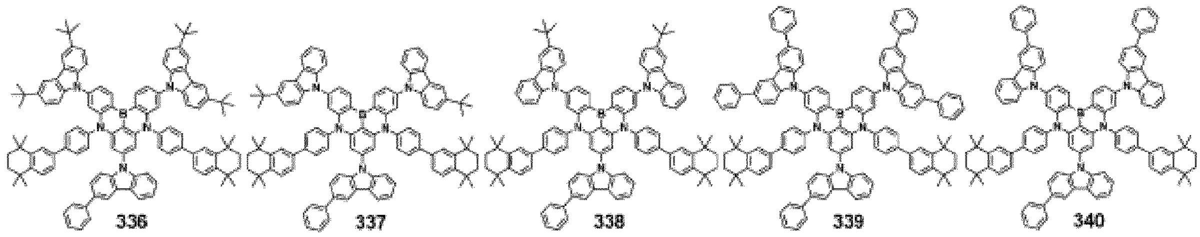

336 337 338 339 340

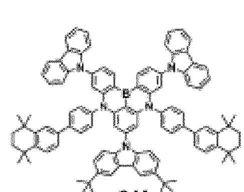
341
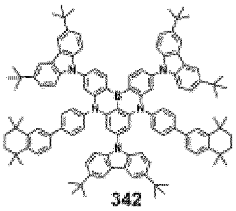
342
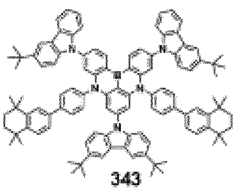
343
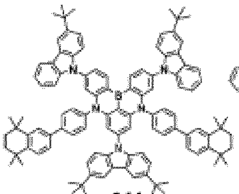
344
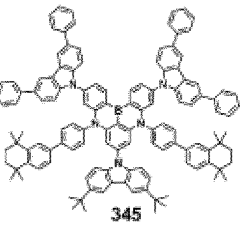
345

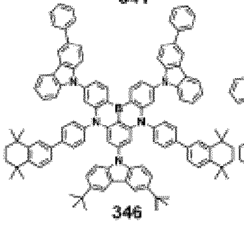
346
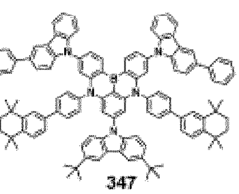
347
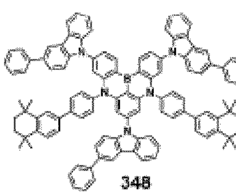
348
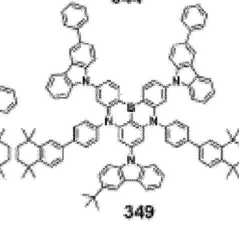
349
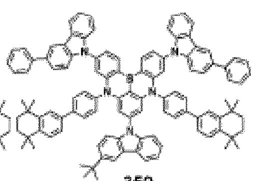
350

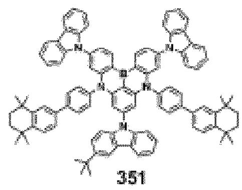
351
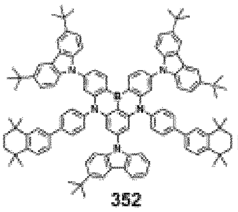
352
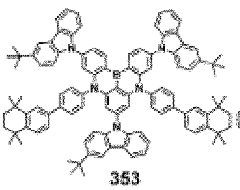
353
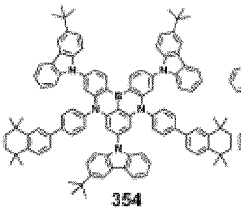
354
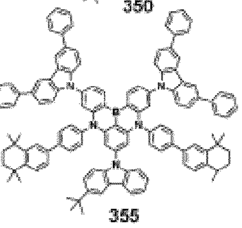
355

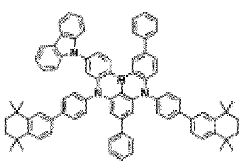
356
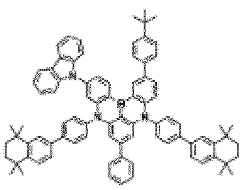
357
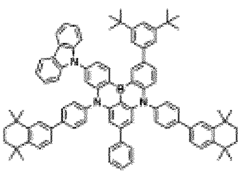
358
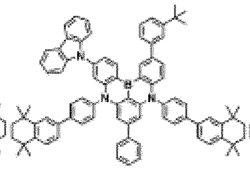
359
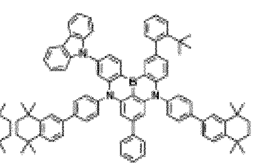
360

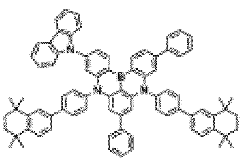
361
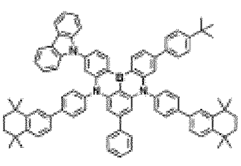
362
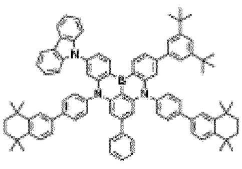
363
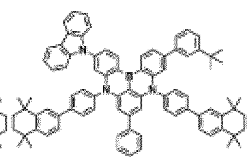
364
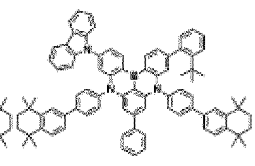
365

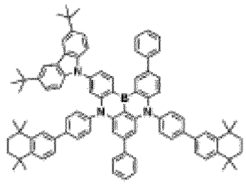
366
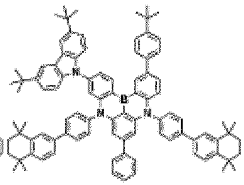
367
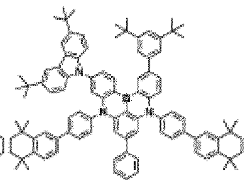
368
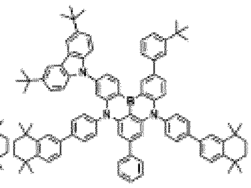
369
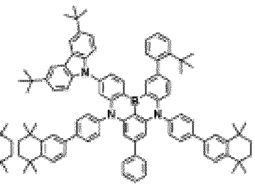
370

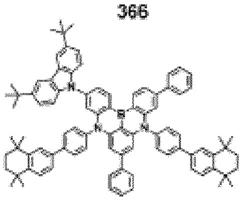
371
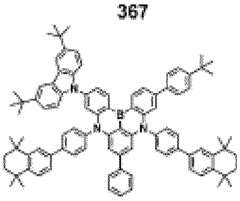
372
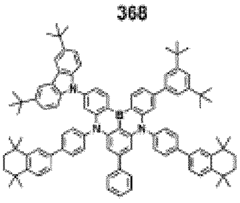
373
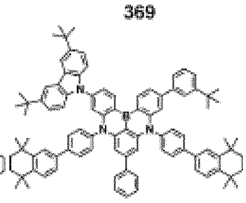
374
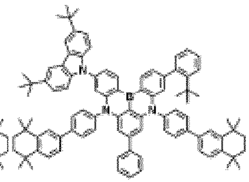
375

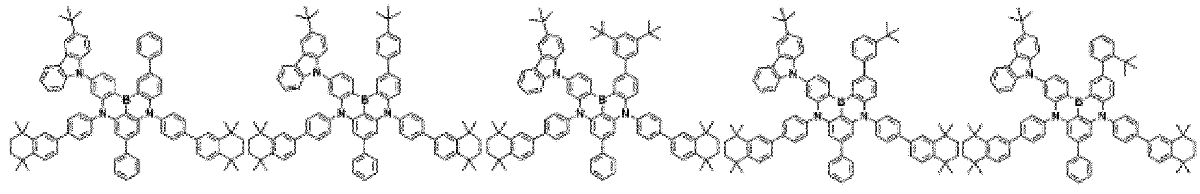

376 377 378 379 380

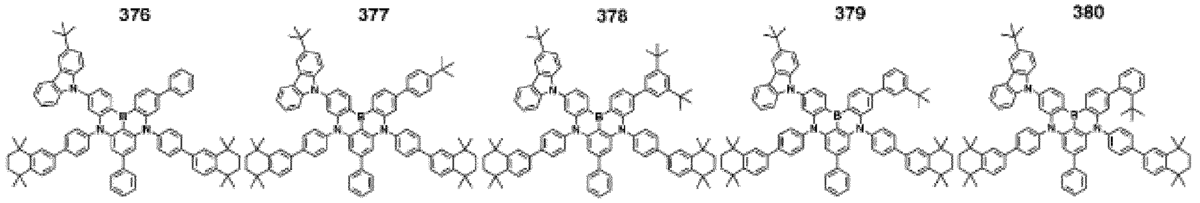

381 382 383 384 385

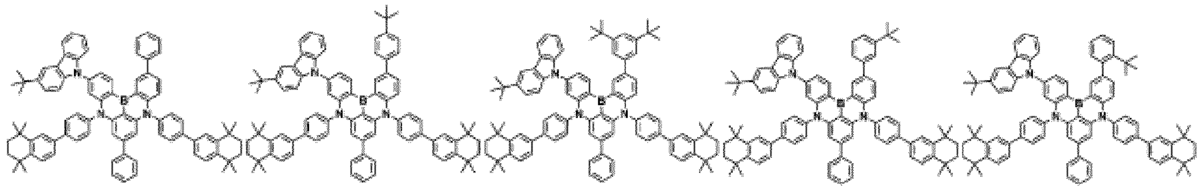

386 387 388 389 390

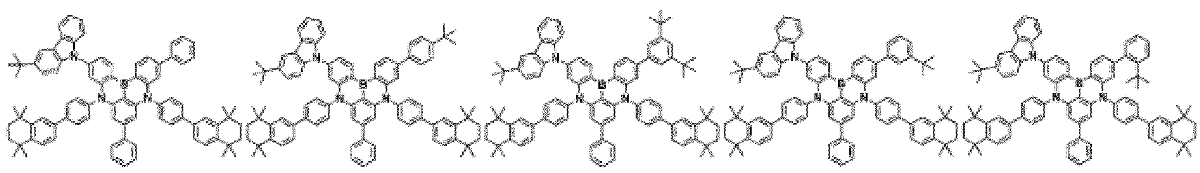

391 392 393 394 395

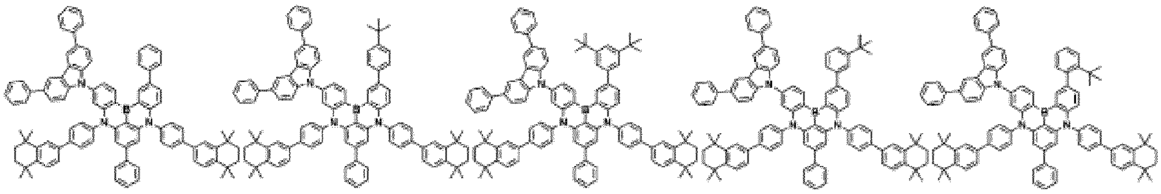

396 397 398 399 400

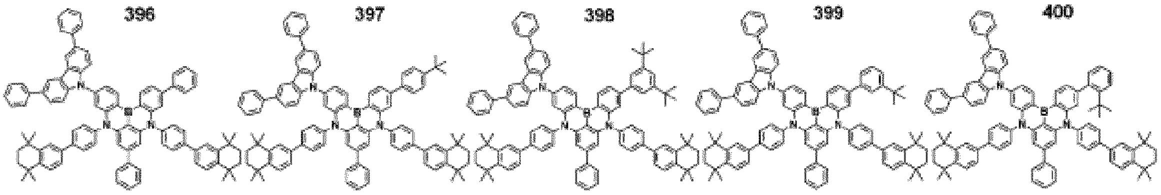

401 402 403 404 405

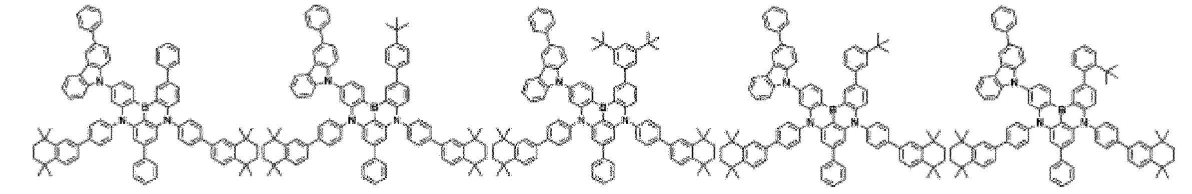

406 407 408 409 410

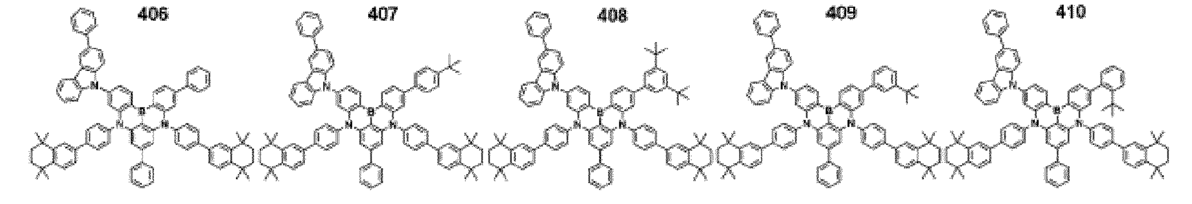

411 412 413 414 415

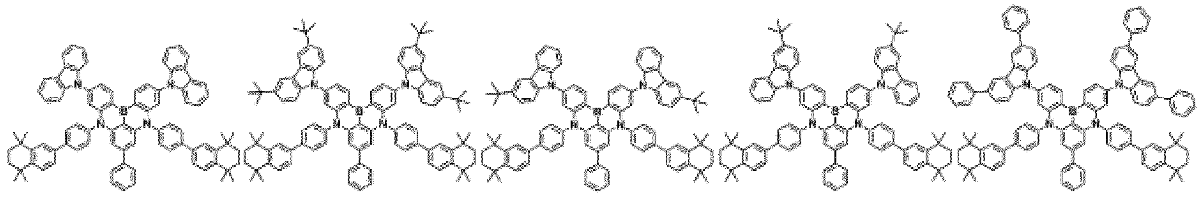

416 417 418 419 420

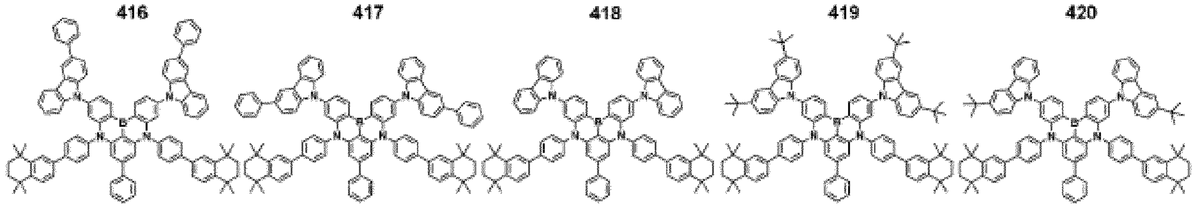

421 422 423 424 425

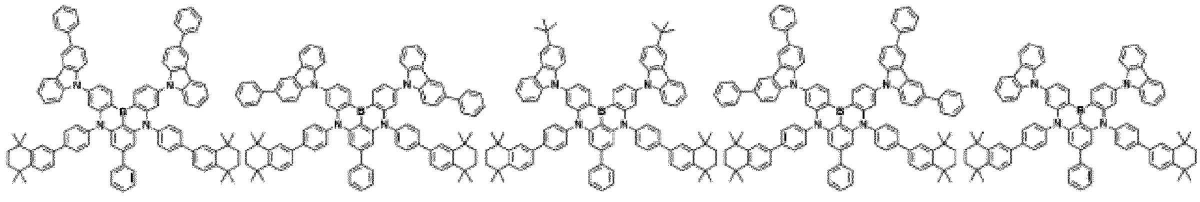

426 427 428 429 430

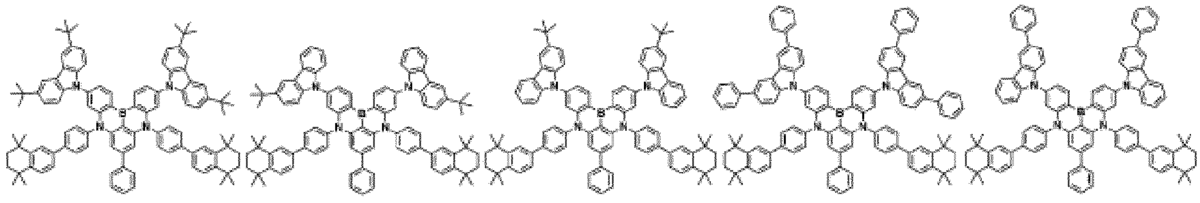

431 432 433 434 435

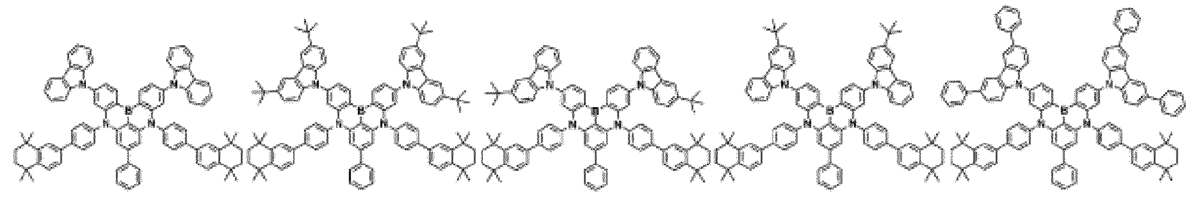

436 437 438 439 440

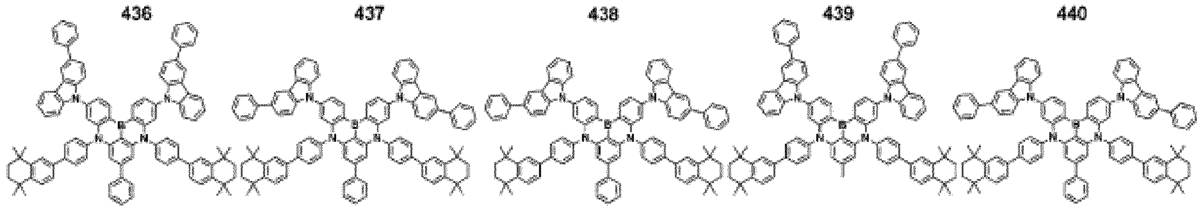

441 442 443 444 445

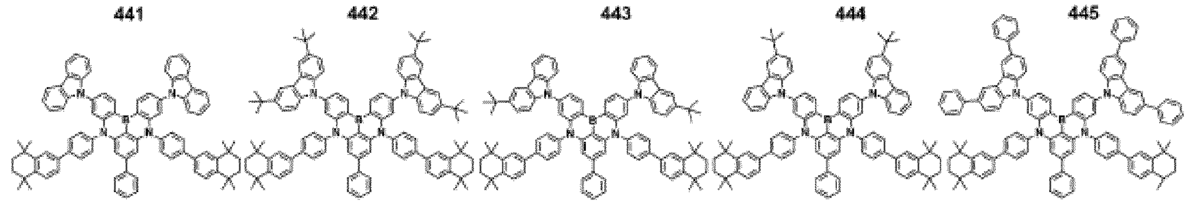

446 447 448 449 450

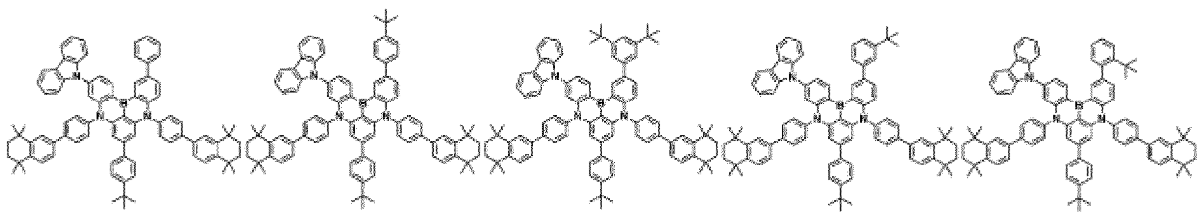

451 452 453 454 455

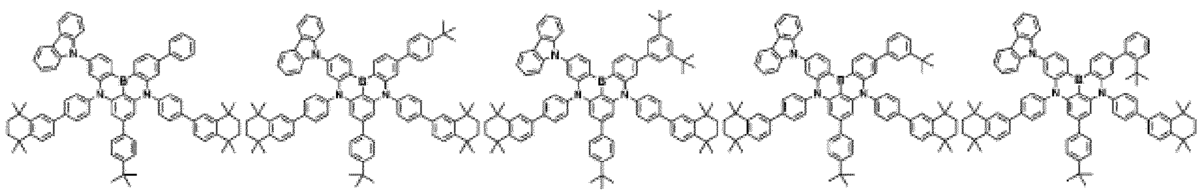

456 457 458 459 460

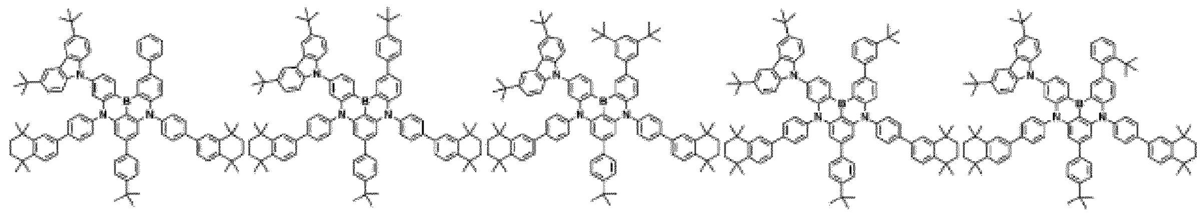

461 462 463 464 465

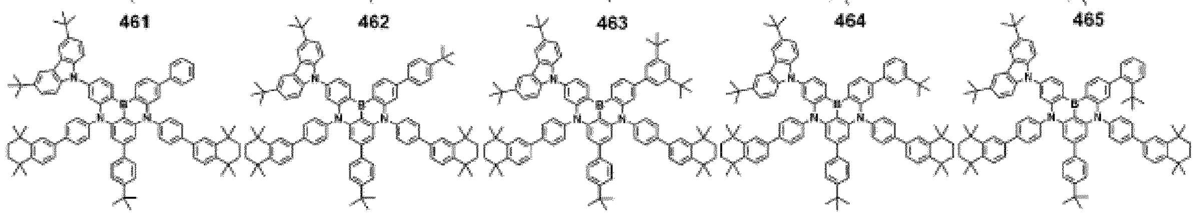

466 467 468 469 470

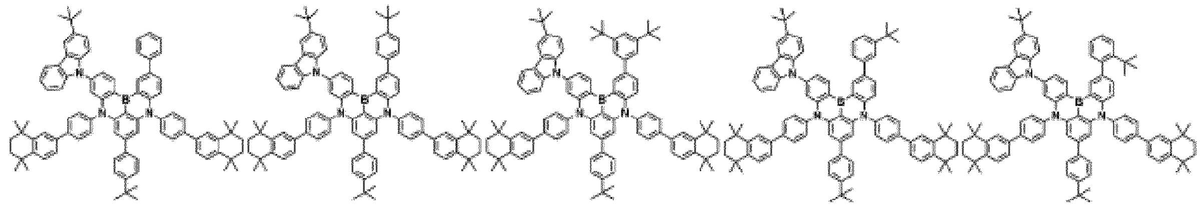

471 472 473 474 475

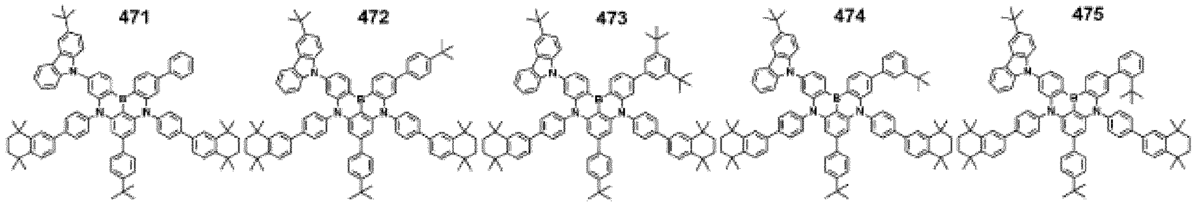

476 477 478 479 480

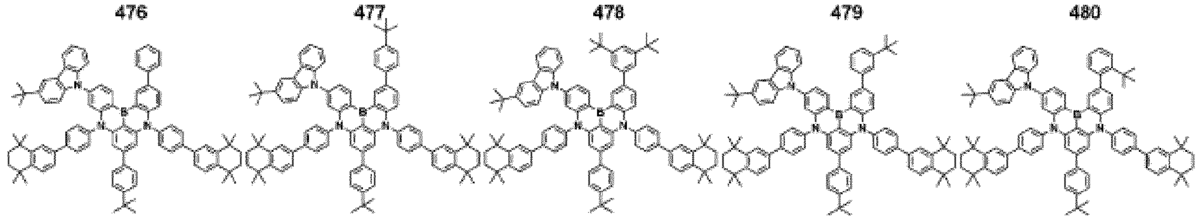

481 482 483 484 485

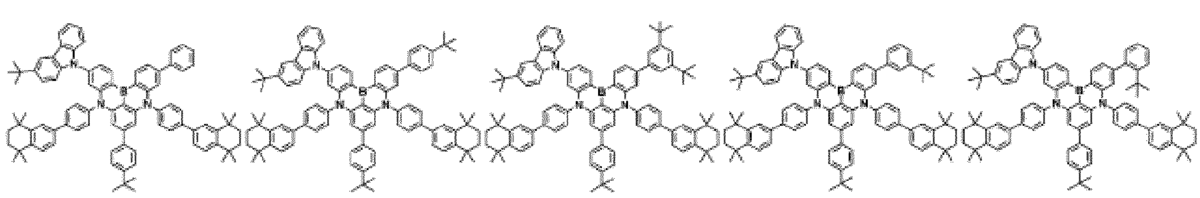

486 487 488 489 490

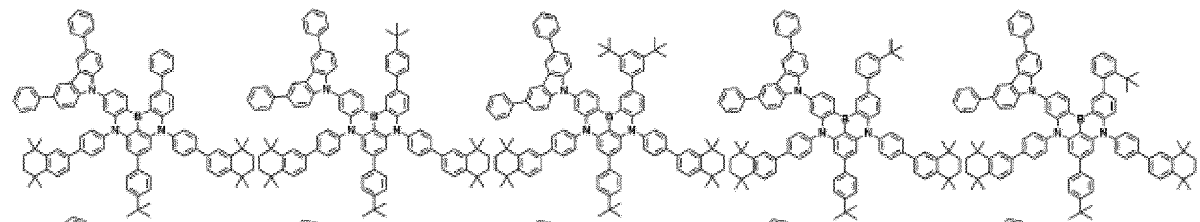

491 492 493 494 495

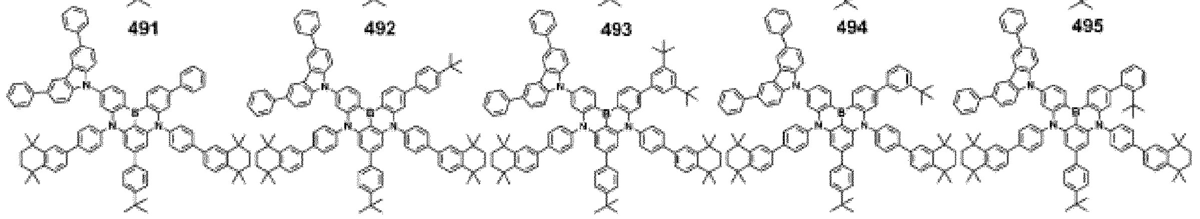

496 497 498 499 500

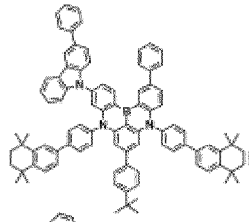
501

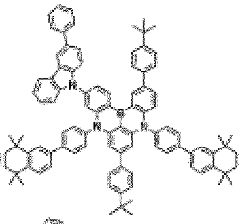
502

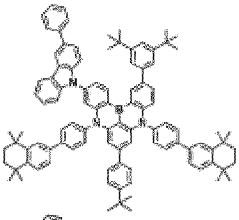
503

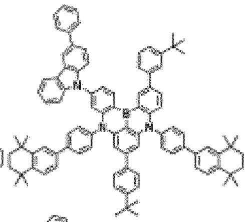
504

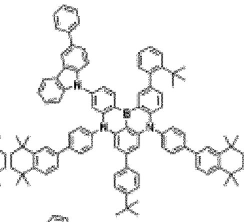
505

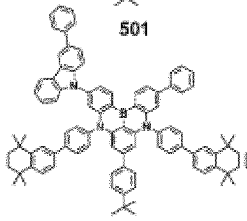
506

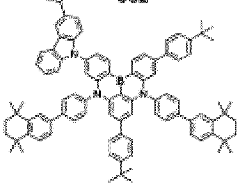
507

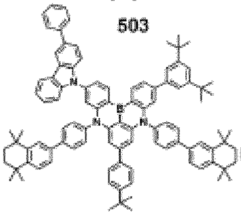
508

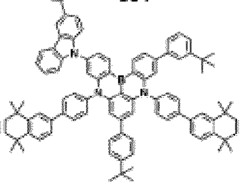
509

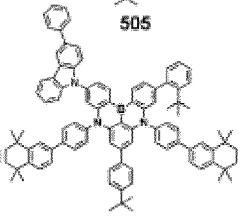
510

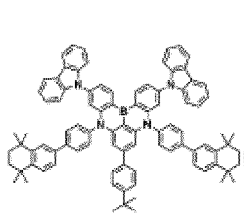
511

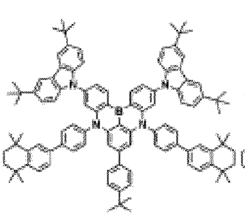
512

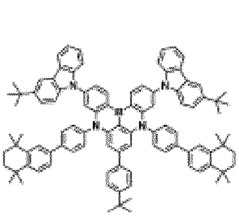
513

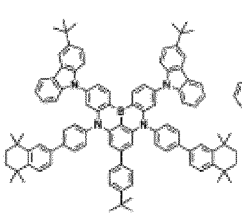
514

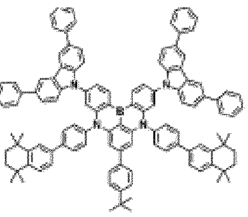
515

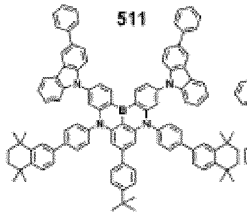
516

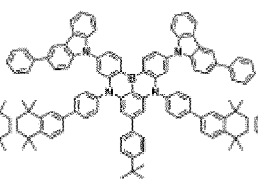
517

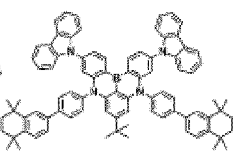
518

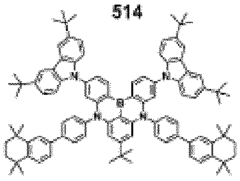
519

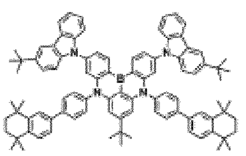
520

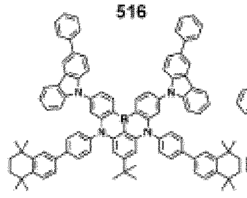
521

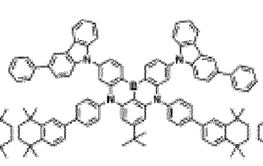
521

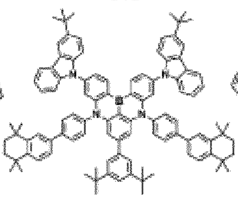
523

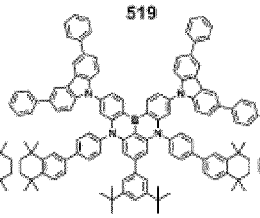
524

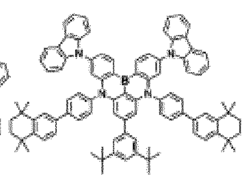
525

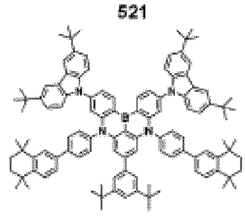
526

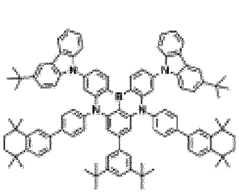
527

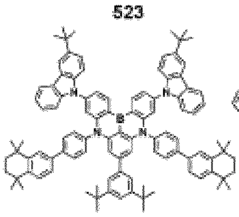
528

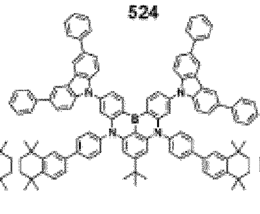
529

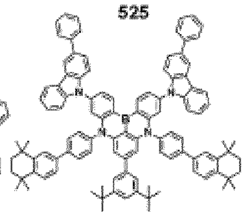
530

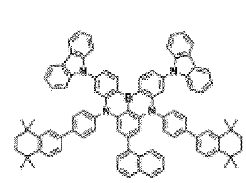
531

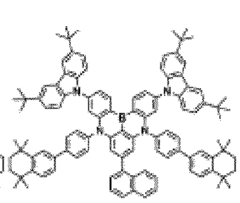
532

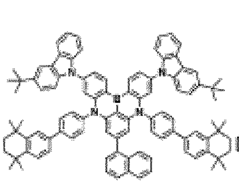
533

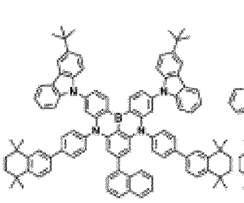
534

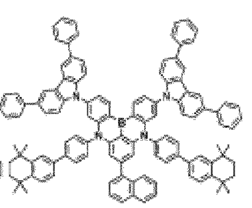
535

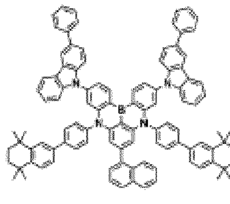
536
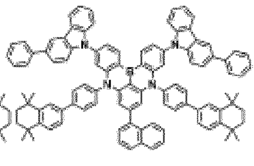
537
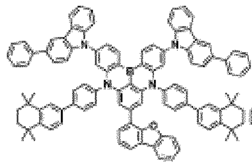
538
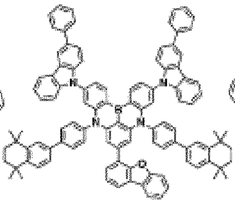
539
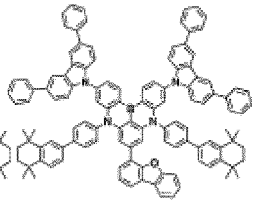
540

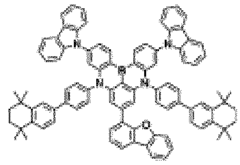
541
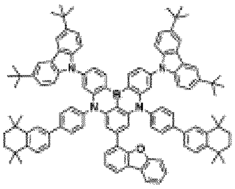
542
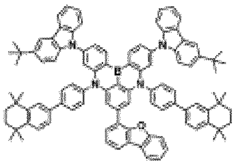
543
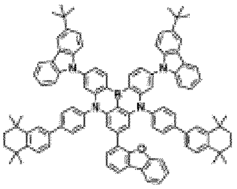
544
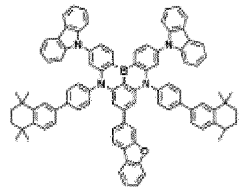
545

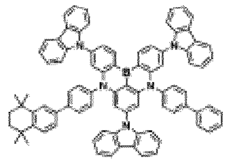
546
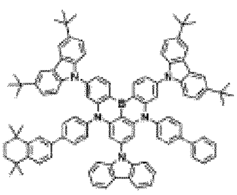
547
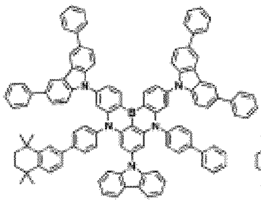
548
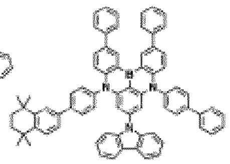
549
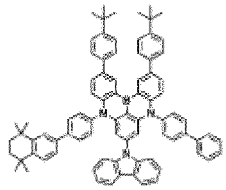
550

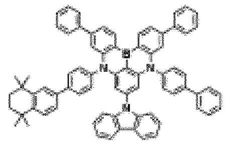
551
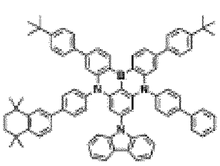
552
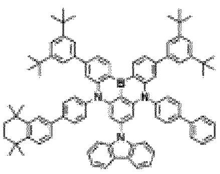
553
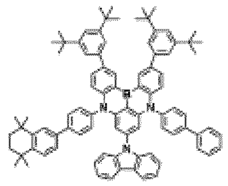
554
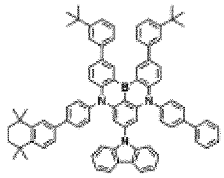
555

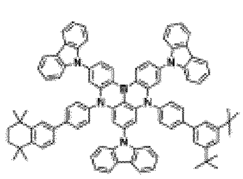
556
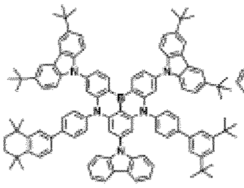
557
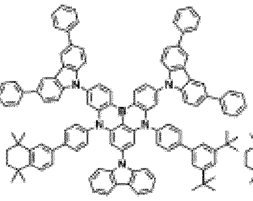
558
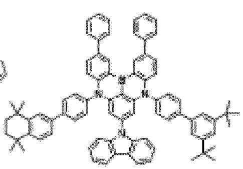
559
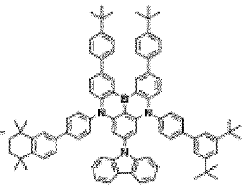
560

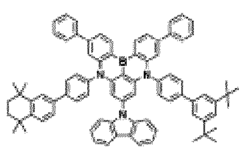
561
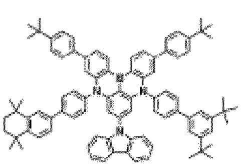
562
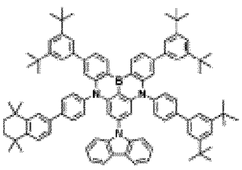
563
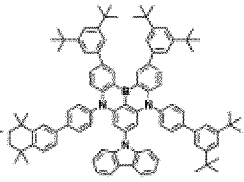
564
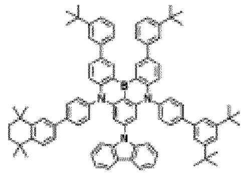
565

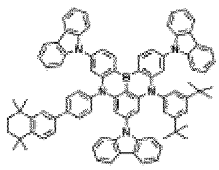
566

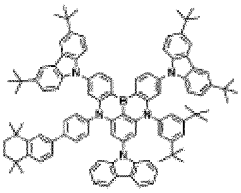
567

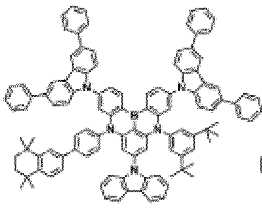
568

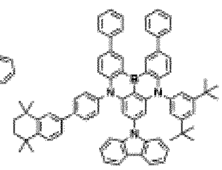
569

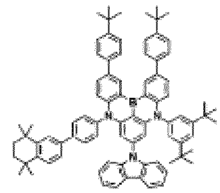
570

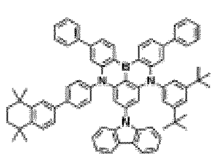
571

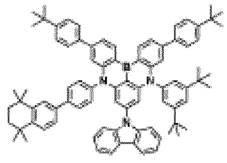
572

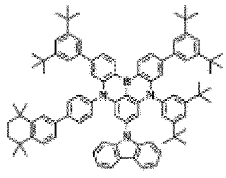
573

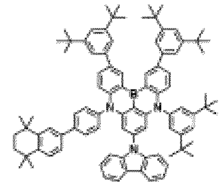
574

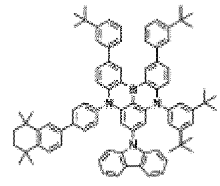
575

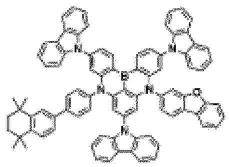
576

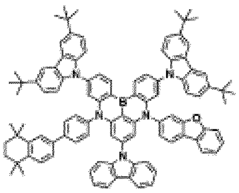
577

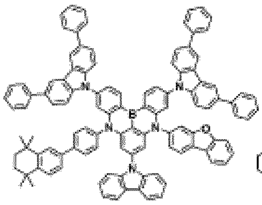
578

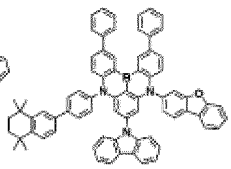
579

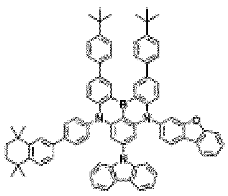
580

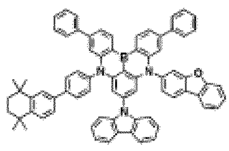
581

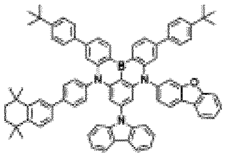
582

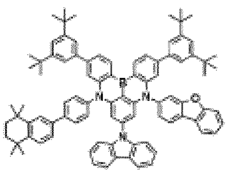
583

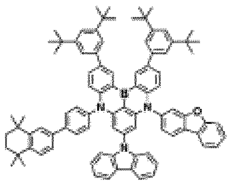
584

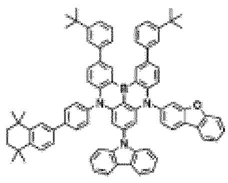
585

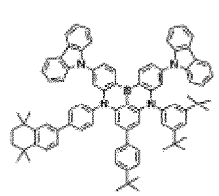
586

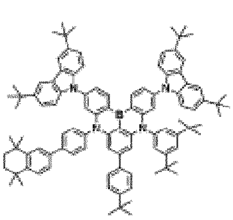
587

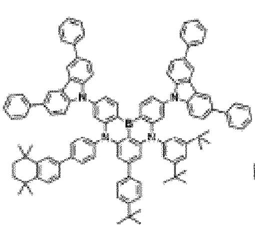
588

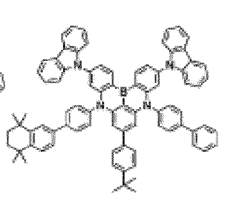
589

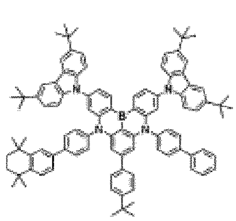
590

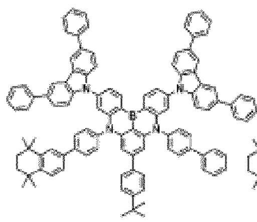
591

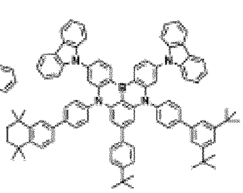
592

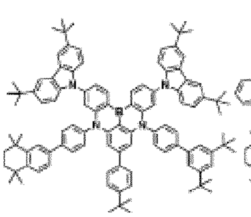
593

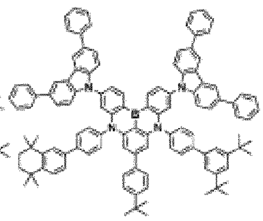
594

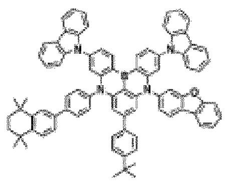
595

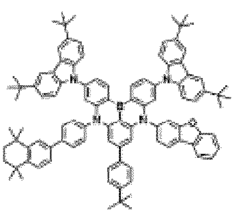
596

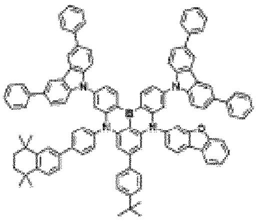
597

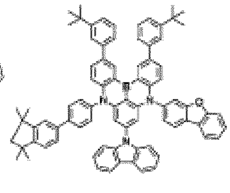
598

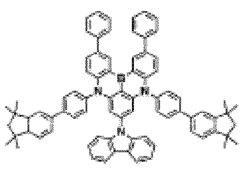
599

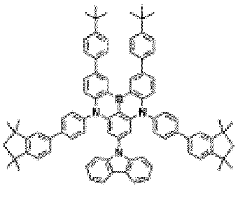
600

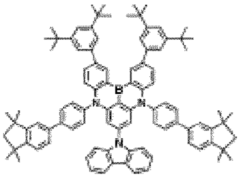
601

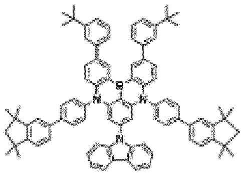
602

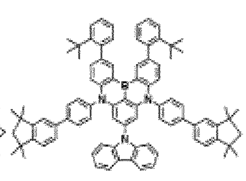
603

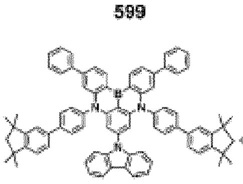
604

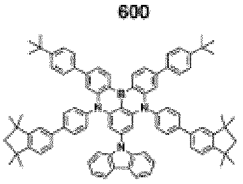
605

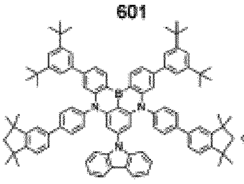
606

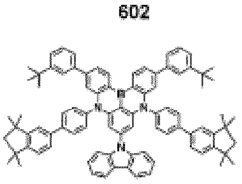
607

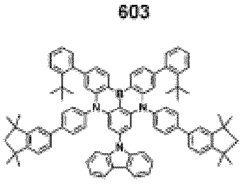
608

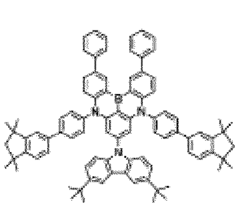
609

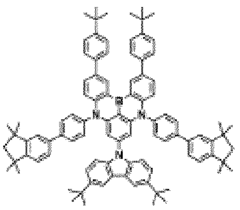
610

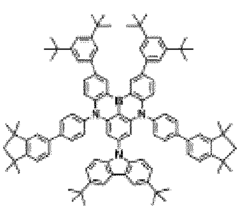
611

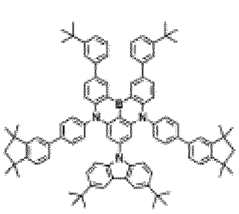
612

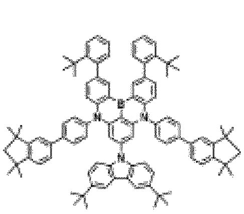
613

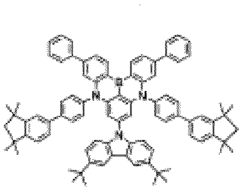
614

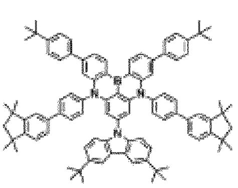
615

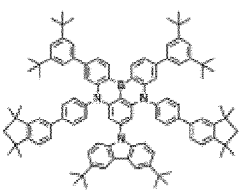
616

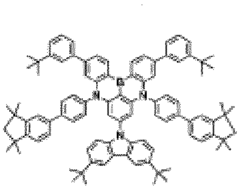
617

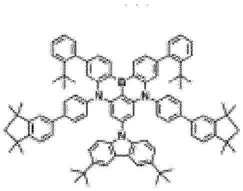
618

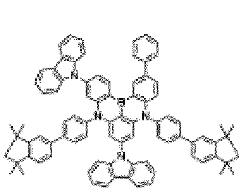
619

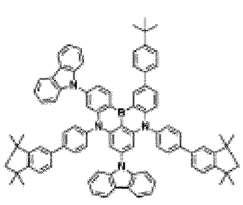
620

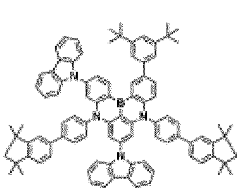
621

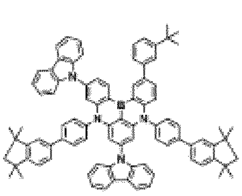
622

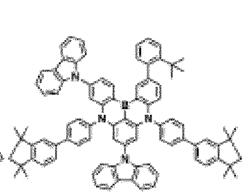
623

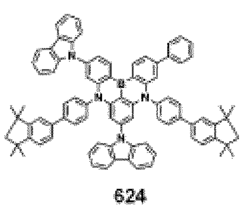
624

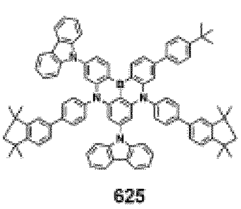
625

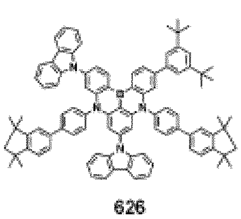
626

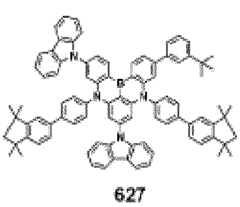
627

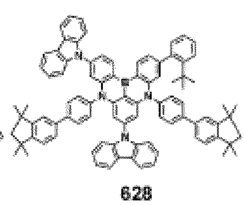
628

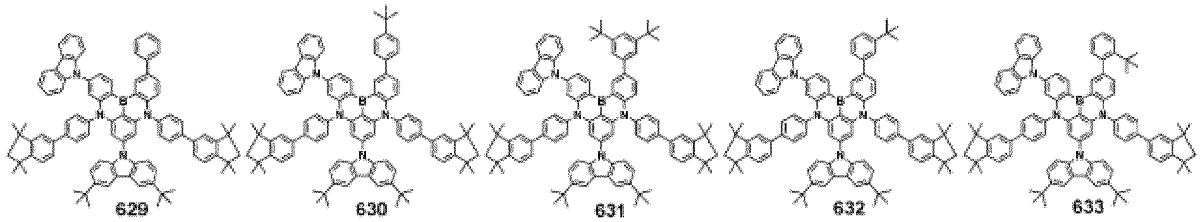

629 630 631 632 633

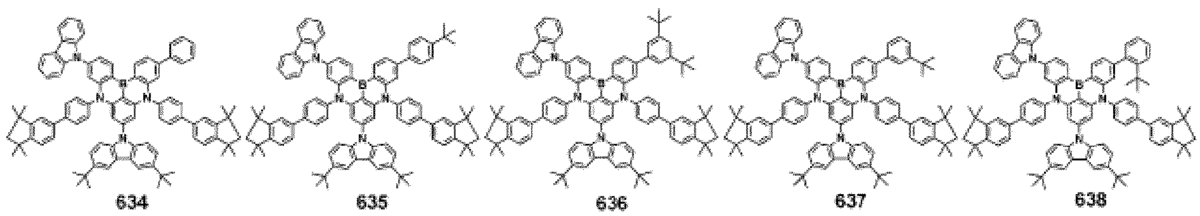

634 635 636 637 638

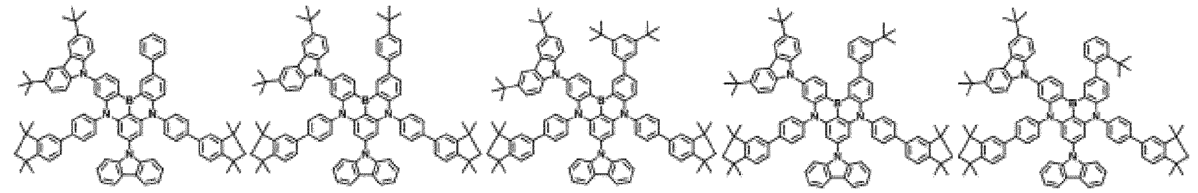

639 640 641 642 643

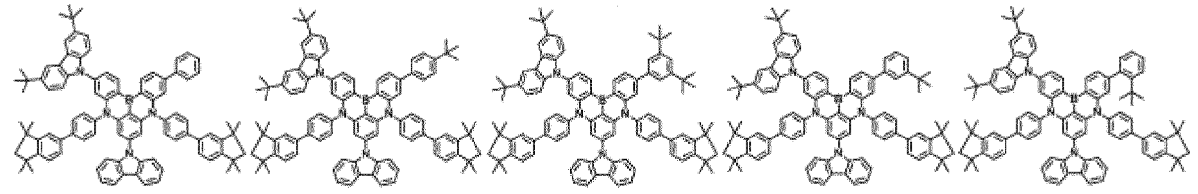

644 645 646 647 648

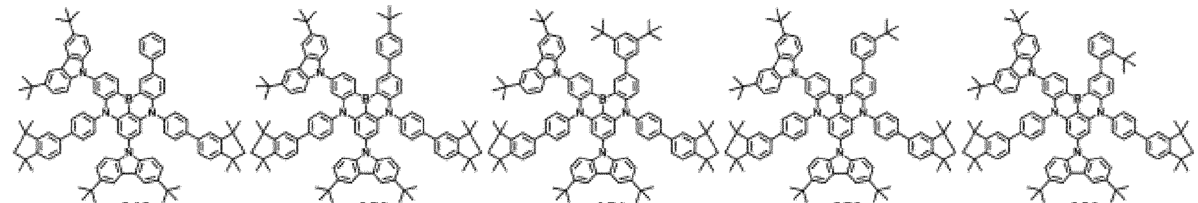

649 650 651 652 653

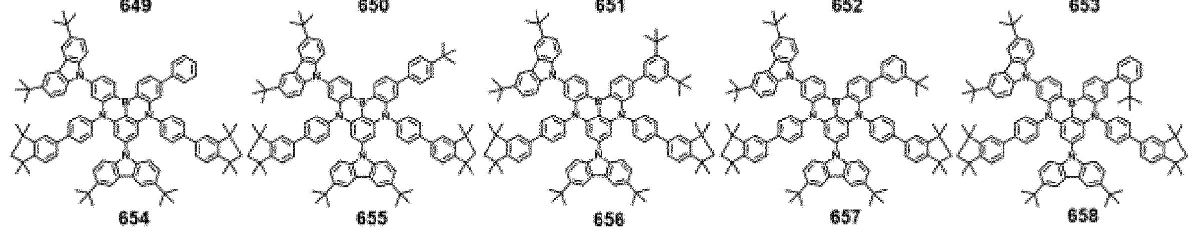

654 655 656 657 658

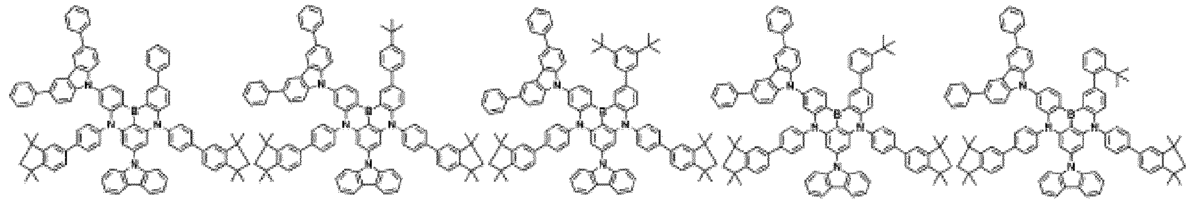

659 660 661 662 663

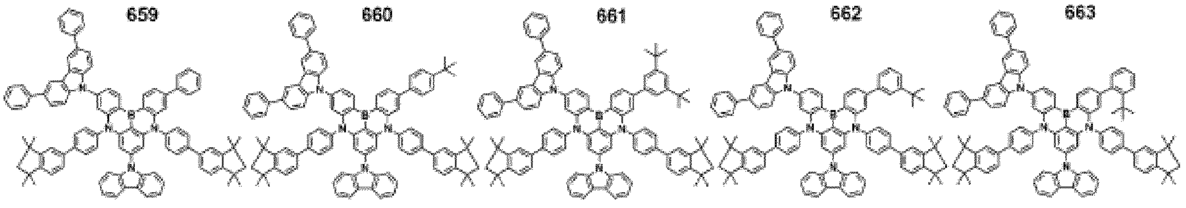

664 665 666 667 668

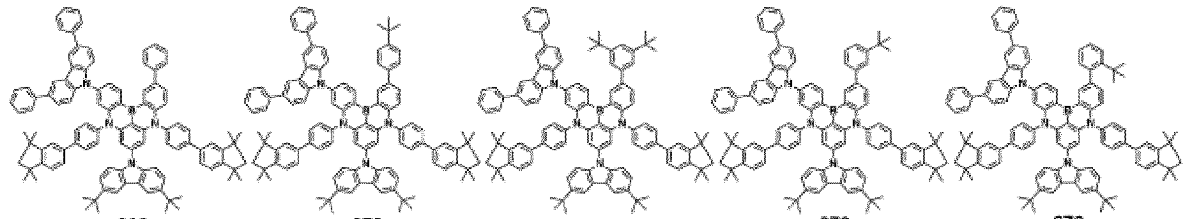

669 670 671 672 673

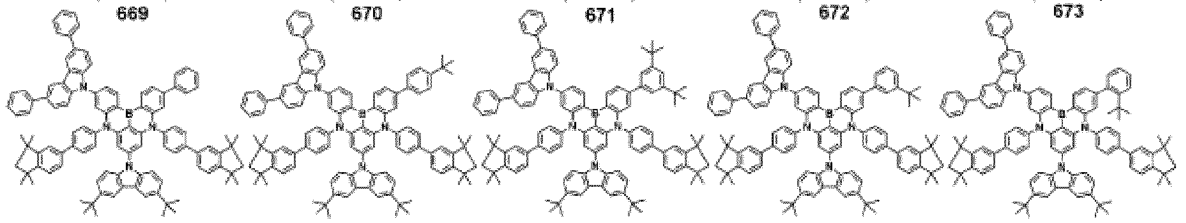

674 675 676 677 678

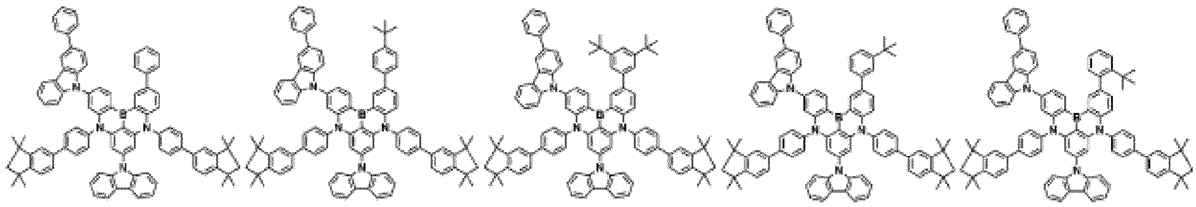

679 680 681 682 683

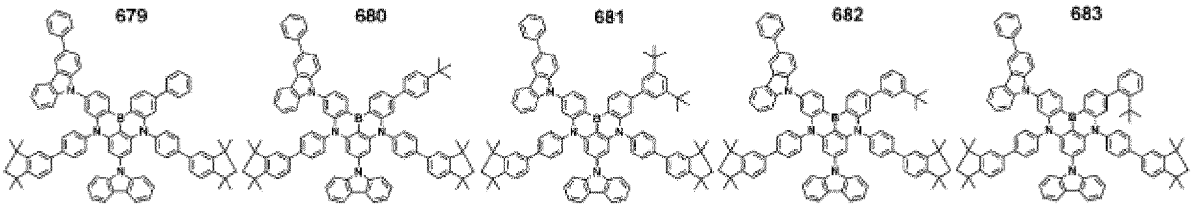

684 685 686 687 688

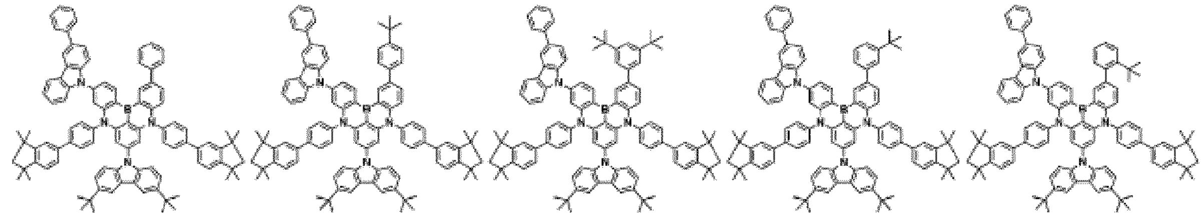

689 690 691 692 693

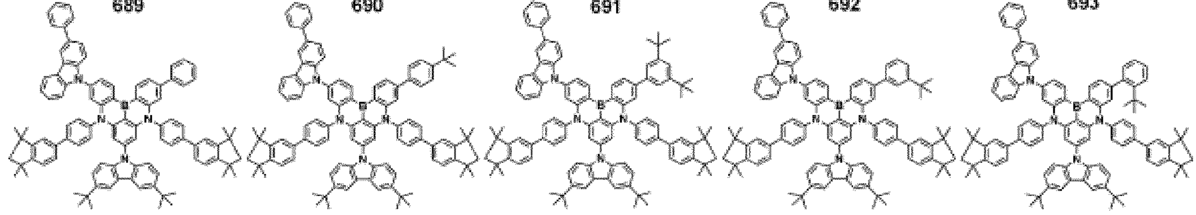

694 695 696 697 698

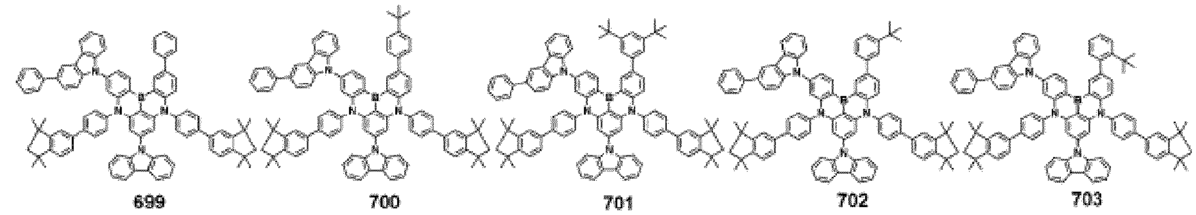

699 700 701 702 703

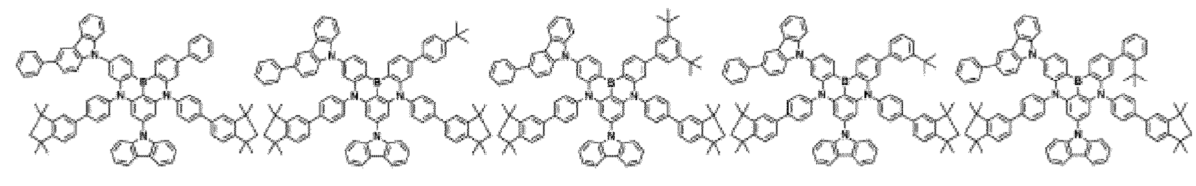

704 705 706 707 708

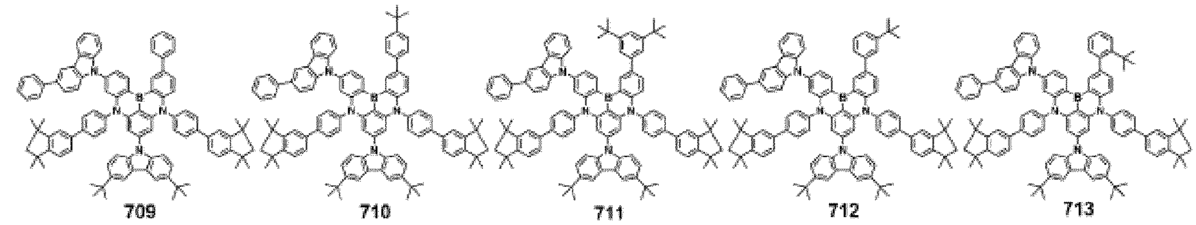

709 710 711 712 713

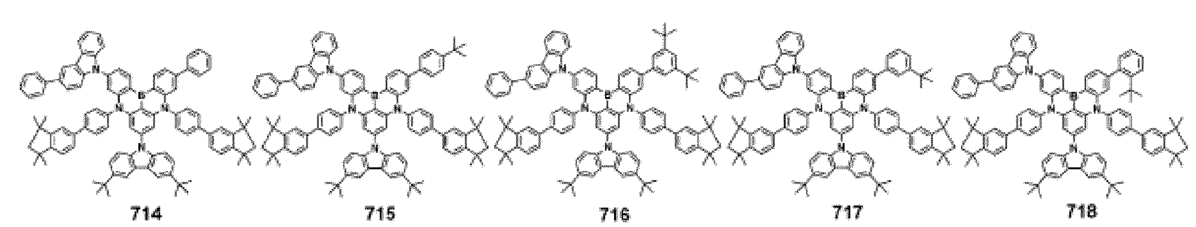

714 715 716 717 718

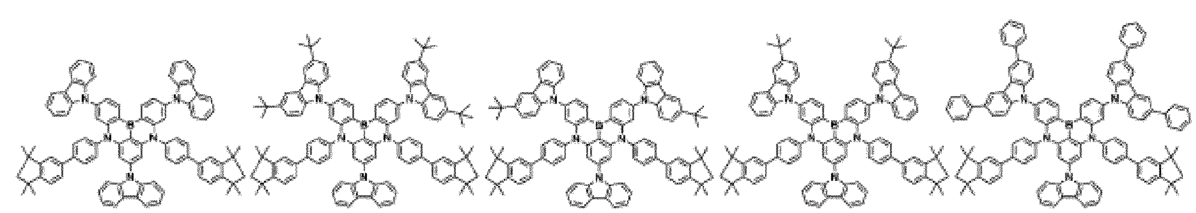

719 720 721 722 723

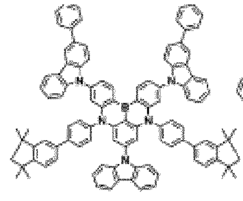
724

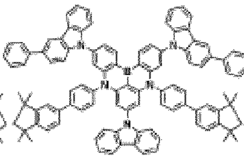
725

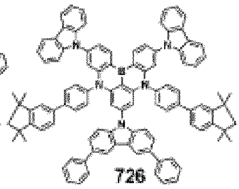
726

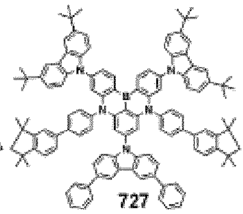
727

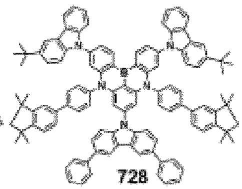
728

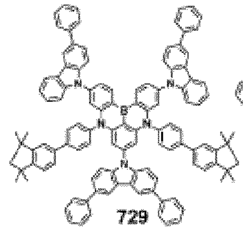
729

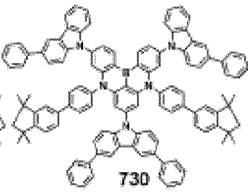
730

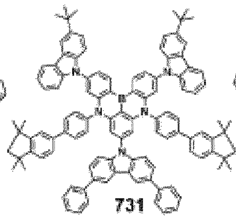
731

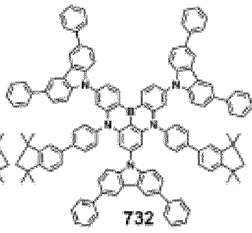
732

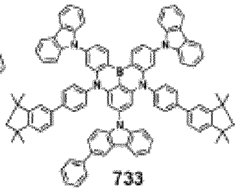
733

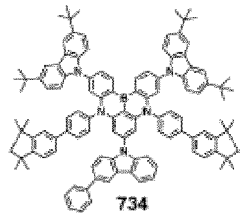
734

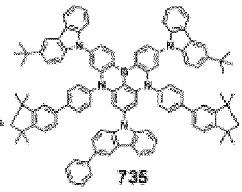
735

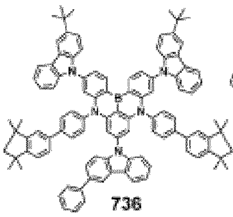
736

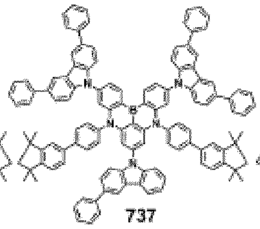
737

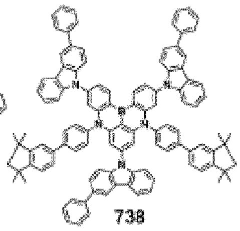
738

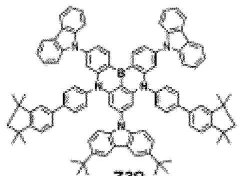
739

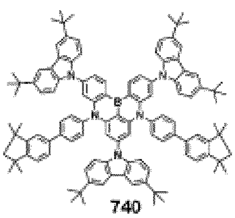
740

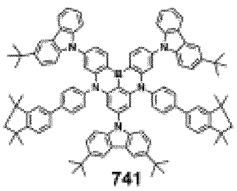
741

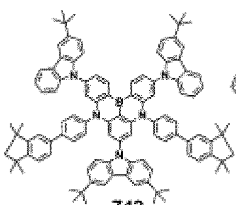
742

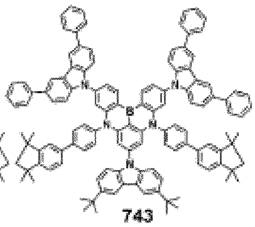
743

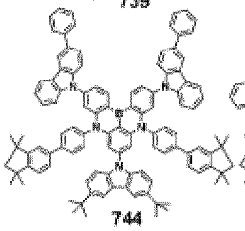
744

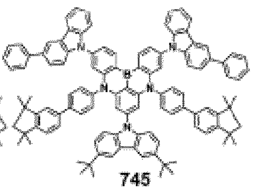
745

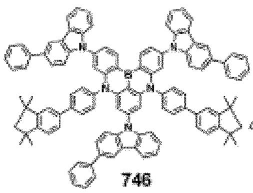
746

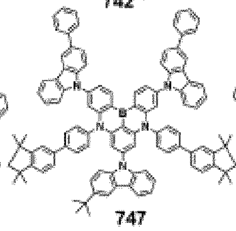
747

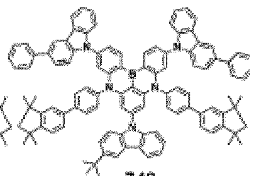
748

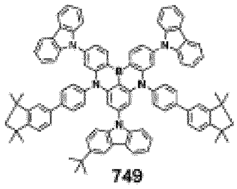
749

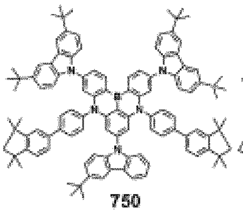
750

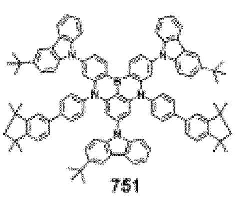
751

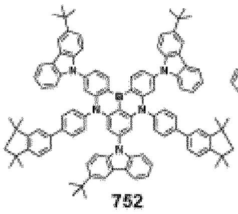
752

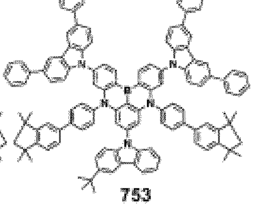
753

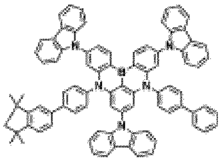
754

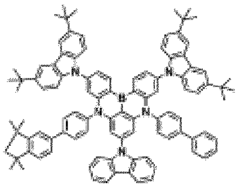
755

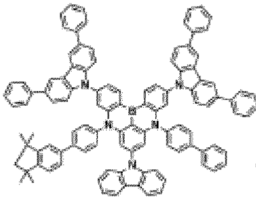
756

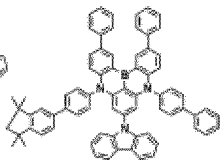
757

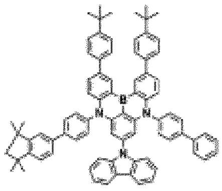
758

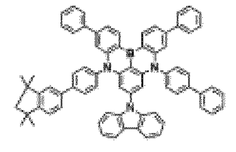
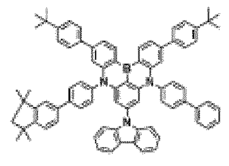
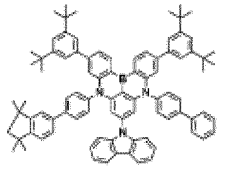
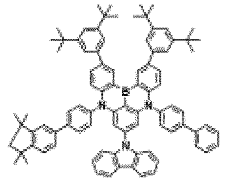
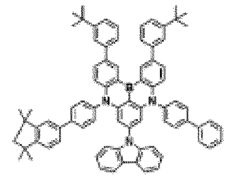

759 760 761 762 763

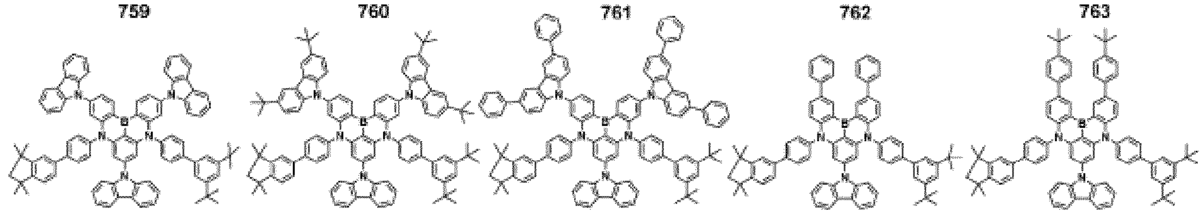

764 765 766 767 768

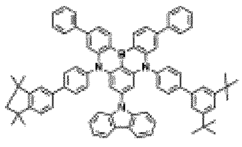
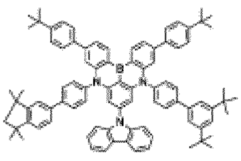
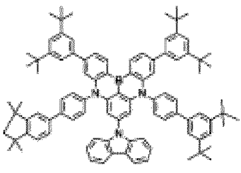
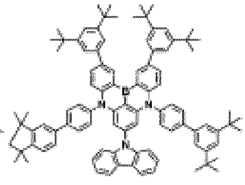
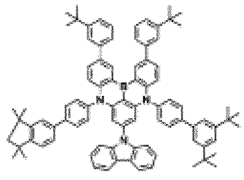

769 770 771 772 773

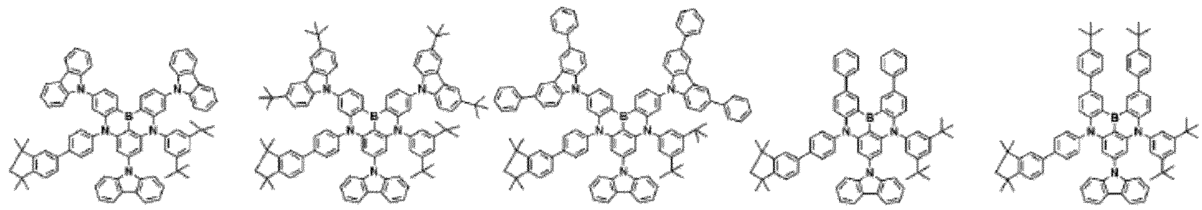

774 775 776 777 778

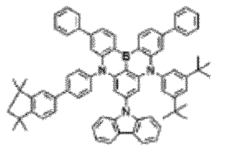
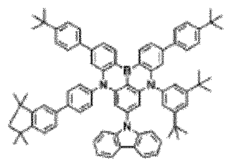
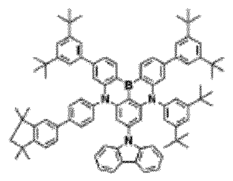
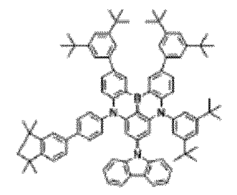
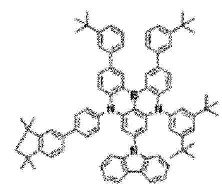

779 780 781 782 783

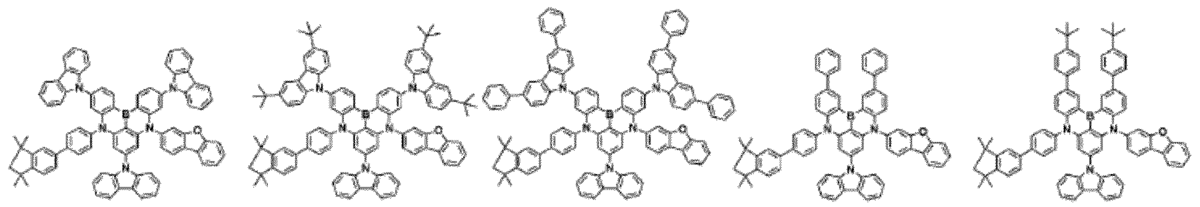

784 785 786 787 788

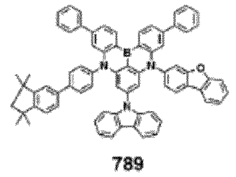
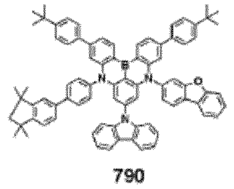
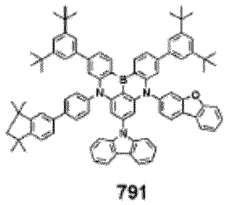
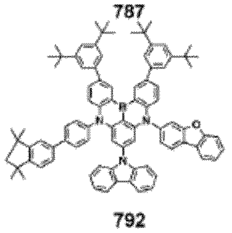

789 790 791 792

.

**10.** Dispositif électroluminescent organique, comprenant :

une première électrode ;
une seconde électrode ; et
une couche organique agencée entre la première électrode et la seconde électrode et comprenant une couche d'émission ;
dans lequel la couche organique comprend le composé hétérocyclique de l'une quelconque des revendications 1 à 9.

**11.** Dispositif électroluminescent organique de la revendication 10, dans lequel la couche d'émission comprend le composé hétérocyclique.

**12.** Dispositif électroluminescent organique de la revendication 11, dans lequel la couche d'émission comprend un hôte et un émetteur, et
l'émetteur comprend le composé hétérocyclique.

**13.** Dispositif électroluminescent organique de la revendication 12, dans lequel une quantité de l'hôte est supérieure à une quantité du composé hétérocyclique ; et/ou
dans lequel la couche d'émission comprend en outre un sensibilisateur, de préférence dans lequel le sensibilisateur comprend un composé phosphorescent, un composé à fluorescence retardée, ou toute combinaison de ceux-ci.

**14.** Dispositif électroluminescent organique de l'une quelconque des revendications 11 à 13, dans lequel la couche d'émission émet une lumière bleue possédant une longueur d'onde d'émission maximale de 400 nm à 490 nm.

**15.** Composition de diagnostic comprenant le composé hétérocyclique de l'une quelconque des revendications 1 à 9.

FIG. 1

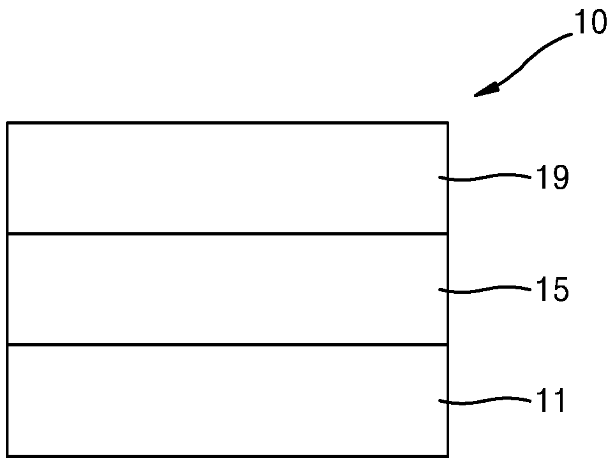

FIG. 2

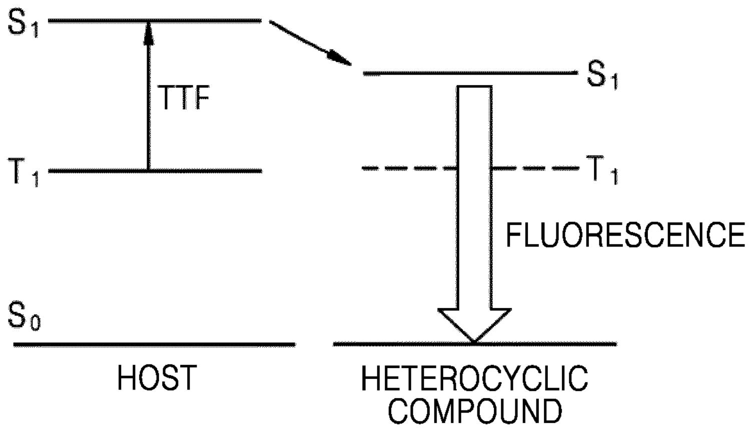

FIG. 3

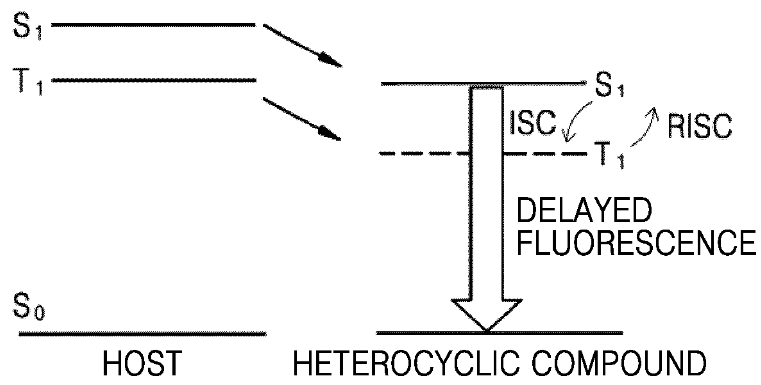
S1
T1
S1
ISC
RISC
T1
DELAYED
FLUORESCENCE
S0
HOST
HETEROCYCLIC COMPOUND

FIG. 4

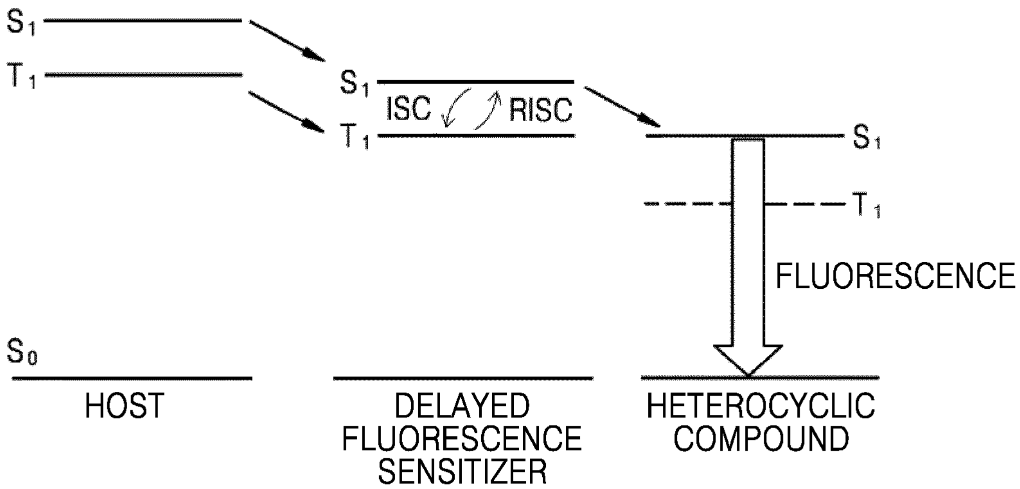
S1
T1
S1
ISC
RISC
T1
S1
T1
FLUORESCENCE
S0
HOST
DELAYED
FLUORESCENCE
SENSITIZER
HETEROCYCLIC
COMPOUND

FIG. 5

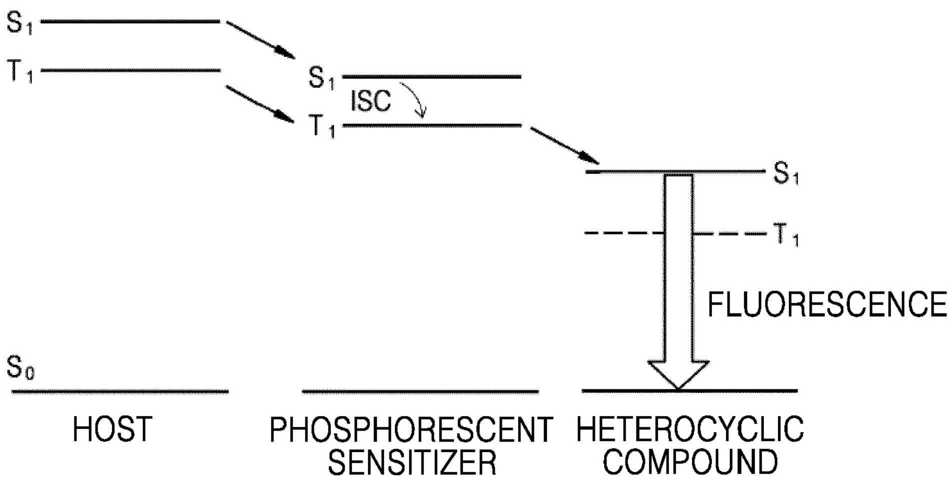
S1
T1
S1
ISC
T1
S1
T1
FLUORESCENCE
S0
HOST
PHOSPHORESCENT SENSITIZER
HETEROCYCLIC COMPOUND

FIG. 6

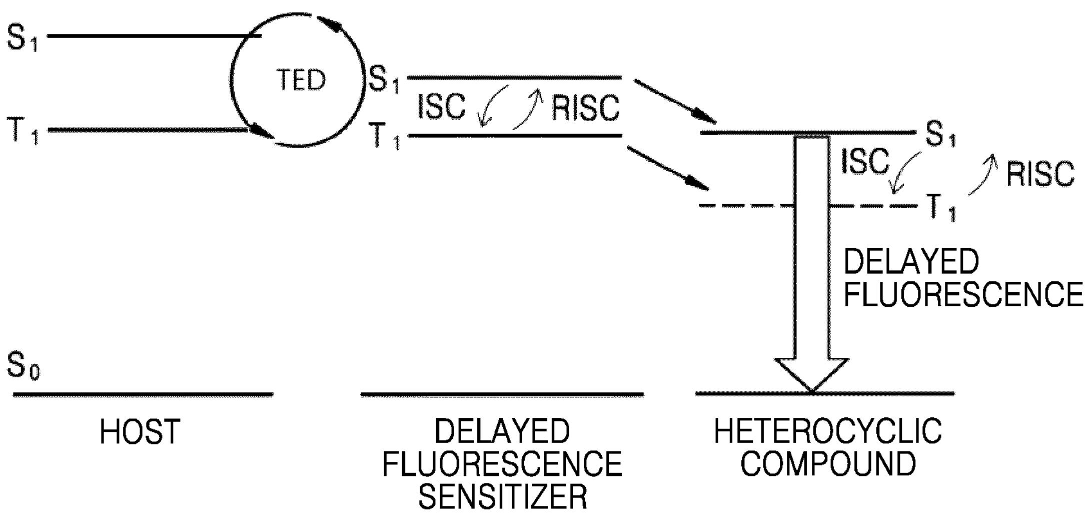
S1
T1
TED
S1
T1
ISC
RISC
S1
ISC
RISC
T1
DELAYED FLUORESCENCE
S0
HOST
DELAYED FLUORESCENCE SENSITIZER
HETEROCYCLIC COMPOUND

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20220073182 A
- CN 114369108 A